# EUROPÄISCHE PATENTSCHRIFT

(11) **EP 2 227 466 B1**
(45) Veröffentlichungstag und Bekanntmachung des Hinweises auf die Patenterteilung: **20.04.2011**
(21) Anmeldenummer: 08854224.6
(22) Anmeldetag: 20.11.2008
(51) Int. Cl.: C07D 413/04, C07D 413/14, C07D 417/14, A61K 31/454, A61K 31/4545, A61P 7/02

(54) **HETEROARYL-SUBSTITUIERTE PIPERIDINE**
HETEROARYL-SUBSTITUTED PIPERIDINES
PIPÉRIDINES À SUBSTITUTION HÉTÉROARYL

(30) Priorität: 30.11.2007 DE 102007057718; 20.02.2008 DE 102008010221
(43) Veröffentlichungstag der Anmeldung: 15.09.2010
(73) Patentinhaber: Bayer Schering Pharma Aktiengesellschaft, 13353 Berlin (DE)
(72) Erfinder: HEIMBACH, Dirk, 40549 Düsseldorf (DE); RÖHRIG, Susanne, 40724 Hilden (DE); SCHNEIDER, Dirk, 42115 Wuppertal (DE); RESTER, Ulrich, 42115 Wuppertal (DE); BENDER, Eckhard, 40764 Langenfeld (DE); MEININGHAUS, Mark, 42327 Wuppertal (DE); ZIMMERMANN, Katja, 40470 Düsseldorf (DE); ZUBOV, Dimitry, 42857 Remscheid (DE); BUCHMÜLLER, Anja, 45259 Essen (DE); VON DEGENFELD, Georges, 51373 Leverkusen (DE); GERDES, Christoph, 51063 Köln (DE); GERISCH, Michael, 42329 Wuppertal (DE); GNOTH, Mark, Jean, 40822 Mettmann (DE); CANCHO GRANDE, Yolanda, 51373 Leverkusen (DE); GERICKE, Kersten, Matthias, 42115 Wuppertal (DE); JESKE, Mario, 42699 Solingen (DE)
(86) Internationale Anmeldenummer: PCT/EP2008/009792
(87) Internationale Veröffentlichungsnummer: WO 2009/068214

(56) Entgegenhaltungen:
- WO-A-03/039440
- WO-A-2007/130898
- US-A- 5 767 144
- US-A1- 2006 004 049
- MOCHIZUKI ET AL: "Design, synthesis, and biological activity of piperidine diamine derivatives as factor Xa inhibitor" BIOORGANIC & MEDICINAL CHEMISTRY LETTERS, PERGAMON, ELSEVIER SCIENCE, GB, Bd. 18, Nr. 2, 17. November 2007 (2007-11-17), Seiten 782-787, XP022424748 ISSN: 0960-894X

## Beschreibung

Die Erfindung betrifft neue Heteroaryl-substituierte Piperidine, Verfahren zu ihrer Herstellung, ihre Verwendung zur Behandlung und/oder Prophylaxe von Krankheiten sowie ihre Verwendung zur Herstellung von Arzneimitteln zur Behandlung und/oder Prophylaxe von Krankheiten, insbesondere von Herz-Kreislauf-Erkrankungen und von Tumorerkrankungen.

Thrombozyten (Blutplättchen) sind ein wesentlicher Faktor sowohl in der physiologischen Blutstillung (Hämostase) als auch bei thromboembolischen Erkrankungen. Insbesondere im arteriellen System kommt Thrombozyten eine zentrale Bedeutung in der komplexen Interaktion zwischen Blutkomponenten und Gefäßwand zu. Unerwünschte Thrombozytenaktivierung kann durch Bildung plättchenreicher Thromben zu thromboembolischen Erkrankungen und thrombotischen Komplikationen mit lebensbedrohlichen Zuständen führen.

Einer der potentesten Plättchenaktivatoren ist die Blutgerinnungsprotease Thrombin, die an verletzten Blutgefäßwänden gebildet wird und neben der Fibrinbildung zur Aktivierung von Thrombozyten, Endothelzellen und mesenchymalen Zellen führt (Vu TKH, Hung DT, Wheaton VI, Coughlin SR, Cell 1991, 64, 1057-1068). An Thrombozyten *in vitro* und in Tiermodellen hemmen Thrombin-Inhibitoren die Plättchenaggregation bzw. die Bildung plättchenreicher Thromben. Beim Menschen können arterielle Thrombosen erfolgreich mit Inhibitoren der Thrombozytenfunktion sowie Thrombin-Inhibitoren verhindert oder behandelt werden (Bhatt DL, Topol EJ, Nat. Rev. Drug Discov. 2003, 2, 15-28). Deshalb besteht eine hohe Wahrscheinlichkeit, dass Antagonisten der Thrombinwirkung auf Blutplättchen die Bildung von Thromben und das Auftreten von klinischen Folgen wie Herzinfarkt und Schlaganfall vermindern. Weitere zelluläre Thrombinwirkungen, z.B. auf Gefäßendothel- und -glattmuskelzellen, Leukozyten und Fibroblasten, sind möglicherweise für entzündliche und proliferative Erkrankungen verantwortlich.

Die zellulären Effekte von Thrombin werden zumindest teilweise über eine Familie G-Proteingekoppelter Rezeptoren (Protease Activated Receptors, PARs) vermittelt, deren Prototyp der PAR-1-Rezeptor darstellt. PAR-1 wird durch Bindung von Thrombin und proteolytische Spaltung seines extrazellulär liegenden N-Terminus aktiviert. Durch die Proteolyse wird ein neuer N-Terminus mit der Aminosäurensequenz SFLLRN... freigelegt, der als Agonist ("Tethered Ligand") zur intramolekularen Rezeptoraktivierung und Übertragung intrazellulärer Signale führt. Von der Tethered-Ligand Sequenz abgeleitete Peptide können als Agonisten des Rezeptors eingesetzt werden und führen auf Thrombozyten zur Aktivierung und Aggregation. Andere Proteasen sind ebenfalls in der Lage, PAR-1 zu aktivieren, hierunter z.B. Plasmin, Faktor VIIa, Faktor Xa, Trypsin, aktiviertes Protein C (aPC), Tryptase, Cathepsin G, Proteinase 3, Granzyme A, Elastase und Matrixmetalloprotease 1 (MMP-1).

Im Gegensatz zur Inhibition der Proteaseaktivität von Thrombin mit direkten Thrombin-Inhibitoren sollte eine Blockade des PAR-1 zur Hemmung der Thrombozytenaktivierung ohne Verminderung der Gerinnungsfähigkeit des Blutes (Antikoagulation) führen.

Antikörper und andere selektive PAR-1-Antagonisten hemmen die Thrombin-induzierte Aggregation von Thrombozyten *in vitro* bei niedrigen bis mittleren Thrombinkonzentrationen (Kahn ML, Nakanishi-Matsui M, Shapiro MJ, Ishihara H, Coughlin SR, J. Clin. Invest. 1999, 103, 879-887). Ein weiterer Thrombinrezeptor mit möglicher Bedeutung für die Pathophysiologie thrombotischer Prozesse, PAR-4, wurde auf humanen und tierischen Thrombozyten identifiziert. In experimentellen Thrombosen an Tieren mit einem dem Menschen vergleichbaren PAR-Expressionsmuster reduzieren PAR-1-Antagonisten die Bildung plättchenreicher Thromben (Derian CK, Damiano BP, Addo MF, Darrow AL, D'Andrea MR, Nedelman M, Zhang H-C, Maryanoff BE, Andrade-Gordon P, J. Pharmacol. Exp. Ther. 2003, 304, 855-861).

In den letzten Jahren wurde eine Vielzahl von Substanzen auf ihre plättchenfunktionshemmende Wirkung geprüft, in der Praxis haben sich aber nur wenige Plättchenfunktionshemmer bewährt. Es besteht daher ein Bedarf an Pharmazeutika, die spezifisch eine gesteigerte Plättchenreaktion hemmen ohne das Blutungsrisiko erheblich zu erhöhen und damit das Risiko von thromboembolischen Komplikationen vermindern.

Effekte von Thrombin, die über den Rezeptor PAR-1 vermittelt werden, haben Auswirkungen auf den Krankheitsverlauf während und nach operativer koronarer Bypassanlage (CABG) sowie anderer Operationen und insbesondere Operationen mit extrakorporalem Kreislauf (z. B. Herz-Lungenmaschine). Im Verlauf der Operation kann es aufgrund der prä- oder intraoperativen Medikation mit gerinnungshemmenden und/oder plättchenhemmenden Substanzen zu Blutungskomplikationen kommen. Aus diesem Grunde muss beispielsweise eine Medikation mit Clopidogrel mehrere Tage vor einer CABG pausiert werden. Außerdem kann es wie erwähnt (z.B. aufgrund des ausgedehnten Kontaktes zwischen Blut und künstlichen Oberflächen bei Einsatz einer extrakorporalen Zirkulation oder bei Bluttransfusionen) zur Ausbildung einer disseminierten intravaskulären Gerinnung oder Verbrauchskoagulopathie (DIC) kommen, die sekundär zu Blutungskomplikationen führen kann. Im weiteren Verlauf kommt es häufig zur Restenose der angelegten venösen oder arteriellen Bypässe (bis hin zum Verschluß) aufgrund von Thrombose, Intimafibrose, Arteriosklerose, Angina pectoris, Myokardinfarkt, Herzinsuffizienz, Arrhythmien, transitorische ischämische Attacke (TIA) und/oder Schlaganfall.

Der Rezeptor PAR-1 wird im Menschen auch auf anderen Zellen exprimiert, hierunter z.B. Endothelzellen, glatte Gefäßmuskelzellen und Tumorzellen. Bösartige Tumorerkrankungen (Krebs) haben eine hohe Inzidenz und sind im Allgemeinen mit einer hohen Sterblichkeit verbunden. Gegenwärtige Therapien erreichen nur in einem Bruchteil der Patienten eine Vollremission und sind typischerweise mit schweren Nebenwirkungen verbunden. Daher besteht ein hoher Bedarf an effektiveren und sichereren Therapien. Der PAR-1-Rezeptor trägt zur Entstehung, dem Wachstum, der Invasivität und der Metastasierung von Krebs bei. Außerdem vermittelt auf Endothelzellen exprimiertes PAR-1 Signale, die in Gefäßwachstum münden ("Angiogenese"), ein Vorgang, der zur Ermöglichung von Tumorwachstum über ca. 1 mm³ hinaus unerläßlich ist. Angiogenese trägt auch zur Enstehung oder Verschlimmerung anderer Erkrankungen bei, hierunter z.B. hämatopoetische Krebserkrankungen, die zur Erblindung fiihrende Makuladegeneration und diabetische Retinopathie, entzündliche Erkrankungen wie rheumatoide Arthritis und Colitis.

Sepsis (oder Septikämie) ist eine häufige Erkrankung mit hoher Letalität. Anfängliche Symptome der Sepsis sind typischerweise unspezifisch (z.B. Fieber, reduziertes Allgemeinbefinden), im weiteren Verlauf kann es jedoch zur generalisierten Aktivierung des Gerinnungssystems kommen ("Disseminated Intravascular coagulation" oder "Verbrauchskoagulopathie" (DIC)) mit Mikrothrombosierung in verschiedenen Organen und sekundärer Blutungskomplikationen. DIC kann auch unabhängig von einer Sepsis auftreten, z.B. im Rahmen von Operationen oder bei Tumorerkrankungen.

Die Therapie der Sepsis besteht einerseits in der konsequenten Beseitigung der infektiösen Ursache, z.B. durch operative Herdsanierung und Antibiose. Andererseits besteht sie in der temporären intensivmedizinischen Unterstützung der beeinträchtigten Organsysteme. Therapien der verschiedenen Stadien dieser Erkrankung sind z.B. in folgender Publikation beschrieben (Dellinger et al., Crit. Care Med. 2004, 32, 858-873). Für die DIC existieren keine erwiesenermaßen effektive Therapien.

Eine Aufgabe der vorliegenden Erfindung ist es daher, neue PAR-1-Antagonisten zur Behandlung von Erkrankungen, wie z. B. Herz-Kreislauf-Erkrankungen und thromboembolischen Erkrankungen, sowie Tumorerkrankungen bei Menschen und Tieren zur Verfügung zu stellen.

WO 2006/012226, WO 2006/020598, WO 2007/038138, WO 2007/130898, WO 2007/101270 und US 2006/0004049 beschreiben strukturell ähnliche Piperidine als 11-β HSD1 Inhibitoren zur Behandlung von unter anderem Diabetes, thromboembolischen Erkrankungen und Schlaganfall.

Gegenstand der Erfindung sind Verbindungen der Formel 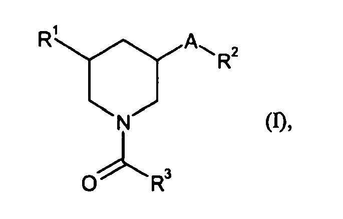 in welcher
- A: für eine Gruppe der Formel 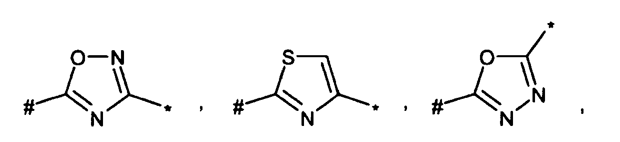 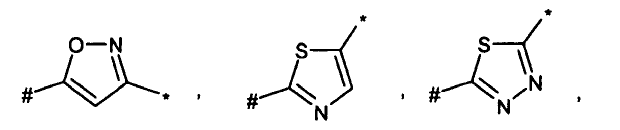 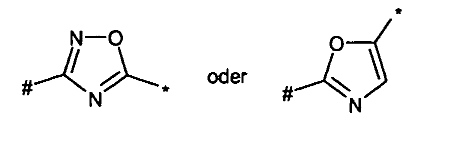 steht,
wobei
# die Anknüpfstelle an den Piperidinring ist,
und
* die Anknüpfstelle an R² ist,
- R¹: für Phenyl steht,
wobei Phenyl substituiert sein kann mit 1 bis 3 Substituenten, unabhängig voneinander ausgewählt aus der Gruppe bestehend aus Monofluormethyl, Difluormethyl, Trifluormethyl, Monofluormethoxy, Difluormethoxy, Trifluormethoxy, Monofluormethylsulfanyl, Difluormethylsulfanyl, Trifluormethylsulfanyl, Methylsulfonyl, C₁C₄-Alkyl, C₁-C₄-Alkoxy, C₁-C₄-Alkoxycarbonyl und C₃-C₆-Cycloalkyl,
worin C₂-C₄-Alkoxy substituiert sein kann mit einem Substituenten ausgewählt aus der Gruppe bestehend aus Methoxy und Ethoxy,
und
worin Cycloalkyl substituiert sein kann mit 1 bis 3 Substituenten, unabhängig voneinander ausgewählt aus der Gruppe bestehend aus Halogen und C₁-C₄-Alkyl,
- R²: für Wasserstoff, Trifluormethyl, Aminomethyl, C₁-C₆-Alkyl, C₂-C₆-Alkenyl, C₁-C₄- Alkoxycarbonyl, C₃-C₆-Cycloalkyl, Cyclopentenyl, 4- bis 6-gliedriges Heterocyclyl, Phenyl, 1,3-Benzodioxolyl, 5- oder 6-gliedriges Heteroaryl oder Pyridylaminocarbonyl steht,
wobei Cycloalkyl, Heterocyclyl, Phenyl und Heteroaryl substituiert sein können mit 1 bis 3 Substituenten, unabhängig voneinander ausgewählt aus der Gruppe bestehend aus Halogen, Cyano, Amino, Monofluormethyl, Difluormethyl, Trifluormethyl, Monofluormethoxy, Difluormethoxy, Trifluormethoxy, Monofluormethylsulfanyl, Difluormethylsulfanyl, Trifluormethylsulfanyl, C₁-C₄-Alkyl, C₁-C₄-Alkoxy, C₁-C₆- Alkylamino, C₁-C₄-Alkoxycarbonylamino, C₃-C₆-Cycloalkyl, 4- bis 6-gliedriges Heterocyclyl, Phenyl und 5- oder 6-gliedriges Heteroaryl,
worin Alkylamino substituiert sein kann mit einem Substituenten ausgewählt aus der Gruppe bestehend aus C₁-C₄-Alkoxy und C₁-C₆-Alkylamino,
und
wobei C₁-C₄-Alkyl substituiert sein kann mit einem Substituenten ausgewählt aus der Gruppe bestehend aus Halogen, Hydroxy, Amino, Aminocarbonyl, C₁-C₄-Alkoxy, C₁-C₆-Alkylamino, C₁-C₄-Alkylthio, C₁-C₄-Alkylcarbonyl, C₁-C₄-Alkylcarbonyloxy, C₁-C₄-Alkylsulfonyl, C₁-C₄-Alkoxycarbonyl, C₁-C₄-Alkoxycarbonylamino, C₃-C₆-Cycloalkyl, C₃-C₆-Cycloalkylamino, 4- bis 6-gliedriges Heterocyclyl, Phenyl, Phenoxy, 5- oder 6-gliedriges Heteroaryl und 5- oder 6-gliedriges Heteroarylthio,
worin Cycloalkyl, Heterocyclyl, Phenyl, Phenoxy, Heteroaryl und Heteroarylthio substituiert sein können mit 1 bis 3 Substituenten, unabhängig voneinander ausgewählt aus der Gruppe bestehend aus Halogen, Cyano, Hydroxymethyl, Monofluormethyl, Difluormethyl, Trifluormethyl, Monofluormethoxy, Difluormethoxy, Trifluormethoxy, Monofluormethylsulfanyl, Difluormethylsulfanyl, Trifluormethylsulfanyl, C₁-C₄-Alkyl, C₁-C₄-Alkoxy, C₃-C₆-Cycloalkyl, 4- bis 6-gliedriges Heterocyclyl, Phenyl und 5- oder 6-gliedriges Heteroaryl, .
- R³: für C₁-C₆-Alkyl, C₁-C₆-Alkoxy, C₁-C₆-Alkylamino, C₃-C₇-Cycloalkyl, 4- bis 6-gliedriges Heterocyclyl, Phenyl oder 5- oder 6-gliedriges Heteroaryl steht,
wobei Alkyl, C₂-C₆-Alkoxy und Alkylamino substituiert sein können mit einem Substituenten ausgewählt aus der Gruppe bestehend aus Hydroxy, Amino, Cyano und C₁- C₄-Alkoxy,
und
wobei Cycloalkyl, Heterocyclyl, Phenyl und Heteroaryl substituiert sein können mit 1 bis 3 Substituenten, unabhängig voneinander ausgewählt aus der Gruppe bestehend aus Halogen, Cyano, Nitro, Oxo, Hydroxy, Amino, Aminomethyl, Monofluormethyl, Difluormethyl, Trifluormethyl, Monofluormethoxy, Difluormethoxy, Trifluormethoxy, Monofluormethylsulfanyl, Difluormethylsulfanyl, Trifluormethylsulfanyl, Hydroxy- carbonyl, Aminocarbonyl, C₁-C₄-Alkyl, C₁-C₄-Alkoxy, C₁-C₆-Alkylamino, C₁-C₄- Alkoxycarbonyl und C₁-C₄-Alkylaminocarbonyl,
und ihre Salze, ihre Solvate und die Solvate ihrer Salze.

Erfindungsgemäße Verbindungen sind die Verbindungen der Formel (I) und deren Salze, Solvate und Solvate der Salze; die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung umfasst deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegenden Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind aber auch Salze, die für pharmazeutische Anwendungen selbst nicht geeignet sind aber beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, *N*-Methylmorpholin, Arginin, Lysin, Ethylendiamin, *N*-Methylpiperidin und Cholin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt.

Außerdem umfasst die vorliegende Erfindung auch Prodrugs der erfindungsgemäßen Verbindungen. Der Begriff "Prodrugs" umfasst Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Im Rahmen der vorliegenden Erfindung haben die Substituenten, soweit nicht anders spezifiziert, die folgende Bedeutung:
Alkyl per se und "Alk" und "Alkyl" in Alkoxy, Alkylamino, Alkylthio, Alkylcarbonyl, Alkoxycarbonyl, Alkoxycarbonylamino, Alkylaminocarbonyl, Alkylcarbonylamino, Alkylcarbonyloxy und Alkylsulfonyl stehen für einen linearen oder verzweigten Alkylrest mit 1 bis 6 Kohlenstoffatomen, beispielhaft und vorzugsweise für Methyl, Ethyl, n-Propyl, iso-Propyl, n-Butyl, tert-Butyl, n-Pentyl und n-Hexyl.
Alkenyl steht für einen linearen oder verzweigten Alkenylrest mit 2 bis 6 Kohlenstoffatomen. Bevorzugt ist ein linearer oder verzweigter Alkenylrest mit 2 bis 4, besonders bevorzugt mit 2 bis 3 Kohlenstoffatomen. Beispielsweise und vorzugsweise seien genannt: Vinyl, Allyl, n-Prop-1-en-1-yl und n-But-2-en-1-yl.
Alkoxy steht beispielhaft und vorzugsweise für Methoxy, Ethoxy, n-Propoxy, iso-Propoxy, n-Butoxy, tert-Butoxy, n-Pentoxy und n-Hexoxy.
Alkylamino steht für einen Alkylaminorest mit einem oder zwei (unabhängig voneinander gewählten) Alkylsubstituenten, beispielhaft und vorzugsweise für Methylamino, Ethylamino, n-Propylamino, iso-Propylamino, tert-Butylamino, *N,N*-Dimethylamino, *N,N*-Diethylamino, *N*-Ethyl-*N-*methylamino, *N*-Methyl-*N*-n-propylamino, *N*-iso-Propyl-*N*-n-propylamino und *N*-tert-Butyl-*N-*methylamino. C₁-C₄-Alkylamino steht beispielsweise für einen Monoalkylaminorest mit 1 bis 4 Kohlenstoffatomen oder für einen Dialkylaminorest mit jeweils 1 bis 4 Kohlenstoffatomen pro Alkylsubstituent.
Alkylthio steht beispielhaft und vorzugsweise für Methylthio, Ethylthio, n-Propylthio, iso-Propylthio, n-Butylthio, tert-Butylthio, n-Pentylthio und n-Hexylthio.
Alkylcarbonyl steht beispielhaft und vorzugsweise für Methylcarbonyl, Ethylcarbonyl, n-Propylcarbonyl, iso-Propylcarbonyl, n-Butylcarbonyl und tert-Butylcarbonyl.
Alkoxycarbonyl steht beispielhaft und vorzugsweise für Methoxycarbonyl, Ethoxycarbonyl, n-Propoxycarbonyl, iso-Propoxycarbonyl, n-Butoxycarbonyl, tert-Butoxycarbonyl, n-Pentoxycarbonyl und n-Hexoxycarbonyl.
Alkoxycarbonylamino steht beispielhaft und vorzugsweise für Methoxycarbonylamino, Ethoxycarbonylamino, n-Propoxycarbonylamino, iso-Propoxycarbonylamino, n-Butoxycarbonylamino und tert-Butoxycarbonylamino.
Alkylaminocarbonyl steht für einen Alkylaminocarbonylrest mit einem oder zwei (unabhängig voneinander gewählten) Alkylsubstituenten, beispielhaft und vorzugsweise für Methylaminocarbonyl, Ethylaminocarbonyl, n-Propylaminocarbonyl, iso-Propylaminocarbonyl, tert-Butylaminocarbonyl, *N,N-*Dimethylaminocarbonyl, *NN*-Diethylaminocarbonyl, *N*-thyl-*N*-methylaminocarbonyl, *N-*Methyl-*N*-n-propylaminocarbonyl, *N*-iso-Propyl-*N*-n-propylaminocarbonyl und *N*-tert-Butyl-*N-*methylaminocarbonyl. C₁-C₄-Alkylaminocarbonyl steht beispielsweise für einen Monoalkylaminocarbonylrest mit 1 bis 4 Kohlenstoffatomen oder für einen Dialkylaminocarbonylrest mit jeweils 1 bis 4 Kohlenstoffatomen pro Alkylsubstituent.
Alkylcarbonylamino steht beispielhaft und vorzugsweise für Methylcarbonylamino, Ethylcarbonylamino, n-Propylcarbonylamino, iso-Propylcarbonylamino, n-Butylcarbonylamino und tert-Butylcarbonylamino.
Alkylcarbonyloxy steht beispielhaft und vorzugsweise für Methylcarbonyloxy, Ethylcarbonyloxy, n-Propylcarbonyloxy, iso-Propylcarbonyloxy, n-Butylcarbonyloxy und tert-Butylcarbonyloxy.
Alkylsulfonyl steht beispielhaft und vorzugsweise für Methylsulfonyl, Ethylsulfonyl, n-Propylsulfonyl, iso-Propylsulfonyl, n-Butylsulfonyl, tert-Butylsulfonyl, n-Pentylsulfonyl und n-Hexylsulfonyl.
Cycloalkyl steht für eine monocyclische Cycloalkylgruppe mit in der Regel 3 bis 7, bevorzugt 5 oder 6 Kohlenstoffatomen, beispielhaft und vorzugsweise für Cycloalkyl seien genannt Cyclopropyl, Cyclobutyl, Cyclopentyl, Cyclohexyl und Cycloheptyl.
Cycloalkylamino steht für eine monocyclische Cycloalkylaminogruppe mit in der Regel 3 bis 6, bevorzugt 3 oder 4 Kohlenstoffatomen, beispielhaft und vorzugsweise für Cycloalkylamino seien genannt Cyclopropylamino, Cyclobutylamino, Cyclopentylamino und Cyclohexylamino.
Heterocyclyl steht für einen monocyclischen, heterocyclischen Rest mit 5 oder 6 Ringatomen und bis zu 3, vorzugsweise bis zu 2 Heteroatomen und/oder Heterogruppen aus der Reihe N, O, S, SO, SO₂, wobei ein Stickstoffatom auch ein N-Oxid bilden kann. Die Heterocyclyl-Reste können gesättigt oder teilweise ungesättigt sein. Bevorzugt sind 5- oder 6-gliedrige, monocyclische gesättigte Heterocyclylreste mit bis zu zwei Heteroatomen aus der Reihe O, N und S, beispielhaft und vorzugsweise für Pyrrolidin-2-yl, Pyrrolidin-3-yl, Pyrrolinyl, Tetrahydrofuranyl, Tetrahydrothienyl, Pyranyl, Piperidin-1-yl, Piperidin-2-yl, Piperidin-3-yl, Piperidin-4-yl, 1,2,5,6-Tetrahydropyridin-3-yl, 1,2,5,6-Tetrahydropyridin-4-yl, Thiopyranyl, Morpholin-1-yl, Morpholin-2-yl, Morpholin-3-yl, Piperazin-1-yl, Piperazin-2-yl.
Heteroaryl steht für einen aromatischen, monocyclischen Rest mit in der Regel 5 oder 6 Ringatomen und bis zu 4 Heteroatomen aus der Reihe S, O und N, wobei ein Stickstoffatom auch ein N-Oxid bilden kann, beispielhaft und vorzugsweise für Thienyl, Furyl, Pyrrolyl, Triazolyl, Oxazolyl, Isoxazolyl, Oxadiazolyl, Pyrazolyl, Imidazolyl, Pyridyl, Pyrimidyl, Pyridazinyl, Pyrazinyl.
Heteroarylthio steht für einen aromatischen, monocyclischen Heteroarylthio-Rest mit in der Regel 5 oder 6 Ringatomen und bis zu 4 Heteroatomen aus der Reihe S, O und N, wobei ein Stickstoffatom auch ein N-Oxid bilden kann, beispielhaft und vorzugsweise für Thienylthio, Furylthio, Pyrrolylthio, Thiazolylthio, Oxazolylthio, Isoxazolylthio, Oxadiazolylthio, Pyrazolylthio, Imidazolylthio, Pyridylthio, Pyrimidylthio, Pyridazinylthio, Pyrazinylthio.
Halogen steht für Fluor, Chlor, Brom und Jod, vorzugsweise für Fluor und Chlor.

In der Formel der Gruppe, die für A stehen kann, steht der Endpunkt der Linie, neben der ein # oder ein * steht, nicht für ein Kohlenstoffatom beziehungsweise eine CH₂-Gruppe, sondern ist Bestandteil der Bindung zu dem Atom, an das A gebunden ist.

Bevorzugt sind Verbindungen der Formel (I), in welcher
in welcher
- A: für eine Gruppe der Formel 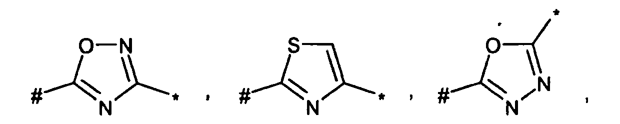 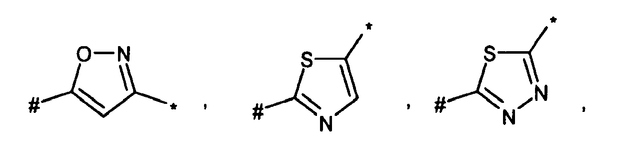 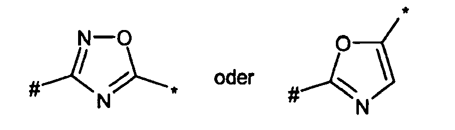 steht,
wobei
# die Anknüpfstelle an den Piperidinring ist,
und
* die Anknüpfstelle an R² ist,
- R¹: für Phenyl steht,
wobei Phenyl substituiert ist mit 1 bis 3 Substituenten, unabhängig voneinander ausgewählt aus der Gruppe bestehend aus Trifluormethyl, Trifluormethoxy, Methylsulfonyl, C₁-C₄-Alkyl und C₁C₄-Alkoxy,
- R²: für Wasserstoff, Trifluormethyl, Aminomethyl, C₁-C₆-Alkyl, C₃-C₆-Alkenyl, Methoxycarbonyl, Ethoxycarbonyl, C₃-C₆-Cycloalkyl, Cyclopentenyl, 4- bis 6-gliedriges Heterocyclyl, Phenyl, 1,3-Benzodioxolyl, 5- oder 6-gliedriges Heteroaryl oder Pyridylaminocarbonyl steht,
wobei Cycloalkyl, Heterocyclyl, Phenyl und Heteroaryl substituiert sein können mit 1 bis 3 Substituenten, unabhängig voneinander ausgewählt aus der Gruppe bestehend aus Halogen, Cyano, Amino, Trifluormethyl, Trifluormethoxy, Methyl, Ethyl, Methoxy, Ethoxy, Ethylamino, tert-Butoxycarbonylamino und 4- bis 6-gliedriges Heterocyclyl,
worin Ethylamino substituiert sein kann mit einem Substituenten ausgewählt aus der Gruppe bestehend aus Methoxy und Dimethylamino,
und
wobei Methyl und Ethyl substituiert sein können mit einem Substituenten ausgewählt aus der Gruppe bestehend aus Halogen, Hydroxy, Amino, Aminocarbonyl, C₁-C₄-Alkoxy, C₁-C₄-Alkylamino, C₁-C₄-Alkylthio, Methylcarbonyl, Ethylcarbonyl, Methylcarbonyloxy, Ethylcarbonyloxy, C₁-C₄-Alkylsulfonyl, Methoxycarbonyl, Ethoxycarbonyl, tert-Butoxycarbonylamino, Cyclopropyl, Cyclopropylamino, 4- bis 6-gliedriges Heterocyclyl, Phenyl, Phenoxy, 5- oder 6-gliedriges Heteroaryl und 5- oder 6-gliedriges Heteroarylthio,
worin Heterocyclyl, Phenyl, Phenoxy, Heteroaryl und Heteroarylthio substituiert sein können mit 1 bis 3 Substituenten, unabhängig voneinander ausgewählt aus der Gruppe bestehend aus Halogen, Cyano, Hydroxymethyl, Trifluormethyl, Trifluormethoxy, Methyl, Ethyl, Methoxy und Ethoxy,
- R³: für C₁-C₆-Alkyl, C₁C₆-Alkylamino, C₃-C₇-Cycloalkyl, 4- bis 6-gliedriges Heterocyclyl, Phenyl oder 5- oder 6-gliedriges Heteroaryl steht,
wobei Alkyl und Alkylamino substituiert sein können mit einem Substituenten ausgewählt aus der Gruppe bestehend aus Hydroxy, Amino, Methoxy und Ethoxy,
und
wobei Cycloalkyl, Heterocyclyl, Phenyl und Heteroaryl substituiert sein können mit 1 bis 3 Substituenten, unabhängig voneinander ausgewählt aus der Gruppe bestehend aus Halogen, Cyano, Oxo, Hydroxy, Amino, Aminomethyl, Trifluormethyl, Trifluormethoxy, Methyl, Ethyl, Methoxy, Ethoxy und Dimethylamino,
und ihre Salze, ihre Solvate und die Solvate ihrer Salze.

Bevorzugt sind auch Verbindungen der Formel (I), in welcher
in welcher
- A: für eine Gruppe der Formel 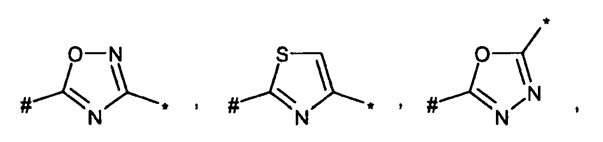 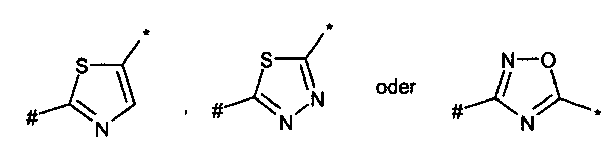 steht,
wobei
# die Anknüpfstelle an den Piperidinring ist,
und
* die Anknüpfstelle an R² ist,
- R¹: für Phenyl steht,
wobei Phenyl substituiert ist mit 1 bis 2 Substituenten, unabhängig voneinander ausgewählt aus der Gruppe bestehend aus Trifluormethyl, Trifluormethoxy, Methyl, Ethyl, Isopropyl und Methoxy,
- R²: für Methyl, Ethyl, Isopropyl, n-Propyl, tert-Butyl, Methoxycarbonyl, Ethoxycarbonyl, Cyclopropyl, Cyclobutyl, Azetidinyl, Oxetanyl, Pyrrolidinyl, Tetrahydropyridinyl, Phenyl, 1,3-Benzodioxolyl, Thienyl, Furanyl, Pyrrolyl, Thiazolyl, Isoxazolyl, Imidazolyl, Triazolyl, Pyridyl, Pyrimidinyl oder Pyrazinyl steht,
wobei Azetidinyl, Oxetanyl, Pyrrolidinyl, Tetrahydropyridinyl, Phenyl, Thienyl, Furanyl, Pyrrolyl, Thiazolyl, Isoxazolyl, Imidazolyl, Triazolyl, Pyridyl, Pyrimidinyl und Pyrazinyl substituiert sein können mit 1 bis 2 Substituenten, unabhängig voneinander ausgewählt aus der Gruppe bestehend aus Halogen, Trifluormethyl, Trifluormethoxy, Methyl, Ethyl, Methoxy, Ethoxy und Ethylamino,
worin Ethylamino substituiert sein kann mit einem Substituenten ausgewählt aus der Gruppe bestehend aus Methoxy und Dimethylamino,
und
wobei Methyl und Ethyl substituiert sein können mit einem Substituenten ausgewählt aus der Gruppe bestehend aus Hydroxy, Amino, Methoxy, Ethoxy, Isopropoxy, Dialkylamino, Methylsulfonyl, Cyclopropylamino, Morpholinyl, Phenyl und Phenoxy,
worin Phenyl substituiert sein kann mit 1 bis 2 Substituenten, unabhängig voneinander ausgewählt aus der Gruppe bestehend aus Halogen, Hydroxymethyl, Trifluormethyl, Trifluormethoxy, Methyl, Ethyl, Methoxy und Ethoxy,
- R³: für tert-Butyl, *N*-Methyl-*N*-ethylamino, Methoxyalkylamino, Cyclopropyl, Cyclopentyl, Azetidinyl, 3,3-Difluorazetidinyl, 3-Hydroxyazetidinyl, 3-Methylazetidinyl, 3- Methoxyazetidinyl, 3-Dimethylaminoazetidinyl, Pyrroldinyl, 3,3-Difluorpyrroldin-1-yl, 3- Hydroxypyrroldin-1-yl, 3-Aminopyrroldin-1-yl, 4,4-Difluorpiperidin-1-yl, 4-Hydroxy- piperidin-1-yl, 4-Aminopiperidin-1-yl, 4-Cyanopiperidin-1-yl, 3-Methoxypiperidin-1-yl, Thiazolidinyl, Morpholin-4-yl, 2,2-Dimethylmorpholin-4-yl, 2-Oxopiperazin-1-yl oder 3- Oxo-4-methyl-piperazin-1-yl steht,
und ihre Salze, ihre Solvate und die Solvate ihrer Salze.

Bevorzugt sind auch Verbindungen der Formel (I), in welcher
in welcher
- A: für eine Gruppe der Formel 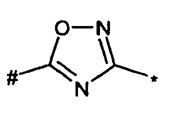 steht,
wobei
# die Anknüpfstelle an den Piperidinring ist,
und
* die Anknüpfstelle an R² ist,
- R¹: für Phenyl steht,
wobei Phenyl substituiert ist mit 1 bis 2 Substituenten, unabhängig voneinander ausgewählt aus der Gruppe bestehend aus Trifluormethyl, Trifluormethoxy, Methyl und Ethyl,
- R²: für Methyl, Ethyl oder Isopropyl steht,
wobei Methyl und Ethyl substituiert sein können mit einem Substituenten Methoxy,
- R³: für 3-Hydroxyazetidinyl, 3-Hydroxypyrroldin-1-yl, 4-Hydroxypiperidin-1-yl, 4-Cyano- piperidin-1-yl oder Morpholin-4-yl steht,
und ihre Salze, ihre Solvate und die Solvate ihrer Salze.

Bevorzugt sind auch Verbindungen der Formel (I), in welcher
- A: für eine Gruppe der Formel 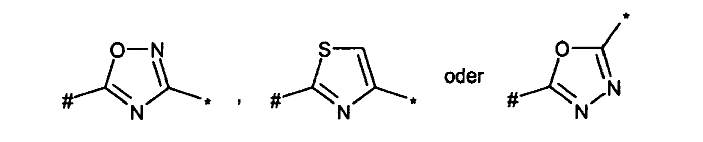 steht,
wobei
# die Anknüpfstelle an den Piperidinring ist,
und
* die Anknüpfstelle an R² ist,
- R¹: für Phenyl steht,
wobei Phenyl substituiert sein kann mit 1 bis 3 Substituenten, unabhängig voneinander ausgewählt aus der Gruppe bestehend aus Monofluormethyl, Difluormethyl, Trifluormethyl, Monofluormethoxy, Difluormethoxy, Trifluormethoxy, Monofluormethylsulfanyl, Difluormethylsulfanyl, Trifluormethylsulfanyl, C₁-C₄-Alkyl, C₁-C₄-Alkoxy, C₁-C₄-Kkoxycarbonyl und C₃-C₆-Cycloalkyl,
worin C₂-C₄-Alkoxy substituiert sein kann mit einem Substituenten ausgewählt aus der Gruppe bestehend aus Methoxy und Ethoxy,
und
worin Cycloalkyl substituiert sein kann mit 1 bis 3 Substituenten, unabhängig voneinander ausgewählt aus der Gruppe bestehend aus Halogen und C₁-C₄-Alkyl,
- R²: für Wasserstoff, Aminomethyl, C₁-C₆-Alkyl, C₁-C₄-Alkoxycarbonyl, C₃-C₆-Cycloalkyl, 4- bis 6-gliedriges Heterocyclyl, Phenyl, 1,3-Benzodioxolyl oder 5- oder 6-gliedriges Heteroaryl steht,
wobei Cycloalkyl, Heterocyclyl, Phenyl und Heteroaryl substituiert sein können mit 1 bis 3 Substituenten, unabhängig voneinander ausgewählt aus der Gruppe bestehend aus Halogen, Cyano, Monofluormethyl, Difluormethyl, Trifluormethyl, Monofluormethoxy, Difluormethoxy, Trifluormethoxy, Monofluormethylsulfanyl, Difluormethylsulfanyl, Trifluormethylsulfanyl, C₁-C₄-Alkyl, C₁-C₄-Alkoxy, C₁-C₆-Alkylamino, C₃-C₆-Cycloalkyl, 4- bis 6-gliedriges Heterocyclyl, Phenyl und 5- oder 6-gliedriges Heteroaryl,
worin Alkylamino substituiert sein kann mit einem Substituenten ausgewählt aus der Gruppe bestehend aus C₁-C₄-Alkoxy und C₁-C₆-Alkylamino,
und
wobei C₁-C₂-Alkyl substituiert sein kann mit einem Substituenten ausgewählt aus der Gruppe bestehend aus C₁-C₄-Alkylsulfonyl, C₁-C₄-Alkoxycarbonylamino, C₃-C₆- Cycloalkyl, 4- bis 6-gliedriges Heterocyclyl, Phenyl, Phenoxy und 5- oder 6-gliedriges Heteroaryl,
worin Cycloalkyl, Heterocyclyl, Phenyl, Phenoxy und Heteroaryl substituiert sein können mit 1 bis 3 Substituenten, unabhängig voneinander ausgewählt aus der Gruppe bestehend aus Halogen, Cyano, Monofluormethyl, Difluormethyl, Trifluormethyl, Monofluormethoxy, Difluormethoxy, Trifluormethoxy, Monofluormethylsulfanyl, Difluormethylsulfanyl, Trifluormethylsulfanyl, C₁-C₄- Alkyl, C₁-C₄-Alkoxy, C₃-C₆-Cycloalkyl, 4- bis 6-gliedriges Heterocyclyl, Phenyl und 5- oder 6-gliedriges Heteroaryl,
- R³: für C₁-C₆-Alkyl, C₁-C₆-Alkoxy, C₁-C₆-Alkylamino, C₃-C₇-Cycloalkyl, 4- bis 6-gliedriges Heterocyclyl, Phenyl oder 5- oder 6-gliedriges Heteroaryl steht,
wobei Alkyl, C₂-C₆-Alkoxy und Alkylamino substituiert sein können mit einem Substituenten ausgewählt aus der Gruppe bestehend aus Hydroxy, Cyano und C₁-C₄- Alkoxy,
und
wobei Cycloalkyl, Heterocyclyl, Phenyl und Heteroaryl substituiert sein können mit 1 bis 3 Substituenten, unabhängig voneinander ausgewählt aus der Gruppe bestehend aus Halogen, Cyano, Nitro, Oxo, Hydroxy, Amino, Monofluormethyl, Difluormethyl, Trifluormethyl, Monofluormethoxy, Difluormethoxy, Trifluormethoxy, Monofluormethylsulfanyl, Difluormethylsulfanyl, Trifluormethylsulfanyl, Hydroxy- carbonyl, Aminocarbonyl, C₁-C₄-Alkyl, C₁-C₄-Alkoxy, C₁-C₆-Alkylamino, C₁-C₄- Alkoxycarbonyl und C₁-C₄-Alkylaminocarbonyl,
und ihre Salze, ihre Solvate und die Solvate ihrer Salze.

Bevorzugt sind auch Verbindungen der Formel (I), in welcher
- A: für eine Gruppe der Formel 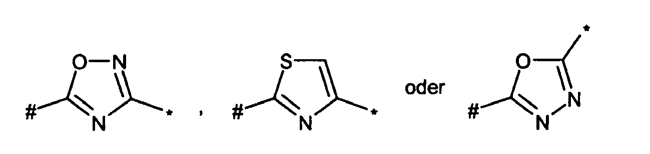 steht,
wobei
# die Anknüpfstelle an den Piperidinring ist,
und
* die Anknüpfstelle an R² ist,
- R¹: für Phenyl steht,
wobei Phenyl substituiert ist mit 1 bis 3 Substituenten, unabhängig voneinander ausgewählt aus der Gruppe bestehend aus Trifluormethyl, Trifluormethoxy, C₁-C₄-Alkyl und C₁-C₄-Alkoxy,
- R²: für Wasserstoff, Aminomethyl, C₁-C₄-Alkyl, Methoxycarbonyl, Ethoxycarbonyl, 4- bis 6- gliedriges Heterocyclyl, Phenyl, 1,3-Benzodioxolyl oder 5- oder 6-gliedriges Heteroaryl steht,
wobei Heterocyclyl, Phenyl und Heteroaryl substituiert sein können mit 1 bis 3 Substituenten, unabhängig voneinander ausgewählt aus der Gruppe bestehend aus Halogen, Cyano, Trifluormethyl, Trifluormethoxy, Methyl, Ethyl, Methoxy, Ethoxy, Ethylamino und 4- bis 6-gliedriges Heterocyclyl,
worin Ethylamino substituiert sein kann mit einem Substituenten ausgewählt aus der Gruppe bestehend aus Methoxy und Dimethylamino,
und
wobei Methyl substituiert sein kann mit einem Substituenten ausgewählt aus der Gruppe bestehend aus Methylsulfonyl, Ethylsulfonyl, tert-Butoxycarbonylamino, 4- bis 6- gliedriges Heterocyclyl, Phenyl, Phenoxy und 5- oder 6-gliedriges Heteroaryl,
worin Heterocyclyl, Phenyl, Phenoxy und Heteroaryl substituiert sein können mit 1 bis 3 Substituenten, unabhängig voneinander ausgewählt aus der Gruppe beste- hend aus Halogen, Cyano, Trifluormethyl, Trifluormethoxy, Methyl, Ethyl, Methoxy und Ethoxy,
- R³: für C₁-C₆-Alkyl, C₁-C₆-Alkylamino, C₃-C₇-Cycloalkyl, 4- bis 6-gliedriges Heterocyclyl, Phenyl oder 5- oder 6-gliedriges Heteroaryl steht,
wobei Alkylamino substituiert sein kann mit einem Substituenten ausgewählt aus der Gruppe bestehend aus Hydroxy, Methoxy und Ethoxy,
und
wobei Cycloalkyl, Heterocyclyl, Phenyl und Heteroaryl substituiert sein können mit 1 bis 3 Substituenten, unabhängig voneinander ausgewählt aus der Gruppe bestehend aus Halogen, Cyano, Oxo, Hydroxy, Amino, Trifluormethyl, Trifluormethoxy, Methyl, Ethyl, Methoxy und Ethoxy,
und ihre Salze, ihre Solvate und die Solvate ihrer Salze.

Bevorzugt sind auch Verbindungen der Formel (I), in welcher
- A: für eine Gruppe der Formel 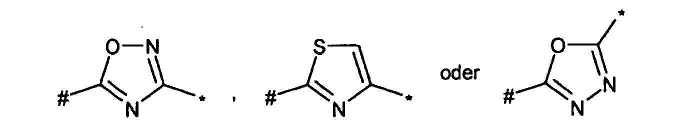 steht,
wobei
# die Anknüpfstelle an den Piperidinring ist,
und
* die Anknüpfstelle an R² ist,
- R¹: für Phenyl steht,
wobei Phenyl substituiert ist mit einem Substituenten ausgewählt aus der Gruppe beste- hend aus Trifluormethyl, Trifluormethoxy, Ethyl, Isopropyl und Methoxy,
- R²: für Wasserstoff, Aminomethyl, Methyl, n-Propyl, tert-Butyl, Methoxycarbonyl, Ethoxycarbonyl, Tetrahydropyridinyl, Phenyl, 1,3-Benzodioxolyl, Thiazolyl, Isoxazolyl, Pyridyl oder Pyrazinyl steht,
wobei Tetrahydropyridinyl, Phenyl, Thiazolyl, Isoxazolyl, Pyridyl und Pyrazinyl substituiert sein können mit 1 bis 3 Substituenten, unabhängig voneinander ausgewählt aus der Gruppe bestehend aus Halogen, Trifluormethyl, Trifluormethoxy, Methyl, Ethyl, Methoxy, Ethoxy, Ethylamino und Morpholinyl,
worin Ethylamino substituiert sein kann mit einem Substituenten ausgewählt aus der Gruppe bestehend aus Methoxy und Dimethylamino,
und
wobei Methyl substituiert sein kann mit einem Substituenten ausgewählt aus der Gruppe bestehend aus Methylsulfonyl, tert-Butoxycarbonylamino, Morpholinyl, Phenyl und Phenoxy,
worin Morpholinyl, Phenyl und Phenoxy substituiert sein können mit 1 bis 3 Substituenten, unabhängig voneinander ausgewählt aus der Gruppe bestehend aus Halogen, Trifluormethyl, Trifluormethoxy, Methyl, Ethyl, Methoxy und Ethoxy,
- R³: für tert-Butyl, *N*-Methyl-*N*-ethylamino, Methoxyalkylamino, Cyclopentyl, Pyrroldinyl, 4- Hydroxypiperidin-1-yl, 4-Cyanopiperidin-1-yl oder Morpholinyl steht,

und ihre Salze, ihre Solvate und die Solvate ihrer Salze.

Bevorzugt sind auch Verbindungen der Formel (I), in welcher
- A: für eine Gruppe der Formel 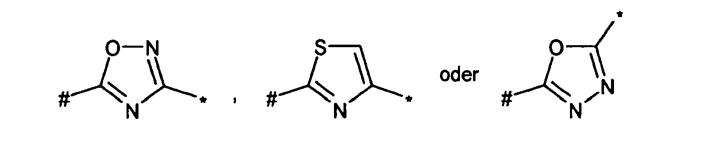 steht,
wobei
# die Anknüpfstelle an den Piperidinring ist,
und
* die Anknüpfstelle an R² ist,
- R¹: für Phenyl steht,
wobei Phenyl substituiert ist mit einem Substituenten ausgewählt aus der Gruppe beste- hend aus Trifluormethyl, Trifluormethoxy, Ethyl, Isopropyl und Methoxy,
- R²: für Wasserstoff, Aminomethyl, Methyl, n-Propyl, tert-Butyl, Methoxycarbonyl, Ethoxycarbonyl, Tetrahydropyridinyl, Phenyl, 1,3-Benzodioxolyl, Thiazolyl, Isoxazolyl, Pyridyl oder Pyrazinyl steht,
wobei Tetrahydropyridinyl, Phenyl, Thiazolyl, Isoxazolyl, Pyridyl und Pyrazinyl substituiert sein können mit 1 bis 3 Substituenten, unabhängig voneinander ausgewählt aus der Gruppe bestehend aus Halogen, Trifluormethyl, Trifluormethoxy, Methyl, Ethyl, Methoxy, Ethoxy, Ethylamino und Morpholinyl,
worin Ethylamino substituiert sein kann mit einem Substituenten ausgewählt aus der Gruppe bestehend aus Methoxy und Dimethylamino,
und
wobei Methyl substituiert sein kann mit einem Substituenten ausgewählt aus der Gruppe bestehend aus Methylsulfonyl, tert-Butoxycarbonylamino, Morpholinyl, Phenyl und Phenoxy,
worin Morpholinyl, Phenyl und Phenoxy substituiert sein können mit 1 bis 3 Substituenten, unabhängig voneinander ausgewählt aus der Gruppe bestehend aus Halogen, Trifluormethyl, Trifluormethoxy, Methyl, Ethyl, Methoxy und Ethoxy,
- R³: für tert-Butyl, *N*-Methyl-*N*-ethylamino, Methoxyalkylamino, Cyclopentyl oder Morpholinyl steht,
und ihre Salze, ihre Solvate und die Solvate ihrer Salze.

Bevorzugt sind auch Verbindungen der Formel (I), in welcher die Substituenten -R' und -A-R² in cis-Position zueinander stehen.

Bevorzugt sind auch Verbindungen der Formel (I), in welcher
- A: für eine Gruppe der Formel 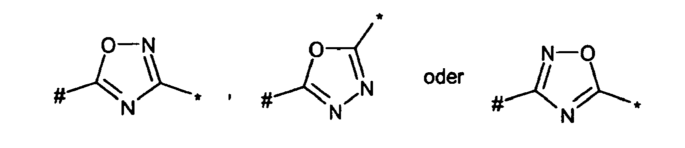 steht,
wobei
# die Anknüpfstelle an den Piperidinring ist,
und
* die Anknüpfstelle an R² ist.

Bevorzugt sind auch Verbindungen der Formel (I), in welcher
- A: für eine Gruppe der Formel 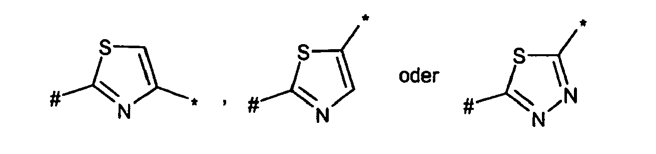 steht,
wobei
# die Anknüpfstelle an den Piperidinring ist,
und
* die Anknüpfstelle an R² ist.

Bevorzugt sind auch Verbindungen der Formel (I), in welcher
- A: für eine Gruppe der Formel 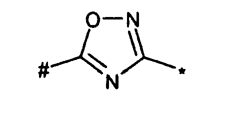 steht,
wobei
# die Anknüpfstelle an den Piperidinring ist,
und
* die Anknüpfstelle an R² ist.

Bevorzugt sind auch Verbindungen der Formel (I), in welcher
- A: für eine Gruppe der Formel 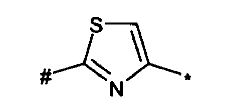 steht,
wobei
# die Anknüpfstelle an den Piperidinring ist,
und
* die Anknüpfstelle an R² ist.

Bevorzugt sind auch Verbindungen der Formel (I), in welcher
- A: für eine Gruppe der Formel 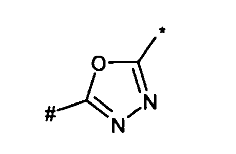 steht,
wobei
# die Anknüpfstelle an den Piperidinring ist,
und
* die Anknüpfstelle an R² ist.

Bevorzugt sind auch Verbindungen der Formel (I), in welcher
- A: für eine Gruppe der Formel 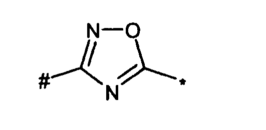 steht,
wobei
# die Anknüpfstelle an den Piperidinring ist,
und
* die Anknüpfstelle an R² ist.

Bevorzugt sind auch Verbindungen der Formel (I), in welcher R¹ für Phenyl steht, wobei Phenyl substituiert ist mit einem Substituenten in para-Position zur Verknüpfungsstelle an den Piperidinring, ausgewählt aus der Gruppe bestehend aus Trifluormethyl, Trifluormethoxy und Ethyl.

Bevorzugt sind auch Verbindungen der Formel (I), in welcher R² für Methyl, Ethyl oder Isopropyl steht, wobei Methyl und Ethyl substituiert sein können mit einem Substituenten Methoxy.

Bevorzugt sind auch Verbindungen der Formel (I), in welcher R² für Methyl, Ethyl oder Isopropyl steht.

Bevorzugt sind auch Verbindungen der Formel (I), in welcher R² für 2-Methoxy-eth-1-yl steht.

Bevorzugt sind auch Verbindungen der Formel (I), in welcher R³ für 3-Hydroxyazetidinyl, 3-Hydroxypyrroldin-1-yl, 4-Hydroxypiperidin-1-yl, 4-Cyanopiperidin-1-yl oder Morpholin-4-yl steht.

Bevorzugt sind auch Verbindungen der Formel (I), in welcher R³ für Morpholin-4-yl steht.

Bevorzugt sind auch Verbindungen der Formel (I), in welcher R³ für 4-Hydroxypiperidin-1-yl steht.

Die in den jeweiligen Kombinationen bzw. bevorzugten Kombinationen von Resten im einzelnen angegebenen Reste-Definitionen werden unabhängig von den jeweiligen angegebenen Kombinationen der Reste beliebig auch durch Reste-Definitionen anderer Kombinationen ersetzt.

Ganz besonders bevorzugt sind Kombinationen von zwei oder mehreren der oben genannten Vorzugsbereiche.

Gegenstand der Erfindung ist weiterhin ein Verfahren zur Herstellung der Verbindungen der Formel (I), oder ihrer Salze, ihrer Solvate oder der Solvate ihrer Salze, wobei entweder
[A] Verbindungen der Formel 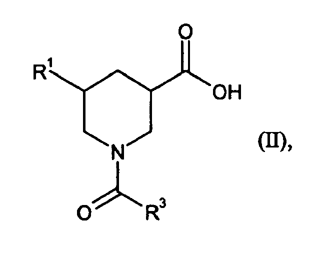 in welcher
   R¹ und R³ die oben angegebene Bedeutung aufweisen,
   mit Verbindungen der Formel 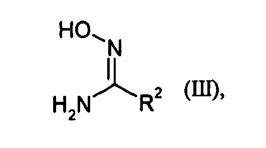 in welcher
   R² die oben angegebene Bedeutung aufweist,
   umgesetzt werden
   oder
[B] Verbindungen der Formel 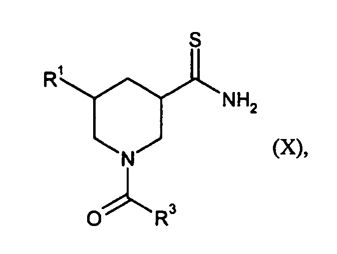 in welcher
   R¹ und R³ die oben angegebene Bedeutung aufweisen,
   mit Verbindungen der Formel 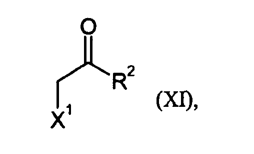 in welcher
   R² die oben angegebene Bedeutung aufweist, und
   X¹ für Brom oder Chlor steht,
   umgesetzt werden
   oder
[C] Verbindungen der Formel (II) mit Verbindungen der Formel

   R²CONHNH₂ (XVI),

   in welcher
   R² die oben angegebene Bedeutung aufweist,
   in Gegenwart von Phosphorylchlorid oder Thionylchlorid umgesetzt werden
   oder
[D] Verbindungen der Formel (II) in der ersten Stufe mit Verbindungen der Formel

   R²COCH₂NH₂ (XVII),

   in welcher
   R² die oben angegebene Bedeutung aufweist,
   in Gegenwart von Phosphorylchlorid oder Thionylchlorid,
   und in der zweiten Stufe mit Lawesson-Reagens umgesetzt werden
   oder
[E] Verbindungen der Formel (II) mit Verbindungen der Formel (XVII) in Gegenwart von Phosphorylchlorid oder Thionylchlorid umgesetzt werden
   oder
[F] Verbindungen der Formel (II) in der ersten Stufe mit Verbindungen der Formel (XVI) in Gegenwart von Phosphorylchlorid oder Thionylchlorid und in der zweiten Stufe mit Lawesson-Reagens umgesetzt werden
   oder
[G] Verbindungen der Formel 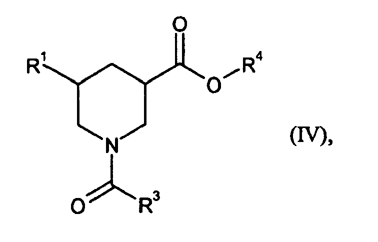 in welcher
   R¹ und R³ die oben angegebene Bedeutung aufweisen, und
   R⁴ für Methyl oder Ethyl steht,
   mit Verbindungen der Formel 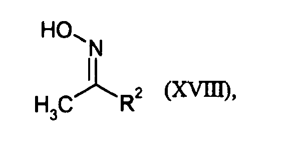 in welcher
   R² die oben angegebene Bedeutung aufweist,
   in Gegenwart von Butyllithium umgesetzt werden
   oder
[H] Verbindungen der Formel 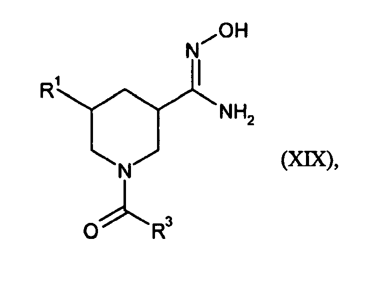 in welcher
   R¹ und R³ die oben angegebene Bedeutung aufweisen,
   mit Verbindungen der Formel 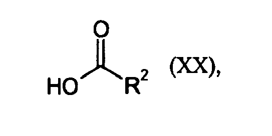 in welcher
   R² die oben angegebene Bedeutung aufweist,
   umgesetzt werden
   oder
[J] Verbindungen der Formel 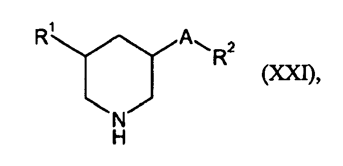 in welcher
   A, R¹ und R² die oben angegebene Bedeutung aufweisen,
   in der ersten Stufe mit 4-Nitrophenylchloroformat und in der zweiten Stufe mit Verbindungen der Formel

   R³-H (XXII),

   in welcher
   R³ die oben angegebene Bedeutung aufweist,
   umgesetzt werden.

Die Umsetzung nach Verfahren [A] erfolgt im Allgemeinen in inerten Lösungsmitteln, in Gegenwart eines Dehydratisierungsreagenzes, gegebenenfalls in Gegenwart einer Base, bevorzugt in einem Temperaturbereich von Raumtemperatur bis zum Rückfluss der Lösungsmittel bei Normaldruck.

Inerte Lösungsmittel sind beispielsweise Halogenkohlenwasserstoffe wie Methylenchlorid, Trichlormethan oder 1,2-Dichlorethan, Ether wie Dioxan, Tetrahydrofuran oder 1,2-Dimethoxyethan, oder andere Lösemittel wie Aceton, Dimethylformamid, Dimethylacetamid, 2-Butanon oder Acetonitril. Ebenso ist es möglich, Gemische der Lösemittel einzusetzen. Bevorzugt ist Dimethylformamid oder ein Gemisch aus Dioxan und Dimethylformamid.

Als Dehydratisierungsreagenzien eignen sich hierbei beispielsweise Carbodiimide wie z.B. *N,N'-*Diethyl-, *N,N*'-Dipropyl-, *N,N*=Diisopropyl-, *N,N*'-Dicyclohexylcarbodiimid, *N*-(3-Dimethylaminoisopropyl)-*N*'-ethylcarbodiimid-hydrochlorid (EDC), *N*-Cyclohexylcarbodiimid-*N*'-propyloxymethyl-Polystyrol (PS-Carbodiimid) oder Carbonylverbindungen wie Carbonyldiimidazol, oder 1,2-Oxazoliumverbindungen wie 2-Ethyl-5-phenyl-1,2-oxazolium-3-sulfat oder 2-tert-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Bis-(2-oxo-3-oxazolidinyl)-phosphorylchlorid oder Benzotriazolyloxy-tri(dimethylamino)-phosphoniumhexafluorophosphat, oder *O*-(Benzotriazol-1-yl)-*N*,*N*,*N'*,*N'*-tetra-methyluroniumhexafluorophosphat (HBTU), 2-(2-Oxo-1-(2H)-pyridyl)-1,1,3,3-tetramethyluroniumtetrafluoroborat (TPTU) oder *O*-(7-Azabenzotriazol-1-yl)-*N*,*N*,*N'*,*N'*-tetramethyl-uroniumhexafluorophosphat (HATU), oder 1-Hydroxybenzotriazol (HOBt), oder Benzotriazol-1-yloxytris(dimethylamino)-phosphoniumhexafluorophosphat (BOP), oder *N*-Hydroxysuccinimid, oder Mischungen aus diesen, mit Basen.

Basen sind beispielsweise Alkalicarbonate, wie z.B. Natrium- oder Kaliumcarbonat, oder -hydrogencarbonat, oder organische Basen wie Trialkylamine z.B. Triethylamin, *N*-Methylmorpholin, *N*-Methylpiperidin, 4-Dimethylaminopyridin oder Diisopropylethylamin, bevorzugt ist Diisopropylethylamin.

Vorzugsweise wird die Kondensation mit HATU in Gegenwart von Diisopropylethylamin oder als Alternative nur mit Carbonyldiimidazol durchgeführt.

Die Verbindungen der Formel (III) sind bekannt oder lassen sich nach bekannten Verfahren aus den entsprechenden Ausgangsverbindungen synthetisieren.

Die Umsetzung nach Verfahren [B] erfolgt im Allgemeinen in inerten Lösungsmitteln, bevorzugt in einem Temperaturbereich von 50°C bis zum Rückfluss der Lösungsmittel bei Normaldruck.

Inerte Lösungsmittel sind beispielsweise Dimethylformamid, Dimethylacetamid, 2-Butanon oder Acetonitril. Bevorzugt ist Dimethylformamid.

Die Verbindungen der Formel (XI) sind bekannt oder lassen sich nach bekannten Verfahren aus den entsprechenden Ausgangsverbindungen synthetisieren.

Die Umsetzung nach Verfahren [C] erfolgt im Allgemeinen in Phosphorylchlorid als Lösungsmittel oder mit Thionylchlorid in einem inerten Lösungsmittel, bevorzugt in einem Temperaturbereich von Raumtemperatur bis zum Rückfluss des Lösungsmittel bei Normaldruck.

Inerte Lösungsmittel sind beispielsweise Halogenkohlenwasserstoffe wie Methylenchlorid, Trichlormethan oder 1,2-Dichlorethan, bevorzugt ist Methylenchlorid.

Die Verbindungen der Formel (XVI) sind bekannt oder lassen sich nach bekannten Verfahren aus den entsprechenden Ausgangsverbindungen synthetisieren.

Die Umsetzung der ersten Stufe nach Verfahren [D] erfolgt im Allgemeinen in inerten Lösungsmitteln, wobei die Umsetzung mit Phosphorylchlorid auch in Phosphorylchlorid als Lösungsmittel erfolgen kann, bevorzugt in einem Temperaturbereich von Raumtemperatur bis zum Rückfluss der Lösungsmittel bei Normaldruck.

Inerte Lösungsmittel sind beispielsweise Halogenkohlenwasserstoffe wie Methylenchlorid, Trichlormethan oder 1,2-Dichlorethan, oder Dioxan, bevorzugt ist Methylenchlorid.

Die Umsetzung der zweiten Stufe nach Verfahren [D] erfolgt wie für die Umsetzung der Verbindungen der Formel (XII) zu Verbindungen der Formel (X) beschrieben.

Die Verbindungen der Formel (XVII) sind bekannt oder lassen sich nach bekannten Verfahren aus den entsprechenden Ausgangsverbindungen synthetisieren.

Die Umsetzung nach Verfahren [E] erfolgt wie für die erste Stufe des Verfahrens [D] beschrieben.

Die Umsetzung der ersten Stufe nach Verfahren [F] erfolgt im Allgemeinen in inerten Lösungsmitteln, wobei die Umsetzung mit Phosphorylchlorid auch in Phosphorylchlorid als Lösungsmittel erfolgen kann, bevorzugt in einem Temperaturbereich von Raumtemperatur bis zum Rückfluss der Lösungsmittel bei Normaldruck.

Inerte Lösungsmittel sind beispielsweise Halogenkohlenwasserstoffe wie Methylenchlorid, Trichlormethan oder 1,2-Dichlorethan, oder Dioxan, bevorzugt ist Dioxan.

Die Umsetzung der zweiten Stufe nach Verfahren [F] erfolgt wie für die Umsetzung der Verbindungen der Formel (XII) zu Verbindungen der Formel (X) beschrieben.

Die Umsetzung nach Verfahren [G] erfolgt im Allgemeinen in inerten Lösungsmitteln, bevorzugt in einem Temperaturbereich von -10°C bis Raumtemperatur bei Normaldruck.

Inerte Lösungsmittel sind beispielsweise Ether wie Tetrahydrofuran, bevorzugt ist Tetrahydrofuran.

Als Butyllithium kann n-Butyllithium, sec-Butyllithium oder tert-Butyllithium verwendet werden, bevorzugt ist n-Butyllithium.

Die Verbindungen der Formel (XVIII) sind bekannt oder lassen sich nach bekannten Verfahren aus den entsprechenden Ausgangsverbindungen synthetisieren.

Die Umsetzung nach Verfahren [H] erfolgt wie für Verfahrens [A] beschrieben.

Die Verbindungen der Formel (XX) sind bekannt oder lassen sich nach bekannten Verfahren aus den entsprechenden Ausgangsverbindungen synthetisieren.

Die Umsetzung der ersten Stufe nach Verfahren [J] erfolgt im Allgemeinen in inerten Lösungsmitteln, in Gegenwart einer Base, bevorzugt in einem Temperaturbereich von 0°C bis 50°C bei Normaldruck.

Inerte Lösungsmittel sind beispielsweise Halogenkohlenwasserstoffe wie Methylenchlorid, Trichlormethan, Tetrachlormethan oder 1,2-Dichlorethan, bevorzugt ist Methylenchlorid.

Basen sind beispielsweise organische Basen wie Trialkylamine z.B. Triethylamin, *N*-Methylmorpholin, *N*-Methylpiperidin, 4-Dimethylaminopyridin oder Diisopropylethylamin, bevorzugt ist Triethylamin.

Die Umsetzung der zweiten Stufe nach Verfahren [J] erfolgt im Allgemeinen in inerten Lösungsmitteln, in Gegenwart einer Base, gegebenenfalls in einer Mikrowelle, bevorzugt in einem Temperaturbereich von 50°C bis 200°C bei Normaldruck bis 5 bar.

Inerte Lösungsmittel sind beispielsweise Dimethylsulfoxid, Dimethylformamid oder N-Methylpyrrolidon, bevorzugt ist Dimethylformamid.

Basen sind beispielsweise Alkalicarbonate, wie z.B. Natrium- oder Kaliumcarbonat, bevorzugt ist Kaliumcarbonat.

Die Verbindungen der Formel (XXI) sind bekannt oder lassen sich nach den allgemeinen Verfahren [A] bis [H] herstellen, wobei die freie Aminogruppe während der Umsetzung durch dem Fachmann bekannte Schutzgruppen geschützt ist. Bevorzugt ist eine tert-Butoxycarbonyl-Schutzgruppe.

Die Verbindungen der Formel (XXII) sind bekannt oder lassen sich nach bekannten Verfahren aus den entsprechenden Ausgangsverbindungen synthetisieren.

Die Verbindungen der Formel (II) sind bekannt oder und können hergestellt werden, indem Verbindungen der Formel 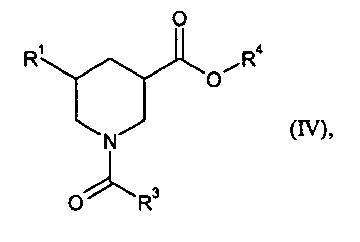 in welcher
R¹ und R³ die oben angegebene Bedeutung aufweisen, und
R⁴ für Methyl oder Ethyl steht,
mit einer Base umgesetzt werden.

Die Umsetzung erfolgt im Allgemeinen in inerten Lösungsmitteln, in Gegenwart einer Base, bevorzugt in einem Temperaturbereich von Raumtemperatur bis zum Rückfluss der Lösungsmittel bei Normaldruck.

Inerte Lösungsmittel sind beispielsweise Halogenkohlenwasserstoffe wie Methylenchlorid, Trichlormethan, Tetrachlormethan oder 1,2-Dichlorethan, Ether wie Diethylether, Methyl-tert-butylether, 1,2-Dimethoxyethan, Dioxan oder Tetrahydrofuran, oder andere Lösungsmittel wie Dimethylformamid, Dimethylacetamid, Acetonitril oder Pyridin, oder Gemische von Lösungsmitteln, oder Gemische von Lösungsmittel mit Wasser, bevorzugt ist ein Gemisch aus Tetrahydrofuran und Wasser.

Basen sind beispielsweise Alkalihydroxide wie Natrium-, Lithium- oder Kaliumhydroxid, oder Alkalicarbonate wie Cäsiumcarbonat, Natrium- oder Kaliumcarbonat, bevorzugt ist Lithiumhydroxid.

Die Verbindungen der Formel (IV) sind bekannt oder und können hergestellt werden, indem Verbindungen der Formel 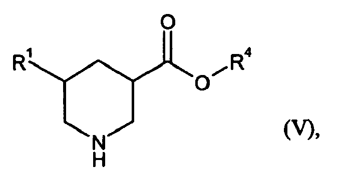 in welcher
R¹ und R⁴ die oben angegebene Bedeutung aufweisen,
mit Verbindungen der Formel 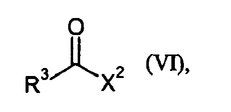 in welcher
R³ die oben angegebene Bedeutung aufweist, und
X² für Halogen, bevorzugt Brom oder Chlor, oder Hydroxy steht,
umgesetzt werden.

Wenn X² für Halogen steht, erfolgt die Umsetzung im Allgemeinen in inerten Lösungsmitteln, gegebenenfalls in Gegenwart einer Base, bevorzugt in einem Temperaturbereich von -30°C bis 50°C bei Normaldruck.

Inerte Lösungsmittel sind beispielsweise Tetrahydrofuran, Methylenchlorid, Pyridin, Dioxan oder Dimethylformamid, bevorzugt ist Methylenchlorid.

Basen sind beispielsweise Triethylamin, Diisopropylethylamin oder *N*-Methylmorpholin, bevorzugt ist Triethylamin oder Diisopropylethylamin.

Wenn X² für Hydroxy steht, erfolgt die Umsetzung im Allgemeinen in inerten Lösungsmitteln, in Gegenwart eines Dehydratisierungsreagenzes, gegebenenfalls in Gegenwart einer Base, bevorzugt in einem Temperaturbereich von -30°C bis 50°C bei Normaldruck.

Inerte Lösungsmittel sind beispielsweise Halogenkohlenwasserstoffe wie Dichlormethan oder Trichlormethan, Kohlenwasserstoff wie Benzol, Nitromethan, Dioxan, Dimethylformamid oder Acetonitril. Ebenso ist es möglich, Gemische der Lösemittel einzusetzen. Besonders bevorzugt ist Dichlormethan oder Dimethylformamid.

Als Dehydratisierungsreagenzien eignen sich hierbei beispielsweise Carbodiimide wie z.B. *N,N'-*Diethyl-, *N*,*N*,'-Dipropyl-, *N*,*N'*-Diisopropyl-, *N*,*N'*-Dicyclohexylcarbodiimid, *N*-(3-Dimethylaminoisopropyl)-*N'*-ethylcarbodiimid-hydrochlorid (EDC), *N*-Cyclohexylcarbodiimid-*N'-*propyloxymethyl-Polystyrol (PS-Carbodiimid) oder Carbonylverbindungen wie Carbonyldiimidazol, oder 1,2-Oxazoliumverbindungen wie 2-Ethyl-5-phenyl-1,2-oxazolium-3-sulfat oder 2-*tert-*Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Bis-(2-oxo-3-oxazolidinyl)-phosphorylchlorid oder Benzotriazolyloxy-tri(dimethylamino)-phosphoniumhexafluorophosphat, oder *O*-(Benzotriazol-1-yl)-*N*,*N,N',N'*-tetra-methyluroniumhexafluorophosphat (HBTU), 2-(2-Oxo-1-(2H)-pyridyl)-1,1,3,3-tetramethyluroniumtetrafluoroborat (TPTU) oder *O*-(7-Azabenzotriazol-1-yl)-*N,N,N',N'*-tetramethyl-uroniumhexafluorophosphat (HATU), oder 1-Hydroxybenzotriazol (HOBt), oder Benzotriazol-1-yloxytris(dimethylamino)-phosphoniumhexafluorophosphat (BOP), oder *N*-Hydroxysuccinimid, oder Mischungen aus diesen, mit Basen.

Basen sind beispielsweise Alkalicarbonate, wie z.B. Natrium- oder Kaliumcarbonat, oder -hydrogencarbonat, oder organische Basen wie Trialkylamine z.B. Triethylamin, *N*-Methylmorpholin, *N*-Methylpiperidin, 4-Dimethylaminopyridin oder Diisopropylethylamin.

Vorzugsweise wird die Kondensation mit HATU oder mit EDC in Gegenwart von HOBt durchgeführt.

Die Verbindungen der Formel (VI) sind bekannt oder lassen sich nach bekannten Verfahren aus den entsprechenden Ausgangsverbindungen synthetisieren.

In einem alternativen Verfahren können die Verbindungen der Formel (IV) hergestellt werden, indem Verbindungen der Formel (V) in der ersten Stufe mit 4-Nitrophenylchloroformat und in der zweiten Stufe mit Verbindungen der Formel (XXII) umgesetzt werden.

Die Umsetzung der ersten und zweiten Strufe erfolgt wie unter Verfahren [J] beschrieben.

Die Verbindungen der Formel (V) sind bekannt oder und können hergestellt werden, indem Verbindungen der Formel 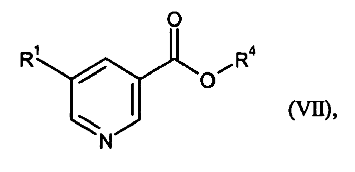 in welcher
R¹ und R⁴ die oben angegebene Bedeutung aufweisen,
hydriert werden.

Die Hydrierung erfolgt im Allgemeinen mit einem Reduktionsmittel in inerten Lösungsmitteln, gegebenenfalls unter Zusatz von Säure wie Mineralsäuren und Carbonsäuren, bevorzugt Essigsäure, bevorzugt in einem Temperaturbereich von Raumtemperatur bis zum Rückfluss der Lösungsmittel und in einem Druckbereich von Normaldruck bis 100 bar, bevorzugt bei 50-80 bar.

Als Reduktionsmittel ist bevorzugt Wasserstoff mit Palladium auf Aktivkohle, mit Rhodium auf Aktivkohle, mit Ruthenium auf Aktivkohle oder daraus gemischte Katalysatom, oder Wasserstoff mit Palladium auf Aluminiumoxid oder mit Rhodium auf Aluminiumoxid, bevorzugt ist Wasserstoff mit Palladium auf Aktivkohle oder mit Rhodium auf Aktivkohle.

Inerte Lösungsmittel sind beispielsweise Alkohole wie Methanol, Ethanol, n-Propanol, isoPropanol, n-Butanol oder tert-Butanol, bevorzugt ist Methanol oder Ethanol.

Die Verbindungen der Formel (VII) sind bekannt oder und können hergestellt werden, indem Verbindungen der Formel 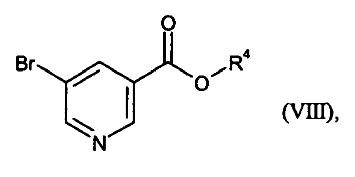 in welcher
R⁴ die oben angegebene Bedeutung aufweist,
mit Verbindungen der Formel 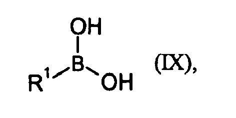 in welcher
R¹ die oben angegebene Bedeutung aufweist,
umgesetzt werden.

Die Umsetzung erfolgt im Allgemeinen in inerten Lösungsmitteln, in Gegenwart eines Katalysators, gegebenenfalls in Gegenwart eines Zusatzreagenzes, bevorzugt in einem Temperaturbereich von Raumtemperatur bis zum Rückfluss des Lösungsmittels bei Normaldruck.

Inerte Lösungsmittel sind beispielsweise Ether wie Dioxan, Tetrahydrofuran oder 1,2-Dimethoxyethan, Kohlenwasserstoffe wie Benzol, Xylol oder Toluol, oder andere Lösungsmittel wie Nitrobenzol, Dimethylformamid, Dimethylacetamid, Dimethylsulfoxid oder *N-*Methylpyrrolidon, gegebenenfalls wird diesen Lösungsmitteln etwas Wasser zugesetzt. Bevorzugt ist Toluol mit Wasser oder eine Mischung aus 1,2-Dimethoxyethan, Dimethylformamid und Wasser.

Katalysatoren sind beispielsweise für Suzuki-Reaktionsbedingungen übliche Palladium-Katalysatoren, bevorzugt sind Katalysatoren wie z.B. Dichlorbis(triphenylphosphin)-palladium, Tetrakistriphenylphosphinpalladium(0), Palladium(II)acetat oder Bis-(diphenylphosphanferrocenyl)-palladium-(II)-chlorid.

Zusatzreagenzien sind beispielsweise Kaliumacetat, Cäsium-, Kalium- oder Natriumcarbonat, Bariumhydroxid, Kalium-tert-butylat, Cäsiumfluorid, Kaliumfluorid oder Kaliumphosphat, bevorzugt sind Kaliumfluorid oder Natriumcarbonat.

Die Verbindungen der Formeln (VIII) und (IX) sind bekannt oder lassen sich nach bekannten Verfahren aus den entsprechenden Ausgangsverbindungen synthetisieren.

Die Verbindungen der Formel (X) sind bekannt oder und können hergestellt werden, indem Verbindungen der Formel 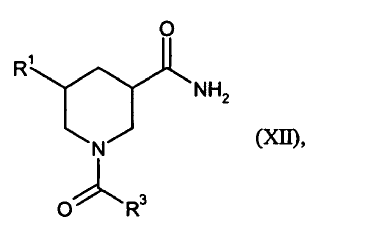 in welcher
R¹ und R³ die oben angegebene Bedeutung aufweisen,
mit Lawesson-Reagens (2,4-Bis[4-methoxyphenyl] 1,3-dithia-2,4-diphosphetan-2,4-disulfid) umgesetzt werden.

Die Umsetzung erfolgt im Allgemeinen in inerten Lösungsmitteln, bevorzugt in einem Temperaturbereich von Raumtemperatur bis zum Rückfluss der Lösungsmittel bei Normaldruck.

Inerte Lösungsmittel sind beispielsweise Ether wie Dioxan, Tetrahydrofuran oder 1,2-Dimethoxyethan, Kohlenwasserstoffe wie Benzol, Xylol oder Toluol, oder Halogenkohlenwasserstoffe wie Methylenchlorid, Trichlormethan, Tetrachlormethan oder 1,2-Dichlorethan. Bevorzugt ist Dioxan.

Die Verbindungen der Formel (XII) sind bekannt oder und können hergestellt werden, indem Verbindungen der Formel 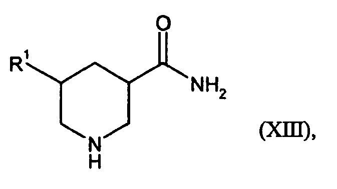 in welcher
R¹ die oben angegebene Bedeutung aufweist,
mit Verbindungen der Formel (VI) umgesetzt werden.

Die Umsetzung erfolgt nach den unter der Umsetzung von Verbindungen der Formel (V) mit Verbindungen der Formel (VI) angegebenen Reaktionsbedingungen.

Die Verbindungen der Formel (XIII) sind bekannt oder und können hergestellt werden, indem Verbindungen der Formel 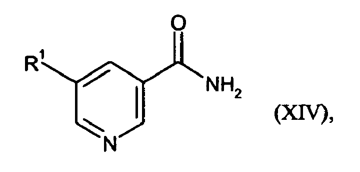 in welcher
R¹ die oben angegebene Bedeutung aufweist,
hydriert werden.

Die Hydrierung erfolgt nach den unter der Hydrierung von Verbindungen der Formel (VII) angegebenen Reaktionsbedingungen.

Die Verbindungen der Formel (XIV) sind bekannt oder und können hergestellt werden, indem die Verbindung der Formel 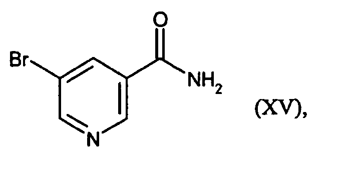 mit Verbindungen der Formel (IX) umgesetzt werden.

Die Umsetzung erfolgt nach den unter der Umsetzung von Verbindungen der Formel (VIII) mit Verbindungen der Formel (IX) angegebenen Reaktionsbedingungen.

Die Verbindung der Formel (XV) ist bekannt oder läßt sich nach bekannten Verfahren aus den entsprechenden Ausgangsverbindungen synthetisieren.

Die Verbindungen der Formel (XIX) sind bekannt oder und können aus Verbindungen der Formel (XII) hergestellt werden, wie in den Beispiel 83A und Beispiel 84A beschrieben.

Die Herstellung der Verbindungen der Formel (I) kann durch folgende Syntheseschemata verdeutlicht werden.

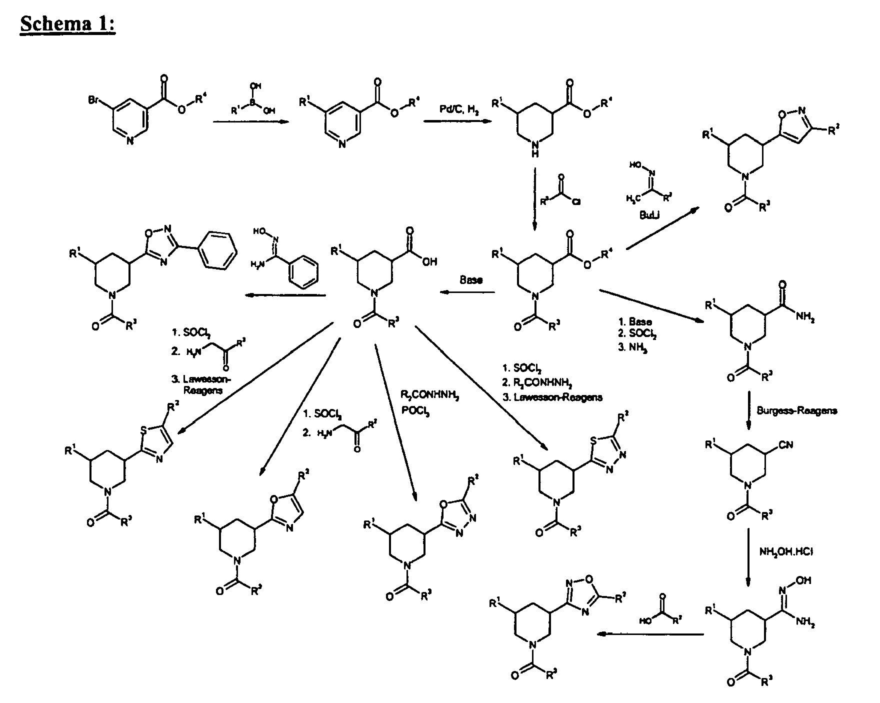

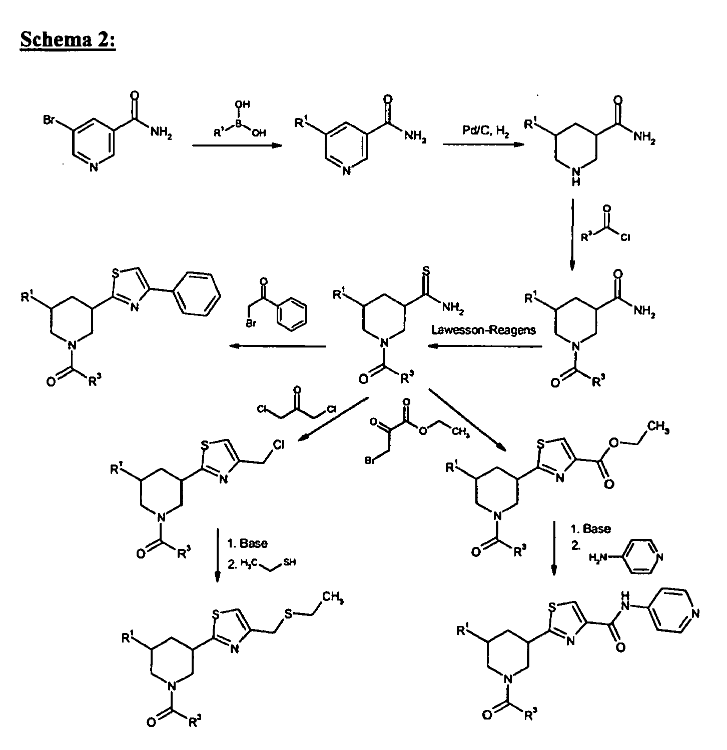

Die erfindungsgemäßen Verbindungen zeigen ein nicht vorhersehbares, wertvolles pharmakologisches und pharmakokinetisches Wirkspektrum. Es handelt sich dabei um selektive Antagonisten des PAR-1-Rezeptors, die insbesondere als Thrombozytenaggregationshemmer, als Hemmer der Endothelproliferation und als Hemmer des Tumorwachstums wirken.

Sie eigenen sich daher zur Verwendung als Arzneimittel zur Behandlung und/oder Prophylaxe von Krankheiten bei Menschen und Tieren.

Weiterer Gegenstand der vorliegenden Erfindung ist der Einsatz der erfindungsgemäßen Verbindungen zur Behandlung und/oder Prophylaxe von Erkrankungen, vorzugsweise von thromboembolischen Erkrankungen und/oder thromboembolischen Komplikationen.

Zu den "thromboembolischen Erkrankungen" im Sinne der vorliegenden Erfindung zählen insbesondere Erkrankungen wie Herzinfarkt mit ST-Segment-Erhöhung (STEMI) und ohne ST-Segment-Erhöhung (non-STEMI), stabile Angina Pectoris, instabile Angina Pectoris, Reokklusionen und Restenosen nach Koronarinterventionen wie Angioplastie, Stentimplantation oder aortokoronarem Bypass, periphere arterielle Verschlusskrankheiten, Lungenembolien, tiefe venöse Thrombosen und Nierenvenenthrombosen, transitorische ischämische Attacken sowie thrombotischer und thromboembolischer Hirnschlag.

Die Substanzen eignen sich daher auch zur Prävention und Behandlung von kardiogenen Thromboembolien, wie beispielsweise Hirn-Ischämien, Schlaganfall (Stroke) und systemischen Thromboembolien und Ischämien, bei Patienten mit akuten, intermittierenden oder persistierenden Herzarrhythmien, wie beispielsweise Vorhofflimmern, und solchen, die sich einer Kardioversion unterziehen, ferner bei Patienten mit Herzklappen-Erkrankungen oder mit intravasalen Körpern, wie z. B. künstlichen Herzklappen, Kathetern, intraaortale Ballongegenpulsation und Schrittmachersonden.

Thromboembolische Komplikationen treten ferner auf bei mikroangiopathischen hämolytischen Anämien, extrakorporalen Blutkreisläufen, wie z. B. Hämodialyse, Hämofiltration, ventricular assist devices und Kunstherz, sowie Herzklappenprothesen.

Außerdem kommen die erfindungsgemäßen Verbindungen auch zur Beeinflussung der Wundheilung, für die Prophylaxe und/oder Behandlung von atherosklerotischen Gefäßerkrankungen und entzündlichen Erkrankungen wie rheumatische Erkrankungen des Bewegungsapparats, koronaren Herzkrankheiten, von Herzinsuffizienz, von Bluthochdruck, von entzündlichen Erkrankungen, wie z.B. Asthma, COPD, entzündlichen Lungenerkrankungen, Glomerulonephritis und entzündlichen Darmerkrankungen in Betracht, darüber hinaus ebenso für die Prophylaxe und/oder Behandlung der Alzheimer'schen Erkrankung, Autoimmunerkrankungen, Morbus Crohn und Kolitis Ulzerosa.

Außerdem können die erfindungsgemäßen Verbindungen zur Inhibition des Tumorwachstums und der Metastasenbildung, bei Mikroangiopathien, altersbedingter Makuladegeneration, diabetischer Retinopathie, diabetischer Nephropathie und anderen mikrovaskulären Erkrankungen sowie zur Prävention und Behandlung thromboembolischer Komplikationen, wie beispielsweise venöser Thromboembolien, bei Tumorpatienten, insbesondere solchen, die sich größeren chirurgischen Eingriffen oder einer Chemo- oder Radiotherapie unterziehen, eingesetzt werden.

Weiterhin eignen sich die erfindungsgemäßen Verbindungen zur Behandlung von Krebs. Krebserkrankungen schließen unter anderem ein: Karzinome (hierunter Brustkrebs, hepatozelluläre karzinome, Lunkenkrebs, kolorektaler Krebs, Kolonkrebs und Melanome), Lympohome (z.B. Non-Hodgkin-Lymphome und Mykosis fungoides), Leukämien, Sarkome, Mesotheliome, Hirnkrebs (z.B. Gliome), Germinome (z.B. Hodenkrebs und Ovarialkrebs), Choriokarzinome, Nierenkrebs, Bauchspeicheldrüsenkrebs, Schilddrüsenkrebs, Kopf- und Halskrebs, Endometrieum-Krebs, Zervix-Krebs, Blasenkrebs, Magenkrebs und multiples Myelom.

Außerdem vermittelt auf Endothelzellen exprimiertes PAR-1 Signale, die in Gefäßwachstum münden ("Angiogenese"), ein Vorgang, der zur Ermöglichung von Tumorwachstum über ca. 1 mm³ hinaus unerläßlich ist. Induktion der Angiogenese ist auch bei anderen Erkrankungen relevant, hierunter Erkrankungen des rheumatischen Formenkreises (z.B. rheumatoide Arthritis), bei Lungenerkrankungen (z.B. Lungenfibrose, pulmonale Hypertonie, insbesondere pulmonalarterielle Hypertonie, Erkrankungen, die durch Lungengefäßverschlüsse charakterisiert sind), Arteriosklerose, Plaqueruptur, diabetische Retinopathie und feuchte Makuladegeneration.

Darüber hinaus sind die erfindungsgemäßen Verbindungen zur Behandlung von Sepsis geeignet. Sepsis (oder Septikämie) ist eine häufige Erkrankung mit hoher Letalität. Anfängliche Symptome der Sepsis sind typischerweise unspezifisch (z.B. Fieber, reduziertes Allgemeinbefinden), im weiteren Verlauf kann es jedoch zur generalisierten Aktivierung des Gerinnungssystems kommen ("Disseminated Intravascular coagulation", oder "Verbrauchskoagulopathie", nachfolgend als "DIC" bezeichnet) mit Mikrothrombosierung in verschiedenen Organen und sekundärer Blutungskomplikationen. Außerdem kann es zur endothelialen Schädigung mit Erhöhung der Gefäßpermeabilität und Austritt von Flüssigkeit und Proteinen in den Extravasalraum kommen. Im weiteren Verlauf kann es zur Dysfunktion oder dem Versagen eines Organs (z.B. Nierenversagen, Leberversagen, Atemversagen, zentralnervöse Defizite und Herz-/Kreislaufversagen) bis hin zum Multiorganversagen kommen. Hiervon kann prinzipiell jedes Organ betroffen sein, am häufigsten tritt Organdysfunktion und -Versagen bei der Lunge, der Niere, dem Herz-Kreislaufsystem, dem Gerinnungssystem, dem zentralnervösen System, endokrinen Drüsen und der Leber auf. Eine Sepsis kann mit einem "Acute Respiratory Distress Syndrome" (nachfolgend als ARDS bezeichnet) einhergehen. Ein ARDS kann auch unabhängig von einer Sepsis auftreten. "Septischer Schock" bezeichnet das Auftreten einer behandlungspflichtigen Blutdruckerniedrigung, die eine weitere Organschädigung begünstigt und mit einer Verschlechterung der Prognose einhergeht.

Krankheitserreger können Bakterien (gram-negativ und gram-positiv), Pilze, Viren und/oder Eukaryonten sein. Eintrittspforte bzw. Primärinfektion können z.B. Pneumonie, Hamwegsinfekt, Peritonitis sein. Die Infektion kann, muß aber nicht zwingend, mit einer Bakteriämie einhergehen.

Sepsis wird definiert als das Vorliegen einer Infektion und eines "systemic inflammatory response syndrome" (nachfolgend mit "SIRS" bezeichnet). SIRS tritt im Rahmen von Infekten, aber auch von anderen Zuständen wie Verletzungen, Verbrennungen, Schock, Operationen, Ischämie, Pankreatitis, Reanimation oder Tumoren auf. Nach der Definition des ACCP/SCCM Consensus Conference Committee von 1992 (*Crit. Care Med.* 1992, *20*, 864-874) werden die zur Diagnose "SIRS" erforderlichen Symptome zur Diagnose und Meßparameter beschrieben (u.a. veränderte Körpertemperatur, erhöhte Herzfrequenz, Atemschwierigkeiten und verändertes Blutbild). In der späteren (2001) SCCM/ESICM/ACCP/ATS/SIS International Sepsis Definitions Conference wurden die Kriterien im Wesentlichen beibehalten, in Details jedoch verfeinert (Levy et al., *Crit. Care Med.* 2003, *31*, 1250-1256).

DIC und SIRS können im Rahmen einer Sepsis, aber auch infolge von Operationen, Tumorerkrankungen, Verbrennungen oder anderen Verletzungen auftreten. Bei der DIC kommt es an der Oberfläche von geschädigten Endothelzellen, Fremdkörperoberflächen oder verletztem extravaskulärem Gewebe zur massiven Aktivierung des Gerinnungssystems. Als Folge kommt es zur Gerinnung in kleinen Gefäßen verschiedener Organe mit Hypoxie und anschließender Organdysfunktion. Sekundär kommt es zum Verbrauch von Gerinnungsfaktoren (z.B. Faktor X, Prothrombin, Fibrinogen) und Plättchen, wodurch die Gerinnungsfähigkeit des Blutes herabgesetzt wird und schwere Blutungen auftreten können.

Die erfindungsgemäßen Verbindungen können darüber hinaus auch zur Verhinderung von Koagulation *ex vivo* eingesetzt werden, z.B. zur Konservierung von Blut- und Plasmaprodukten, zur Reinigung/Vorbehandlung von Kathetern und anderen medizinischen Hilfsmitteln und Geräten, einschließlich extrakorporaler Kreisläufe, zur Beschichtung künstlicher Oberflächen von *in vivo* oder *ex vivo* eingesetzten medizinischen Hilfsmitteln und Geräten oder bei biologischen Proben, die Blutplättchen enthalten.

Weiterer Gegenstand der vorliegenden Erfindung ist die Verwendung der erfindungsgemäßen Verbindungen zur Beschichtung von medizinischen Instrumenten und Implantaten, z.B. Kathetern, Prothesen, Stents oder künstlichen Herzklappen. Die erfindungsgemäßen Verbindungen können dabei fest an die Oberfläche gebunden sein oder über einen bestimmten Zeitraum aus einer Trägerbeschichtung in die unmittelbare Umgebung zur lokalen Wirkung freigesetzt werden.

Weiterer Gegenstand der vorliegenden Erfindung ist die Verwendung der erfindungsgemäßen Verbindungen zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Weiterer Gegenstand der vorliegenden Erfindung ist ein Verfahren zur Behandlung und/oder Prophylaxe von Erkrankungen, insbesondere der zuvor genannten Erkrankungen, unter Verwendung einer therapeutisch wirksamen Menge einer erfindungsgemäßen Verbindung.

Weiterer Gegenstand der vorliegenden Erfindung sind Arzneimittel, enthaltend eine erfindungsgemäße Verbindung und einen oder mehrere weitere Wirkstoffe, insbesondere zur Behandlung und/oder Prophylaxe der zuvor genannten Erkrankungen. Als geeignete Kombinationswirkstoffe seien beispielhaft und vorzugsweise genannt:
Kalziumkanalblocker, z.B. Amlodipin Besilat (z.B. Norvasc^{®}), Felodipin, Diltiazem, Verapamil, Nifedipin, Nicardipin, Nisoldipin und Bepridil;
Iomerizin;
Statine, z.B. Atorvastatin, Fluvastatin, Lovastatin, Pitavastatin, Pravastatin, Rosuvastatin, und Simvastatin;
Cholesterinabsorption-Inhibitoren, z.B. Ezetimibe und AZD4121;
Cholesteryl Ester Transfer Protein ("CETP") Inhibitoren, z.B. Torcetrapib;
Niedrigmolekualre Heparine, z.B. Dalteparin Natrium, Ardeparin, Certoparin, Enoxaparin, Parnaparin, Tinzaparin, Reviparin und Nadroparin;
Weitere Antikoagulanzien, z.B. Warfarin, Marcumar, Fondaparinux;
Antiarrhythmika, z.B. Dofetilid, Ibutilid, Metoprolol, Metoprololtartrat, Propranolol, Atenolol, Ajmalin, Disopyramid, Prajmalin, Procainamid, Quinidin, Spartein, Aprindine, Lidocain, Mexiletin, Tocamid, Encamid, Flecamid, Lorcamid, Moricizin, Propafenon, Acebutolol, Pindolol, Amiodaron, Bretylium-Tosylat, Bunaftin, Sotalol, Adenosin, Atropin und Digoxin;
Alpha-adrenerge Agonisten, z.B. Doxazosin-Mesylat, Terazoson und Prazosin;
Beta-adrenerge Blocker, z.B. Carvedilol, Propranolol, Timolol, Nadolol, Atenolol, Metoprolol, Bisoprolol, Nebivolol, Betaxolol, Acebutolol und Bisoprolol;
Aldosteron-Antagonisten, z.B. Eplerenon und Spironolacton;
Angiotensin-converting-enzyme Inhbitoren ("ACE-Inhibitoren"), z.B. Moexipril, Quinapril-Hydrochlorid, Ramipril, Lisinopril, Benazepril-Hydrochlorid, Enalapril, Captopril, Spirapril, Perindopril, Fosinopril und Trandolapril,;
Angiotensin II Receptorblockers ("ARBs"), z.B. Olmesartan-Medoxomil, Candesartan, Valsartan, Telmisartan, Irbesartan, Losartan und Eprosartan,;
Endothelinantagonisten, z.B. Tezosentan, Bosentan und Sitaxsentan-Natrium;
Neutral Endopeptidaseinhibitoren, z.B. Candoxatril und Ecadotril;
Phosphodiesteraseinhibitoren, z.B. Milrinoon, Theophyllin, Vinpocetin, EHNA (erythro-9-(2-hydroxy-3-nonyl)adenine), Sildenafil, Vardenafil und Tadalafil;
Fibrinolytika, z.B. Reteplase, Alteplase und Tenecteplase;
GP IIb/IIIa-Antagonisten, z.B. Integrillin, Abciximab und Tirofiban;
Direkte Thrombininhibitoren, z.B. AZD0837, Argatroban, Bivalirudin und Dabigatran;
Indirekte Thrombininhibitoren, z.B. Odiparcil;
Direkte und indirekte Faktor Xa Inhibitoren, z.B. Fondaparinux-Natrium, Apixaban, Razaxaban, Rivaroxaban (BAY 59-7939), KFA-1982, DX-9065a, AVE3247, Otamixaban (XRP0673), AVE6324, SAR377142, Idraparinux, SSR126517, DB-772d, DT-831j, YM-150, 813893, LY517717 und DU-1766.;
Direkte und indirekte Faktor Xa/IIa Inhibitoren, z.B. Enoxaparin-Natrium, AVE5026, SSR128428, SSR128429 und BIBT-986 (Tanogitran);
Lipoprotein-assoziierte Phospholipase A2 ("LpPLA2") Modulatoren;
Diuretika, z.B. Chlorthalidon, Ethacrynsäure, Furosemid, Amilorid. Chlorothiazid, Hydrochlorothiazid, Methylchtothiazid und Benzthiazid;
Nitrate, z.B. Isosorbide-5-Mononitrat;
Thromboxan-Antagonisten, z.B. Seratrodast, Picotamid und Ramatroban;
Plättchenaggregations-Inhibitoren, z.B. Clopidogrel, Tiklopidin, Cilostazol, Aspirin, Abciximab, Limaprost, Eptifibatide und CT-50547;
Cyklooxygenase-Inhibitoren, z.B. Meloxicam, Rofecoxib und Celecoxib;
B-Typ Natriuretic Peptide, z.B. Nesiritid und Ularitide;
NV1FGF-Modulatoren, z.B. XRP0038;
HT1B/5-HT2A-Antagonisten, z.B. SL65.0472;
Guanylatcyclase-Aktivatoren, z.B. Ataciguat (HMR1766) und HMR1069;
e-NOS Transkriptions-Enhancers, z.B. AVE9488 and AVE3085;
Anti-atherogene Substanzen, z.B. AGI-1067:
   CPU-Inhibitoren, z.B. AZD9684;
   Renininhibitoren, z.B. Aliskirin und VNP489;
   Inhibitoren der Adenosindiphosphat-induzierten Plättchenaggregation, z.B. Clopidogrel, Tiklopidin, Prasugrel und AZD6140;
   NHE-1 Inhibitoren, z.B. AVE4454 und AVE4890.

Antibiotische Therapie: Verschiedene Antibiotika oder antifungale Medikamenten-Kombinationen kommen in Frage, entweder als kalkulierte Therapie (vor Vorliegen des mikrobiellen Befundes) oder als spezifische Therapie; Flüssigkeitstherapie, z.B. Kristalloide oder kolloidale Flüssigkeiten; Vasopressoren, z.B. Norepinephrine, Dopamine oder Vasopressin; Inotrope Therapie, z.B. Dobutamin; Kortikosteroide, z.B. Hydrokortison, oder Fludrokortison; rekombinantes humanes aktivierte Protein C, Xigris; Blutprodukte, z.B. Erythrozytenkonzentrate, Thrombozytenkonzentrate, Erythropietin oder Fresh Frozen Plasma; Maschinelle Beatmung bei sepsisinduziertem Acute Lung Injury (ALI) bzw. Acute Respiratory Distress Syndrome (ARDS), z.B. Permissive Hyperkapnie, niedrigere Tidalvolumina; Sedierung: z.B. Diazepam, Lorazepam, Midazolam oder Propofol. Opioide: z.B. Fentanyl, Hydromorphon, Morphin, Meperidin oder Remifentanil. NSAIDs: z.B. Ketorolac, Ibuprofen oder Acetaminophen. Neuromuskuläre Blockade: z.B. Pancuronium; Glukose-Kontrolle, z.B. Insulin, Glukose; Nierenersatzverfahren, z.B. kontinuierliche veno-venöse-Hämofiltration oder intermittierende Hämodialyse. Dopamin niedrig-dosiert zur renalen Protektion; Antikoagulantien, z.B. zur Thromboseprophylaxe oder bei Nierenersatzverfahren, z.B. unfraktionierte Heparine, low molecular weight Heparine, Heparinoide, Hirudin, Bivalirudin oder Argatroban; Bikarbonat-Therapie; Streßulkusprophylaxe, z.B. H2-Rezeptorinhibitoren, Antazida

Medikamente bei proliferativen Erkrankungen: Urazil, Chlormethin, Cyklophosphamid, Ifosfamid, Melphalan, Chlorambucil, Pipobroman, Triethylenemelamin, Triethylenethiophosphoramin, Busulfan, Carmustine, Lomustine, Streptozocin, Dacarbazine, Methotrexate, 5- Fluorouracil, Floxuridine, Cytarabine, 6-Mercaptopurine, 6-Thioguanine, Fludarabine phosphate, Pentostatine, Vinblastine, Vincristine, Vindesine, Bleomycin, Dactinomycin, Daunorubicin, Doxorubicin, Epirubicin, Idarubicin, Paclitaxel, Mithramycin, Deoxycoformycin, Mitomycin-C, L-Asparaginase, Interferons, Etoposide, Teniposide 17.alpha.- Ethinylestradiol, Diethylstilbestrol, Testosterone, Prednisone, Fluoxymesterone, Dromostanolone propionate, Testolactone, Megestrolacetate, Tamoxifen, Methylprednisolone, Methyltestosterone, Prednisolone, Triamcinolone, Chlorotrianisene, Hydroxyprogesterone, Aminoglutethimide, Estranrustine, Medroxyprogesteroneacetate, Leuprolide, Flutamide, Toremifene, Goserelin, Cisplatin, Carboplatin, Hydroxyurea, Amsacrine, Procarbazine, Mitotane, Mitoxantrone, Levamisole, Navelbene, Anastrazole, Letrazole, Capecitabine, Reloxafme, Droloxafine, Hexamethylmelamine, Oxaliplatin (Eloxatin^{®}, Iressa (gefmitib, Zdl839), XELODA^{®} (capecitabine), Tarceva^{®} (erlotinib), Azacitidine (5-Azacytidine; 5-AzaC), Temozolomide (Temodar^{®}), Gemcitabine (e.g., GEMZAR^{®} (gemcitabine HCl)), Vasostatin oder eine Kombination zweier oder mehrerer der oben genannten.

Weiterer Gegenstand der vorliegenden Erfindung ist ein Verfahren zur Verhinderung der Blutkoagulation *in vitro,* insbesondere bei Blutkonserven oder biologischen Proben, die Blutplättchen enthalten, das dadurch gekennzeichnet ist, dass eine antikoagulatorisch wirksame Menge der erfindungsgemäßen Verbindung zugegeben wird.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende schnell und/oder modifiziert die erfindungsgemäßen Verbindungen abgebende Applikationsformen, die die erfindungsgemäßen Verbindungen in kristalliner und/ oder amorphisierter und/oder gelöster Form enthalten, wie z.B. Tabletten (nicht überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Bevorzugt ist die orale Applikation.

Für die sonstigen Applikationswege eignen sich z.B. Inhalationsarzneiformen (u.a. Pulverinhalatoren, Nebulizer), Nasentropfen, -lösungen, -sprays; lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme (wie beispielsweise Pflaster), Milch, Pasten, Schäume, Streupuder, Implantate oder Stents.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Laktose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und / oder Geruchskorrigentien.

Weiterer Gegenstand der vorliegenden Erfindung sind Arzneimittel, die mindestens eine erfindungsgemäße Verbindung, vorzugsweise zusammen mit einem oder mehreren inerten nichttoxischen, pharmazeutisch geeigneten Hilfsstoff enthalten, sowie deren Verwendung zu den zuvor genannten Zwecken.

Im Allgemeinen hat es sich als vorteilhaft erwiesen, bei parenteraler Applikation Mengen von etwa 5 bis 250 mg je 24 Stunden zur Erzielung wirksamer Ergebnisse zu verabreichen. Bei oraler Applikation beträgt die Menge etwa 5 bis 100 mg je 24 Stunden.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen. Die Angabe "w/v" bedeutet "weight/volume" (Gewicht/Volumen). So bedeutet beispielsweise "10% w/v": 100 ml Lösung oder Suspension enthalten 10 g Substanz.

### A) Beispiele

### Abkürzungen:

- ca.: circa
- CDI: Carbonyldiimidazol
- d: Tag(e), Dublett (bei NMR)
- DC: Dünnschicht-Chromatographie
- DCI: direkte chemische Ionisation (bei MS)
- dd: Doppeltes Dublett (bei NMR)
- DMAP: 4-Dimethylaminopyridin
- DMF: NN-Dimethylformamid
- DMSO: Dimethylsulfoxid
- d. Th.: der Theorie (bei Ausbeute)
- eq.: Äquivalent(e)
- ESI: Elektrospray-Ionisation (bei MS)
- h: Stunde(n)
- HATU: *O*-(7-Azabenzotriazol-1-yl)-*N,N,N'N'*-tetramethyluronium- Hexafluorphosphat
- HPLC: Hochdruck-, Hochleistungsflüssigchromatographie
- LC-MS: Flüssigchromatographie-gekoppelte Massenspektroskopie
- LDA: Lithiumdiisopropylamid
- m: Multiplett (bei NMR)
- min: Minute(n)
- MS: Massenspektroskopie
- NMR: Kernresonanzspektroskopie
- PYBOP: Benzotriazol-1-yloxy-tris(pyrrolidino)phosphonium- Hexafluorophosphat
- RP: reverse phase (bei HPLC)
- RT: Raumtemperatur
- Rₜ: Retentionszeit (bei HPLC)
- s: Singulett (bei NMR)
- THF: Tetrahydrofuran

### HPLC-Methoden

Methode 1A: Instrument: HP 1100 mit DAD-Detektion; Säule: Kromasil 100 RP-18, 60 mm x 2.1 mm, 3.5 µm; Eluent A: 5 ml Perchlorsäure (70%-ig) / 1 Wasser, Eluent B: Acetonitril; Gradient: 0 min 2% B → 0.5 min 2% B → 4.5 min 90% B → 6.5 min 90% B → 6.7 min 2% B → 7.5 min 2% B; Fluss: 0.75 ml/min; Säulentemperatur: 30°C; UV-Detektion: 210 nm.

Methode 2A: Instrument: HP 1100 mit DAD-Detektion; Säule: Kromasil 100 RP-18, 60 mm x 2.1 mm, 3.5 µm; Eluent A: 5 ml Perchlorsäure (70%-ig) / 1 Wasser, Eluent B: Acetonitril; Gradient: 0 min 2% B → 0.5 min 2% B → 4.5 min 90% B → 9 min 0% B → 9.2 min 2% B → 10 min 2% B; Fluss: 0.75 ml/min; Säulentemperatur: 30°C; UV-Detektion: 210 nm.

Methode 3A: Instrument: HP 1100 mit DAD-Detektion; Säule: Kromasil 100 RP-18, 60 mm x 2.1 mm, 3.5 µm; Eluent A: 5 ml Perchlorsäure (70%-ig) / 1 Wasser, Eluent B: Acetonitril; Gradient: 0 min 2% B → 0.5 min 2% B → 4.5 min 90% B → 15 min 90% B → 15.2 min 2% B → 16 min 2% B; Fluss: 0.75 ml/min; Säulentemperatur: 30°C; UV-Detektion: 210 nm.

Methode 4A: Phase: Kromasil 100, C18, 5 µm, 250 mm x 4 mm; Eluent: Wasser/Acetonitril 50:50; Fluss: 1 ml/min; T: 40°C; UV: 210 nm.

### LC-MS-Methoden:

Methode 1B: Gerätetyp MS: Micromass ZQ; Gerätetyp HPLC: HP 1100 Series; UV DAD; Säule: Phenomenex Gemini 3 µ, 30 mm x 3.0 mm; Eluent A: 11 Wasser + 0.5 ml 50%ige Ameisensäure, Eluent B: 11 Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min 2 ml/min; Ofen: 50°C; UV-Detektion: 210 nm.

Methode 2B: Instrument: Micromass QuattroPremier mit Waters UPLC Acquity; Säule: Thermo Hypersil GOLD 1.9 µ, 50 mm x 1 mm; Eluent A: 11 Wasser + 0.5 ml 50%ige Ameisensäure, Eluent B: 11 Acetonitril + 0.5 ml 50%ige Ameisensäure; Gradient: 0.0 min 90%A → 0.1 min 90%A → 1.5 min 10%A → 2.2 min 10%A; Ofen: 50°C; Fluss: 0.33 ml/min; UV-Detektion: 210 mm.

Methode 3B: Gerätetyp MS: Micromass ZQ; Gerätetyp HPLC: Waters Alliance 2795; Säule: Phenomenex Synergi 2.5 µ MAX-RP 100A Mercury, 20 mm x 4 mm; Eluent A: 11 Wasser + 0.5 ml 50%ige Ameisensäure, Eluent B: 11 Acetonitril + 0.5 ml 50%ige Ameisensäure; Gradient: 0.0 min 90%A → 0.1 min 90%A → 3.0 min 5%A → 4.0 min 5%A → 4.01 min 90%A; Fluss: 2 ml/min; Ofen: 50°C; UV-Detektion: 210 nm.

Methode 4B: Gerätetyp MS: Waters ZQ; Gerätetyp HPLC: Waters Alliance 2795; Säule: Phenomenex Onyx Monolithic C18, 100 mm x 3 mm; Eluent A: 11 Wasser + 0.5 ml 50%ige Ameisensäure, Eluent B: 1 1 Acetonitril + 0.5 ml 50%ige Ameisensäure; Gradient: 0.0 min 90%A → 2 min 65%A → 4.5 min 5%A → 6 min 5%A; Fluss: 2 ml/min; Ofen: 40°C; UV-Detektion: 210 nm.

Methode 5B: Instrument: Micromass Quattro Micro MS mit HPLC Agilent Serie 1100; Säule: Thermo Hypersil GOLD 3 µ 20 mm x 4 mm; Eluent A: 11 Wasser + 0.5 ml 50%ige Ameisensäure, Eluent B: 11 Acetonitril + 0.5 ml 50%ige Ameisensäure; Gradient: 0.0 min 100%A → 3.0 min 10%A → 4.0 min 10%A → 4.01 min 100%A → 5.00 min 100%A; Ofen: 50°C; Fluss: 2 ml/min; UV-Detektion: 210 nm.

Methode 6B: Instrument: Micromass Quattro LCZ mit HPLC Agilent Serie 1100; Säule: Phenomenex Synergi 2.5 µ MAX-RP 100A Mercury 20 mm x 4 mm; Eluent A: 11 Wasser + 0.5 ml 50%ige Ameisensäure, Eluent B: 11 Acetonitril + 0.5 ml 50%ige Ameisensäure; Gradient: 0.0 min 90%A → 0.1 min 90%A → 3.0 min 5%A → 4.0 min 5%A → 4.1 min 90%A; Fluss: 2 ml/min; Ofen: 50°C; UV-Detektion: 208-400 nm.

Methode 7B: Instrument: Micromass Quattro LCZ mit HPLC Agilent Serie 1100; Säule: Phenomenex Onyx Monolithic C18, 100 mm x 3 mm. Eluent A: 1 1 Wasser + 0.5 ml 50%ige Ameisensäure, Eluent B: 1 1 Acetonitril + 0.5 ml 50%ige Ameisensäure; Gradient: 0.0 min 90%A → 2 min 65%A → 4.5 min 5%A → 6 min 5%A; Fluss: 2 ml/min; Ofen: 40°C; UV-Detektion: 208-400 nm.

Methode 8B: Instrument: Micromass Platform LCZ mit HPLC Agilent Serie 1100; Säule: Thermo HyPURITY Aquastar 3µ 50 mm x 2.1 mm; Eluent A: 11 Wasser + 0.5 ml 50%-ige Ameisensäure, Eluent B: 1 1 Acetonitril + 0.5 ml 50%-ige Ameisensäure; Gradient: 0.0 min 100% A → 0.2 min 100% A → 2.9 min 30% A → 3.1 min 10% A → 5.5 min 10% A; Ofen: 50°C; Fluss: 0.8 ml/min; UV-Detektion: 210 nm.

Methode 9B: Instrument: Waters ACQUITY SQD UPLC System; Säule: Waters Acquity UPLC HSS T3 1.8µ 50 mm x 1 mm; Eluent A: 11 Wasser + 0.25 ml 99%ige Ameisensäure, Eluent B: 11 Acetonitril + 0.25 ml 99%ige Ameisensäure; Gradient: 0.0 min 90%A → 1.2 min 5%A → 2.0 min 5%A; Ofen: 50°C; Fluss: 0.40 ml/min; UV-Detektion: 210 - 400 nm.

Methode 10B: Instrument MS: Waters ZQ 2000; Instrument HPLC: Agilent 1100, 2-Säulen-Schaltung, Autosampler: HTC PAL; Säule: YMC-ODS-AQ, 50 mm x 4.6 mm, 3.0 µm; Eluent A: Wasser + 0.1% Ameisensaeure, Eluent B: Acetonitril + 0.1% Ameisensaeure; Gradient: 0.0 min 100%A → 0.2 min 95%A → 1.8 min 25%A → 1.9 min 10%A → 2.0 min 5%A → 3.2 min 5%A →3.21 min 100%A → 3.35 min 100%A; Ofen: 40°C; Fluss: 3.0 ml/min; UV-Detektion: 210 nm.

### Präparative Diastereomerentrennung:

Methode IC: Phase: Kromasil 100 C18, 5 µm 250 mm x 20 mm, Eluent: 0.2%-ige wässrige Trifluoressigsäure/Acetonitril 47:53; Fluss: 25 ml/min, Temperatur: 23°C; W-Detektion: 210 nm.

Methode 2C: Phase: Xbrdge C18, 5 µm OBD 19 mm x 150 mm, Eluent: Acetonitril/0.2%ige Trifluoressigsäure 50:50; Fluss: 25 ml/min, Temperatur: RT; UV-Detektion: 210 nm.

Methode 3C: Phase: Kromasil 100 C18, 5 µm 250 mm x 20 mm, Eluent: Acetonitril/0.2%ige Trifluoressigsäure 50:50; Fluss: 25 ml/min, Temperatur: RT; UV-Detektion: 210 nm.

Methode 4C: Phase: Kromasil 100 C18, 5 µm 250 mm x 20 mm, Eluent: Acetonitril/0.2%ige Trifluoressigsäure 57:43; Fluss: 25 ml/min, Temperatur: RT; UV-Detektion: 210 nm.

Methode 5C: Phase: Kromasil 100 C 18, 5 µm 250 mm x 20 mm, Eluent: Wasser : Acetonitril 25:75; Fluss: 25 ml/min, Temperatur: 35°C; UV-Detektion: 220 nm.

Methode 6C: Phase: Kromasil 100 C18, 5 µm 250 mm x 20 mm, Eluent: Acetonitril/Wasser 35:65; Fluss: 25 ml/min, Temperatur: 30°C; UV-Detektion: 210 nm.

Methode 7C: Phase: Sunfire C18, 5 µm 150 mm x 19 mm, Eluent: Wasser/Acetonitril 50:50; Fluss: 25 ml/min, Temperatur: 24°C; UV-Detektion: 225 nm.

Methode 8C: Phase: Kromasil 100 C18, 5 µm 250 mm x 20 mm, Eluent: Wasser/Acetonitril 50:50; Fluss: 25 ml/min, Temperatur: 40°C; UV-Detektion: 210 nm.

Methode 9C: Phase: Kromasil 100 C18, 5 µm 250 mm x 20 mm, Eluent: Wasser/Acetonitril 35:65; Fluss: 25 ml/min, Temperatur: 30°C; UV-Detektion: 210 nm.

Methode 10C: Phase: Sunfire C 18, 5 µm 150 mm x 30 mm, Eluent: Wasser/Acetonitril 50:50; Fluss: 56 ml/min, Temperatur: 30°C; UV-Detektion: 210 nm.

Methode 11C: Phase: Xbrdge C18, 5 µm OBD 19 mm x 150 mm, Eluent: Acetonitril/0.1% Amoniaklösung 55:45; Fluss: 25 ml/min, Temperatur: 28°C; UV-Detektion: 210 nm.

Methode 12C: Phase: Sunfire C 18, 5 µm OBD 19 mm x 150 mm, Eluent: Acetonitril/Wasser 42:58; Fluss: 25 ml/min, Temperatur: 45°C; UV-Detektion: 210 nm.

Methode 13C: Phase: Sunfire C18, 5 µm OBD 19 mm x 150 mm, Eluent: Acetonitril/Wasser 38:62; Fluss: 25 ml/min, Temperatur: 40°C; UV-Detektion: 210 nm.

Methode 14C: Phase: Sunfire C18, 5 µm OBD 19 mm x 150 mm, Eluent: Wasser/Acetonitril 52:48; Fluss: 25 ml/min, Temperatur: 40°C; UV-Detektion: 210 nm.

Methode 15C: Phase: Sunfire C18, 5 µm OBD 19 mm x 150 mm, Eluent: Wasser/Acetonitril 95:5; Fluss: 25 ml/min, Temperatur: 40°C; UV-Detektion: 210 nm.

### Präparative Enantiomerentrennung:

Methode 1D: Phase: Daicel Chiralcel OD-H, 5 µm 250 mm x 20 mm, Eluent: Isopropanol/iso-Hexan 40:60; Fluss: 15 ml/min, Temperatur: 24°C; UV-Detektion: 230 nm.

Methode 2D: Phase: Daicel Chiralpak AD-H, 5 µm 250 mm x 20 mm; Eluent: Isopropanol/iso-Hexan 20:80; Fluss: 20 ml/min; Temperatur: 25°C; UV-Detektion: 260 nm.

Methode 3D: Phase: Daicel Chiralcel OD-H, 5 µm 250 mm x 20 mm, Eluent: Ethanol/iso-Hexan 30:70; Fluss: 15 ml/min, Temperatur: 25°C; UV-Detektion: 230 nm.

Methode 4D: Phase: Daicel Chiralcel OD-H, 5 µm 250 mm x 20 mm, Eluent: Isopropanol/iso-Hexan 40:60; Fluss: 18 ml/min, Temperatur: 25°C; UV-Detektion: 230 nm.

Methode 5D: Phase: Daicel Chiralpak AD-H, 5 µm 250 mm x 20 mm; Eluent: Isopropanol/iso-Hexan 50:50; Fluss: 20 ml/min; Temperatur: 24°C; UV-Detektion: 260 nm.

Methode 6D: Phase: Daicel Chiralpak AD-H, 5 µm 250 mm x 20 mm; Eluent: Isopropanol/iso-Hexan 50:50; Fluss: 18 ml/min; T: 24°C; UV-Detektion: 230 nm.

Methode 7D: Phase: Daicel Chiralcel OD-H, 5 µm 250 mm x 20 mm, Eluent: Isopropanol/iso-Hexan 30:70; Fluss: 18 ml/min, Temperatur: 24°C; UV-Detektion: 230 nm.

Methode 8D: Phase: Daicel Chiralpak AD-H, 5 µm 250 mm x 20 mm, Eluent: iso-Hexan/Ethanol 25:75; Fluss: 15 ml/min, Temperatur: 40°C; UV-Detektion: 220 nm.

Methode 9D: Phase: Daicel Chiralpak AD-H, 5 µm 250 mm x 20 mm, Eluent: Ethanol 100%; Fluss: 12 ml/min, Temperatur: 40°C; UV-Detektion: 220 nm.

Methode 10D: Phase: Daicel Chiralpak AD-H, 5 µm 250 mm x 20 mm, Eluent: iso-Hexan/Isopropanol 40:60; Fluss: 15 ml/min, Temperatur: 40°C; UV-Detektion: 220 nm.

Methode 11D: Phase: Daicel Chiralpak AD-H, 5 µm 250 mm x 20 mm, Eluent: Ethanol 100%; Fluss: 15 ml/min, Temperatur: 40°C; UV-Detektion: 220 nm.

Methode 12D: Phase: Daicel Chiralpak AD-H, 5 µm 250 mm x 20 mm; Eluent: iso-Hexan/Isopropanol 30:70; Fluss: 15 ml/min; Temperatur: 40°C; W-Detektion: 220 nm.

Methode 13D: Phase: Daicel Chiralpak AD-H, 5 µm 250 mm x 20 mm, Eluent: iso-Hexan/Ethanol 30:70; Fluss: 15 ml/min, Temperatur: 40°C; UV-Detektion: 220 nm.

Methode 14D: Phase: Daicel Chiralpak AS-H, 5 µm 250 mm x 20 mm, Eluent: iso-Hexan/Ethanol 50:50; Fluss: 15 ml/min, Temperatur: 40°C; UV-Detektion: 220 nm.

Methode 15D: Phase: Daicel Chiralpak AS-H, 5 µm 250 mm x 20 mm, Eluent: iso-Hexan/Ethanol 70:30; Fluss: 15 ml/min, Temperatur: 40°C; UV-Detektion: 220 nm.

Methode 16D: Phase: Daicel Chiralpak AD-H, 5 µm 250 mm x 20 mm, Eluent: iso-Hexan/Isopropanol 50:50; Fluss: 15 ml/min, Temperatur: 40°C; UV-Detektion: 220 nm.

Methode 17D: Phase: Daicel Chiralpak OD-H, 5 µm 250 mm x 20 mm, Eluent: iso-Hexan/Isopropanol 40:60; Fluss: 15 ml/min, Temperatur: 24°C; UV-Detektion: 230 nm.

Methode 18D: Phase: Daicel Chiralpak OJ-H, 5 µm 250 mm x 20 mm, Eluent: iso-Hexan/Ethanol 60:40; Fluss: 20 ml/min, Temperatur: 28°C; UV-Detektion: 230 nm.

Methode 19D: Phase: Daicel Chiralpak AS-H, 5 µm 250 mm x 20 mm, Eluent: iso-Hexan/Isopropanol 50:50; Fluss: 20 ml/min, Temperatur: 24°C; UV-Detektion: 230 nm.

Methode 20D: Phase: Daicel Chiralpak AD-H, 5 µm 250 mm x 20 mm, Eluent: Ethanol/Heptan 50:50; Fluss: 25 ml/min, Temperatur: 40°C; UV-Detektion: 220 nm.

Methode 21D: Phase: Daicel Chiralpak AD-H, 5 µm 250 mm x 20 mm; Eluent: iso-Hexan/Isopropanol 30:70; Fluss: 15 ml/min; Temperatur: 30°C; UV-Detektion: 220 nm.

Methode 22D: Phase: Daicel Chiralpak AD-H, 5 µm 250 mm x 20 mm; Eluent: iso-Hexan/Isopropanol 50:50; Fluss: 25 ml/min; Temperatur: 40°C; UV-Detektion: 220 nm.

Methode 23D: Phase: Daicel Chiralpak AD-H, 5 µm 250 mm x 20 mm; Eluent: iso-Hexan/Ethanol 50:50; Fluss: 18 ml/min; Temperatur: 24°C; UV-Detektion: 230 nm.

Methode 24D: Phase: Daicel Chiralpak AD-H, 5 µm 250 mm x 20 mm, Eluent: iso-Hexan/Isopropanol 50:50; Fluss: 25 ml/min, Temperatur: 50°C; UV-Detektion: 210 nm.

Methode 25D: Phase: Daicel Chiralpak AD-H, 5 µm 250 mm x 20 mm, Eluent: iso-Hexan/Ethanol 50:50; Fluss: 20 ml/min, Temperatur: 40°C; UV-Detektion: 220 nm.

### Analytische Enantiomerentrennung:

Methode 1E: Phase: Daicel Chiralcel OD-H, 5 µm 250 mm x 4.6 mm, Eluent: Isopropanol/iso-Hexan 50:50; Fluss: 1 ml/min, Temperatur: 40°C; UV-Detektion: 220 nm.

Methode 2E: Phase: Daicel Chiralpak AD-H, 5 µm 250 mm x 4 mm; Eluent: Isopropanol/iso-Hexan: 20:80; Fluss: 1 ml/min; Temperatur: 24°C; UV-Detektion: 230 nm.

Methode 3E: Phase: Daicel Chiralcel OD-H, 5 µm 250 mm x 4 mm, Eluent: Ethanol/iso-Hexan 30:70; Fluss: 1 ml/min, Temperatur: 24°C; UV-Detektion: 230 nm.

Methode 4E: Phase: Daicel Chiralpak AD-H, 5 µm 250 mm x 4 mm; Eluent: IsopropanoUiso-Hexan: 50:50; Fluss: 1 ml/min; Temperatur: 24°C; UV-Detektion: 230 nm.

Methode 5E: Phase: Daicel Chiralpak AD-H, 5 µm 250 mm x 4 mm; Eluent: Isopropanol/iso-Hexan: 50:50; Fluss: 1 ml/min; Temperatur: 24°C; UV-Detektion: 230 nm.

Methode 6E: Phase: Daicel Chiralpak AD-H, 5 µm 250 mm x 4.6 mm; Eluent: iso-Hexan/Ethanol + 0.2% Diethylamin 25:75; Fluss: 1 ml/min; Temperatur: 40°C; UV-Detektion: 220 nm.

Methode 7E: Phase: Daicel Chiralpak AD-H, 5 µm 250 mm x 4.6 mm; Eluent: Ethanol 100%; Fluss: 1 ml/min; Temperatur: 40°C; UV-Detektion: 220 nm.

Methode 8E: Phase: Daicel Chiralpak AD-H, 5 µm 250 mm x 4.6 mm; Eluent: iso-Hexan/ Isopropanol + 0.2% Diethylamin 40:60; Fluss: 1 ml/min; Temperatur: 40°C; UV-Detektion: 235 nm.

Methode 9E: Phase: Daicel Chiralpak AD-H, 5 µm 250 mm x 4.6 mm; Eluent: iso-Hexan/ Isopropanol 30:70; Fluss: 1 ml/min; Temperatur: 45°C; UV-Detektion: 220 nm.

Methode 10E: Phase: Daicel Chiralpak AD-H, 5 µm 250 mm x 4.6 mm; Eluent: iso-Hexan/Ethanol 30:70; Fluss: 1 ml/min; Temperatur: 45°C; UV-Detektion: 220 nm.

Methode 11E: Phase: Daicel Chiralpak AS-H, 5 µm 250 mm x 4.6 mm; Eluent: iso-Hexan/Ethanol 50:50; Fluss: 1 ml/min; Temperatur: 40°C; UV-Detektion: 220 nm.

Methode 12E: Phase: Daicel Chiralpak AS-H, 5 µm 250 mm x 4.6 mm; Eluent: iso-Hexan/Ethanol 70:30; Fluss: 1 ml/min; Temperatur: 40°C; UV-Detektion: 220 nm.

Methode 13E: Phase: Daicel Chiralpak OD-H, 5 µm 250 mm x 4.0 mm; Eluent: iso-Hexan/ Isopropanol 50:50; Fluss: 1 ml/min; Temperatur: 40°C; UV-Detektion: 230 nm.

Methode 14E: Phase: Daicel Chiralpak OJ-H, 5 µm 250 mm x 4.0 mm; Eluent: iso-Hexan/Ethanol 50:50; Fluss: 1 ml/min; Temperatur: 40°C; UV-Detektion: 230 nm.

Methode 15E: Phase: Daicel Chiralpak AS-H, 5 µm 250 mm x 4.0 mm; Eluent: iso-Hexan/ Isopropanol 50:50; Fluss: 1 ml/min; Temperatur: 40°C; UV-Detektion: 230 nm.

Methode 16E: Phase: Daicel Chiralpak AD-H, 5 µm 250 mm x 4.6 mm; Eluent: iso-Hexan/ Isopropanol 50:50; Fluss: 1 ml/min; Temperatur: 40°C; UV-Detektion: 220 nm.

Methode 17E: Phase: Daicel Chiralpak AS-H, 5 µm 250 mm x 4.6 mm; Eluent: iso-Hexan[Ethanol 60:40; Fluss: 1 ml/min; Temperatur: 40°C; UV-Detektion: 220 nm.

Methode 18E: Phase: Daicel Chiralpak AD-H, 5 µm 250 mm x 4.6 mm; Eluent: iso-Hexan/ Isopropanol 30:70; Fluss: 1 ml/min; Temperatur: 40°C; UV-Detektion: 220 nm.

Methode 19E: Phase: Daicel Chiralpak AS-H, 5 µm 250 mm x 4.6 mm; Eluent: iso-Hexan/Ethanol 30:70; Fluss: 1 ml/min; Temperatur: 40°C; UV-Detektion: 220 nm.

Methode 20E: Phase: Daicel Chiralpak AD-H, 5 µm 250 mm x 4 mm; Eluent: iso-Hexan/Ethanol 50:50; Fluss: 1 ml/min; Temperatur: 24°C; UV-Detektion: 230 nm.

Methode 21E: Phase: Daicel Chiralpak AD-H, 5 µm 250 mm x 4.6 mm; Eluent: iso-Hexan/Ethanol 50:50; Fluss: 1 ml/min; Temperatur: 40°C; UV-Detektion: 220 nm.

### GC-MS-Methoden:

Methode 1F: Instrument: Micromass GCT, GC6890; Säule: Restek RTX-35, 15 m x 200 µm x 0.33 µm; konstanter Fluss mit Helium: 0.88 ml/min; Ofen: 70°C; Inlet: 250°C; Gradient: 70°C, 30°C/min → 310°C (3 min halten).

Als Mikrowellenreaktor wurde ein "single mode" Gerät vom Typ Emrys^{™} Optimizer verwendet.

### Ausgangsverbindungen

### Allgemeine Methode 1A: Suzuki-Kupplung

Eine Mischung des entsprechenden Brompyridins in Toluol (1.8 ml/mmol) wird unter Argon bei RT mit Tetrakis-(triphenylphosphin)-palladium (0.02 eq.), mit einer Lösung der entsprechenden Arylboronsäure (1.2 eq.) in Ethanol (0.5 ml/mmol) und mit einer Lösung aus Kaliumfluorid (2.0 eq.) in Wasser (0.2 ml/mmol) versetzt. Das Reaktionsgemisch wird mehrere Stunden bis zur weitgehend vollständigen Umsetzung unter Rückfluß gerührt. Nach Zugabe von Ethylacetat und Phasentrennung wird die organische Phase einmal mit Wasser und einmal mit gesättigter, wässriger Natriumchlorid-Lösung gewaschen, getrocknet (Magnesiumsulfat), filtriert und im Vakuum eingeengt. Das Rohprodukt wird mittels Flash-Chromatographie (Kieselgel-60, Eluent: Dichlormethan-Methanol-Gemische) aufgereinigt.

### Allgemeine Methode 2A: Hydrierung des Pyridins

Eine Lösung des Pyridins in Ethanol (9 ml/mmol) wird mit Palladium auf Aktivkohle (angefeuchtet mit ca. 50% Wasser, 0.3 g/mmol) versetzt und bei 60°C über Nacht in einer 50 bar Wasserstoff-Atmosphäre hydriert. Anschließend wird der Katalysator über eine Filterschicht abfiltriert und mehrmals mit Ethanol gewaschen. Die vereinigten Filtrate werden im Vakuum eingeengt.

### Allgemeine Methode 3A: Umsetzung mit Carbamoylchloriden oder Carbonylchloriden

Eine Lösung des Piperidins in Dichlormethan (2.5 ml/mmol) wird unter Argon bei 0°C tropfenweise mit N,N Diisopropylethylamin (1.2 eq.) und dem entsprechenden Carbamoylchlorid oder Carbonylchlorid (1.2 eq.) versetzt. Das Reaktionsgemisch wird bei RT gerührt. Nach Zugabe von Wasser und Phasentrennung wird die organische Phase dreimal mit Wasser und einmal mit gesättigter, wässriger Natriumchlorid-Lösung gewaschen, getrocknet (Natriumsulfat), filtriert und im Vakuum eingeengt.

### Allgemeine Methode 4A: Verseifung

Eine Lösung des entsprechenden Esters in einem Gemisch aus Tetrahydrofuran/Wasser (3:1, 12.5 ml/mmol) wird bei RT mit Lithiumhydroxid (2 eq.) versetzt. Das Reaktionsgemisch wird bei 60°C gerührt und anschließend mit wässriger, 1 N Salzsäure-Lösung auf pH 1 gestellt. Nach Zugabe von Wasser/Ethylacetat wird die wässrige Phase dreimal mit Ethylacetat extrahiert. Die vereinigten organischen Phasen werden getrocknet (Natriumsulfat), filtriert und im Vakuum eingeengt.

### Allgemeine Methode 5A: N'-Hydroxyimidamidbildung

Eine Lösung aus dem entsprechenden Nitril (1.0 eq) in Ethanol (1.2 ml/mmol) wird bei RT mit Hydroxylammoniumchlorid (1.5 eq.) und Triethylamin (1.2 eq.) versetzt. Das Reaktionsgemisch wird über Nacht bei Raumtemperatur gerührt. Zur Aufarbeitung wird Ethanol im Vakuum entfernt, das Reaktionsgemisch mit gesättigter, wässriger Natriumhydrogencarbonat-Lösung versetzt und mit Essigsäureethylester extrahiert. Die organische Phase wird über Natriumsulfat getrocknet und eingeengt. Der Rückstand wird ohne weitere Reinigung umgesetzt.

### Allgemeine Methode 6A: N'-Hydroxyimidamidbildung

Eine Lösung aus dem entsprechenden Nitril (1.0 eq) in einem Gemisch aus Ethanol (1.9 ml/mmol) und Wasser (0.5 ml/mmol) wird bei RT mit Hydroxylammoniumchlorid (1.08 eq.) und Natriumhydroxid (1.12 eq.) versetzt. Das Reaktionsgemisch wird 16 h bei Raumtemperatur gerührt. Zur Aufarbeitung wird das Reaktionsgemisch im Vakuum eingeengt, mit Dichlormethan versetzt und filtriert. Das Filtrat wird im Vakuum eingeengt und der Rückstand ohne weitere Aufreinigung umgesetzt.

### Allgemeine Methode 7A: Umsetzung mit Carbonylchloriden

Eine Lösung des Piperidins in Dichlormethan (4 ml/mmol) wird unter Argon bei 0°C tropfenweise mit Triethylamin (1.5 eq.) und dem entsprechenden Carbonylchlorid (2.0 eq.) versetzt. Das Reaktionsgemisch läßt man langsam auf RT erwärmen. Nach Zugabe von Wasser und Phasentrennung wird die organische Phase zweimal mit Wasser gewaschen, über Natriumsulfat getrocknet, filtriert und im Vakuum eingeengt. Falls die Reinheit des Rohproduktes nicht ausreicht, wird die Substanz mittels präparativer HPLC aufgereinigt.

### Allgemeine Methode 8A: Harnstoffbildung

Eine Lösung des Nitrophenylcarbamates (1.0 eq.) in Dimethylformamid (10 ml/mmol) wird bei RT mit dem entsprechenden Amin (2.0-3.0 eq.) und Kaliumcarbonat (1.0 eq.) versetzt und in 15 ml-Portionen in einer *Single* Mode-Mikrowelle (Emrys Optimizer) für 0.5-1 h bei 150°C gerührt. Die Reaktionslösung wird filtriert und das Filtrat mittels präparativer HPLC aufgereinigt.

### Allgemeine Methode 9A: Methylesterverseifung/Epimerisierung

Zu einer Lösung des entsprechenden Methylesters (1.0 eq.) in Methanol (35-40 ml/mmol) wird bei RT Kalium-tert.-butylat (10 eq.) gegeben. Die Mischung wird über Nacht bei 60°C gerührt. Bei unvollständiger Umsetzung wird Wasser (1.0 eq.) zugesetzt und bis zum vollständigen Umsatz bei 60°C gerührt. Zur Aufarbeitung wird im Vakuum das Methanol entfernt, der Rückstand mit Wasser versetzt und mit wässriger 1 N Salzsäure-Lösung sauer (pH 1) gestellt. Die Mischung wird mit Essigsäureethylester extrahiert, die organische Phase mit Magnesiumsulfat getrocknet, filtriert und im Vakuum eingeengt.

### Allgemeine Methode 10A: Thioamidbildung

Zu einer Lösung der entsprechenden Säure (1.0 eq.) in Dioxan (5.6 ml/mmol) wird Lawesson-Reagens (2,4-Bis[4-methoxyphenyl]1,3-dithia-2,4-diphosphetan-2,4-disulfid) (0.6 eq.) hinzugegeben. Das Reaktionsgemisch wird 30 Minuten bei 60°C gerührt. Nach Zugabe von gesättigter, wässriger Natriumhydrogencarbonat-Lösung und Entfernung des Dioxans wird der Rückstand mit Ethylacetat extrahiert. Die vereinigten organischen Phasen werden mit einer gesättigten, wässrigen Natriumhydrogencarbonat-Lösung gewaschen, über Magnesiumsulfat getrocknet, filtriert und im Vakuum eingeengt.

### Allgemeine Methode 11A: Carbohydrazidbildung (J. Med. Chem.1996, 39, 2753-2763)

Zu einer Lösung der entsprechenden Säure (1.0 eq.) in Dichlormethan (1 ml/0.24 mmol) wird bei Raumtemperatur Oxalsäuredichlorid (2.0 eq.) gegeben. Die Mischung wird 1 h bei RT gerührt. Zur Aufarbeitung wird im Vakuum Dichlormethan entfernt und der Rückstand noch einmal in Dichlormethan versetzt und eingeengt. Der Rückstand wird in Dichlormethan vorgelegt und mit dem entsprechenden Hydrazid versetzt. Nach 20 Minuten bei RT wird das Reaktionsgemisch mit einer gesättigten, wässrigen Ammoniumhydroxid-Lösung versetzt und mit Dichlormethan extrahiert. Die vereinigten organischen Phasen werden mit einer gesättigten, wässrigen Natriumchlorid-Lösung gewaschen, über Magnesiumsulfat getrocknet, filtriert und im Vakuum eingeengt.

### Allgemeine Methode 12A: Hydrierung des Pyridins mittels DurchFLuss-Hydrierapparatur

Eine Lösung des Pyridins in konzentrierter Essigsäure (ca. 35 ml/mmol) wird in einer Durchfluss-Hydrierapparatur ("H-Cube" der Firma ThalesNano, Budapest, Ungarn) hydriert (Bedingungen: 10% Pd/C-Katalysator, "controlled"-Modus, 60 bar, 0.5 ml/min, 85°C). Nach Entfernen des Lösungsmittels am Rotationsverdampfer wird das entsprechende Rohprodukt erhalten, welches gegebenenfalls mittels präparativer HPLC gereinigt wird.

### Beispiel 1A

### 5-(4-Ethylphenyl)pyridin-3-carbonsäuremethylester

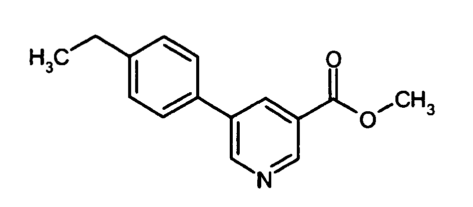

Nach der Allgemeinen Methode 1A wurden 32 g (148 mmol) 5-Bromnicotinsäuremethylester und 27 g (178 mmol, 1.2 eq.) 4-Ethylphenylboronsäure umgesetzt. Ausbeute: 24 g (64% d. Th.)

LC-MS (Methode 3B): Rₜ = 2.03 min; MS (ESIpos): m/z = 242 [M+H]⁺.

### Beispiel 2A

### 5-(4-Ethylphenyl)piperidin-3-carbonsäuremethylester [racemisches cis-/trans-Isomerengemisch]

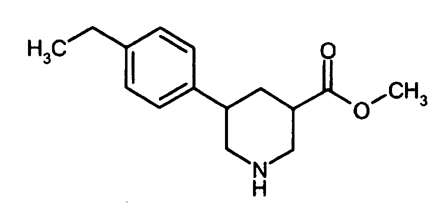

Nach der Allgemeinen Methode 2A wurden 24 g (94 mmol) 5-(4-Ethylphenyl)pyridin-3-carbonsäuremethylester hydriert. Ausbeute: 20 g (77% d. Th.)

LC-MS (Methode 5B): Rₜ = 1.43 min; MS (ESIpos): m/z = 248 [M+H]⁺.

### Beispiel 3A

### 5-(4-Ethylphenyl)pyridin-3-carbonsäureethylester

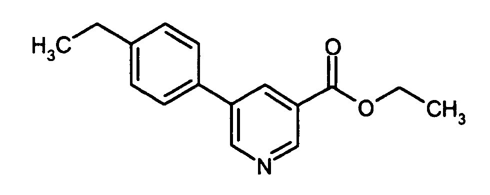

Nach der Allgemeinen Methode 1A wurden 29 g (126 mmol) 5-Bromnicotinsäureethylester und 23 g (152 mmol, 1.2 eq.) 4-Ethylphenylboronsäure umgesetzt. Ausbeute: 32 g (82% d. Th.)

LC-MS (Methode 4B): Rₜ = 3.80 min; MS (ESIpos): m/z = 256 [M+H]⁺.

### Beispiel 4A

### 5-(4-Ethylphenyl)piperidin-3-carbonsäureethylester [racemisches cis-/trans-Isomerengemisch]

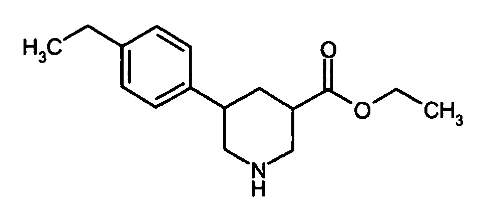

Nach der Allgemeinen Methode 2A wurden 24 g (71 mmol) 5-(4-Ethylphenyl)pyridin-3-carbonsäureethylester hydriert. Ausbeute: 15 g (81% d. Th.)

LC-MS (Methode 5B): Rₜ = 1.78 min und 1.91 min (cis-/trans-Isomere); MS (ESIpos): m/z = 262 [M+H]⁺.

### Beispiel 5A

### 1-(Cyclopentylcarbonyl)-5-(4-ethylphenyl)piperidin-3-carbonsäureethylester [racemisches cis-/ trans-Isomerengemisch]

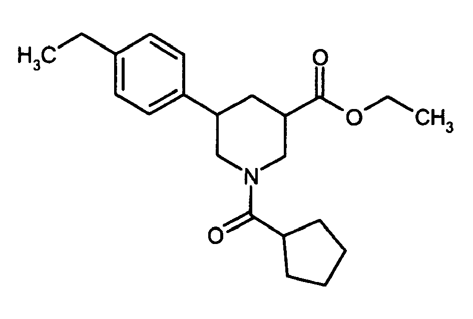

Nach der Allgemeinen Methode 3A wurden 5.2 g (14.0 mmol) 5-(4-Ethylphenyl)piperidin-3-carbonsäureethylester mit 2.1 g (2.1 mmol, 1.2 eq.) Cyclopentancarbonylchlorid umgesetzt. Ausbeute: 4.8 g (96% d. Th.)

LC-MS (Methode 4B): Rₜ = 4.04 min und 4.14 min (cis-/trans-Isomere); MS (ESIpos): m/z = 358 [M+H]⁺.

### Beispiel 6A

### 1-(Cyclopentylcarbonyl)-5-(4-ethylphenyl)piperidin-3-carbonsäure [racemisches cis-/trans-Isomerengemisch]

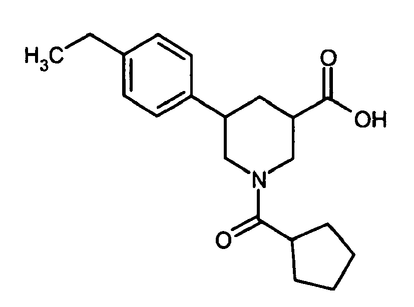

Nach der Allgemeinen Methode 4A wurden 13.8 g (38.6 mmol) 1-(Cyclopentylcarbonyl)-5-(4-ethylphenyl)piperidin-3-carbonsäureethylester verseift. Ausbeute: 11.5 g (87% d. Th.)

LC-MS (Methode 1B): Rₜ = 2.50 min und 2.57 min (cis-/trans-Isomere); MS (ESIpos): m/z = 330 (M+H]⁺.

Diastereomerentrennung von 11.5 g des cis-/trans-Isomerengemisches nach Methode 1C ergab 4.1 g der Titelverbindung 7A (cis-Isomer) und 4.1 g des trans-Isomers.

### Beispiel 7A

### 1-(Cyclopentylcarbonyl)-5-(4-ethylphenyl)piperidin-3-carbonsäure [racemisches cis-Isomer]

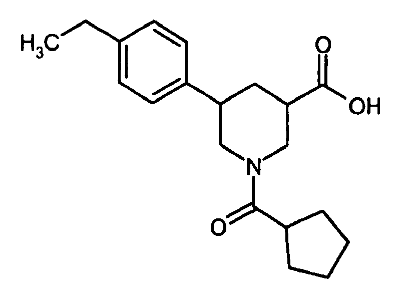

LC-MS (Methode 1B): Rₜ = 2.57 min; MS (ESIpos): m/z = 330 [M+H]⁺.

### Beispiel 8A

### 5-[4-(1-Methylethyl)phenyl]pyridin-3-carbonsäureethylester

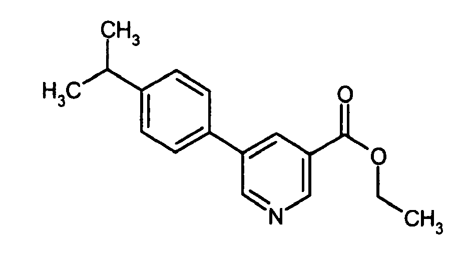

4.68 g (20.32 mmol) 5-Bromnicotinsäureethylester, 5.00 g (30.49 mmol) 4-(1-Methylethyl)phenylboronsäure, 0.12 g (0.10 mmol) Tetrakis(triphenylphosphin)palladium(0) und 4.31 g (40.65 mmol) Natriumcarbonat wurden in einer Mischung von 37 ml 1,2-Dimethoxyethan, 10.5 ml Wasser und 84 ml Dimethylformamid gelöst und 18 h bei 85°C gerührt. Zur Aufarbeitung wurde ein Teil des Dimethylformamids im Vakuum entfernt, das Reaktionsgemisch mit Wasser verdünnt und mit Dichlormethan extrahiert. Die organische Phase wurde über Natriumsulfat getrocknet und das Rohprodukt mittels präparativer HPLC gereinigt. Ausbeute: 2.42 g (44% d. Th.)

LC-MS (Methode 6B): Rₜ = 2.56 min; MS (ESIpos): m/z = 270 [M+H]⁺.

### Beispiel 9A

### 5-[4-(1-Methylethyl)phenyl]piperidin-3-carbonsäureethylester [racemisches cis-/trans-Isomerengemisch]

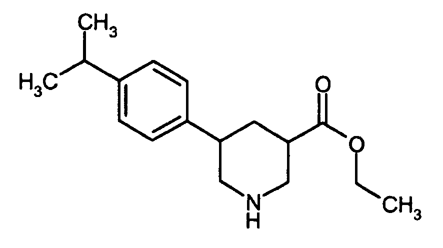

2.4 g (8.9 mmol) 5-[4-(1-Methylethyl)phenyl]pyridin-3-carbonsäureethylester wurden in 60 ml Ethanol gelöst, mit 1.33 g Pd/C (10%ig) versetzt und in einem Autoklaven bei 60°C bei einem Wasserstoffdruck von 50 bar über Nacht hydriert. Das Reaktionsgemisch wurde über Kieselgel filtriert. Die erhaltene Lösung wurde im Vakuum eingeengt. Der Rückstand wurde mit Wasser versetzt, die Lösung mit wässriger 1 N Natriumhydroxid-Lösung auf pH 8 gestellt. Anschließend wurde mit Dichlormethan extrahiert. Die organische Phase wurde über Natriumsulfat getrocknet und eingeengt. Ausbeute: 2.1 g (81% d. Th.)

LC-MS (Methode 1B): Rₜ = 1.57 min und 2.59 min (cis-/trans-Isomere); MS (ESIpos): m/z = 276 [M+H]⁺.

### Beispiel 10A

### 1-(Cyclopentylcarbonyl)-5-[4-(1-methylethyl)phenyl]piperidin-3-carbonsäureethylester [racemisches cis-/trans-Isomerengemisch]

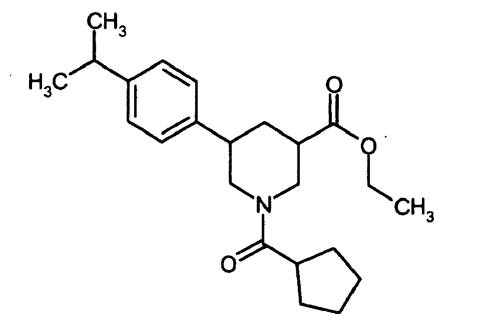

1.5 g (5.3 mmol) 5-[4-(1-Methylethyl)phenyl]piperidin-3-carbonsäureethylester wurden in 18 ml Dichlormethan gelöst und bei 0°C mit 0.81 g (7.9 mmol) Triethylamin versetzt. Anschließend wurden 1.43 g (10.5 mmol) Cyclopentancarbonylchlorid zugetropft. Die Reaktionsmischung wurde für 2 h bei RT gerührt. Zur Aufarbeitung wurde zweimal mit Wasser gewaschen. Die organische Phase wurde über Natriumsulfat getrocknet und im Vakuum eingeengt. Ausbeute: 2.0 g (100% d. Th.)

LC-MS (Methode 3B): Rₜ = 2.56 min und 2.63 min (cis-/trans-Isomere); MS (ESIpos): m/z = 372 [M+H]⁺.

### Beispiel 11A

### 1-(Cyclopentylcarbonyl)-5-[4-(1-methylethyl)phenyl]piperidin-3-carbonsäure [racemisches cis-/ trans-Isomerengemisch]

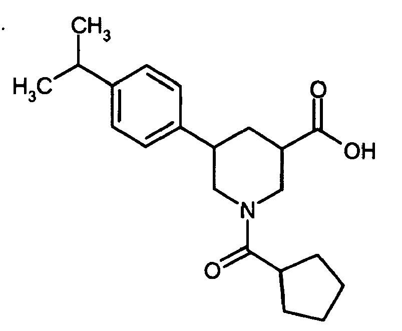

Zu 2.40 g (6.46 mmol) 1-(Cyclopentylcarbonyl)-5-[4-(1-methylethyl)phenyl]piperidin-3-carbonsäureethylester wurden 50 ml Dioxan, 25 ml Wasser und 0.63 g (25.8 mmol) Lithiumhydroxid gegeben. Die Mischung wurde über Nacht bei RT gerührt. Zur Aufarbeitung wurde im Vakuum Dioxan entfernt, das Reaktionsgemisch mit Wasser versetzt und mit wässriger 1 N Salzsäure-Lösung sauer gestellt. Die Mischung wurde mit Dichlormethan extrahiert. Die organische Phase wurde mit Natriumsulfat getrocknet und im Vakuum eingeengt. Ausbeute: 1.65 g (98% d. Th.)

Diastereomerentrennung von 1.65 g des cis-/trans-Isomerengemisches nach Methode 2C ergab 553 mg der Titelverbindung 12A (cis-Isomer) und 638 mg des trans-Isomers.

### Beispiel 12A

### 1-(Cyclopentylcarbonyl)-5-[4-(1-methylethyl)phenyl]piperidin-3-carbonsäure [racemisches cis-Isomer]

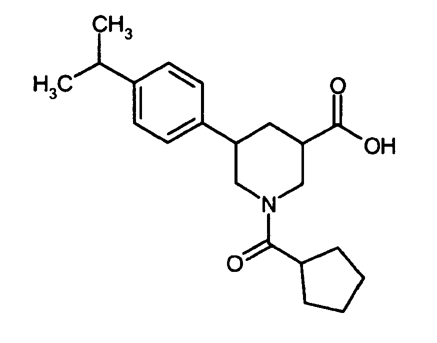

LC-MS (Methode 1B): Rₜ = 2.72 min; MS (ESIpos): m/z = 344 [M+H]⁺.

### Bespiel 1

### 5-[4-(Trifluormethyl)phenyl]pyridin-3-carbonsäureethylester

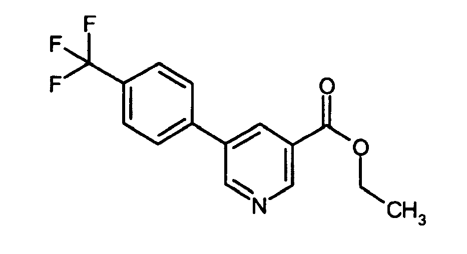

6.74 g (29.3 mmol) 5-Bromnicotinsäureethylester, 8.35 g (43.9 mmol) 4-Trifluormethylphenylboronsäure, 0.17 g (0.15 mmol) Tetrakis(triphenylphosphin)palladium(0) und 6.21 g (58.6 mmol) Natriumcarbonat wurden in einer Mischung von 75 ml 1,2-Dimethoxyethan, 15 ml Wasser und 184 ml Dimethylformamids gelöst und 18 h bei 85°C gerührt. Zur Aufarbeitung wurde ein Teil des Dimethylformamids im Vakuum entfernt, das Reaktionsgemisch mit Wasser verdünnt und mit Dichlormethan extrahiert. Die organische Phase wurde über Natriumsulfat getrocknet und das Rohprodukt mittels Flashchromatographie gereinigt (Dichlormethan/Acetonitril 100:2→ 100:5). Ausbeute: 6.22 g (72% d. Th.)

LC-MS (Methode 4B): Rₜ = 3.71 min; MS (ESIpos): m/z = 296 [M+H]⁺.

### Beispiel 14A

### 5-[4-(Trifluormethyl)phenyl]piperidin-3-carbonsäureethylester [racemisches cis-/trans-Isomerengemisch]

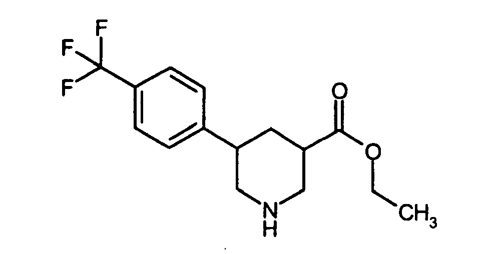

5.9 g (20.0 mmol) 5-[4-(Trifluormethyl)phenyl]pyridin-3-carbonsäureethylester wurden in 140 ml Ethanol gelöst, mit 2.98 g Pd/C (10%ig) versetzt und in einem Autoklaven bei 60°C bei einem Wasserstoffdruck von 50 bar über Nacht hydriert. Das Reaktionsgemisch wurde über Kieselgel filtriert. Die erhaltene Lösung wurde im Vakuum eingeengt. Der Rückstand wurde mit Wasser versetzt, die Lösung mit wässriger 1 N Natriumhydroxid-Lösung auf pH 8 gestellt. Anschließend wurde mit Dichlormethan extrahiert. Die organische Phase wurde über Natriumsulfat getrocknet und eingeengt. Ausbeute: 4.2 g (67% d. Th.)

LC-MS (Methode 4B): Rₜ = 1.95 min und 2.03 min (cis-/trans-Isomere); MS (ESIpos): m/z = 302 [M+H]⁺.

### Beispiel 15A

### 1-(Cyclopentylcarbonyl)-5-[4-(trifluormethyl)phenyl]piperidin-3-carbonsäureethylester [racemisches cis-/trans-Isomerengemisch]

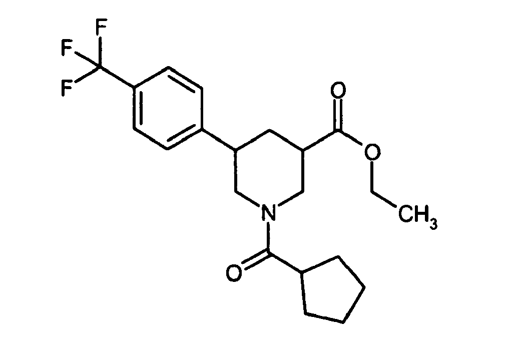

4.2 g (13.5 mmol) 5-[4-(Trifluormethyl)phenyl]piperidin-3-carbonsäureethylester wurden in 33 ml Dichlormethan gelöst und bei 0°C mit 2.04 g (20.2 mmol) Triethylamin versetzt. Anschließend wurden 2.37 g (17.5 mmol) Cyclopentancarbonylchlorid zugetropft. Die Reaktionsmischung wurde für 2 h bei RT gerührt. Zur Aufarbeitung wurde zweimal mit Wasser gewaschen. Die organische Phase wurde über Natriumsulfat getrocknet und im Vakuum eingeengt. Ausbeute: 5.9 g (100% d. Th.)

LC-MS (Methode 7B): Rₜ = 4.08 min und 4.1 min (cis-/trans-Isomere); MS (ESIpos): m/z = 398 [M+H]⁺.

### Beispiel 16A

### 1-(Cyclopentylcarbonyl)-5-[4-(trifluormethyl)phenyl]piperidin-3-carbonsäure [racemisches cis-/ trans-Isomerengemisch]

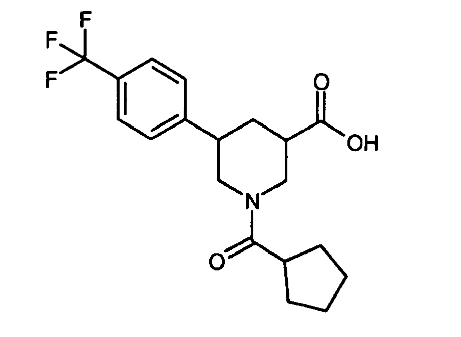

Zu 5.9 g (14.3 mmol) 1-(Cyclopentylcarbonyl)-5-[4-(trifluormethyl)phenyl]piperidin-3-carbonsäureethylester wurden 99 ml Dioxan, 50 ml Wasser und 1.37 g (57.0 mmol) Lithiumhydroxid gegeben. Die Mischung wurde über Nacht bei RT gerührt. Zur Aufarbeitung wurde im Vakuum Dioxan entfernt, das Reaktionsgemisch mit Wasser versetzt und mit wässriger 1 N Salzsäure-Lösung sauer gestellt. Die Mischung wurde mit Dichlormethan extrahiert. Die organische Phase wurde mit Natriumsulfat getrocknet und im Vakuum eingeengt. Ausbeute: 5.3 g (95% d. Th.)

Diastereomerentrennung von 5.3 g des cis-/trans-Isomerengemisches nach Methode 3C ergab 1.53 g der Titelverbindung 17A (cis-Isomer) und 1.949 g des trans-Isomers.

### Beispiel 17A

### 1-(Cyclopentylcarbonyl)-5-[4-(trifluormethyl)phenyl]piperidin-3-carbonsäure [racemisches cis-Isomer]

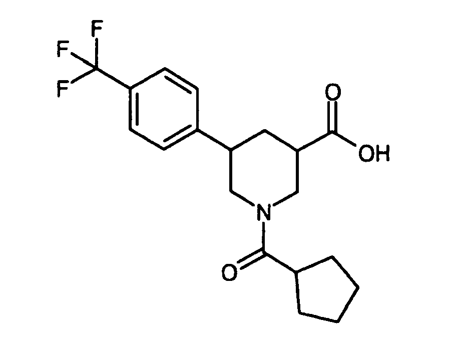

HPLC (Methode 3C): Rₜ = 3.66 min (cis-Isomer).

### Beispiel 18A

### 5-[4-(Trifluormethoxy)phenyl]pyridin-3-carbonsäuremethylester

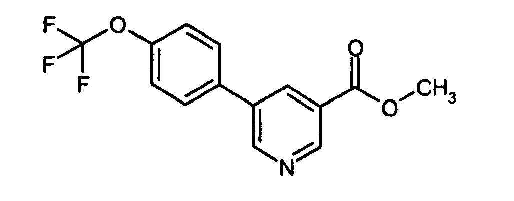

Nach der Allgemeinen Methode 1A wurden 23 g (105 mmol) 5-Bromnicotinsäuremethylester und 26 g (126 mmol, 1.2 eq.) 4-Trifluormethoxyphenylboronsäure umgesetzt. Ausbeute: 14 g (41% d. Th.)

LC-MS (Methode 1B): Rₜ = 2.44 min; MS (ESIpos): m/z = 298 [M+H]⁺.

### Beispiel 19A

### 5-[4-(Trifluormethoxy)phenyl]piperidin-3-carbonsäuremethylester [racemisches cis-/trans-Isomerengemisch]

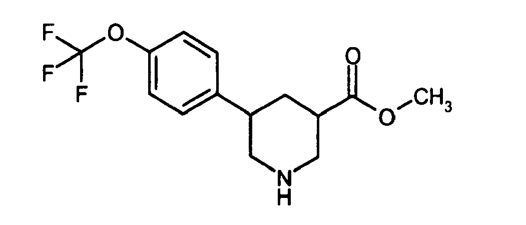

Nach der Allgemeinen Methode 2A wurden 14 g (45 mmol) 5-[4-(Trifluormethoxy)-phenyl]pyridin-3-carbonsäuremethylester hydriert. Ausbeute: 8 g (59% d. Th.)

LC-MS (Methode 1B): Rₜ = 1.29 min und 1.33 min (cis-/trans-Isomere); MS (ESIpos): m/z = 304 IM+H]⁺.

### Beispiel 20A

### 5-[4-(Trifluormethoxy)phenyl]pyridin-3-carbonsäureethylester

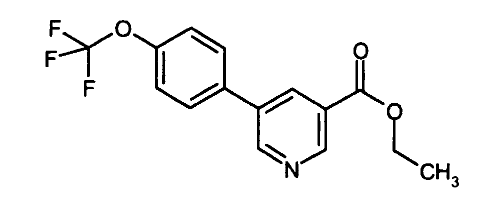

3.35 g (14.6 mmol) 5-Bromnicotinsäureethylester, 4.50 g (21.9 mmol) 4-(Trifluormethoxy)-phenylboronsäure, 0.84 g (0.73 mmol) Tetrakis(triphenylphosphin)palladium(0) und 3.01 g (29.1 mmol) Natriumcarbonat wurden in einer Mischung von 38 ml 1,2-Dimethoxyethan, 7.5 ml Wasser und 91 ml Dimethylformamid gelöst und 18 h bei 85°C gerührt. Zur Aufarbeitung wurde ein Teil des Dimethylformamids im Vakuum entfernt, das Reaktionsgemisch mit Wasser verdünnt und mit Dichlormethan extrahiert. Die organische Phase wurde über Natriumsulfat getrocknet und das Rohprodukt mittels Flashchromatographie (Dichlormethan/Acetonitril 100:0→100:5) gereinigt. Ausbeute: 2.64 g (55% d. Th.)

LC-MS (Methode 4B): R₁ = 3.78 min; MS (ESIpos): m/z = 312 [M+H]⁺.

### Beispiel 21A

### 5-[4-(Trifluormethoxy)phenyl]piperidin-3-carbonsäureethylester [racemisches cis-/trans-Isomerengemisch]

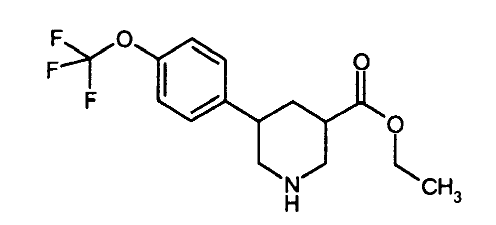

2.5 g (7.6 mmol) 5-[4-(Trifluormethoxy)phenyl]pyridin-3-carbonsäureethylester wurden in 60 ml Ethanol gelöst, mit 1.14 g Pd/C (10%ig) versetzt und in einem Autoklaven bei 60°C bei einem Wasserstoffdruck von 50 bar über Nacht hydriert. Das Reaktionsgemisch wurde über Kieselgel filtriert. Die erhaltene Lösung wurde im Vakuum eingeengt. Der Rückstand wurde mit Wasser versetzt, die Lösung mit wässriger 1 N Natriumhydroxid-Lösung auf pH 8 gestellt. Anschließend wurde mit Dichlormethan extrahiert. Die organische Phase wurde über Natriumsulfat getrocknet und eingeengt. Ausbeute: 1.76 g (66% d. Th.)

LC-MS (Methode 7B): Rₜ = 2.47 min und 2.56 min (cis-/trans-Isomere); MS (ESIpos): m/z = 318 [M+H]⁺.

### Beispiel 22A

### 1-(Cyclopentylcarbonyl)-5-[4-(trifluormethoxy)phenyl]piperidin-3-carbonsäureethylester [racemisches cis-/trans-Isomerengemisch]

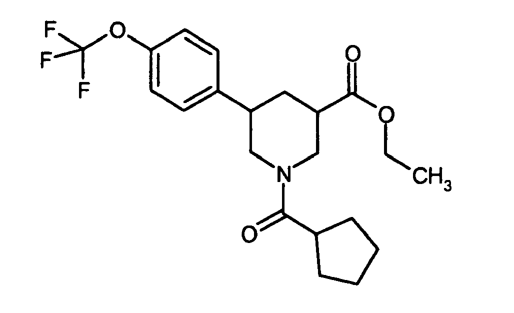

1.76 g (5.08 mmol) 5-[4-(Trifluormethoxy)phenyl]piperidin-3-carbonsäureethylester wurden in 12 ml Dichlormethan gelöst und bei 0°C mit 0.77 g (7.62 mmol) Triethylamin versetzt. Anschließend wurden 0.89 g (6.61 mmol) Cyclopentancarbonylchlorid zugetropft. Die Reaktionsmischung wurde für 2 h bei RT gerührt. Zur Aufarbeitung wurde zweimal mit Wasser gewaschen. Die organische Phase wurde über Natriumsulfat getrocknet und im Vakuum eingeengt. Ausbeute: 2.1 g (100% d. Th.)

LC-MS (Methode 7B): Rₜ = 4.14 min und 4.22 min (cis-/trans-Isomere); MS (ESIpos): m/z = 414 [M+H]⁺.

### Beispiel 23A

### 1-(Cyclopentylcarbonyl)-5-[4-(trifluormethoxy)phenyl]piperidin-3-carbonsäure [racemisches cis-/ trans-Isomerengemisch]

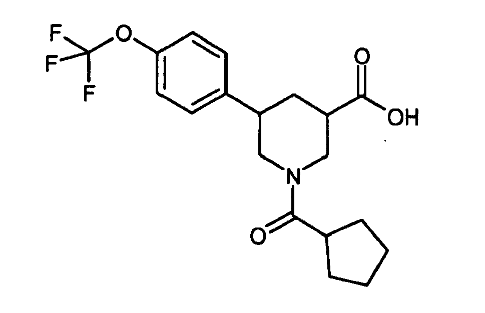

Zu 2.30 g (5.34 mmol) 1-(Cyclopentylcarbonyl)-5-[4-(trifluormethoxy)phenyl]piperidin-3-carbonsäureethylester wurden 37 ml Dioxan, 18.5 ml Wasser und 0.51 g (21.4 mmol) Lithiumhydroxid gegeben. Die Mischung wurde über Nacht bei RT gerührt. Zur Aufarbeitung wurde im Vakuum Dioxan entfernt, das Reaktionsgemisch mit Wasser versetzt und mit wässriger 1 N Salzsäure-Lösung sauer gestellt. Die Mischung wurde mit Dichlormethan extrahiert. Die organische Phase wurde mit Natriumsulfat getrocknet und im Vakuum eingeengt. Ausbeute: 2.17 g (99% d. Th.)

Diastereomerentrennung von 2.17 g des cis-/trans-Isomerengemisches nach Methode 4C ergab 514 mg der Titelverbindung 24A (cis-Isomer) und 796 mg des trans-Isomers.

### Beispiel 24A

### 1-(Cyclopentylcarbonyl)-5-[4-(trifluormethoxy)phenyl]piperidin-3-carbonsäure [racemisches cis-Isomer]

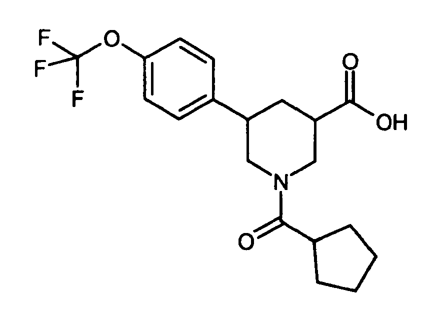

LC-MS (Methode 4B): Rₜ = 3.51 min; MS (ESIpos): m/z = 386 [M+H]⁺.

### Beispiel 25A

### 5-(4-Methoxyphenyl)pyridin-3-carbonsäureethylester

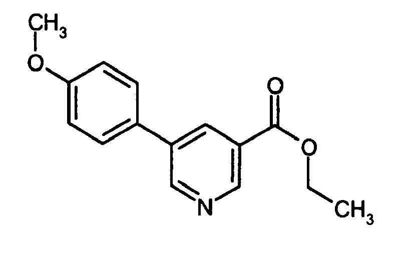

22.26 g (96.78 mmol) 5-Bromnicotinsäureethylester, 25.00 g (164.5 mmol) 4-Methoxyphenylboronsäure, 0.56 g (0.48 mmol) Tetrakis(triphenylphosphin)palladium(0) und 20.51 g (193.6 mmol) Natriumcarbonat wurden in einer Mischung von 180 ml 1,2-Dimethoxyethan, 50 ml Wasser und 400 ml Dimethylformamid gelöst und 18 h bei 85°C gerührt. Zur Aufarbeitung wurde ein Teil des Dimethylformamids im Vakuum entfernt, das Reaktionsgemisch mit Wasser verdünnt und mit Dichlormethan extrahiert. Die organische Phase wurde über Natriumsulfat getrocknet und das Rohprodukt mittels Flashchromatographie (Dichlormethan/Acetonitril 100:2→100:5) gereinigt. Ausbeute: 20.18 g (72% d. Th.)

LC-MS (Methode 4B): Rₜ = 3.21 min; MS (ESIpos): m/z = 258 [M+H]⁺.

### Beispiel 26A

### 5-(4-Methoxyphenyl)piperidin-3-carbonsäureethylester [racemisches cis-/trans-Isomerengemisch]

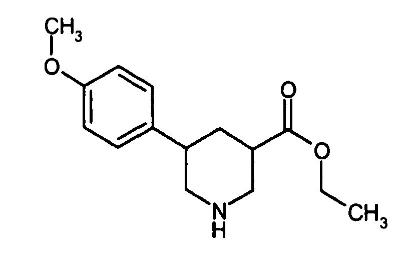

18.2 g (70.6 mmol) 5-[4-(Methoxy)phenyl]pyridin-3-carbonsäureethylester wurden gemäß der Allgemeinen Methode 2A hydriert. Ausbeute: 18.0 g (100% d. Th.)

LC-MS (Methode 3B): Rₜ = 0.82 min; MS (ESIpos): m/z = 264 [M+H]⁺.

### Beispiel 27A

### 5-(4-Methoxyphenyl)piperidin-1,3-dicarbonsäure-1-tert.-butyl-3-ethylester [racemisches cis-/trans-Isomerengemisch]

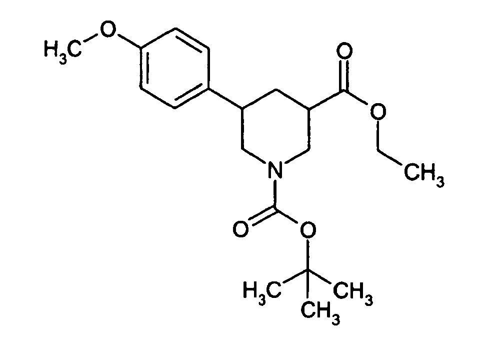

5.2g (17.8 mmol) 5-(4-Methoxyphenyl)piperidin-3-carbonsäureethylester wurden in 12 ml Dichlormethan gelöst und bei RT mit 3.88 g (17.8 mmol) Di-*tert.*-butyldicarbonat versetzt. Die Reaktionsmischung wurde für 1 h bei RT gerührt. Zur Aufarbeitung wurde zweimal mit Wasser gewaschen. Die organische Phase wurde über Natriumsulfat getrocknet und im Vakuum eingeengt. Ausbeute: 6.8 g (81 % d. Th.)

Diastereomerentrennung von 6.8 g des cis-/trans-Isomerengemisches nach Methode 5C ergab 1998 mg der Titelverbindung 28A (cis-Isomer) und 3375 mg des trans-Isomers.

### Beispiel 28A

### 5-(4-Methoxyphenyl)piperidin-1,3-dicarbonsäure-1-tert.-butyl-3-ethylester [racemisches cis-Isomer]

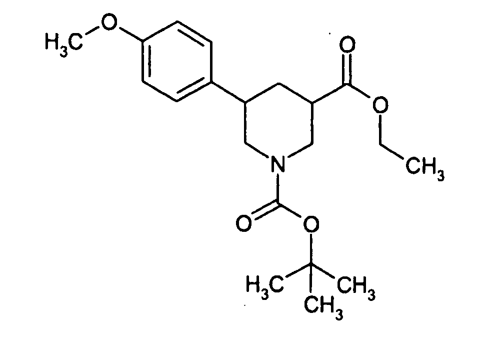

LC-MS (Methode 1B): Rₜ = 2.93 min; MS (ESIpos): m/z = 364 [M+H]⁺.

### Beispiel 29A

### 5-(4-Methoxyphenyl)piperidin-3-carbonsäure-Hydrochlorid [racemisches cis-Isomer]

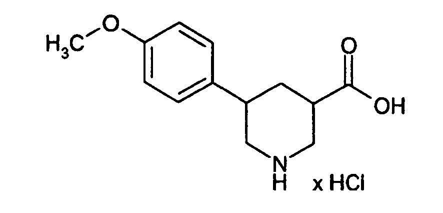

1.13 g (3.11 mmol) 5-(4-Methoxyphenyl)piperidin-1,3-dicarbonsäure-1-*tert.*-butyl-3-ethylester wurden in 40 ml wässriger 1 N Salzsäure-Lösung versetzt und am Roationsverdampfer eingeengt. Ausbeute: 829 mg (98% d. Th.)

LC-MS (Methode 8B): Rₜ = 2.08 min; MS (ESIpos): m/z = 236 [M+H]⁺.

### Beispiel 30A

### 1-(Cyclopentylcarbonyl)-5-(4-methoxyphenyl)piperidin-3-carbonsäure [racemisches cis-Isomer]

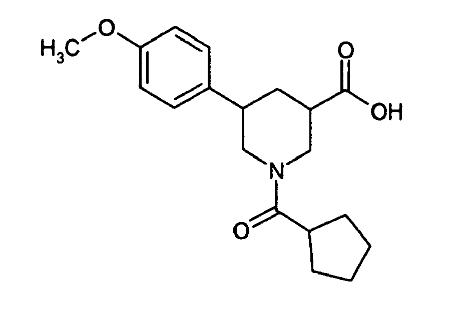

0.83 g (3.05 mmol) 5-(4-Methoxyphenyl)piperidin-3-carbonsäureethylester-Hydrochlorid wurden in 10 ml Dichlormethan gelöst und bei 0°C mit 0.77 g (7.62 mmol) Triethylamin versetzt. Anschließend wurden 0.83 g (6.09 mmol) Cyclopentancarbonylchlorid zugetropft. Die Reaktionsmischung wurde für 2 h bei RT gerührt. Zur Aufarbeitung wurde zweimal mit Wasser gewaschen. Die organische Phase wurde über Natriumsulfat getrocknet und im Vakuum eingeengt. Das Rohprodukt wurde mit 35 ml Dioxan, 17.5 ml Wasser und 0.29 mg (12.2 mmol) Lithiumhydroxid versetzt. Die Mischung wurde 1 h bei RT gerührt. Zur Aufarbeitung wurde im Vakuum Dioxan entfernt, das Reaktionsgemisch mit Wasser versetzt und mit wässriger 1 N Salzsäure-Lösung sauer gestellt. Der ausgefallene Feststoff wurde abfiltriert und im Vakuum getrocknet. Ausbeute: 0.84 g (75% d. Th.)

LC-MS (Methode 3B): Rₜ = 1.71 min; MS (ESIpos): m/z = 332 [M+H]⁺.

### Beispiel 31A

### 1-(2,2-Dimethylpropanoyl)-5-(4-methoxyphenyl)piperidin-3-carbonsäureethylester [racemisches cis-/trans-Isomerengemisch]

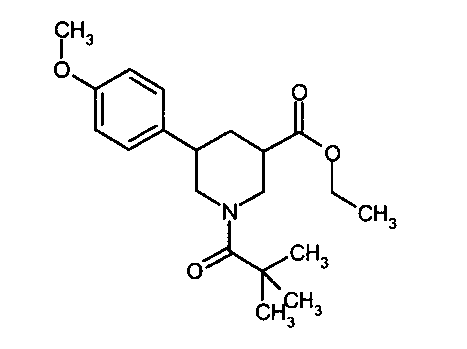

10.0 g (18.99 mmol) 1-(Cyclopentylcarbonyl)-5-[4-(methoxy)phenyl]piperidin-3-carbonsäureethylester wurden in 50 ml Dichlormethan gelöst und bei 0°C mit 2.88 g (28.48 mmol) Triethylamin versetzt. Anschließend wurden 3.00 g (24.68 mmol) Pivalinsäurechlorid zugetropft. Die Reaktionsmischung wurde für 2 h bei RT gerührt. Zur Aufarbeitung wurde zweimal mit Wasser gewaschen. Die organische Phase wurde über Natriumsulfat getrocknet und im Vakuum eingeengt.

Diastereomerentrennung von 10 g des Rohproduktes als cis-/trans-Isomerengemisch nach Methode 6C ergab 2.80 g der Titelverbindung 32A (cis-Isomer) und 4.61 g des trans-Isomers.

### Beispiel 32A

### 1-(2,2-Dimethylpropanoyl)-5-(4-methoxyphenyl)piperidin-3-carbonsäureethylester [racemisches cis-Isomer]

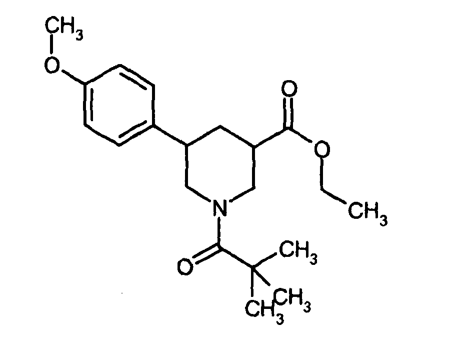

LC-MS (Methode 5B): Rₜ = 2.41 min; MS (ESIpos): m/z = 348 [M+H]⁺.

### Beispiel 33A

### 1-(2,2-Dimethylpropanoyl)-5-(4-methoxyphenyl)piperidin-3-carbonsäure [racemisches cis-Isomer]

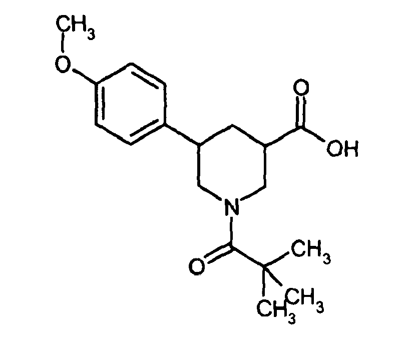

Zu 2.8 g (14.3 mmol) 1-(Cyclopentylcarbonyl)-5-[4-(methoxy)phenyl]piperidin-3-carbonsäureethylester wurden 55 ml Dioxan, 28 ml Wasser und 0.77 g (32.2 mmol) Lithiumhydroxid gegeben. Die Mischung wurde über Nacht bei RT gerührt. Zur Aufarbeitung wurde im Vakuum Dioxan entfernt, das Reaktionsgemisch mit Wasser versetzt und mit wässriger 1 N Salzsäure-Lösung sauer gestellt. Die Mischung wurde mit Dichlormethan extrahiert. Die organische Phase wurde mit Natriumsulfat getrocknet und im Vakuum eingeengt. Ausbeute: 2.44 g (95% d. Th.)

LC-MS (Methode 3B): Rₜ = 1.55 min; MS (ESIpos): m/z = 320 [M+H]⁺.

### Beispiel 34A

### 5-(4-Ethylphenyl)-1-(morpholin-4-ylcarbonyl)piperidin-3-carbonsäuremethylester [racemisches cis-/trans-Isomerengemisch]

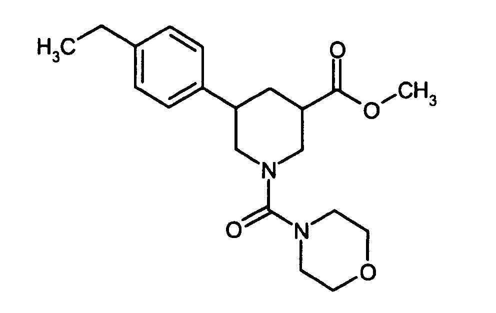

Nach der Allgemeinen Methode 3A wurden 6.2 g (22.6 mmol) 5-(4-Ethylphenyl)piperidin-3-carbonsäuremethylester mit 4.4 g (29.4 mmol, 1.3 eq.) Morpholin-4-carbonylchlorid umgesetzt. Ausbeute: 7.9 g (97% d. Th.)

LC-MS (Methode 5B): Rₜ = 2.14 min und 2.22 min (cis-/trans-Isomere); MS (ESIpos): m/z = 361 [M+H]⁺.

Diastereomerentrennung von 7.9 g des cis-/trans-Isomerengemisches nach Methode 7C ergab 2.8 g der Titelverbindung 35A (cis-Isomer) und 3.9 g des trans-Isomers.

### Beispiel 35A

### 5-(4-Ethylphenyl)-1-(morpholin-4-ylcarbonyl)piperidin-3-carbonsäuremethylester [racemisches cis-Isomer]

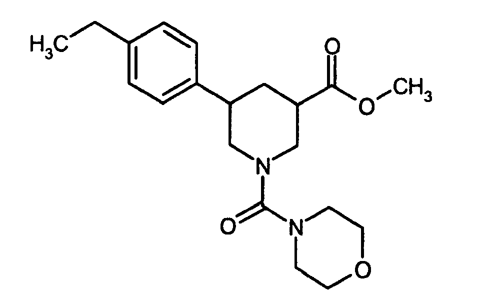

HPLC (Methode 4A): Rₜ = 9.61 min; MS (ESIpos): m/z = 361 [M+H]⁺.

### Beispiel 36A

### 5-(4-Ethylphenyl)-1-(morpholin-4-ylcarbonyl)piperidin-3-carbonsäureethylester [racemisches cis-/ trans-Isomerengemisch]

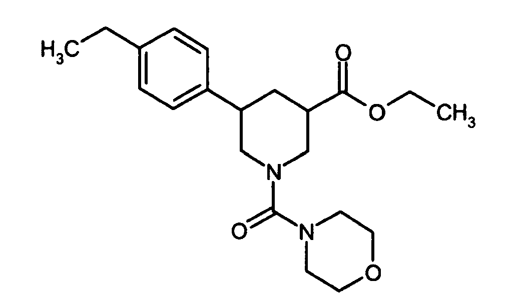

Nach der Allgemeinen Methode 3A wurden 10.0 g (36.0 mmol) 5-(4-Ethylphenyl)piperidin-3-carbonsäureethylester mit 7.0 g (46.8 mmol, 1.3 eq.) Morpholin-4-carbonylchlorid umgesetzt. Ausbeute: 12.0 g (89% d. Th.)

LC-MS (Methode 1B): Rₜ = 2.38 min und 2.48 min (cis-/trans-Isomere); MS (ESIpos): m/z = 375 [M+H]⁺.

Diastereomerentrennung von 12.0 g des cis-/trans-Isomerengemisches nach Methode 8C ergab 4.4 g der Titelverbindung aus Beispiel 37A (cis-Isomer) und 5.4 g des trans-Isomers.

### Beispiel 37A

### 5-(4-Ethylphenyl)-1-(morpholin-4-ylcarbonyl)piperidin-3-carbonsäureethylester [racemisches cis-Isomer]

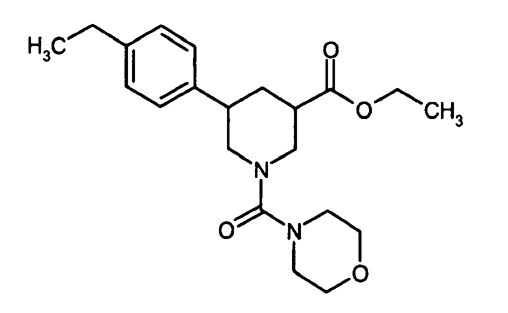

Diastereomerentrennung von 12.0 g des cis-/trans-Isomerengemisches aus Beispiel 36A nach Methode 8C ergab 4.4 g der Titelverbindung (cis-Isomer) und 5.4 g des trans-Isomers.

LC-MS (Methode 1B): Rₜ = 2.48 min; MS (ESIpos): m/z = 375 [M+H]⁺.

### Beispiel 38A

### 5-(4-Ethylphenyl)-1-(morpholin-4-ylcarbonyl)piperidin-3-carbonsäure [racemisches cis-Isomer]

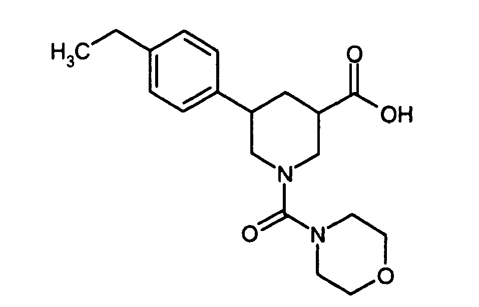

Nach der Allgemeinen Methode 4A wurden 4.4 g (11.7 mmol) 5-(4-Ethylphenyl)-1-(morpholin-4-ylcarbonyl)piperidin-3-carbonsäureethylester (Beispiel 37A) verseift. Ausbeute: 3.4 g (84% d. Th.)

LC-MS (Methode 1B): Rₜ = 2.06 min; MS (ESIpos): m/z = 347 [M+H]⁺.

### Beispiel 39A

### 5-(4-Ethylphenyl)-1-(pyrrolidin-1-ylcarbonyl)piperidin-3-carbonsäuremethylester [racemisches cis-/trans-Isomerengemisch]

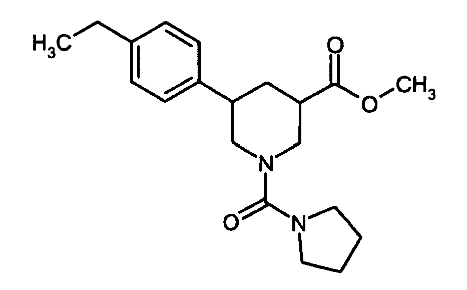

Nach der Allgemeinen Methode 3A wurden 6.7 g (24.1 mmol) 5-(4-Ethylphenyl)piperidin-3-carbonsäuremethylester mit 4.2 g (31.4 mmol, 1.3 eq.) Pyrrolidin-1-carbonylchlorid umgesetzt. Ausbeute: 7.6 g (91% d. Th.)

LC-MS (Methode 3B): Rₜ = 2.08 min und 2.16 min (cis-/trans-Isomere); MS (ESIpos): m/z = 345 [M+H]⁺.

Diastereomerentrennung von 7.6 g des cis-/trans-Isomerengemisches nach Methode 9C ergab 1.6 g der Titelverbindung 40A (cis-Isomer) und 4.1 g des trans-Isomers.

### Beispiel 40A

### 5-(4-Ethylphenyl)-1-(pyrrolidin-1-ylcarbonyl)piperidin-3-carbonsäuremethylester [racemisches cis-Isomer]

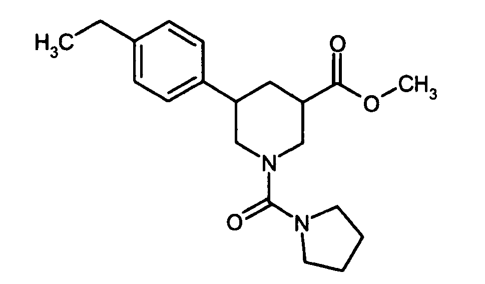

LC-MS (Methode 1B): Rₜ = 2.55 min; MS (ESIpos): m/z = 345 [M+H]⁺.

### Beispiel 41A

### 5-(4-Ethylphenyl)-1-(pyrrolidin-1-ylcarbonyl)piperidin-3-carbonsäure [racemisches cis-Isomer]

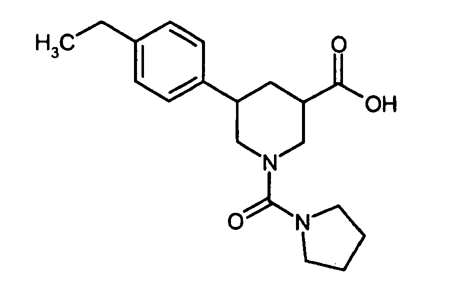

Nach der Allgemeinen Methode 4A wurden 1.4 g (3.9 mmol) 5-(4-Ethylphenyl)-1-(pyrrolidin-1-ylcarbonyl)piperidin-3-carbonsäuremethylester verseift. Ausbeute: 1.2 g (92% d. Th.)

LC-MS (Methode 2B): Rₜ = 1.18 min; MS (ESIpos): m/z = 331 [M+H]⁺.

### Beispiel 42A

### 1-(Morpholin-4-ylcarbonyl)-5-[4-(trifluormethoxy)phenyl]piperidin-3-carbonsäuremethylester [racemisches cis-/trans-Isomerengemisch]

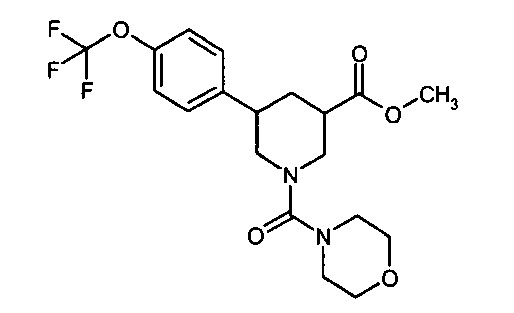

Nach der Allgemeinen Methode 3A wurden 8.3 g (26.5 mmol) 5-[4-(Trifluormethoxy)-phenyl]piperidin-3-carbonsäuremethylester mit 5.2 g (34.5 mmol, 1.3 eq.) Morpholin-4-carbonylchlorid umgesetzt. Ausbeute: 11.0 g (100% d. Th.)

LC-MS (Methode 2B): Rₜ = 1.21 min und 1.24 min (cis-/trans-Isomere); MS (ESIpos): m/z = 417 [M+H]⁺.

Diastereomerentrennung von 11.0 g des cis-/trans-Isomerengemisches nach Methode 10C ergab 4.3 g der Titelverbindung aus Beispiel 43A (cis-Isomer) und 5.0 g des trans-Isomers.

### Beispiel 43A

### 1-(Morpholin-4-ylcarbonyl)-5-[4-(trifluormethoxy)phenyl]piperidin-3-carbonsäuremethylester [racemisches cis-Isomer]

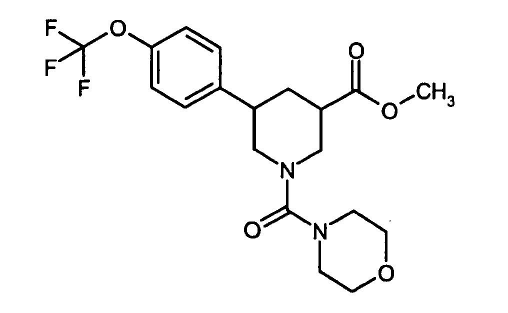

Diastereomerentrennung von 11.0 g des cis-/trans-Isomerengemisches aus Beispiel 42A nach Methode 10C ergab 4.3 g der Titelverbindung (cis-Isomer) und 5.0 g des trans-Isomers.

LC-MS (Methode 3B): Rₜ = 1.98 min; MS (ESIpos): m/z = 417 [M+H]⁺.

### Beispiel 44A

### 1-(Morpholin-4-ylcarbonyl)-5-[4-(trifluormethoxy)phenyl]piperidin-3-carbonsäure [racemisches cis-Isomer]

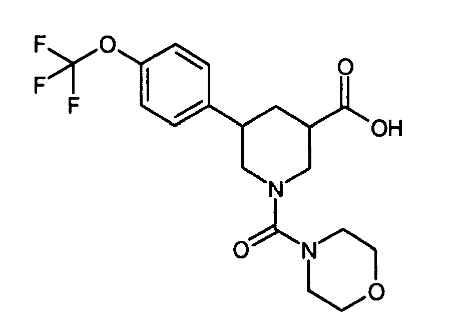

Nach der Allgemeinen Methode 4A wurden 4.3 g (10.4 mmol) 1-(Morpholin-4-ylcarbonyl)-5-[4-(trifluormethoxy)phenyl]piperidin-3-carbonsäuremethylester (Beispiel 43A) verseift. Ausbeute: 4.1 g (98% d. Th.)

LC-MS (Methode 1B): Rₜ = 2.12 min; MS (ESIpos): m/z = 403 [M+H]⁺.

### Beispiel 45A

### 1-[Ethyl(methyl)carbamoyl]-5-(4-ethylphenyl)piperidin-3-carbonsäure [racemisches cis-Isomer]

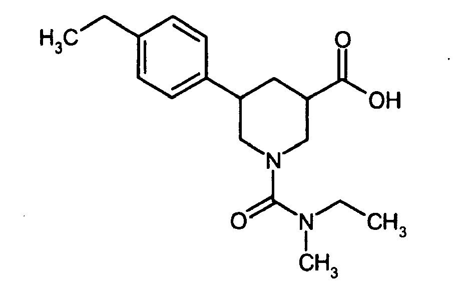

### Stufe a): 1-[Ethyl(methyl)carbamoyl]-5-(4-ethylphenyl)piperidin-3-carbonsäuremethylester [racemisches cis-/trans-Isomerengemisch]

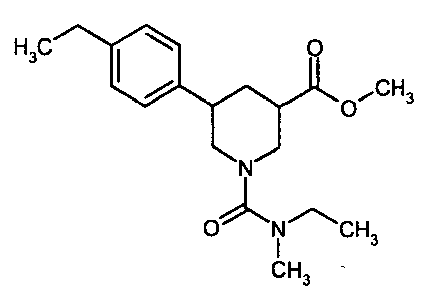

Nach der Allgemeinen Methode 3A wurden 7.0 g (89%ig, 25.1 mmol) 5-(4-Ethylphenyl)piperidin-3-carbonsäuremethylester mit 4.0 g (32.6 mmol, 1.3 eq.) Ethyl(methyl)carbamoylchlorid umgesetzt. Ausbeute: 8.0 g (96% d. Th.)

LC-MS (Methode 3B): Rₜ = 2.10 min und 2.18 min (cis-/trans-Isomere); MS (ESIpos): m/z = 333 [M+H]⁺.

### Stufe b). 1-[Ethyl(methyl)carbamoyl]-5-(4-ethylphenyl)piperidin-3-carbonsäure [racemisches cis-/trans-Isomerengemisch]

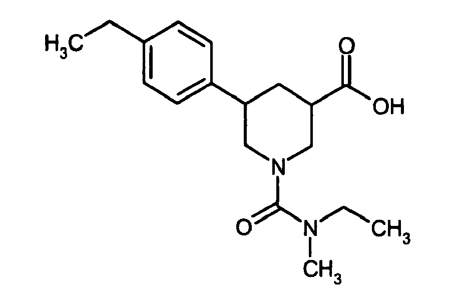

Nach der Allgemeinen Methode 4A wurden 8.0 g (23.2 mmol) 1-[Ethyl(methyl)carbamoyl]-5-(4-ethylphenyl)piperidin-3-carbonsäuremethylester verseift. Ausbeute: 1.2 g (16% d. Th.)

LC-MS (Methode 3B): Rₜ = 1.79 min und 1.84 min (cis-/trans-Isomere); MS (ESIpos): m/z = 319 [M+H]⁺.

Diastereomerentrennung von 1.2 g des cis-/trans-Isomerengemisches nach Methode 9C ergab 538 mg der Titelverbindung 45A (cis-Isomer).

LC-MS (Methode 3B): Rₜ = 1.79 min; MS (ESIpos): m/z = 319 [M+H]⁺.

### Beispiel 46A

### 5-[4-(Trifluormethyl)phenyl]pyridin-3-carbonsäuremethylester

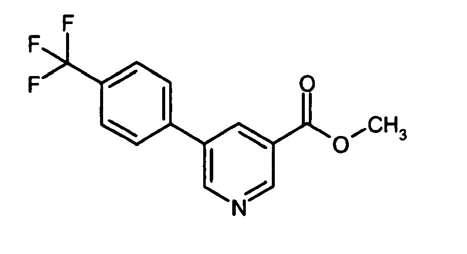

Nach der Allgemeinen Methode 1A wurden 28 g (132 mmol) 5-Bromnicotinsäuremethylester und 30 g (158 mmol, 1.2 eq.) 4-Ethylphenylboronsäure umgesetzt. Ausbeute: 32 g (85% d. Th.)

LC-MS (Methode 5B): Rₜ = 2.27 min; MS (ESIpos): m/z = 282 [M+H]⁺.

### Beispiel 47A

### 5-[4-(Trifluormethyl)phenyl]piperidin-3-carbonsäuremethylester [racemisches cis-/trans-Isomerengemisch]

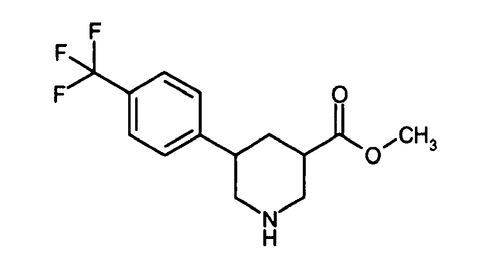

Nach der Allgemeinen Methode 2A wurden 32 g (112 mmol) 5-[4-(Trifluormethyl)phenyl]-pyridin-3-carbonsäuremethylester (Beispiel 46A) hydriert. Ausbeute: 26 g (82% d. Th.)

LC-MS (Methode 1B): Rₜ = 1.35 und 1.41 min (cis-/trans-Isomere); MS (ESIpos): m/z = 288 [M+H]⁺.

### Beispiel 48A

### 1-(Morpholin-4-ylcarbonyl)-5-[4-(trifluormethyl)phenyl]piperidin-3-carbonsäuremethylester [racemisches cis-/trans-Isomerengemisch]

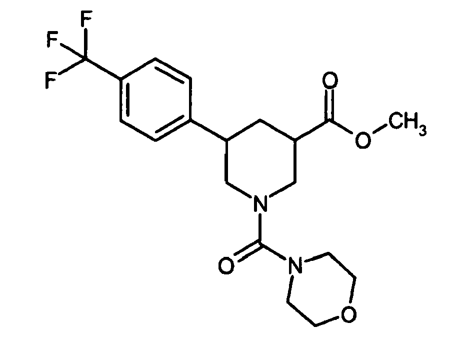

Nach der Allgemeinen Methode 3A wurden 9.25 g (32.2 mmol) 5-[4-(Trifluormethyl)phenyl]-piperidin-3-carbonsäuremethylester mit 9.63 g (64.7 mmol) Morpholin-4-carbonylchlorid umgesetzt. Man erhielt so 16.3 g Rohprodukt in 76%-iger Reinheit (LC-MS), welches ohne weitere Reinigungsoperationen umgesetzt wurde.

LC-MS (Methode 2B): Rₜ = 1.19 und 1.22 min (cis-/trans-Isomere); MS (ESIpos): m/z = 401 [M+H]⁺.

### Beispiel 49A

### 1-(Morpholin-4-ylcarbonyl)-5-[4-(trifluormethyl)phenyl]piperidin-3-carbonsäure [racemisches cis-Isomer]

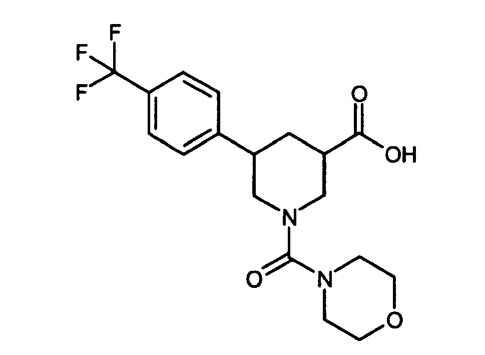

Nach der Allgemeinen Methode 4A wurden 12.9 g (25.7 mmol) 1-(Morpholin-4-ylcarbonyl)-5-[4-(trifluormethyl)phenyl]piperidin-3-carbonsäuremethylester verseift. Man erhielt so 12.1 g Rohprodukt in 80%-iger Reinheit (LC-MS), welches ohne weitere Reinigungsoperationen umgesetzt wurde.

LC-MS (Methode 2B): Rₜ = 1.08 min; MS (ESIpos): m/z = 387 [M+H]⁺.

### Beispiel 50A

### 5-[4-(Trifluormethyl)phenyl]pyridin-3-carboxamid

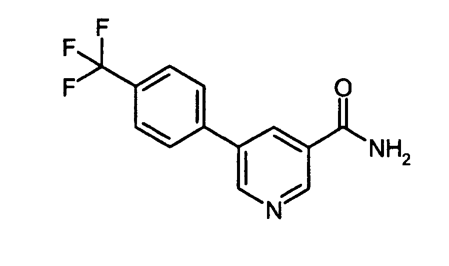

2.0 g (9.75 mmol) 5-[4-(Trifluormethyl)phenyl]pyridin-3-carboxamid und 2.78 g (14.6 mmol) 4-(Trifluormethyl)-phenylboronsäure wurden in 25 ml 1,2-Dimethoxyethan und 9.8 ml Wasser unter Argon vorgelegt. Dann wurden 2.07 g (19.5 mmol) Natriumcarbonat hinzugegeben und 15 Minuten bei Raumtemperatur gerührt. Es wurden 0.28 g (0.24 mmol) Tetrakis-(triphenylphosphin)-palladium addiert. Das Reaktionsgemisch wurde über Nacht bei 120°C gerührt. Zur Aufarbeitung wurde das Reaktionsgemisch über Celite filtriert und ein Teil des 1,2-Dimethoxyethans im Vakuum entfernt. Das Reaktionsgemisch wurde mit Wasser verdünnt und mit Ethylacetat und Dichlormethan extrahiert. Die organische Phase wurde mit Magnesiumsulfat getrocknet und das Lösungsmittel unter vermindertem Druck destillativ abgetrennt. Man erhielt 2.1 g (81% d. Th.) Rohprodukt, welches ohne weitere Reinigungsoperationen umgesetzt wurde.

LC-MS (Methode 1B): Rₜ = 1.88 min; MS (ESIpos): m/z = 267 [M+H]⁺;

¹H-NMR (400 MHz, DMSO-d₆): δ = 9.08 (dd, 2H), 8.55 (t, 1H), 8.28 (bs, 1H), 8.05 (d, 2H), 7.90 (d, 2H), 7.71 (bs, 1H).

### Beispiel 51A

### 5-[4-(Trifluormethyl)phenyl]piperidin-3-carboxamid-Acetat [racemisches cis-/ trans-Isomerengemisch]

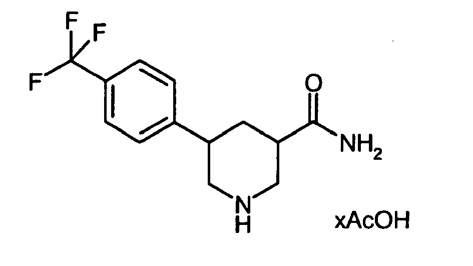

Eine Lösung von 500 mg (1.88 mmol) 5-[4-(Trifluormethyl)phenyl]pyridin-3-carboxamid (Beispiel 50A) in 60 ml Essigsäure wurde mit Palladium auf Aktivkohle (angefeuchtet mit ca. 50% Wasser, 0.3 g/mmol) versetzt und bei 85°C über Nacht in einer 60 bar Wasserstoff-Atmosphäre hydriert. Anschließend wurde der Katalysator über eine Filterschicht abfiltriert und mehrmals mit Ethanol gewaschen. Die vereinigten Filtrate wurden im Vakuum eingeengt. Man erhielt so 0.61 g (98% d. Th.) der Zielverbindung, welche ohne weitere Reinigungsoperationen umgesetzt wurde.

LC-MS (Methode 5B): Rₜ = 1.25 min; MS (ESIpos): m/z = 273 [M+H-AcOH]⁺.

### Beispiel 52A

### 1-(Morpholin-4-ylcarbonyl)-5-[4-(trifluormethyl)phenyl]piperidin-3-carboxamid [racemisches cis-/ trans-Isomerengemisch]

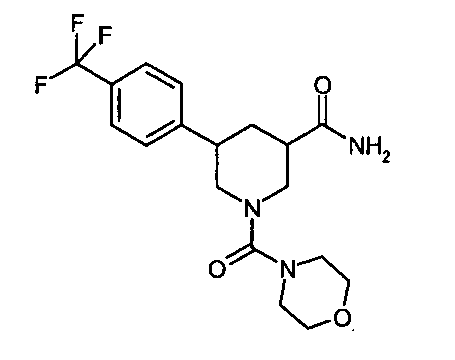

Eine Lösung von 0.61 mg (1.85 mmol) 5-[4-(Trifluormethyl)phenyl]piperidin-3-carboxamid-Acetat (Beispiel 51A) in 10 ml Dichlormethan wurde bei 0°C mit 0.56 g (5.52 mmol) Triethylamin und 0.55 g (3.70 mmol) Morpholin-4-carbonylchlorid versetzt. Das Reaktionsgemisch wurde 1 h bei RT gerührt. Nach Zugabe von Wasser und Phasentrennung wurde die organische Phase über Natriumsulfat getrocknet, filtriert und im Vakuum eingeengt. Man erhielt so 0.61 g (100% d. Th.) Rohprodukt in 77%-iger Reinheit (LC-MS), welches ohne weitere Reinigungsoperationen umgesetzt wurde.

LC-MS (Methode 1B): Rₜ = 1.89 min; MS (ESIpos): m/z = 386 [M+H]⁺.

### Beispiel 53A

### 1-(Morpholin-4-ylcarbonyl)-5-[4-(trifluormethyl)phenyl]piperidin-3-carbothioamid [racemisches cis-/ trans-Isomerengemisch]

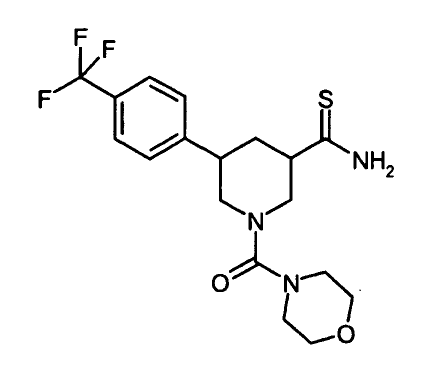

Zu einer Lösung von 10 mg (0.20 mmol) 1-(Morpholin-4-ylearbonyl)-5-[4-(trifluormethyl)-phenyl]piperidin-3-carboxamid (Beispiel 52A) in 1 ml Dioxan wurden 45 mg (0.11 mmol) Lawesson-Reagens (2,4-Bis[4-methoxyphenyl]1,3-dithia-2,4-diphosphetan-2,4-disulfid) hinzugegeben, und das Reaktionsgemisch wurde 30 Minuten bei 60°C und dann 3 h bei Raumtemperatur gerührt. Nach Zugabe von gesättigter wässriger Natriumhydrogencarbonat-Lösung und Entfernung des Dioxans wurde der Rückstand mit Ethylacetat extrahiert. Die organische Phase wurde mit einer gesättigten wässrigen Natriumhydrogencarbonat-Lösung gewaschen, über Magnesiumsulfat getrocknet, filtriert und im Vakuum eingeengt. Man erhielt so 43 mg (100% d. Th.) Rohprodukt, welches ohne weitere Reinigungsoperationen umgesetzt wurde.

LC-MS (Methode 5B): Rₜ = 2.08 min; MS (ESIpos): m/z = 402 [M+H]⁺.

### Beispiel 54A

### 5-(4-Ethylphenyl)piperidin-3-carbonsäureethylester [racemisch cis-Isomer]

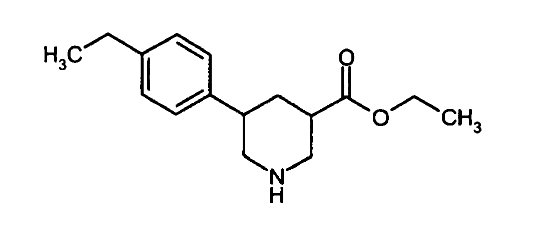

Nach chromatographischer Trennung der Diastereomere aus Beispiel 4A nach Methode 11C wurden aus 15 g (124 mmol) 5-(4-Ethylphenyl)piperidin-3-carbonsäureethylester die beiden Isomere erhalten. Ausbeute: 2.5 g des cis-Isomers (17% d. Th.)

LC-MS (Methode 3B): Rₜ = 1.02 min; MS (ESIpos): m/z = 262 [M+H]⁺.

### Beispiel 55A

### 5-(4-Ethylphenyl)piperidin-3-carbonsäureethylester [racemisches trans-Isomer]

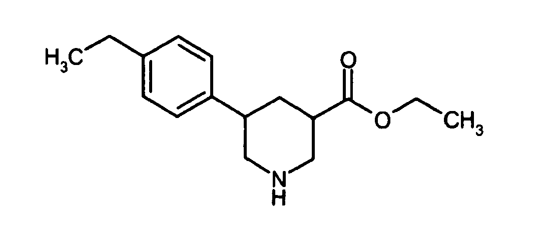

Nach chromatographischer Trennung der Diastereomere aus Beispiel 4A nach Methode 11C wurden aus 15 g (124 mmol) 5-(4-Ethylphenyl)piperidin-3-carbonsäureethylester die beiden Isomere erhalten. Ausbeute: 3.0 g des trans-Isomers (20% d. Th.)

LC-MS (Methode 3B): Rₜ = 1.09 min; MS (ESIpos): m/z = 262 [M+H]⁺.

### Beispiel 56A

### 3-Ethyl-1-(4-nitrophenyl)-5-(4-ethylphenyl)piperidin-1,3-dicarboxylat [racemisch cis-Isomer]

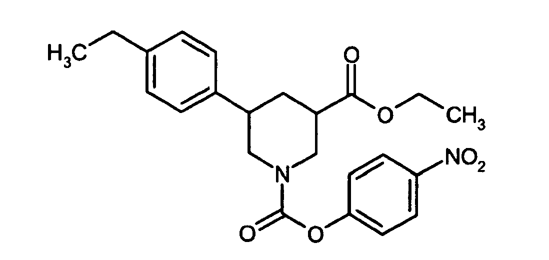

Zu 2.5 g (9.57 mmol) 5-(4-Ethylphenyl)piperidin-3-carbonsäureethylester (Beispiel 54A) und 1.94 g (19.1 mmol) Triethylamin in 292 ml Dichlormethan wurden langsam bei 0°C 1.93 g (9.57 mmol) 4-Nitrophenylchloroformat gegeben. Die Mischung wurde für 2 h bei RT gerührt. Zur Aufarbeitung wurde das Reaktionsgemisch erst mit gesättigter Natriumhydrogencarbonatlösung, dann mit Wasser gewaschen. Die organische Phase wurde mit Natriumsulfat getrocknet und im Vakuum eingeengt. Der Rückstand wurde mittels präparativer HPLC gereinigt. Ausbeute: 2.66 g (64% d. Th.)

LC-MS (Methode 2B): Rₜ = 1.57 min; MS (ESIpos): m/z = 427 [M+H]⁺.

### Beispiel 57A

### Ethyl-5-(4-ethylphenyl)-1-[(4-hydroxypiperidin-1-yl)carbonyl]piperidin-3-carboxylat [racemisches cis-Isomer]

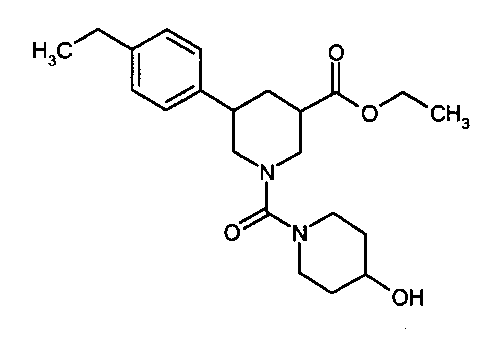

370 mg (0.81 mmol) 3-Ethyl-1-(4-nitrophenyl)-5-(4-ethylphenyl)piperidin-1,3-dicarboxylat, 245 mg (2.42 mmol) 4-Hydroxypiperidin und 112 mg (0.81 mmol) Kaliumcarbonat wurden in 9 ml DMF gegeben und bei 150°C für 15 min in einer *Single Mode*-Mikrowelle (Emrys Optimizer) erhitzt. Zur Aufarbeitung wurde die Reaktionslösung mit Wasser versetzt und mit Essigsäureethylester extrahiert. Die organische Phase wurde mit Natriumsulfat getrocknet und im Vakuum eingeengt. Der Rückstand wurde mittels präparativer HPLC gereinigt. Ausbeute: 208 mg (66% d. Th.)

LC-MS (Methode 2B): Rₜ = 1.23 min; MS (ESIpos): m/z = 389 [M+H]⁺.

### Beispiel 58A

### Ethyl-5-(4-ethylphenyl)-1-[(4-cyanopiperidin-1-yl)carbonyl]piperidin-3-carboxylat [racemisches cis-Isomer]

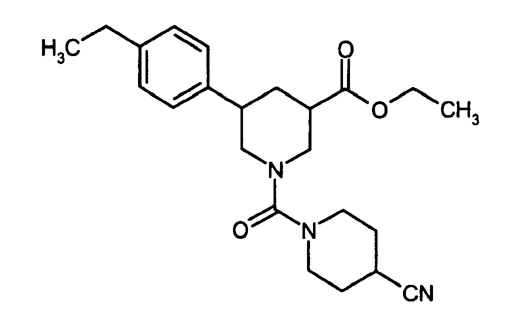

370 mg (0.81 mmol) 3-Ethyl-1-(4-nitrophenyl)-5-(4-ethylphenyl)piperidin-1,3-dicarboxylat, 267 mg (2.42 mmol) 4-Cyanopiperidin und 112 mg (0.81 mmol) Kaliumcarbonat wurden in 9 ml DMF gegeben und bei 150°C für 15 min in einer *Single Mode*-Mikrowelle (Emrys Optimizer) erhitzt. Zur Aufarbeitung wurde die Reaktionslösung mit Wasser versetzt und mit Essigsäureethylester extrahiert. Die organische Phase wurde mit Natriumsulfat getrocknet und im Vakuum eingeengt. Der Rückstand wurde mittels präparativer HPLC gereinigt. Ausbeute: 73 mg (23% d. Th.)

LC-MS (Methode 2B): Rₜ = 1.36 min; MS (ESIpos): m/z = 398 [M+H]⁺.

### Beispiel 59A

### 5-(4-Ethylphenyl)-1-[(4-hydroxypiperidin-1-yl)carbonyl]piperidin-3-carbonsäure [racemisches cis-Isomer]

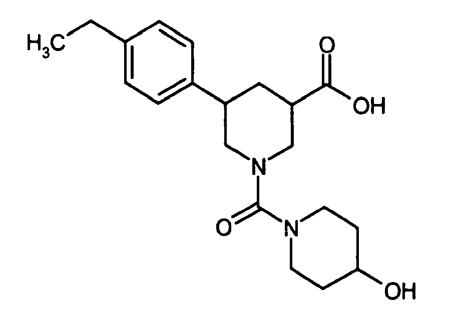

880 mg (2.24 mmol) Ethyl-5-(4-ethylphenyl)-1-[(4-hydroxypiperidin-1-yl)carbonyl]piperidin-3-carboxylat wurden in einem Gemisch von 15.5 ml Dioxan und 7.7 ml Wasser gelöst und mit 215 mg (8.97 mmol) Lithiumhydroxid versetzt und bei RT über Nacht gerührt. Zur Aufarbeitung wurde die Reaktionslösung im Vakuum eingeengt, danach mit Wasser versetzt und mit 1N Salzsäure sauer gestellt. Der entstandene Niederschlag wurde abfiltriert, gewaschen und im Vakuum getrocknet. Das Filtrat wurde mit Essigsäureethylester extrahiert. Die vereinten organischen Phasen wurden mit Natriumsulfat getrocknet und im Vakuum eingeengt. Die beiden Feststoffe ergaben eine Gesamtausbeute von 764 mg (95% d. Th.).

LC-MS (Methode 3B): Rₜ = 1.49 min; MS (ESIpos): m/z = 361 [M+H]⁺.

### Beispiel 60A

### 5-(4-Ethylphenyl)-1-[(4-cyanopiperidin-1-yl)carbonyl]piperidin-3-carbonsäure [racemisches cis-Isomer]

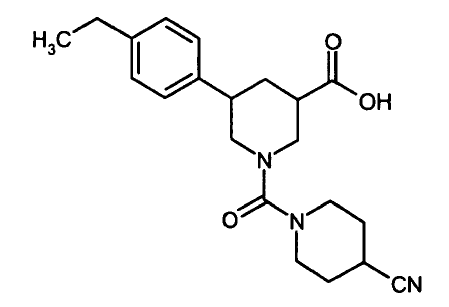

650 mg (1.60 mmol) Ethyl-5-(4-ethylphenyl)-1-[(4-cyanopiperidin-1-yl)carbonyl]piperidin-3-carboxylat wurden in einem Gemisch von 20 ml Dioxan und 10 ml Wasser gelöst und mit 153 mg (6.40 mmol) Lithiumhydroxid versetzt und bei RT für 15 min gerührt. Zur Aufarbeitung wurde die Reaktionslösung im Vakuum eingeengt, danach mit Wasser versetzt und mit 1N Salzsäure sauer gestellt. Der entstandene Niederschlag wurde abfiltriert, gewaschen und im Vakuum getrocknet. Der Rückstand wurde mittels präparativer HPLC gereinigt. Ausbeute: 347 mg (59% d. Th.)

LC-MS (Methode 3B): Rₜ = 1.13 min; MS (ESIpos): m/z = 370 [M+H]⁺.

### Beispiel 61A

### 3-Methyl-1-(4-nitrophenyl)-5-[4-(trifluormethoxy)phenyl]piperidin-1,3-dicarboxylat [racemisches cis-/trans-Isomerengemisch]

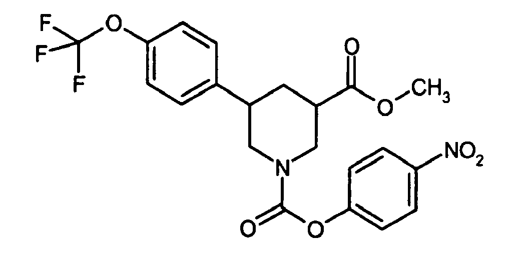

Zu 8.0 g (26.4 mmol) 5-(4-(Trifluormethoxy)phenyl)piperidin-3-carbonsäuremethylester (Beispiel 19A) und 5.34 g (26.3 mmol) Triethylamin in 666 ml Dichlormethan wurden langsam bei 0°C 5.32 g (26.4 mmol) 4-Nitrophenylchloroformat gegeben. Die Mischung wurde für 2 h bei RT gerührt. Zur Aufarbeitung wurde das Reaktionsgemisch erst mit gesättigter Natriumhydrogencarbonatlösung, dann mit Wasser gewaschen. Die organische Phase wurde mit Natriumsulfat getrocknet und im Vakuum eingeengt. Der Rückstand wurde mittels Flashchromatographie (Cyclohexan/Essigsäureethylester 1:2 bis 1:1) gereinigt. Ausbeute: 7.32 g (54% d. Th.)

LC-MS (Methode 3B): Rₜ = 2.47 min; MS (ESIpos): m/z = 469 [M+H]⁺.

### Beispiel 62A

### Methyl-1-[(4-hydroxypiperidin-1-yl)carbonyl]-5-[4-(trifluormethoxy)phenyl]piperidin-3-carboxylat [racemisches cis-/trans-Isomerengemisch]

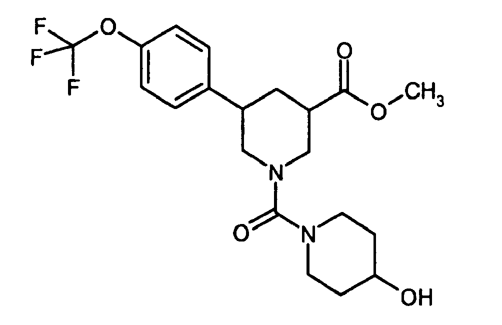

1780 mg (3.80 mmol) 3-Methyl-1-(4-nitrophenyl)-5-[4-(trifluormethoxy)phenyl]piperidin-1,3-dicarboxylat, 1153 mg (11.40 mmol) 4-Hydroxypiperidin und 525 mg (3.80 mmol) Kaliumcarbonat wurden in 37 ml DMF gegeben und in 2 Portionen bei 150°C für 15 min in einer *Single Mode*-Mikrowelle (Emrys Optimizer) erhitzt. Zur Aufarbeitung wurden die beiden Reaktionslösungen vereint, mit Wasser versetzt und mit Essigsäureethylester extrahiert. Die organische Phase wurde mit Natriumsulfat getrocknet und im Vakuum eingeengt. Der Rückstand wurde mittels präparativer HPLC gereinigt. Ausbeute: 849 mg (50% d. Th.)

LC-MS (Methode 2B): Rₜ = 1.23 min; MS (ESIpos): m/z = 431 [M+H]⁺.

### Beispiel 63A

### 1-[(4-Hydroxypiperidin-1-yl)carbonyl]-5-[4-(trifluormethoxy)phenyl]piperidin-3-carbonsäure [racemisches cis-Isomer]

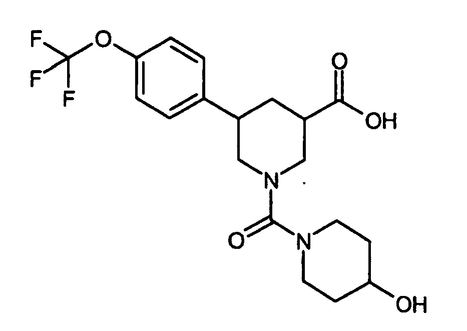

828 mg (1.92 mmol) Methyl-5-(4-(trifluormethoxy)phenyl)-1-[(4-hydroxypiperidin-1-yl)carbonyl]-piperidin-3-carboxylat wurden in 70 ml Methanol gelöst, mit 2159 mg (19.24 mmol) Kalium-*tert.-*butylat versetzt und bei 60°C über Nacht gerührt. Zur Aufarbeitung wurde die Reaktionslösung mit Wasser verdünnt und mit 1N Salzsäure sauer gestellt (pH 1). Das Gemisch wurde mit Essigsäureethylester extrahiert. Die vereinten organischen Phasen wurden mit Natriumsulfat getrocknet und im Vakuum eingeengt. Ausbeute: 749 mg (94% d. Th.)

LC-MS (Methode 2B): Rₜ = 1.04 min; MS (ESIpos): m/z = 417 [M+H]⁺.

### Beispiel 64A

### N'-Hydroxy-3-methoxypropanimidamid

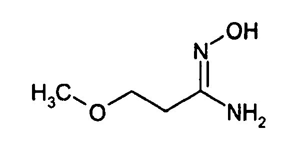

Nach der Allgemeinen Methode 6A wurden 20.0 g (235.0 mmol) 3-Methoxypropionitril umgesetzt. Ausbeute: 18.1 g (49% d. Th., 74% Reinheit)

HPLC (Methode 1A): Rₜ = 0.35 min; MS (ESIpos): m/z = 119 [M+H]⁺.

### Beispiel 65A

### N'-Hydroxy-3-(propan-2-yloxy)propanimidamid

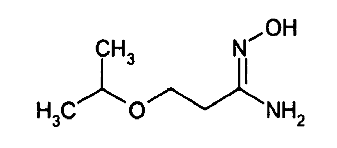

Nach der Allgemeinen Methode 5A wurden 5.0 g (44.2 mmol) 3-(Propan-2-yloxy)propanonitril umgesetzt. Ausbeute: 3.0 g (40% d. Th., 86% Reinheit)

HPLC (Methode 1A): Rₜ = 1.24 min; MS (ESIpos): m/z = 147 [M+H]⁺.

### Beispiel 66A

### N'-3-Dihydroxy-3-methylbutanimidamid

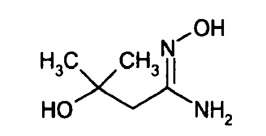

Nach der Allgemeinen Methode 6A wurden 5.0 g (50.4 mmol) 3-Hydroxy-3-methylbutyronitril umgesetzt. Ausbeute: 4.8 g (52% d. Th., 72% Reinheit)

HPLC (Methode 1A): Rₜ = 0.28 min; MS (ESIpos): m/z = 133 [M+H]⁺.

### Beispiel 67A

### N'-Hydroxy-2-[1-(hydroxymethyl)cyclopropyl]ethanimidamid

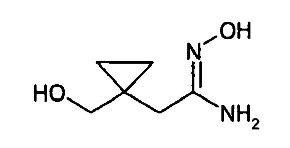

Nach der Allgemeinen Methode 5A wurden 1.1 g (9.4 mmol) [1-(Hydroxymethyl)-cyclopropyl]acetonitril umgesetzt. Ausbeute: 0.6 g (38% d. Th., 87% Reinheit)

HPLC (Methode 1A): Rₜ = 0.43 min; MS (ESIpos): m/z = 145 [M+H]⁺.

### Beispiel 68A

### 3,4-Difluor-N'-hydroxybenzolcarboximidamid

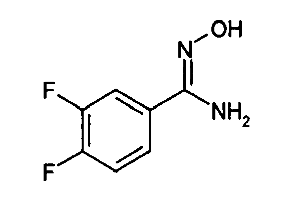

1.0 g (7.2 mmol) 3,4-Difluorbenzonitril wurden in 30 ml Ethanol vorgelegt und bei RT mit einer Lösung von 0.5 g (7.2 mmol) Hydroxylammoniumchlorid und 0.6 g (7.2 mmol) Natriumhydrogencarbonat in 11 ml Wasser versetzt. Das Reaktionsgemisch wurde 16 h bei RT gerührt, im Vakuum eingeengt und ohne weitere Aufreinigung umgesetzt.

HPLC (Methode 1A): Rₜ = 1.36 min; MS (ESIpos): m/z = 173 [M+H]⁺.

### Beispiel 69A

### N'-Hydroxypyridin-2-carboximidamid

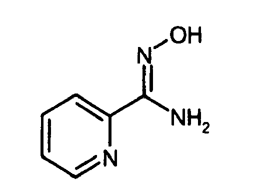

Nach der Allgemeinen Methode 5A wurden 1.0 g (9.6 mmol) Pyridin-2-carbonitril, 1.0 g (14.4 mmol) Hydroxylammoniumchlorid und 1.6 ml (11.5 mmol) Triethylamin umgesetzt. Ausbeute: 1.06 g (80% d. Th.)

¹H-NMR (400 MHz, DMSO-d₆): δ = 9.90 (s, 1H), 8.55 (d, 1H), 7.86-7.78 (m, 2H), 7.40 (ddd, 1H), 5.83 (s, 2H).

### Beispiel 70A

### N'-Hydroxy-4-methylpyridin-3-carboximidamid

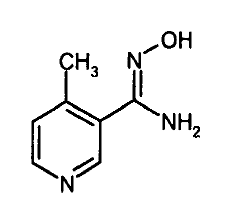

Nach der Allgemeinen Methode 5A wurden 850 mg (7.20 mmol) 4-Methylpyridin-3-carbonitril, 750 mg (10.79 mmol) Hydroxylammoniumchlorid und 1.2 ml (8.6 mmol) Triethylamin umgesetzt. Ausbeute: 666 mg (61 % d. Th.)

¹H-NMR (400 MHz, DMSO-d₆): δ = 9.52 (s, 1H), 8.40 (d, 2H), 7.25 (d, 1H), 5.89 (s, 2H), 3.32 (s, 3H).

### Beispiel 71A

### N'-Hydroxy-6-methylpyridin-3-carboximidamid

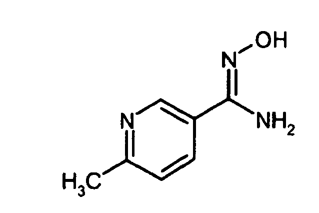

Nach der Allgemeinen Methode 5A wurden 1.0 g (8.5 mmol) 6-Methylpyridin-3-carbonitril, 882 mg (12.7 mmol) Hydroxylammoniumchlorid und 1.4 ml (10.2 mmol) Triethylamin umgesetzt. Ausbeute: 1.11 g (87% d. Th.)

¹H-NMR (400 MHz, DMSO-d₆): δ = 9.74 (s, 1H), 8.71 (d, 1H), 7.89 (dd, 1H), 7.25 (d, 1H), 5.93 (s, 2H), 2.47 (s, 3H).

### Beispiel 72A

### 2,3-Difluor-N'-hydroxybenzolcarboximidamid

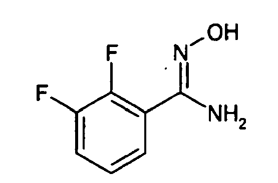

5.0 g (35.2 mmol) 2,3-Difluorbenzonitril wurden in 43 ml Ethanol vorgelegt und bei RT mit einer Lösung von 3.7 g (52.8 mmol) Hydroxylammoniumchlorid und 5.9 ml (4.3 g, 42.3 mmol) Triethylamin versetzt. Das Reaktionsgemisch wurde 16 h bei RT gerührt, im Vakuum eingeengt, in Essigsäureethylester aufgenommen und mit gesättigter, wässriger Natriumhydrogencarbonat-Lösung gewaschen. Die organischen Extrakte wurden über Natriumsulfat getrocknet, filtriert und im Vakuum eingeengt. Ausbeute: 5.7 g (94% d. Th.)

LC-MS (Methode 5B): Rₜ = 0.46 min; MS (ESIpos): m/z = 173 [M+H]⁺.

### Beispiel 73A

### 3-Fluor-N'-hydroxybenzolcarboximidamid

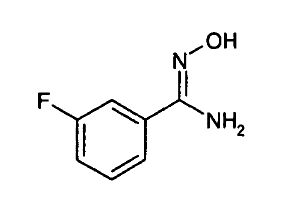

Nach der Allgemeinen Methode 5A wurden 10.0 g (82.6 mmol) 3-Fluorbenzonitril umgesetzt. Ausbeute: 12.6 g (99% d. Th.)

HPLC (Methode 1A): Rₜ = 1.14 min; MS (ESIpos): m/z = 155 [M+H]⁺.

### Beispiel 74A

### 2,5-Difluor-N'-hydroxybenzolcarboximidamid

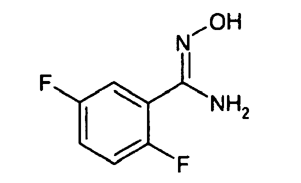

Nach der Allgemeinen Methode 5A wurden 5.0 g (35.9 mmol) 2,5-Difluorbenzonitril umgesetzt. Ausbeute: 5.8 g (94% d. Th.)

LC-MS (Methode 5B): Rₜ = 0.43 min; MS (ESIpos): m/z = 173 [M+H]⁺.

### Beispiel 75A

### 1-Cyclopropyl-N'-hydroxypiperidin-4-carboximidamid

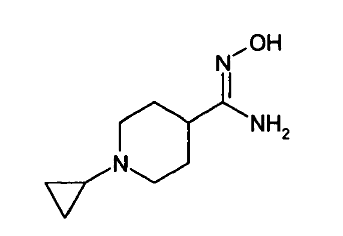

### Stufe a): 1-Cyclopropylpiperidin-4-carbonitril

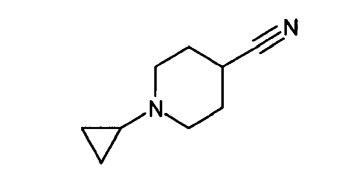

5.0 g (45.4 mmol) 4-Cyanopiperidin wurden in 83 ml Methanol gelöst und mit 11.9 g (68.1 mmol) [(1-Ethoxy-1-cyclopropyl)-oxy]-trimethylsilan, 2.9 ml (3.0 g, 49.9 mmol) Essigsäure sowie 6.0 g (91.0 mmol) Natriumcyanoborhydrid versetzt. Das Reaktionsgemisch wurde 16 h bei 60°C gerührt, nach Abkühlen auf RT über Kieselgur filtriert, mit Methanol nachgewaschen, und das Filtrat wurde im Vakuum eingeengt. Der Rückstand wurde in Essigsäureethylester aufgenommen, zweimal mit 1 N Natronlauge und einmal mit gesättigter, wässriger Natriumchlorid-Lösung gewaschen. Die organische Phase wurde über Natriumsulfat getrocknet, filtriert und im Vakuum eingeengt. Ausbeute: 6.0 g (88% d. Th.)

GC-MS (Methode 1F): Rₜ = 3.83 min; MS (ESIpos): m/z = 151 [M+H]⁺.

### Stufe b): 1-Cyclopropyl-N'-hydroxypiperidin-4-carboximidamid

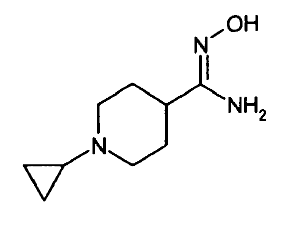

Nach der Allgemeinen Methode 5A wurden 6.0 g (39.9 mmol) 1-Cyclopropylpiperidin-4-carbonitril umgesetzt. Ausbeute: 5.3 g (72% d. Th.)

MS (ESIpos): m/z = 184 [M+H]⁺.

### Beispiel 76A

### 3-Ethoxy-N'-hydroxypropanimidamid

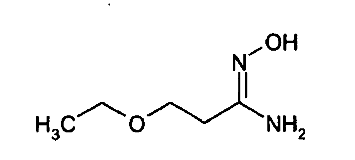

Nach der Allgemeinen Methode 5A wurden 5.0 g (50.4 mmol) 3-Ethoxypropionitril umgesetzt. Ausbeute: 0.6 g (8% d. Th., 90% Reinheit)

HPLC (Methode 1A): Rₜ = 0.60 min; MS (ESIpos): m/z =133 [M+H]⁺.

### Beispiel 77A

### N',2-Dihydroxy-2-methylpropanimidamid

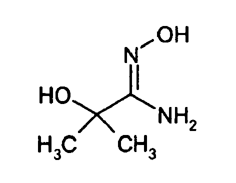

Nach der Allgemeinen Methode 5A wurden 5.0 g (57.6 mmol) 2-Hydroxy-2-methylpropanonitril umgesetzt. Ausbeute: 4.0 g (57% d. Th., 96% Reinheit)

HPLC (Methode 1A): Rₜ = 0.45 min; MS (ESIpos): m/z = 119 [M+H]⁺.

### Beispiel 78A

### N'-Hydroxycyclopropancarboximidamid

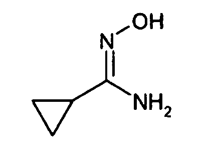

Nach der Allgemeinen Methode 5A wurden 7.2 g (107.3 mmol) Cyclopropancarbonsäurenitril umgesetzt. Ausbeute: 4.8 g (44% d. Th.)

LC-MS (Methode 2B): Rₜ = 0.16 min; MS (ESIpos): m/z = 101 [M+H]⁺.

### Beispiel 79A

### tert-Butyl-{[1-({3-[3-(3-fluorphenyl)-1,2,4-oxadiazol-5-yl]-5-[4-(trifluormethoxy)phenyl]-piperidin-1-yl}carbonyl)cyclopropyl]methyl}carbamat [racemisches cis-Isomer]

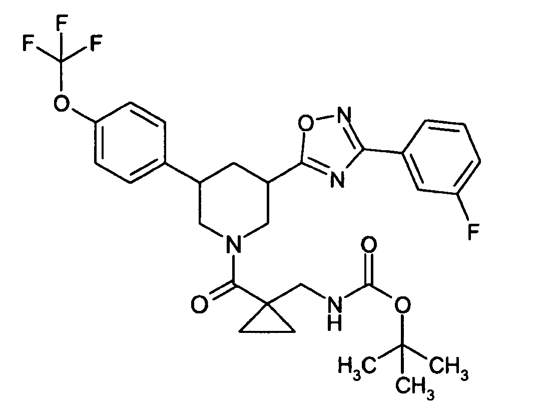

150 mg (0.30 mmol) der Verbindung aus Beispiel 178A wurden nach der Allgemeinen Methode 7 mit 70 mg (0.32 mmol) 1-{[(*tert*-Butoxycarbonyl)amino]methyl}cyclopropancarbonsäure umgesetzt. Ausbeute: 126 mg (70% d. Th.)

LC-MS (Methode 3B): Rₜ = 2.76 min; MS (ESIpos): m/z = 606 [M+H]⁺.

### Beispiel 80A

### N'-Hydroxy-3-methoxy-2,2-dimethylpropanimidamid

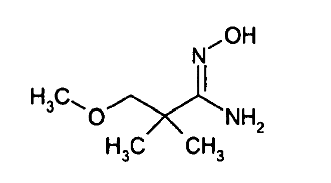

Nach der Allgemeinen Methode 6A wurden 5.0 g (44.2 mmol) 3-Methoxy-2,2-dimethylpropionitril umgesetzt. Ausbeute: 4.4 g (68% d. Th.)

GC-MS (Methode 1F): Rₜ = 1.20 min; MS (ESIpos): m/z = 147 [M+H]⁺.

### Beispiel 81A

### 1-(Morpholin-4-ylcarbonyl)-5-[4-(trifluormethyl)phenyl]piperidin-3-carbonsäure [racemisches cis-Isomer]

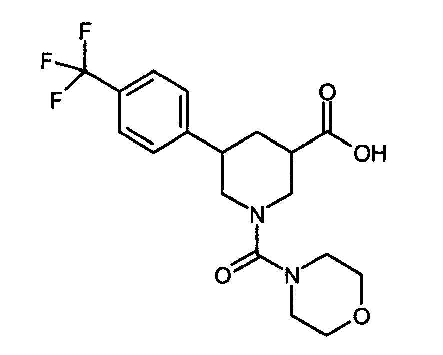

Nach der Allgemeinen Methode 9A wurden 22.19 g (39.90 mmol) der Verbindung aus Beispiel 48A und 44.78 g (399.0 mmol) Kalium-*tert-*butylat umgesetzt. Ausbeute: 18.29 g (100% d. Th.).

LC-MS (Methode 5B): Rₜ = 1.95 min; MS (ESIpos): m/z = 387 [M+H]⁺.

¹H-NMR (400 MHz, DMSO-d₆): δ = 12.36 (bs, 1H), 7.68 (d, 2H), 7.54 (d, 2H), 3.84 (br d, 1H), 3.58-3.52 (m, 5H), 3.19-3.14 (m, 4H), 2.91-2.80 (m, 3H), 2.60 (tt, 1H), 2.15 (br d, 1H), 1.78 (dd, 1H).

### Beispiel 82A

### 1-(Morpholin-4-ylcarbonyl)-5-[4-(trifluormethyl)phenyl]piperidin-3-carboxamid [racemisches cis-Isomer]

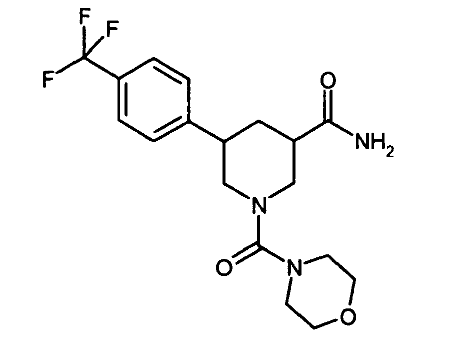

Zu einer Lösung von 5.16 g (13.4 mmol) der Verbindung aus Beispiel 81A in 180 ml Tetrahydrofuran wurden 4.77 g (40.1 mmol) Thionylchlorid hinzugegeben. Die Mischung wurde 1 h bei Rückflußtemperatur gerührt. Zur Aufarbeitung wurde im Vakuum Dichlormethan entfernt, der Rückstand noch mal mit Dichlormethan versetzt und eingeengt. Der Rückstand wurde in 75 ml Tetrahydrofuran vorgelegt und auf 0°C gekühlt. Anschließend wurden 19.0 ml (133.4 mmol) einer 7M Ammoniak-Lösung in Methanol zugegeben. Das Reaktionsgemisch wurde 1 h bei 0°C gerührt. Zur Aufarbeitung wurde das Lösungsmittel im Vakuum entfernt und der Rückstand in Essigsäureethylester aufgenommen. Die organische Phase wurde nacheinander mit Wasser und gesättigter, wässriger Natriumchlorid-Lösung gewaschen, über Magnesiumsulfat getrocknet und eingeengt. Ausbeute: 4.42 g (86% d. Th.).

LC-MS (Methode 2B): Rₜ = 1.00 min; MS (ESIpos): m/z = 386 [M+H]⁺;

¹H-NMR (400 MHz, DMSO-d₆): δ = 12.36 (br s, 1H), 7.68 (d, 2H), 7.54 (d, 2H), 3.85 (br d, 1H), 3.57-3.52 (m, 5H), 3.18-3.14 (m, 4H), 2.90-2.80 (m, 3H), 2.60 (tt, 1H), 2.15 (br d, 1H), 1.76 (dd, 1H).

### Beispiel 83A

### 1-(Morpholin-4-ylcarbonyl)-5-[4-(trifluormethyl)phenyl]piperidin-3-carbonitril [racemisches cis-Isomer]

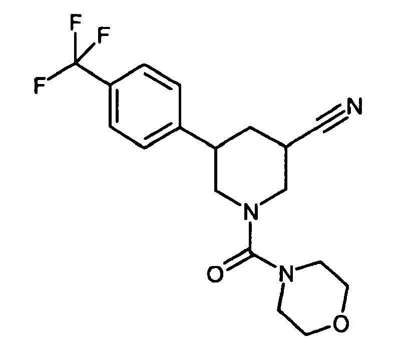

Zu einer Lösung von 4.42 g (11.5 mmol) der Verbindung aus Beispiel 82A in 208 ml Tetrahydrofuran unter Argon wurden 3.83 g (16.1 mmol) Burgess-Reagenz hinzugegeben. Die Mischung wurde 1 h bei 70°C gerührt. Zur Aufarbeitung wurde das Lösungsmittel im Vakuum entfernt und der Rückstand in Essigsäureethylester aufgenommen. Die organische Phase wurde nacheinander mit Wasser und gesättigter wässriger Natriumchlorid-Lösung gewaschen, über Magnesiumsulfat getrocknet und eingeengt. Ausbeute: 4.19 g (99% d. Th.)

LC-MS (Methode 1B): Rₜ = 2.20 min; MS (ESIpos): m/z = 368 [M+H]⁺.

### Beispiel 84A

### 1-N'-Hydroxy-(morpholin-4-ylcarbonyl)-5-[4-(trifluormethyl)phenyl]piperidin-3-carboximidamid [racemisches cis-Isomer]

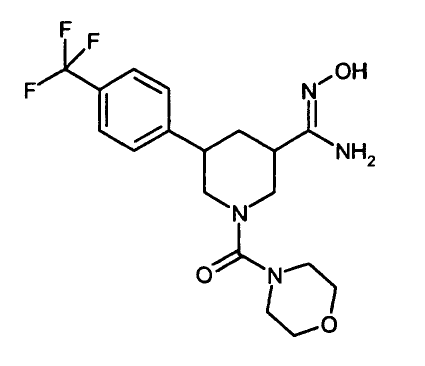

Zu einer Lösung von 4.19 g (9.81 mmol) der Verbindung aus Beispiel 83A in 144 ml Ethanol wurden 1.7 ml (12.8 mmol) Triethylamin und 1.02 g (14.71 mmol) Hydroxylammoniumchlorid hinzugegeben. Die Mischung wurde 9 h bei 50°C gerührt. Das Lösungsmittel wurde im Vakuum entfernt und der Rückstand in Essigsäureethylester aufgenommen. Die organische Phase wurde mit Wasser gewaschen, über Magnesiumsulfat getrocknet und eingeengt. Man erhält so 3.41 g Rohprodukt in 85%-iger Reinheit (LC-MS), welches ohne weitere Reinigungsoperationen umgesetzt wurde.

LC-MS (Methode 1B): Rₜ = 1.39 min; MS (ESIpos): m/z = 401 [M+H]⁺.

### Beispiel 85A

### tert-Butyl-[1-(3-{1-(morpholin-4-ylcarbonyl)-5-[4-(trifluormethyl)phenyl]piperidin-3-yl}-1,2,4-oxadiazol-5-yl)cyclopropyl]carbamat [racemisches cis-Isomer]

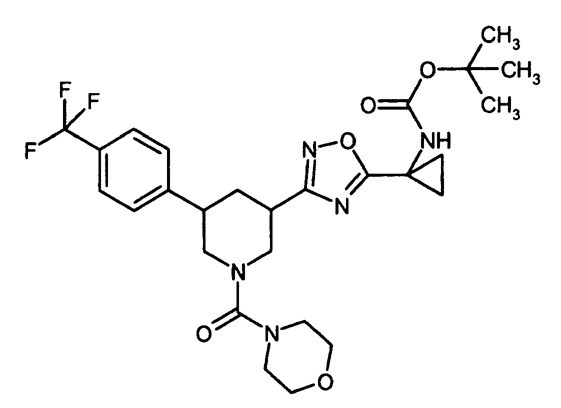

Nach der Allgemeinen Methode 2 wurden 170 mg (0.85 mmol) 1-[(*tert-*Butoxycarbonyl)amin]cyclopropancarbonsäure und 200 mg (0.43 mmol) der Verbindung aus Beispiel 84A umgesetzt. Ausbeute: 134 mg (53% d. Th.).

LC-MS (Methode 3B): Rₜ = 2.25 min; MS (ESIpos): m/z = 568 [M+H]⁺;

¹H-NMR (400 MHz, DMSO-d₆): δ = 8.02 (s, 1H), 7.69 (d, 2H), 7.55 (d, 2H), 3.92 (br d, 1H), 3.64 (d, 1H), 3.57-3.53 (m, 4H), 3.20-3.11 (m, 5H), 3.03-2.91 (m, 2H), 2.22 (br d, 1H), 1.90 (q, 1H), 1.52-1.49 (m, 2H), 1.39 (s, 9H), 1.37-1.33 (m, 1H), 1.29-1.24 (m, 2H).

### Beispiel 86A

### tert-Butyl-[2-(3-{1-(morpholin-4-ylcarbonyl)-5-[4-(trifluormethyl)phenyl]piperidin-3-yl}-1,2,4-oxadiazol-5-yl)propan-2-yl]carbamat [racemisches cis-Isomer]

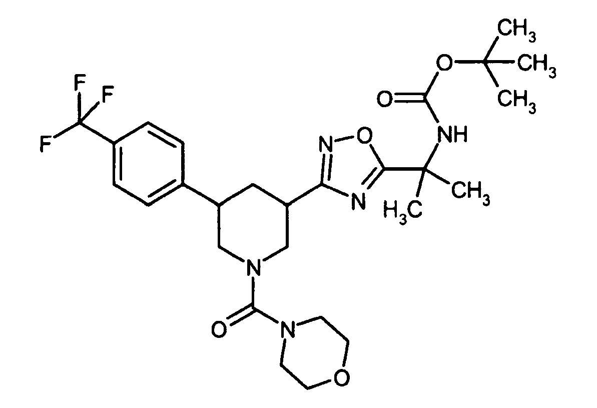

Nach der Allgemeinen Methode 2 wurden 860 mg (0.43 mmol) *N-*[(*tert-*Butoxycarbonyl)-2-methylalanin und 100 mg (0.21 mmol) der Verbindung aus Beispiel 84A umgesetzt. Ausbeute: 40 mg (34% d. Th.).

LC-MS (Methode 1B): Rₜ = 2.61 min; MS (ESIpos): m/z = 568 [M+H]⁺;

¹H-NMR (400 MHz, DMSO-d₆): δ = 7.69 (d, 2H), 7.56 (d, 2H), 3.93 (br d, 1H), 3.64 (d, 1H), 3.58-3.54 (m, 4H), 3.22-3.12 (m, 5H), 3.08-3.02 (m, 2H), 2.98 (t, 1H), 2.24 (br d, 1H), 1.91 (q, 1H), 1.54 (s, 6H), 1.32 (s, 9H).

### Beispiel 87A

### tert-Butyl-[(3-{1-(morpholin-4-ylcarbonyl)-5-[4-(trifluormethyl)phenyl]piperidin-3-yl}-1,2,4-oxadiazol-5-yl)methyl]carbamat [racemisches cis-Isomer]

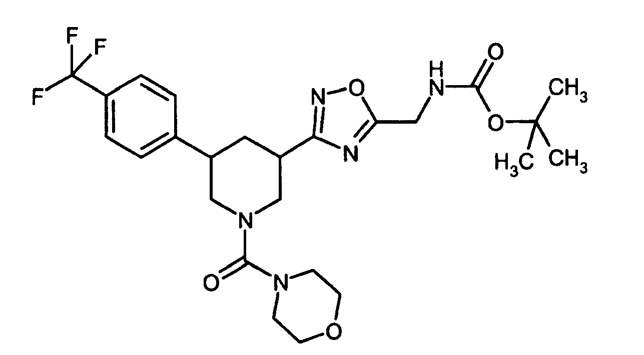

Nach der Allgemeinen Methode 1 wurden 149 mg (0.85 mmol) *N-*(*tert-*Butoxycarbonyl)glycin und 200 mg (0.43 mmol) der Verbindung aus Beispiel 84A umgesetzt. Ausbeute: 74 mg (32% d. Th.).

LC-MS (Methode 5B): Rₜ = 2.40 min; MS (ESIpos): m/z = 540 [M+H]⁺;

¹H-NMR (400 MHz, DMSO-d₆): δ = 7.69 (d, 2H), 7.65 (t, 1H), 7.56 (d, 2H), 4.40 (d, 2H), 3.93 (br d, 1H), 3.65 (d, 1H), 3.59-3.55 (m, 4H), 3.22-3.15 (m, 5H), 3.05-2.94 (m, 3H), 2.26 (br d, 1H), 1.93 (q, 1H), 1.39 (s, 9H).

### Beispiel 88A

### tert-Butyl-[(1S)-1-(3-{1-(morpholin-4-ylcarbonyl)-5-[4-(trifluormethyl)phenyl]piperidin-3-yl}-1,2,4-oxadiazol-5-yl)ethyl]carbamat [racemisches cis-Isomer]

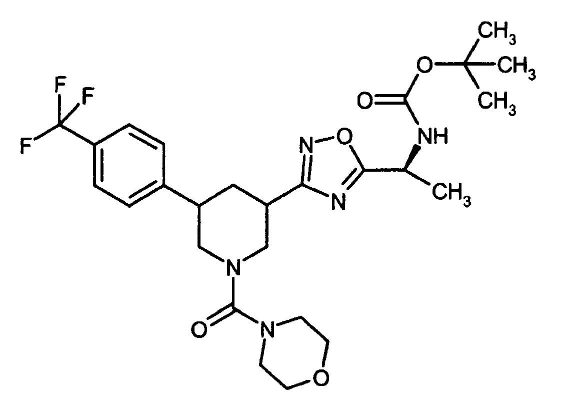

Nach der Allgemeinen Methode 2 wurden 80 mg (0.43 mmol) *N-*(*tert-*Butoxycarbonyl)-L-alanin und 100 mg (0.21 mmol) der Verbindung aus Beispiel 84A umgesetzt. Ausbeute: 41 mg (35% d. Th.).

LC-MS (Methode 2B): Rₜ = 1.36 min; MS (ESIpos): m/z = 554 [M+H]⁺;

¹H-NMR (400 MHz, DMSO-d₆): δ = 7.69 (d, 1H), 7.56 (d, 2H), 4.92-4.84 (m, 1H), 3.95 (br d, 1H), 3.65 (d, 1H), 3.58-3.53 (m, 4H), 3.22-3.13 (m, 5H), 3.06-2.95 (m, 3H), 2.25 (br d, 1H), 1.92 (q, 1H), 1.43 (d, 3H), 1.38 (s. 9H).

### Beispiel 89A

### Methyl-1-[(3-oxopiperazin-1-yl)carbonyl]-5-[4-(trifluormethyl)phenyl]piperidin-3-carboxylat [racemisches cis-/trans-Isomerengemisch]

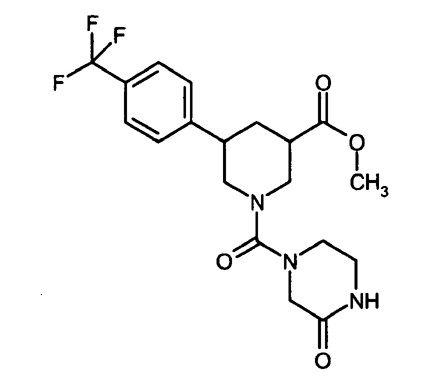

Nach der Allgemeinen Methode 8A wurden 4.00 g (8.84 mmol) 3-Methyl-1-(4-nitrophenyl)-5-[4-(trifluormethyl)phenyl]piperidin-1,3-dicarboxylat und 1.77 g (17.7 mmol) Piperazin-2-on umgesetzt. Ausbeute: 1.92 g (51% d. Th.).

LC-MS (Methode 2B): Rₜ = 1.05 min und 1.08 min (cis-/trans-Isomere); MS (ESIpos): m/z = 414 [M+H]⁺.

### Beispiel 90A

### tert-Butyl-methyl-[(3-{1-(morpholin-4-ylcarbonyl)-5-[4-(trifluormethyl)phenyl]piperidin-3-yl}-1,2,4-oxadiazol-5-yl)methyl]carbamat [racemisches cis-Isomer]

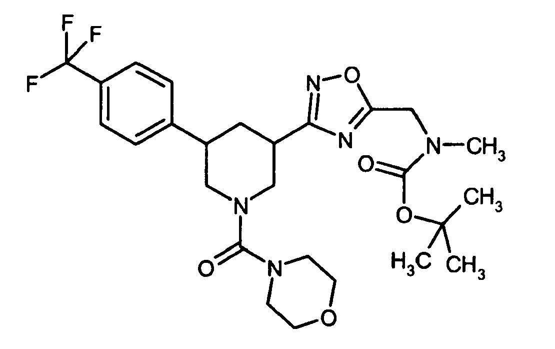

Nach der Allgemeinen Methode 2 wurden 96 mg (0.50 mmol) *N-*(*tert-*Butoxycarbonyl)-*N-*methylglycin und 140 mg (0.25 mmol) der Verbindung aus Beispiel 84A umgesetzt. Ausbeute: 102 mg (73% d. Th.).

LC-MS (Methode 2B): Rₜ = 1.39 min; MS (ESIpos): m/z = 554 [M+H]⁺;

### Beispiel 91A

### tert-Butyl-(2S)-2-(3-{1-(morpholin-4-ylcarbonyl)-5-[4-(trifluormethyl)phenyl]piperidin-3-yl}-1,2,4-oxadiazol-5-yl)pyrrolidin-1-carboxylat

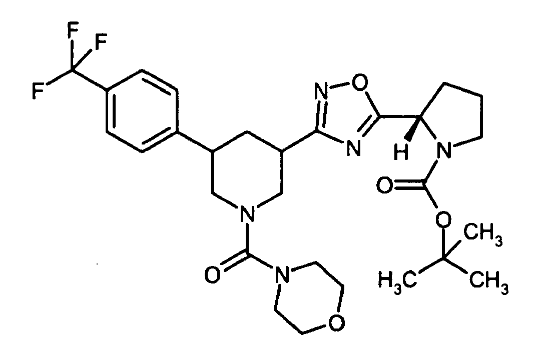

Nach der Allgemeinen Methode 2 wurden 108 mg (0.50 mmol) 1-(*tert-*Butoxycarbonyl)-L-prolin und 140 mg (0.25 mmol) der Verbindung aus Beispiel 84A umgesetzt. Ausbeute: 91 mg (62% d. Th.).

LC-MS (Methode 1B): Rₜ = 2.67 min; MS (ESIpos): m/z = 580 [M+H]⁺.

### Beispiel 92A

### tert-Butyl-3-(3-{1-(morpholin-4-ylcarbonyl)-5-[4-(trifluormethyl)phenyl]piperidin-3-yl}-1,2,4-oxadiazol-5-yl)azetidin-1-carboxylat

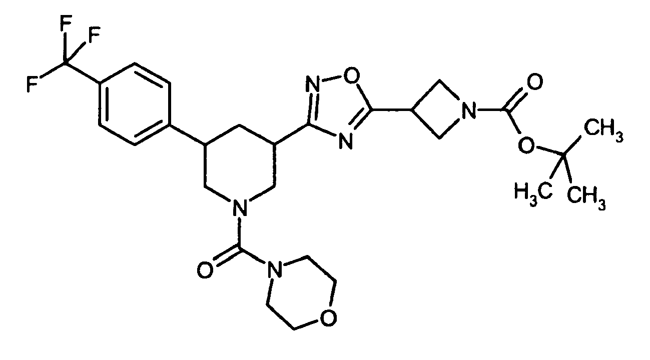

### Stufe a): 1-(tert-Butoxycarbonyl)azetidin-3-carbonsäure

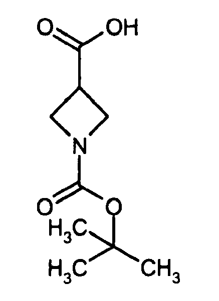

Eine Mischung von 100 mg (0.99 mmol) Azetidin-3-carbonsäure und 237 mg (1.09 mmol) Di-*tert-*butyldicarbonat in 1 ml Dichlormethan wurde bei Raumtemperatur 16 h gerührt. Zur Aufarbeitung wurde das Lösungsmittel im Vakuum entfernt, und der Rückstand wurde ohne weitere Reinigung eingesetzt. Ausbeute: 175 mg (88% d. Th.).

¹H-NMR (400 MHz, DMSO-d₆): δ = 4.00-3.96 (m, 2H), 3.90-3.80 (m, 2H), 3.35-3.28 (m, 1H), 1.37 (s, 9H).

### Stufe b): tert-Butyl-3-(3-{-1-(morpholin-4-ylcarbonyl)-5-[4-(trifluormethyl)phenyl]-piperidin-3-yl}-1,2,4-oxadiazol-5-yl)azetidin-1-carboxylat

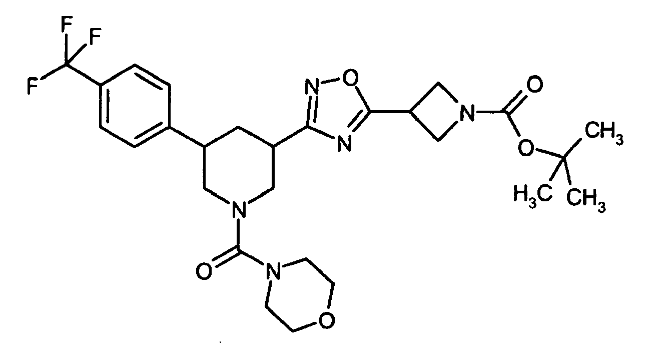

Nach der Allgemeinen Methode 2 wurden 202 mg (1.01 mmol) 1-(*tert-*Butoxycarbonyl)azetidin-3-carbonsäure und 140 mg (0.25 mmol) der Verbindung aus Beispiel 84A umgesetzt. Ausbeute: 74 mg (52% d. Th.).

LC-MS (Methode 2B): Rₜ = 1.39 min; MS (ESIpos): m/z = 567 [M+H]⁺;

¹H-NMR (400 MHz, DMSO-d₆): δ = 7.70 (d, 2H), 7.57 (d, 2H), 4.28-4.24 (m, 2H), 4.18-4.11 (m, 1H), 4.08-4.00 (m, 2H), 3.96 (br d, 1H), 3.66 (d, 1H), 3.58-3.54 (m, 4H), 3.25-3.15 (m, 5H), 3.10-2.98 (m, 3H), 2.27 (br d, 1H), 1.95 (q, 1H), 1.38 (s. 9H).

### Beispiel 93A

### 3-Methyl-1-(4-nitrophenyl)-5-[4-(trifluormethyl)phenyl]piperidin-1,3-dicarboxylat [racemisches cis-/trans-Isomerengemisch]

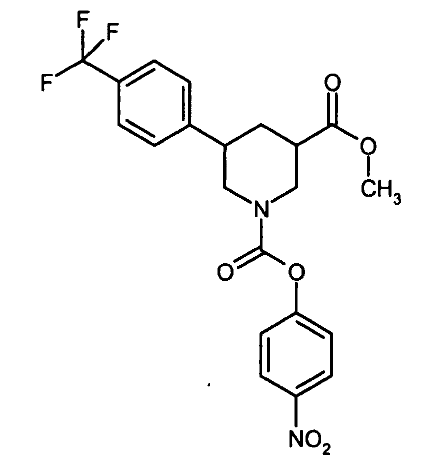

20.0 g (69.6 mmol) Methyl-5-[4-(trifluormethyl)phenyl]piperidin-3-carboxylat (Beispiel 47A) wurden in 1.01 Dichlormethan gelöst und bei 0 °C mit 14.1 g (139 mmol) Triethylamin versetzt. Anschließend wurden 14.0 g (69.6 mmol) 4-Nitrophenylchlorocarbonat zugetropft. Die Reaktionsmischung wurde für 2 h bei 0°C und anschließend 16 h bei RT gerührt. Zur Aufarbeitung wurde mit gesättigter, wässriger Natriumhydrogencarbonat-Lösung gewaschen. Die organische Phase wurde über Magnesiumsulfat getrocknet, filtriert und im Vakuum eingeengt. Es wurden 31.3 g Rohprodukt erhalten, welches ohne weitere Reinigungsoperationen umgesetzt wurde.

LC-MS (Methode 3B): Rₜ = 2.44 min und 2.48 min (cis-/trans-Isomere); MS (ESIpos): m/z = 453 [M+H]⁺.

### Beispiel 94A

### Methyl-1-[(4-hydroxypiperidin-1-yl)carbonyl]-5-[4-(trifluormethyl) phenyl]piperidin-3-carboxylat [racemisches cis-/trans-Isomerengemisch]

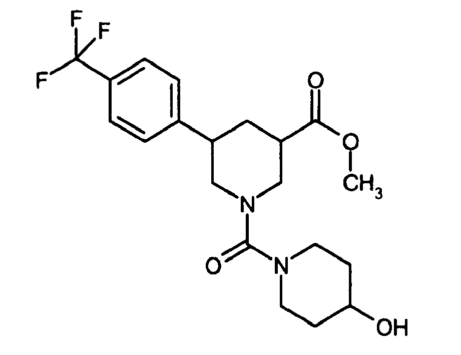

Nach der Allgemeinen Methode 8A wurden 4.00 g (8.84 mmol) 3-Methyl-1-(4-nitrophenyl)-5-[4-(trifluormethyl)phenyl]piperidin-1,3-dicarboxylat (Beispiel 93A) und 2.68 g (26.5 mmol) 4-Hydroxypiperidin umgesetzt. Ausbeute: 3.10 g (83% d. Th.).

LC-MS (Methode 3B): Rₜ = 2.72 min und 2.78 min (cis-/trans-Isomere); MS (ESIpos): m/z = 415 [M+H]⁺.

¹H-NMR (400 MHz, DMSO-d₆): δ = 7.69 (d, 2H), 7.53 (t, 2H), 4.67 (br d, 1H), 3.91-3.78 (m, 1H), 3.66-3.34 (m, 7H), 3.15-3.05 (m, 1H), 2.96-2.65 (m, 5H), 2.25-2.11 (m, 1H), 1.96-1.63 (m, 3H), 1.38-1.18 (m, 2H).

### Beispiel 95A

### Methyl-1-[(4-cyanpiperidin-1-yl)carbonyl]-5-[4-(trifluormethyl)phenyl]piperidin-3-carboxylat [racemisches cis-/trans-Isomerengemisch]

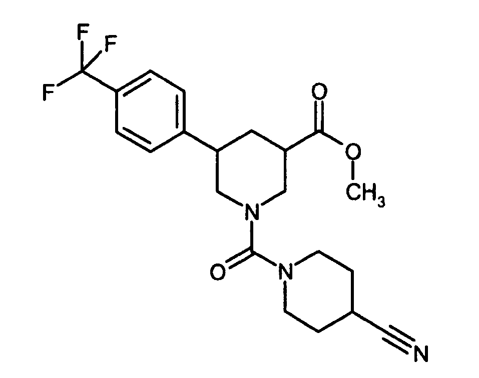

Nach der Allgemeinen Methode 8A wurden 4.00 g (8.84 mmol) 3-Methyl-1-(4-nitrophenyl)-5-(4-(trifluormethyl)phenyl]piperidin-1,3-dicarboxylat (Beispiel 93A) und 2.92 g (26.5 mmol) Piperidin-4-carbonitril umgesetzt. Ausbeute: 3.15 g (77% d. Th.).

LC-MS (Methode 1B): Rₜ = 2.35 min und 2.41 min (cis-/trans-Isomere); MS (ESIpos): m/z = 424 [M+H]⁺.

### Beispiel 96A

### Methyl-1-[(3-hydroxyazetidin-1-yl)carbonyl]-5-[4-(trifluormethyl)phenyl]piperidin-3-carboxylat [racemisches cis-/trans-Isomerengemisch]

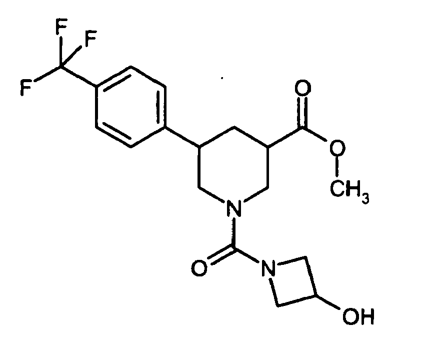

Nach der Allgemeinen Methode 8A wurden 4.00 g (8.84 mmol) 3-Methyl-1-(4-nitrophenyl)-5-[4-(trifluormethyl)phenyl]piperidin-1,3-dicarboxylat (Beispiel 93A), 2.91 g (26.5 mmol) 4-Azetidin-3-ol Hydrochlorid und Kaliumcarbonat (2.5 eq.) umgesetzt. Ausbeute: 2.48 g (70% d. Th.).

LC-MS (Methode 2B): Rₜ = 1.08 min und 1.10 min (cis-/trans-Isomere); MS (ESIpos): m/z = 387 [M+H]⁺.

### Beispiel 97A

### Methyl-1-(1,3-thiazolidin-3-ylcarbonyl)-5-[4-(trifluormethyl)phenyl]piperidin-3-carboxylat [racemisches cis-/trans-Isomerengemisch]

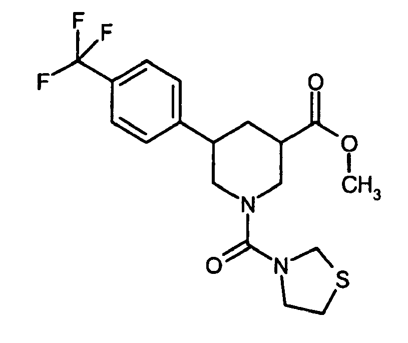

Nach der Allgemeinen Methode 8A wurden 4.00 g (8.84 mmol) 3-Methyl-1-(4-nitrophenyl)-5-[4-(trifluormethyl)phenyl]piperidin-1,3-dicarboxylat (Beispiel 93A) und 1.58 g (17.7 mmol) 1,3-Thiazolidin umgesetzt. Ausbeute: 0.54 g (15% d. Th.).

LC-MS (Methode 3B): Rₜ = 2.11 min und 2.18 min (cis-/trans-Isomere); MS (ESIpos): m/z = 403 [M+H]⁺.

### Beispiel 98A

### Methyl-1-[(3-methoxyazetidin-1-yl)carbonyl]-5-[4-(trifluormethyl)phenyl]piperidin-3-carboxylat [racemisches cis-/trans-Isomerengemisch]

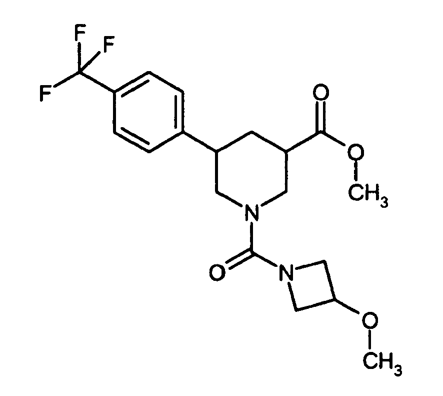

Nach der Allgemeinen Methode 8A wurden 4.00 g (8.84 mmol) 3-Methyl-1-(4-nitrophenyl)-5-[4-(trifluormethyl)phenyl]piperidin-1,3-dicarboxylat (Beispiel 93A), 2.73 g (22.1 mmol) 3-Methoxyazetidin-Hydrochlorid und Kaliumcarbonat (2.5 eq.) umgesetzt. Ausbeute: 2.98 g (82% d. Th.).

LC-MS (Methode 2B): Rₜ = 1.19 min und 1.22 min (cis-/trans-Isomere); MS (ESIpos): m/z = 401 [M+H]⁺.

### Beispiel 99A

### 1-[(4-Hydroxypiperidin-1-yl)carbonyl]-5-[4-(trifluormethyl)phenyl]piperidin-3-carbonsäure [racemisches cis-Isomer]

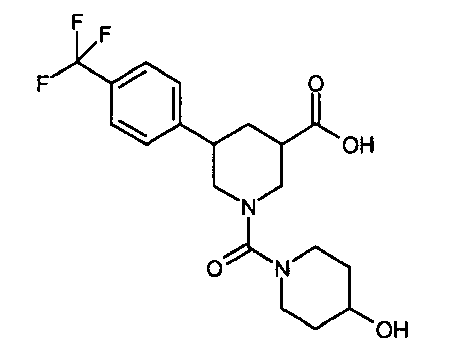

Nach der Allgemeinen Methode 9A wurden 2.10 g (5.07 mmol) Methyl-1-[(4-hydroxypiperidin-1-yl)carbonyl]-5-[4-(trifluormethyl)phenyl]piperidin-3-carboxylat (Beispiel 94A) umgesetzt. Ausbeute: 2.02 g (99% d. Th.).

LC-MS (Methode 2B): Rₜ = 1.01 min; MS (ESIpos): m/z = 401 [M+H]⁺.

### Beispiel 100A

### 1-[(4-Cyanpiperidin-1-yl)carbonyl]-5-[4-(trifluormethyl)phenyl]piperidin-3-carbonsäure [racemisches cis-Isomer]

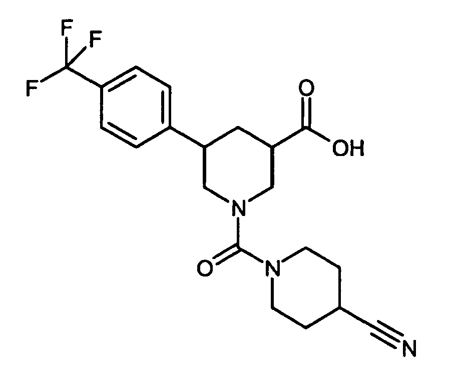

Nach der Allgemeinen Methode 9A (Reaktionszeit: 2 h) wurden 2.90 g (6.85 mmol) Methyl-1-[(4-cyanpiperidin-1-yl)carbonyl]-5-[4-(trifluormethyl)phenyl]piperidin-3-carboxylat (Beispiel 95A) umgesetzt. Ausbeute: 2.86 g (98% d. Th.).

LC-MS (Methode 1B): Rₜ = 2.15 min; MS (ESIpos): m/z = 410 [M+H]⁺.

¹H-NMR (400 MHz, DMSO-d₆): δ = 12.4 (br s, 1H), 7.69 (d, 2H), 7.53 (d, 2H), 3.82 (br d, 1H), 3.56 (br d, 1H), 3.35 (verdeckt, 1H), 3.10-2.99 (m, 3H), 2.95-2.79 (m, 3H), 2.65-2.54 (m, 1H), 2.15 (br d, 1H), 1.91-1.59 (m, 5H).

### Beispiel 101A

### 1-[(3-Hydroxyazetidin-1-yl)carbonyl]-5-[4-(trifluormethyl)phenyl]piperidin-3-carbonsäure [racemisches cis-Isomer]

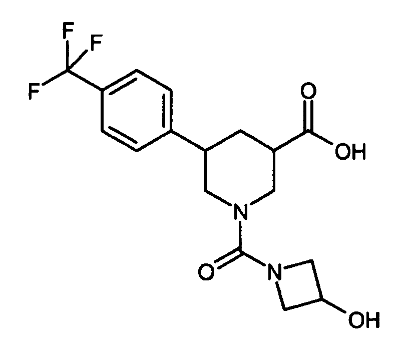

Nach der Allgemeinen Methode 9A (Reaktionszeit: 2 h) wurden 2.48 g (6.42 mmol) Methyl-1-[(3-hydroxyazetidin-1-yl)carbonyl]-5-[4-(trifluormethyl)phenyl]piperidin-3-carboxylat (Beispiel 96A) umgesetzt. Ausbeute: 2.33 g (94% d. Th.).

LC-MS (Methode 1B): Rₜ = 1.84 min; MS (ESIpos): m/z = 373 [M+H]⁺.

¹H-NMR (400 MHz, DMSO-d₆): δ = 12.4 (br s, 1H), 7.69 (d, 2H), 7.53 (d, 2H), 5.57 (d, 1H), 4.42-4.32 (m, 1H), 4.11-3.95 (m, 3 H), 3.77-3.63 (m, 3H), 3.32 (verdeckt, 1H), 2.88-2.76 (m, 3H), 2.14 (br d, 1H), 1.85-1.72 (m, 1 H).

### Beispiel 102A

### 1-(1,3-Thiazolidin-3-ylcarbonyl)-5-[4-(trifluormethyl)phenyl]piperidin-3-carbonsäure [racemisches cis-Isomer]

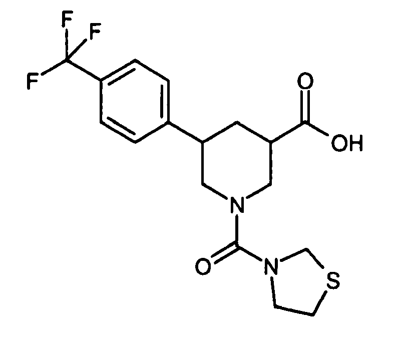

Nach der Allgemeinen Methode 9A wurden 0.54 g (1.33 mmol) Methyl-1-(1,3-thiazolidin-3-ylcarbonyl)-5-[4-(trifluormethyl)phenyl]piperidin-3-carboxylat (Beispiel 97A) umgesetzt. Ausbeute: 0.51 g (74% d. Th., 75%ige Reinheit).

LC-MS (Methode 2B): Rₜ = 1.17 min; MS (ESIpos): m/z = 389 [M+H]⁺.

### Beispiel 103A

### 1-[(3-Methoxyazetidin-1-yl)carbonyl]-5-[4-(trifluormethyl)phenyl]piperidin-3-carbonsäure [racemisches cis-Isomer]

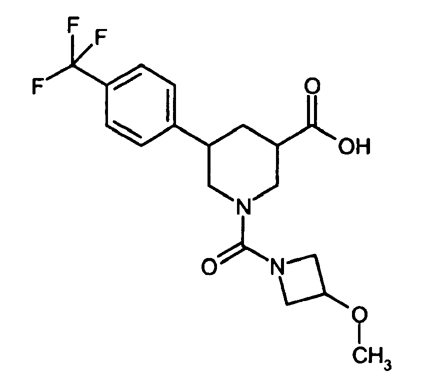

Nach der Allgemeinen Methode 9A wurden 2.90 g (7.24 mmol) Methyl-1-[(3-methoxyazetidin-1-yl)carbonyl]-5-[4-(trifluormethyl)phenyl]piperidin-3-carboxylat (Beispiel 98A) umgesetzt. Ausbeute: 2.77 g (99% d. Th.).

LC-MS (Methode 3B): Rₜ = 1.71 min; MS (ESIpos): m/z = 387 [M+H]⁺.

¹H-NMR (400 MHz, DMSO-d₆): δ = 12.5 (br s, 1H), 7.69 (d, 2H), 7.53 (d, 2H), 4.16-3.95 (m, 4H), 3.80-3.69 (m, 3H), 3.32 (verdeckt, 1H), 3.18 (s, 3H), 2.89-2.77 (m, 3H), 2.14 (br d, 1H), 1.85-1.72 (m, 1 H).

### Beispiel 104A

### Methyl-1-[(2-methoxyethyl)carbamoyl]-5-[4-(trifluormethyl)phenyl]piperidin-3-carboxylat [racemisches cis-/trans-Isomerengemisch]

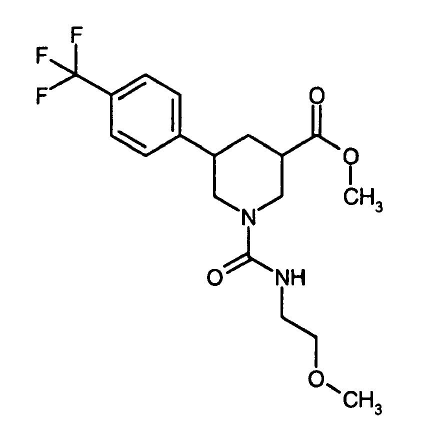

Nach der Allgemeinen Methode 8A wurden 8.83 g (ca. 14.3 mmol) der Verbindung aus Beispiel 93A und 3.21 g (14.3 mmol) 2-Methoxyethanamin umgesetzt. Ausbeute: 2.94 g (51% d. Th.).

LC-MS (Methode 1B): Rₜ = 2.14 min und 2.19 min (cis-/trans-Isomere); MS (ESIpos): m/z = 389 [M+H]⁺.

### Beispiel 105A

### 1-[(2-Methoxyethyl)carbamoyl]-5-[4-(trifluormethyl)phenyl]piperidin-3-carbonsäure [racemisches cis-/trans-Isomerengemisch]

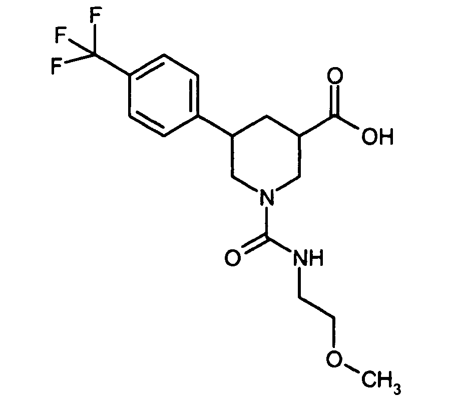

Nach der Allgemeinen Methode 9A wurden 2.94 g (7.57 mmol) der Verbindung aus Beispiel 104A und 8.49 g (75.70 mmol) Kalium- *tert.*-butylat umgesetzt. Ausbeute: 2.46 g (80% d. Th., 92% Reinheit)

LC-MS (Methode 1B): Rₜ = 1.97 min und 2.02 min (cis-/trans-Isomere); MS (ESIpos): m/z = 375 [M+H]⁺.

### Beispiel 106A

### 1-(Morpholin-4-ylcarbonyl)-N-(2-oxo-2-phenylethyl)-5-[4-(trifluormethyl)phenyl]piperidin-3-carboxamid [racemisches cis-/trans-Isomerengemisch]

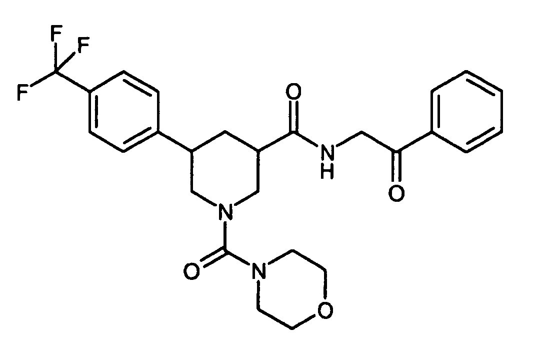

Zu einer Lösung von 200 mg (0.52 mmol) der Verbindung aus Beispiel 49A in 1 ml Dichlormethan wurden 97 mg (0.57 mmol) Thionylchlorid hinzugegeben. Die Mischung wurde 1 h unter Rückfluß gerührt. Zur Aufarbeitung wurde im Vakuum Dichlormethan entfernt, der Rückstand noch einmal mit Dichlormethan versetzt und eingeengt. Der Rückstand wurde in 5 ml Dichlormethan vorgelegt und anschließend wurden 78 mg (0.78 mmol) Triethylamin und 97 mg (0.57 mmol) 2-Amino-1-phenylethanon zugegeben. Das Reaktionsgemisch wurde 3 h bei Raumtemperatur gerührt. Zur Aufarbeitung wurde die Mischung mit Wasser versetzt, die organische Phase abgetrennt und mehrmals mit gesättigter, wässriger Natriumchlorid-Lösung gewaschen. Die organische Phase wurde über Magnesiumsulfat getrocknet und im Vakuum zu Trockne eingeengt. Ausbeute: 300 mg (62% d. Th., 70% Reinheit)

LC-MS (Methode 2B): Rₜ = 1.21 min; MS (ESIpos): m/z = 504 [M+H]⁺.

### Beispiel 107A

### Methyl-1-[(4-cyanpiperidin-1-yl)carbonyl]-5-[4-(trifluormethoxy) phenyl]piperidin-3-carboxylat [racemisches cis-/ trans-Isomerengemisch]

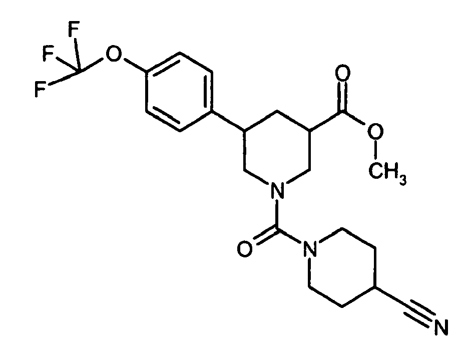

Nach der Allgemeinen Methode 8A wurden 5.15 g (6.60 mmol) der Verbindung aus Beispiel 61A mit 2.18 g (19.79 mmol) 4-Cyanopiperidin umgesetzt. Ausbeute: 1.69 g (40% d. Th., 69% Reinheit)

LC-MS (Methode 1B): Rₜ = 2.40 min und 2.46 min (cis-/trans-Isomere); MS (ESIpos): m/z = 440 [M+H]⁺.

### Beispiels 108A

### 1-[(4-Cyanpiperidin-1-yl)carbonyl]-5-[4-(trifluormethoxy)phenyl ]piperidin-3-carbonsäure [racemisches cis-Isomer]

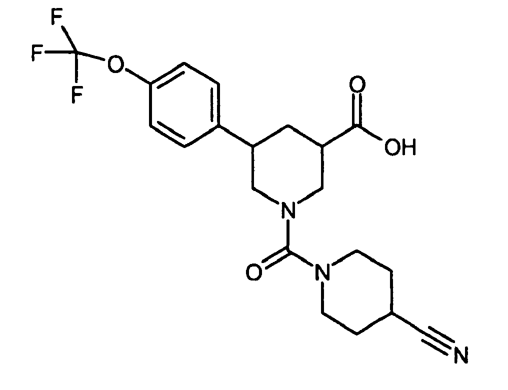

Nach der Allgemeinen Methode 9A wurden 1.69 g (ca. 2.62 mmol) der Verbindung aus Beispiel 107A und 2.9 g (26.2 mmol) Kalium-*tert*-butylat umgesetzt. Ausbeute: 1.53 g (90% d. Th., 65% Reinheit)

LC-MS (Methode 3B): Rₜ = 1.78 min und 1.83 min (cis-/trans-Isomere); MS (ESIpos): m/z = 426 [M+H]⁺.

### Beispiel 109A

### Methyl-1-[(3-hydroxyazetidin-1-yl)earbonyl]-5-(4-(trifluormethoxy)phenyl]piperidin-3-carboxylat [racemisches cis-/trans-Isomerengemisch]

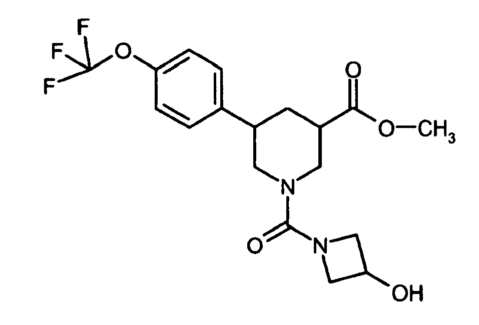

Nach der Allgemeinen Methode 8A wurden 300 mg (0.38 mmol) der Verbindung aus Beispiel 61A und 89 mg (1.15 mmol) 3-Hydroxyazetidin umgesetzt. Ausbeute: 100 mg (63% d. Th.).

LC-MS (Methode 9B): Rₜ = 0.97 min und 0.99 min (cis-/trans-Isomere); MS (ESIpos): m/z = 403 [M+H]⁺.

### Beispiel 110A

### 1-[(3-Hydroxyazetidin-1-yl)carbonyl]-5-[4-(trifluormethoxy)phenyl]piperidin-3-carbonsäure [racemisches cis-Isomer]

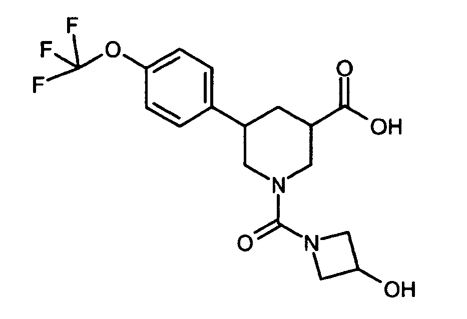

Nach der Allgemeinen Methode 9A wurden 700 mg (1.43 mmol) der Verbindung aus Beispiel 109A und 1.61 g (14.3 mmol) Kalium-*tert*-butylat umgesetzt. Ausbeute: 700 g (99% d. Th., 84% Reinheit)

LC-MS (Methode 9B): Rₜ = 0.87 min ; MS (ESIpos): m/z = 389 [M+H]⁺.

### Beispiels 111A

### Methyl-1-[(3-hydroxypyrrolidin-1-yl)carbonyl]-5-[4-(trifluormethoxy)phenyl]piperidin-3-carboxylat [racemisches cis-/trans-Isomerengemisch]

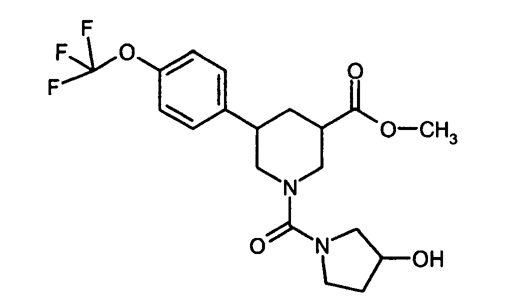

Nach der Allgemeinen Methode 8A wurden 20.0 g (25.6 mmol) der Verbindung aus Beispiel 61A und 6.69 g (76.9 mmol) 3-Pyrrolidinol umgesetzt. Ausbeute: 6.69 g (63% d. Th.).

LC-MS (Methode 9B): Rₜ = 0.99 min und 1.01 min (cis-/trans-Isomere); MS (ESIpos): m/z = 417 [M+H]⁺.

### Beispiel 112A

### 1-[(3-Hydroxypyrrolidin-1-yl)carbonyl]-5-[4-(trifluormethoxy)phenyl]piperidin-3-carbonsäure [racemisches cis-Isomer]

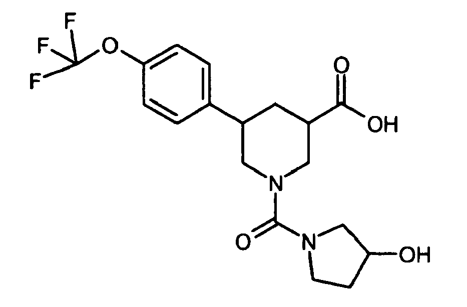

Nach der Allgemeinen Methode 9A wurden 26.05 g (16.06 mmol) der Verbindung aus Beispiel 111A mit 26.05 g (160.6 mmol) Kalium-*tert*-butylat umgesetzt. Ausbeute: 5.9 g (91% d. Th.).

LC-MS (Methode 9B): Rₜ = 0.89 min und 1.01 min; MS (ESIpos): m/z = 403 [M+H]⁺.

### Beispiel 113A

### Methyl-1-({4-[(tert-butoxycarbonyl)amino]piperidin-1-yl} carbonyl)-5-[4-(trifluormethoxy)-phenyl]piperidin-3-carboxylat [racemisches cis-/trans Isomer]

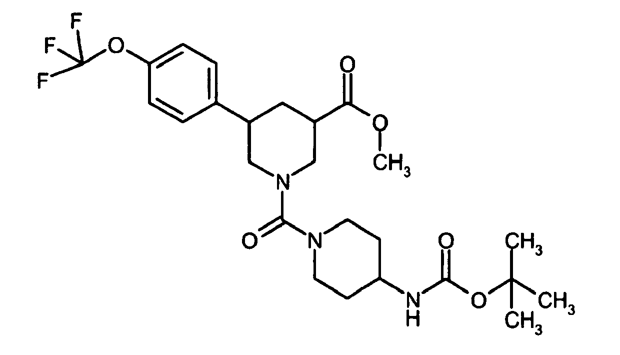

Nach der Allgemeinen Methode 8A wurden 2 g (2.6 mmol) der Verbindung aus Beispiel 61A mit 1.54 g (7.69 mmol) 4-*tert-*Butyl-piperidin-4-ylcarbamat umgesetzt. Ausbeute: 785 mg (58% d. Th).

LC-MS (Methode 2B): Rₜ = 1.37; MS (ESIpos): m/z = 530 [M+H]⁺.

### Beispiel 114A

### 1-({4-[(tert-Butoxycarbonyl)amin]piperidin-1-yl}carbonyl)-5-[4-(trifluormethoxy)phenyl]-piperidin-3-carbonsäure [racemisches cis-Isomer]

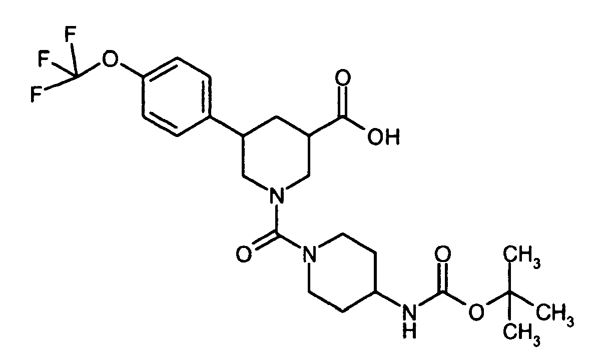

Nach der Allgemeinen Methode 9A wurden 785 mg (1.48 mmol) der Verbindung aus Beispiel 113A und 1.66 g (14.8 mmol) Kalium-*tert*-butylat umgesetzt. Ausbeute: 740 mg (97% d. Th.).

LC-MS (Methode 9B): Rₜ = 1.13 min; MS (ESIpos): m/z = 516 [M+H]⁺.

### Beispiel 115A

### 1-(Morpholin-4-ylcarbonyl)-5-[4-(trifluormethoxy)phenyl]piperidin-3-carbothioamid [racemisches cis-/trans-Isomerengemisch]

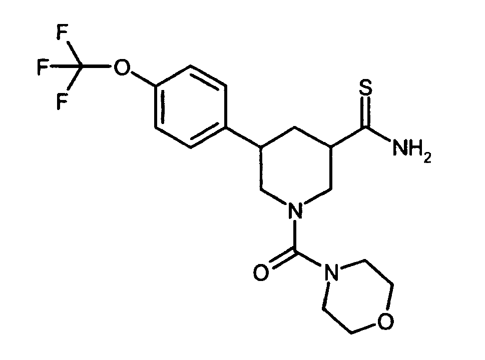

Nach der Allgemeinen Methode 10A wurden 820 mg (1.94 mmol) 1-(Morpholin-4-ylcarbonyl)-5-[4-(trifluormethoxy)phenyl]piperidin-3-carbonsäure Beispiel 44A und 432 mg (1.07 mol) Lawesson-Reagenz umgesetzt. Ausbeute: 530 mg (47% d. Th, 72% Reinheit)

LC-MS (Methode 1B): Rₜ = 2.17 min und 2.26 min; MS (ESIpos): m/z = 418 [M+H]⁺.

### Beispiel 116A

### 1-[(4-Cyanpiperidin-1-yl)carbonyl]-5-[4-(trifluormethoxy)phenyl]piperidin-3-carboxamid [racemisches cis-Isomerengemisch]

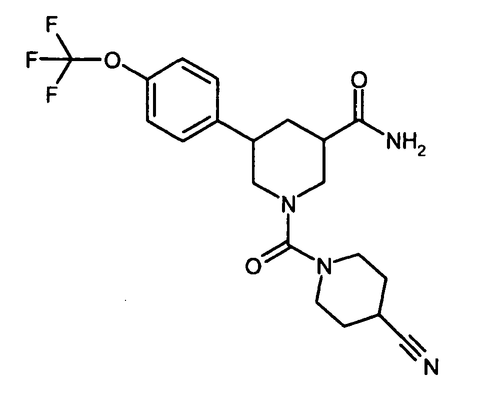

Zu einer Lösung von 3.0 g (4.4 mmol) 1-[(4-Cyanpiperidin-1-yl)carbonyl]-5-[4-(trifluormethoxy)phenyl]piperidin-3-carbonsäure (Beispiel 108A) in 79 ml Dichlormethan wurden 1.56 g (13.12 mmol) Thionylchlorid hinzugegeben. Die Mischung wurde 3 h unter Rückfluß gerührt. Zur Aufarbeitung wurde im Vakuum Dichlormethan entfernt, der Rückstand noch einmal mit Dichlormethan versetzt und eingeengt. Der Rückstand wurde in 28 ml Tetrahydrofuran vorgelegt und auf 0°C abgekühlt. Anschließend wurden 6.24 ml (43.72 mmol) einer 7M Ammoniak-Lösung in Methanol zugegeben. Das Reaktionsgemisch wurde 15 Minuten bei 0°C gerührt. Zur Aufarbeitung wurde das Lösungsmittel im Vakuum entfernt und der Rückstand in Essigsäureethylester aufgenommen. Die organische Phase wurde nacheinander mit Wasser und gesättigter, wässriger Natriumchlorid-Lösung gewaschen, über Magnesiumsulfat getrocknet und eingeengt. Ausbeute: 2.92 g (99% d. Th., 70% Reinheit)

LC-MS (Methode 1B): Rₜ = 2.02 min; MS (ESIpos): m/z = 425 [M+H]⁺.

### Beispiel 117A

### 1-[(4-Cyanpiperidin-1-yl)carbonyl)-5-[4-(trifluormethoxy)phenyl]piperidin-3-carbothioamid [racemisches cis-/trans-Isomerengemisch]

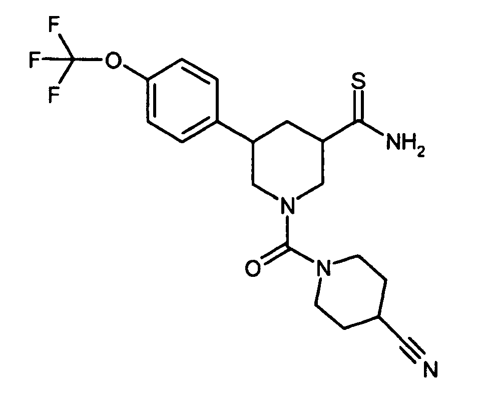

Nach der Allgemeinen Methode 10A wurden 1.50 g (3.53 mmol) der Verbindung aus Beispiel 116A und 786 mg (1.94 mmol) Lawesson-Reagenz umgesetzt. Ausbeute: 1.34 mg (39% d. Th., 45% Reinheit)

LC-MS (Methode 9B): Rₜ = 1.04 min und 1.05 (cis-/trans-Isomere); MS (ESIpos): m/z = 441 [M+H]⁺.

### Beispiel 118A

### 1-(Morpholin-4-ylcarbonyl)-5-[4-(trifluormethoxy)phenyl]piperidin-3-carboxamid

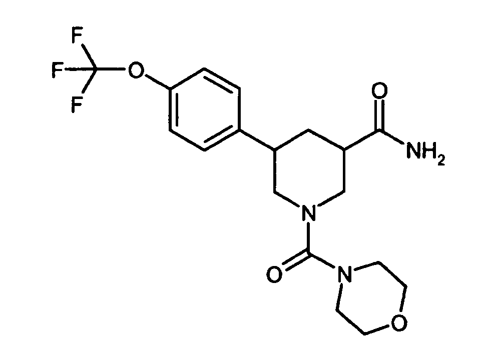

Zu einer Lösung von 3.0 g (4.4 mmol) der Verbindung aus Beispiel 44A in 58 ml Dichlormethan wurden 6.34 g (119.0 mmol) Thionylchlorid hinzugegeben. Die Mischung wurde 2 h bei Rückflußtemperatur gerührt. Zur Aufarbeitung wurde im Vakuum Dichlormethan entfernt, der Rückstand noch einmal mit Dichlormethan versetzt und eingeengt. Der Rückstand wurde in 58 ml Dichlormethan vorgelegt und auf 0°C gekühlt. Anschließend wurden 3.02 ml (21.1 mmol) einer 7M Ammoniak-Lösung in Methanol zugegeben. Das Reaktionsgemisch wurde 4 h bei 0°C gerührt und 3 Tage bei RT. Zur Aufarbeitung wurde das Lösungsmittel im Vakuum entfernt und der Rückstand in Essigsäureethylester aufgenommen. Die organische Phase wurde nacheinander mit Wasser und gesättigter, wässriger Natriumchlorid-Lösung gewaschen, über Magnesiumsulfat getrocknet und eingeengt. Ausbeute: 820 mg (92% d. Th.).

LC-MS (Methode 2B): Rₜ = 1.03 min; MS (ESIpos): m/z = 402 [M+H]⁺.

### Beispiel 119A

### Methyl-1-[(3-methoxypiperidin-1-yl)carbonyl]-5-[4-(trifluormethyl)phenyllpiperidin-3-carboxylat [racemisches cis-/trans-Isomerengemisch]

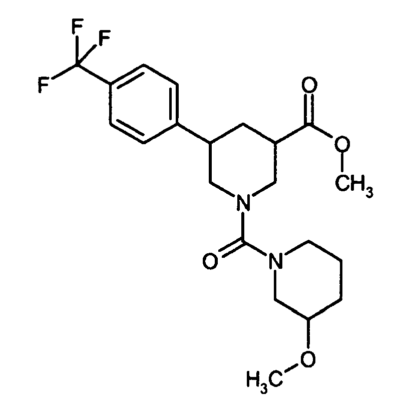

Nach der Allgemeinen Methode 8A wurden 4.00 g (8.84 mmol) 3-Methyl-1-(4-nitrophenyl)-5-[4-(trifluormethyl)phenyl]piperidin-1,3-dicarboxylat und 3.35 g (22.1 mmol) 3-Methoxypiperidin Hydrochlorid umgesetzt. Ausbeute: 2.90 g (57% d. Th., 74% Reinheit).

LC-MS (Methode 2B): Rₜ = 1.31 min und 1.34 min (cis-/trans-Isomere); MS (ESIpos): m/z = 429 [M+H]⁺.

### Beispiel 120A

### N'-(3-Methoxypropanoyl)-1-(morpholin-4-ylcarbonyl)-5-[4-(trifluormethoxy)phenyl]piperidin-3-carbohydrazid [racemisches cis-/trans-Isomerengemisch]

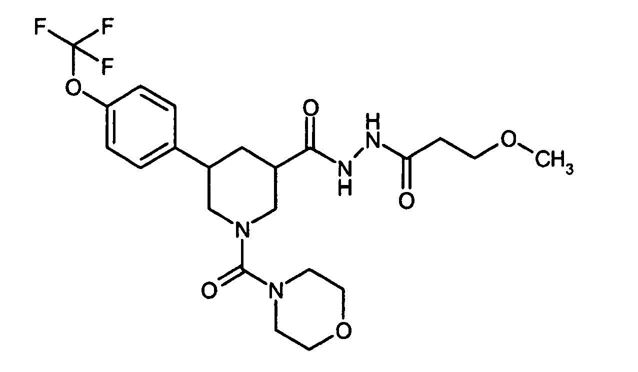

Nach der Allgemeinen Methode 11A wurden 100 mg (0.25 mmol) der Verbindung aus Beispiel 44A und 58 mg (0.50 mmol) 3-Methoxypropanhydrazid umgesetzt. Ausbeute: 93 mg (75% d. Th.)

LC-MS (Methode 2B): Rₜ = 1.02 min; MS (ESIpos): m/z = 503 [M+H]⁺.

### Beispiel 121A

### N'-Acetyl-1-(morpholin-4-ylcarbonyl)-5-[4-(trifluormethoxy)phenyl]piperidin-3-carbohydrazid [racemisches cis-/trans-Isomerengemisch]

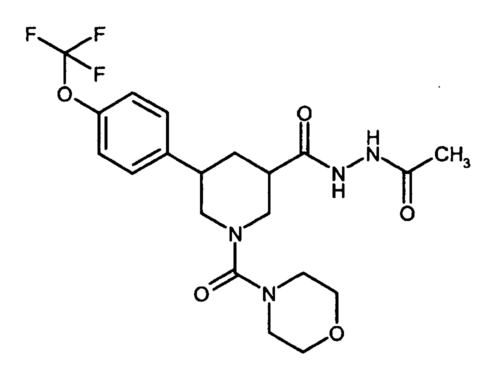

Nach der Allgemeinen Methode 11A wurden 100 mg (0.25 mmol) der Verbindung aus Beispiel 44A und 37 mg (0.50 mmol) Acetohydrazid umgesetzt. Ausbeute: 106 mg (93% d. Th.)

LC-MS (Methode 2B): Rₜ = 0.99 min; MS (ESIpos): m/z = 459 [M+H]⁺.

### Beispiel 122A

### N'-(2-Methylpropanoyl)-1-(morpholin-4-ylcarbonyl)-5-[4-(trifluormethoxy)phenyl]piperidin-3-carbohydrazid [racemisches cis-/trans-Isomerengemisch]

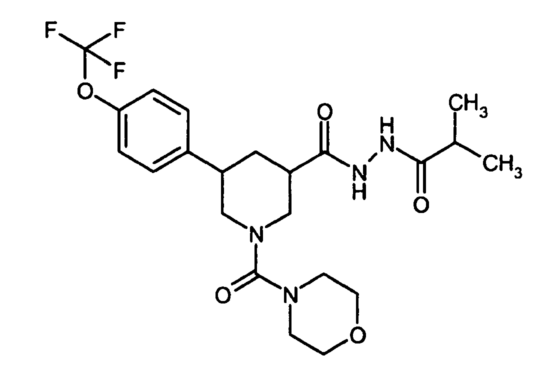

Nach der Allgemeinen Methode 11A wurden 100 mg (0.25 mmol) der Verbindung aus Beispiel 44A und 51 mg (0.50 mmol) 2-Methylpropanhydrazid umgesetzt. Ausbeute: 80 mg (66% d. Th.)

LC-MS (Methode 2B): Rₜ = 1.07 min; MS (ESIpos): m/z = 487 [M+H]⁺.

### Beispiel 123A

### 1-(Morpholin-4-ylcarbonyl)-N'-(phenylcarbonyl)-5-[4-(trifluormethoxy)phenyl]piperidin-3-carbohydrazid [racemisches cis-/trans-Isomerengemisch]

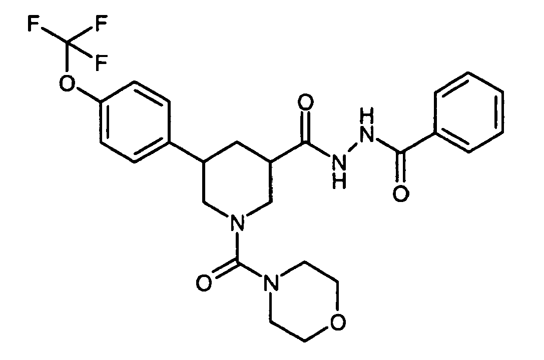

Nach der Allgemeinen Methode 11A wurden 100 mg (0.25 mmol) der Verbindung aus Beispiel 44A und 65 mg (0.50 mmol) Benzolcarbohydrazid umgesetzt.

LC-MS (Methode 2B): Rₜ = 1.12 min; MS (ESIpos): m/z = 521 [M+H]⁺.

### Beispiel 124A

### 5-(3,4-Dimethylphenyl)pyridin-3-carbonsäuremethylester

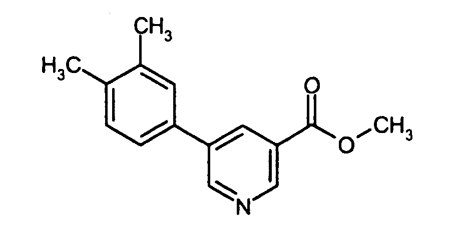

Nach der Allgemeinen Methode 1A wurden 11.0 g (50.1 mmol) 5-Bromnikotinsäuremethylester mit 7.7 g (50.1 mmol) 3,4-Dimethylphenylboronsäure umgesetzt. Ausbeute: 4.0 g (31% d. Th.)

LC-MS (Methode 3B): Rₜ = 2.05 min; MS (ESIpos): m/z = 242 [M+H]⁺.

¹H-NMR (400 MHz, DMSO-d₆): δ = 9.10 (d, 1H), 9.04 (d, 1H), 8.43 (dd, 1H), 7.59 (s, 1H), 7.52-7.50 (m, 1H), 7.28 (d, 1H), 3.92 (s, 3H), 2.32 (s, 3H), 2.28 (s, 3H).

### Beispiel 125A

### 5-(3,4-Dimethylphenyl)piperidin-3-carbonsäuremethylester [racemisches cis-/trans-Isomerengemisch]

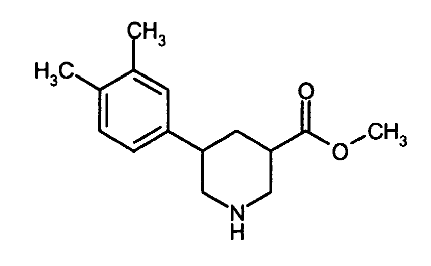

Nach der Allgemeinen Methode 2A wurden 9.1 g (37.8 mmol) der Verbindung aus Beispiel 124A umgesetzt. Ausbeute: 5.5 g (58% d. Th.)

LC-MS (Methode 5B): Rₜ = 1.39 min und 1.43 min (cis-/trans-Isomere); MS (ESIpos): m/z = 247 [M+H]⁺.

### Beispiel 126A

### 3-Methyl-1-(4-nitrophenyl)-5-(3,4-dimethylphenyl)piperidin-1,3-dicarboxylat [racemische cis-/trans-Isomerengemisch]

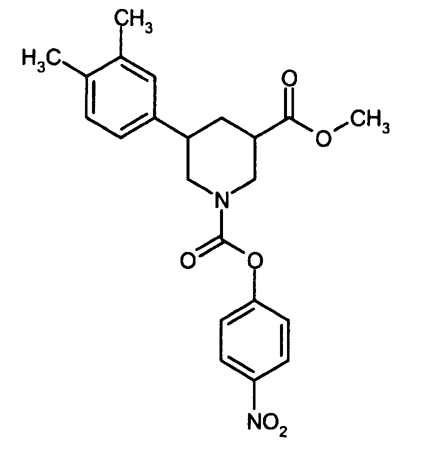

4.0 g (16.2 mmol) der Verbindung aus Beispiel 125A wurden in 80 ml Dichlormethan vorgelegt, auf 0°C abgekühlt und mit 4.5 ml (3.3 g, 32.3 mmol) Triethylamin sowie 3.3 g (16.2 mmol) Chlorameisensäure-4-nitrophenylester versetzt. Das Reaktionsgemisch ließ man langsam auf RT erwärmen. Es wurde zweimal mit Wasser gewaschen, über Natriumsulfat getrocknet, filtriert und im Vakuum eingeengt. Der Rückstand wurde mit Methanol versetzt, filtriert, erneut im Vakuum eingeengt und mittels präparativer HPLC aufgereinigt. Ausbeute: 4.0 g (53% d. Th., 89% Reinheit)

LC-MS (Methode 2B): Rₜ = 1.49 min; MS (ESIpos): m/z = 413 [M+H]⁺.

### Beispiel 127A

### 5-(3,4-Dimethylphenyl)-1-[(4-hydroxypiperidin-1-yl)carbonyl]piperidin-3-carbonsäuremethylester [racemisches cis-/trans-Isomerengemisch]

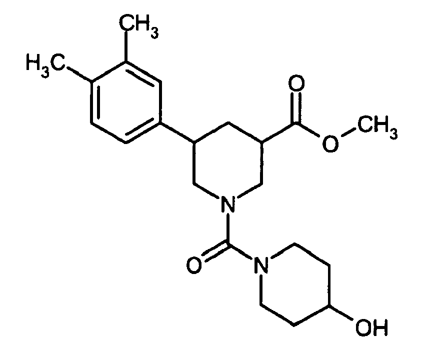

1.5 g (3.6 mmol) der Verbindung aus Beispiel 126A, 1.1 g (10.9 mmol) 4-Hydroxypiperidin und 0.5 g (3.6 mmol) Kaliumcarbonat wurden in 35 ml *N,N*-Dimethylformamid vorgelegt und 15 min bei 150°C in einer *Single Mode*-Mikrowelle (Emrys Optimizer) umgesetzt. Das Rohprodukt wurde mittels präparativer HPLC aufgereinigt. Ausbeute: 0.9 g (56% d. Th., 82% Reinheit)

LC-MS (Methode 3B): Rₜ = 1.68 min und 1.74 [cis-/trans-Isomere]; MS (ESIpos): m/z = 375 [M+H]⁺.

### Beispiel 128A

### 5-(3,4-Dimethylphenyl)-1-[(4-hydroxypiperidin-1-yl)carbonyl]piperidin-3-carbonsäure [racemisches cis-Isomer]

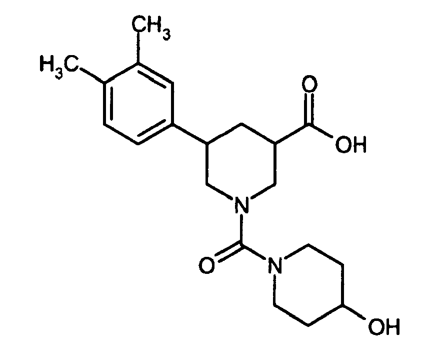

0.9 g (2.5 mmol) der Verbindung aus Beispiel 127A wurden nach der Allgemeinen Methode 9A umgesetzt. Ausbeute: 0.9 g (92% d. Th.)

LC-MS (Methode 5B): Rₜ = 1.82 min; MS (ESIpos): m/z = 361 [M+H]⁺.

¹H-NMR (400 MHz, DMSO-d₆): δ = 12.42 (br s, COOH), 7.07-7.03 (m, 2H), 6.97 (d, 1H), 4.66 (d, OH), 3.79 (br d, 1H), 3.58 (br d, 1H), 3.50-3.37 (m, 3H), 2.85 (t, 2H), 2.76-2.62 (m, 3H), 2.58-2.52 (m, 1H), 2.20 (s, 3H), 2.18 (s, 3H), 2.08 (br d, 1H), 1.73-1.64 (m, 3H), 1.36-1.26 (m, 2H).

### Beispiel 129A

### 5-(3,4-Dimethylphenyl)-1-(morpholin-4-ylcarbonyl)piperidin-3-carbonsäuremethylester [racemisches cis-/trans-Isomerengemisch]

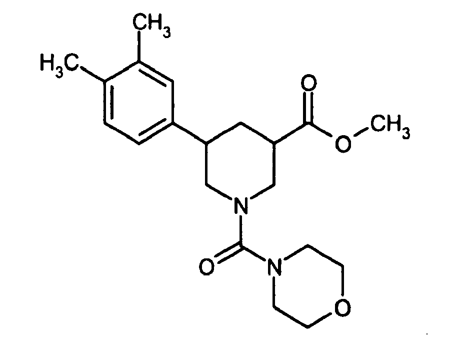

1.5 g (6.1 mmol) der Verbindung aus Beispiel 125A wurden nach der Allgemeinen Methode 3A umgesetzt. Ausbeute: 0.6 g (28% d. Th.)

LC-MS (Methode 2B): Rₜ = 1.17 min; MS (ESIpos): m/z = 361 [M+H]⁺.

### Beispiel 130A

### 5-(3,4-Dimethylphenyl)-1-(morpholin-4-ylcarbonyl)piperidin-3-carbonsäure [racemisches cis-Isomer]

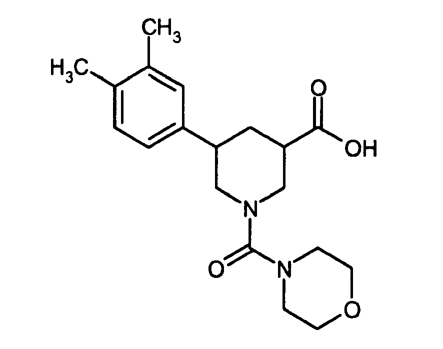

1.6 g (4.4 mmol) der Verbindung aus Beispiel 129A wurden nach der Allgemeinen Methode 9A umgesetzt. Ausbeute: 1.6 g (99% d. Th.)

LC-MS (Methode 2B): Rₜ = 1.05 min; MS (ESIpos): m/z = 347 [M+H]⁺.

¹H-NMR (400 MHz, DMSO-d₆): δ = 12.44 (br s, COOH), 7.08-7.02 (m, 2H), 6.96 (d, 1H), 3.84 (br d, 1H), 3.56-3.54 (m, 5H), 3.15-3.13 (m, 4H), 2.82-2.73 (m, 2H), 2.68-2.61 (m, 1H), 2.58-2.54 (m, 1H), 2.20 (s, 3H), 2.18 (s, 3H), 2.09 (br d, 1H), 1.69 (q, 1H).

### Beispiel 131A

### 1-[(4-Cyanpiperidin-1-yl)carbonyl]-5-(3,4-dimethylphenyl)piperidin-3-carbonsäuremethylester [racemisches cis-/trans-Isomerengemisch]

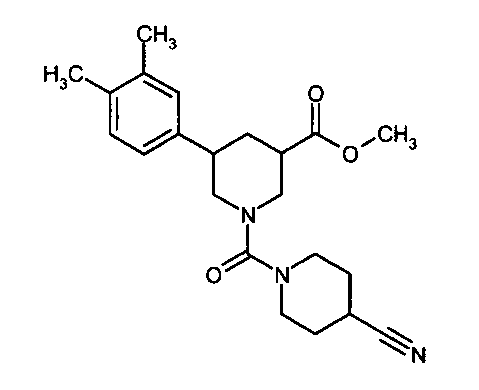

2.0 g (4.3 mmol) der Verbindung aus Beispiel 126A, 1.4 g (12.8 mmol) Piperidin-4-carbonitril und 0.6 g (4.3 mmol) Kaliumcarbonat wurden in 41 ml *N,N*-Dimethylformamid vorgelegt und 15 min bei 150°C in einer *Single Mode*-Mikrowelle (Emrys Optimizer) umgesetzt. Das Rohprodukt wurde mittels präparativer HPLC aufgereinigt. Ausbeute: 0.5 g (32% d. Th.)

LC-MS (Methode 2B): Rₜ = 1.23 min und 1.26 [cis-/trans-Isomere]; MS (ESIpos): m/z = 384 [M+H]⁺.

### Beispiel 132A

### 1-[(4-Cyanpiperidin-1-yl)carbonyl]-5-(3,4-dimethylphenyl)piperidin-3-carbonsäure [racemisches cis-Isomer]

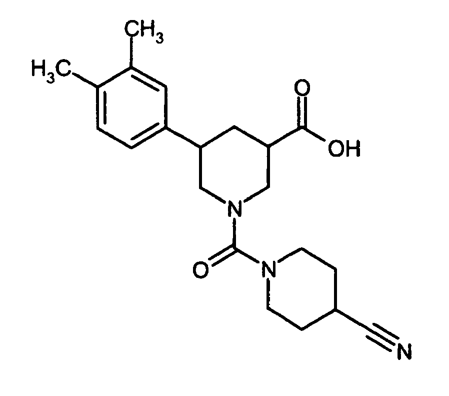

1.2 g (3.1 mmol) der Verbindung aus Beispiel 131A wurden nach der Allgemeinen Methode 9A umgesetzt. Ausbeute: 1.1 g (92% d. Th.)

LC-MS (Methode 1B): Rₜ = 2.12 min; MS (ESIpos): m/z = 370 [M+H]⁺.

### Beispiel 133A

### 5-(3,4-Dimethylphenyl)-1-[(3-hydroxyazetidin-1-yl)carbonyl]piperidin-3-carbonsäuremethylester [racemisches cis-/trans-Isomerengemisch]

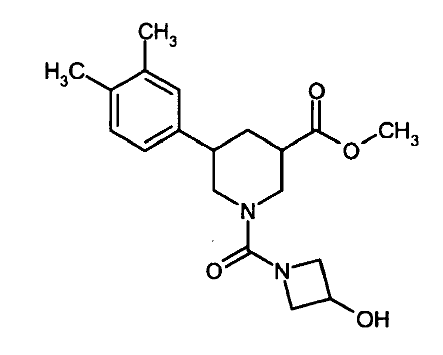

2.0 g (4.3 mmol) der Verbindung aus Beispiel 126A, 1.4 g (12.8 mmol) 3-Hydroxyazetidin-Hydrochlorid und 3.5 g (25.6 mmol) Kaliumcarbonat wurden in 41 ml *N,N*-Dimethylformarmid vorgelegt und 15 min bei 150°C in einer *Single Mode*-Mikrowelle (Emrys Optimizer) umgesetzt. Das Rohprodukt wurde mittels präparativer HPLC aufgereinigt. Ausbeute: 0.9 g (54% d. Th.)

LC-MS (Methode 2B): Rₜ = 1.94 min und 1.99 [cis-/trans-Isomere]; MS (ESIpos): m/z = 347 [M+H]⁺.

### Beispiel 134A

### 5-(3,4-Dimethylphenyl)-1-[(3-hydroxyazetidin-1-yl)carbonyl]piperidin-3-carbonsäure [racemisches cis-Isomer]

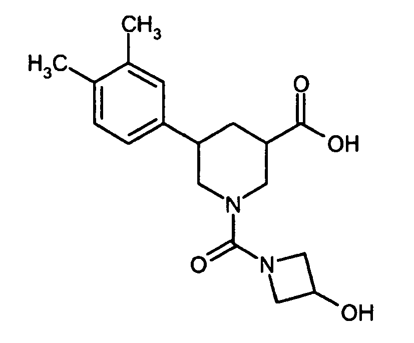

1.0 g (2.8 mmol) der Verbindung aus Beispiel 133A wurden nach der Allgemeinen Methode 9A umgesetzt. Ausbeute: 0.8 g (84% d. Th.)

LC-MS (Methode 3B): Rₜ = 1.40 min; MS (ESIpos): m/z = 333 [M+H]⁺.

¹H-NMR (400 MHz, DMSO-d₆): δ = 12.44 (br s, COOH), 7.08-7.04 (m, 2H), 6.96 (d, 1H), 5.55 (d, OH), 4.39-4.33 (m, 1H), 4.06-3.97 (m, 3H), 3.67-3.56 (m, 3H), 2.73 (q, 2H), 2.61-2.43 (m, 2H, tw. unter DMSO-Signal), 2.20 (s, 3H), 2.18 (s, 3H), 2.07 (br d, 1H), 1.71 (q, 1H).

### Beispiel 135A

### 3-Methyl-1-(4-nitrophenyl)-5-(4-ethylphenyl)piperidin-1,3-dicarboxylat [racemisches cis-/trans-Isomerengemisch]

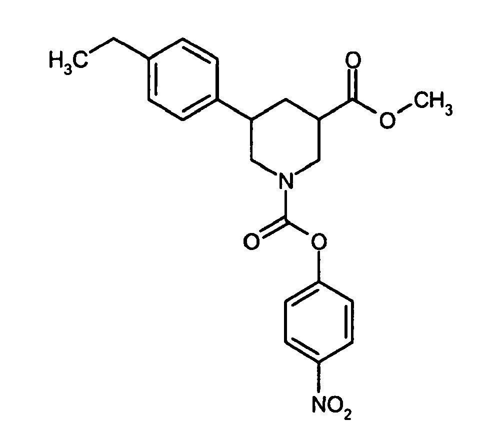

3.0 g (12.1 mmol) der Verbindung aus Beispiel 2A wurden in 30 ml Dichlormethan vorgelegt, auf 0°C abgekühlt und mit 3.4 ml (2.4 g, 12.1 mmol) Triethylamin sowie 2.4 g (12.1 mmol) Chlorameisensäure-4-nitrophenylester versetzt. Das Reaktionsgemisch ließ man langsam auf RT erwärmen und rührte 16 h bei RT. Es wurde mehrmals mit Wasser gewaschen, über Natriumsulfat getrocknet, filtriert und im Vakuum eingeengt. Der Rückstand wurde säulenchromatographisch an Kieselgel aufgereinigt (Laufmittel Dichlormethan → Dichlormethan/Methanol 100:2). Ausbeute: 4.7 g (83% d. Th., 89% Reinheit)

HPLC (Methode 1A): Rₜ = 4.94 min und 5.00 min (cis-/trans-Isomer); MS (ESIpos): m/z = 413 [M+H]⁺.

### Beispiel 136A

### 1-[(4-Cyanpiperidin-1-yl)carbonyl]-5-(4-ethylphenyl)piperidin-3-carbonsäuremethylester [racemisches cis-/trans-Isomerengemisch]

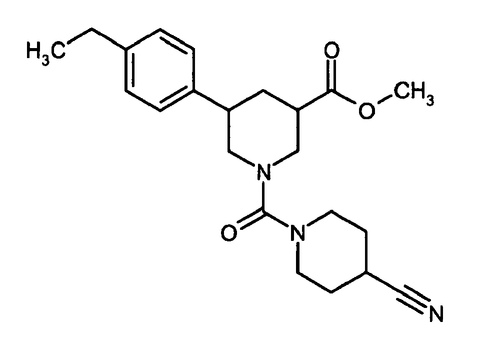

3.1 g (7.5 mmol) der Verbindung aus Beispiel 135A, 2.5 g (22.4 mmol) Piperidin-4-carbonitril und 2.1 g (15.0 mmol) Kaliumcarbonat wurden in 56 ml *N,N*-Dimethylformamid vorgelegt und 30 min bei 150°C in einer *Single Mode*-Mikrowelle (Emrys Optimizer) umgesetzt. Das Reaktionsgemisch wurde in Essigsäureethylester aufgenommen, mehrmals mit Wasser und gesättigter, wässriger Natriumchlorid-Lösung gewaschen. Die organische Phase wurde über Magnesiumsulfat getrocknet, filtriert und im Vakuum eingeengt. Der Rückstand wurde säulenchromatographisch an Kieselgel aufgereinigt (Laufmittel: Cyclohexan/Essigsäureethylester 1:1). Ausbeute: 2.4 g (73% d. Th.)

HPLC (Methode 1A): Rₜ = 4.42 min und 4.49 min [cis-/trans-Isomere]; MS (ESIpos): m/z = 384 [M+H]⁺.

### Beispiel 137A

### 5-(4-Ethylphenyl)-1-[(3-hydroxyazetidin-1-yl)carbonyl]piperidin-3-carbonsäuremethylester [racemisches cis-/trans-Isomerengemisch]

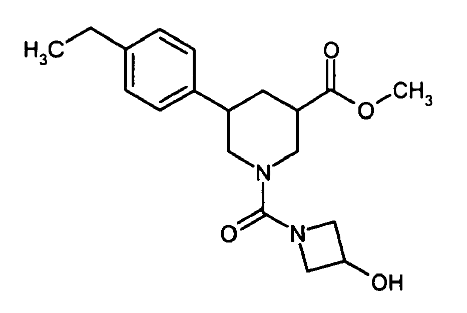

0.3 g (0.7 mmol) der Verbindung aus Beispiel 135A, 0.2 g (2.18 mmol) 3-Hydroxyazetidin-Hydrochlorid und 0.2 g (1.4 mmol) Kaliumcarbonat wurden in 6 ml *N,N*-Dimethylformamid vorgelegt und 30 min bei 150°C in einer *Single Mode*-Mikrowelle (Emrys Optimizer) umgesetzt. Das Rohprodukt wurde mittels präparativer HPLC aufgereinigt. Ausbeute: 21 mg (8% d. Th.)

LC-MS (Methode 3B): Rₜ = 1.76 min und 1.85 [cis-/trans-Isomere]; MS (ESIpos): m/z = 361 [M+H]⁺.

### Beispiel 138A

### 5-(4-Ethylphenyl)-1-[(3-hydroxyazetidin-1-yl)carbonyl]piperidin-3-carbonsäure [racemisches cis-Isomer]

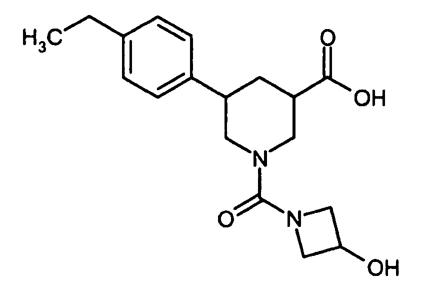

300 mg (0.83 mmol) der Verbindung aus Beispiel 137A wurden nach der Allgemeinen Methode 9A umgesetzt. Ausbeute: 250 mg (90% d. Th.)

LC-MS (Methode 3B): Rₜ = 1.44 min; MS (ESIpos): m/z = 333 [M+H]⁺.

¹H-NMR (400 MHz, DMSO-d₆): δ = 12.42 (br s, COOH), 7.18-7.13 (m, 4H), 5.54 (br s, OH), 4.39-4.33 (m, 1H), 4.08-3.97 (m, 3H), 3.68-3.62 (m, 3H), 2.78-2.70 (m, 2H), 2.68-2.54 (m, 3H), 2.48-2.42 (m, 1H), 2.08 (br d, 1H), 1.71 (q, 1H), 1.15 (t, 3H).

### Bespiel 139A

### 5-(4-Ethylphenyl)-1-(morpholin-4-ylcarbonyl)piperidin-3-carboxamid [racemisches cis-Isomerengemisch]

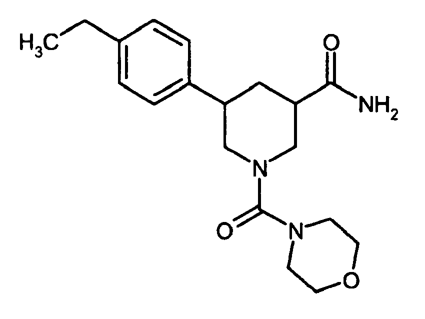

Zu einer Lösung von 4.80 g (13.39 mmol) der Verbindung aus Beispiel 38A in 180 ml Dichlormethan wurden 4.77 g (40.15 mmol) Thionylchlorid hinzugegeben. Die Mischung wurde 2 h unter Rückfluß gerührt. Zur Aufarbeitung wurde im Vakuum Dichlormethan entfernt, der Rückstand noch einmal mit Dichlormethan versetzt und eingeengt. Der Rückstand wurde in 55 ml Tetrahydrofuran vorgelegt und auf 0°C gekühlt. Anschließend wurden 17 ml (133.4 mmol) einer 7M Ammoniak-Lösung in Methanol zugegeben. Das Reaktionsgemisch wurde 1 h bei 0°C und dann über Nacht bei RT gerührt. Zur Aufarbeitung wurde das Lösungsmittel im Vakuum entfernt und der Rückstand in Essigsäureethylester aufgenommen. Die organische Phase wurde nach einander mit Wasser und gesättigter, wässriger Natriumchlorid-Lösung gewaschen, über Magnesiumsulfat getrocknet und eingeengt. Ausbeute: 3.74 g (82% d. Th.).

LC-MS (Methode 1B): Rₜ = 1.89 min; MS (ESIpos): m/z = 346 [M+H]⁺;

¹H-NMR (400 MHz, DMSO-d₆): δ = 7.39 (br s, 1H), 7.18 (d, 2H), 7.15 (d, 2H), 6.89 (br s, 1H), 3.70 (br d, 1H), 3.60-3.52 (5H), 3.20-3.09 (m, 4H), 2.83-2.64 (m, 3H), 2.57 (q, 2H), 2.44 (tt, 1H), 1.98 (br d, 1H), 1.73 (q, 1H), 1.16 (t, 3H).

### Beispiel 140A

### 5-(4-Ethylphenyl)-1-(morpholin-4-ylcarbonyl)piperidin-3-carbothioamid [racemisches cis-Isomerengemisch]

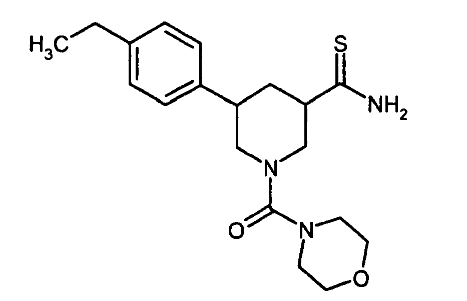

Nach der Allgemeinen Methode 10A wurden 910 mg (2.63 mmol) 5-(4-Ethylphenyl)-1-(morpholin-4-ylcarbonyl)piperidin-3-carboamid (Beispiel 139A) und 586 mg (1.45 mmol) Lawesson-Reagens umgesetzt. Ausbeute: 849 mg (89% d. Th).

LC-MS (Methode 2B): Rₜ = 1.73 min; MS (ESIpos): m/z = 362 [M+H]⁺.

### Beispiel 141A

### 4-({3-[4-(Chlormethyl)-1,3-thiazol-2-yl]-5-(4-ethylphenyl)piperidin-1-yl}carbonyl)morpholin [racemisches cis-/trans-Isomerengemisch]

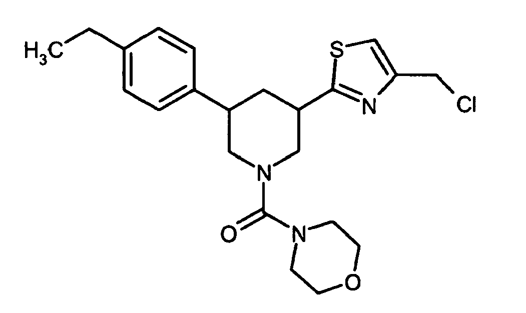

Nach der Allgemeinen Methode 3 wurden 860 mg (2.38 mmol) 5-(4-Ethylphenyl)-1-(morpholin-4-ylcarbonyl)piperidin-3-carbothioamid (Beispiel 140A) und 362 mg (2.85 mmol) Dichloraceton umgesetzt. Ausbeute: 498 mg (43 % d. Th.).

LC-MS (Methode 5B): Rₜ = 2.36 min und 2.44 min (cis/trans-Isomere); MS (ESIpos): m/z = 434 [M+H]⁺.

### Beispiel 142A

### Ethyl-2-[5-(4-ethylphenyl)-1-(morpholin-4-ylcarbonyl)piperidin-3-yl]-1,3-thiazol-4-carbonsäure-ethylester [racemisches cis-/trans-Isomerengemisch]

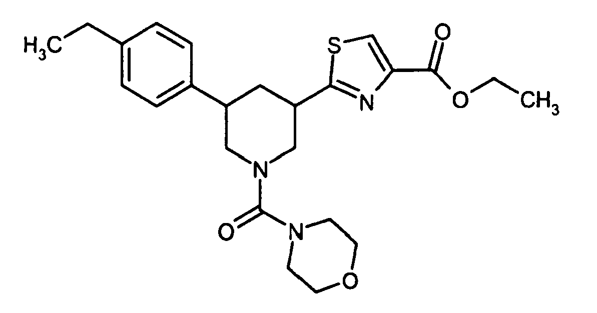

Nach der Allgemeinen Methode 3 wurden 850 mg der Verbindung aus Beispiel 140A und 550 mg (2.82 mmol) Ethyl-3-brom-oxopropanoat umgesetzt. Ausbeute: 150 mg (12 % d. Th.).

LC-MS (Methode 2B): Rₜ = 1.33 min; MS (ESIpos): m/z = 458 [M+H]⁺.

### Beispiel 143A

### 2-[5-(4-Ethylphenyl)-1-(morpholin-4-ylcarbonyl)piperidin-3-yl]-1,3-thiazol-4-carbonsäure [racemisches cis-/ trans-Isomerengemisch]

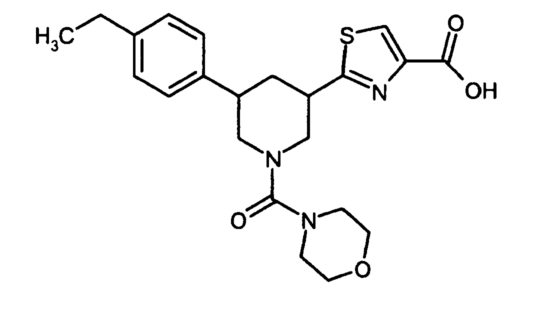

Nach der Allgemeinen Methode 4A wurden 650 mg (1.42 mmol) der Verbindung aus Beispiel 142A umgesetzt. Ausbeute: 590 mg (97% d. Th.)

LC-MS (Methode 3B): Rₜ = 1.67 min und 1.76 min (cis-/trans-Isomere); MS (ESIpos): m/z = 430 [M+H]⁺.

### Beispiel 144A

### 5-[3-(Trifluormethyl)phenyl)pyridin-3-carbonsäuremethylester

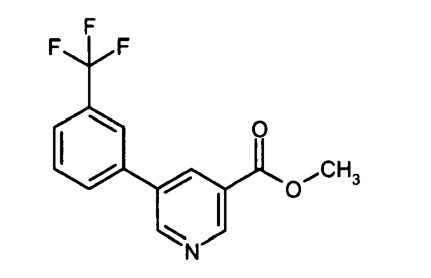

Nach der Allgemeinen Methode 1A wurden 10.0 g (46.3 mmol) 5-Bromnikotinsäuremethylester mit 10.6 g (55.5 mmol) 3-Trifluormethylphenylboronsäure umgesetzt. Ausbeute: 11.9 g (91% d. Th.)

LC-MS (Methode 2B): Rₜ = 1.25 min; MS (ESIpos): m/z = 282 [M+H]⁺;

¹H-NMR (400 MHz, DMSO-d₆): δ = 9.22 (d, 1H), 9.12 (d, 1H), 8.57 (dd, 1H), 8.16-8.12 (m, 2H), 7.84 (d, 1H), 7.79-7.75 (m, 1H), 3.94 (s, 3H).

### Beispiel 145A

### 5-[3-(Trifluormethyl)phenyl]piperidin-3-carbonsäuremethylester [racemisches cis-/trans-Isomerengemisch]

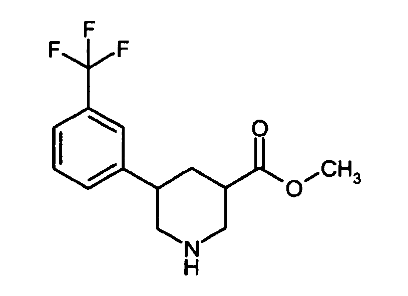

Nach der Allgemeinen Methode 2A wurden 11.0 g (39.1 mmol) der Verbindung aus Beispiel 144A umgesetzt. Ausbeute: 9.3 g (79% d. Th.)

LC-MS (Methode 2B): Rₜ = 0.83 min und 0.85 min (cis-/trans-Isomere); MS (ESIpos): m/z = 247 [M+H]⁺.

### Beispiel 146A

### 1-(Morpholin-4-ylcarbonyl)-5-[3-(trifluormethyl)phenyl]piperidin-3-carbonsäuremethylester [racemisches cis-/trans-Isomerengemisch]

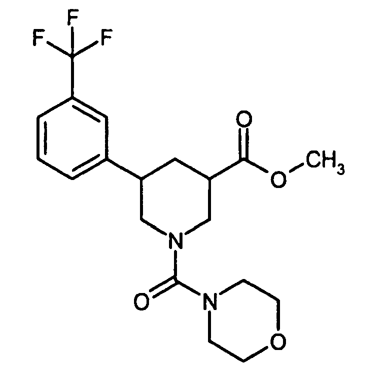

7.0 g (24.4 mmol) der Verbindung aus Beispiel 145A wurden nach der Allgemeinen Methode 3A umgesetzt. Ausbeute: 9.8 g (94% d. Th.)

LC-MS (Methode 1B): Rₜ = 2.24 min; MS (ESIpos): m/z = 401 [M+H]⁺.

### Beispiel 147A

### 1-(Morpholin-4-ylcarbonyl)-5-[3-(trifluormethyl)phenyl]piperidin-3-carbonsäure [racemisches cis-Isomer]

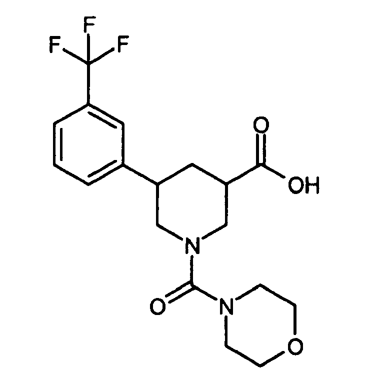

9.8 g (24.5 mmol) der Verbindung aus Beispiel 146A wurden nach der Allgemeinen Methode 9A umgesetzt. Ausbeute: 8.7 g (92% d. Th.)

LC-MS (Methode 2B): Rₜ = 1.06 min; MS (ESIpos): m/z = 387 [M+H]⁺;

¹H-NMR (400 MHz, DMSO-d₆): δ = 12.48 (br s, COOH), 7.67-7.55 (m, 4H), 3.83 (br d, 1H), 3.61-3.54 (m, 5H), 3.17-3.11 (m, 4H), 2.91-2.82 (m, 3H), 2.62-2.55 (m, 1H), 2.14 (br d, 1H), 1.79 (q, 1H).

### Beispiel 148A

### 3-Methyl-1-(4-nitrophenyl)-5-[3-(trifluormethyl)phenyl]piperidin-1,3-dicarboxylat [racemisches cis-/trans-Isomerengemisch]

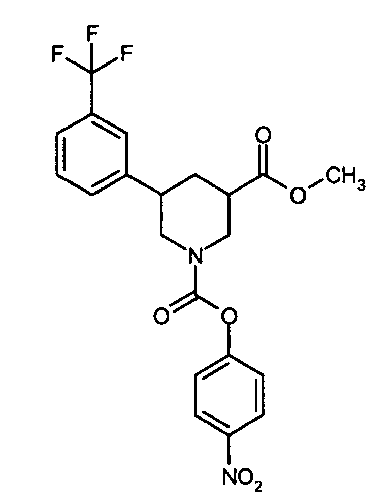

5.0 g (16.2 mmol) der Verbindung aus Beispiel 145A wurden in 80 ml Dichlormethan vorgelegt, auf 0°C abgekühlt und mit 4.5 ml (3.3 g, 32.5 mmol) Triethylamin sowie 3.3 g (16.2 mmol) Chlorameisensäure-4-nitrophenylester versetzt. Das Reaktionsgemisch ließ man langsam auf RT erwärmen. Es wurde zweimal mit Wasser gewaschen, über Natriumsulfat getrocknet, filtriert und im Vakuum eingeengt. Ausbeute: 6.4 g (78% d. Th., 89% Reinheit)

LC-MS (Methode 2B): Rₜ = 1.44 min und 1.46 min (cis-/trans-Isomer); MS (ESIpos): m/z = 453 [M+H]⁺.

### Beispiel 149A

### 1-[(4-Hydroxypiperidin-1-yl)carbonyl]-5-[3-trifluormethyl)phenyl]piperidin-3-carbonsäuremethyl ester [racemisches cis-/trans-Isomerengemisch]

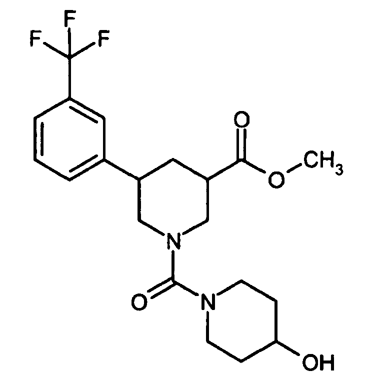

2.2 g (4.3 mmol) der Verbindung aus Beispiel 148A, 1.3 g (13.0 mmol) 4-Hydroxypiperidin und 0.6 g (4.3 mmol) Kaliumcarbonat wurden in 42 ml *N,N*-Dimethylformamid vorgelegt und 15 min bei 150°C in einer *Single Mode*-Mikrowelle (Emrys Optimizer) umgesetzt. Das Reaktionsgemisch wurde mittels präparativer HPLC aufgereinigt. Ausbeute: 1.0 g (57% d. Th.)

LC-MS (Methode 3B): Rₜ = 1.70 min und 1.76 min [cis-/trans-Isomere]; MS (ESIpos): m/z = 415 [M+H]⁺.

### Beispiel 150A

### 1-[(4-Hydroxypiperidin-1-yl)carbonyl]-5-[3-(trifluormethyl)phenyl]piperidin-3-carbonsäure [racemisches cis-Isomer]

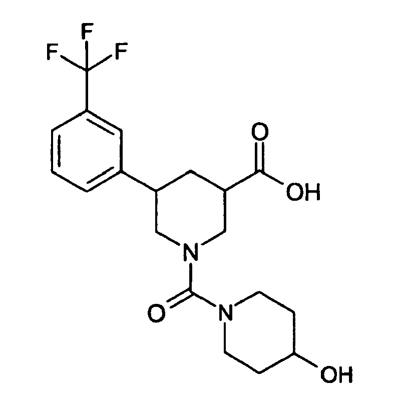

1.0 g (2.5 mmol) der Verbindung aus Beispiel 149A wurden nach der Allgemeinen Methode 9A umgesetzt. Ausbeute: 0.7 g (58% d. Th.)

LC-MS (Methode 2B): Rₜ = 0.99 min; MS (ESIpos): m/z = 401 [M+H]⁺;

¹H-NMR (400 MHz, CDCl₃): δ= 12.40 (br s, COOH), 7.64-7.55 (m, 4H), 3.79 (br d, 1H), 3.62-3.56 (m, 1H), 3.52 (br d, 1H), 3.46-3.43 (m, 2H), 2.96-2.82 (m, 5H), 2.61-2.56 (m, 1H), 2.13 (br d, 1H), 1.78 (q, 1H), 1.72-1.70 (m, 2H), 1.33-1.26 (m, 2H).

### Beispiel 151A

### 1-[(4-Cyanpiperidin-1-yl)carbonyl]-5-[3-(trifluormethyl)phenyl]piperidin-3-carbonsäuremethylester [racemisches cis-/trans-Isomerengemisch]

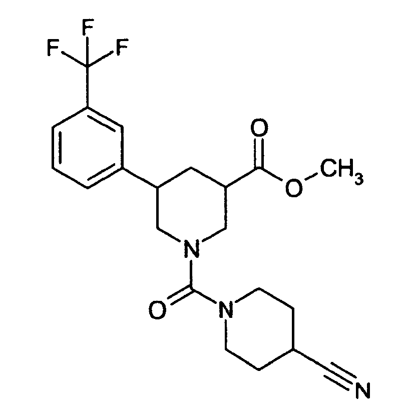

2.5 g (4.9 mmol) der Verbindung aus Beispiel 148A, 1.6 g (14.8 mmol) Piperidin-4-carbonitril und 0.7 g (4.9 mmol) Kaliumcarbonat wurden in 47 ml *N,N*-Dimethylformamid vorgelegt und 15 min bei 150°C in einer *Single Mode*-Mikrowelle (Emrys Optimizer) umgesetzt. Das Reaktionsgemisch wurde in Essigsäureethylester aufgenommen und mehrmals mit Wasser gewaschen. Die organische Phase wurde über Natriumsulfat getrocknet, filtriert und im Vakuum eingeengt. Der Rückstand wurde säulenchromatographisch an Kieselgel aufgereinigt (Laufmittel: Dichlormethan/Methanol 100 : 5). Das Rohprodukt wurde ohne weitere Aufreinigung weiter umgesetzt.

LC-MS (Methode 5B): Rₜ = 2.22 min und 2.28 min [cis-/trans-Isomere]; MS (ESIpos): m/z = 424 [M+H]⁺.

### Beispiel 152A

### 1-[(4-Cyanpiperidin-1-yl)carbonyl]-5-[3-(trifluormethyl)phenyl]piperidin-3-carbonsäure [racemisches cis-Isomer]

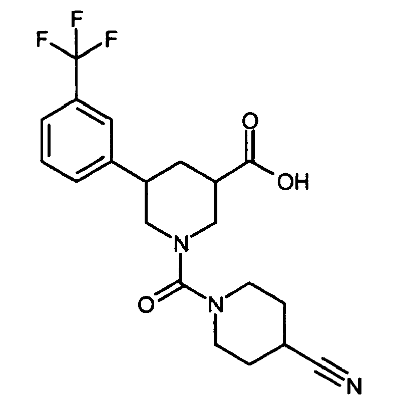

2.9 g (5.6 mmol) der Verbindung aus Beispiel 151A wurden nach der Allgemeinen Methode 9A umgesetzt. Ausbeute: 1.5 g (64% d. Th.)

LC-MS (Methode 2B): Rₜ = 1.12 min; MS (ESIpos): m/z = 410 [M+H]⁺.

¹H-NMR (400 MHz, CDCl₃): δ = 12.48 (br s, COOH), 7.66 (s, 1H), 7.62-7.54 (m, 3H), 3.79 (br d, 1H), 3.54 (br d, 1H), 3.36-3.32 (m, 3H), 3.07-3.02 (m, 3H), 2.91-2.81 (m, 2H), 2.62-2.56 (m, 1H), 2.14 (br d, 1H), 1.88-1.76 (m, 3H), 1.70-1.63 (m, 2H).

### Beispiel 153A

### 1-[(3-Hydroxyazetidin-1-yl)carbonyl]-5-[3-(trifluormethyl)phenyl]piperidin-3-carbonsäuremethylester [racemisches cis-/trans-Isomerengemisch]

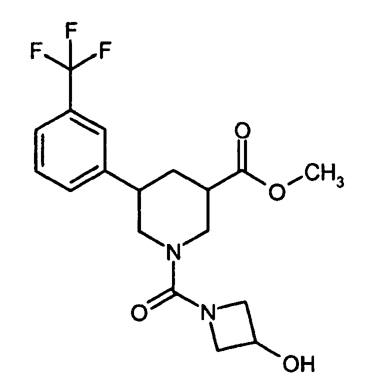

2.5 g (4.9 mmol) der Verbindung aus Beispiel 148A, 1.6 g (14.8 mmol) 3-Hydroxyazetidin Hydrochlorid und 1.4 g (9.8 mmol) Kaliumcarbonat wurden in 47 ml *N,N*-Dimethylformamid vorgelegt und 15 min bei 150°C in einer *Single Mode*-Mikrowelle (Emrys Optimizer) umgesetzt. Das Rohprodukt wurde mittels präparativer HPLC aufgereinigt. Ausbeute: 0.4 g (21% d. Th.)

LC-MS (Methode 5B): Rₜ = 1.93 min und 1.98 min [cis-/trans-Isomere]; MS (ESIpos): m/z = 387 [M+H]⁺.

### Beispiel 154A

### 1-[(3-Hydroxyazetidin-1-yl)carbonyl]-5-[3-(trifluormethyl)phenyl]piperidin-3-carbonsäuremethylester [racemisches cis-Isomer]

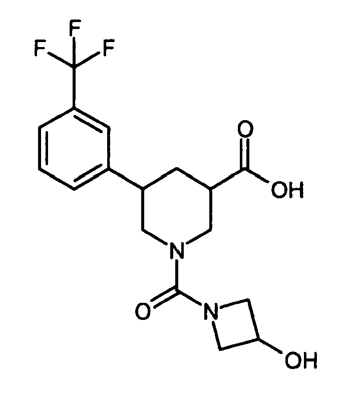

400 mg (1.01 mmol) der Verbindung aus Beispiel 153A wurden nach der Allgemeinen Methode 9A umgesetzt. Ausbeute: 340 mg (89% d. Th.)

LC-MS (Methode 2B): Rₜ = 0.97 min; MS (ESIpos): m/z = 373 [M+H]⁺.

¹H-NMR (400 MHz, DMSO-d₆): δ = 12.49 (br s, COOH), 7.66-7.55 (m, 4H), 5.56 (br s, OH), 4.41-4.34 (m, 1H), 4.05 (q, 2H), 3.97 (br d, 1H), 3.69-3.58 (m, 3H), 2.91-2.79 (m, 3H), 2.12 (br d, 1H), 1.85 (q, 1H).

### Beispiel 155A

### 1-[(3-Hydroxypyrrolidin-1-yl)carbonyl]-5-[3-(trifluormethyl)phenyl]piperidin-3-carbonsäuremethylester [Diastereomerengemisch]

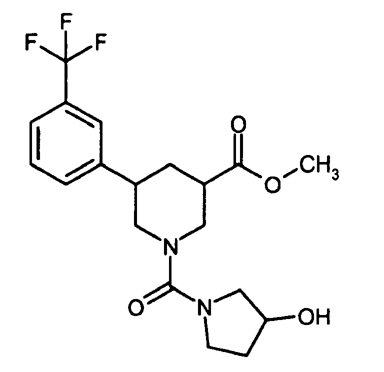

5.0 g (9.8 mmol) der Verbindung aus Beispiel 148A, 2.7 g (29.5 mmol) 3-Pyrrolidinol und 1.4 g (9.8 mmol) Kaliumcarbonat wurden in 95 ml *N,N*-Dimethylformamid vorgelegt und 15 min bei 150°C in einer *Single Mode*-Mikrowelle (Emrys Optimizer) umgesetzt. Das Reaktionsgemisch wurde mit Wasser versetzt und mit Essigsäureethylester extrahiert. Die vereinigten organischen Extrakte wurden über Natriumsulfat getrocknet, filtriert und im Vakuum eingeengt. Das Rohprodukt wurde säulenchromatographisch an Kieselgel aufgereinigt (Laufmittel Dichlormethan/Methanol 100:1 → 100:5). Ausbeute: 3.6 g (83% d. Th.)

LC-MS (Methode 9B): Rₜ = 0.96 min und 0.98 min; MS (ESIpos): m/z = 401 [M+H]⁺.

### Beispiel 156A

### 1-[(3-Hydroxypyrrolidin-1-yl)carbonyl]-5-[3-(trifluormethyl)phenyl]piperidin-3-carbonsäure [Diastereomerengemisch, cis-Isomer]

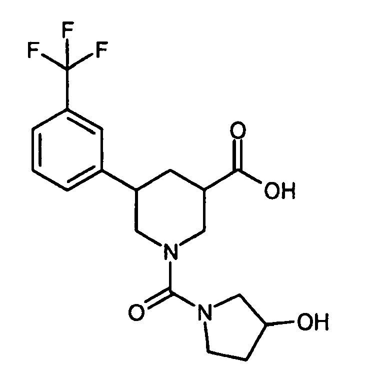

1.3 g (1.6 mmol) der Verbindung aus Beispiel 155A wurden nach der Allgemeinen Methode 9A umgesetzt. Ausbeute: 0.6 g (91% d. Th.)

LC-MS (Methode 2B): Rₜ = 1.00 min; MS (ESIpos): m/z = 387 [M+H]⁺.

### Beispiel 157A

### 5-[4-Methyl-3-(trifluormethyl)phenyl]pyridin-3-carbonsäuremethylester

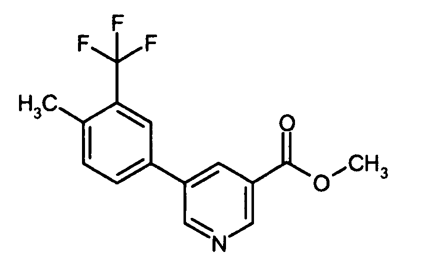

### Stufe a): [4-Methyl-3-(trifluormethyl)phenyl]boronsäure

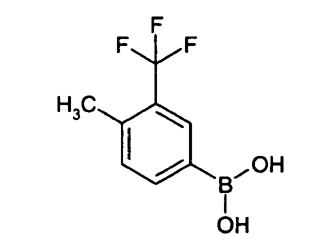

9.2 ml (1.5 g, 23.0 mmol) einer 2.5M Lösung von n-Butyllithium in Hexan wurden bei -78°C in 30 ml THF vorgelegt. 5.0 g (20.9 mmol) 4-Methyl-3-(trifluormethyl)-brombenzol wurden in 15 ml THF gelöst und langsam zugetropft. Anschließend wurden 3.3 g (31.4 mmol) Trimethylboronsäure zugetropft. Es wurde 2 h bei -78°C gerührt, bevor 11 ml 2 N Salzsäure zugegeben wurden. Man ließ auf RT erwärmen und trennte die organische Phase ab. Das Lösungsmittel wurde im Vakuum entfernt und der Rückstand aus n-Heptan umkristallisiert. Ausbeute: 2.6 g (60% d. Th.)

LC-MS (Methode 9B): Rₜ = 0.91 min; MS (ESIpos): m/z = 204 [M+H]⁺.

### Stufe b): 5-[4-Methyl-3-(trifluormethyl)phenyl]pyridin-3-carbonsäuremethylester

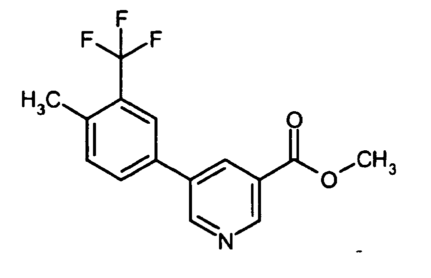

Nach der Allgemeinen Methode 1A wurden 2.2 g (10.3 mmol) 5-Bromnikotinsäuremethylester mit 2.5 g (12.4 mmol) [4-Methyl-3-(trifluormethyl)phenyl]boronsäure umgesetzt. Ausbeute: 2.6 g (86% d. Th.)

LC-MS (Methode 3B): Rₜ = 2.18 min; MS (ESIpos): m/z = 296 [M+H]⁺.

¹H-NMR (400 MHz, DMSO-d₆): δ = 9.20 (d, 1H), 9.10 (d, 1H), 8.54 (dd, 1H), 8.04-8.00 (m, 2H), 7.61 (d, 1H), 3.94 (s, 3H).

### Beispiel 158A

### 5-[4-Methyl-3-(trifluormethyl)phenyl]piperidin-3-carbonsäuremethylester [racemisches cis-/trans-Isomerengemisch]

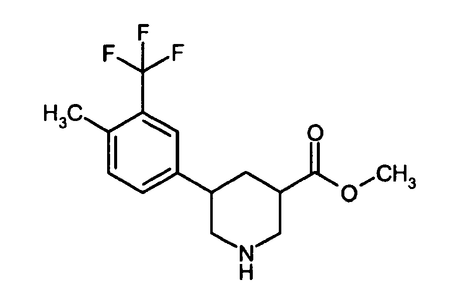

Nach der Allgemeinen Methode 12A wurden 1.0 g (3.4 mmol) der Verbindung aus Beispiel 157A umgesetzt. Ausbeute: 0.7 g (70% d. Th.)

LC-MS (Methode 9B): Rₜ = 0.76 min (cis-/trans-Isomere); MS (ESIpos): m/z = 302 [M+H]⁺.

### Beispiel 159A

### 5-[4-Metllyl-3-(trifluormethyl)phenyl]-1-(morpholin-4-ylcarbonyl)piperidin-3-carbonsäuremethylester [racemisches cis-/trans-Isomerengemisch]

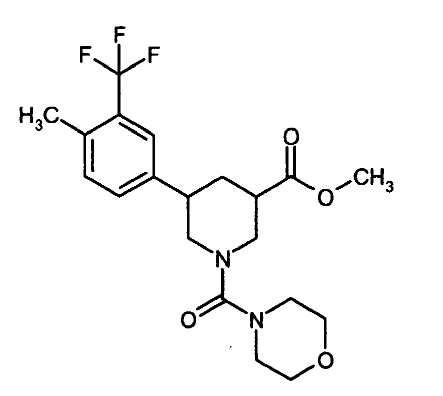

920 mg (3.1 mmol) der Verbindung aus Beispiel 158A wurden nach der Allgemeinen Methode 3A umgesetzt. Ausbeute: 674 mg (53% d. Th.)

LC-MS (Methode 2B): Rₜ = 1.26 min und 1.29 min [cis-/trans-Isomere]; MS (ESIpos): m/z = 415 [M+H]⁺.

### Beispiel 160A

### 5-[4-Methyl-3-(trifluormethyl)phenyl]-1-(morpholin-4-ylcarbonyl)piperidin-3-carbonsäure [racemisches cis-Isomer]

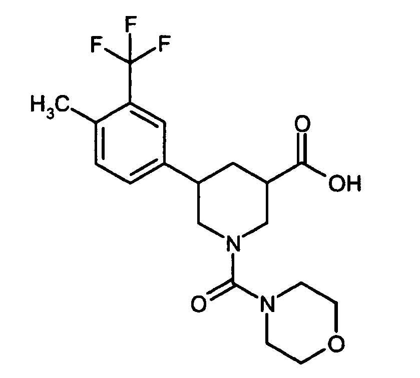

354 mg (0.85 mmol) der Verbindung aus Beispiel 159A wurden nach der Allgemeinen Methode 9A umgesetzt. Ausbeute: 279 mg (78% d. Th.)

LC-MS (Methode 2B): Rₜ = 1.14 min; MS (ESIpos): m/z = 401 [M+H]⁺.

¹H-NMR (400 MHz, DMSO-d₆): δ = 12.47 (br s, COOH), 7.57 (s, 1H), 7.52-7.49 (m, 1H), 7.39 (d, 1H), 3.83 (br d, 1H), 3.56-3.53 (m, 5H), 3.16-3.14 (m, 4H), 2.87-2.80 (m, 3H), 2.62-2.55 (m, 1H), 2.12 (br d, 1H), 1.76 (q, 1H).

### Beispiel 161A

### 3-Methyl-1-(4-nitrophenyl)-5-[4-methyl-3-(trifluormethyl)phenyl]piperidin-1,3-dicarboxylat [racemisches cis-/trans-Isomerengemisch]

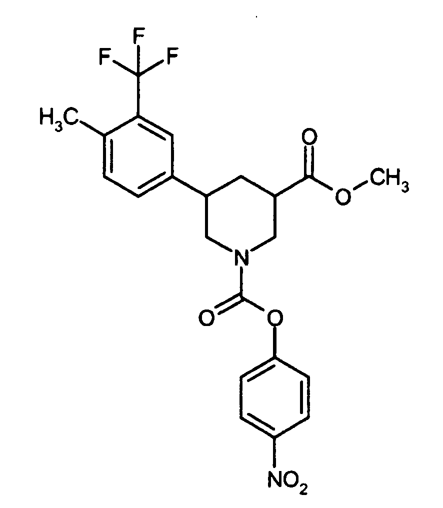

915 mg (3.04 mmol) der Verbindung aus Beispiel 158A wurden in 10 ml Dichlormethan vorgelegt, auf 0°C abgekühlt und mit 0.85 ml (615 mg, 6.07 mmol) Triethylamin sowie 612 mg (3.04 mmol) Chlorameisensäure-4-nitrophenylester versetzt. Das Reaktionsgemisch ließ man langsam auf RT erwärmen. Es wurde zweimal mit Wasser gewaschen, über Magnesiumsulfat getrocknet, filtriert und im Vakuum eingeengt. Der Rückstand wurde mit Methanol versetzt, filtriert, erneut im Vakuum eingeengt und säulenchromatographisch an Kieselgel aufgereinigt (Laufmittel Cyclohexan/Essigsäureethylester 1:1). Ausbeute: 921 mg (63% d. Th.)

LC-MS (Methode 2B): Rₜ = 1.52 min; MS (ESIpos): m/z = 467 [M+H]⁺.

### Beispiel 162A

### 1-[(4-Hydroxypiperidin-1-yl)carbonyl]-5-[4-methyl-3-(trifluormethyl)phenyl]piperidin-3-carbonsäwemethylester [racemisches cis-/trans-Isomerengemisch]

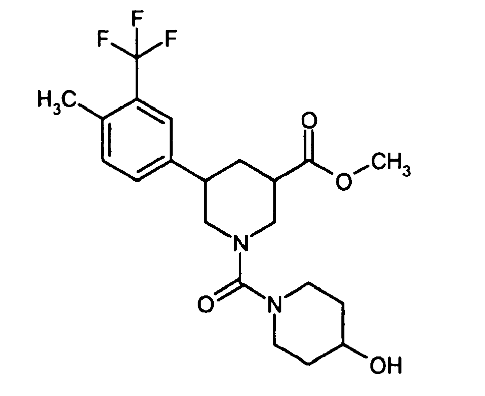

332 mg (0.67 mmol) der Verbindung aus Beispiel 161A, 203 mg (2.01 mmol) 4-Hydroxypiperidin und 92 mg (0.67 mmol) Kaliumcarbonat wurden in 6 ml *N,N*-Dimethylformamid vorgelegt und 30 min bei 150°C in einer *Single Mode*-Mikrowelle (Emrys Optimizer) umgesetzt. Das Rohprodukt wurde mittels präparativer HPLC aufgereinigt. Ausbeute: 201 mg (70% d. Th.)

LC-MS (Methode 9B): Rₜ = 1.03 min und 1.06 min [cis-/trans-Isomere]; MS (ESIpos): m/z = 429 [M+H]⁺.

### Beispiel 163A

### 1-[(4-Hydroxypiperidin-1-yl)carbonyl]-5-[4-methyl-3-(trifluormethyl)phenyl]piperidin-3-carbonsäure [racemisches cis-Isomer]

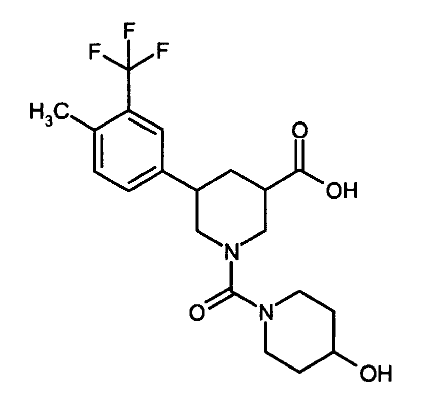

775 mg (1.81 mmol) der Verbindung aus Beispiel 162A wurden nach der Allgemeinen Methode 9A umgesetzt. Ausbeute: 727 mg (97% d. Th.)

LC-MS (Methode 9B): Rₜ = 0.93 min; MS (ESIpos): m/z = 415 [M+H]⁺.

### Beispiel 164A

### 5-[3-Methyl-4-(trifluormethoxy)phenyl]pyridin-3-carbonsäuremethylester

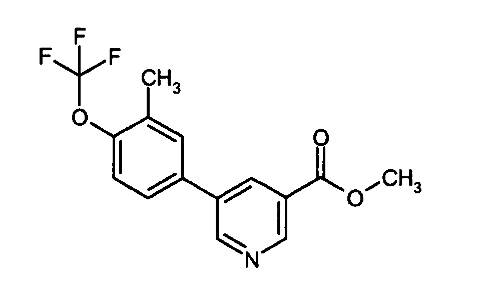

### Stufe a): [3-Methyl-4-(trifluormethoxy)phenyl]boronsäure

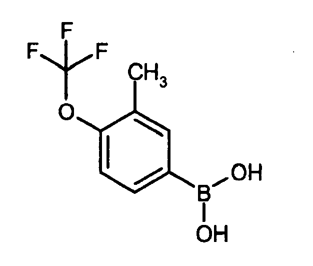

8.5 ml (1.4 g, 21.1 mmol) einer 2.5M Lösung von n-Butyllithium in Hexan wurden bei -78°C in 30 ml THF vorgelegt. 4.9 g (19.2 mmol) 4-Brom-2-methyl-(trifluormethoxy)benzol wurden in 15 ml THF gelöst und langsam zugetropft. Anschließend wurden 3.0 g (28.8 mmol) Trimethylboronsäure zugetropft. Es wurde 2 h bei -78°C gerührt, bevor 11 ml 2 N Salzsäure zugegeben wurden. Man ließ auf RT erwärmen und trennte die organische Phase ab. Das Lösungsmittel wurde im Vakuum entfernt und der Rückstand aus n-Heptan umkristallisiert. Ausbeute: 3.3 g (78% d. Th.)

LC-MS (Methode 2B): Rₜ = 1.08 min; MS (ESIpos): m/z = 221 [M+H]⁺.

### Stufe b): 5-[3-Methyl-4-(trifluormethoxy)phenyl]pyridin-3-carbonsäuremethylester

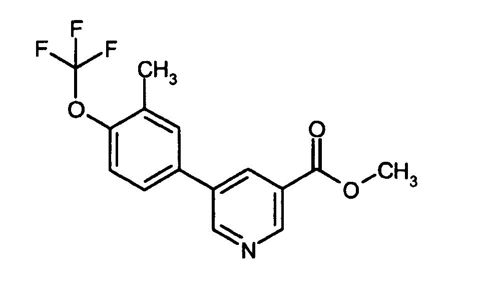

Nach der Allgemeinen Methode 1A wurden 2.7 g (12.5 mmol) 5-Bromnikotinsäuremethylester mit 3.3 g (15.0 mmol) [3-Methyl-4-(trifluormethoxy)phenyl]boronsäure umgesetzt. Ausbeute: 3.1 g (80% d. Th.)

LC-MS (Methode 2B): Rₜ = 1.36 min; MS (ESIpos): m/z = 312 [M+H]⁺.

¹H-NMR (400 MHz, DMSO-d₆): δ = 9.16 (d, 1H), 9.09 (d, 1H), 8.51 (dd, 1H), 7.88 (d, 1H), 7.77-7.75 (m, 1H), 7.46 (dd, 1H), 3.93 (s, 3H), 2.38 (s, 3H).

### Beispiel 165A

### 5-[3-Methyl-4-(trifluormethoxy)phenyl]piperidin-3-carbonsäuremethylester [racemisches cis-/trans-Isomerengemisch]

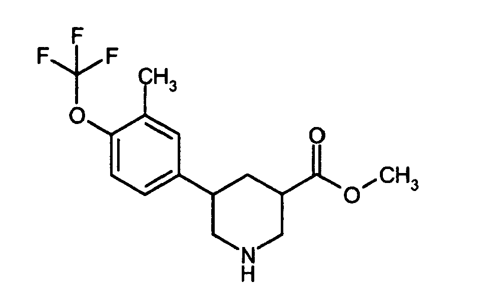

Nach der Allgemeinen Methode 12A wurden 1.0 g (3.4 mmol) der Verbindung aus Beispiel 164A umgesetzt. Ausbeute: 841 mg (78% d. Th.)

LC-MS (Methode 2B): Rₜ = 0.90 min und 0.94 min (cis-/trans-Isomere); MS (ESIpos): m/z = 318 [M+H]⁺.

### Beispiel 166A

### 5-[3-Methyl-4-(trifluormethoxy)phenyl]-1-(morpholin-4-ylcarbonyl)piperidin-3-carbonsäuremethylester [racemisches cis-/trans-Isomerengemisch]

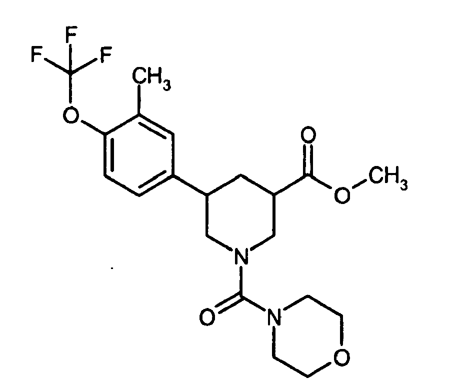

1.2 g (3.6 mmol) der Verbindung aus Beispiel 165A wurden nach der Allgemeinen Methode 3A umgesetzt. Ausbeute: 1.3 g (82% d. Th.)

LC-MS (Methode 2B): Rₜ = 2.06 min und 2.13 min [cis-/trans-Isomere]; MS (ESIpos): m/z = 431 [M+H]⁺.

### Beispiel 167A

### 5-[3-Methyl-4-(trifluormethoxy)phenyl]-1-(morpholin-4-ylcarbonyl)piperidin-3-carbonsäure [racemisches cis-Isomer]

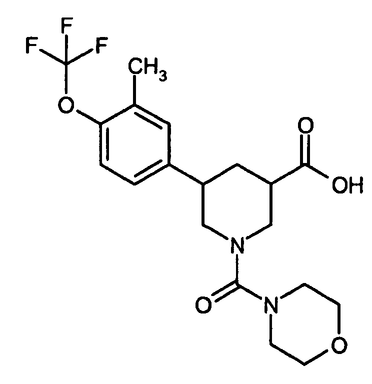

1.28 g (2.97 mmol) der Verbindung aus Beispiel 166A wurden nach der Allgemeinen Methode 9A umgesetzt. Ausbeute: 1.15 g (93% d. Th.)

LC-MS (Methode 2B): Rₜ = 1.16 min; MS (ESIpos): m/z = 417 [M+H]⁺.

¹H-NMR (400 MHz, DMSO-d₆): δ = 12.46 (br s, COOH), 7.32 (s, 1H), 7.27-7.21 (m, 2H), 3.84 (br d, 1H), 3.60-3.51 (m, 5H), 3.17-3.15 (m, 4H), 2.85-2.74 (m, 3H), 2.61-2.54 (m, 1H), 2.67 (s, 3H), 2.12 (br d, 1H), 1.72 (q, 1H).

### Beispiel 168A

### 3-Methyl-1-(4-nitrophenyl)-5-[3-methyl-4-(trifluormethoxy)phenyl]piperidin-1,3-dicarboxylat [racemisches cis-/trans-Isomerengemisch]

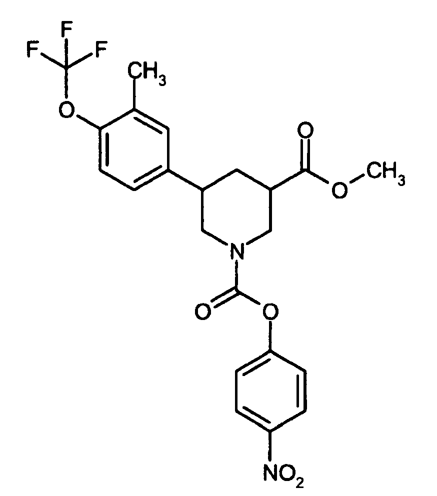

1.15 g (3.62 mmol) der Verbindung aus Beispiel 165A wurden in 10 ml Dichlormethan vorgelegt, auf 0°C abgekühlt und mit 1.01 ml (734 mg, 7.25 mmol) Triethylamin sowie 731 mg (3.62 mmol) Chlorameisensäure-4-nitrophenylester versetzt. Das Reaktionsgemisch ließ man langsam auf RT erwärmen und 16 h bei RT rühren. Es wurde mehrmals mit Wasser gewaschen, über Magnesiumsulfat getrocknet, filtriert und im Vakuum eingeengt. Ausbeute: 1.62 g (93% d. Th.)

LC-MS (Methode 2B): Rₜ = 1.53 min und 1.55 min [cis-/trans-Isomere]; MS (ESIpos): m/z = 483 [M+H]⁺.

### Beispiel 169A

### 1-[(4-Hydroxypiperidin-1-yl)carbonyl]-5-[3-methyl-4-(trifluormethoxy)phenyl]piperidin-3-carbonsäuremethylester [racemisches cis-/trans-Isomerengemisch]

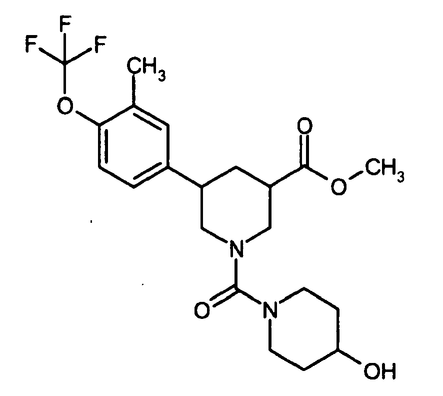

800 mg (1.66 mmol) der Verbindung aus Beispiel 168A, 503 mg (5.00 mmol) 4-Hydroxypiperidin und 229 mg (1.66 mmol) Kaliumcarbonat wurden in 18 ml *N,N*-Dimethylformamid vorgelegt und 15 min bei 150°C in einer *Single Mode*-Mikrowelle (Emrys Optimizer) umgesetzt. Das Rohprodukt wurde mittels präparativer HPLC aufgereinigt. Ausbeute: 490 mg (67% d. Th.)

LC-MS (Methode 3B): Rₜ = 1.88 min und 1.95 min [cis-/trans-Isomere]; MS (ESIpos): m/z = 445 [M+H]⁺.

### Beispiel 170A

### 1-[(4-Hydroxypiperidin-1-yl)carbonyl]-5-[3-methyl-4-(trifluormethoxy)phenyl]piperidin-3-carbonsäure [racemisches cis-Isomer]

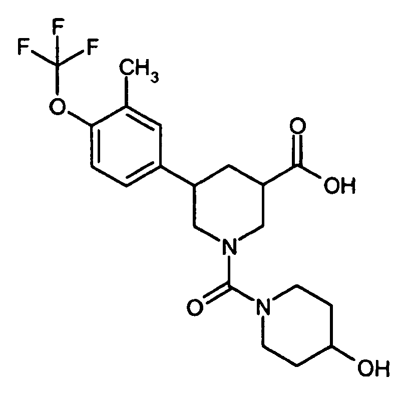

950 mg (2.14 mmol) der Verbindung aus Beispiel 169A wurden nach der Allgemeinen Methode 9A umgesetzt. Ausbeute: 933 mg (99% d. Th.)

LC-MS (Methode 9B): Rₜ = 0.96 min; MS (ESIpos): *m*/*z* = 431 [M+H]⁺.

¹H-NMR (400 MHz, DMSO-d₆): δ = 12.40 (br s, COOH), 7.32 (s, 1H), 7.27-7.21 (m, 2H), 4.66 (br s, OH), 3.79 (br d, 1H), 3.62-3.57 (m, 1H), 3.51 (br d, 1H), 3.46-3.42 (m, 2H), 2.87 (t, 2H), 2.80-2.75 (m, 3H), 2.60-2.54 (m, 1H), 2.27 (s, 3H), 2.12 (br d, 1H), 1.73-1.70 (m, 3H), 1.33-1.23 (m, 2H).

### Beispiel 171A

### 2,4-Difluor-N'-hydroxybenzolcarboximidamid

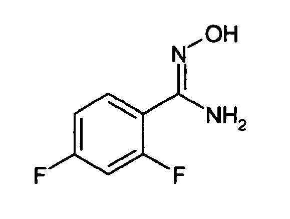

Nach der Allgemeinen Methode 5A wurden 1.00 g (7.19 mmol) 2,4-Difluorbenzolcarbonitril umgesetzt. Ausbeute: 1.23 g (99% d. Th.)

LC-MS (Methode 9B): Rₜ = 0.24 min; MS (ESIpos): m/z = 173 [M+H]⁺.

¹H-NMR (400 MHz, DMSO-d₆): δ = 9.64 (s, 1H), 7.54 (ddd, 1H), 7.28 (ddd, 1H), 7.10 (ddd, 1H), 5.83(br s, 2H).

### Beispiel 172A

### 1-tert-Butyl-3-methyl-5-[4-(trifluormethoxy)phenyl]piperidin-1,3-dicarboxylat [racemisches cis-/trans-Isomerengemisch]

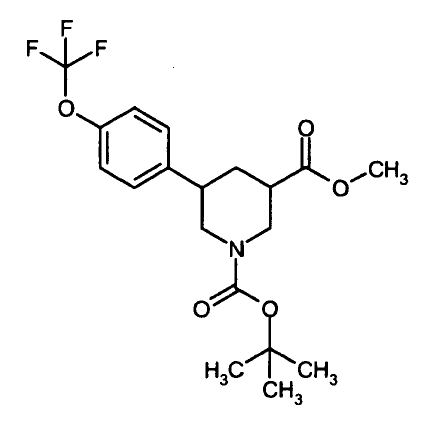

1.0 g (3.40 mmol) der Verbindung aus Beispiel 19A und 0.47 ml (0.34 g, 3.40 mmol) Triethylamin wurden in 50 ml Dichlormethan vorgelegt und bei RT mit 0.78 ml (0.74 g, 3.40 mmol) Di-*tert*-butyldicarboxylat versetzt. Das Reaktionsgemisch wurde 16 h bei RT gerührt, mit Wasser gewaschen, über Natriumsulfat getrocknet, filtriert und im Vakuum eingeengt. Der Rückstand wurde säulenchromatographisch an Kieselgel aufgereinigt (Laufmittel Dichlormethan/Methanol 50:1 -> 20:1). Ausbeute: 1.32 g (78% d. Th., 81% Reinheit)

LC-MS (Methode 5B): Rₜ = 2.66 min und 2.72 min [cis-/trans-Isomere]; MS (ESIpos): m/z = 404 [M+H]⁺.

### Beispiel 173A

### 1-(tert-Butoxycarbonyl)-5-[4-(trifluormethoxy)phenyl]piperidin-3-carbonsäure [racemisches cis-Isomer]

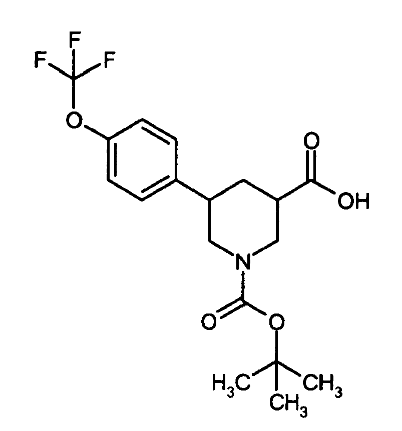

1.3 g (3.27 mmol) der Verbindung aus Beispiel 172A wurden nach der Allgemeinen Methode 9A umgesetzt. Ausbeute: 1.31 g (87% d. Th., 85% Reinheit)

LC-MS (Methode 5B): Rₜ = 2.46 min; MS (ESIpos): m/z = 390 [M+H]⁺.

### Beispiel 174A

### tert-Butyl-3-(3-cyclopropyl-1,2,4-oxadiazol-5-yl)-5-[4-(trifluormethoxy)phenyl]piperidin-1-carboxylat [racemisches cis-Isomer]

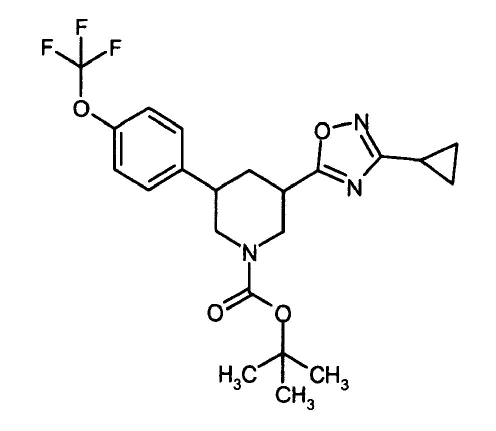

1.00 g (2.57 mmol) der Verbindung aus Beispiel 173A wurden nach der Allgemeinen Methode 2 mit 386 mg (3.86 mmol) der Verbindung aus Beispiel 78A umgesetzt. Ausbeute: 185 mg (16% d. Th.)

LC-MS (Methode 5B): Rₜ = 2.94 min; MS (ESIpos): m/z = 454 [M+H]⁺.

### Beispiel 175A

### 3-(3-Cyclopropyl-1,2,4-oxadiazol-5-yl)-5-[4-(trifluornnethoxy)phenyl]piperidin [racemisches cis-Isomer]

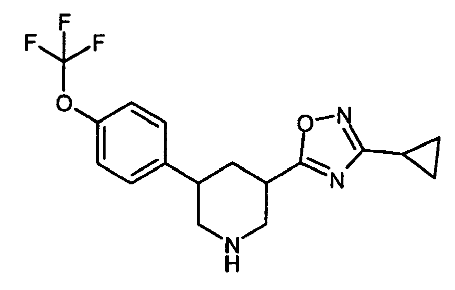

185 mg (0.41 mmol) der Verbindung aus Beispiel 174A wurden in 25 ml Dichlormethan vorgelegt und bei RT mit 0.32 ml (466 mg, 4.08 mmol) Trifluoressigsäure umgesetzt. Das Reaktionsgemisch wurde 16 h bei RT gerührt und anschließend im Vakuum eingeengt. Der Rückstand wurde in Essigsäureethylester aufgenommen, mit gesättigter, wässriger Natriumhydrogencarbonat-Lösung gewaschen, über Natriumsulfat getrocknet, filtriert und im Vakuum eingeengt. Ausbeute: 126 mg (87% d. Th.)

LC-MS (Methode 2B): Rₜ = 0.99 min; MS (ESIpos): m/z = 354 [M+H]⁺.

### Beispiel 176A

### 4-Nitrophenyl-3-(3-cyclopropyl-1,2,4-oxadiazol-5-yl)-5-[4-(trifluormethoxy)phenyl]piperidin-1-carboxylat [racemisches cis-Isomer]

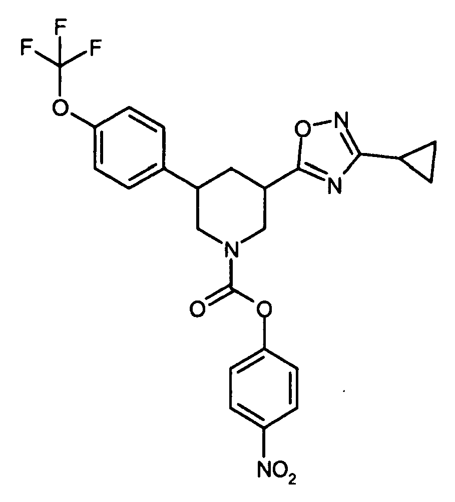

704 mg (1.99 mmol) der Verbindung aus Beispiel 175A wurden analog der Vorschrift aus Beispiel 126A umgesetzt. Ausbeute: 879 mg (85% d. Th.)

LC-MS (Methode 2B): Rₜ = 1.57 min; MS (ESIpos): m/z = 519 [M+H]⁺.

### Beispiel 177A

### tert-Butyl-3-[3-(3-fluorphenyl)-1,2,4-oxadiazol-5-yl]-5-[4-(trifluormethoxy)phenyl]piperidin-1-carboxylat [racemisches cis-Isomer]

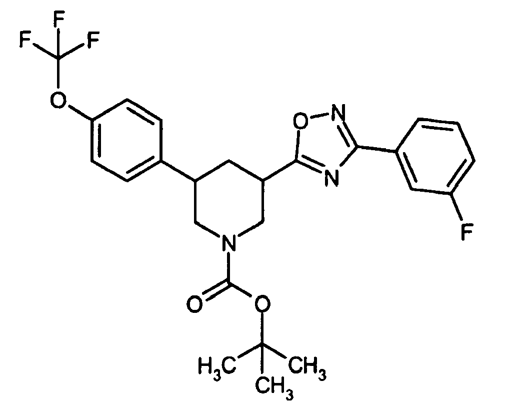

1.03 g (2.65 mmol) der Verbindung aus Beispiel 173A wurden nach der Allgemeinen Methode 2 umgesetzt. Ausbeute: 463 mg (31% d. Th.)

LC-MS (Methode 2B): Rₜ = 1.76 min; MS (ESIpos): m/z = 508 [M+H]⁺.

### Beispiel 178A

### 3-[3-(3-Fluorphenyl)-1,2,4-oxadiazol-5-yl]-5-[4-(trifluormethoxy)phenyl]piperidin [racemisches cis-Isomer]

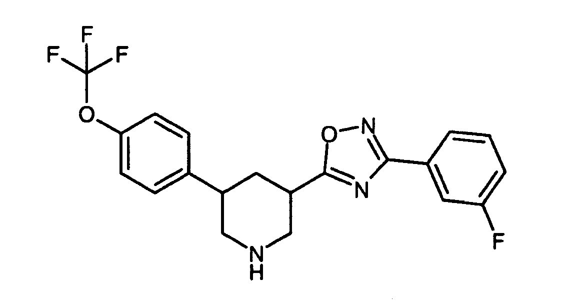

425 mg (0.84 mmol) der Verbindung aus Beispiel 177A wurden in 55 ml Dichlormethan vorgelegt und bei RT mit 0.65 ml (955 mg, 8.37 mmol) Trifluoressigsäure umgesetzt. Das Reaktionsgemisch wurde 20 h bei RT gerührt und anschließend im Vakuum eingeengt. Der Rückstand wurde in Essigsäureethylester aufgenommen, mit gesättigter, wässriger Natriumhydrogencarbonat-Lösung gewaschen, über Natriumsulfat getrocknet, filtriert und im Vakuum eingeengt. Ausbeute: 340 mg (80% d. Th., 80% Reinheit)

LC-MS (Methode 1B): Rₜ = 1.75 min; MS (ESIpos): m/z = 408 [M+H]⁺.

### Beispiel 179A

### 3-[3-(3-Fluorphenyl)-1,2,4-oxadiazol-5-yl]-5-[4-(trifluormethoxy)phenyl]piperidin-1-carbonylchlorid [racemisches cis-Isomer]

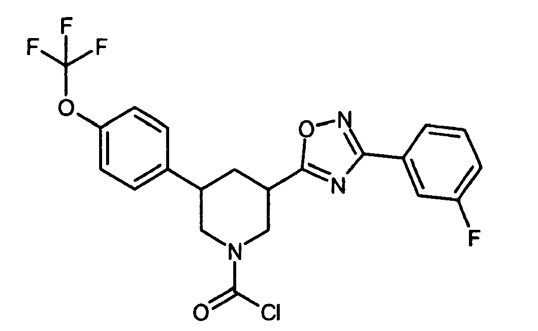

1.00 g (2.46 mmol) der Verbindung aus Beispiel 178A und 0.34 ml (248 mg, 2.46 mmol) Triethylamin wurden bei 0°C in 167 ml Dichlormethan vorgelegt. Es wurden 0.12 ml (242 mg, 1.23 mmol) Trichlormethylchlorformiat (Diphosgen) zugetropft. Das Reaktionsgemisch wurde 16 h bei RT gerührt, rasch mit wenig Eiswasser gewaschen, über Natriumsulfat getrocknet, filtriert und im Vakuum eingeengt. Das Rohprodukt wurde säulenchromatographisch an Kieselgel aufgereinigt. Ausbeute: 944 mg (68% d. Th., 83% d. Th.)

HPLC (Methode 2A): Rₜ = 5.51 min; MS (ESIpos): m/z = 470 [M+H]⁺.

### Beispiel 180A

### 1-tert-Butyl-3-methyl-5-[4-(trifluormethyl)phenyl]piperidin-1,3-dicarboxylat [racemisches cis-/trans-Isomerengemisch]

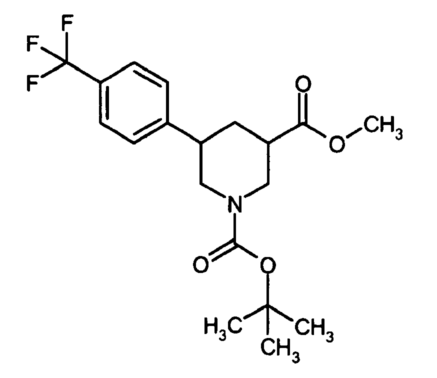

1.00 g (3.48 mmol) der Verbindung aus Beispiel 47A und 0.49 ml (0.35 g, 3.48 mmol) Triethylamin wurden in 50 ml Dichlormethan vorgelegt und bei RT mit 0.76 g (3.48 mmol) Di-*tert*-butyldicarboxylat versetzt. Das Reaktionsgemisch wurde 16 h bei RT gerührt, mit Wasser und gesättigter, wässriger Natriumchlorid-Lösung gewaschen, über Natriumsulfat getrocknet, filtriert und im Vakuum eingeengt. Der Rückstand wurde säulenchromatographisch an Kieselgel aufgereinigt (Laufmittel Dichlormethan/Methanol 100:1). Ausbeute: 1.01 g (67% d. Th., 90% Reinheit)

HPLC (Methode 2A): Rₜ = 5.21 min und 5.30 min [cis-/trans-Isomere]; MS (DCIpos): m/z = 388 [M+H]⁺.

### Beispiel 181A

### 1-(tert-Butoxycarbonyl)-5-[4-(trifluormethyl)phenyl]piperidin-3-carbonsäure [racemisches cis-Isomer]

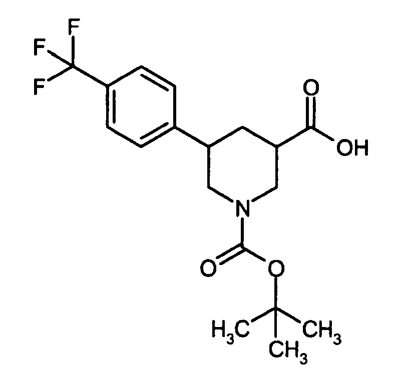

1.18 g (3.07 mmol) der Verbindung aus Beispiel 180A wurden nach der Allgemeinen Methode 9A umgesetzt. Ausbeute: 1.17 g (86% d. Th., 84% Reinheit)

HPLC (Methode 2A): Rₜ = 4.81 min; MS (ESIpos): m/z = 374 [M+H]⁺.

### Beispiel 182A

### tert-Butyl-3-(3-cyclopropyl-1,2,4-oxadiazol-5-yl)-5-[4-(trifluormethyl)phenyl]piperidin-1-carboxylat [racemisches cis-Isomer]

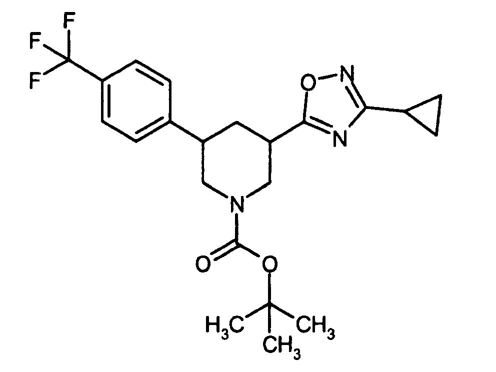

3.75 g (10.0 mmol) der Verbindung aus Beispiel 181A wurden nach der Allgemeinen Methode 2 umgesetzt. Ausbeute: 3.45 g (72% d. Th.)

HPLC (Methode 2A): Rₜ = 5.47 min; MS (ESIpos): m/z = 438 [M+H]⁺.

### Beispiel 183A

### 3-(3-Cyclopropyl-1,2,4-oxadiazol-5-yl)-5-[4-(trifluormethyl)phenyl]piperidin [racemisches cis-Isomer]

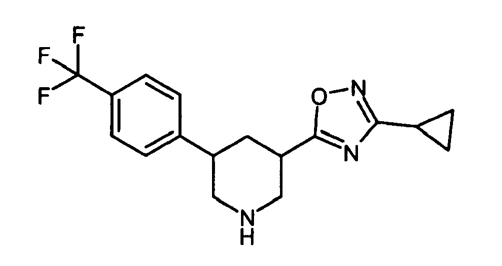

3.23 g (7.38 mmol) der Verbindung aus Beispiel 182A wurden in 60 ml Dichlormethan vorgelegt und bei RT mit 5.7 ml (8.42 g, 73.8 mmol) Trifluoressigsäure umgesetzt. Das Reaktionsgemisch wurde 16 h bei RT gerührt und anschließend im Vakuum eingeengt. Der Rückstand wurde in Essigsäureethylester aufgenommen, mit gesättigter, wässriger Natriumhydrogencarbonat-Lösung gewaschen, über Magnesiumsulfat getrocknet, filtriert und im Vakuum eingeengt. Ausbeute: 2.48 g (92% d. Th.)

HPLC (Methode 2A): Rₜ = 4.15 min; MS (ESIpos): m/z = 338 [M+H]⁺.

### Beispiel 184A

### 4-Nitrophenyl-3-(3-cyclopropyl-1,2,4-oxadiazol-5-yl)-5-[4-(trifluormethyl)phenyl]piperidin-1-carboxylat [racemisches cis-Isomer]

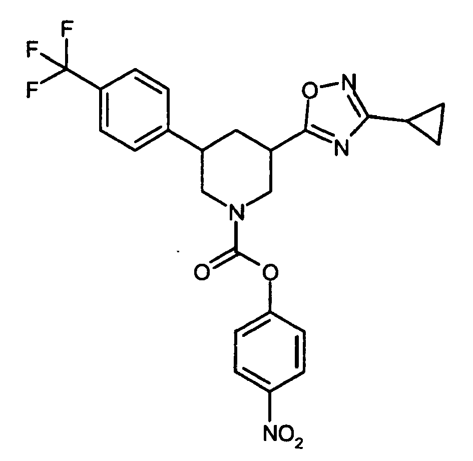

1.00 g (2.96 mmol) der Verbindung aus Beispiel 183A wurden analog der Vorschrift aus Beispiel 126A umgesetzt. Ausbeute: 1.34 g (90% d. Th.)

HPLC (Methode 2A): Rₜ = 5.14 min; MS (ESIpos): m/z = 503 [M+H]⁺.

### Beispiel 185A

### tert-Butyl-3-[3-(3-fluorphenyl)-1,2,4-oxadiazol-5-yl]-5-[4-(trifluormethyl)phenyl]piperidin-1-carboxylat [racemisches cis-Isomer]

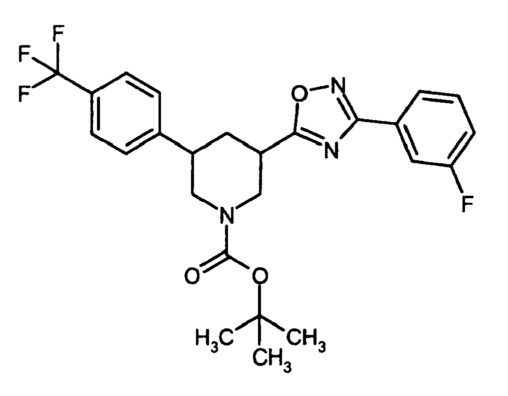

0.75 g (2.01 mmol) der Verbindung aus Beispiel 181A wurden nach der Allgemeinen Methode 2 umgesetzt. Ausbeute: 0.84 g (79% d. Th.)

HPLC (Methode 2A): Rₜ = 5.83 min; MS (ESIpos): m/z = 492 [M+H]⁺.

### Beispiel 186A

### 3-[3-(3-Fluorphenyl)-1,2,4-oxadiazol-5-yl]-5-[4-(trifluormethyl)phenyl]piperidin [racemisches cis-Isomer]

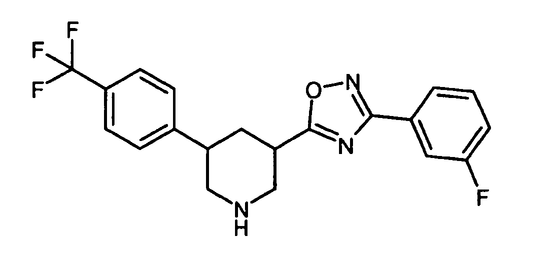

0.82 g (1.67 mmol) der Verbindung aus Beispiel 185A wurden in 13 ml Dichlormethan vorgelegt und bei RT mit 1.3 ml (1.90 g, 16.78 mmol) Trifluoressigsäure vergesetzt. Das Reaktionsgemisch wurde 16 h bei RT gerührt und anschließend im Vakuum eingeengt. Der Rückstand wurde in Essigsäureethylester aufgenommen, mit gesättigter, wässriger Natriumhydrogencarbonat-Lösung gewaschen, über Magnesiumsulfat getrocknet, filtriert und im Vakuum eingeengt. Ausbeute: 0.65 g (99% d. Th.)

HPLC (Methode 2A): Rₜ = 4.46 min; MS (ESIpos): m/z = 392 [M+H]⁺.

### Beispiel 187A

### 4-Nitrophenyl-3-[3-(3-fluorphenyl)-1,2,4-oxadiazol-5-yl]-5-[4-(trifluormethyl)phenyl]piperidin-1 - carboxylat [racemisches cis-Isomer]

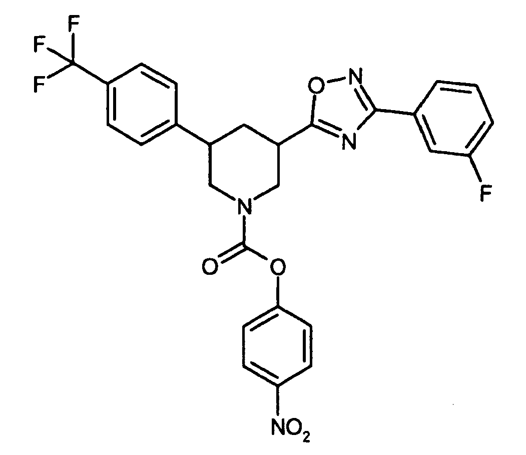

1.00 g (2.56 mmol) der Verbindung aus Beispiel 186A wurden analog der Vorschrift aus Beispiel 126A umgesetzt. Ausbeute: 1.29 g (9 1 % d. Th.)

HPLC (Methode 2A): Rₜ = 5.43 min; MS (ESIpos): m/z = 557 [M+H]⁺.

### Beispiel 188A

### tert-Butyl-(1-{3-[3-(3-fluorphenyl)-1,2,4-oxadiazol-5-yl]-5-[4-(trifluormethoxy)phenyl]piperidin-1-yl}-2-methyl-1-oxopropan-2-yl)carbamat [racemisches cis-Isomer]

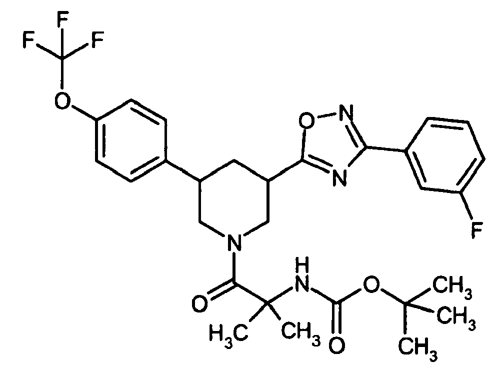

150 mg (0.30 mmol) der Verbindung aus Beispiel 178A wurden nach der Allgemeinen Methode 7 mit 66 mg (0.32 mmol) *N-*(*tert*-Butoxycarbonyl)-2-methylalanin umgesetzt. Ausbeute: 117 mg (64% d. Th.)

LC-MS (Methode 3B): Rₜ = 2.77 min; MS (ESIpos): m/z = 593 [M+H]⁺.

### Beispiel 189A

### tert-Butyl-[1-({3-(3-cyclopropyl-1,2,4-oxadiazol-5-yl)-5-[4-(trifluormethyl)phenyl]piperidin-1-yl}carbonyl)cyclopentyl]carbamat [racemisches cis-Isomer]

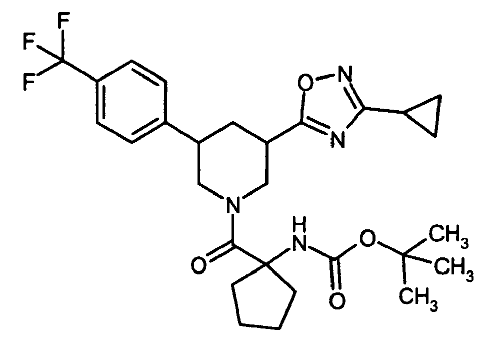

100 mg (0.30 mmol) der Verbindung aus Beispiel 183A wurden nach der Allgemeinen Methode 7 mit 75 mg (0.33 mmol) 1-[(*tert*-Butoxycarbonyl)amino]cyclopentancarbonsäure umgesetzt. Ausbeute: 127 mg (78% d. Th.)

HPLC (Methode 2A): Rₜ = 5.13 min; MS (ESIpos): m/z = 549 [M+H]⁺.

### Beispiel 190A

### 5-[3-(Trifluormethoxy)phenyl]pyridine-3-carbonsäuremethylester

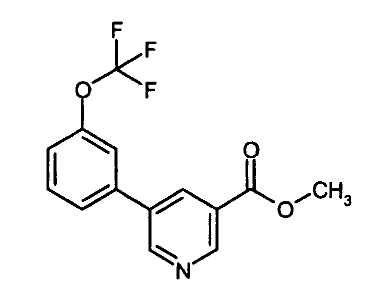

Nach der Allgemeinen Methode 1A wurden 2.61 g (12.09 mmol) 5-Bromnikotinsäuremethylester und 2.49 g (12.09 mmol) 3-(Trifluormethoxy)-phenylboronsäure umgesetzt. Ausbeute: 2.44 g (68% d. Th.)

LC-MS (Methode 2B): Rₜ = 1.29 min; MS (ESIpos): m/z = 298 [M+H]⁺.

### Beispiel 191A

### 5-[3-(Trifluormethoxy)phenyl]piperidin-3-carbonsäuremethylester [racemisches cis-/trans-Isomerengemisch]

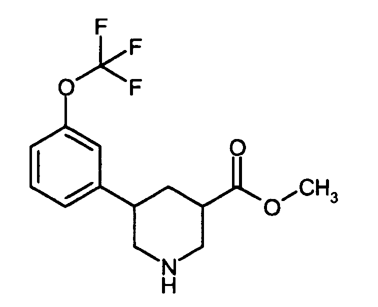

Nach der Allgemeinen Methode 2A wurde 2.42 g (8.14 mmol) der Verbindung aus Beispiel 190A umgesetzt. Ausbeute: 1.34 g (50% d. Th.)

LC-MS (Methode 3B): Rₜ = 0.89 min und 0.97 min [cis-/trans-Isomere]; MS (ESIpos): m/z = 304 [M+H]⁺.

### Beispiel 192A

### 3-Methyl-1-(4-nitrophenyl)-5-[3-(trifluormethoxy)phenyl]piperidin-1,3-dicarboxylat

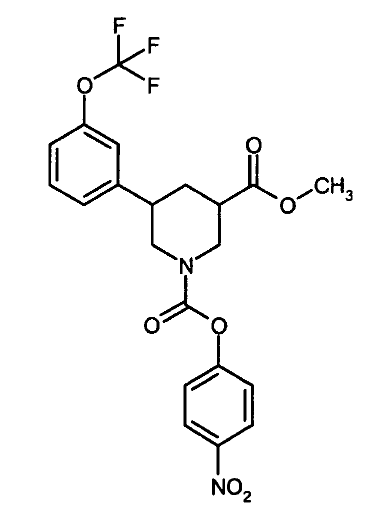

5.0 g (15.4 mmol) der Verbindung aus Beispiel 191 A wurden analog der Vorschrift von Beispiel 126 A umgesetzt. Ausbeute: 3.8 g (52% d. Th.)

LC-MS (Methode 2B): Rₜ = 1.46 min und 1.48 min [cis-/trans-Isomere]; MS (ESIpos): m/z = 469 [M+H]⁺.

### Beispiel 193A

### 1-[(4-Hydroxypiperidin-1-yl)carbonyl]-5-[3-(trifluormethoxy)phenyl]piperidin-3-carbonsäuremethylester [racemisches cis-/trans-Isomerengemisch]

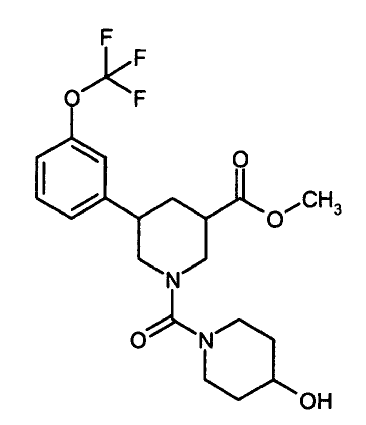

1.5 g (3.1 mmol) der Verbindung aus Beispiel 192A wurden nach der Allgemeinen Methode 6 mit 0.9 g (9.2 mmol) 4-Hydroxypiperidin umgesetzt. Ausbeute: 1.1 g (84% d. Th.)

LC-MS (Methode 2B): Rₜ = 1.13 min und 1.16 min [cis-/trans-Isomere]; MS (ESIpos): m/z = 431 [M+H]⁺.

### Beispiel 194A

### 1-[(4-Hydroxypiperidin-1-yl)carbonyl]-5-[3-(trifluormethoxy)phenyl]piperidin-3-carbonsäure [racemisches cis-Isomer]

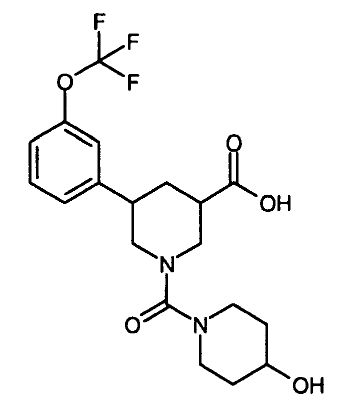

1.1 g (2.6 mmol) der Verbindung aus Beispiel 193A wurden nach der Allgemeinen Methode 9A umgesetzt. Ausbeute: 0.4 g (36% d. Th.)

LC-MS (Methode 3B): Rₜ = 1.56 min; MS (ESIpos): m/z = 417 [M+H]⁺.

### Beispiel 195A

### 5-[4-(Methylsulfonyl)phenyl]pyridin-3-carbonsäuremethylester

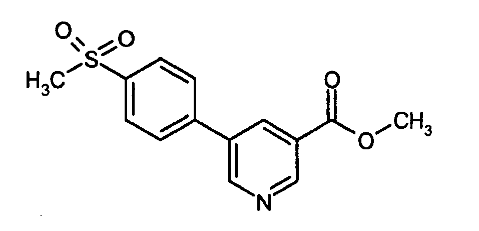

Nach der Allgemeinen Methode 1A wurden 4.5 g (20.8 mmol) 5-Bromnikotinsäuremethylester mit 5.0 g (25.0 mmol) 4-(Methylsulfonyl)phenylboronsäure umgesetzt. Ausbeute: 1.4 g (24% d. Th.)

LC-MS (Methode 2B): Rₜ = 0.84 min; MS (ESIpos): m/z = 292 [M+H]⁺.

### Beispiel 196A

### 5-[4-(Methylsulfonyl)phenyl]piperidin-3-carbonsäuremethylester [racemisches cis-/trans-Isomerengemisch]

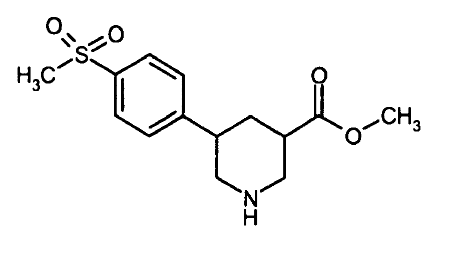

Nach der Allgemeinen Methode 12A wurden 1.5 g (5.1 mmol) der Verbindung aus Beispiel 195A umgesetzt. Ausbeute: 1.0 g (65% d. Th.)

LC-MS (Methode 2B): Rₜ = 0.28 min; MS (ESIpos): m/z = 298 [M+H]⁺.

### Beispiel 197A

### 5-[4-(Methylsulfonyl)phenyl]-1-(morpholin-4-ylcarbonyl)piperidin-3-carbonsäuremethylester [racemisches cis-/trans-Isomerengemisch]

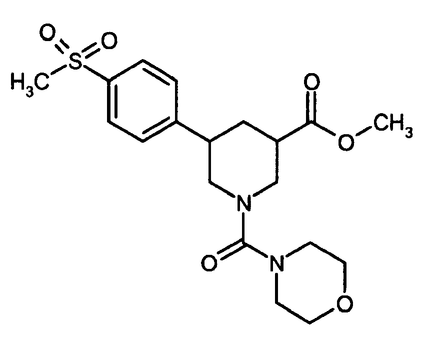

0.22 g (0.74 mmol) der Verbindung aus Beispiel 196A wurden nach der Allgemeinen Methode 3A umgesetzt. Ausbeute: 0.20 g (66% d. Th.)

LC-MS (Methode 1B): Rₜ = 1.56 min und 1.63 min [cis-/trans-Isomere]; MS (ESIpos): m/z = 411 [M+H]⁺.

### Beispiel 198A

### 5-[4-(Methylsulfonyl)phenyl]-1-(morpholin-4-ylcarbonyl)piperidin-3-carbonsäure [racemisches cis-Isomer]

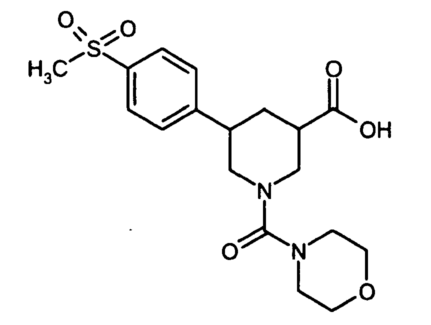

1.1 g (2.7 mmol) der Verbindung aus Beispiel 197A wurden nach der Allgemeinen Methode 9A umgesetzt. Ausbeute: 373 mg (35% d. Th..)

LC-MS (Methode 2B): Rₜ = 0.71 min; MS (ESIpos): m/z = 397 [M+H]⁺.

### Beispiel 199A

### 5-(4-Ethylphenyl)-1-(morpholin-4-ylcarbonyl)-N-(prop-2-in-1-yl)piperidin-3-carboxamid [racemisches cis-Isomer]

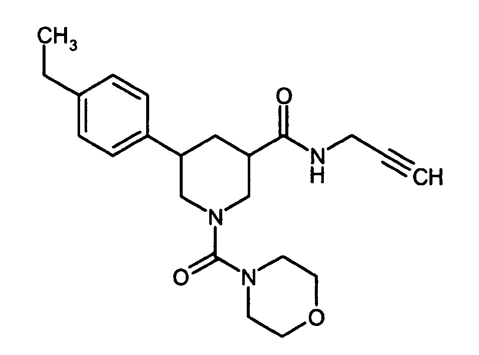

### Stufe a): 5-(4-Ethylphenyl)-1-(morpholin-4-ylcarbonyl)piperidin-3-carbonylchlorid [racemisches cis-Isomer]

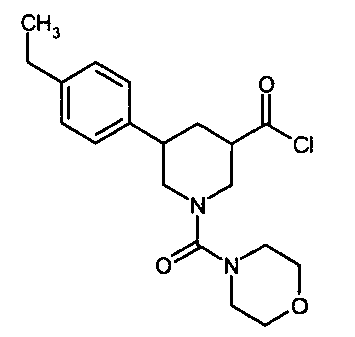

150 mg (0.43 mmol) der Verbindung aus Beispiel 38A wurden in 3 ml Dichlormethan vorgelegt und mit 63 µl (103 mg, 0.87 mmol) Thionylchlorid versetzt. Das Reaktionsgemisch wurde 2 h unter RF gerührt, im Vakuum eingeengt und abschließend dreimal mit Toluol versetzt und jeweils im Vakuum wieder eingeengt.

### Stufe b): 5-(4-Ethylphenyl)-1-(morpholin-4-ylcarbonyl)-N-(prop-2-in-1-yl)piperidin-3-carboxamid

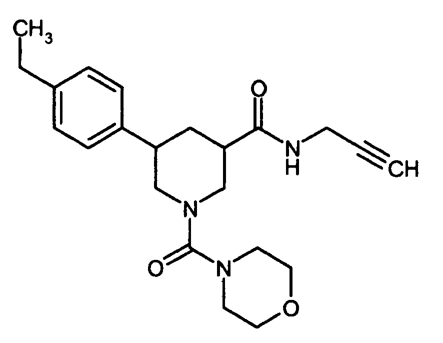

18 µl (16 mg, 0.29 mmol) Propargylamin, 40 µl (29 mg, 0.29 mmol) Triethylamin und 1 mg (0.01 mmol) *N,N-*Dimethylaminopyridin wurden in 0.5 ml Dichlormethan vorgelegt und mit 105 mg (0.29 mmol) 5-(4-Ethylphenyl)-1-(morpholin-4-ylcarbonyl)piperidin-3-carbonylchlorid, gelöst in 1 ml Dichlormethan, versetzt. Das Reaktionsgemisch wurde 30 Minuten bei 0°C und 16 h bei RT gerührt. Zur Aufarbeitung wurde das Reaktionsgemisch mit Wasser versetzt und mit Essigsäureethylester extrahiert. Die vereinigten organischen Extrakte wurden über Magnesiumsulfat getrocknet, filtriert und im Vakuum eingeengt. Der Rückstand wurde säulenchromatographisch an Kieselgel aufgereinigt (Laufmittel Dichlormethan/Methanol 100:5). Ausbeute: 108 mg (98% d. Th..)

HPLC (Methode 2A): Rₜ = 4.10 min; MS (ESIpos): m/z = 384 [M+H]⁺.

### Beispiel 200A

### 1-(Morpholin-4-ylcarbonyl)-5-[3-(trifluonnethoxy)phenyl]piperidin-3-carbonsäuremethylester [racemisches cis-/trans-Isomerengemisch]

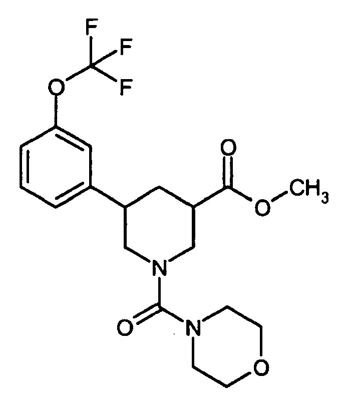

1.67 g (5.14 mmol) der Verbindung aus Beispiel 191A wurden nach der Allgemeinen Methode 3A umgesetzt. Ausbeute: 1.91 g (89% d. Th.)

LC-MS (Methode 2B): Rₜ = 1.21 min und 1.24 min [cis-/trans-Isomere]; MS (ESIpos): m/z = 417 [M+H]⁺.

### Beispiel 201A

### 1-(Morpholin-4-ylcarbonyl)-5-[3-(trifluormethoxy)phenyl]piperidin-3-carbonsäure [racemisches cis-Isomer]

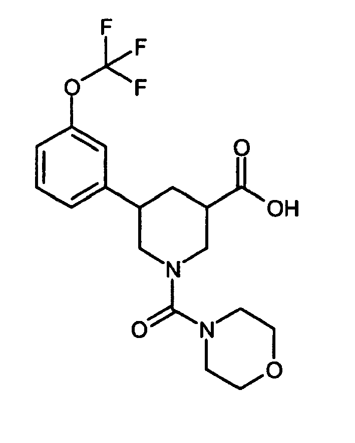

1.9 g (4.6 mmol) der Verbindung aus Beispiel 200A wurden nach der Allgemeinen Methode 9A umgesetzt. Ausbeute: 1.8 g (99% d. Th..)

LC-MS (Methode 5B): Rₜ = 2.01 min; MS (ESIpos): m/z = 403 [M+H]⁺.

### Beispiel 202A

### 1-[(4-Cyanpiperidin-1-yl)carbonyl]-5-[3-(trifluormethoxy)phenyl]piperidin-3-carbonsäuremethylester [racemisches cis-/trans-Isomerengemisch]

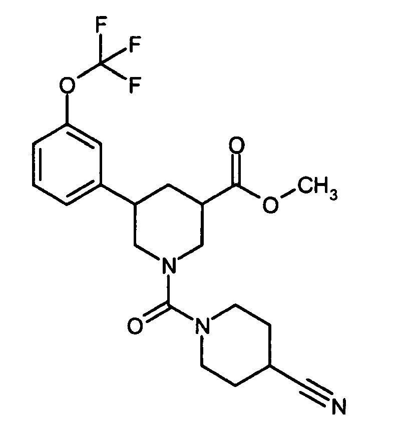

Nach der Allgemeinen Methode 6 wurden 850 mg (1.82 mmol) der Verbindung aus Beispiel 192A mit 599 mg (5.44 mmol) 4-Cyanopiperidin umgesetzt. Ausbeute: 464 mg (58% d. Th..)

LC-MS (Methode 2B): Rₜ = 1.27 min und 1.29 min [cis-/trans-Isomere]; MS (ESIpos): m/z = 440 [M+H]⁺.

### Beispiel 203A

### 1-[(4-Cyanpiperidin-1-yl)carbonyl]-5-[3-(trifluormethoxy)phenyl]piperidin-3-carbonsäure [racemisches cis-Isomer]

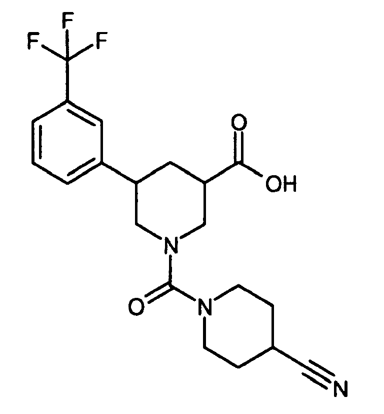

420 mg (0.95 mmol) der Verbindung aus Beispiel 202A wurden nach der Allgemeinen Methode 9A umgesetzt. Ausbeute: 247 mg (59% d. Th.)

LC-MS (Methode 5B): Rₜ = 2.08 min; MS (ESIpos): m/z = 426 [M+H]⁺.

### Beispiel 204A

### Methyl-1-[(2,2-dimethylmorpholin-4-yl)carbonyl]-5-[4-(trifluormethyl)phenyl]piperidin-3-carboxylat [racemisches cis-/trans-Isomerengemisch]

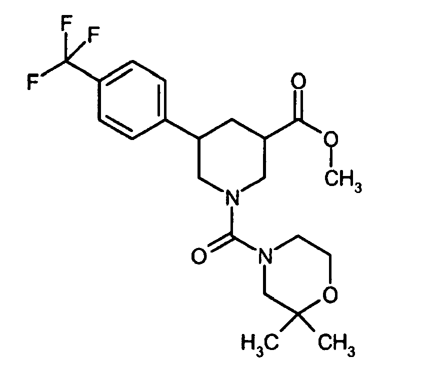

Nach der Allgemeinen Methode 8A wurden 3.00 g (6.63 mmol) 3-Methyl-1-(4-nitrophenyl)-5-[4-(trifluormethyl)phenyl]piperidin-1,3-dicarboxylat (Beispiel 93A) und 2.21 g (14.6 mmol) 2,2-Dimethylmorpholin Hydrochlorid umgesetzt. Ausbeute: 1.79 g (39% d. Th., 62% Reinheit).

LC-MS (Methode 2B): Rₜ = 1.28 min und 1.32 min (cis-/trans-Isomere); MS (ESIpos): m/z = 429 [M+H]⁺.

### Beispiel 205A

### Methyl-1-[(4-methyl-3-oxopiperazin-1-yl)carbonyl]-5-[4-(trifluormethyl)phenyl]piperidin-3-carboxylat [racemisches cis-/trans-Isomerengemisch]

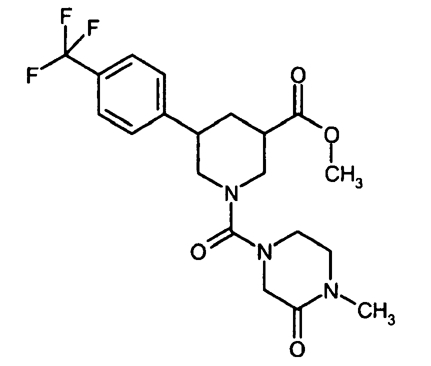

Nach der Allgemeinen Methode 8A wurden 4.00 g (8.84 mmol) 3-Methyl-1-(4-nitrophenyl)-5-[4-(trifluormethyl)phenyl]piperidin-1,3-dicarboxylat (Beispiel 93A) und 5.05 g (22.1 mmol) 1-Methylpiperazin-2-on umgesetzt. Ausbeute: 2.41 g (62% d. Th.).

LC-MS (Methode 3B): Rₜ = 1.69 min und 1.74 min (cis-/trans-Isomere); MS (ESIpos): m/z = 428 [M+H]⁺.

### Beispiel 206A

### 1-[(3-Oxopiperazin-1-yl)carbonyl]-5-[4-(trifluormethyl)phenyl]piperidin-3-carbonsäure [racemisches cis-Isomer]

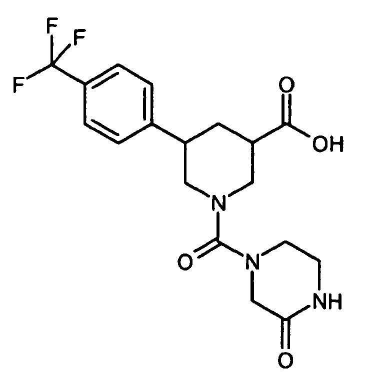

Nach der Allgemeinen Methode 9A wurden 1.90 g (4.60 mmol) Methyl-1-[(3-oxopiperazin-1-yl)carbonyl]-5-[4-(trifluormethyl)phenyl]piperidin-3-carboxylat (Beispiel 89A) umgesetzt. Ausbeute: 1.89 g (97% d. Th.).

LC-MS (Methode 2B): Rₜ = 0.95 min; MS (ESIpos): m/z = 400 [M+H]⁺.

### Beispiel 207A

### 1-[(3-Methoxypiperidin-1-yl)carbonyl]-5-[4-(trifluormethyl)phenyl]piperidin-3-carbonsäure [racemisches cis-Diastereomerenpaar]

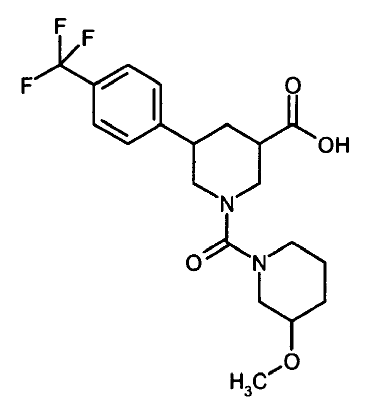

Nach der Allgemeinen Methode 9A wurden 2.90 g (5.01 mmol, 74% Reinheit) Methyl-1-[(3-methoxypiperidin-1-yl)carbonyl]-5-[4-(trifluormethyl)phenyl]piperidin-3-carboxylat (Beispiel 119A) umgesetzt. Ausbeute: 2.84 g (98% d. Th., 72% Reinheit).

LC-MS (Methode 3B): Rₜ = 1.85 min; MS (ESIpos): m/z = 415 [M+H]⁺.

### Beispiel 208A

### 1-[(2,2-Dimethylmorpholin-4-yl)carbonyl]-5-[4-(trifluormethyl)phenyl]piperidin-3-carbonsäure [racemisches cis-Isomer]

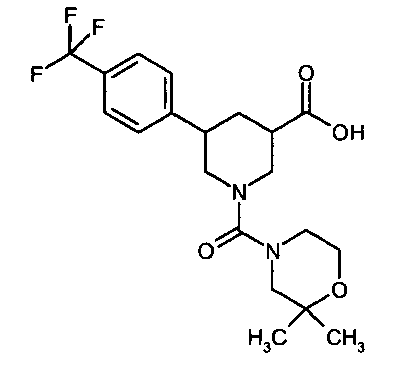

Nach der Allgemeinen Methode 9A wurden 1.79 g (3.09 mmol, 74% Reinheit) Methyl-1-[(2,2-dimethylmorpholin-4-yl)carbonyl]-5-[4-(trifluormethyl)phenyl]piperidin-3-carboxylat (Beispiel 204A) umgesetzt. Ausbeute: 1.65 g (87% d. Th., 68% Reinheit).

LC-MS (Methode 9B): Rₜ = 1.02 min; MS (ESIpos): m/z = 415 [M+H]⁺.

### Beispiel 209A

### 1-[(4-Methyl-3-oxopiperazin-1-yl)carbonyl]-5-[4-(trifluormethyl)phenyl]piperidin-3-carbonsäure [racemisches cis-Isomer]

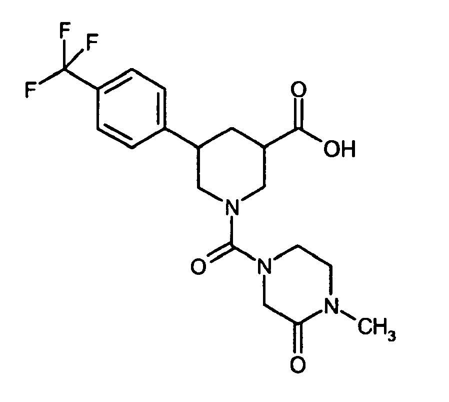

Nach der Allgemeinen Methode 9A wurden 2.41 g (5.64 mmol) Methyl-1-[(4-methyl-3-oxopiperazin-1-yl)carbonyl]-5-[4-(trifluormethyl)phenyl]piperidin-3-carboxylat (Beispiel 205A) umgesetzt. Ausbeute: 1.62 g (69% d. Th.).

LC-MS (Methode 2B): Rₜ = 1.01 min; MS (ESIpos): m/z = 414 [M+H]⁺.

### Beispiel 210A

### tert-Butyl-[1-({3-[3-(propan-2-yl)-1,2,4-oxadiazol-5-yl]-5-[4-(trifluormethoxy)phenyl]piperidin-1-yl}carbonyl)piperidin-4-yl]carbamat [racemisches cis-Isomer]

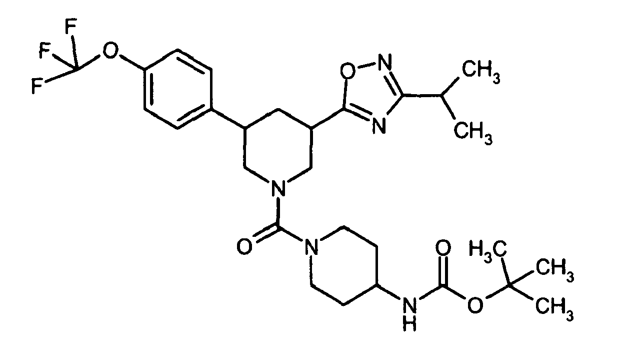

Nach der Allgemeinen Methode 2 wurden 144 mg (0.28 mmol) der Verbindung aus Beispiel 114A und 43 mg (0.42 mmol) *N'*-Hydroxy-2-methylpropanimidamid umgesetzt. Ausbeute: 54 mg (33% d. Th.)

LC-MS (Methode 9B): Rₜ = 1.37 min; MS (ESIpos): m/z = 582 [M+H]⁺;

### Beispiel 211A

### tert-Butyl-[1-({3-[3-(2-methoxyethyl)-1,2,4-oxadiazol-5-yl]-5-[4-(trifluormethoxy)phenyl]-piperidin-1-yl}carbonyl)piperidin-4-yl]carbamat [racemisches cis-Isomer]

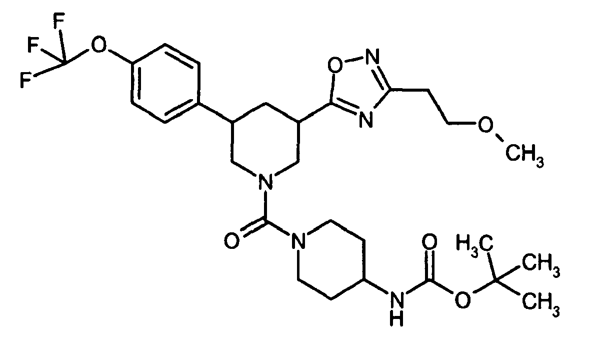

Nach der Allgemeinen Methode 2 wurden 200 mg (0.39 mmol) der Verbindung aus Beispiel 114A und 92 mg (0.58 mmol) *N'*-Hydroxy-3-methoxypropanimidamid umgesetzt. Ausbeute: 52 mg (23% d. Th.)

LC-MS (Methode 9B): Rₜ = 1.26 min; MS (ESIpos): m/z = 598 [M+H]⁺;

### Beispiel 212A

### tert-Butyl-[1-({3-(3-methyl-1,2,4-oxadiazol-5-yl)-5-[4-(trifluormethoxy)phenyl]piperidin-1-yl}carbonyl)piperidin-4-yl]carbamat [racemisches cis-Isomer]

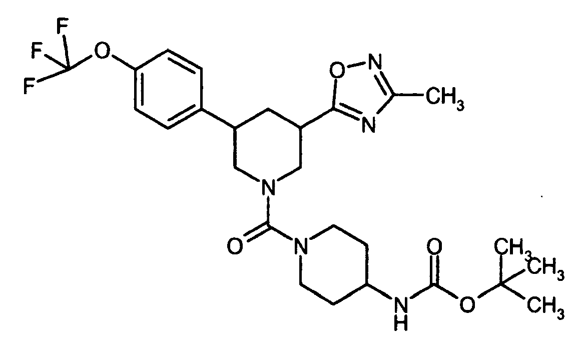

Nach der Allgemeinen Methode 2 wurden 200 mg (0.39 mmol) der Verbindung aus Beispiel 114A und 43 mg (0.58 mmol) *N'*-Hydroxyethanimidamid umgesetzt. Ausbeute: 73 mg (34% d. Th.)

LC-MS (Methode 9B): Rₜ = 1.26 min; MS (ESIpos): m/z = 554 [M+H]⁺.

### Beispiel 213A

### 2,4-Difluor-N'-hydroxybenzolcarboximidamid

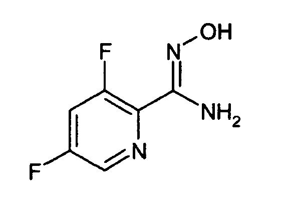

Nach der Allgemeinen Methode 5A wurden 1.00 g (7.14 mmol) 3,5-Difluorpyridin-2-carbonitril umgesetzt. Ausbeute: 893 mg (72% d. Th.)

HPLC (Methode 5B): Rₜ = 0.58 min; MS (ESIpos): m/z = 174 [M+H]⁺.

¹H-NMR (400 MHz, DMSO-d₆): δ = 10.1 (s, 1H), 8.55 (d, 1H), 8.00 (ddd, 1H), 5.89 (br s, 2H).

### Beispiel 214 A

### {3-[4-(Chlormethyl)-1,3-thiazol-2-yl]-5-[4-(trifluormethyl)phenyl]pipendin-1-yl}(morpholin-4-yl)methanon

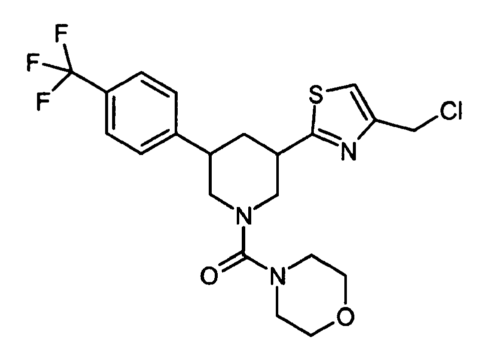

Nach der Allgemeinen Methode 3 wurden 250 mg (ca. 0.44 mmol) der Verbindung aus Beispiel 53A und 60 mg (0.52 mmol) 1,3-Dichlorpropan-2-on umgesetzt. Ausbeute: 150 mg (12% d. Th.).

LC-MS (Methode 2B): Rₜ = 1.30 min und 1.34 min (cis-/trans-Isomere); MS (ESIpos): m/z = 474 [M+H]⁺.

### Ausführungsbeispiele

### Allgemeine Methode 1: Oxadiazolbildung

Eine Lösung aus der entsprechenden Piperidin-3-carbonsäure in Dimethylformamid (10 ml/mmol) wird unter Argon bei RT mit HATU (1.2 eq.), *N,N-*Diisopropylethylamin (2.2 eq.) und dem entsprechenden *N'*-Hydroxyimidamid (1.1 eq.) versetzt. Das Reaktionsgemisch wird bei RT gerührt bis zur kompletten Bildung der Zwischenstufe, dann bei 120°C weiter gerührt bis zur Bildung des gewünschten Produktes aus dieser Zwischenstufe. Das Reaktionsgemisch wird anschließend mittels präparativer HPLC aufgereinigt.

### Allgemeine Methode 2: Oxadiazolbildung

Die Carbonsäure wird in Dioxan/Dimethylformamid (3:1, 1 ml/mmol) gelöst und auf 60°C erwärmt. Nach Zugabe von *N*,*N'*-Carbonyldiimidazol (1.5 eq.), gelöst in Dioxan/Dimethylformamid (4:1, 1.6 ml/mmol), wird 3 h bei 60°C gerührt. Nach Abkühlen auf RT wird das Carboximidamid, gelöst in Dioxan/Dimethylformamid 1:1, zugetropft und über Nacht bei 40°C gerührt. Danach wird das Dioxan im Vakuum entfernt. Der in Dimethylformamid gelöste Rückstand wird anschließend für 1 h bei 115°C gerührt. Die Reaktionsmischung wird nach dem Abkühlen mit Wasser verdünnt. Nach Extraktion mit Dichlormethan wird die organsiche Phase über Natriumsulfat getrocknet und das Rohprodukt mittels präparativer HPLC gereinigt.

### Allgemeine Methode 3: Thiazolbildung

Eine Mischung des entsprechenden Piperidin-3-carbonthiamid in Dimethylformamid (4.6 ml/mmol) und Bromketon oder Chlorketon (1.2 eq.) wird über Nacht bei 125°C gerührt. Das Reaktionsgemisch wird ohne weitere Aufarbeitung mittels präparativer HPLC aufgereinigt.

### Allgemeine Methode 4: 1,3,4-Oxadiazolbildung (J. Med. Chem. 1996, 39, 2753-2763)

Eine Lösung aus der entsprechenden Piperidin-3-carbonsäure in Phosphorylchlorid (1 ml/mmol) wird unter Argon 2 h bei Rückflußtemperatur mit dem entsprechenden Hydrazid (1.1 eq.) gerührt. Das Reaktionsgemisch wird auf Eis gegeben und mit Ammoniak auf pH 7 gestellt. Nach Extraktion mit Dichlormethan wird die organsiche Phase über Natriumsulfat getrocknet und einrotiert. Das Rohprodukt wird mittels präparativer HPLC gereinigt.

### Allgemeine Methode 5: Harnstoffbildung I

1.0 Äquivalente eines Piperidincarbonylchlorids und 1.0 Äquivalente Triethylamin werden mit 10 ml Dichlormethan vorgelegt. Bei 0°C werden 1.0 Äquivalente eines Amins zugegeben und 16 h bei RT gerührt. Das Reaktionsgemisch wird mit Wasser gewaschen, über Magnesiumsulfat getrocknet, filtriert und im Vakuum eingeengt. Der Rückstand wird mittels präparativer HPLC aufgereinigt.

### Allgemeine Methode 6: Harnstoffbildung II

1.0 Äquivalente eines 4-Nitrophenylpiperidincarboxylates, 3.0 Äquivalente Amin und 2.0 Äquivalente Kaliumcarbonat werden mit 3 ml *N,N* Dimethylformamid versetzt und 30 Minuten bei 150°C in der Mikrowelle umgesetzt. Das Reaktionsgemisch wird in Essigsäureethylester aufgenommen, mehrmals mit Wasser sowie einer gesättigten, wässrigen Natriumchlorid-Lösung gewaschen, über Magnesiumsulfat getrocknet, filtriert und im Vakuum eingeengt. Der Rückstand wird mittels präparativer HPLC aufgereinigt.

### Allgemeine Methode 7: Amidbildung

Eine Lösung aus 1.1 Äquivalenten der entsprechenden Carbonsäure in *N,N-*Dimethylformamid wird unter Argon bei RT mit HATU (1.2 eq.) und *N,N-*Diisopropylethylamin (3.0 eq.) versetzt. Nach 30 Minuten werden 1.0 Äquivalenten des entsprechenden Piperidinderivates, gelöst in *N,N-*Dimethylformamid, zugegeben. Das Reaktionsgemisch wird 16 h bei RT gerührt. Das Reaktionsgemisch wird anschließend mittels präparativer HPLC aufgereinigt.

### Allgemeine Methode 8: Oxadiazolbildung

Je 0.1 mmol der entsprechenden Carbonsäuren werden auf eine Mikrotiterplatte verteilt. 1.55 g (9.57 mmol) N,N-Carbonyldiimidazol wird in 10 ml DMF und 5 ml Dioxan gelöst und je 1/48 zu jeder der 48 Carbonsäuren pipettiert. Es wird 3 h bei 60°C geschüttelt, danach gibt man 1/48 der Lösung von 1.922 g (4.8 mmol) der Verbindung aus Beispiel 84A in DMF zu, schüttelt 2 h bei 40°C und anschließend 3 h bei 115°C. Das Lösemittel wird in einer Vakuumzentrifuge abgedampft, der Rückstand wird in je 0.6 ml DMSO gelöst, filtriert und per präparativer HPLC/MS gereinigt.

### Allgemeine Methode 9: Thiadiazolbildung (J. Med. Chem. 1996, 39, 2753-2763)

Eine Lösung aus dem entsprechenden Carbohydrazid in Dioxan (20 ml/mmol) wird unter Argon 2.5 h bei Rückflußtemperatur mit dem Lawesson-Reagens (2,4-Bis[4-methoxyphenyl]1,3-dithia-2,4-diphosphetan-2,4-disulfid) (2.0 eq.) versetzt. Nach Zugabe von gesättigter, wässriger Natriumhydrogencarbonat-Lösung und Entfernung des Dioxans wird der Rückstand mit Ethylacetat extrahiert. Die vereinigte organische Phase wird mit einer gesättigten, wässrigen Natriumhydrogencarbonat-Lösung gewaschen, über Magnesiumsulfat getrocknet, filtriert und im Vakuum eingeengt.

### Beispiel 1

### 1-(Cyclopentylcarbonyl)-3-(4-ethylphenyl)-5-(3-phenyl-1,2,4-oxadiazol-5-yl)piperidin [racemisches cis-Isomer]

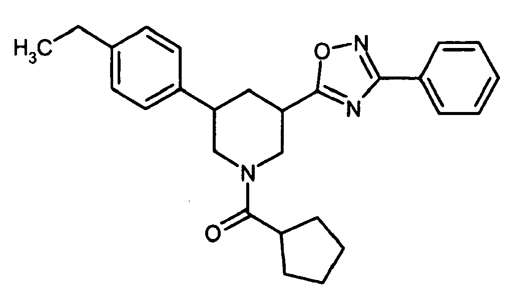

Nach der Allgemeinen Methode 1 wurden 329 mg (1.0 mmol) 1-(Cyclopentylcarbonyl)-5-(4-ethylphenyl)piperidin-3-carbonsäure (Beispiel 7A) und 149mg (1.1 mmol, 1.1 eq.) *N'-*Hydroxybenzencarboximidamid umgesetzt. Ausbeute: 235 mg (55% d. Th.)

HPLC (Methode 2A): Rₜ = 5.64 min; MS (ESIpos): m/z = 430 [M+H]⁺;

¹H-NMR (500 MHz, DMSO-d₆): δ = 8.01 (d, 2H), 7.63-7.54 (m, 3H), 7.28 (d, 1H), 7.23 (d, 1H), 7.19 (d, 2H), 4.95 (br d, 0.5H), 4.53 (br d, 0.5H), 4.48 (br d, 0.5H), 4.03 (br d, 0.5H), 3.50-3.40 (m, 1H), 3.37-3.28 (m, 0.5H), 3.27-3.20 (m, 0.5H), 3.19-3.01 (m, 1H), 2.95-2.82 (m, 1H), 2.70-2.68 (m, 1H), 2.58 (q, 2H), 2.42-2.33 (m, 1H), 2.20-2.02 (m, 1H), 1.90-1.47 (m, 8H), 1.17 (t, 3H).

### Beispiel 2

### 1-(Cyclopentylcarbonyl)-3-(4-ethylphenyl)-5-(3-phenyl-1,2,4-oxadiazol-5-yl)piperidin [enantiomerenreines cis-Isomer]

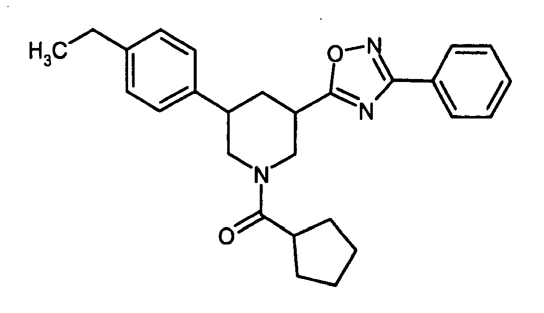

Enantiomerentrennung von 235 mg des Racemates aus Beispiel 1 nach Methode 1D ergab 117 mg der Titelverbindung aus Beispiel 2 (Enantiomer 1) und 119 mg der Titelverbindung aus Beispiel 3 (Enantiomer 2).

HPLC (Methode 1E): Rₜ = 5.25 min, >99.5% ee; MS (ESIpos): m/z = 430 [M+H]⁺.

### Beispiel 3

### 1-(Cyclopentylcarbonyl)-3-(4-ethylphenyl)-5-(3-phenyl-1,2,4-oxadiazol-5-yl)piperidin [enantiomerenreines cis-Isomer]

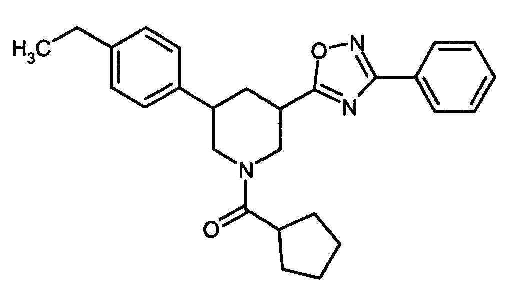

Enantiomerentrennung von 235 mg des Racemates aus Beispiel 1 nach Methode 1D ergab 117 mg der Titelverbindung aus Beispiel 2 (Enantiomer 1) und 119 mg der Titelverbindung aus Beispiel 3 (Enantiomer 2).

HPLC (Methode 1E): Rₜ = 6.98 min, >99.5% ee; MS (ESIpos): m/z = 430 [M+H]⁺.

### Beispiel 4

### Cis-(3,5)-1-(Cyclopentylcarbonyl)-3-[4-(1-methylethyl)phenyl]-5-(3-phenyl-1,2,4-oxadiazol-5-yl)piperidin [racemisches cis-Isomer]

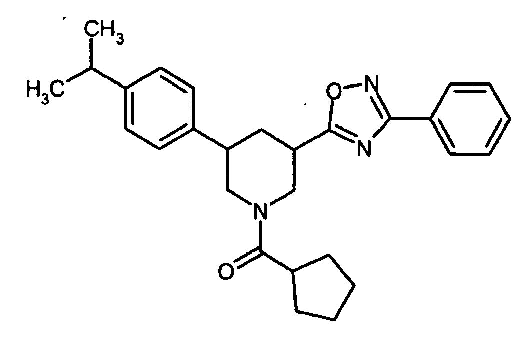

Nach der Allgemeinen Methode 2 wurden 60 mg (0.18 mmol) cis-(3,5)-1-(Cyclopentylcarbonyl)-5-[4-(1-methylethyl)phenyl]piperidin-3-carbonsäure (Beispiel 12A) und 36 mg (0.26 mmol, 1.5 eq.) *N-*Hydroxybenzencarboximidamid umgesetzt. Ausbeute: 64 mg (83% d. Th.)

LC-MS (Methode 1B): Rₜ = 3.28 min; MS (ESIpos): m/z = 444 [M+H]⁺.

### Beispiel 5

### Cis-(3,5)-1-(Cyclopentylcarbonyl)-3-[4-(1-methylethyl)phenyl]-5-(3-phenyl-1,2,4-oxadiazol-5-yl)piperidin [enantiomerenreines cis-Isomer]

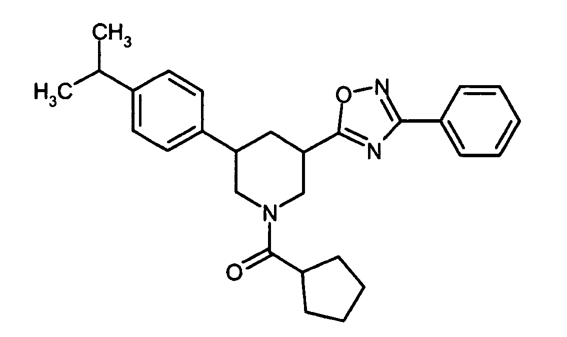

Enantiomerentrennung von 58 mg des Racemates aus Beispiel 4 nach Methode 2D ergab 15 mg der Titelverbindung aus Beispiel 5 (Enantiomer 1) und 17 mg der Titelverbindung aus Beispiel 6 (Enantiomer 2).

HPLC (Methode 2E): Rₜ = 7.09 min.

### Beispiel 6

### Cis-(3,5)-1-(Cyclopentylcarbonyl)-3-[4-(1-methylethyl)phenyl]-5-(3-phenyl-1,2,4-oxadiazol-5-yl)piperidin [enantiomerenreines cis-Isomer]

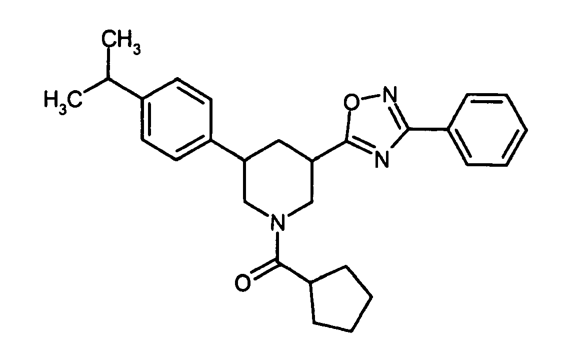

Enantiomerentrennung von 58 mg des Racemates aus Beispiel 4 nach Methode 2D ergab 15 mg der Titelverbindung aus Beispiel 5 (Enantiomer 1) und 17 mg der Titelverbindung aus Beispiel 6 (Enantiomer 2).

HPLC (Methode 2E): Rₜ = 7.79 min.

### Beispiel 7

### Cis-(3,5)-1-(Cyclopentylcarbonyl)-3-(3-phenyl-1,2,4-oxadiazol-5-yl)-5-[4-(trifluormethyl)phenyl]-piperidin [racemisches cis-Isomer]

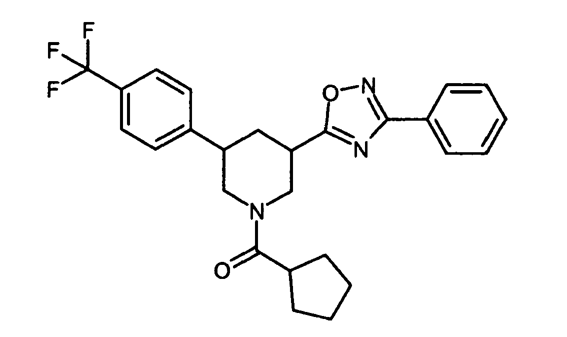

Nach der Allgemeinen Methode 2 wurden 150 mg (0.41 mmol) cis-(3,5)-1-(Cyclopentylcarbonyl)-5-[4-(trifluormethyl)phenyl]piperidin-3-carbonsäure (Beispiel 17A) und 83 mg (0.61 mmol, 1.5 eq.) *N'-*Hydroxybenzencarboximidamid umgesetzt. Ausbeute: 152 mg (80% d. Th.)

LC-MS (Methode 1B): Rₜ = 3.28 min; MS (ESIpos): m/z = 470 [M+H]⁺;

¹H-NMR (400 MHz, DMSO-d₆): δ = 8.05-7.99 (dd, 2H), 7.67-7.54 (t, 2H), 7.86 (m, 5H), 4.96 (br d, 0.5H), 4.57 (br dd, 1H), 4.09 (br d, 0.5H), 3.98 (br d, 2H), 3.19-2.78 (m, 3H), 2.42 (br t, 1H), 2.28-2.10 (m, 1H), 1.91-1.46 (m, 8H).

### Beispiel 8

### Cis-(3,5)-1-(Cyclopentylcarbonyl)-3-(3-phenyl-1,2,4-oxadiazol-5-yl)-5-[4-(trifluormethyl)phenyl]-piperidin [enantiomerenreines cis-Isomer]

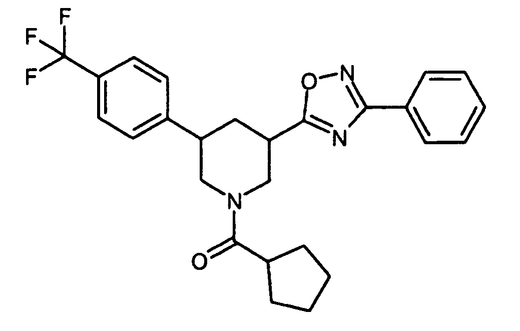

Enantiomerentrennung von 142 mg des Racemates aus Beispiel 7 nach Methode 3D ergab 58 mg der Titelverbindung aus Beispiel 8 (Enantiomer 1) und 58 mg der Titelverbindung aus Beispiel 9 (Enantiomer 2).

HPLC (Methode 3E): Rₜ = 5.62 min.

### Beispiel 9

### Cis-(3,5)-1-(Cyclopentylcarbonyl)-3-(3-phenyl-1,2,4-oxadiazol-5-yl)-5-[4-(trifluormethyl)phenyl]-piperidin [enantiomerenreines cis-Isomer]

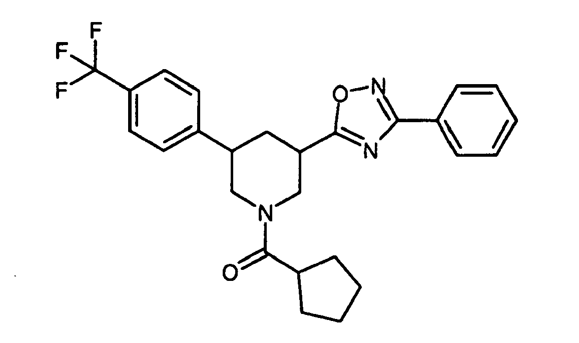

Enantiomerentrennung von 142 mg des Racemates aus Beispiel 7 nach Methode 3D ergab 58 mg der Titelverbindung aus Beispiel 8 (Enantiomer 1) und 58 mg der Titelverbindung aus Beispiel 9 (Enantiomer 2).

HPLC (Methode 3E): Rₜ = 6.16 min.

### Beispiel 10

### Cis-(3,5)-1-(Cyclopentylcarbonyl)-3-(3-phenyl-1,2,4-oxadiazol-5-yl)-5-[4-(trifluormethoxy)-phenyl]piperidin [racemisches cis-Isomer]

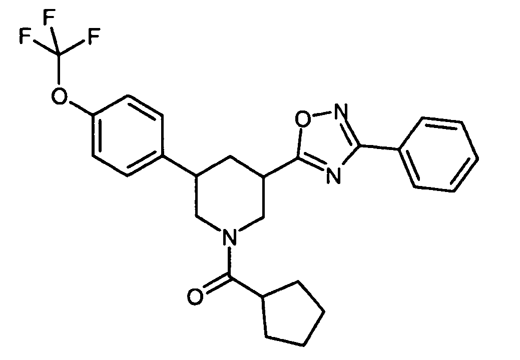

Nach der Allgemeinen Methode 2 wurden 60 mg (0.16 mmol) cis-(3,5)-1-(Cyclopentylcarbonyl)-5-[4-(trifluormethoxy)phenyl]piperidin-3-carbonsäure (Beispiel 24A) und 32 mg (0.23 mmol, 1.5 eq.) *N-*Hydroxybenzencarboximidamid umgesetzt. Ausbeute: 62 mg (83% d. Th.)

LC-MS (Methode 3B): Rₜ = 3.33 min; MS (ESIpos): m/z = 486 [M+H]⁺.

### Beispiel 11

### Cis-(3,5)-1-(2,2-Dimethylpropanoyl)-3-(4-methoxyphenyl)-5-(3-phenyl-1,2,4-oxadiazol-5-yl)piperidin [racemisches cis-Isomer]

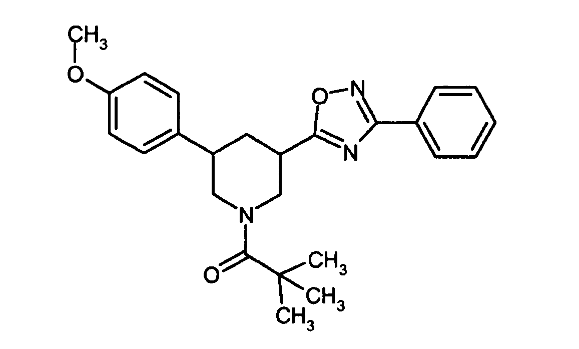

Nach der Allgemeinen Methode 2 wurden 150 mg (0.41 mmol) cis-(3,5)-1-(2,2-Dimethylpropanoyl)-5-(4-methoxyphenyl)piperidin-3-carbonsäure (Beispiel 33A) und 96 mg (0.70 mmol, 1.5 eq.) *N'*-Hydroxybenzencarboximidamid umgesetzt. Ausbeute: 126 mg (64% d. Th.)

LC-MS (Methode 3B): Rₜ = 2.49 min; MS (ESIpos): m/z = 420 [M+H]⁺.

### Beispiel 12

### Cis-(3,5)-1-(2,2-Dimethylpropanoyl)-3-(4-methoxyphenyl)-5-(3-phenyl-1,2,4-oxadiazol-5-yl)piperidin [enantiomerenreines cis-Isomer]

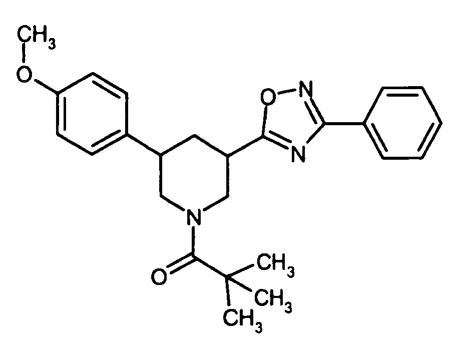

Enantiomerentrennung von 119 mg des Racemates aus Beispiel 11 nach Methode 4D ergab 20 mg der Titelverbindung aus Beispiel 12 (Enantiomer 1) und 17 mg der Titelverbindung aus Beispiel 13 (Enantiomer 2).

HPLC (Methode 4E): Rₜ = 5.72 min.

### Beispiel 13

### Cis-(3,5)-1-(2,2-Dimethylpropanoyl)-3-(4-methoxyphenyl)-5-(3-phenyl-1,2,4-oxadiazol-5-yl)piperidin [enantiomerenreines cis-Isomer]

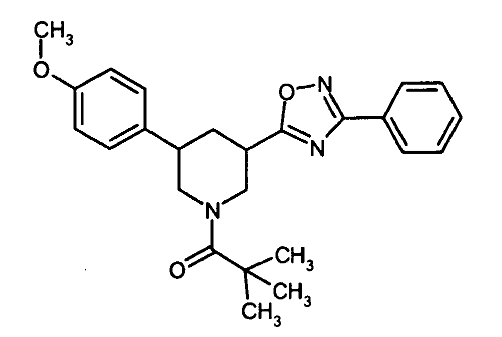

Enantiomerentrennung von 119 mg des Racemates aus Beispiel 11 nach Methode 4D ergab 20 mg der Titelverbindung aus Beispiel 12 (Enantiomer 1) und 17 mg der Titelverbindung aus Beispiel 13 (Enantiomer 2).

HPLC (Methode 4E): Rₜ = 6.42 min.

### Beispiel 14

### 4-{[3-(4-Ethylphenyl)-5-(3-phenyl-1,2,4-oxadiazol-5-yl)piperidin-1-yl]carbonyl}morpholin [racemisches cis-Isomer]

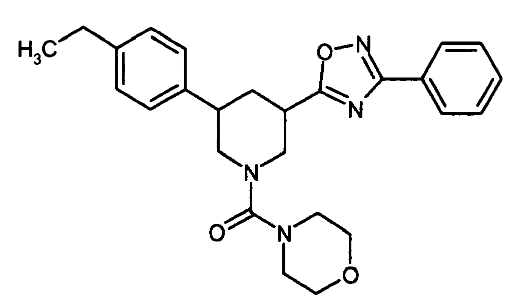

Nach der Allgemeinen Methode 1 wurden 400 mg (1.16 mmol) 5-(4-Ethylphenyl)-1-(morpholin-4-ylcarbonyl)piperidin-3-carbonsäure (Beispiel 38A) und 173 mg (1.27 mmol, 1.1 eq.) *N'-*Hydroxybenzencarboximidamid umgesetzt. Ausbeute: 373 mg (72% d. Th.)

HPLC (Methode 3A): Rₜ = 4.98 min; MS (ESIpos): m/z = 447 [M+H]⁺;

¹H-NMR (400 MHz, DMSO-d₆): δ = 8.01 (d, 2H), 7.63-7.52 (m, 3H), 7.25 (d, 2H), 7.18 (d, 2H), 4.09 (br d, 1H), 3.62 (br d, 1H), 3.60-3.54 (m, 4H), 3.53-3.44 (m, 1H), 3.26-3.17 (m, 4H), 3.11 (t, 1H), 2.97 (q, 1H), 2.94-2.85 (m, 1H), 2.58 (q, 2H), 2.38 (br d, 1H), 2.04 (q, 1H), 1.17 (t, 3H).

### Beispiels 15

### 4-{[3-(4-Ethylphenyl)-5-(3-phenyl-1,2,4-oxadiazol-5-yl)piperidin-1-yl]carbonyl}morpholin [enantiomerenreines cis-Isomer]

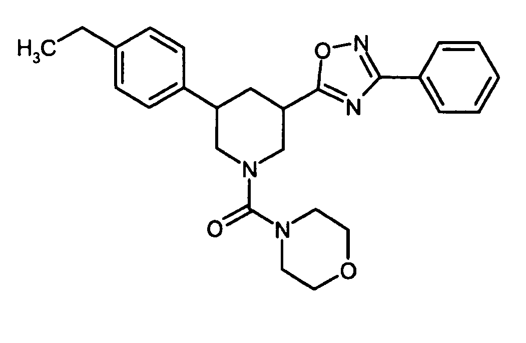

Enantiomerentrennung von 440 mg des Racemates aus Beispiel 14 nach Methode 5D ergab 187 mg der Titelverbindung aus Beispiel 15 (Enantiomer 1) und 190 mg der Titelverbindung aus Beispiel 16 (Enantiomer 2).

HPLC (Methode 5E): Rₜ = 7.95 min, >99.5% ee; MS (ESIpos): m/z = 447 [M+H]⁺.

### Beispiel 16

### 4-{[3-(4-Ethylphenyl)-5-(3-phenyl-1,2,4-oxadiazol-5-yl)piperidin-1-yl]carbonyl}morpholin [enantiomerenreines cis-Isomer]

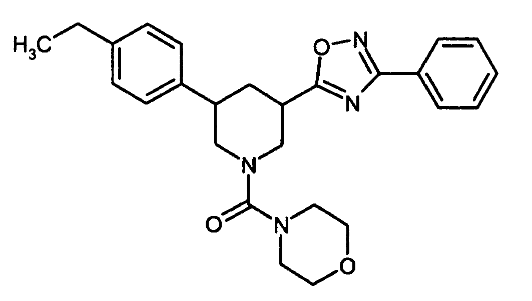

Enantiomerentrennung von 440 mg des Racemates aus Beispiel 14 nach Methode 5D ergab 187 mg der Titelverbindung aus Beispiel 15 (Enantiomer 1) und 190 mg der Titelverbindung aus Beispiel 16 (Enantiomer 2).

HPLC (Methode 5E): Rₜ = 11.13 min, >99.5% ee; MS (ESIpos): m/z = 447 [M+H]⁺.

### Beispiel 17

### 4-({3-(3-Phenyl-1,2,4-oxadiazol-5-yl)-5-[4-(trifluormethoxy)phenyl]piperidin-1-yl}carbonyl)-morpholin [racemisches cis-Isomer]

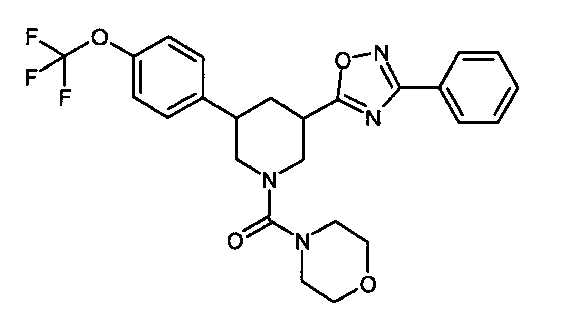

Nach der Allgemeinen Methode 1 wurden 80 mg (0.20 mmol) 1-(Morpholin-4-ylcarbonyl)-5-[4-(trifluormethoxy)phenyl]piperidin-3-carbonsäure (Beispiel 44A) und 30 mg (0.22 mmol, 1.1 eq.) *N'-*Hydroxybenzencarboximidamid umgesetzt. Ausbeute: 10 mg (10% d. Th.)

HPLC (Methode 2A): Rₜ = 5.15 min; MS (ESIpos): m/z = 503 [M+H]⁺;

¹H-NMR (500 MHz, DMSO-d₆): δ = 8.01 (d, 2H), 7.62-7.55 (m, 2H), 7.50 (d, 2H), 7.42 (t, 1H), 7.35 (d, 2H), 4.09 (br d, 1H), 3.64 (br d, 1H), 3.61-3.55 (m, 4H), 3.55-3.46 (m, 1H), 3.25-3.19 (m, 4H), 3.14 (t, 1H), 3.07-2.97 (m, 2H), 2.40 (br d, 1H), 2.07 (q, 1H).

### Beispiel 18

### 4-({3-[3-(2-Chlorphenyl)-1,2,4-oxadiazol-5-yl]-5-(4-ethylphenyl)piperidin-1-yl}carbonyl)-morpholin [racemisches cis-Isomer]

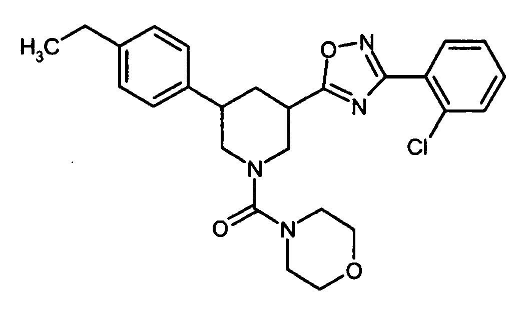

Nach der Allgemeinen Methode 1 wurden 69 mg (0.20 mmol) 5-(4-Ethylphenyl)-1-(morpholin-4-ylcarbonyl)piperidin-3-carbonsäure (Beispiel 38A) und 38 mg (0.22 mmol, 1.1 eq.) 2-Chlor-*N'-*hydroxybenzencarboximidamid umgesetzt. Ausbeute: 49 mg (51% d. Th.)

HPLC (Methode 3A): Rₜ = 5.01 min; MS (ESIpos): m/z = 481 [M+H]⁺;

¹H-NMR (400 MHz, DMSO-d₆): δ = 7.92 (dd, 1H), 7.68 (dd, 1H), 7.61 (dt, 1H), 7.54 (dt, 1H), 7.25 (d, 2H), 7.18 (d, 2H), 4.10 (br d, 1H), 3.62 (br d, 1H), 3.61-3.54 (m, 4H), 3.58-3.47 (m, 1H), 3.25-3.18 (m, 4H), 3.11 (t, 1H), 2.97 (q, 1H), 2.95-2.86 (m, 1H), 2.58 (q, 2H), 2.38 (br d, 1H), 2.04 (q, 1H), 1.17 (t, 3H).

### Beispiel 19

### 4-({3-[3-(2-Chlorphenyl)-1,2,4-oxadiazol-5-yl]-5-[4-(trifluormethoxy)phenyl]piperidin-1-yl}carbonyl)morpholin [racemisches cis-Isomer]

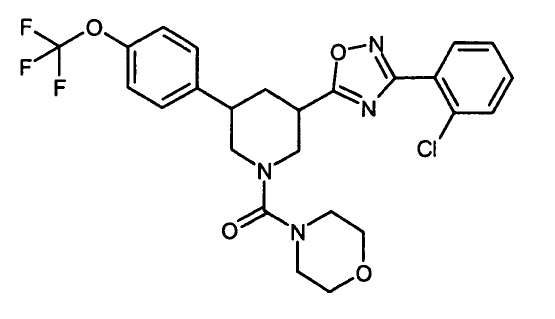

Nach der Allgemeinen Methode 1 wurden 80 mg (0.20 mmol) 1-(Morpholin-4-ylcarbonyl)-5-[4-(trifluormethoxy)phenyl]piperidin-3-carbonsäure (Beispiel 44A) und 38 mg (0.22 mmol, 1.1 eq.) 2-Chlor-*N'-*hydroxybenzencarboximidamid umgesetzt. Ausbeute: 56 mg (52% d. Th.)

HPLC (Methode 2A): Rₜ = 5.17 min; MS (ESIpos): m/z = 537 [M+H]⁺;

¹H-NMR (400 MHz, DMSO-d₆): δ = 7.92 (dd, 1H), 7.69 (dd, 1H), 7.61 (dt, 1H), 7.54 (dt, 1H), 7.49 (d, 2H), 7.34 (d, 2H), 4.09 (br d, 1H), 3.64 (br d, 1H), 3.62-3.48 (m, 5H), 3.26-3.18 (m, 4H), 3.13 (t, 1H), 3.07-2.97 (m, 2H), 2.42 (br d, 1H), 2.07 (q, 1H).

### Beispiel 20

### 4-({3-(4-Ethylphenyl)-5-[3-(2-methylphenyl)-1,2,4-oxadiazol-5-yl]piperidin-1-yl}carbonyl)-morpholin [racemisches cis-Isomer]

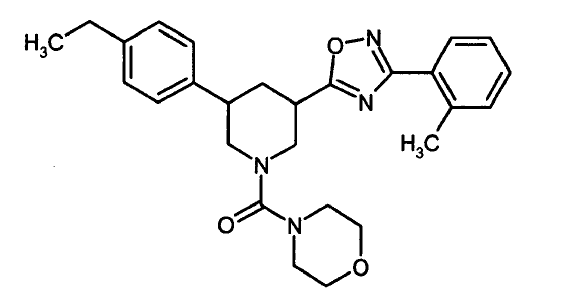

Nach der Allgemeinen Methode 1 wurden 69 mg (0.20 mmol) 5-(4-Ethylphenyl)-1-(morpholin-4-ylcarbonyl)piperidin-3-carbonsäure (Beispiel 38A) und 33 mg (0.22 mmol, 1.1 eq.) *N'-*Hydroxy-2-methylbenzencarboximidamid umgesetzt. Ausbeute: 62 mg (68% d. Th.)

HPLC (Methode 1A): Rₜ = 5.12 min; MS (ESIpos): m/z = 461 [M+H]⁺;

¹H-NMR (400 MHz, DMSO-d₆): δ = 7.93 (d, 1H), 7.51-7.44 (m, 1H), 7.43-7.35 (m, 2H), 7.25 (d, 2H), 7.18 (d, 2H), 4.10 (br d, 1H), 3.62 (br d, 1H), 3.60-3.53 (m, 4H), 3.52-3.44 (m, 1H), 3.25-3.18 (m, 4H), 3.11 (t, 1H), 2.97 (q, 1H), 2.95-2.86 (m, 1H), 2.58 (q, 2H), 2.56 (s, 3H), 2.38 (br d, 1H), 2.04 (q, 1H), 1.17 (t, 3H).

### Beispiel 21

### 4-({3-[3-(2-Methylphenyl)-1,2,4-oxadiazol-5-yl]-5-[4-(trifluormethoxy)phenyl]piperidin-1-yl}carbonyl)morpholin [racemisches cis-Isomer]

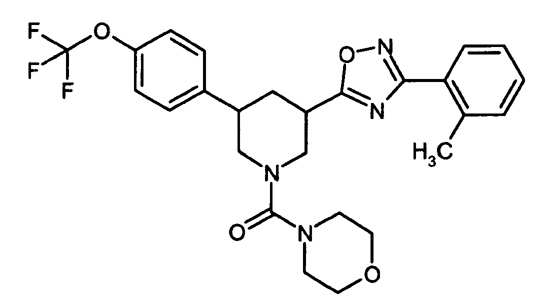

Nach der Allgemeinen Methode 1 wurden 80 mg (0.20 mmol) 1-(Morpholin-4-ylcarbonyl)-5-[4-(trifluormethoxy)phenyl]piperidin-3-carbonsäure (Beispiel 44A) und 33 mg (0.22 mmol, 1.1 eq.) *N'-*Hydroxy-2-methylbenzencarboximidamid umgesetzt. Ausbeute: 14 mg (13% d. Th.)

HPLC (Methode 2A): Rₜ = 5.30 min; MS (ESIpos): m/z = 517 [M+H]⁺;

¹H-NMR (400 MHz, DMSO-d₆): δ = 7.92 (dd, 1H), 7.53-7.45 (m, 3H), 7.43-7.33 (m, 4H), 4.09 (br d, 1H), 3.64 (br d, 1H), 3.60-3.55 (m, 4H), 3.54-3.46 (m, 1H), 3.24-3.20 (m, 4H), 3.14 (t, 1H), 3.06-2.99 (m, 2H), 2.42 (br d, 1H), 2.07 (q, 1H).

### Beispiel 22

### 1-(Cyclopentylcarbonyl)-3-(4-ethylphenyl)-5-[3-(3-fluorphenyl)-1,2,4-oxadiazol-5-yl]piperidin [racemisches cis-Isomer]

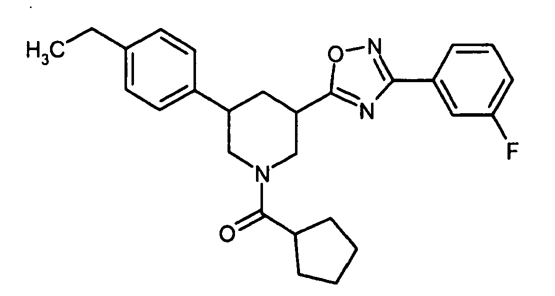

Nach der Allgemeinen Methode 1 wurden 66 mg (0.20 mmol) 1-(Cyclopentylcarbonyl)-5-(4-ethylphenyl)piperidin-3-carbonsäure (Beispiel 7A) und 34 mg (0.22 mmol, 1.1 eq.) 3-Fluor-*N'-*hydroxybenzencarboximidamid umgesetzt. Ausbeute: 47 mg (53% d. Th.)

HPLC (Methode 2A): Rₜ = 5.78 min; MS (ESIpos): m/z = 448 [M+H]⁺;

¹H-NMR (500 MHz, DMSO-d₆): δ = 7.87 (d, 1H), 7.76 (d, 1H), 7.64 (q, 1H), 7.47 (t, 1H), 7:28 (d, 1H), 7.23 (d, 1H), 7.19 (t, 2H), 4.94 (br d, 0.5H), 4.53 (br d, 0.5H), 4.48 (br d, 0.5H), 4.03 (br d, 0.5H), 3.50-3.40 (m, 1H), 3.38-3.28 (m, 0.5H), 3.27-3.17 (m, 0.5H), 3.17-3.03 (m, 1H), 2.94-2.83 (m, 1H), 2.81-2.68 (m, 1H), 2.58 (q, 2H), 2.44-2.34 (m, 1H), 2.19-2.03 (m, 1H), 1.89-1.46 (m, 8H), 1.17 (t, 3H).

### Beispiel 23

### Cis-(3,5)-1-(Cyclopentylcarbonyl)-3-[3-(3-fluorphenyl)-1,2,4-oxadiazol-5-yl]-5-[4-(trifluormethyl)phenyl]piperidin [racemisches cis-Isomer]

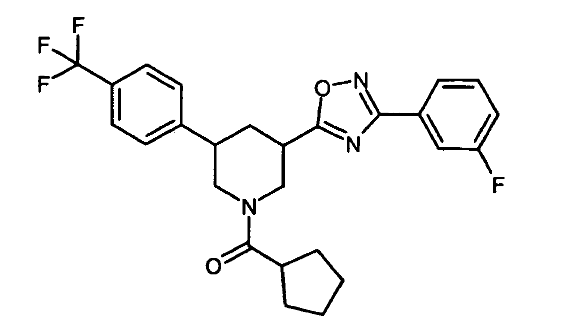

Nach der Allgemeinen Methode 2 wurden 150 mg (0.41 mmol) cis-(3,5)-1-(Cyclopentylcarbonyl)-5-[4-(trifluormethyl)phenyl]piperidin-3-carbonsäure (Beispiel 17A) und 93 mg (0.61 mmol, 1.5 eq.) 3-Fluor-*N'*- Hydroxybenzencarboximidamid umgesetzt. Ausbeute: 150 mg (76% d. Th.)

LC-MS (Methode 1B): Rₜ = 3.31 min; MS (ESIpos): m/z = 488 [M+H]⁺.

### Beispiel 24

### Cis-(3,5)-1-(Cyclopentylcarbonyl)-3-[3-(3-fluorphenyl)-1,2,4-oxadiazol-5-yl]-5-[4-(trifluormethoxy)phenyl]piperidin [racemisches cis-Isomer]

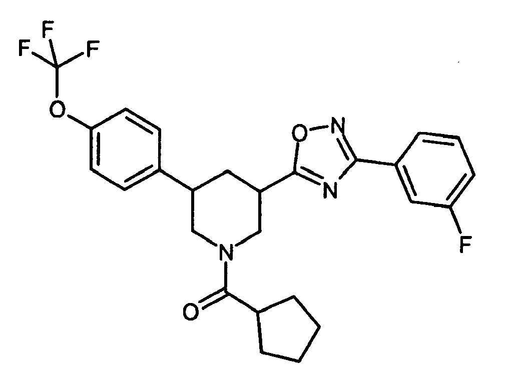

Nach der Allgemeinen Methode 2 wurden 70 mg (0.18 mmol) cis-(3,5)-1-(Cyclopentylcarbonyl)-5-[4-(trifluormethoxy)phenyl]piperidin-3-carbonsäure (Beispiel 24A) und 42 mg (0.27 mmol, 1.5 eq.) 3-Fluor-*N'-*hydroxybenzencarboximidamid umgesetzt. Ausbeute: 70 mg (76% d. Th.)

LC-MS (Methode 1B): Rₜ = 3.36 min; MS (ESIpos): m/z = 504 [M+H]⁺.

### Beispiel 25

### 4-({3-(4-Ethylphenyl)-5-[3-(3-fluorphenyl)-1,2,4-oxadiazol-5-yl]piperidin-1-yl}carbonyl)-morpholin [racemisches cis-Isomer]

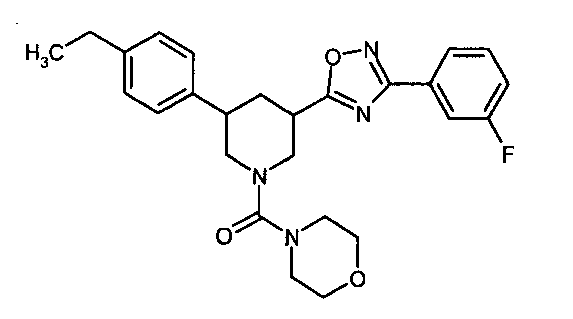

Nach der Allgemeinen Methode 1 wurden 69 mg (0.20 mmol) 5-(4-Ethylphenyl)-1-(morpholin-4-ylcarbonyl)piperidin-3-carbonsäure (Beispiel 38A) und 34 mg (0.22 mmol, 1.1 eq.) 3-Fluor-*N'-*hydroxybenzencarboximidamid umgesetzt. Ausbeute: 55 mg (58% d. Th.)

HPLC (Methode 3A): Rₜ = 5.06 min; MS (ESIpos): m/z = 465 [M+H]⁺;

¹H-NMR (400 MHz, DMSO-d₆): δ = 7.81 (d, 1H), 7.76 (d, 1H), 7.64 (dt, 1H), 7.46 (dt, 1H), 7.24 (d, 2H), 7.18 (d, 2H), 4.09 (br d, 1H), 3.63 (br d, 1H), 3.62-3.54 (m, 4H), 3.54-3.45 (m, 1H), 3.26-3.18 (m, 4H), 3.11 (t, 1H), 2.97 (q, 1H), 2.94-2.84 (m, 1H), 2.58 (q, 2H), 2.38 (br d, 1H), 2.05 (q, 1H), 1.17 (t, 3H).

### Beispiel 26

### 4-({3-[3-(3-Fluorphenyl)-1,2,4-oxadiazol-5-yl]-5-[4-(trifluormethoxy)phenyl]piperidin-1-yl}carbonyl)morpholin [racemisches cis-Isomer]

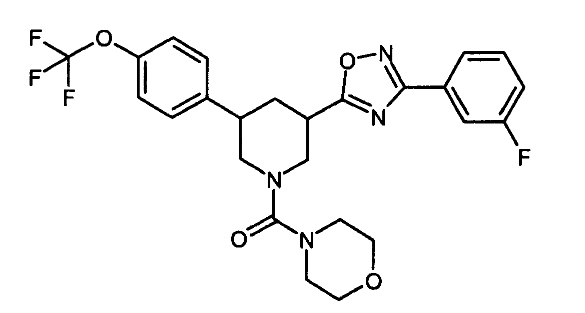

Nach der Allgemeinen Methode 1 wurden 80 mg (0.20 mmol) 1-(Morpholin-4-ylcarbonyl)-5-[4-(trifluonnethoxy)phenyl]piperidin-3-carbonsäure (Beispiel 44A) und 34 mg (0.22 mmol, 1.1 eq.) 3-Fluor-*N'-*hydroxybenzencarboximidamid umgesetzt. Ausbeute: 71 mg (68% d. Th.)

HPLC (Methode 2A): Rₜ = 5.21 min; MS (ESIpos): m/z = 521 [M+H]⁺;

¹H-NMR (400 MHz, DMSO-d₆): δ = 7.88 (d, 1H), 7.76 (d, 1H), 7.64 (dd, 1H), 7.54-7.44 (m, 3H), 7.34 (d, 2H), 4.09 (br d, 1H), 3.64 (br d, 1H), 3.62-3.53 (m, 4H), 3.56-3.47 (m, 1H), 3.26-3.18 (m, 4H), 3.13 (t, 1H), 3.08-2.97 (m, 2H), 2.41 (br d, 1H), 2.07 (q, 1H).

### Beispiel 27

### 4-({3-(4-Ethylphenyl)-5-[3-(3-methylphenyl)-1,2,4-oxadiazol-5-yl]piperidin-1-yl}carbonyl)-morpholin [racemisches cis-Isomer]

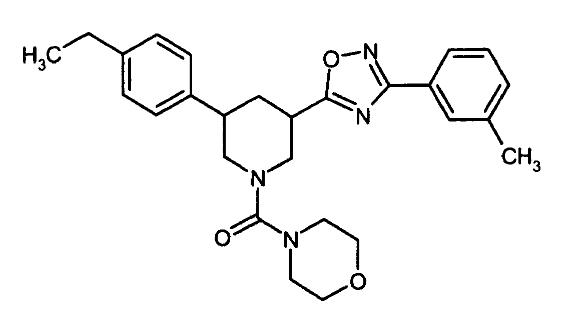

Nach der Allgemeinen Methode 1 wurden 69 mg (0.20 mmol) 5-(4-Ethylphenyl)-1-(morpholin-4-ylcarbonyl)piperidin-3-carbonsäure (Beispiel 38A) und 33 mg (0.22 mmol, 1.1 eq.) *N'*-Hydroxy-3-methylbenzencarboximidamid umgesetzt. Ausbeute: 16 mg (18% d. Th.)

HPLC (Methode 1A): Rₜ = 5.45 min; MS (ESIpos): m/z = 461 [M+H]⁺;

¹H-NMR (400 MHz, DMSO-d₆): δ = 7.84 (s, 1H), 7.81 (d, 1H), 7.45 (t, 1H), 7.42 (t, 1H), 7.25 (d, 2H), 7.18 (d, 2H), 4.09 (br d, 1H), 3.63 (br d, 1H), 3.63-3.53 (m, 4H), 3.53-3.43 (m, 1H), 3.26-3.18 (m, 4H), 3.11 (t, 1H), 2.97 (q, 1H), 2.94-2.85 (m, 1H), 2.58 (q, 2H), 2.40 (s, 3H), 2.38 (br d, 1H), 2.04 (q, 1H), 1.17 (t, 3H).

### Beispiel 28

### 4-({3-(4-Ethylphenyl)-5-[3-(3-methoxyphenyl)-1,2,4-oxadiazol-5-yl]piperidin-1-yl}carbonyl)-morpholin [racemisches cis-Isomer]

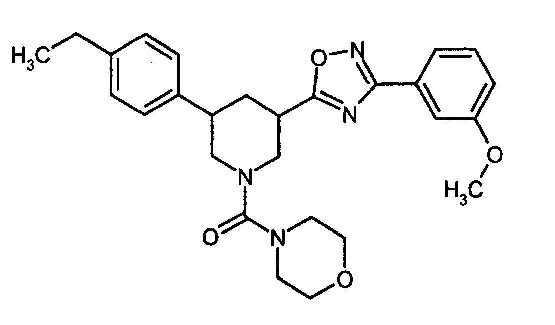

Nach der Allgemeinen Methode 1 wurden 69 mg (0.20 mmol) 5-(4-Ethylphenyl)-1-(morpholin-4-ylcarbonyl)piperidin-3-carbonsäure (Beispiel 38A) und 37 mg (0.22 mmol, 1.2 eq.) *N'-*Hydroxy-3-methoxybenzencarboximidamid umgesetzt. Ausbeute: 60 mg (63% d. Th.)

HPLC (Methode 1A): Rₜ = 5.25 min; MS (ESIpos): m/z = 477 [M+H]⁺;

¹H-NMR (400 MHz, DMSO-d₆): δ = 7.60 (d, 1H), 7.53-7.46 (m, 2H), 7.25 (d, 2H), 7.21-7.13 (m, 3H), 4.09 (br d, 1H), 3.84 (s, 3H), 3.62 (br d, 1H), 3.62-3.54 (m, 4H), 3.54-3.43 (m, 1H), 3.24-3.18 (m, 4H), 3.11 (t, 1H), 2.97 (q, 1H), 2.95-2.85 (m, 1H), 2.58 (q, 2H), 2.37 (br d, 1H), 2.04 (q, 1H), 1.17 (t, 3H).

### Beispiel 29

### 4-({3-[3-(3-Chlorphenyl)-1,2,4-oxadiazol-5-yl]-5-(4-ethylphenyl)piperidin-1-yl}carbonyl)-morpholin [racemisches cis-Isomer]

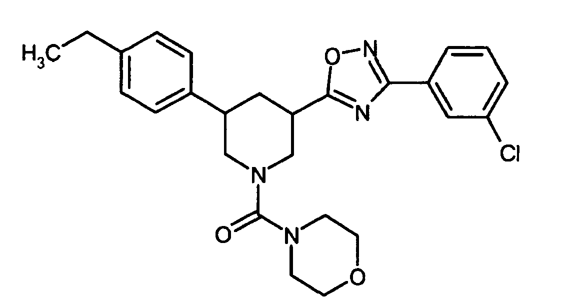

Nach der Allgemeinen Methode 1 wurden 69 mg (0.20 mmol) 5-(4-Ethylphenyl)-1-(morpholin-4-ylcarbonyl)piperidin-3-carbonsäure (Beispiel 38A) und 38 mg (0.22 mmol, 1.1 eq.) 3-Chlor-*N'-*hydroxybenzencarboximidamid umgesetzt. Ausbeute: 40 mg (42% d. Th.)

HPLC (Methode 1A): Rₜ = 5.55 min; MS (ESIpos): m/z = 481 [M+H]⁺;

¹H-NMR (400 MHz, DMSO-d₆): δ = 8.03-7.96 (m, 2H), 7.72-7.67 (m, 1H), 7.62 (t, 1H), 7.25 (d, 2H), 7.18 (d, 2H), 4.09 (br d, 1H), 3.63 (br d, 1H), 3.61-3.54 (m, 4H), 3.55-3.45 (m, 1H), 3.26-3.17 (m, 4H), 3.11 (t, 1H), 2.97 (q, 1H), 2.95-2.85 (m, 1H), 2.58 (q, 2H), 2.38 (br d, 1H), 2.04 (q, 1H), 1.17 (t, 3H).

### Beispiel 30

### 4-{[3-(4-Ethylphenyl)-5-{3-[3-(trifluormethyl)phenyl]-1,2,4-oxadiazol-5-yl}piperidin-1-yl]carbonyl}morpholin [racemisches cis-Isomer]

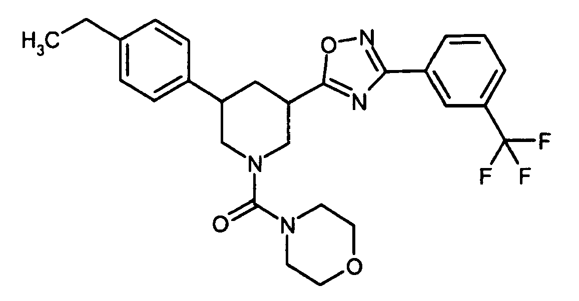

Nach der Allgemeinen Methode 1 wurden 69 mg (0.20 mmol) 5-(4-Ethylphenyl)-1-(morpholin-4-ylcarbonyl)piperidin-3-carbonsäure (Beispiel 38A) und 45 mg (0.22 mmol, 1.1 eq.) *N'*-Hydroxy-3-(trifluormethyl)benzencarboximidamid umgesetzt. Ausbeute: 60 mg (59% d. Th.)

HPLC (Methode 1A): Rₜ = 5.59 min; MS (ESIpos): m/z = 515 [M+H]⁺;

¹H-NMR (400 MHz, DMSO-d₆): δ = 8.33 (d, 1H), 8.25 (s, 1H), 8.00 (d, 1H), 7.85 (t, 1H), 7.25 (d, 2H), 7.18 (d, 2H), 4.09 (br d, 1H), 3.62 (br d, 1H), 3.61-3.55 (m, 4H), 3.58-3.47 (m, 1H), 3.26-3.18 (m, 4H), 3.13 (t, 1H), 2.98 (q, 1H), 2.96-2.86 (m, 1H), 2.58 (q, 2H), 2.38 (br d, 1H), 2.06 (q, 1H), 1.17 (t, 3H).

### Beispiel 31

### 4-{[3-(4-Ethylphenyl)-5-{3-[3-(trifluormethoxy)phenyl]-1,2,4-oxadiazol-5-yl}piperidin-1-yl]carbonyl}morpholin [racemisches cis-Isomer]

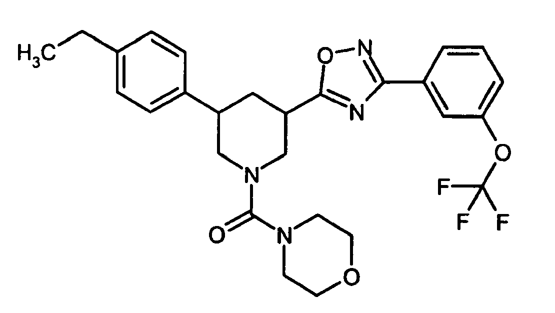

Nach der Allgemeinen Methode 1 wurden 69 mg (0.20 mmol) 5-(4-Ethylphenyl)-1-(morpholin-4-ylcarbonyl)piperidin-3-carbonsäure (Beispiel 38A) und 48 mg (0.22 mmol, 1.1 eq.) *N'-*Hydroxy-3-(trifluormethoxy)benzencarboximidamid umgesetzt. Ausbeute: 68 mg (64% d. Th.)

HPLC (Methode 1A): Rₜ = 5.65 min; MS (ESIpos): m/z = 531 [M+H]⁺;

¹H-NMR (400 MHz, DMSO-d₆): δ = 8.06 (d, 1H), 7.90 (s, 1H), 7.74 (t, 1H), 7.64 (d, 1H), 7.25 (d, 2H), 7.18 (d, 2H), 4.09 (br d, 1H), 3.62 (br d, 1H), 3.60-3.53 (m, 4H), 3.57-3.46 (m, 1H), 3.25-3.18 (m, 4H), 3.12 (t, 1H), 2.97 (q, 1H), 2.95-2.84 (m, 1H), 2.58 (q, 2H), 2.37 (br d, 1H), 2.05 (q, 1H), 1.17 (t, 3H).

### Beispiel 32

### 1-(Cyclopentylcarbonyl)-3-(4-ethylphenyl)-5-[3-(4-fluorphenyl)-1,2,4-oxadiazol-5-yl]piperidin [racemisches cis-Isomer]

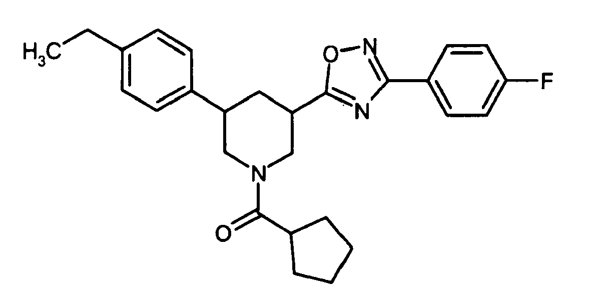

Nach der Allgemeinen Methode 1 wurden 66 mg (0.20 mmol) 1-(Cyclopentylcarbonyl)-5-(4-ethylphenyl)piperidin-3-carbonsäure (Beispiel 7A) und 34 mg (0.22 mmol, 1.1 eq.) 4-Fluor-*N'-*hydroxybenzencarboximidamid umgesetzt. Ausbeute: 45 mg (50% d. Th.)

HPLC (Methode 2A): Rₜ = 5.71 min; MS (ESIpos): m/z = 448 [M+H]⁺;

¹H-NMR (500 MHz, DMSO-d₆): δ = 8.07 (dd, 2H), 7.41 (t, 2H), 7.28 (d, 1H), 7.23 (d, 1H), 7.19 (t, 2H), 4.95 (br d, 0.5H), 4.53 (br d, 0.5H), 4.48 (br d, 0.5H), 4.03 (br d, 0.5H), 3.49-3.40 (m, 1H), 3.37-3.29 (m, 0.5H), 3.26-3.18 (m, 0.5H), 3.17-3.03 (m, 1H), 2.93-2.83 (m, 1H), 2.80-2.68 (m, 1H), 2.58 (q, 2H), 2.43-2.33 (m, 1H), 2.18-2.03 (m, 1H), 1.88-1.47 (m, 8H), 1.17 (t, 3H).

### Beispiel 33

### 4-({3-(4-Ethylphenyl)-5-[3-(4-fluorphenyl)-1,2,4-oxadiazol-5-yl]piperidin-1-yl}carbonyl)-morpholin [racemisches cis-Isomer]

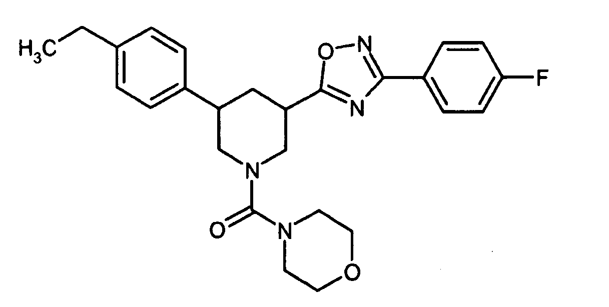

Nach der Allgemeinen Methode 1 wurden 69 mg (0.20 mmol) 5-(4-Ethylphenyl)-1-(morpholin-4-ylcarbonyl)piperidin-3-carbonsäure (Beispiel 38A) und 34 mg (0.22 mmol, 1.1 eq.) 4-Fluor-*N'-*hydroxybenzencarboximidamid umgesetzt. Ausbeute: 56 mg (60% d. Th.)

HPLC (Methode 1A): Rₜ = 5.31 min; MS (ESIpos): m/z = 465 [M+H]⁺;

¹H-NMR (400 MHz, DMSO-d₆): δ = 8.08 (dd, 2H), 7.41 (dd, 2H), 7.24 (d, 2H), 7.18 (d, 2H), 4.09 (br d, 1H), 3.62 (br d, 1H), 3.61-3.54 (m, 4H), 3.54-3.44 (m, 1H), 3.25-3.18 (m, 4H), 3.11 (t, 1H), 2.97 (q, 1H), 2.94-2.85 (m, 1H), 2.58 (q, 2H), 2.37 (br d, 1H), 2.03 (q, 1H), 1.17 (t, 3H).

### Beispiel 34

### 4-({3-[3-(4-Fluorphenyl)-1,2,4-oxadiazol-5-yl]-5-[4-(trifluormethoxy)phenyl]piperidin-1-yl}carbonyl)morpholin [racemisches cis-Isomer]

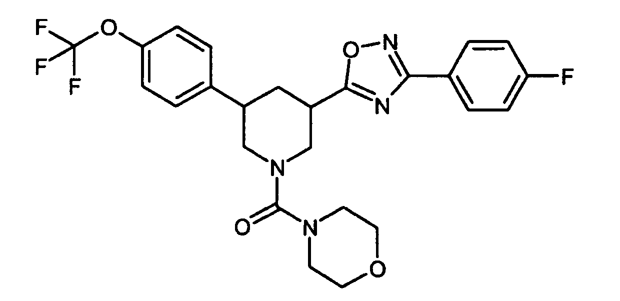

Nach der Allgemeinen Methode 1 wurden 80 mg (0.20 mmol) 1-(Morpholin-4-ylcarbonyl)-5-[4-(trifluormethoxy)phenyl]piperidin-3-carbonsäure (Beispiel 44A) und 34 mg (0.22 mmol, 1.1 eq.) 4-Fluor-*N'*-hydroxybenzencarboximidamid umgesetzt. Ausbeute: 55 mg (53% d. Th.)

HPLC (Methode 2A): Rₜ = 5.18 min; MS (ESIpos): m/z = 521 [M+H]⁺;

¹H-NMR (400 MHz, DMSO-d₆): δ = 8.07 (d, 2H), 7.49 (d, 2H), 7.42 (t, 2H), 7.34 (d, 2H), 4.09 (br d, 1H), 3.64 (br d, 1H), 3.63-3.54 (m, 4H), 3.54-3.45 (m, 1H), 3.26-3.18 (m, 4H), 3.13 (t, 1H), 3.07-2.97 (m, 2H), 2.40 (br d, 1H), 2.06 (q, 1H).

### Beispiel 35

### 4-({3-(4-Ethylphenyl)-5-[3-(4-chlorphenyl)-1,2,4-oxadiazol-5-yl]piperidin-1-yl} carbonyl)-morpholin [racemisches cis-Isomer]

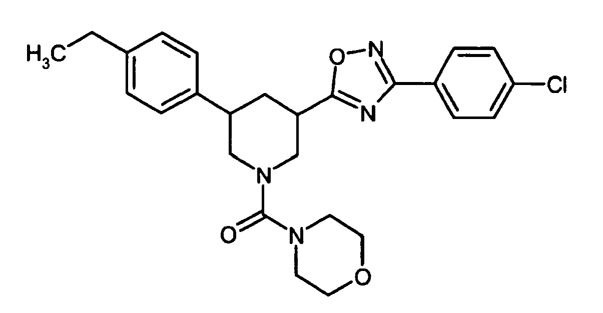

Nach der Allgemeinen Methode 1 wurden 69 mg (0.20 mmol) 5-(4-Ethylphenyl)-1-(morpholin-4-ylcarbonyl)piperidin-3-carbonsäure (Beispiel 38A) und 38 mg (0.22 mmol, 1.1 eq.) 4-Chlor-*N'-*hydroxybenzencarboximidamid umgesetzt. Ausbeute: 61 mg (64% d. Th.)

HPLC (Methode 2A): Rₜ = 5.49 min; MS (ESIpos): m/z = 481 [M+H]⁺;

¹H-NMR (400 MHz, DMSO-d₆): δ = 8.03 (d, 2H), 7.65 (d, 2H), 7.25 (d, 2H), 7.18 (d, 2H), 4.09 (br d, 1H), 3.62 (br d, 1H), 3.60-3.54 (m, 4H), 3.53-3.45 (m, 1H), 3.25-3.17 (m, 4H), 3.11 (t, 1H), 2.98 (q, 1H), 2.95-2.84 (m, 1H), 2.58 (q, 2H), 2.42 (br d, 1H), 2.04 (q, 1H), 1.17 (t, 3H).

### Beispiel 36

### 4-({3-[3-(1,3-Benzodioxol-5-yl)-1,2,4-oxadiazol-5-yl]-5-(4-ethylphenyl)piperidin-1-yl}carbonyl)-morpholin [racemisches cis-Isomer]

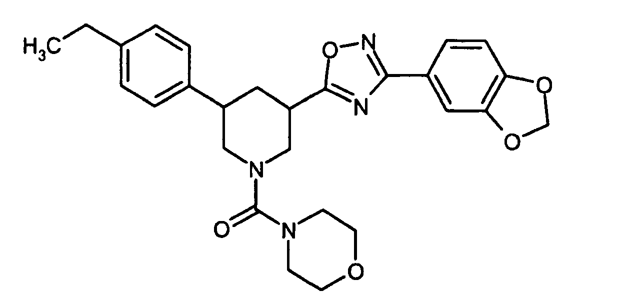

Nach der Allgemeinen Methode 1 wurden 69 mg (0.20 mmol) 5-(4-Ethylphenyl)-1-(morpholin-4-ylcarbonyl)piperidin-3-carbonsäure (Beispiel 38A) und 40 mg (0.22 mmol, 1.1 eq.) *N'-*Hydroxy-1,3-benzodioxol-5-carboximidamid umgesetzt. Ausbeute: 55 mg (54% d. Th.)

HPLC (Methode 1A): Rₜ = 4.63 min; MS (ESIpos): m/z = 491 [M+H]⁺;

¹H-NMR (400 MHz, DMSO-d₆): δ = 7.58 (dd, 2H), 7.45 (d, 2H), 7.24 (d, 2H), 7.17 (d, 2H), 7.09 (d, 1H), 6.14 (s, 2H), 4.08 (br d, 1H), 3.62 (br d, 1H), 3.60-3.53 (m, 4H), 3.51-3.42 (m, 1H), 3.25-3.17 (m, 4H), 3.09 (t, 1H), 2.95 (q, 1H), 2.93-2.84 (m, 1H), 2.58 (q, 2H), 2.36 (br d, 1H), 2.02 (q, 1H), 1.17 (t, 3H).

### Beispiel 37

### 4-({3-[3-(2,4-Difluorphenyl)-1,2,4-oxadiazol-5-yl]-5-(4-ethylphenyl)piperidin-1-yl}carbonyl)-morpholin [racemisches cis-Isomer]

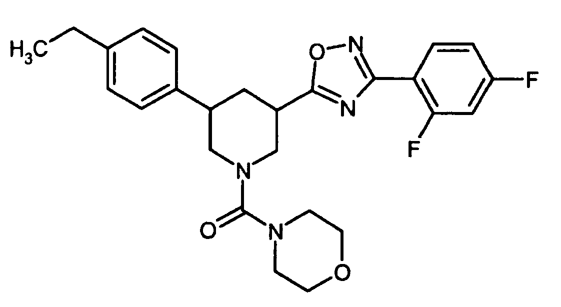

Nach der Allgemeinen Methode 1 wurden 69 mg (0.20 mmol) 5-(4-Ethylphenyl)-1-(morpholin-4-ylcarbonyl)piperidin-3-carbonsäure (Beispiel 38A) und 38 mg (0.22 mmol, 1.1 eq.) 2,4-Difluor-*N'-*hydroxybenzencarboximidamid umgesetzt. Ausbeute: 47 mg (47% d. Th.)

HPLC (Methode 1A): Rₜ = 5.27 min; MS (ESIpos): m/z = 483 [M+H]⁺;

¹H-NMR (400 MHz, DMSO-d₆): δ = 8.10 (dd, 1H), 7.54 (dt, 1H), 7.32 (dt, 1H), 7.24 (d, 2H), 7.18 (d, 2H), 4.09 (br d, 1H), 3.62 (br d, 1H), 3.61-3.54 (m, 4H), 3.56-3.46 (m, 1H), 3.25-3.17 (m, 4H), 3.11 (t, 1H), 2.97 (q, 1H), 2.95-2.85 (m, 1H), 2.58 (q, 2H), 2.38 (br d, 1H), 2.03 (q, 1H), 1.17 (t, 3H).

### Beispiel 38

### 4-({3-[3-(2,4-Difluorphenyl)-1,2,4-oxadiazol-5-yl]-5-[4-(trifluormethoxy)phenyl]piperidin-1-yl}carbonyl)morpholin [racemisches cis-Isomer]

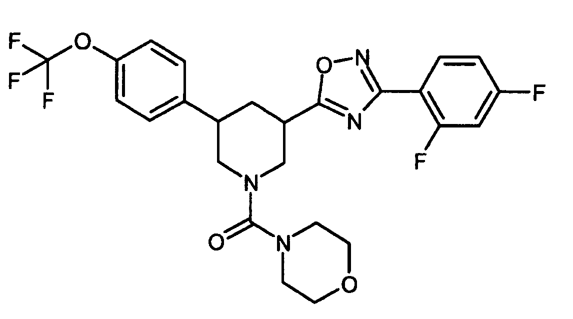

Nach der Allgemeinen Methode 1 wurden 80 mg (0.20 mmol) 1-(Morpholin-4-ylcarbonyl)-5-[4-(trifluormethoxy)phenyl]piperidin-3-carbonsäure (Beispiel 44A) und 38 mg (0.22 mmol, 1.1 eq.) 2,4-Difluor-*N'*-hydroxybenzencarboximidamid umgesetzt. Ausbeute: 52 mg (48% d. Th.)

HPLC (Methode 2A): Rₜ = 5.12 min; MS (ESIpos): m/z = 539 [M+H]⁺;

¹H-NMR (400 MHz, DMSO-d₆): δ = 8.14-8.05 (m, 1H), 7.59-7.46 (m, 3H), 7.38-7.28 (m, 3H), 4.09 (br d, 1H), 3.64 (br d, 1H), 3.62-3.47 (m, 5H), 3.27-3.17 (m, 4H), 3.13 (t, 1H), 3.08-2.97 (m, 2H), 2.41 (br d, 1H), 2.06 (q, 1H).

### Beispiel 39

### 4-({3-[3-(2,6-Difluorphenyl)-1,2,4-oxadiazol-5-yl]-5-(4-ethylphenyl)piperidin-1-yl}carbonyl)-morpholin [racemisches cis-Isomer]

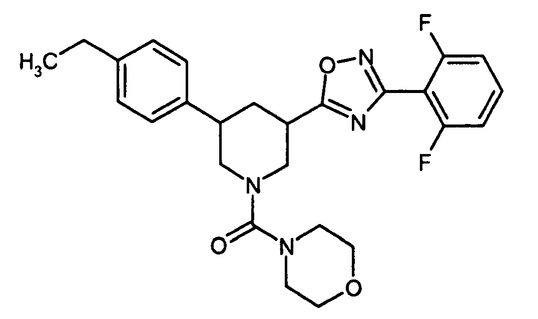

Nach der Allgemeinen Methode 1 wurden 69 mg (0.20 mmol) 5-(4-Ethylphenyl)-1-(morpholin-4-ylcarbonyl)piperidin-3-carbonsäure (Beispiel 38A) und 38 mg (0.22 mmol, 1.1 eq.) 2,6-Difluor-*N'-*hydroxybenzencarboximidamid umgesetzt. Ausbeute: 17 mg (18% d. Th.)

HPLC (Methode 2A): Rₜ = 5.03 min; MS (ESIpos): m/z = 483 [M+H]⁺;

¹H-NMR (400 MHz, DMSO-d₆): δ = 7.80-7.72 (m, 1H), 7.39 (t, 2H), 7.27 (d, 2H), 7.20 (d, 2H), 4.11 (br d, 1H), 3.64 (br d, 1H), 3.63-3.53 (m, 5H), 3.29-3.19 (m, 4H), 3.14 (t, 1H), 3.00 (t, 1H), 2.97-2.88 (m, 1H), 2.59 (q, 2H), 2.40 (br d, 1H), 2.07 (q, 1H), 1.19 (t, 3H).

### Beispiel 40

### 4-({3-[3-(2,6-Difluorphenyl)-1,2,4-oxadiazol-5-yl]-5-[4-(trifluormethoxy)phenyl]piperidin-1-yl}carbonyl)morpholin [racemisches cis-Isomer]

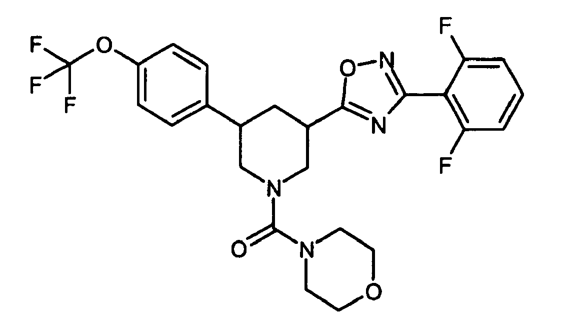

Nach der Allgemeinen Methode 1 wurden 80 mg (0.20 mmol) 1-(Morpholin-4-ylcarbonyl)-5-[4-(trifluormethoxy)phenyl]piperidin-3-carbonsäure (Beispiel 44A) und 38 mg (0.22 mmol, 1.1 eq.) 2,6-Difluor-*N*'-hydroxybenzencarboximidamid umgesetzt. Ausbeute: 42 mg (39% d. Th.)

HPLC (Methode 2A): Rₜ = 4.98 min; MS (ESIpos): m/z = 539 [M+H]⁺;

¹H-NMR (400 MHz, DMSO-d₆): δ = 7.78-7.69 (m, 1H), 7.49 (d, 2H), 7.40-7.32 (m, 4H), 4.09 (br d, 1H), 3.63 (br d, 1H), 3.61-3.52 (m, 5H), 3.27-3.18 (m, 4H), 3.13 (t, 1H), 3.07-2.97 (m, 2H), 2.41 (br d, 1H), 2.07 (q, 1H).

### Beispiel 41

### 4-({3-[3-(2,5-Dichlorphenyl)-1,2,4-oxadiazol-5-yl]-5-(4-ethylphenyl)piperidin-1-yl}carbonyl)-morpholin [racemisches cis-Isomer]

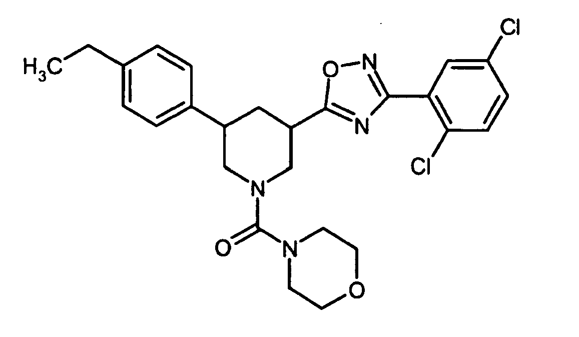

Nach der Allgemeinen Methode 1 wurden 69 mg (0.20 mmol) 5-(4-Ethylphenyl)-1-(morpholin-4-ylcarbonyl)piperidin-3-carbonsäure (Beispiel 38A) und 45 mg (0.22 mmol, 1.1 eq.) 2,5-Dichlor-*N'-*hydroxybenzencarboximidamid umgesetzt. Ausbeute: 28 mg (27% d. Th.)

HPLC (Methode 1A): Rₜ = 5.64 min; MS (ESIpos): m/z = 515 [M+H]⁺;

¹H-NMR (400 MHz, DMSO-d₆): δ = 7.97 (d, 1H), 7.77-7.68 (m, 2H), 7.24 (d, 2H), 7.18 (d, 2H), 4.08 (br d, 1H), 3.62 (br d, 1H), 3.61-3.54 (m, 4H), 3.58-3.48 (m, 1H), 3.25-3.17 (m, 4H), 3.11 (t, 1H), 2.97 (q, 1H), 2.95-2.85 (m, 1H), 2.57 (q, 2H), 2.38 (br d, 1H), 2.04 (q, 1H), 1.17 (t, 3H).

### Beispiel 42

### 4-({3-[3-(3-Chlor-4-fluorphenyl)-1,2,4-oxadiazol-5-yl]-5-(4-ethylphenyl)piperidin-1-yl}carbonyl)-morpholin [racemisches cis-Isomer]

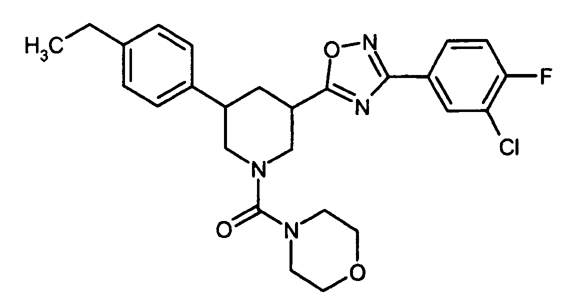

Nach der Allgemeinen Methode 1 wurden 69 mg (0.20 mmol) 5-(4-Ethylphenyl)-1-(morpholin-4-ylcarbonyl)piperidin-3-carbonsäure (Beispiel 38A) und 41 mg (0.22 mmol, 1.1 eq.) 3-Chlor-4-fluor-*N*'-hydroxybenzencarboximidamid umgesetzt. Ausbeute: 51 mg (51% d. Th.)

HPLC (Methode 1A): Rₜ = 5.53 min; MS (ESIpos): m/z = 499 [M+H]⁺;

¹H-NMR (400 MHz, DMSO-d₆): δ = 8.15 (dd, 1H), 8.07 (m, 1H), 7.64 (t, 1H), 7.24 (d, 2H), 7.18 (d, 2H), 4.09 (br d, 1H), 3.63 (br d, 1H), 3.61-3.55 (m, 4H), 3.54-3.45 (m, 1H), 3.25-3.17 (m, 4H), 3.11 (t, 1H), 2.97 (q, 1H), 2.94-2.85 (m, 1H), 2.58 (q, 2H), 2.41 (br d, 1H), 2.03 (q, 1H), 1.17 (t, 3H).

### Beispiel 43

### 4-({3-[3-(3-Chlor-4-fluorphenyl)-1,2,4-oxadiazol-5-yl]-5-[4-(trifluormethoxy)phenyl]piperidin-1-yl}carbonyl)morpholin [racemisches cis-Isomer]

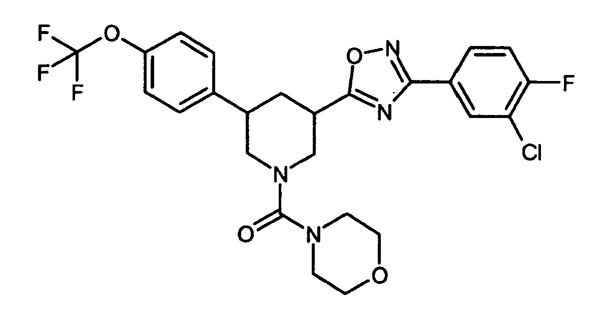

Nach der Allgemeinen Methode 1 wurden 80 mg (0.20 mmol) 1-(Morpholin-4-ylcarbonyl)-5-[4-(trifluormethoxy)phenyl]piperidin-3-carbonsäure (Beispiel 44A) und 41 mg (0.22 mmol, 1.1 eq.) 3-Chlor-4-fluor-*N*'-hydroxybenzencarboximidamid umgesetzt. Ausbeute: 51 mg (46% d. Th.)

HPLC (Methode 2A): Rₜ = 5.45 min; MS (ESIpos): m/z = 555 [M+H]⁺;

¹H-NMR (400 MHz, DMSO-d₆): δ = 8.18-8.12 (m, 1H), 8.07-8.00 (m, 1H), 7.64 (t, 1H), 7.49 (d, 2H), 7.34 (d, 2H), 4.09 (br d, 1H), 3.64 (br d, 1H), 3.61-3.47 (m, 5H), 3.26-3.18 (m, 4H), 3.13 (t, 1H), 3.08-2.97 (m, 2H), 2.40 (br d, 1H), 2.07 (q, 1H).

### Beispiel 44

### 2-{5-[1-(Cyclopentylcarbonyl)-5-(4-ethylphenyl)piperidin-3-yl]-1,2,4-oxadiazol-3-yl}pyridin [racemisches cis-Isomer]

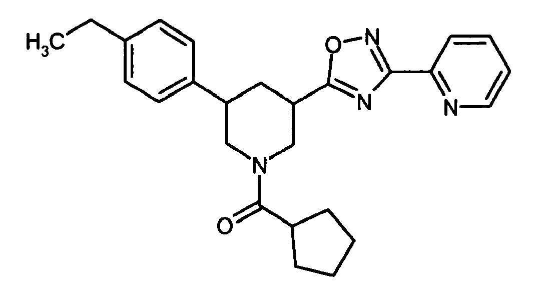

Nach der Allgemeinen Methode 1 wurden 66 mg (0.20 mmol) 1-(Cyclopentylcarbonyl)-5-(4-ethylphenyl)piperidin-3-carbonsäure (Beispiel 7A) und 30 mg (0.22 mmol, 1.1 eq.) *N'-*Hydroxypyridin-2-carboximidamid umgesetzt. Ausbeute: 27 mg (30% d. Th.)

HPLC (Methode 2A): Rₜ = 4.98 min; MS (ESIpos): m/z = 431 [M+H]⁺;

¹H-NMR (500 MHz, DMSO-d₆): δ = 8.76 (d, 1H), 8.08 (d, 1H), 8.02 (t, 1H), 7.61 (dd, 1H), 7.28 (d, 1H), 7.24 (d, 1H), 7.19 (t, 2H), 4.96 (br d, 0.5H), 4.54 (br d, 0.5H), 4.49 (br d, 0.5H), 4.03 (br d, 0.5H), 3.50-3.40 (m, 1H), 3.38-3.30 (m, 0.5H), 3.26-3.18 (m, 0.5H), 3.18-3.03 (m, 1H), 2.94-2.83 (m, 1H), 2.82-2.67 (m, 1H), 2.58 (q, 2H), 2.44-2.32 (m, 1H), 2.21-2.04 (m, 1H), 1.90-1.45 (m, 8H), 1.17 (t, 3H).

### Beispiel 45

### 3-{5-[1-(Cyclopentylcarbonyl)-5-(4-ethylphenyl)piperidin-3-yl]-1,2,4-oxadiazol-3-yl}pyridin [racemisches cis-Isomer]

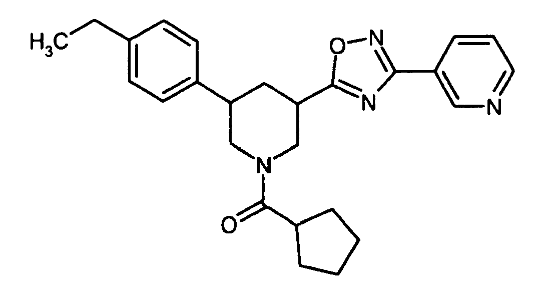

Nach der Allgemeinen Methode 1 wurden 66 mg (0.20 mmol) 1-(Cyclopentylcarbonyl)-5-(4-ethylphenyl)piperidin-3-carbonsäure (Beispiel 7A) und 30mg (0.22 mmol, 1.1 eq.) *N'-*Hydroxypyridin-3-carboximidamid umgesetzt. Ausbeute: 37 mg (43% d. Th.)

HPLC (Methode 2A): Rₜ = 4.66 min; MS (ESIpos): m/z = 431 [M+H]⁺;

¹H-NMR (500 MHz, DMSO-d₆): δ = 9.18 (s, 1H), 8.79 (d, 1H), 8.37 (d, 1H), 7.62 (dd, 1H), 7.28 (d, 1H), 7.24 (d, 1H), 7.19 (t, 2H), 4.96 (br d, 0.5H), 4.53 (br d, 0.5H), 4.49 (br d, 0.5H), 4.03 (br d, 0.5H), 3.51-3.41 (m, 1H), 3.39-3.32 (m, 0.5H), 3.27-3.19 (m, 0.5H), 3.18-3.02 (m, 1H), 2.97-2.83 (m, 1H), 2.82-2.68 (m, 1H), 2.58 (q, 2H), 2.43-2.34 (m, 1H), 2.21-2.03 (m, 1H), 1.89-1.47 (m, 8H), 1.17 (t, 3H).

### Beispiel 46

### 3-(5-{cis-(3,5)-1-{(Cyclopentylcarbonyl)-5-[4-(trifluormethyl)phenyl]piperidin-3-yl}-1,2,4-oxadiazol-3-yl)pyridin [racemisches cis-Isomer]

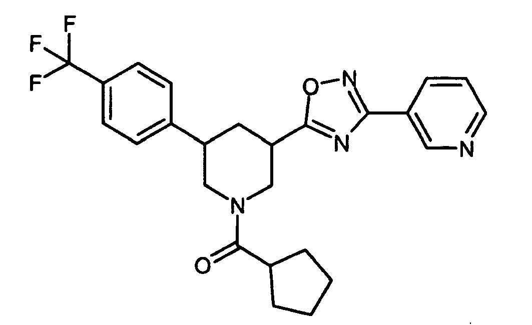

Nach der allgemeinen Methode 2 wurden 150 mg (0.41 mmol) cis-(3,5)-1-(Cyclopentylcarbonyl)-5-[4-(trifluormethyl)phenyl]piperidin-3-carbonsäure (Beispiel 17A) und 83 mg (0.61 mmol, 1.5 eq.) *N*'-Hydroxypyridin-3-carboximidamid umgesetzt. Ausbeute: 149 mg (78% d. Th.)

LC-MS (Methode 6B): Rₜ = 2.51 min; MS (ESIpos): m/z = 471 [M+H]⁺.

### Beispiel 47

### 3-(5-{cis-(3,5)-{1-(Cyclopentylcarbonyl)-5-[4-(trifluormethoxy)phenyl}piperidin-3-yl}-1,2,4-oxadiazol-3-yl)pyridin [racemisches cis-Isomer]

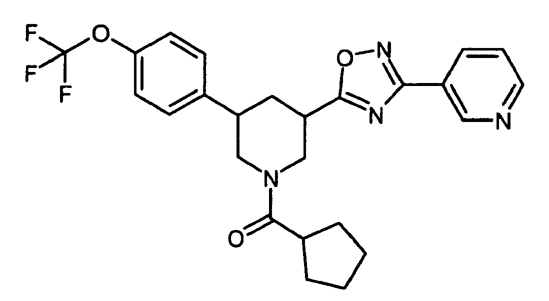

Nach der allgemeinen Methode 2 wurden 70 mg (0.18 mmol) cis-(3,5)-1-(Cyclopentylcarbonyl)-5-[4-(trifluormethoxy)phenyl]piperidin-3-carbonsäure (Beispiel 24A) und 41 mg (0.27 mmol, 1.5 eq.) *N*'-Hydroxypyridin-3-carboximidamid umgesetzt. Ausbeute: 56 mg (63% d. Th.)

LC-MS (Methode 1B): Rₜ = 2.98 min; MS (ESIpos): m/z = 487 [M+H]⁺.

### Beispiel 48

### 4-{5-[1-(Cyclopentylcarbonyl)-5-(4-ethylphenyl)piperidin-3-yl]-1,2,4-oxadiazol-3-yl} pyridin [racemisches cis-Isomer]

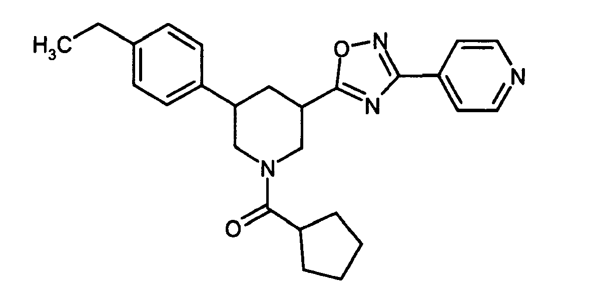

Nach der Allgemeinen Methode 1 wurden 66 mg (0.20 mmol) 1-(Cyclopentylcarbonyl)-5-(4-ethylphenyl)piperidin-3-carbonsäure (Beispiel 7A) und 30mg (0.22 mmol, 1.1 eq.) *N*'-Hydroxypyridin-4-carboximidamid umgesetzt. Ausbeute: 39 mg (45% d. Th.)

HPLC (Methode 2A): Rₜ = 4.59 min; MS (ESIpos): m/z = 431 [M+H]⁺;

¹H-NMR (500 MHz, DMSO-d₆): δ = 8.80 (d, 2H), 7.94 (d, 2H), 7.28 (d, 1H), 7.23 (d, 1H), 7.19 (t, 2H), 4.96 (br d, 0.5H), 4.53 (br d, 0.5H), 4.49 (br d, 0.5H), 4.03 (br d, 0.5H), 3.54-3.40 (m, 1H), 3.40-3.32 (m, 0.5H), 3.26-3.18 (m, 0.5H), 3.17-3.03 (m, 1H), 2.96-2.82 (m, 1H), 2.81-2.67 (m, 1H), 2.58 (q, 2H), 2.44-2.34 (m, 1H), 2.20-2.03 (m, 1H), 1.89-1.47 (m, 8H), 1.17 (t, 3H).

### Beispiel 49

### 4-({3-(3-Pyridin-4-yl-1,2,4-oxadiazol-5-yl)-5-[4-(trifluormethoxy)phenyl]piperidin-1-yl}carbonyl)-morpholin [racemisches cis-Isomer]

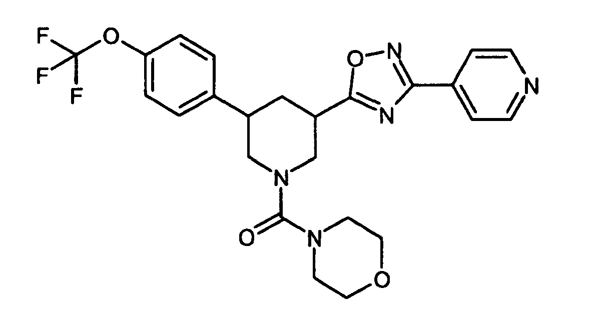

Nach der Allgemeinen Methode 1 wurden 80 mg (0.20 mmol) 1-(Morpholin-4-ylcarbonyl)-5-[4-(trifluormethoxy)phenyl]piperidin-3-carbonsäure (Beispiel 44A) und 30 mg (0.22 mmol, 1.1 eq.) *N*'-Hydroxypyridin-4-carboximidamid umgesetzt. Ausbeute: 52 mg (52% d. Th.)

HPLC (Methode 1A): Rₜ = 4.23 min; MS (ESIpos): m/z = 503 [M+H]⁺;

¹H-NMR (400 MHz, DMSO-d₆): δ = 8.81 (d, 2H), 7.95 (d, 2H), 7.49 (d, 2H), 7.34 (d, 2H), 4.10 (br d, 1H), 3.64 (br d, 1H), 3.61-3.50 (m, 5H), 3.27-3.19 (m, 4H), 3.14 (t, 1H), 3.09-2.98 (m, 2H), 2.41 (br d, 1H), 2.08 (q, 1H).

### Beispiel 50

### 4-{[3-(4-Ethylphenyl)-5-(3-pyrazin-2-yl-1,2,4-oxadiazol-5-yl)piperidin-1-yl]carbonyl morpholin [racemisches cis-Isomer]

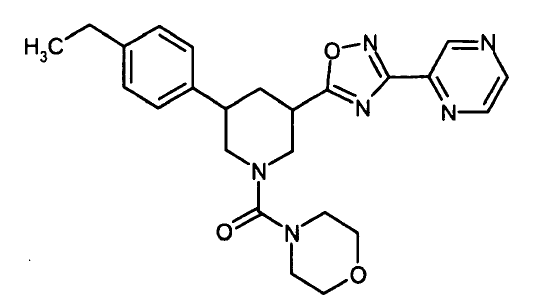

Nach der Allgemeinen Methode 1 wurden 69 mg (0.20 mmol) 5-(4-Ethylphenyl)-1-(morpholin-4-ylcarbonyl)piperidin-3-carbonsäure (Beispiel 38A) und 30 mg (0.22 mmol, 1.1 eq.) *N'-*Hydroxypyrazin-2-carboximidamid umgesetzt. Ausbeute: 45 mg (50% d. Th.)

HPLC (Methode 2A): Rₜ = 4.52 min; MS (ESIpos): m/z = 449 [M+H]⁺;

¹H-NMR (400 MHz, DMSO-d₆): δ = 9.27 (s, 1H), 8.90-8.85 (m, 2H), 7.25 (d, 2H), 7.18 (d, 2H), 4.12 (br d, 1H), 3.63 (br d, 1H), 3.61-3.50 (m, 5H), 3.26-3.18 (m, 4H), 3.14 (t, 1H), 2.97 (q, 1H), 2.95-2.86 (m, 1H), 2.58 (q, 2H), 2.39 (br d, 1H), 2.07 (q, 1H), 1.17 (t, 3H).

### Beispiel 51

### 4-({3-(4-Ethylphenyl)-5-[3-(4-methylpyridin-2-yl)-1,2,4-oxadiazol-5-yl]piperidin-1-yl}carbonyl)-morpholin [racemisches cis-Isomer]

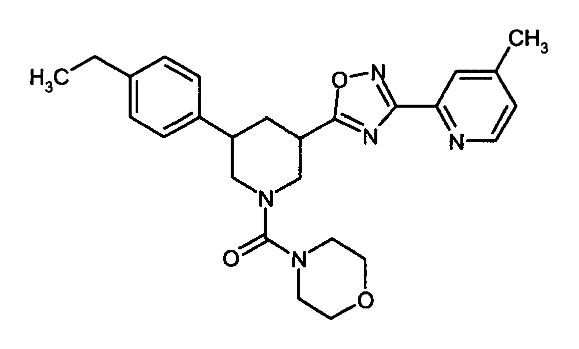

Nach der Allgemeinen Methode 1 wurden 69 mg (0.20 mmol) 5-(4-Ethylphenyl)-1-(morpholin-4-ylcarbonyl)piperidin-3-carbonsäure (Beispiel 38A) und 33 mg (0.22 mmol, 1.1 eq.) *N*'-Hydroxy-4-methylpyridin-2-carboximidamid umgesetzt. Ausbeute: 35 mg (38% d. Th.)

HPLC (Methode 1A): Rₜ = 4.49 min; MS (ESIpos): m/z = 462 [M+H]⁺;

¹H-NMR (400 MHz, DMSO-d₆): δ = 8.06 (d, 1H), 7.93 (s, 1H), 7.43 (d, 1H), 7.25 (d, 2H), 7.18 (d, 2H), 4.09 (br d, 1H), 3.62 (br d, 1H), 3.61-3.54 (m, 4H), 3.55-3.46 (m, 1H), 3.27-3.18 (m, 4H), 3.11 (t, 1H), 2.96 (q, 1H), 2.95-2.85 (m, 1H), 2.58 (q, 2H), 2.43 (s, 3H), 2.38 (br d, 1H), 2.04 (q, 1H), 1.17 (t, 3H).

### Beispiel 52

### 4-({3-(4-Ethylphenyl)-5-[3-(6-methylpyädin-2-yl)-1,2,4-oxadiazol-5-yl]piperidin-1-yl}carbonyl)-morpholin [racemisches cis-Isomer]

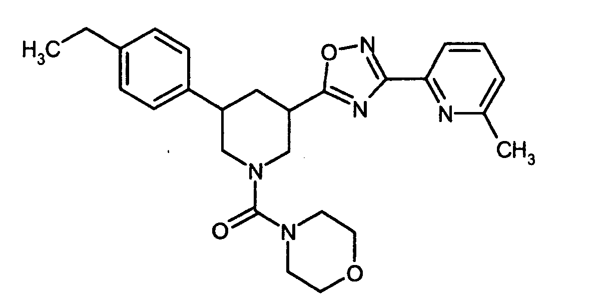

Nach der Allgemeinen Methode 1 wurden 69 mg (0.20 mmol) 5-(4-Ethylphenyl)-1-(morpholin-4-ylcarbonyl)piperidin-3-carbonsäure (Beispiel 38A) und 33 mg (0.22 mmol, 1.1 eq.) *N*'-Hydroxy-6-methylpyridin-2-carboximidamid umgesetzt. Ausbeute: 44 mg (47% d. Th.)

HPLC (Methode 1A): Rₜ = 4.54 min; MS (ESIpos): m/z = 462 [M+H]⁺;

¹H-NMR (400 MHz, DMSO-d₆): δ = 7.93-7.85 (m, 2H), 7.51-7.42 (m, 1H), 7.25 (d, 2H), 7.18 (d, 2H), 4.09 (br d, 1H), 3.63 (br d, 1H), 3.62-3.53 (m, 4H), 3.55-3.45 (m, 1H), 3.25-3.18 (m, 4H), 3.12 (t, 1H), 2.95 (q, 1H), 2.94-2.83 (m, 1H), 2.57 (q, 2H), 2.37 (br d, 1H), 2.05 (q, 1H), 1.17 (t, 3H).

### Beispiel 53

### 1-(Cyclopentylcarbonyl)-3-(4-ethylphenyl)-5-[3-(2-methyl-1,3-thiazol-4-yl)-1,2,4-oxadiazol-5-yl]piperidin [racemisches cis-Isomer]

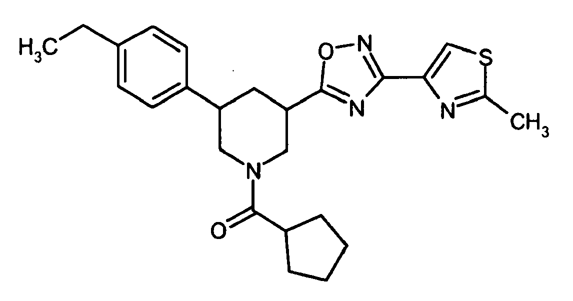

Nach der Allgemeinen Methode 1 wurden 66 mg (0.20 mmol) 1-(Cyclopentylcarbonyl)-5-(4-ethylphenyl)piperidin-3-carbonsäure (Beispiel 7A) und 35 mg (0.22 mmol, 1.1 eq.) *N*'-Hydroxy-2-methyl-1,3-thiazol-4-carboximidamid umgesetzt. Ausbeute: 36 mg (40% d. Th.)

HPLC (Methode 2A): Rₜ = 5.17 min; MS (ESIpos): m/z = 451 [M+H]⁺;

¹H-NMR (500 MHz, DMSO-d₆): δ = 8.28 (s, 1H), 7.28 (d, 1H), 7.23 (d, 1H), 7.19 (t, 2H), 4.94 (br d, 0.5H), 4.53 (br d, 0.5H), 4.47 (br d, 0.5H), 4.02 (br d, 0.5H), 3.48-3.37 (m, 1H), 3.35-3.27 (m, 0.5H), 3.25-3.18 (m, 0.5H), 3.16-3.03 (m, 1H), 2.92-2.82 (m, 1H), 2.79-2.67 (m, 1H), 2.74 (s, 3H), 2.58 (q, 2H), 2.43-2.34 (m, 1H), 2.18-2.02 (m, 1H), 1.88-1.46 (m, 8H), 1.17 (t, 3H).

### Beispiel 54

### 1-(Cyclopentylcarbonyl)-3-(4-ethylphenyl)-5-[3-(5-methylisoxazol-3-yl)-1,2,4-oxadiazol-5-yl]piperidin [racemisches cis-Isomer]

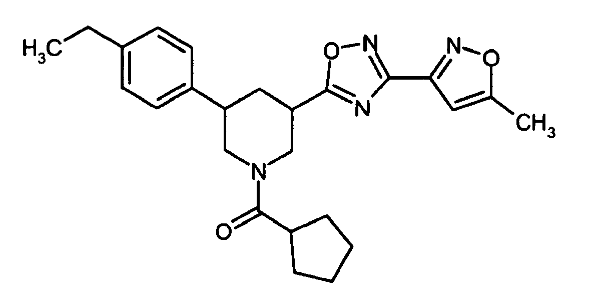

Nach der Allgemeinen Methode 1 wurden 66 mg (0.20 mmol) 1-(Cyclopentylcarbonyl)-5-(4-ethylphenyl)piperidin-3-carbonsäure (Beispiel 7A) und 31 mg (0.22 mmol, 1.1 eq.) *N'-*Hydroxy-5-methylisoxazol-3-carboximidamid umgesetzt. Ausbeute: 50 mg (58% d. Th.)

HPLC (Methode 1A): Rₜ = 5.23 min; MS (ESIpos): m/z = 435 [M+H]⁺;

¹H-NMR (500 MHz, DMSO-d₆): δ = 7.27 (d, 1H), 7.23 (d, 1H), 7.19 (t, 2H), 6.81 (s, 1H), 4.94 (br d, 0.5H), 4.52 (br d, 0.5H), 4.48 (br d, 0.5H), 4.02 (br d, 0.5H), 3.50-3.40 (m, 1H), 3.40-3.32 (m, 0.5H), 3.25-3.18 (m, 0.5H), 3.17-3.02 (m, 1H), 2.94-2.81 (m, 1H), 2.80-2.65 (m, 1H), 2.58 (q, 2H), 2.42-2.32 (m, 1H), 2.19-2.02 (m, 1H), 1.88-1.47 (m, 8H), 1.17 (t, 3H).

### Beispiel 55

### 3-(3-Benzyl-1,2,4-oxadiazol-5-yl)-1-(cyclopentylcarbonyl)-5-(4-ethylphenyl)piperidin [racemisches cis-Isomer]

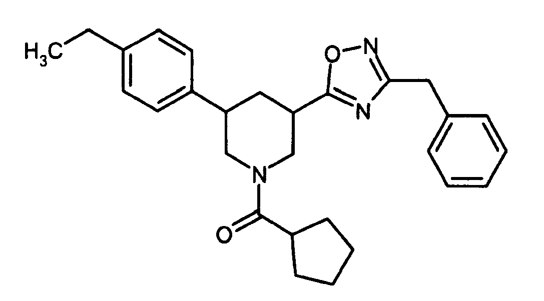

Nach der Allgemeinen Methode 1 wurden 66 mg (0.20 mmol) 1-(Cyclopentylcarbonyl)-5-(4-ethylphenyl)piperidin-3-carbonsäure (Beispiel 7A) und 33 mg (0.22 mmol, 1.1 eq.) *N*'-Hydroxy-2-phenylethanimidamid umgesetzt. Ausbeute: 50 mg (56% d. Th.)

HPLC (Methode 1A): Rₜ = 5.48 min; MS (ESIpos): m/z = 444 [M+H]⁺;

¹H-NMR (500 MHz, DMSO-d₆): δ = 7.35-7.28 (m, 4H), 7.28-7.22 (m, 2H), 7.21-7.14 (m, 3H), 4.84 (br d, 0.5H), 4.48 (br d, 0.5H), 4.36 (br d, 0.5H), 4.09 (s, 2H), 3.98 (br d, 0.5H), 3.38-3.29 (m, 1H), 3.22-3.13 (m, 1H), 3.11-3.00 (m, 1H), 2.85-2.73 (m, 1H), 2.72-2.61 (m, 1H), 2.57 (q, 2H), 2.32-2.22 (m, 1H), 2.08-1.92 (m, 1H), 1.84-1.45 (m, 8H), 1.16 (t, 3H).

### Beispiel 56

### 3-(3-Benzyl-1,2,4-oxadiazol-5-yl)-1-(cyclopentylcarbonyl)-5-[4-(trifluormethoxy)phenyl]piperidin [racemisches cis-Isomer]

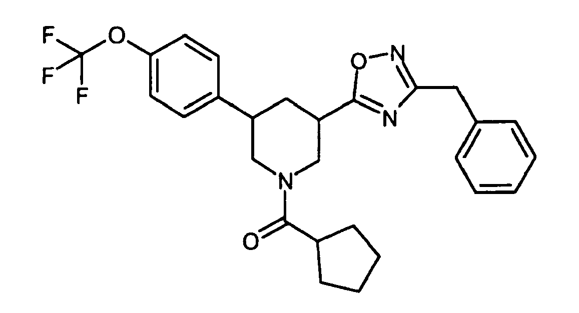

Nach der Allgemeinen Methode 1 wurden 70 mg (0.18 mmol) cis-(3,5)-1-(Cyclopentylcarbonyl)-5-[4-(trifluormethoxy)phenyl]piperidin-3-carbonsäure (Beispiel 24A) und 41 mg (0.27 mmol, 1.5 eq.) *N*'-Hydroxy-2-phenylethanimidamid umgesetzt. Ausbeute: 55 mg (61 % d. Th.)

LC-MS (Methode 1B): Rₜ = 3.25 min; MS (ESIpos): m/z = 500 [M+H]⁺;

### Beispiel 57

### 4-{[3-(3-Benzyl-1,2,4-oxadiazol-5-yl)-5-(4-ethylphenyl)piperidin-1-yl]carbonyl}morpholin [racemisches cis-Isomer]

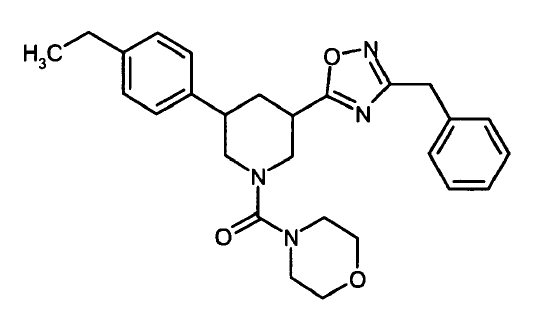

Nach der Allgemeinen Methode 1 wurden 69 mg (0.20 mmol) 5-(4-Ethylphenyl)-1-(morpholin-4-ylcarbonyl)piperidin-3-carbonsäure (Beispiel 38A) und 33 mg (0.22 mmol, 1.1 eq.) *N*'-Hydroxy-2-phenylethanimidamid umgesetzt. Ausbeute: 43 mg (47% d. Th.)

HPLC (Methode 1A): Rₜ = 4.85 min; MS (ESIpos): m/z = 461 [M+H]⁺;

¹H-NMR (400 MHz, DMSO-d₆): δ = 7.38-7.23 (m, 5H), 7.20 (d, 2H), 7.15 (d, 2H), 4.08 (s, 2H), 3.98 (br d, 1H), 3.58 (br d, 1H), 3.58-3.50 (m, 4H), 3.43-3.32 (m, 1H), 3.22-3.13 (m, 4H), 3.00 (t, 1H), 2.91 (q, 1H), 2.89-2.79 (m, 1H), 2.56 (q, 2H), 2.27 (br d, 1H), 1.93 (q, 1H), 1.16 (t, 3H).

### Beispiel 58

### 4-({3-(3-Benzyl-1,2,4-oxadiazol-5 -yl)-5-[4-(trifluormethoxy)phenyl]piperidin-1-yl}carbonyl)-morpholin [racemisches cis-Isomer]

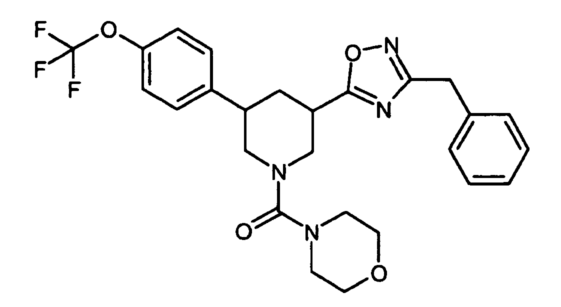

Nach der Allgemeinen Methode 1 wurden 80 mg (0.20 mmol) 1-(Morpholin-4-ylcarbonyl)-5-[4-(trifluormethoxy)phenyl]piperidin-3-carbonsäure (Beispiel 44A) und 33 mg (0.22 mmol, 1.1 eq.) *N*'-Hydroxy-2-phenylethanimidamid umgesetzt. Ausbeute: 46 mg (44% d. Th.)

HPLC (Methode 1A): Rₜ = 5.01 min; MS (ESIpos): m/z = 517 [M+H]⁺;

¹H-NMR (400 MHz, DMSO-d₆): δ = 7.45 (d, 2H), 7.36-7.22 (m, 7H), 4.08 (s, 2H), 3.97 (br d, 1H), 3.59 (br d, 1H), 3.59-3.51 (m, 4H), 3.43-3.33 (m, 1H), 3.22-3.14 (m, 4H), 3.02 (t, 1H), 3.01-2.91 (m, 2H), 2.29 (br d, 1H), 1.96 (q, 1H).

### Beispiel 59

### 3-[3-(2-Chlorbenzyl)-1,2,4-oxadiazol-5-yl]-1-(cyclopentylcarbonyl)-5-(4-ethylphenyl)piperidin [racemisches cis-Isomer]

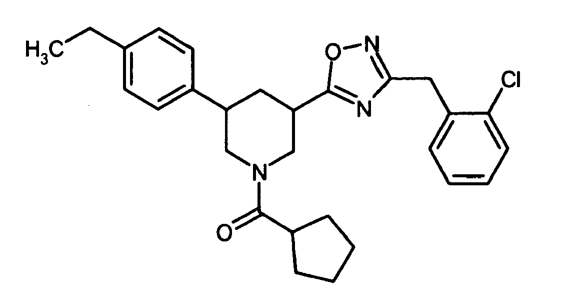

Nach der Allgemeinen Methode 1 wurden 66 mg (0.20 mmol) 1-(Cyclopentylcarbonyl)-5-(4-ethylphenyl)piperidin-3-carbonsäure (Beispiel 7A) und 41 mg (0.22 mmol, 1.1 eq.) 2-(2-Chlorphenyl)-N'-hydroxyethanimidamid umgesetzt. Ausbeute: 21 mg (22% d. Th.)

HPLC (Methode 2A): Rₜ = 5.64 min; MS (ESIpos): m/z = 478 [M+H]⁺;

¹H-NMR (500 MHz, DMSO-d₆): δ = 7.47 (dd, 1H), 7.42 (dd, 1H), 7.33 (dd, 2H), 7.24 (d, 1H), 7.22-7.13 (m, 3H), 4.83 (br d, 0.5H), 4.48 (br d, 0.5H), 4.37 (br d, 0.5H), 4.21 (s, 2H), 3.98 (br d, 0.5H), 3.38-3.30 (m, 1H), 3.25-3.14 (m, 1H), 3.13-2.99 (m, 1H), 2.86-2.74 (m, 1H), 2.73-2.62 (m, 1H), 2.57 (q, 2H), 2.32-2.22 (m, 1H), 2.08-1.92 (m, 1H), 1.84-1.45 (m, 8H), 1.16 (t, 3H).

### Beispiel 60

### 4-({3-[3-(2-Chlorbenzyl)-1,2,4-oxadiazol-5-yl]-5-(4-ethylphenyl)piperidin-1-yl}carbonyl)-morpholin [racemisches cis-Isomer]

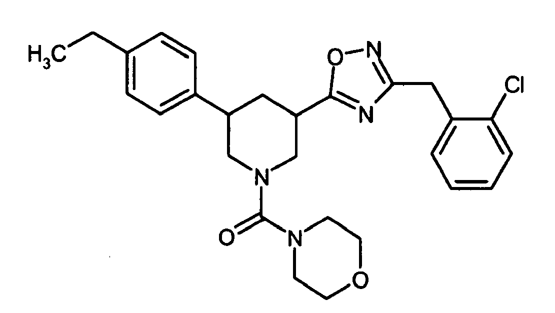

Nach der Allgemeinen Methode 1 wurden 69 mg (0.20 mmol) 5-(4-Ethylphenyl)-1-(morpholin-4-ylcarbonyl)piperidin-3-carbonsäure (Beispiel 38A) und 41 mg (0.22 mmol, 1.1 eq.) 2-(2-Chlorphenyl)-N'-hydroxyethanimidamid umgesetzt. Ausbeute: 10 mg (10% d. Th.)

LC-MS (Methode 2B): Rₜ = 1.49 min; MS (ESIpos): m/z = 495 [M+H]⁺;

¹H-NMR (400 MHz, DMSO-d₆): δ = 7.50-7.44 (m, 1H), 7.45-7.40 (m, 1H), 7.36-7.30 (m, 2H), 7.21 (d, 2H), 7.16 (d, 2H), 4.21 (s, 2H), 3.98 (br d, 1H), 3.58 (br d, 1H), 3.57-3.50 (m, 4H), 3.43-3.33 (m, 1H), 3.22-3.12 (m, 4H), 3.00 (t, 1H), 2.92 (q, 1H), 2.89-2.79 (m, 1H), 2.56 (q, 2H), 2.27 (br d, 1H), 1.93 (q, 1H), 1.16 (t, 3H).

### Beispiel 61

### 4-({3-(4-Ethylphenyl)-5-[3-(3-methylbenzyl)-1,2,4-oxadiazol-5-yl]piperidin-1-yl}carbonyl)-morpholin [racemisches cis-Isomer]

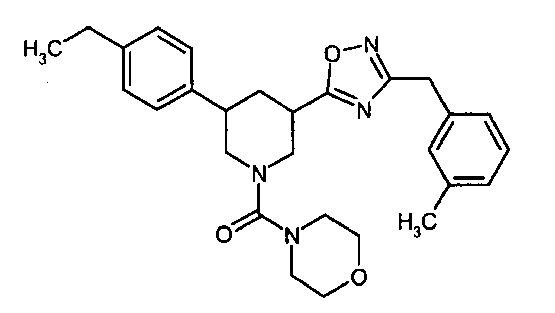

Nach der Allgemeinen Methode 1 wurden 69 mg (0.20 mmol) 5-(4-Ethylphenyl)-1-(morpholin-4-ylcarbonyl)piperidin-3-carbonsäure (Beispiel 38A) und 36 mg (0.22 mmol, 1.1 eq.) *N*'-Hydroxy-2-(3-methylphenyl)ethanimidamid umgesetzt. Ausbeute: 57 mg (60% d. Th.)

HPLC (Methode 1A): Rₜ = 5.26 min; MS (ESIpos): m/z = 475 [M+H]⁺;

¹H-NMR (400 MHz, DMSO-d₆): δ = 7.25-7.13 (m, 5H), 7.13-7.04 (m, 3H), 4.03 (s, 2H), 3.98 (br d, 1H), 3.57 (br d, 1H), 3.57-3.51 (m, 4H), 3.42-3.33 (m, 1H), 3.21-3.14 (m, 4H), 3.00 (t, 1H), 2.91 (q, 1H), 2.88-2.79 (m, 1H), 2.57 (q, 2H), 2.28 (s, 3H), 2.25 (br d, 1H), 1.93 (q, 1H), 1.16 (t, 3H).

### Beispiel 62

### 1-(Cyclopentylcarbonyl)-3-(4-ethylphenyl)-5-[3-(4-fluorbenzyl)-1,2,4-oxadiazol-5-yl]piperidin [racemisches cis-Isomer]

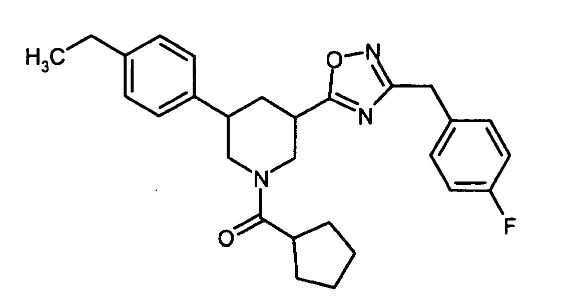

Nach der Allgemeinen Methode 1 wurden 66 mg (0.20 mmol) 1-(Cyclopentylcarbonyl)-5-(4-ethylphenyl)piperidin-3-carbonsäure (Beispiel 7A) und 37mg (0.22 mmol, 1.1 eq.) 2-(4-Fluorphenyl)-*N*'-hydroxyethanimidamid umgesetzt. Ausbeute: 22 mg (24% d. Th.)

HPLC (Methode 2A): Rₜ = 5.51 min; MS (ESIpos): m/z = 462 [M+H]⁺;

¹H-NMR (500 MHz, DMSO-d₆): δ = 7.35 (dd, 2H), 7.24 (d, 1H), 7.22-7.13 (5H), 4.84 (br d, 0.5H), 4.49 (br d, 0.5H), 4.37 (br d, 0.5H), 4.09 (s, 2H), 3.98 (br d, 0.5H), 3.38-3.28 (m, 1H), 3.23-3.13 (m, 1H), 3.12-3.00 (m, 1H), 2.86-2.74 (m, 1H), 2.73-2.62 (m, 1H), 2.57 (q, 2H), 2.32-2.22 (m, 1H), 2.08-1.91 (m, 1H), 1.85-1.44 (m, 8H), 1.16 (t, 3H).

### Beispiel 63

### 4-({3-(4-Ethylphenyl)-5-[3-(4-fluorbenzyl)-1,2,4-oxadiazol-5-yl]piperidin-1-yl}carbonyl)-morpholin [racemisches cis-Isomer]

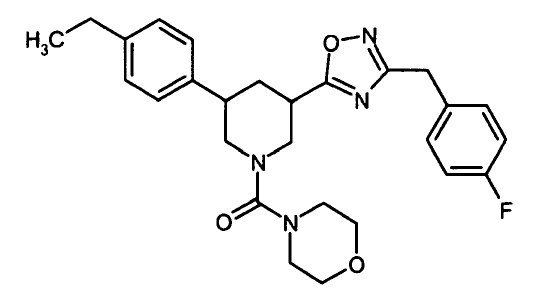

Nach der Allgemeinen Methode 1 wurden 69 mg (0.20 mmol) 5-(4-Ethylphenyl)-1-(morpholin-4-ylcarbonyl)piperidin-3-carbonsäure (Beispiel 38A) und 37mg (0.22 mmol, 1.1 eq.) 2-(4-Fluorphenyl)-N'-hydroxyethanimidamid umgesetzt. Ausbeute: 44 mg (45% d. Th.)

HPLC (Methode 1A): Rₜ = 5.14 min; MS (ESIpos): m/z = 479 [M+H]⁺;

¹H-NMR (400 MHz, DMSO-d₆): δ = 7.39-7.30 (m, 2H), 7.21 (d, 2H), 7.18-7.10 (m, 4H), 4.09 (s, 2H), 3.98 (br d, 1H), 3.57 (br d, 1H), 3.57-3.51 (m, 4H), 3.43-3.33 (m, 1H), 3.21-3.13 (m, 4H), 3.00 (t, 1H), 2.92 (q, 1H), 2.89-2.79 (m, 1H), 2.56 (q, 2H), 2.27 (br d, 1H), 1.92 (q, 1H), 1.16 (t, 3H).

### Beispiel 64

### 4-({3-[3-(4-Fluorbenzyl)-1,2,4-oxadiazol-5-yl]-5-[4-(trifluormethoxy)phenyl]piperidin-1-yl}carbonyl)morpholin [racemisches cis-Isomer]

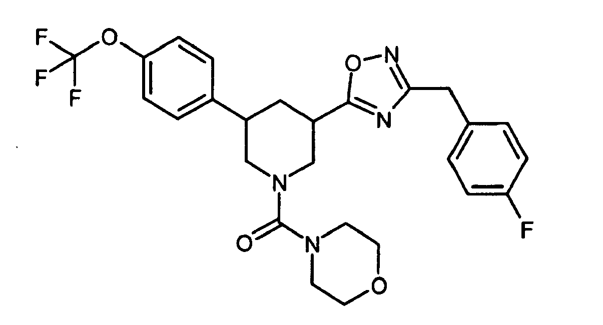

Nach der Allgemeinen Methode 1 wurden 80 mg (0.20 mmol) 1-(Morpholin-4-ylcarbonyl)-5-[4-(trifluormethoxy)phenyl]piperidin-3-carbonsäure (Beispiel 44A) und 37 mg (0.22 mmol, 1.1 eq.) 2-(4-Fluorphenyl)-N'-hydroxyethanimidamid umgesetzt. Ausbeute: 61 mg (57% d. Th.)

HPLC (Methode 2A): Rₜ = 5.02 min; MS (ESIpos): m/z = 535 [M+H]⁺;

¹H-NMR (400 MHz, DMSO-d₆): δ = 7.45 (d, 2H), 7.38-7.30 (m, 4H), 7.15 (t, 2H), 4.09 (s, 2H), 3.97 (br d, 1H), 3.59 (br d, 1H), 3.59-3.51 (m, 4H), 3.43-3.32 (m, 1H), 3.22-3.15 (m, 4H), 3.02 (t, 1H), 3.00-2.91 (m, 2H), 2.29 (br d, 1H), 1.96 (q, 1H).

### Beispiel 65

### 4-({3-[3-(4-Chlorbenzyl)-1,2,4-oxadiazol-5-yl]-5-(4-ethylphenyl)piperidin-1-yl}carbonyl)-morpholin [racemisches cis-Isomer]

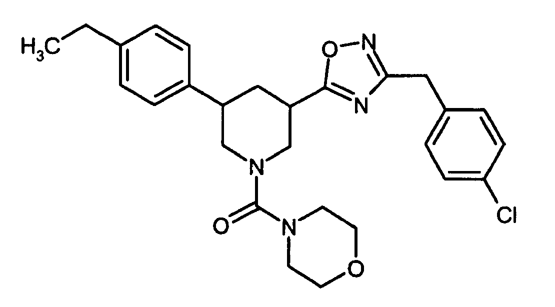

Nach der Allgemeinen Methode 1 wurden 69 mg (0.20 mmol) 5-(4-Ethylphenyl)-1-(morpholin-4-ylcarbonyl)piperidin-3-carbonsäure (Beispiel 38A) und 41 mg (0.22 mmol, 1.1 eq.) 2-(4-Chlorphenyl)-N'-hydroxyethanimidamid umgesetzt. Ausbeute: 62 mg (63% d. Th.)

HPLC (Methode 1A): Rₜ = 5.33 min; MS (ESIpos): m/z = 495 [M+H]⁺;

¹H-NMR (400 MHz, DMSO-d₆): δ = 7.39 (d, 2H), 7.33 (d, 2H), 7.21 (d, 2H), 7.16 (d, 2H), 4.10 (s, 2H), 3.98 (br d, 1H), 3.57 (br d, 1H), 3.57-3.51 (m, 4H), 3.43-3.34 (m, 1H), 3.21-3.14 (m, 4H), 2.99 (t, 1H), 2.92 (q, 1H), 2.89-2.79 (m, 1H), 2.56 (q, 2H), 2.27 (br d, 1H), 1.93 (q, 1H), 1.16 (t, 3H).

### Beispiel 66

### 4-({3-(4-Ethylphenyl)-5-[3-(4-methylbenzyl)-1,2,4-oxadiazol-5-yl]piperidin-1-yl}carbonyl)-morpholin [racemisches cis-Isomer]

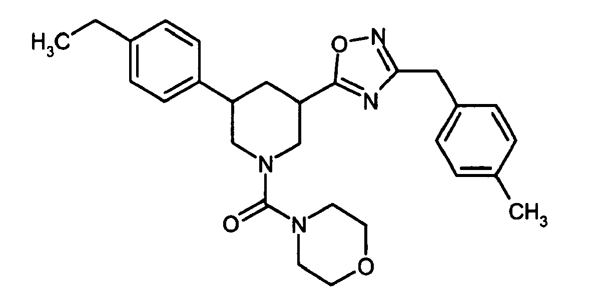

Nach der Allgemeinen Methode 1 wurden 69 mg (0.20 mmol) 5-(4-Ethylphenyl)-1-(morpholin-4-ylcarbonyl)piperidin-3-carbonsäure (Beispiel 38A) und 36 mg (0.22 mmol, 1.1 eq.) *N*'-Hydroxy-2-(4-methylphenyl)ethanimidamid umgesetzt. Ausbeute: 57 mg (60% d. Th.)

HPLC (Methode 1A): Rₜ= 5.31 min; MS (ESIpos): m/z = 475 [M+H]⁺;

¹H-NMR (400 MHz, DMSO-d₆): δ = 7.23-7.10 (m, 8H), 4.02 (s, 2H), 3.97 (br d, 1H), 3.57 (br d, 1H), 3.57-3.50 (m, 4H), 3.42-3.31 (m, 1H), 3.21-3.12 (m, 4H), 2.99 (t, 1H), 2.91 (q, 1H), 2.88-2.79 (m, 1H), 2.56 (q, 2H), 2.26 (s, 3H), 2.25 (br d, 1H), 1.92 (q, 1H), 1.16 (t, 3H).

### Beispiel 67

### 4-({3-[3-(2-Chlor-4-fluorbenzyl)-1,2,4-oxadiazol-5-yl]-5-(4-ethylphenyl)piperidin-1-yl}carbonyl)-morpholin [racemisches cis-Isomer]

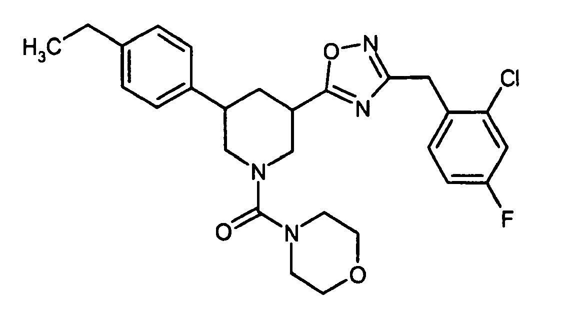

Nach der Allgemeinen Methode 1 wurden 69 mg (0.20 mmol) 5-(4-Ethylphenyl)-1-(morpholin-4-ylcarbonyl)piperidin-3-carbonsäure (Beispiel 38A) und 45 mg (0.22 mmol, 1.1 eq.) 2-(2-Chlor-4-fluorphenyl)-N'-hydroxyethanimidamid umgesetzt. Ausbeute: 59 mg (57% d. Th.)

HPLC (Methode 1A): Rₜ = 5.29 min; MS (ESIpos): m/z = 513 [M+H]⁺;

¹H-NMR (400 MHz, DMSO-d₆): δ = 7.53-7.45 (m, 2H), 7.26-7.19 (m, 3H), 7.16 (d, 2H), 4.20 (s, 2H), 3.97 (br d, 1H), 3.58 (br d, 1H), 3.58-3.51 (m, 4H), 3.43-3.34 (m, 1H), 3.22-3.14 (m, 4H), 3.05-2.89 (m, 2H), 2.89-2.79 (m, 1H), 2.56 (q, 2H), 2.27 (br d, 1H), 1.92 (q, 1H), 1.16 (t, 3H).

### Beispiel 68

### 4-({3-(4-Ethylphenyl)-5-[3-(phenoxymethyl)-1,2,4-oxadiazol-5-yl]piperidin-1-yl}carbonyl)-morpholin [racemisches cis-Isomer]

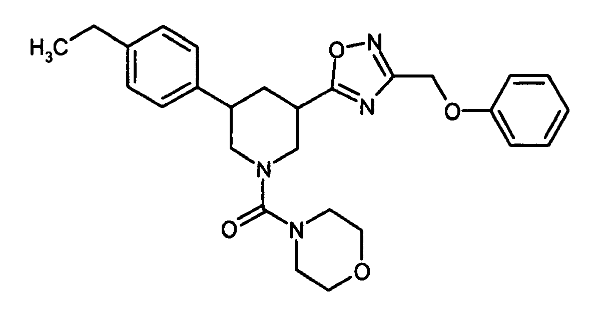

Nach der Allgemeinen Methode 1 wurden 69 mg (0.20 mmol) 5-(4-Ethylphenyl)-1-(morpholin-4-ylcarbonyl)piperidin-3-carbonsäure (Beispiel 38A) und 37 mg (0.22 mmol, 1.1 eq.) *N*'-Hydroxy-2-phenoxyethanimidamid umgesetzt. Ausbeute: 40 mg (42% d. Th.)

HPLC (Methode 1A): Rₜ = 5.06 min; MS (ESIpos): m/z = 477 [M+H]⁺;

¹H-NMR (400 MHz, DMSO-d₆): δ = 7.32 (t, 2H), 7.23 (d, 2H), 7.17 (d, 2H), 7.05 (d, 2H), 6.99 (t, 1H), 5.26 (s, 2H), 4.02 (br d, 1H), 3.60 (br d, 1H), 3.60-3.52 (m, 4H), 3.50-3.40 (m, 1H), 3.23-3.16 (m, 4H), 3.06 (t, 1H), 2.93 (q, 1H), 2.91-2.82 (m, 1H), 2.57 (q, 2H), 2.32 (br d, 1H), 1.98 (q, 1H), 1.16 (t, 3H).

### Beispiel 69

### 1-(Cyclopentylcarbonyl)-3-(4-ethylphenyl)-5-(3-methyl-1,2,4-oxadiazol-5-yl)piperidin [racemisches cis-Isomer]

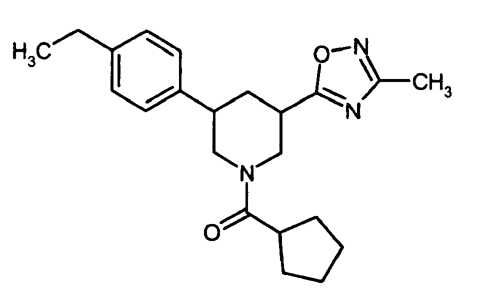

Nach der Allgemeinen Methode 1 wurden 66 mg (0.20 mmol) 1-(Cyclopentylcarbonyl)-5-(4-ethylphenyl)piperidin-3-carbonsäure (Beispiel 7A) und 24 mg (0.22 mol, 1.1 eq.) *N'-*Hydroxyethanimidamid umgesetzt. Ausbeute: 58 mg (79% d. Th.)

HPLC (Methode 2A): Rₜ = 5.04 min; MS (ESIpos): m/z = 368 [M+H]⁺;

¹H-NMR (500 MHz, DMSO-d₆): δ = 7.25 (d, 1H), 7.23-7.14 (m, 3H), 4.85 (br d, 0.5H), 4.50 (br d, 0.5H), 4.38 (br d, 0.5H), 3.99 (br d, 0.5H), 3.37-3.28 (m, 1H), 3.23-3.13 (m, 1H), 3.12-3.01 (m, 1H), 2.88-2.74 (m, 1H), 2.73-2.62 (m, 1H), 2.58 (q, 2H), 2.33 (s, 3H), 2.33-2.25 (m, 1H), 2.09-1.94 (m, 1H), 1.86-1.47 (m, 8H), 1.17 (t, 3H).

### Beispiel 70

### 1-(Cyclopentylcarbonyl)-3-(4-ethylphenyl)-5-(3-propyl-1,2,4-oxadiazol-5-yl)piperidin [racemisches cis-Isomer]

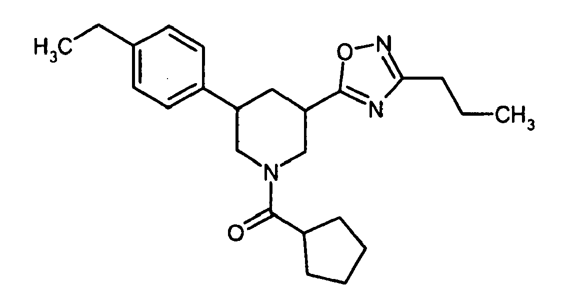

Nach der Allgemeinen Methode 1 wurden 66 mg (0.20 mmol) 1-(Cyclopentylcarbonyl)-5-(4-ethylphenyl)piperidin-3-carbonsäure (Beispiel 7A) und 22 mg (0.22 mmol, 1.1 eq.) *N'-*Hydroxybutanimidamid umgesetzt. Ausbeute: 12 mg (15% d. Th.)

HPLC (Methode 2A): Rₜ = 5.41 min; MS (ESIpos): m/z = 396 [M+H]⁺;

¹H-NMR (500 MHz, DMSO-d₆): δ = 7.30-7.13 (m, 4H), 4.85 (br d, 0.5H), 4.50 (br d, 0.5H), 4.38 (br d, 0.5H), 3.99 (br d, 0.5H), 3.39-3.29 (m, 1H), 3.24-3.14 (m, 1H), 3.14-3.00 (m, 1H), 2.88-2.75 (m, 1H), 2.75-2.62 (m, 1H), 2.67 (t, 2H), 2.57 (q, 2H), 2.34-2.25 (m, 1H), 2.10-1.93 (m, 1H), 1.86-1.45 (m, 8H), 1.73-1.66 (m, 2H), 1.17 (t, 3H), 0.92 (t, 3H).

### Beispiel 71

### 3-(3-tert.-Butyl-1,2,4-oxadiazol-5-yl)-1-(cyclopentylcarbonyl)-5-(4-ethylphenyl)piperidin [racemisches cis-Isomer]

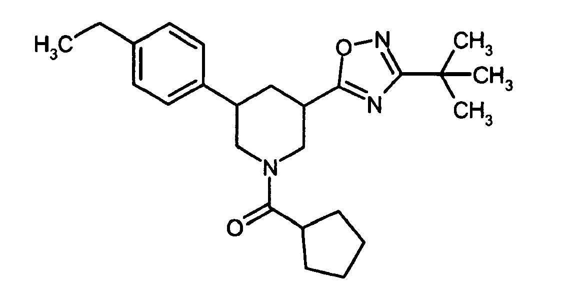

Nach der Allgemeinen Methode 1 wurden 66 mg (0.20 mmol) 1-(Cyclopentylcarbonyl)-5-(4-ethylphenyl)piperidin-3-carbonsäure (Beispiel 7A) und 26 mg (0.22 mmol, 1.1 eq.) *N'*-Hydroxy-2,2-dimethylpropanimidamid umgesetzt. Ausbeute: 49 mg (60% d. Th.)

HPLC (Methode 2A): Rₜ = 5.63 min; MS (ESIpos): m/z = 410 [M+H]⁺;

¹H-NMR (500 MHz, DMSO-d₆): δ = 7.26 (d, 1H), 7.24-7.14 (m, 3H), 4.85 (br d, 0.5H), 4.49 (br d, 0.5H), 4.37 (br d, 0.5H), 3.99 (br d, 0.5H), 3.40-3.31 (m, 1H), 3.25-3.16 (m, 1H), 3.14-3.01 (m, 1H), 2.87-2.76 (m, 1H), 2.75-2.67 (m, 1H), 2.58 (q, 2H), 2.33-2.23 (m, 1H), 2.09-1.92 (m, 1H), 1.85-1.44 (m, 8H), 1.31 (s, 9H), 1.17 (t, 3H).

### Beispiel 72

### cis-(3,5)-3-(3-tert.-Butyl-1,2,4-oxadiazol-5-yl)-1-(cyclopentylcarbonyl)-5-[4-(1-methylethyl)-phenyl]piperidin [racemisches cis-Isomer]

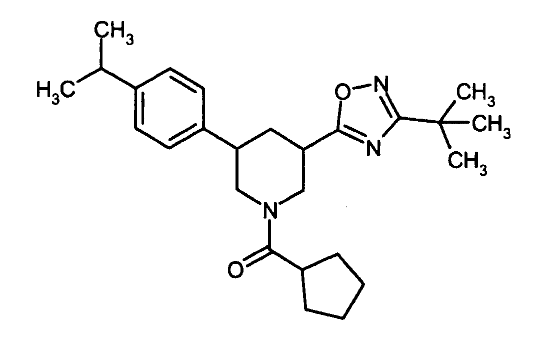

Nach der Allgemeinen Methode 2 wurden 73 mg (0.21 mmol) cis-(3,5)-1-(Cyclopentylcarbonyl)-5-[4-(1-methylethyl)phenyl]piperidin-3-carbonsäure (Beispiel 12A) und 37 mg (0.32 mmol, 1.5 eq.) *N'*-Hydroxy-2,2-dimethylpropanimidamid umgesetzt. Ausbeute: 29 mg (33% d. Th.)

LC-MS (Methode 3B): Rₜ = 2.88 min; MS (ESIpos): m/z = 424 [M+H]⁺.

### Beispiel 73

### 4-{[3-(3-tert.-Butyl-1,2,4-oxadiazol-5-yl)-5-(4-ethylphenyl)piperidin-1-yl]carbonyl} morpholin [racemisches cis-Isomer]

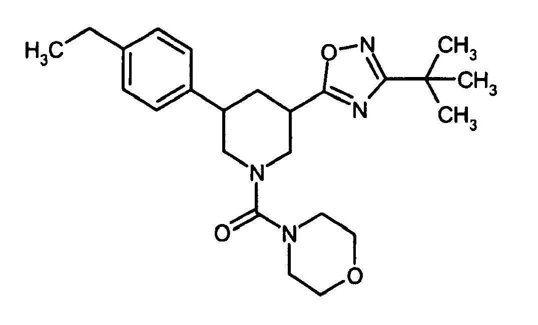

Nach der Allgemeinen Methode 1 wurden 69 mg (0.20 mmol) 5-(4-Ethylphenyl)-1-(morpholin-4-ylcarbonyl)piperidin-3-carbonsäure (Beispiel 38A) und 26 mg (0.22 mmol, 1.1 eq.) *N'*-Hydroxy-2,2-dimethylpropanimidamid umgesetzt. Ausbeute: 60 mg (70% d. Th.)

HPLC (Methode 1A): Rₜ = 4.90 min; MS (ESIpos): m/z = 427 [M+H]⁺;

¹H-NMR (400 MHz, DMSO-d₆): δ = 7.22 (d, 2H), 7.16 (d, 2H), 3.99 (br d, 1H), 3.59 (br d, 1H), 3.59-3.50 (m, 4H), 3.43-3.32 (m, 1H), 3.23-3.13 (m, 4H), 2.99 (q, 2H), 2.92-2.80 (m, 1H), 2.57 (q, 2H), 2.29 (br d, 1H), 1.94 (q, 1H), 1.30 (s, 9H), 1.16 (t, 3H).

### Beispiel 74

### 4-({3-(3-tert.-Butyl-1,2,4-oxadiazol-5-yl)-5-[4-(trifluormethoxy)phenyl]piperidin-1-yl} carbonyl)-morpholin [racemisches cis-Isomer]

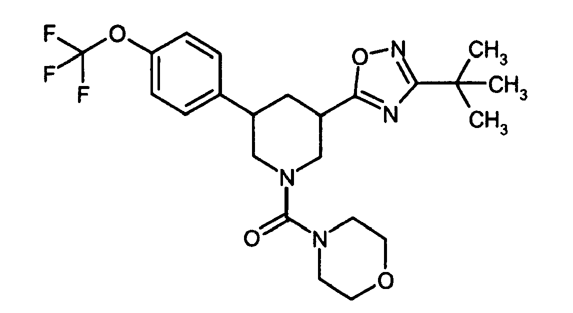

Nach der Allgemeinen Methode 1 wurden 80 mg (0.20 mmol) 1-(Morpholin-4-ylcarbonyl)-5-[4-(trifluormethoxy)phenyl]piperidin-3-carbonsäure (Beispiel 44A) und 26 mg (0.22 mmol, 1.1 eq.) *N'*-Hydroxy-2,2-dimethylpropanimidamid umgesetzt. Ausbeute: 68 mg (70% d. Th.)

HPLC (Methode 2A): Rₜ = 5.08 min; MS (ESIpos): m/z = 483 [M+H]⁺;

¹H-NMR (400 MHz, DMSO-d₆): δ = 7.48 (d, 2H), 7.33 (d, 2H), 3.99 (br d, 1H), 3.60 (br d, 1H), 3.59-3.51 (m, 4H), 3.43-3.33 (m, 1H), 3.23-3.15 (m, 4H), 3.09-2.91 (m, 3H), 2.31 (br d, 1H), 1.96 (q, 1H), 1.30 (s, 9H).

### Beispiel 75

### 5-[1-(Cyclopentylcarbonyl)-5-(4-ethylphenyl)piperidin-3-yl]-1,2,4-oxadiazol-3-carbonsäure-ethylester [racemisches cis-Isomer]

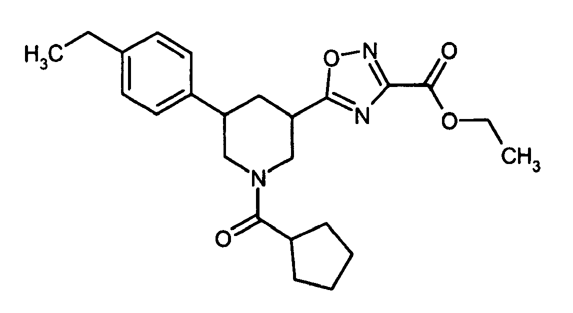

Nach der Allgemeinen Methode 1 wurden 198 mg (0.60 mmol) 1-(Cyclopentylcarbonyl)-5-(4-ethylphenyl)piperidin-3-carbonsäure (Beispiel 7A) und 87mg (0.66 mmol, 1.1 eq.) 2-Amino(hydroxyimino)ethansäureethylester umgesetzt. Ausbeute: 73 mg (27% d. Th.)

HPLC (Methode 1A): Rₜ = 5.28 min; MS (ESIpos): m/z = 426 [M+H]⁺;

¹H-NMR (500 MHz, DMSO-d₆): δ = 7.30-7.12 (m, 4H), 4.89 (br d, 0.5H), 4.50 (br d, 0.5H), 4.47-4.37 (m, 2.5H), 4.01 (br d, 0.5H), 3.52-3.32 (m, 1H), 3.20 (t, 1H), 3.14-3.00 (m, 1H), 2.82-2.79 (m, 1H), 2.79-2.63 (m, 1H), 2.58 (q, 2H), 2.39-2.27 (m, 1H), 2.18-1.99 (m, 1H), 1.89-1.43 (m, 8H), 1.33 (t, 3H), 1.17 (t, 3H).

### Beispiel 76

### cis-(3,5)-1-(Cyclopentylcarbonyl)-3-(4-methoxyphenyl)-5-{3-[(methylsulfonyl)methyl]-1,2,4-oxadiazol-5-yl}piperidin [racemisches cis-Isomer]

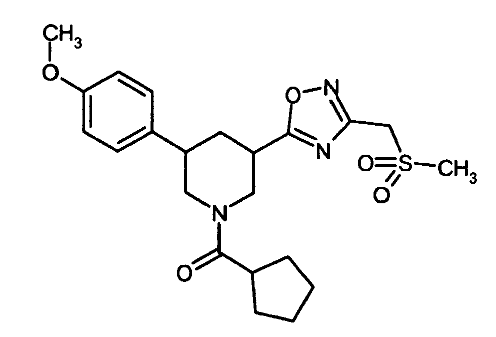

Nach der Allgemeinen Methode 2 wurden 100 mg (0.27 mmol) cis-(3,5)-1-(Cyclopentylcarbonyl)-5-(4-methoxyphenyl)piperidin-3-carbonsäure (Beispiel 30A) und 63 mg (0.41 mmol, 1.5 eq.) *N'-*Hydroxy-2-(methylsulfonyl)ethanimidamid umgesetzt. Ausbeute: 17 mg (14% d. Th.)

LC-MS (Methode 3B): Rₜ = 1.88 min; MS (ESIpos): m/z = 448 [M+H]⁺.

### Beispiel 77

### 4-[(3-{3-[(Methylsulfonyl)methyl]-1,2,4-oxadiazol-5-yl}-5-[4-(trifluormethoxy)phenyl]piperidin-1-yl)carbonyl]morpholin [racemisches cis-Isomer]

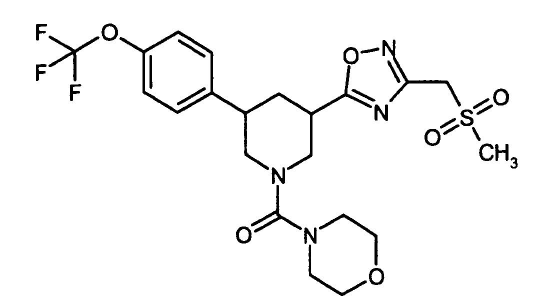

Nach der Allgemeinen Methode 1 wurden 80 mg (0.20 mmol) 1-(Morpholin-4-ylcarbonyl)-5-[4-(trifluonnethoxy)phenyl]piperidin-3-carbonsäure (Beispiel 44A) und 33 mg (0.22 mmol, 1.1 eq.) *N'*-Hydroxy-2-(methylsulfonyl)ethanimidamid umgesetzt. Ausbeute: 50 mg (48% d. Th.)

HPLC (Methode 2A): Rₜ = 4.37 min; MS (ESIpos): m/z = 519 [M+H]⁺;

¹H-NMR (400 MHz, DMSO-d₆): δ = 7.48 (d, 2H), 7.33 (d, 2H), 4.85 (s, 2H), 4.03 (br d, 1H), 3.61 (br d, 1H), 3.60-3.53 (m, 4H), 3.52-3.43 (m, 1H), 3.25-3.18 (m, 4H), 3.17 (s, 3H), 3.07 (t, 1H), 3.03-2.94 (m, 2H), 2.35 (br d, 1H), 2.00 (q, 1H).

### Beispiel 78

### 4-({5-[1-(Cyclopentylcarbonyl)-5-(4-ethylphenyl)piperidin-3-yl]-1,2,4-oxadiazol-3-yl}methyl)-morpholin [racemisches cis-Isomer]

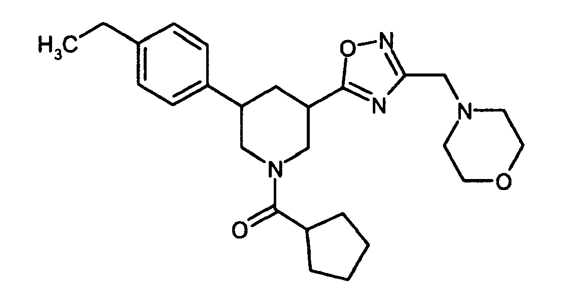

Nach der Allgemeinen Methode 1 wurden 66 mg (0.20 mmol) 1-(Cyclopentylcarbonyl)-5-(4-ethylphenyl)piperidin-3-carbonsäure (Beispiel 7A) und 35 mg (0.22 mmol, 1.1 eq.) *N'*-Hydroxy-2-morpholin-4-ylethanimidamid umgesetzt. Ausbeute: 7 mg (8% d. Th.)

HPLC (Methode 2A): Rₜ = 4.46 min; MS (ESIpos): m/z = 453 [M+H]⁺;

¹H-NMR (500 MHz, DMSO-d₆): δ = 7.30-7.13 (m, 4H), 4.89 (br d, 0.5H), 4.51 (br d, 0.5H), 4.40 (br d, 0.5H), 4.41 (br d, 0.5H), 3.43-3.30 (m, 1H), 3.24-3.16 (m, 1H), 3.14-3.00 (m, 1H), 2.90-2.77 (m, 1H), 2.77-2.61 (m, 1H), 2.58 (q, 2H), 2.38-2.27 (m, 1H), 2.11-1.94 (m, 1H), 1.87-1.44 (m, 8H), 1.17 (t, 3H).

### Beispiel 79

### 4-[(5- {1-(Morpholin-4-ylcarbonyl)-5-[4-(trifluormethoxy)phenyl]piperidin-3-yl} -1 ,2,4-oxadiazol-3-yl)methyl]morpholin [racemisches cis-Isomer]

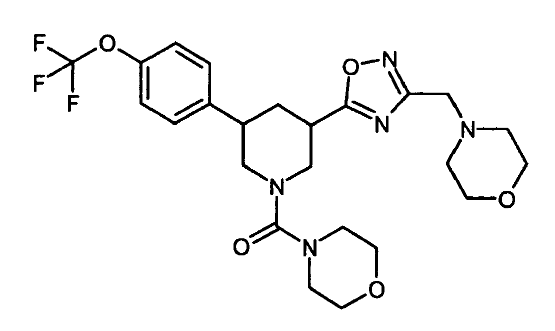

Nach der Allgemeinen Methode 1 wurden 80 mg (0.20 mmol) 1-(Morpholin-4-ylcarbonyl)-5-[4-(trifluormethoxy)phenyl]piperidin-3-carbonsäure (Beispiel 44A) und 35 mg (0.22 mmol, 1.1 eq.) *N'*-Hydroxy-2-morpholin-4-ylethanimidamid umgesetzt. Ausbeute: 28 mg (26% d. Th.)

HPLC (Methode 2A): Rₜ = 4.13 min; MS (ESIpos): m/z = 526 [M+H]⁺;

¹H-NMR (400 MHz, DMSO-d₆): δ = 7.47 (d, 2H), 7.33 (d, 2H), 4.00 (br d, 1H), 3.64 (s, 2H), 3.61 (br d, 1H), 3.59-3.52 (m, 4H), 3.47-3.36 (m, 1H), 3.32-3.26 (m, 4H), 3.05 (t, 1H), 3.03-2.93 (m, 2H), 2.32 (br d, 1H), 1.98 (q, 1H).

### Beispiel 80

### 4-({3-(4-Ethylphenyl)-5-[3-(1-methyl-1,2,5,6-tetrahydropyridin-3-yl)-1,2,4-oxadiazol-5-yl]piperidin-1-yl}carbonyl)morpholin [racemisches cis-Isomer]

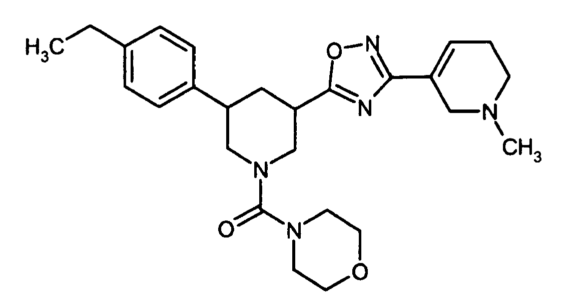

Nach der Allgemeinen Methode 1 wurden 69 mg (0.20 mmol) 5-(4-Ethylphenyl)-1-(morpholin-4-ylcarbonyl)piperidin-3-carbonsäure (Beispiel 38A) und 34 mg (0.22 mmol, 1.1 eq.) *N'*-Hydroxy-1-methyl-1,2,5,6-tetrahydropyridin-3-carboximidamid [P. Sauerberg et al. J.Med.Chem. 1991, 34, 687-692] umgesetzt. Ausbeute: 35 mg (38% d. Th.)

HPLC (Methode 2A): Rₜ = 4.17 min; MS (ESIpos): m/z = 466 [M+H]⁺;

¹H-NMR (500 MHz, DMSO-d₆): δ = 7.22 (d, 2H), 7.17 (d, 2H), 6.90-6.84 (m, 1H), 4.01 (br d, 1H), 3.61 (br d, 1H), 3.60-3.53 (m, 4H), 3.44-3.33 (m, 1H), 3.23-3.14 (m, 6H), 3.03 (t, 1H), 2.95 (q, 1H), 2.91-2.82 (m, 1H), 2.57 (q, 2H), 2.38-2.26 (m, 6H), 1.97 (q, 1H), 1.16 (t, 3H).

### Beispiel 81

### 4-({3-[3-(1-Methyl-1,2, 5,6-tetrahydropyridin-3-yl)-1,2,4-oxadiazol-5-yl]-5-[4-(trifluormethoxy)-phenyl]piperidin-1-yl}carbonyl)morpholin [racemisches cis-Isomer]

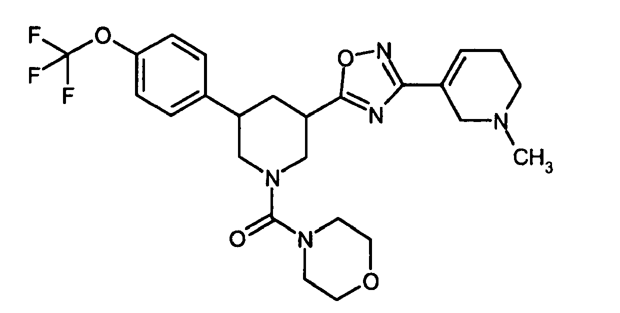

Nach der Allgemeinen Methode 1 wurden 80 mg (0.20 mmol) -(Morpholin-4-ylcarbonyl)-5-[4-(trifluormethoxy)phenyl]piperidin-3-carbonsäure (Beispiel 44A) und 34 mg (0.22 mmol, 1.1 eq.) *N'*-Hydroxy-1-methyl-1,2,5,6-tetrahydropyridin-3-carboximidamid [P. Sauerberg et al. J.Med.Chem. 1991, 34, 687-692] umgesetzt. Ausbeute: 23 mg (22% d. Th.)

HPLC (Methode 2A): Rₜ = 4.21 min; MS (ESIpos): m/z = 522 [M+H]⁺;

¹H-NMR (400 MHz, DMSO-d₆): δ = 7.48 (d, 2H), 7.33 (d, 2H), 6.90-6.84 (m, 1H), 4.01 (br d, 1H), 3.61 (br d, 1H), 3.59-3.53 (m, 4H), 3.46-3.35 (m, 1H), 3.24-3.14 (m, 6H), 3.05 (t, 1H), 3.03-2.93 (m, 2H), 2.38-2.28 (m, 6H), 1.99 (q, 1H).

### Beispiel 82

### [(5-{1-(Morpholin-4-ylcarbonyl)-5-[4-(trifluormethoxy)phenyl]piperidin-3-yl}-1,2,4-oxadiazol-3-yl)methyl]-tert.-butylcarbamat [racemisches cis-Isomer]

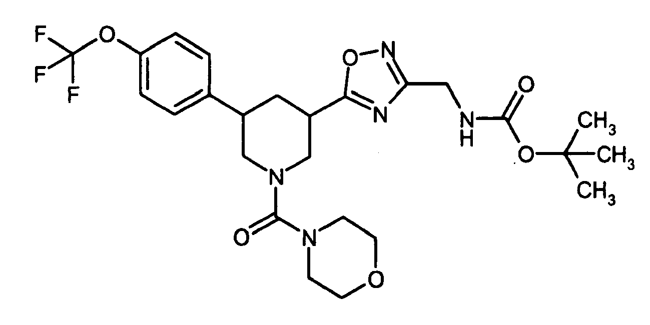

Nach der Allgemeinen Methode 1 wurden 161 mg (0.40 mmol) 1-(Morpholin-4-ylcarbonyl)-5-[4-(trifluormethoxy)phenyl]piperidin-3-carbonsäure (Beispiel 44A) und 83 mg (0.44 mmol, 1.1 eq.) [(2-Amino-2-(hydroxyimino)ethyl]-*tert.*-butylcarbamat umgesetzt. Ausbeute: 127 mg (57% d. Th.)

HPLC (Methode 2A): Rₜ = 4.73 min; MS (ESIpos): m/z = 556 [M+H]⁺;

¹H-NMR (400 MHz, DMSO-d₆): δ = 7.52-7.44 (m, 3H), 7.33 (d, 2H), 4.23 (d, 1H), 3.99 (br d, 1H), 3.67-3.57 (m, 1H), 3.59-3.53 (m, 4H), 3.46-3.36 (m, 1H), 3.23-3.15 (m, 4H), 3.04 (t, 1H), 3.03-2.93 (m, 2H), 2.31 (br d, 1H), 1.98 (q, 1H), 1.38 (s, 9H).

### Beispiel 83

### 1-(5-{1-(Morpholin-4-ylcarbonyl)-5-[4-(trifluormethoxy)phenyl]piperidin-3-yl}-1,2,4-oxadiazol-3-yl)methanamin [racemisches cis-Isomer]

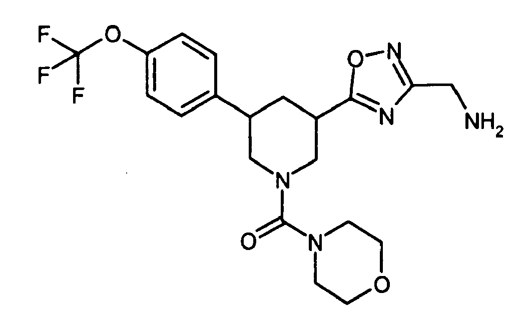

Eine Lösung aus 111 mg (0.2 mmol) [(5-{1-(Morpholin-4-y1carbonyl)-5-[4-(trinfluormethoxy)-phenyl]piperidin-3-yl}-1,2,4-oxadiazol-3-yl)methyl]-*tert*.-butylcarbamat (Beispiel 82) in 3 ml Dichlormethan wurde bei RT mit insgesamt 185 µl (2.4 mmol, 12 eq.) Trifluoressigsäure umgesetzt und anschließend im Vakuum eingeengt. Das Rohprodukt wurde mittels präparativer HPLC (Säule: Reprosil C18, 10 µm, 250 mm x 30 mm; Eluent: Acetonitril/Wasser mit 0.1% Triethylamin-Gradient) aufgereinigt. Ausbeute: 76 mg (83% d. Th.)

LC-MS (Methode 3B): Rₜ = 1.23 min; MS (ESIpos): m/z = 456 [M+H]⁺;

¹H-NMR (400 MHz, DMSO-d₆): δ = 7.47 (d, 2H), 7.33 (d, 2H), 3.99 (br d, 1H), 3.77 (s, 2H), 3.61 (br d, 1H), 3.60-3.52 (m, 4H), 3.45-3.35 (m, 1H), 3.23-3.16 (m, 4H), 3.04 (t, 1H), 3.02-2.93 (m, 2H), 2.32 (br d, 1H), 1.98 (q, 1H), 1.98-1.83 (brs, 2H).

### Beispiel 84

### N-Ethyl-3-(4-ethylphenyl)-N-methyl-5-(3-phenyl-1,2,4-oxadiazol-5-yl)piperidin-1-carbonsäureamid [racemisches cis-Isomer]

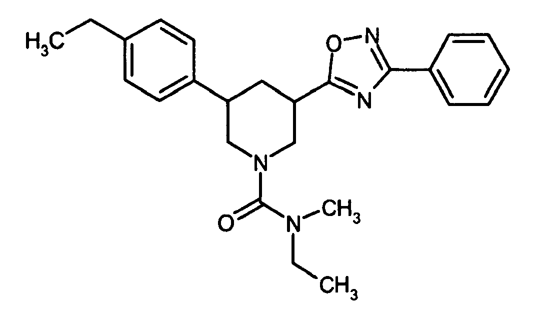

Nach der Allgemeinen Methode 1 wurden 64 mg (0.20 mmol) 1-[Ethyl(methyl)carbamoyl]-5-(4-ethylphenyl)piperidin-3-carbonsäure (Beispiel 45A) und 30 mg (0.22 mmol, 1.1 eq.) *N'*-Hydroxybenzencarboximidamid umgesetzt. Ausbeute: 12 mg (14% d. Th.)

HPLC (Methode 2A): Rₜ = 5.36 min; MS (ESIpos): m/z = 419 [M+H]⁺;

¹H-NMR (400 MHz, DMSO-d₆): δ = 8.05-8.00 (m, 2H), 7.62-7.53 (m, 3H), 7.24 (d, 2H), 7.18 (d, 2H), 4.01 (br d, 1H), 3.58-3.46 (m, 2H), 3.17 (q, 2H), 3.06 (t, 1H), 2.95-2.87 (m, 2H), 2.79 (s, 3H), 2.58 (q, 2H), 2.38 (br d, 1H), 2.03 (q, 1H), 1.17 (t, 3H), 1.08 (t, 3H).

### Beispiel 85

### N-Ethyl-3-(4-ethylphenyl)-5-[3-(4-fluorphenyl)-1,2,4-oxadiazol-5-yl]-N-methylpiperidin-1-carbonsäureamid [racemisches cis-Isomer]

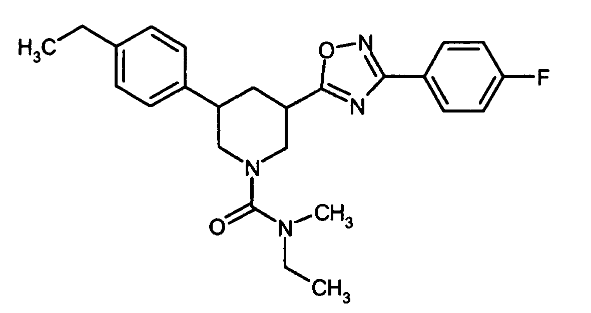

Nach der Allgemeinen Methode 1 wurden 64 mg (0.20 mmol) 1-[Ethyl(methyl)carbamoyl]-5-(4-ethylphenyl)piperidin-3-carbonsäure (Beispiel 45A) und 34 mg (0.22 mmol, 1.1 eq.) 4-Fluor-*N'-*hydroxybenzencarboximidamid umgesetzt. Ausbeute: 46 mg (52% d. Th.)

HPLC (Methode 2A): Rₜ = 5.40 min; MS (ESIpos): m/z = 437 [M+H]⁺;

¹H-NMR (500 MHz, DMSO-d₆): δ = 8.12-8.04 (m, 2H), 7.46-7.38 (m, 2H), 7.24 (d, 2H), 7.17 (d, 2H), 4.01 (br d, 1H), 3.58-3.46 (m, 2H), 3.17 (q, 2H), 3.05 (t, 1H), 2.97-2.85 (m, 2H), 2.79 (s, 3H), 2.57 (q, 2H), 2.37 (br d, 1H), 2.03 (q, 1H), 1.17 (t, 3H), 1.08 (t, 3H).

### Beispiel 86

### 4-({3-[3-(4-Methylphenyl)-1,2,4-oxadiazol-5-yl]-5-[4-(trifluonnethoxy)phenyl]piperidin-1-yl}-carbonyl)morpholin [racemisches cis-Isomer]

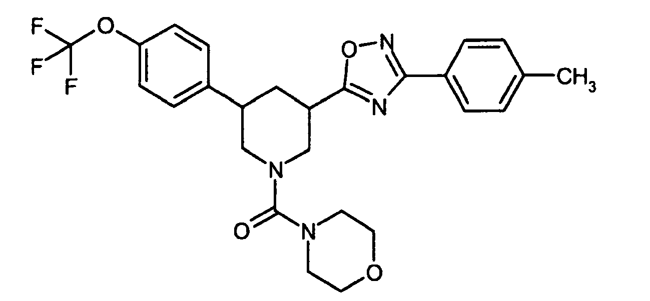

Nach der Allgemeinen Methode 1 wurden 80 mg (0.20 mmol) 1-(Morpholin-4-ylcarbonyl)-5-[4-(trifluormethoxy)phenyl]piperidin-3-carbonsäure (Beispiel 44A) und 33 mg (0.22 mmol, 1.1 eq.) *N'*-Hydroxy-4-methylbenzencarboximidamid umgesetzt. Ausbeute: 68 mg (66% d. Th.)

HPLC (Methode 2A): Rₜ = 5.25 min; MS (ESIpos): m/z = 517 [M+H]⁺;

¹H-NMR (400 MHz, DMSO-d₆): δ = 7.90 (d, 2H), 7.49 (d, 2H), 7.37 (d, 2H), 7.34 (d, 2H), 4.08 (br d, 1H), 3.64 (br d, 1H), 3.62-3.54 (m, 4H), 3.54-3.44 (m, 1H), 3.27-3.18 (m, 4H), 3.13 (t, 1H), 3.07-2.97 (m, 2H), 2.40 (br d, 1H), 2.39 (s, 3H), 2.06 (q, 1H).

### Beispiel 87

### 3-[3-(2,6-Difluorphenyl)-1,2,4-oxadiazol-5-yl]-N-ethyl-5-(4-ethylphenyl)-N-methylpiperidin-1-carbonsäureamid [racemisches cis-Isomer]

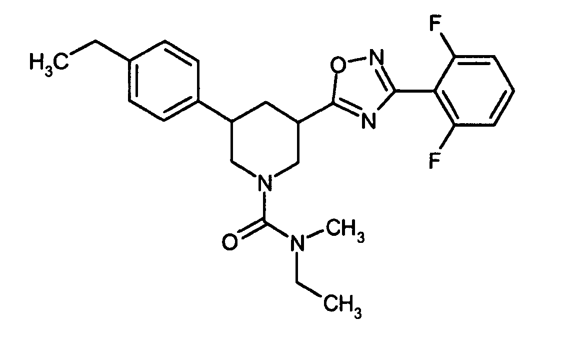

Nach der Allgemeinen Methode 1 wurden 64 mg (0.20 mmol) 1-[Ethyl(methyl)carbamoyl]-5-(4-ethylphenyl)piperidin-3-carbonsäure (Beispiel 45A) und 38 mg (0.22 mmol, 1.1 eq.) 2,6-Difluor-*N'*-hydroxybenzencarboximidamid umgesetzt. Ausbeute: 23 mg (26% d. Th.)

HPLC (Methode 2A): Rₜ= 5.18 min; MS (ESIpos): m/z = 455 [M+H]⁺;

¹H-NMR (400 MHz, DMSO-d₆): δ = 7.77-7.68 (m, 1H), 7.36 (t, 2H), 7.24 (d, 2H), 7.16 (d, 2H), 4.01 (br d, 1H), 3.62-3.50 (m, 2H), 3.17 (q, 2H), 3.05 (t, 1H), 2.97-2.85 (m, 2H), 2.78 (s, 3H), 2.57 (q, 2H), 2.38 (br d, 1H), 2.03 (q, 1H), 1.17 (t, 3H), 1.07 (t, 3H).

### Beispiel 88

### 4-({3-[3-(2-Chlorpyridin-4-yl)-1,2,4-oxadiazol-5-yl]-5-[4-(trifluormethoxy)phenyl]piperidin-1-yl}-carbonyl)morpholin [racemisches cis-Isomer]

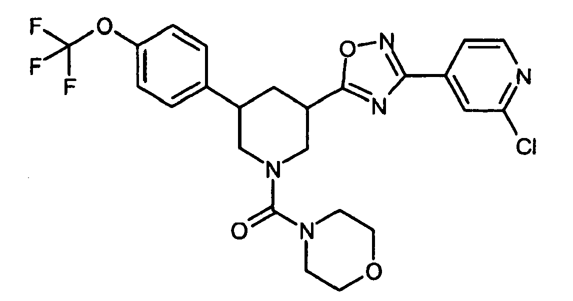

Nach der Allgemeinen Methode 1 wurden 600 mg (1.49 mmol) 1-(Morpholin-4-ylcarbonyl)-5-[4-(trifluormethoxy)phenyl]piperidin-3-carbonsäure (Beispiel 44A) und 281 mg (1.64 mmol, 1.1 eq.) 2-Chlor-*N'*-hydroxypyridin-4-carboximidamid umgesetzt. Ausbeute: 315 mg (39% d. Th.)

HPLC (Methode 2A): Rₜ = 4.96 min; MS (ESIpos): m/z = 538 [M+H]⁺;

¹H-NMR (400 MHz, DMSO-d₆): δ = 8.65 (dd, 1H), 8.00 (s, 1H), 7.98 (dd, 1H), 7.49 (d, 2H), 7.36 (d, 2H), 4.10 (br d, 1H), 3.64 (br d, 1H), 3.62-3.50 (m, 5H), 3.27-3.18 (m, 4H), 3.14 (t, 1H), 3.08-2.97 (m, 2H), 2.41 (br d, 1H), 2.08 (q, 1H).

### Beispiel 89

### 3-[3-(2-Chlorpyridin-4-yl)-1,2,4-oxadiazol-5-yl]-N-ethyl-5-(4-ethylphenyl)-N-methylpiperidin-1-carbonsäureamid [racemisches cis-Isomer]

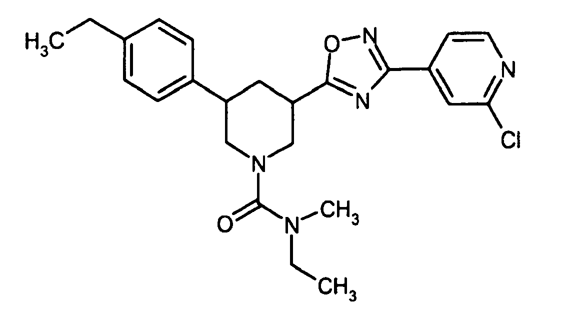

Nach der Allgemeinen Methode 1 wurden 64 mg (0.20 mmol) 1-[Ethyl(methyl)carbamoyl]-5-(4-ethylphenyl)piperidin-3-carbonsäure (Beispiel 45A) und 38 mg (0.22 mmol, 1.1 eq.) 2-Chlor-*N'-*hydroxypyridin-4-carboximidamid umgesetzt. Ausbeute: 13 mg (14% d. Th.)

HPLC (Methode 2A): Rₜ = 5.22 min; MS (ESIpos): m/z = 454 [M+H]⁺;

¹H-NMR (400 MHz, DMSO-d₆): δ = 8.65 (dd, 1H), 8.00 (s, 1H), 7.98 (dd, 1H), 7.24 (d, 2H), 7.18 (d, 2H), 4.02 (br d, 1H), 3.61-3.50 (m, 2H), 3.17 (q, 2H), 3.06 (t, 1H), 2.98-2.86 (m, 2H), 2.79 (s, 3H), 2.58 (q, 2H), 2.38 (br d, 1H), 2.05 (q, 1H), 1.17 (t, 3H), 1.08 (t, 3H).

### Beispiel 90

### 4-({3-(1,2,4-Oxadiazol-5-yl)-5-[4-(trifluormethoxy)phenyl]piperidin-1-yl}carbonyl)morpholin [racemisches cis-Isomer]

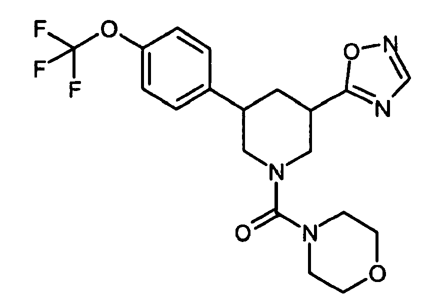

Die Titelverbindung wurde aus der Umsetzung von Beispiel 92 mit Lithiumhydroxid in Tetrahydrofuran/Wasser bei Raumtemperatur isoliert. Ausbeute: 9 mg (57% d. Th.)

HPLC (Methode 2B): Rₜ = 1.11 min; MS (ESIpos): m/z = 427 [M+H]⁺;

¹H-NMR (500 MHz, DMSO-d₆): δ = 12.02-11.82 (br s, 1H), 7.45 (d, 2H), 7.33 (d, 2H), 3.80 (br d, 1H), 3.61-3.52 (m, 5H), 3.22-3.13 (m, 4H), 2.92-2.77 (m, 3H), 2.71-2.64 (m, 1H), 2.10 (br d, 1H), 1.80 (q, 1H).

### Beispiel 91

### 3-(3-tert.-Butyl-1,2,4-oxadiazol-5-yl)-N-ethyl-5-(4-ethylphenyl)-N-methylpiperidin-1-carbonsäureamid [racemisches cis-Isomer]

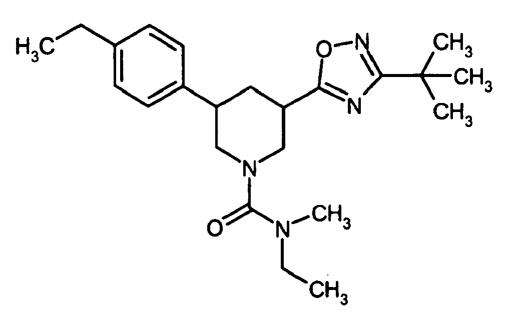

Nach der Allgemeinen Methode 1 wurden 64 mg (0.20 mmol) 1-[Ethyl(methyl)carbamoyl]-5-(4-ethylphenyl)piperidin-3-carbonsäure (Beispiel 45A) und 26 mg (0.22 mmol, 1.1 eq.) *N'*-Hydroxy-2,2-dimethylpropanimidamid umgesetzt. Ausbeute: 52 mg (65% d. Th.)

HPLC (Methode 2A): Rₜ = 5.30 min; MS (ESIpos): m/z = 399 [M+H]⁺;

¹H-NMR (400 MHz, DMSO-d₆): δ = 7.22 (d, 2H), 7.16 (d, 2H), 3.91 (br d, 1H), 3.53 (br d, 1H), 3.43-3.33 (m, 1H), 3.15 (q, 2H), 3.00-2.80 (m, 3H), 2.76 (s, 3H), 2.57 (q, 2H), 2.29 (br d, 1H), 1.93 (q, 1H), 1.30 (s, 9H), 1.16 (t, 3H), 1.06 (t, 3H).

### Beispiel 92

### 5- {1-(Morpholin-4-ylcarbonyl)-5-[4-(trifluormethoxy)phenyl]piperidin-3-yl}-1,2,4-oxadiazol-3-carbonsäureethylester [racemisches cis-Isomer]

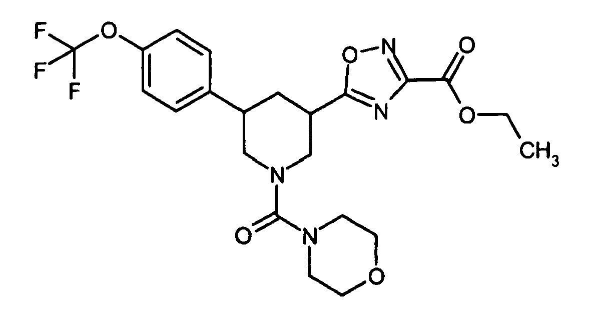

Nach der Allgemeinen Methode 1 wurden 80 mg (0.20 mmol) 1-(Morpholin-4-ylcarbonyl)-5-[4-(trifluormethoxy)phenyl]piperidin-3-carbonsäure (Beispiel 44A) und 29 mg (0.22 mmol, 1.1 eq.) 2-Amino(hydroxyimino)ethansäureethylester umgesetzt. Ausbeute: 24 mg (22% d. Th.)

HPLC (Methode 2A): Rₜ = 4.69 min; MS (ESIpos): m/z = 499 [M+H]⁺;

¹H-NMR (400 MHz, DMSO-d₆): δ = 7.48 (d, 2H), 7.34 (d, 2H), 4.41 (q, 2H), 4.03 (br d, 1H), 3.62 (br d, 1H), 3.60-3.47 (m, 5H), 3.25-3.17 (m, 4H), 3.10 (t, 1H), 3.05-2.94 (m, 2H), 2.35 (br d, 1H), 2.03 (q, 1H), 1.33 (t, 3H).

### Beispiel 93

### N-Ethyl-3-(4-ethylphenyl)-N-methyl-5-[3-(morpholin-4-ylmethyl)-1,2,4-oxadiazol-5-yl]piperidin-1-carbonsäureamid [racemisches cis-Isomer]

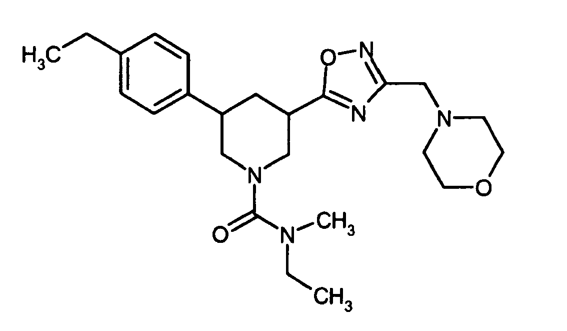

Nach der Allgemeinen Methode 1 wurden 63 mg (0.20 mmol) 1-[Ethyl(methyl)carbamoyl]-5-(4-ethylphenyl)piperidin-3-carbonsäure (Beispiel 45A) und 35 mg (0.22 mmol, 1.1 eq.) *N'*-Hydroxy-2-morpholin-4-ylethanimidamid umgesetzt. Ausbeute: 5 mg (5% d. Th.)

HPLC (Methode 2A): Rₜ = 4.24 min; MS (ESIpos): m/z = 442 [M+H]⁺;

¹H-NMR (400 MHz, DMSO-d₆): δ = 7.22 (d, 2H), 7.16 (d, 2H), 3.93 (br d, 1H), 3.64 (s, 2H), 3.60-3.50 (m, 5H), 3.47-3.37 (m, 1H), 3.06 (q, 2H), 2.97 (t, 1H), 2.91-2.84 (m, 2H), 2.77 (s, 3H), 2.57 (q, 2H), 2.30 (br d, 1H), 2.00-1.89 (m, 1H), 1.16 (t, 3H), 1.06 (t, 3H).

### Beispiel 94

### 4-({3-(4-Ethylphenyl)-5-[3-(5-fluorthiophen-2-yl)-1,2,4-oxadiazol-5-yl]piperidin-1-yl}carbonyl)-morpholin [racemisches cis-Isomer]

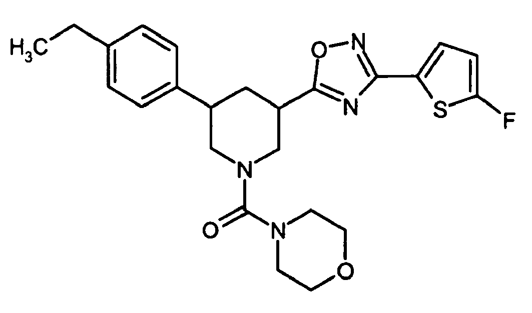

Nach der Allgemeinen Methode 1 wurden 69 mg (0.20 mmol) 5-(4-Ethylphenyl)-1-(morpholin-4-ylcarbonyl)piperidin-3-carbonsäure (Beispiel 38A) und 35 mg (0.22 mmol, 1.1 eq.) 5-Fluor-*N'-*hydroxythiophen-2-carboximidamid umgesetzt. Ausbeute: 51 mg (54% d. Th.)

HPLC (Methode 2A): Rₜ = 5.11 min; MS (ESIpos): m/z = 471 [M+H]⁺;

¹H-NMR (400 MHz, DMSO-d₆): δ = 7.56 (t, 1H), 7.23 (d, 2H), 7.17 (d, 2H), 6.95 (dd, 1H), 4.05 (br d, 1H), 3.61 (br d, 1H), 3.60-3.52 (m, 4H), 3.51-3.41 (m, 1H), 3.24-3.15 (m, 4H), 3.08 (t, 1H), 2.96 (q, 1H), 2.93-2.82 (m, 1H), 2.57 (q, 1H), 2.34 (br d, 1H), 2.01 (q, 1H), 1.17 (t, 3H).

### Beispiel 95

### N-(2-Methoxyethyl)-4-(5-{1-(morpholin-4-ylcarbonyl)-5-[4-(trifluormethoxy)phenyl]piperidin-3-yl}-1,2,4-oxadiazol-3-yl)pyridin-2-amin [racemisches cis-Isomer]

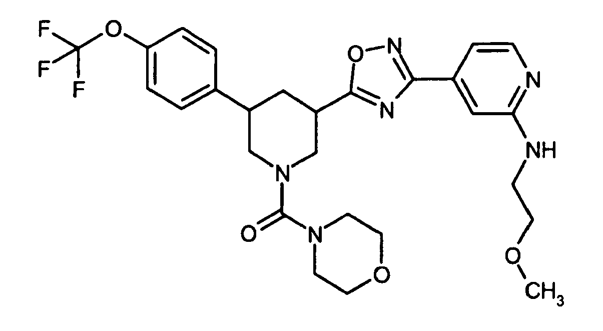

Nach der Allgemeinen Methode 3 wurden 69 mg (0.13 mmol) 4-({3-[3-(2-Chlorpyridin-4-yl)-1,2,4-oxadiazol-5-yl]-5-[4-(trifluormethoxy)phenyl]piperidin-1-yl}carbonyl)morpholin (Beispiel 88) in 1.2 ml 2-Methoxyethylamin umgesetzt. Ausbeute: 31 mg (43% d. Th.)

HPLC (Methode 2A): Rₜ = 4.28 min; MS (ESIpos): m/z = 577 [M+H]⁺;

¹H-NMR (400 MHz, DMSO-d₆): δ = 8.12 (d, 1H), 7.49 (d, 2H), 7.34 (d, 2H), 7.15 (s, 1H), 7.02 (t, 1H), 6.97 (dd, 1H), 4.08 (br d, 1H), 3.64 (br d, 1H), 3.61-3.54 (m, 4H), 3.54-3.46 (m, 1H), 3.46-3.43 (m, 4H), 3.27 (s, 3H), 3.25-3.18 (m, 4H), 3.11 (t, 1H), 3.07-2.97 (m, 2H), 2.39 (br d, 1H), 2.05 (q, 1H).

### Beispiel 96

### 4-[4-(5-{1-(Morpholin-4-ylcarbonyl)-5-[4-(trifluormethoxy)phenyl]piperidin-3-yl}-1,2,4-oxadiazol-3-yl)pyridin-2-yl]morpholin [racemisches cis-Isomer]

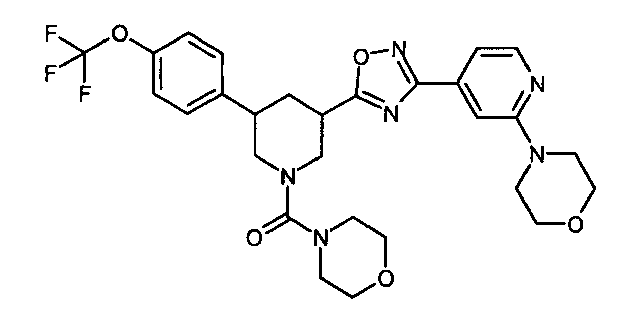

Nach der Allgemeinen Methode 3 wurden 69 mg (0.13 mmol) 4-({3-[3-(2-Chlorpyridin-4-yl)-1,2,4-oxadiazol-5-yl]-5-[4-(trifluormethoxy)phenyl]piperidin-1-yl}carbonyl)morpholin (Beispiel 88) in 0.6 ml Morpholin umgesetzt. Ausbeute: 38 mg (50% d. Th.)

HPLC (Methode 2A): Rₜ = 4.29 min; MS (ESIpos): m/z = 589 [M+H]⁺;

¹H-NMR (400 MHz, DMSO-d₆): δ = 8.31 (d, 1H), 7.49 (d, 2H), 7.34 (d, 2H), 7.28 (s, 1H), 7.20 (dd, 1H), 4.09 (br d, 1H), 3.76-3.68 (m, 4H), 3.63 (br d, 1H), 3.60-3.54 (m, 4H), 3.54-3.47 (m, 5H), 3.27-3.18 (m, 4H), 3.13 (t, 1H), 3.08-2.97 (m, 2H), 2.39 (br d, 1H), 2.13-2.02 (m, 1H).

### Beispiel 97

### N,N-Dimethyl-N'-[4-(5-{1-(morpholin-4-ylcarbonyl)-5-[4-(trifluormethoxy)phenyl]pipendin-3-yl}-1,2,4-oxadiazol-3-yl)pyridin-2-yl]ethan-1,2-diamin [racemisches cis-Isomer]

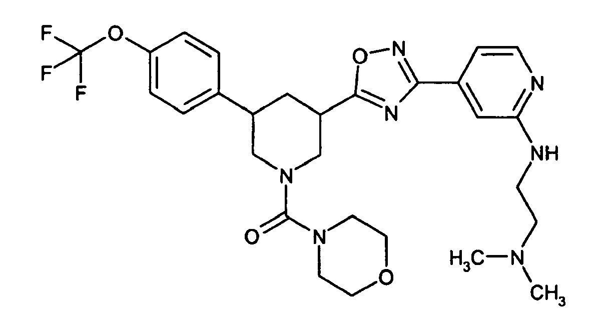

Nach der Allgemeinen Methode 3 wurden 69 mg (0.13 mmol) 4-({3-[3-(2-Chlorpyridin-4-yl)-1,2,4-oxadiazol-5-yl]-5-[4-(trifluormethoxy)phenyl]piperidin-1-yl}carbonyl)morpholin (Beispiel 88) in 0.6 ml *N*,*N*-Dimethylendiamin umgesetzt. Ausbeute: 8 mg (10% d. Th.)

HPLC (Methode 2A): Rₜ = 4.09 min; MS (ESIpos): m/z = 590 [M+H]⁺;

¹H-NMR (500 MHz, DMSO-d₆): δ = 8.15 (d, 1H), 7.51 (d, 2H), 7.37 (d, 2H), 7.16 (s, 1H), 6.99 (d, 1H), 6.88 (t, 1H), 4.10 (br d, 1H), 3.65 (br d, 1H), 3.64-3.57 (m, 4H), 3.56-3.48 (m, 1H), 3.39 (q, 2H), 3.28-3.20 (m, 4H), 3.14 (t, 1H), 3.08-2.98 (m, 2H), 2.48-2.38 (m, 3H), 2.20 (s, 6H), 2.07 (q, 1H).

### Beispiel 98

### 4-({3-[3-(2-Fluorphenyl)-1,2,4-oxadiazol-5-yl]-5-[4-(trifluormethoxy)phenyl]piperidin-1-yl}carbonyl)morpholin [racemisches cis-Isomer]

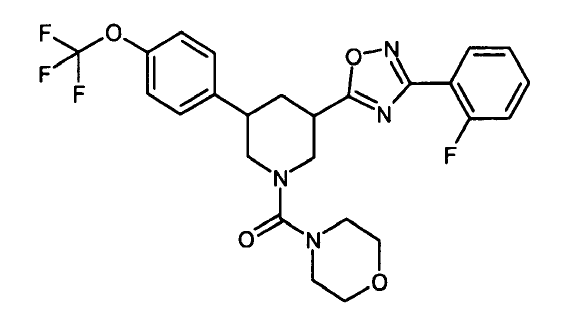

Nach der Allgemeinen Methode 1 wurden 80 mg (0.20 mmol) 1-(Morpholin-4-ylcarbonyl)-5-[4-(trifluormethoxy)phenyl]piperidin-3-carbonsäure (Beispiel 44A) und 42 mg (0.22 mmol, 1.1 eq.) 2-Fluor-*N'*-hydroxybenzencarboximidamid umgesetzt. Ausbeute: 59 mg (57% d. Th.)

HPLC (Methode 1A): Rₜ = 5.11 min; MS (ESIpos): m/z = 521 [M+H]⁺;

¹H-NMR (400 MHz, DMSO-d₆): δ = 8.03 (dt, 1H), 7.70-7.64 (m, 1H), 7.53-7.39 (m, 4H), 7.35 (d, 2H), 4.09 (br d, 1H), 3.64 (br d, 1H), 3.62-3.53 (m, 4H), 3.58-3.48 (m, 1H), 3.27-3.18 (m, 4H), 3.14 (t, 1H), 3.08-2.97 (m, 2H), 2.41 (br d, 1H), 2.07 (q, 1H).

### Beispiel 99

### 4-({3-[3-(5-Chlorthiophen-2-yl)-1,2,4-oxadiazol-5-yl]-5-[4-(trifluormethoxy)phenyl]piperidin-1-yl}carbonyl)morpholin [racemisches cis-Isomer]

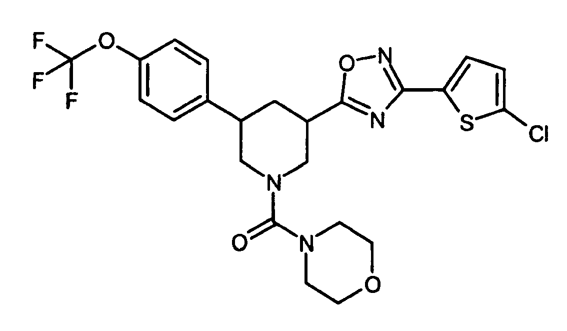

Nach der Allgemeinen Methode 1 wurden 80 mg (0.20 mmol) 1-(Morpholin-4-ylcarbonyl)-5-[4-(trifluormethoxy)phenyl]piperidin-3-carbonsäure (Beispiel 44A) und 39 mg (0.22 mmol, 1.1 eq.) 5-Chlor-*N'*-hydroxythiophen-2-carboximidamid umgesetzt. Ausbeute: 57 mg (53% d. Th.)

HPLC (Methode 1A): Rₜ = 5.47 min; MS (ESIpos): m/z = 543 [M+H]⁺;

¹H-NMR (400 MHz, DMSO-d₆): δ = 7.69 (d, 1H), 7.49 (d, 2H), 7.37-7.31 (m, 3H), 4.05 (br d, 1H), 3.62 (br d, 1H), 3.60-3.53 (m, 4H), 3.52-3.43 (m, 1H), 3.26-3.18 (m, 4H), 3.11 (t, 1H), 3.06-2.95 (m, 2H), 2.37 (br d, 1H), 2.04 (q, 1H).

### Beispiel 100

### 4-({3-[3-(5-Chlorthiophen-2-yl)-1,2,4-oxadiazol-5-yl]-5-(4-ethylphenyl)piperidin-1-yl}carbonyl)morpholin [racemisches cis-Isomer]

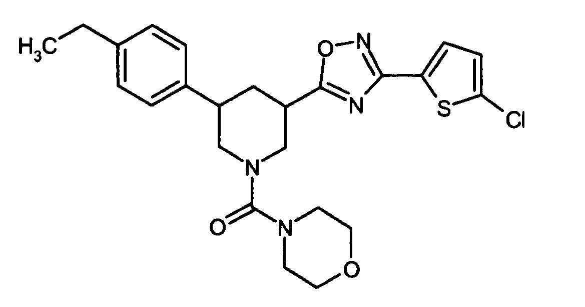

Nach der Allgemeinen Methode 1 wurden 69 mg (0.20 mmol) 5-(4-Ethylphenyl)-1-(morpholin-4-ylcarbonyl)piperidin-3-carbonsäure (Beispiel 38A) und 39 mg (0.22 mmol, 1.1 eq.) 5-Chlor-*N'-*hydroxythiophen-2-carboximidamid umgesetzt. Ausbeute: 60 mg (62% d. Th.)

HPLC (Methode 2A):Rₜ = 5.51 min; MS (ESIpos): m/z = 487 [M+H]⁺;

¹H-NMR (400 MHz, DMSO-d₆): δ = 7.70 (d, 1H), 7.32 (d, 1H), 7.24 (d, 2H), 7.18 (d, 2H), 4.06 (br d, 1H), 3.61 (br d, 1H), 3.60-3.53 (m, 4H), 3.52-3.42 (m, 1H), 3.23-3.16 (m, 4H), 3.09 (t, 1H), 2.98 (t, 1H), 2.93-2.83 (m, 1H), 2.58 (q, 2H), 2.34 (br d, 1H), 2.01 (q, 1H), 1.17 (t, 3H).

### Beispiel 101

### 4-({3-[3-(5-Fluorthiophen-2-yl)-1,2,4-oxadiazol-5-yl]-5-[4-(trifluormethoxy)phenyl]piperidin-1-yl}carbonyl)morpholin [racemisches cis-Isomer]

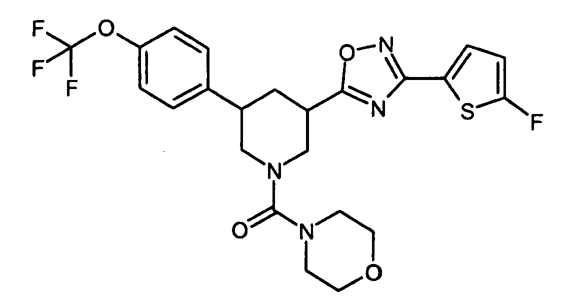

Nach der Allgemeinen Methode 1 wurden 80 mg (0.20 mmol) 1-(Morpholin-4-ylcarbonyl)-5-[4-(trifluormethoxy)phenyl]piperidin-3-carbonsäure (Beispiel 44A) und 35 mg (0.22 mmol, 1.1 eq.) 5-Fluor-*N'*-hydroxythiophen-2-carboximidamid umgesetzt. Ausbeute: 61 mg (58% d. Th.)

HPLC (Methode 1A): Rₜ = 5.27 min; MS (ESIpos): m/z = 527 [M+H]⁺;

¹H-NMR (400 MHz, DMSO-d₆): δ = 7.56 (dd, 1H), 7.49 (d, 2H), 7.34 (d, 2H), 6.95 (dd, 1H), 4.05 (br d, 1H), 3.62 (br d, 1H), 3.61-3.53 (m, 4H), 3.52-3.43 (m, 1H), 3.24-3.16 (m, 4H), 3.10 (t, 1H), 3.06-2.94 (m, 2H), 2.37 (br d, 1H), 2.03 (q, 1H).

### Beispiel 102

### 4-({3-(4-Ethylphenyl)-5-[3-(2-fluorphenyl)-1,2,4-oxadiazol-5-yl]piperidin-1-yl}carbonyl)-morpholin [racemisches cis-Isomer]

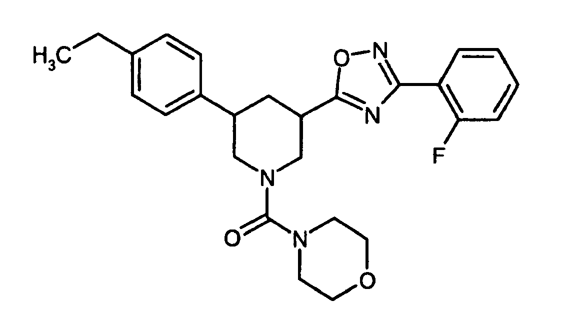

Nach der Allgemeinen Methode 1 wurden 69 mg (0.20 mmol) 5-(4-Ethylphenyl)-1-(morpholin-4-ylcarbonyl)piperidin-3-carbonsäure (Beispiel 38A) und 34 mg (0.22 mmol, 1.1 eq.) 2-Fluor-*N'-*hydroxybenzencarboximidamid umgesetzt. Ausbeute: 39 mg (42% d. Th.)

HPLC (Methode 1A): Rₜ = 5.14 min; MS (ESIpos): m/z = 465 [M+H]⁺;

¹H-NMR (400 MHz, DMSO-d₆): δ = 8.03 (dt, 1H), 7.70-7.63 (m, 1H), 7.49-7.39 (m, 2H), 7.25 (d, 2H), 7.18 (d, 2H), 4.09 (br d, 1H), 3.62 (br d, 1H), 3.59-3.54 (m, 4H), 3.56-3.46 (m, 1H), 3.24-3.18 (m, 4H), 3.11 (t, 1H), 2.99 (t, 1H), 2.96-2.85 (m, 1H), 2.58 (q, 2H), 2.38 (br d, 1H), 2.04 (q, 1H), 1.17 (t, 3H).

### Beispiel 103

### 4-({3-[3-(4-Fluorphenyl)-1,2,4-oxadiazol-5-yl]-5-[4-(tritluormethoxy)phenyl]piperidin-1-yl}carbonyl)morpholin [enantiomerenreines cis-Isomer]

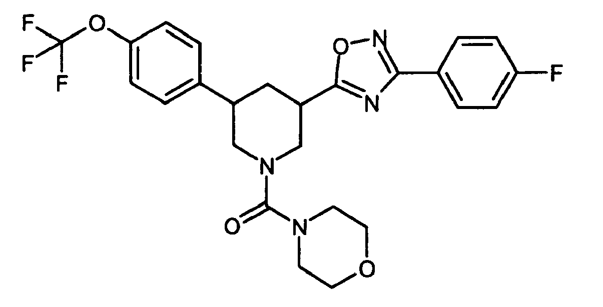

Enantiomerentrennung von 44 mg des Racemates aus Beispiel 34 nach Methode 6D ergab 18 mg der Titelverbindung aus Beispiel 103 (Enantiomer 1) und 19 mg der Titelverbindung aus Beispiel 104 (Enantiomer 2).

HPLC (Methode 4E): Rₜ = 10.16 min; MS (ESIpos): m/z = 521 [M+H]⁺.

### Beispiel 104

### 4-({3-[3-(4-Fluorphenyl)-1,2,4-oxadiazol-5-yl]-5-[4-(trifluormethoxy)phenyl]piperidin-1-yl}carbonyl)morpholin [enantiomerenreines cis-Isomer]

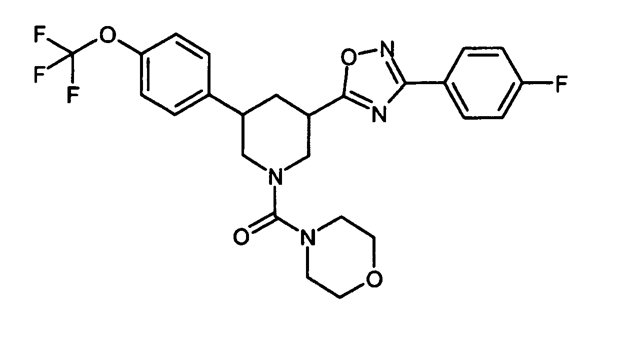

Enantiomerentrennung von 44 mg des Racemates aus Beispiel 34 nach Methode 6D ergab 18 mg der Titelverbindung aus Beispiel 103 (Enantiomer 1) und 19 mg der Titelverbindung aus Beispiel 104 (Enantiomer 2).

HPLC (Methode 4E): Rₜ = 12.64 min; MS (ESIpos): m/z = 521 [M+H]⁺.

### Beispiel 105

### 4-({3-(3-tert.-Butyl-1,2,4-oxadiazol-5-yl)-5-[4-(trifluormethoxy)phenyl)piperidin-1-yl} carbonyl)-morpholin [enantiomerenreines cis-Isomer]

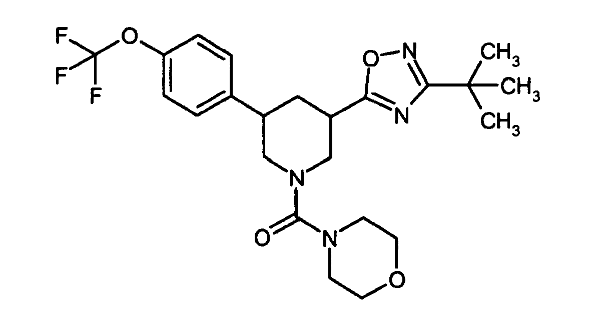

Enantiomerentrennung von 57 mg des Racemates aus Beispiel 74 nach Methode 7D ergab 23 mg der Titelverbindung aus Beispiel 105 (Enantiomer 1) und 26 mg der Titelverbindung aus Beispiel 106 (Enantiomer 2).

HPLC (Methode 3E): Rₜ = 4.68 min; MS (ESIpos): m/z = 483 [M+H]⁺.

### Beispiel 106

### 4-({3-(3-tert.-Butyl-1,2,4-oxadiazol-5-yl)-5-[4-(trifluormethoxy)phenyl]piperidin-1-yl}carbonyl)-morpholin [enantiomerenreines cis-Isomer]

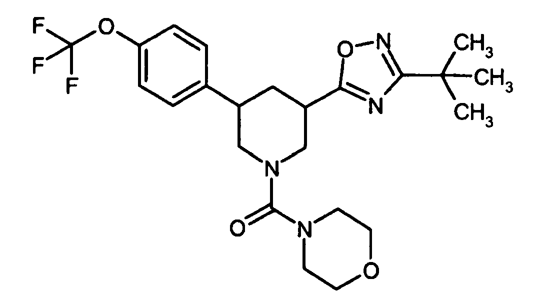

Enantiomerentrennung von 57 mg des Racemates aus Beispiel 74 nach Methode 7D ergab 23 mg der Titelverbindung aus Beispiel 105 (Enantiomer 1) und 26 mg der Titelverbindung aus Beispiel 106 (Enantiomer 2).

HPLC (Methode 3E): Rₜ = 5.34 min; MS (ESIpos): m/z = 483 [M+H]⁺.

### Beispiel 107

### 4-({3-[3-(2,4-Difluorphenyl)-1,2,4-oxadiazol-5-yl]-5-[4-(trifluormethoxy)phenyl]piperidin-1-yl]carbonyl)morpholin [enantiomerenreines cis-Isomer]

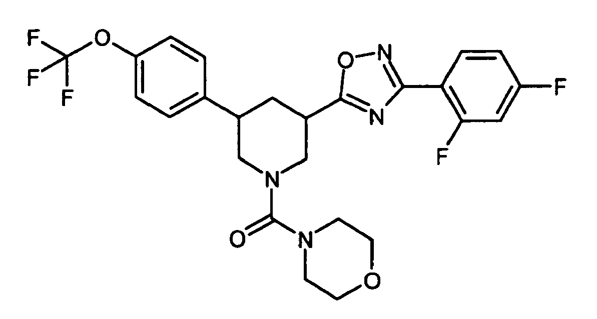

Enantiomerentrennung von 40 mg des Racemates aus Beispiel 38 nach Methode 4D ergab 14 mg der Titelverbindung aus Beispiel 107 (Enantiomer 1) und 14 mg der Titelverbindung aus Beispiel 108 (Enantiomer 2).

HPLC (Methode 1E): Rₜ = 6.89 min; MS (ESIpos): m/z = 539 [M+H]⁺.

### Beispiel 108

### 4-({3-[3-(2,4-Difluorphenyl)-1,2,4-oxadiazol-5-yl]-5-[4-(trifluormethoxy)phenyl]piperidin-1-yl}carbonyl)morpholin [enantiomerenreines cis-Isomer]

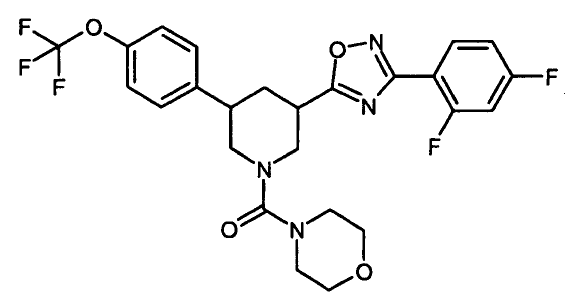

Enantiomerentrennung von 40 mg des Racemates aus Beispiel 38 nach Methode 4D ergab 14 mg der Titelverbindung aus Beispiel 107 (Enantiomer 1) und 14 mg der Titelverbindung aus Beispiel 108 (Enantiomer 2).

HPLC (Methode 1E): Rₜ = 8.40 min; MS (ESIpos): m/z = 539 [M+H]⁺.

### Beispiel 109

### 3-(4-Ethylphenyl)-5-(3-phenyl-1,2,4-oxadiazol-5-yl)-1-(pyrrolidin-1-ylcarbonyl)piperidin [racemisches cis-Isomer]

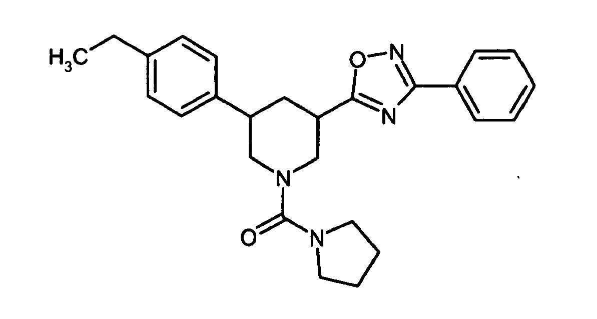

Nach der Allgemeinen Methode 1 wurden 73 mg (0.22 mmol) 5-(4-Ethylphenyl)-1-(pyrrolidin-1-ylcarbonyl)piperidin-3-carbonsäure (Beispiel 41 A) und 33 mg (0.24 mmol, 1.1 eq.) *N'*-Hydroxybenzencarboximidamid umgesetzt. Ausbeute: 32 mg (33% d. Th.)

HPLC (Methode 2A): Rₜ = 5.39 min; MS (ESIpos): m/z = 431 [M+H]⁺;

¹H-NMR (400 MHz, DMSO-d₆): δ = 8.05-8.00 (m, 2H), 7.63-7.54 (m, 3H), 7.25 (d, 2H), 7.18 (d, 2H), 4.16 (br d, 1H), 3.72 (br d, 1H), 3.54-3.44 (m, 1H), 3.06 (t, 1H), 2.97-2.85 (m, 2H), 2.58 (q, 2H), 2.39 (br d, 1H), 2.05 (q, 1H), 1.82-1.71 (m, 4H), 1.17 (t, 3H).

### Beispiel 110

### 3-(4-Ethylphenyl)-5-[3-(2-fluorphenyl)-1,2,4-oxadiazol-5-yl]-1-(pyrrolidin-1-ylcarbonyl)piperidin [racemisches cis-Isomer]

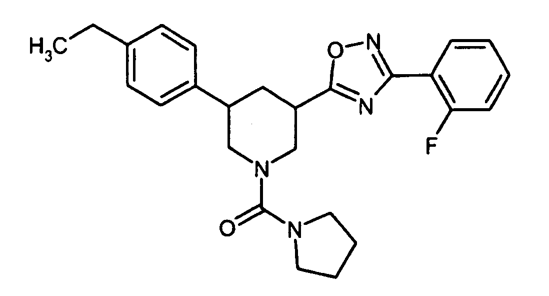

Nach der Allgemeinen Methode 1 wurden 73 mg (0.22 mmol) 5-(4-Ethylphenyl)-1-(pyrrolidin-1-ylcarbonyl)piperidin-3-carbonsäure (Beispiel 41A) und 47 mg (0.24 mmol, 1.1 eq.) 2-Fluor-*N'-*hydroxybenzencarboximidamid umgesetzt. Ausbeute: 46 mg (47% d. Th.)

HPLC (Methode 2A): Rₜ = 5.29 min; MS (ESIpos): m/z = 449 [M+H]⁺;

¹H-NMR (400 MHz, DMSO-d₆): δ = 8.04 (dt, 1H), 7.70-7.63 (m, 1H), 7.49-7.38 (m, 2H), 7.25 (d, 2H), 7.18 (d, 2H), 4.17 (br d, 1H), 3.72 (br d, 1H), 3.55-3.45 (m, 1H), 3.06 (t, 1H), 2.97-2.85 (m, 2H), 2.58 (q, 2H), 2.39 (br d, 1H), 2.11-1.99 (m, 1H), 1.81-1.72 (m, 4H), 1.17 (t, 3H).

### Beispiel 111

### 3-(3-tert.-Butyl-1,2,4-oxadiazol-5-yl)-5-(4-ethylphenyl)-1-(pyrrolidin-1-ylcarbonyl)piperidin [racemisches cis-Isomer]

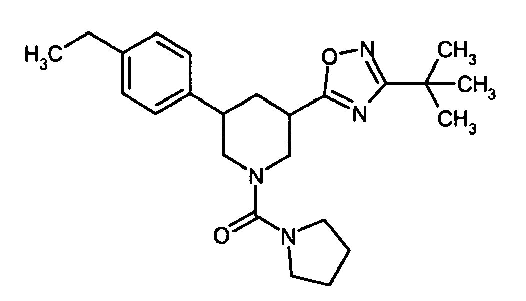

Nach der Allgemeinen Methode 1 wurden 73 mg (0.22 mmol) 5-(4-Ethylphenyl)-1-(pyrrolidin-1-ylcarbonyl)piperidin-3-carbonsäure (Beispiel 41A) und 35 mg (0.24 mmol, 1.1 eq.) *N'*-Hydroxy-2,2-dimethylpropanimidamid umgesetzt. Ausbeute: 71 mg (79% d. Th.)

HPLC (Methode 2A): Rₜ = 5.32 min; MS (ESIpos): m/z = 411 [M+H]⁺;

¹H-NMR (400 MHz, DMSO-d₆): δ = 7.23 (d, 2H), 7.16 (d, 2H), 4.05 (br d, 1H), 3.69 (br d, 1H), 3.41-3.30 (m, 1H), 3.30-3.25 (m, 4H), 2.96 (t, 1H), 2.91 (t, 1H), 2.89-2.80 (m, 1H), 2.57 (d, 2H), 2.29 (br d, 1H), 1.94 (q, 1H), 1.80-1.70 (m, 4H), 1.30 (s, 9H), 1.16 (t, 3H).

### Beispiel 112

### 4-({3-(3-tert.-Butyl-1,2,4-oxadiazol-5-yl)-5-[4-(trifluormethyl)phenyl]piperidin-1-yl}carbonyl)-morpholin [racemisches cis-Isomer]

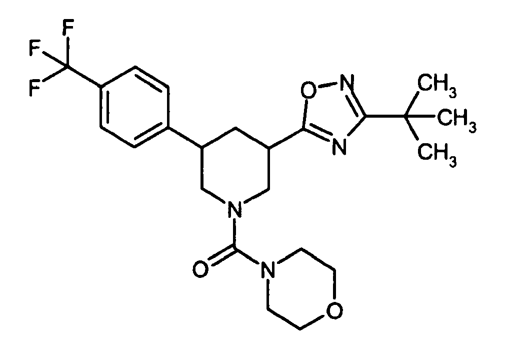

Nach der Allgemeinen Methode 1 wurden 250 mg (0.65 mmol) 1-(Morpholin-4-ylearbonyl)-5-[4-(trifluormethyl)phenyl]piperidin-3-carbonsäure (Beispiel 49A) und 83 mg (0.71 mmol) *N'-*Hydroxy-2,2-dimethylpropanimidamid umgesetzt. Ausbeute: 70 mg (70% d. Th.)

LC-MS (Methode 5B): Rₜ = 2.51 min; MS (ESIpos): m/z = 467 [M+H]⁺;

¹H-NMR (400 MHz, DMSO-d₆): δ = 7.70 (d, 2H), 7.58 (d, 2H), 3.99 (br d, 1H), 3.62 (d, 1H), 3.61-3.55 (m, 4H), 3.40 (tt, 1H), 3.22-3.19 (m, 4H), 3.09-3.02 (3H), 2.33 (br d, 1H), 2.01 (q, 1H), 1.30 (s, 9H).

### Beispiel 113

### 4-({3-[4-(4-Methylpyridin-3-yl)-1,3-thiazol-2-yl]-5-[4-(trifluormethyl)phenyl]piperidin-1-yl}-carbonyl)morpholin [racemisches cis-Isomer]

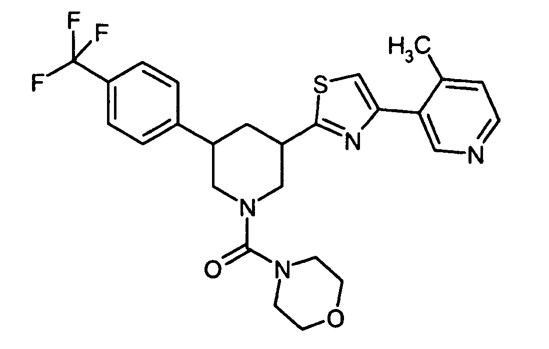

Nach der Allgemeinen Methode 3 wurden 100 mg (0.224 mmol) 1-(Morpholin-4-ylcarbonyl)-5-[4-(trifluormethyl)phenyl]piperidin-3-carbothioamid (Beispiel 53A) und 67 mg (0.269 mmol) 2-Brom-1-(4-methylpyridin-3-yl)ethanon-Hydrochlorid umgesetzt. Ausbeute: 13 mg (12% d. Th.).

LC-MS (Methode 2B): Rₜ = 1.15 min; MS (ESIpos): m/z = 517 [M+H]⁺;

¹H-NMR (400 MHz, DMSO-d₆): δ = 8.75 (s, 1H), 8.42 (d, 1H), 7.89 (s, 1H), 7.72 (br d, 2H), 7.60 (br d, 2H), 7.33 (d, 1H), 4.07 (br d, 1H), 3.68 (br d, 1H), 3.58-3.56 (m, 4H), 3.46 (tt, 1H), 3.22-3.19 (m, 4H), 3.13-3.01 (m, 3H), 2.46 (s, 3H), 2.38 (br d, 1H), 2.06 (q, 1H).

### Beispiel 114

### 4-({3-(4-tert-Butyl-1,3-thiazol-2-yl)-5-[4-(trifluormethyl)phenyl]piperidin-1-yl}carbonyl)-morpholin [racemisches cis-Isomer]

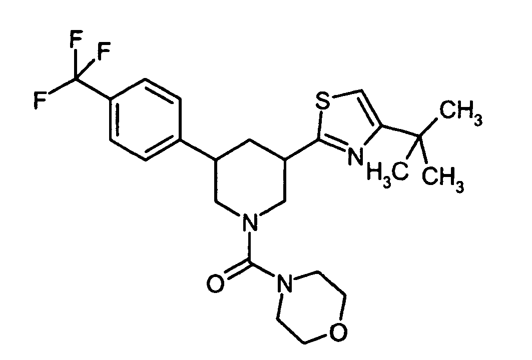

Nach der Allgemeinen Methode 3 wurden 100 mg (0.224 mmol) 1-(Morpholin-4-ylcarbonyl)-5-[4-(trifluormethyl)phenyl]piperidin-3-carbothioamid (Beispiel 53A) und 48 mg (0.269 mmol) 1-Brom-3,3-dimethylbutan-2-on umgesetzt. Ausbeute: 19 mg (17% d. Th.)

LC-MS (Methode 2B): Rₜ = 1.52 min; MS (ESIpos): m/z = 482 [M+H]⁺;

¹H-NMR (400 MHz, DMSO-d₆): δ = 7.70 (d, 2H), 7.59 (d, 2H), 7.16 (s, 1H), 4.00 (br d, 1H), 3.65 (br d, 1H), 3.58-3.55 (m, 4H), 3.20-3.18 (m, 4H), 3.05 (d, 2H), 2.97 (t, 1H), 2.31 (br d, 1H), 1.95 (q, 1H), 1.28 (s, 9H). 1H wurde nicht zugewiesen

### Beispiel 115

### 4-({3-[4-(6-Methylpyridin-3-yl)-1,3-thiazol-2-yl]-5-[4-(trifluormethyl)phenyl]piperidin-1-yl}-carbonyl)morpholin [racemisches cis-Isomer]

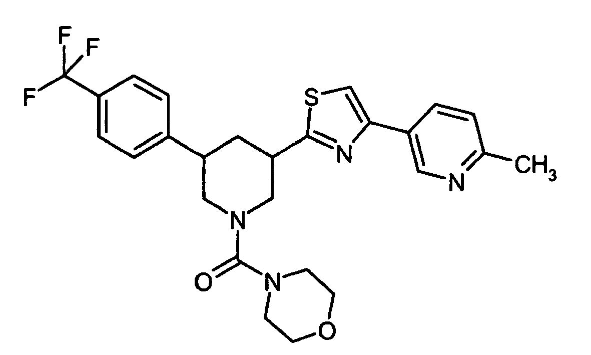

Nach der Allgemeinen Methode 3 wurden 100 mg (0.224 mmol) 1-(Morpholin-4-ylcarbonyl)-5-[4-(trifluormethyl)phenyl]piperidin-3-carbothioamid (Beispiel 53A) und 58 mg (0.269 mmol) 2-Brom-1-(6-methylpyridin-3-yl)ethanon umgesetzt. Ausbeute: 18 mg (15% d. Th.)

LC-MS (Methode 2B): Rₜ = 1.31 min; MS (ESIpos): m/z = 517 [M+H]⁺;

¹H-NMR (400 MHz, DMSO-d₆): δ = 8.75 (s, 1H), 8.43 (d, 1H), 7.89 (s, 1H), 7.71 (br d, 2H), 7.60 (br d, 2H), 7.33 (d, 1H), 4.07 (br d, 1H), 3.68 (br d, 1H), 3.58-3.56 (m, 4H), 3.48 (tt, 1H), 3.22-3.19 (m, 4H), 3.11-3-01 (m, 3H), 2.46 (s, 3H), 2.39 (br d, 1H), 2.07 (q, 1H).

### Beispiel 116

### 4-({3-(4-Pyridin-2-yl-1,3-thiazol-2-yl)-5-[4-(trifluormethyl)phenyl]piperidin-1-yl} carbonyl)-morpholin [racemisches cis-Isomer]

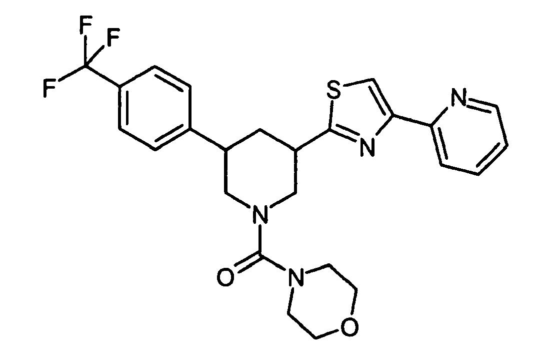

Nach der Allgemeinen Methode 3 wurden 100 mg (0.224 mmol) 1-(Morpholin-4-ylcarbonyl)-5-[4-(trifluormethyl)phenyl]piperidin-3-carbothioamid (Beispiel 53A) und 76 mg (0.269 mmol) 2-Bromo-1-pyridin-2-ylethanon umgesetzt. Ausbeute: 8 mg (7% d. Th.)

LC-MS (Methode 2B): Rₜ = 1.30 min; MS (ESIpos): m/z = 503 [M+H]⁺;

¹H-NMR (400 MHz, DMSO-d₆): δ = 8.61 (br d, 2H), 8.21 (s, 1H), 8.07 (d, 1H), 7.90 (td, 1H), 7.71 (br d, 2H), 7.61 (br d, 2H), 7.35 (dd, 1H), 4.08 (br d, 1H), 3.69 (d, 1H), 3.59-3.57 (m, 4H), 3.47 (t, 1H), 3.23-3.21 (m, 4H), 3.12-3.04 (m, 3H), 2.42 (br d, 1H), 2.07 (q, 1H).

### Beispiel 117

### 4-({3-[4-(2,6-Dimethylpyridin-3-yl)-1,3-thiazol-2-yl]-5-[4-(trifluormethyl)phenyl]piperidin-1-yl}-carbonyl)morpholin [racemisches cis-Isomer]

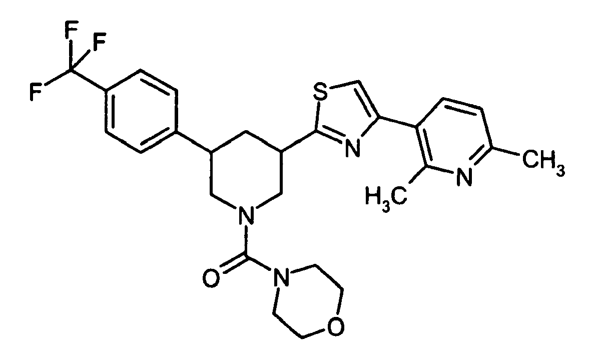

Nach der Allgemeinen Methode 3 wurden 100 mg (0.224 mmol) 1-(Morpholin-4-ylcarbonyl)-5-[4-(trifluormethyl)phenyl]piperidin-3-carbothioamid (Beispiel 53A) und 83 mg (0.269 mmol) 2-Brom-1-(2,6-dimethylpyridin-3-yl)ethanon umgesetzt. Ausbeute: 13 mg (10% d. Th.)

LC-MS (Methode 2B): Rₜ = 1.09 min; MS (ESIpos): m/z = 531 [M+H]⁺;

¹H-NMR (400 MHz, DMSO-d₆): δ = 7.95 (bs, 1H), 7.81 (s, 1H), 7.71 (d, 2H), 7.60 (d, 2H), 7.22 (bs, 1H), 4.06 (br d, 1H), 3.68 (r d, 1H), 3.58-3.56 (m, 4H), 3.45 (tt, 1H), 3.22-3.19 (m, 4H), 3.10-3.01 (m, 3H), 2.62 (s, 3H), 2.38 (br d, 1H), 2.06 (q, 1H). 3H's wurden nicht zugewiesen.

### Beispiel 118

### 4-({3-[4-(6-Chlorpyridin-3-yl)-1,3-thiazol-2-yl]-5-[4-(trifluormethyl)phenyl]piperidin-1-yl}-carbonyl)morpholin [racemisches cis-Isomer]

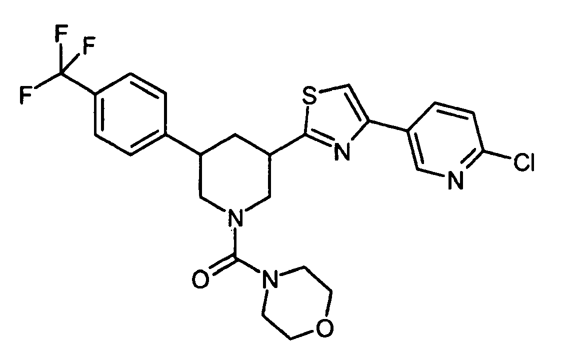

Nach der Allgemeinen Methode 3 wurden 100 mg (0.224 mmol) 1-(Morpholin-4-ylcarbonyl)-5-[4-(trifluormethyl)phenyl]piperidin-3-carbothioamid (Beispiel 53A) und 85 mg (0.269 mmol) 2-Brom-1-(6-chlorpyridin-3-yl)ethanon umgesetzt. Ausbeute: 17 mg (14% d. Th.)

LC-MS (Methode 2B): Rₜ = 1.40 min; MS (ESIpos): m/z = 537 [M+H]⁺;

¹H-NMR (400 MHz, DMSO-d₆): δ 9.09 (d, 1H), 8.36 (dd, 1H), 8.29 (s, 1H), 7.71 (br d, 2H), 7.61 (br d, 3H), 4.06 (br d, 1H), 3.68 (br d, 1H), 3.61-3.57 (m, 4H), 3.47 (t, 1H), 3.24-3.21 (m, 4H), 3.11-3.03 (m, 3H), 2.38 (br d, 1H), 2.06 (q, 1H).

### Beispiel 119

### 4-({3-(4-Pyridin-3-yl-1,3-thiazol-2-yl)-5-[4-(trifluormethyl)phenyl]piperidin-1-yl}carbonyl)-morpholin [racemisches cis-Isomer]

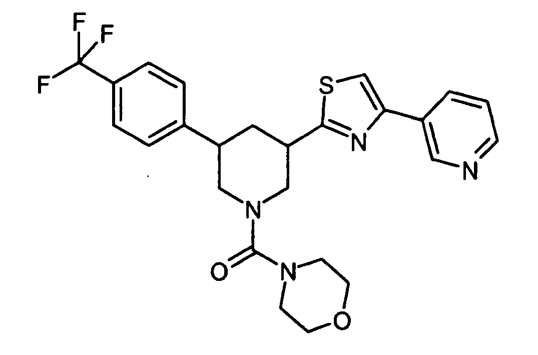

Nach der Allgemeinen Methode 3 wurden 100 mg (0.224 mmol) 1-(Morpholin-4-ylcarbonyl)-5-[4-(trifluormethyl)phenyl]piperidin-3-carbothioamid (Beispiel 53A) und 54 mg (0.269 mmol) 2-Brom-1-pyridin-3-ylethanon umgesetzt. Ausbeute: 24 mg (21 % d. Th.)

LC-MS (Methode 5B): Rₜ = 2.10 min; MS (ESIpos): m/z = 503 [M+H]⁺;

¹H-NMR (400 MHz, DMSO-d₆): δ = 9.17 (d, 1H), 8.54 (dd, 1H), 8.30 (dt, 1H), 8.23 (s, 1H), 7.71 (br d, 2H), 7.61 (br d, 2H), 7.46 (dd, 1H), 4.07 (br d, 1H), 3.68 (br d, 1H), 3.59-3.57 (m, 4H), 3.48 (tt, 1H), 3.23-3.20 (m, 4H), 3.12-3.03 (m, 3H), 2.40 (br d, 1H), 2.07 (q, 1H).

### Beispiel 120

### 4-({(3-[4-(2,4-Dichlorphenyl)-1,3-thiazol-2-yl]-5-[4-(trifluormethyl)phenyl]piperidin-1-yl}-carbonyl)morpholin [racemisches cis-Isomer]

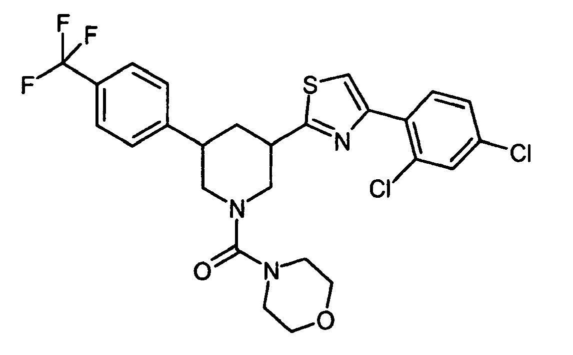

Nach der Allgemeinen Methode 3 wurden 100 mg (0.224 mmol) 1-(Morpholin-4-ylcarbonyl)-5-[4-(trifluormethyl)phenyl]piperidin-3-carbothioamid (Beispiel 53A) und 72 mg (0.269 mmol) 2-Brom-1-(2,4-dichlorphenyl)ethanon umgesetzt. Ausbeute: 43 mg (34% d. Th.)

LC-MS (Methode 58): Rₜ = 3.04 min; MS (ESIpos): m/z = 570 [M+H]⁺;

¹H-NMR (400 MHz, DMSO-d₆): δ = 8.09 (s, 1H), 7.94 (d, 1H), 7.74 (d, 1H), 7.72 (br d, 2H), 7.60 (br d, 2H), 6.53 (dd, 1H), 4.05 (br d, 1H), 3.68 (d, 1H), 3.58-3.56 (m, 4H), 3.46 (tt, 1H), 3.21-3.19 (m, 4H), 3.11-3.02 (m, 3H), 2.38 (br d, 1H), 2.06 (q, 1H).

### Beispiels 121

### 4-({(3-[4-(4-Fluorphenyl)-1,3-thiazol-2-yl]-5-[4-(trifluormethyl)phenyl]piperidin-1-yl}carbonyl)-morpholin [racemisches cis-Isomer]

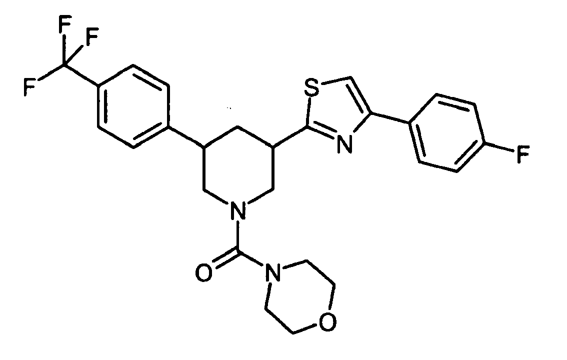

Nach der Allgemeinen Methode 3 wurden 100 mg (0.152 mmol) 1-(Morpholin-4-ylcarbonyl)-5-[4-(trifluormethyl)phenyl]piperidin-3-carbothioamid (Beispiel 53A) und 32 mg (0.182 mmol) 2-Chlor-1-(4-fluorphenyl)ethanon umgesetzt. Ausbeute: 30 mg (39% d. Th.)

LC-MS (Methode 5B): Rₜ = 2.76 min; MS (ESIpos): m/z = 520 [M+H]⁺;

¹H-NMR (400 MHz, DMSO-d₆): δ = 8.03-7.99 (3H), 7.71 (d, 2H), 7.61 (d, 2H), 7.28 (t, 2H), 4.06 (br d, 1H), 3.68 (d, 1H), 3.60-3.57 (m, 4H), 3.44 (tt, 1H), 3.23-3.20 (m, 4H), 3.10-3.02 (m, 3H), 2.38 (br d, 1H), 2.06 (q, 1H).

### Beispiel 122

### 4-({(3-[4-(4-Chlorphenyl)-1,3-thiazol-2-yl]-5-[4-(trifluormethyl)phenyl]piperidin-1-yl}carbonyl)-morpholin [racemisches cis-Isomer]

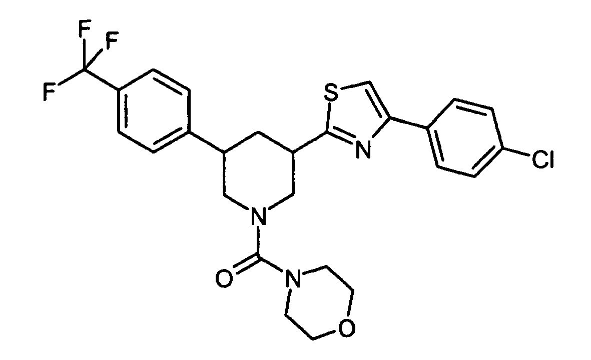

Nach der Allgemeinen Methode 3 wurden 100 mg (0224 mmol) 1-(Morpholin-4-ylcarbonyl)-5-[4-(trifluormethyl)phenyl]piperidin-3-carbothioamid (Beispiel 53A) und 63 mg (0.269 mmol) 2-Brom-1-(4-chlorphenyl)ethanon umgesetzt. Ausbeute: 15 mg (12% d. Th.)

LC-MS (Methode 5B): Rₜ = 2.89 min; MS (ESIpos): m/z = 536 [M+H]⁺;

¹H-NMR (400 MHz, DMSO-d₆): δ = 8.11 (s, 1H), 8.08 (d, 2H), 7.72 (br d, 2H), 7.61 (br d, 2H), 7.50 (d, 2H), 4.06 (br d, 1H), 3.68 (d, 1H), 3.60-3.57 (m, 4H), 3.45 (tt, 1H), 3.23-3.20 (m, 4H), 3.11-3.03 (m, 3H), 2.35 (br d, 1H), 2.06 (q, 1H).

### Beispiel 123

### 4-({3-(4-Phenyl-1,3-thiawl-2-yl)-5-[4-(trifluormethyl)phenyl]piperidin-1-yl}carbonyl)morpholin [racemisches cis-Isomer]

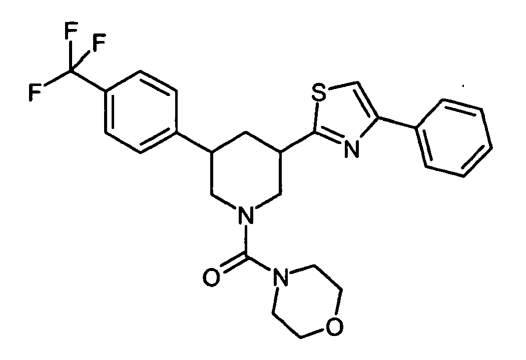

Nach der Allgemeinen Methode 3 wurden 42 mg (0.064 mmol) 1-(Morpholin-4-ylcarbonyl)-5-[4-(trifluormethyl)phenyl]piperidin-3-carbothioamid (Beispiel 53A) und 15 mg (0.077 mmol) 2-Brom-1-phenylethanon umgesetzt. Ausbeute: 11 mg (34% d. Th.)

LC-MS (Methode 5B): Rₜ = 2.74 min; MS (ESIpos): m/z = 502 [M+H]⁺;

¹H-NMR (400 MHz, DMSO-d₆): δ = 8.04 (s, 1H), 7.96 (d, 2H), 7.71 (d, 2H), 7.61 (d, 2H), 7.44 (t, 2H), 7.34 (t, 1H), 4.07 (br d, 1H), 3.68 (d, 1H), 3.60-3.27 (m, 4H), 3.45 (tt, 1H), 3.23-3.20 (m, 4H), 3.11-3.03 (m, 3H), 2.39 (br d, 1H), 2.06 (q, 1H).

### Beispiel 124

### 4-({3-[4-(2,5-Dimethoxyphenyl)-1,3-thiazol-2-yl]-5-(4-(trifluormethyl)phenyl]piperidin-1-yl}-carbonyl)morpholin [racemisches cis-Isomer]

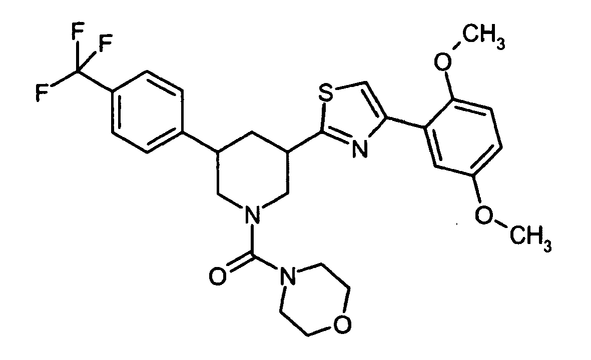

Nach der Allgemeinen Methode 3 wurden 100 mg (0.152 mmol) 1-(Morpholin-4-ylcarbonyl)-5-[4-(trifluormethyl)phenyl]piperidin-3-carbothioamid (Beispiel 53A) und 49 mg (0.182 mmol) 2-Brom-1-(2,5-dimethoxyphenyl)ethanon umgesetzt. Ausbeute: 20 mg (24% d. Th.)

LC-MS (Methode 5B): Rₜ = 2.76 min; MS (ESIpos): m/z = 562[M+H]⁺;

¹H-NMR (400 MHz, DMSO-d₆): δ = 8.03 (s, 1H), 7.72 (d, 1H), 7.71 (br d, 2H), 7.61 (br d, 2H), 7.07 (d, 1H), 6.91 (dd, 1H), 4.07 (br d, 1H), 3.86 (s, 3H), 3.76 (s, 3H), 3.68 (br d, 1H), 3.60-3.57 (m, 4H), 3.45 (tt, 1H), 3.22-3.20 (m, 4H), 3.13-3.02 (m, 3H), 2.38 (br d, 1H), 2.06 (q, 1H).

### Beispiel 125

### 4-({3-[4-(3-Methoxyphenyl)-1,3-thiazol-2-yl]-5-[4-(trifluormethyl)phenyl]piperidin-1-yl}-carbonyl)morpholin [racemisches cis-Isomer]

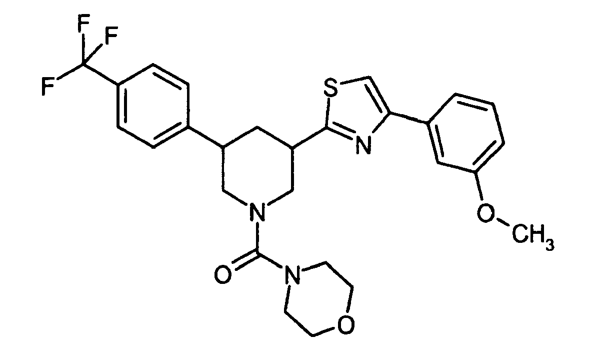

Nach der Allgemeinen Methode 3 wurden 100 mg (0.152 mmol) 1-(Morpholin-4-ylcarbonyl)-5-[4-(trifluormethyl)phenyl]piperidin-3-carbothioamid (Beispiel 53A) und 42 mg (0.182 mmol) 2-Brom-1-(3-methoxyphenyl)ethanon umgesetzt. Ausbeute: 29 mg (35% d. Th.)

LC-MS (Methode 5B): Rₜ = 2.72 min; MS (ESIpos): m/z = 532 [M+H]⁺;

¹H-NMR (400 MHz, DMSO-d₆): δ = 8.07 (s, 1H), 7.71 (br d, 2H), 7.61 (br d, 2H), 7.54 (d, 1H), 7.50 (bs, 1H), 7.35 (t, 1H), 6.92 (dd, 1H), 4.07 (br d, 1H), 3.81 (s, 3H), 3.68 (d, 1H), 3.59-3.57 (m, 4H), 3.44 (tt, 1H), 3.24-3.20 (m, 4H), 3.13-3.03 (m, 3H), 2.38 (br d, 1H), 2.06 (q, 1H).

### Beispiel 126

### (4-Hydroxypiperidin-1-yl) {3-(3-phenyl-1,2,4-oxadiazol-5-yl)-5-[4-(trifluormethoxy)phenyl]-piperidin-1-yl}methanon [racemisches cis-Isomer]

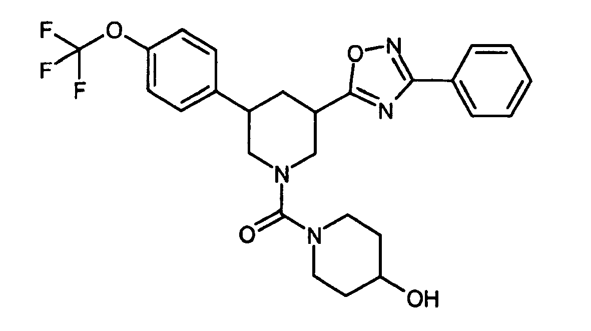

Nach der Allgemeinen Methode 2 wurden 100 mg (0.24 mmol) 1-[(4-Hydroxypiperidin-1-yl)carbonyl]-5-[4-(trifluormethoxy)phenyl]piperidin-3-carbonsäure (Beispiel 63A) und 49 mg (0.36 mmol, 1.5 eq.) *N'*-Hydroxybenzencarboximidamid umgesetzt. Ausbeute: 58 mg (47% d. Th.)

LC-MS (Methode 3B): Rₜ = 2.28 min; MS (ESIpos): m/z = 517 [M+H]⁺;

¹H-NMR (400 MHz, DMSO-d₆): δ = 8.05-7.99 (m, 2H), 7.63-7.54 (m, 3H), 7.49 (d, 2H), 7.35 (d, 2H), 4.69 (d, 1H), 4.05 (br d, 1H), 3.67-3.55 (m, 1H und br d, 1H), 3.55-3.45 (m, 3H), 3.10 (t, 1H), 3.05-2.98 (m, 2H), 2.93 (br t, 2H), 2.41 (br d, 1H), 2.07 (q, 1H), 1.79-1.68 (m, 2H), 1.39-1.27 (m, 2H).

### Beispiel 127

### {3-[3-(3-Fluorphenyl)-1,2,4-oxadiazol-5-yl]-5-[4-(trifluormethoxy)phenyl]piperidin-1-yl}(4-hydroxypiperidin-1-yl)methanon [racemisches cis-Isomer]

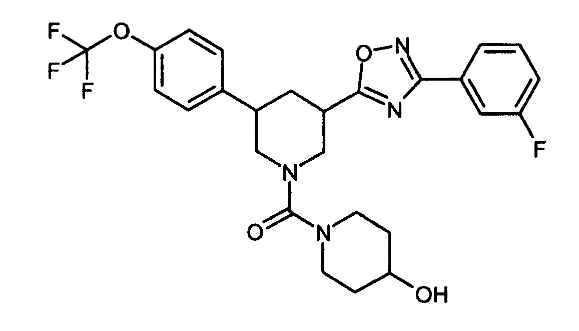

Nach der Allgemeinen Methode 2 wurden 60 mg (0.14 mmol) 1-[(4-Hydroxypiperidin-1-yl)carbonyl]-5-[4-(trifluormethoxy)phenyl]piperidin-3-carbonsäure (Beispiel 63A) und 35 mg (0.22 mmol, 1.5 eq.) 3-Fluor-*N'-*hydroxybenzencarboximidamid umgesetzt. Ausbeute: 35 mg (45% d. Th.)

LC-MS (Methode 3B): Rₜ = 2.33 min; MS (ESIpos): m/z = 535 [M+H]⁺;

¹H-NMR (400 MHz, DMSO-d₆): δ = 7.87 (d, 1H), 7.76 (dt, 1H), 7.64 (dt, 1H), 7.52-7.44 (d, 2H und dt, 1H), 7.35 (d, 2H), 4.69 (d, 1H), 4.05 (br d, 1H), 3.67-3.55 (m, 1H und br d, 1H), 3.55-3.45 (m, 3H), 3.10 (t, 1H), 3.05-2.98 (m, 2H), 2.98-2.88 (m, 2H), 2.41 (br d, 1H), 2.07 (q, 1H), 1.78-1.69 (m, 2H), 1.38-1.27 (m, 2H).

### Beispiel 128

### (3-[3-(3-Chlorphenyl)-1,2,4-oxadiazol-5-yl]-5-[4-(trifluormethoxy)phenyl]piperidin-1-yl)(4-hydroxypiperidin-1-yl)methanon [racemisches cis-Isomer]

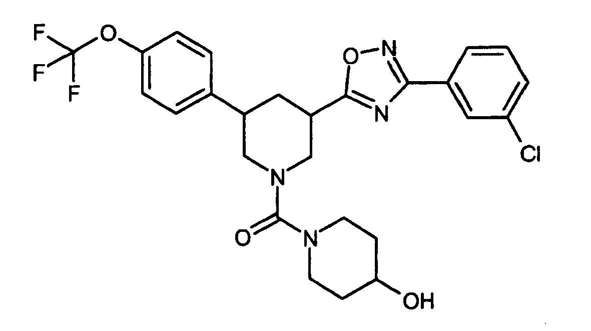

Nach der Allgemeinen Methode 2 wurden 100 mg (0.24 mmol) 1-[(4-Hydroxypiperidin-1-yl)carbonyl]-5-[4-(trifluormethoxy)phenyl]piperidin-3-carbonsäure (Beispiel 63A) und 61 mg (0.36 mmol, 1.5 eq.) 3-Chlor-*N'*-hydroxybenzencarboximidamid umgesetzt. Ausbeute: 69 mg (52% d. Th.)

LC-MS (Methode 3B): Rₜ = 2.47 min; MS (ESIpos): m/z = 551 [M+H]⁺;

¹H-NMR (400 MHz, DMSO-d₆): δ = 8.02-7.97 (m, 2H), 7.72-7.68 (m, 1H), 7.62 (t, 1H), 7.49 (d, 2H), 7.34 (d, 2H), 4.69 (d, 1H), 4.04 (br d, 1H), 3.68-3.56 (m, 1H und br d, 1H), 3.56-3.46 (m, 3H), 3.10 (t, 1H), 3.05-2.98 (m, 2H), 2.93 (br t, 2H), 2.41 (br d, 1H), 2.05 (q, 1H), 1.78-1.68 (m, 2H), 1.38-1.27 (m, 2H).

### Beispiels 129

### {3-[3-(2-Fluorphenyl)-1,2,4-oxadiazol-5-yl]-5-[4-(trifluormethoxy)phenyl]piperidin-1-yl}(4-hydroxypiperidin-1-yl)methanon [racemisches cis-Isomer]

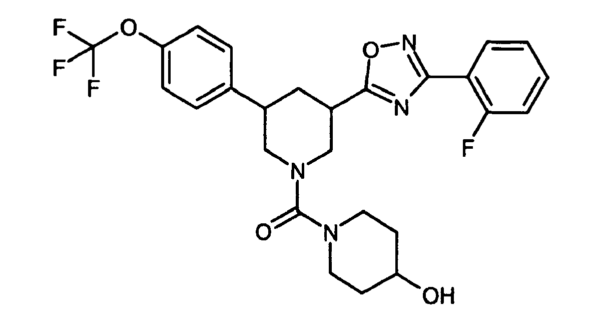

Nach der Allgemeinen Methode 2 wurden 100 mg (0.24 mmol) 1-[(4-Hydroxypiperidin-1-yl)carbonyl]-5-[4-(trifluormethoxy)phenyl]piperidin-3-carbonsäure (Beispiel 63A) und 56 mg (0.36 mmol, 1.5 eq.) 2-Fluor-*N'*-hydroxybenzencarboximidamid umgesetzt. Ausbeute: 73 mg (57% d. Th.)

LC-MS (Methode 2B): Rₜ = 1.38 min; MS (ESIpos): m/z = 535 [M+H]⁺;

¹H-NMR (400 MHz, DMSO-d₆): δ = 8.03 (dt, 1H), 7.70-7.64 (m, 1H), 7.49 (d, 2H), 7.48-7.39 (m, 2H), 7.35 (d, 2H), 4.69 (d, 1H), 4.05 (br d, 1H), 3.67-3.56 (m, 1H und br d, 1H), 3.56-3.46 (m, 3H), 3.10 (t, 1H), 3.05-2.98 (m, 2H), 2.93 (br t, 2H), 2.41 (br d, 1H), 2.07 (q, 1H), 1.79-1.69 (m, 2H), 1.38-1.27 (m, 2H).

### Beispiel 130

### (4-Hydroxypiperidin-1-yl){3-[3-(pyrazin-2-yl)-1,2,4-oxadiazol-5-yl]-5-[4-(trifluormethoxy)-phenyl]piperidin-1-yl}methanon [racemisches cis-Isomer]

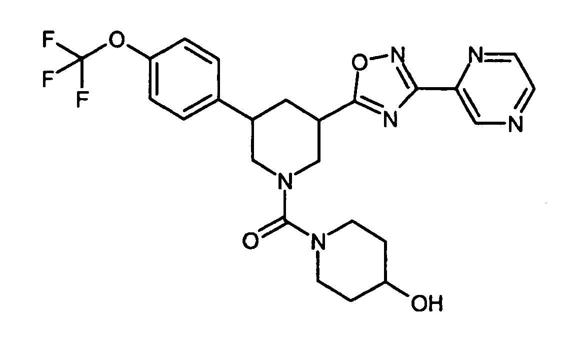

Nach der Allgemeinen Methode 2 wurden 100 mg (0.24 mmol) 1-[(4-Hydroxypiperidin-1-yl)carbonyl]-5-[4-(trifluormethoxy)phenyl]piperidin-3-carbonsäure (Beispiel 63A) und 50 mg (0.36 mmol, 1.5 eq.) *N'*-Hydroxypyrazin-2-carboximidamid umgesetzt. Ausbeute: 34 mg (27% d. Th.)

LC-MS (Methode 1B): Rₜ = 2.18 min; MS (ESIpos): m/z = 519 [M+H]⁺;

¹H-NMR (400 MHz, DMSO-d₆): δ = 9.27 (d, 1H), 8.90-8.85 (m, 2H), 7.49 (d, 2H), 7.35 (d, 2H), 4.69 (d, 1H), 4.07 (br d, 1H), 3.68-3.46 (m, 5H), 3.12 (t, 1H), 3.08-2.98 (m, 2H), 2.93 (br t, 2H), 2.43 (br d, 1H), 2.09 (q, 1H), 1.79-1.68 (m, 2H), 1.38-1.27 (m, 2H).

### Beispiel 131

### {3-(3-tert.-Butyl-1,2,4-oxadiazol-5-yl)-5-[4-(trifluormethoxy)phenyl]piperidin-1-yl} (4-hydroxypiperidin-1-yl)methanon [racemisches cis-Isomer]

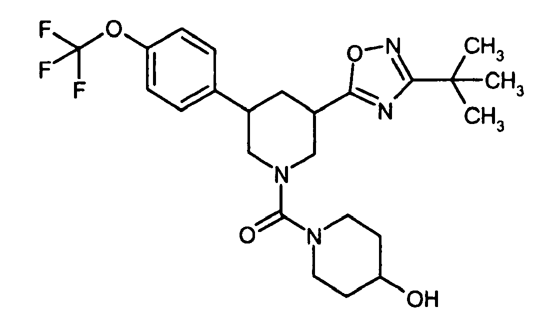

Nach der Allgemeinen Methode 2 wurden 100 mg (0.24 mmol) 1-[(4-Hydroxypiperidin-1-yl)carbonyl]-5-[4-(trifluormethoxy)phenyl]piperidin-3-carbonsäure (Beispiel 63A) und 42mg (0.36 mmol, 1.5 eq.) *N'*-Hydroxy-2,2-dimethylpropanimidamid umgesetzt. Ausbeute: 60 mg (51% d. Th.)

LC-MS (Methode 2B): Rₜ = 1.40 min; MS (ESIpos): m/z = 497 [M+H]⁺;

¹H-NMR (400 MHz, DMSO-d₆): δ = 7.47 (d, 2H), 7.33 (d, 2H), 4.68 (d, 1H), 3.95 (br d, 1H), 3.66-3.57 (m, 1H), 3.55 (br d, 1H), 3.52-3.43 (m, 2H), 3.43-3.33 (m, 1H), 3.05-2.94 (m, 3H), 2.90 (br t, 2H), 2.33 (br d, 1H), 1.97 (q, 1H), 1.77-1.68 (m, 2H), 1.35-1.26 (m, 2H), 1.30 (s, 9H).

### Beispiel 132

### [3-(4-Ethylphenyl)-5-(3-phenyl-1,2,4-oxadiazol-5-yl)piperidin-1-yl] (4-hydroxypiperidin-1-yl)methanon [racemisches cis-Isomer]

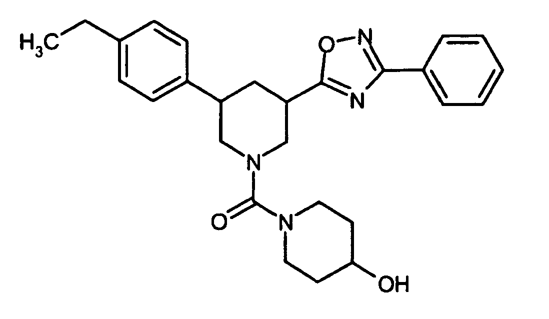

Nach der Allgemeinen Methode 2 wurden 80 mg (0.22 mmol) 5-(4-Ethylphenyl)-1-[(4-hydroxypiperidin-1-yl)carbonyl]piperidin-3-carbonsäure (Beispiel 59A) und 45 mg (0.33 mmol, 1.5 eq.) *N'*-Hydroxybenzencarboximidamid umgesetzt. Ausbeute: 76 mg (74% d. Th.)

LC-MS (Methode 2B): Rₜ = 1.41 min; MS (ESIpos): m/z = 461 [M+H]⁺;

¹H-NMR (400 MHz, DMSO-d₆): δ = 8.05-8.00 (m, 2H), 7.63-7.54 (m, 3H), 7.24 (d, 2H), 7.18 (d, 2H), 4.69 (d, 1H), 4.05 (br d, 1H), 3.67-3.58 (m, 1H), 3.57 (br d, 1H), 3.54-3.43 (m, 3H), 3.08 (t, 1H), 3.00-2.85 (m, 4H), 2.58 (q, 2H), 2.38 (br d, 1H), 2.05 (q, 1H), 1.78-1.69 (m, 2H), 1.38-1.26 (m, 2H), 1.17 (t, 3H).

### Beispiel 133

### {3-(4-Ethylphenyl)-5-[3-(3-fluorphenyl)-1,2,4-oxadiazol-5-yl]piperidin-1-yl}(4-hydroxypiperidin-1-yl)methanon [racemisches cis-Isomer]

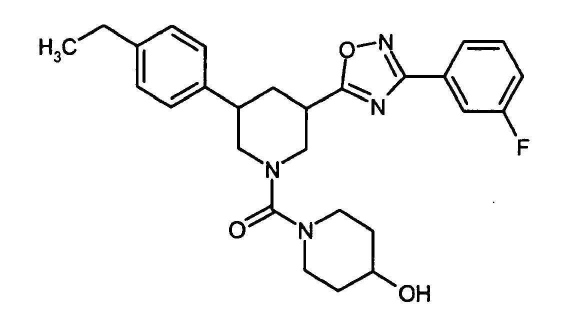

Nach der Allgemeinen Methode 2 wurden 71 mg (0.20 mmol) 5-(4-Ethylphenyl)-1-[(4-hydroxypiperidin-1-yl)carbonyl]piperidin-3-carbonsäure (Beispiel 59A) und 46 mg (0.30 mmol, 1.5 eq.) 3-Fluor-*N'*-hydroxybenzencarboximidamid umgesetzt. Ausbeute: 68 mg (72% d. Th.)

LC-MS (Methode 2B): Rₜ = 1.45 min; MS (ESIpos): m/z = 479 [M+H]⁺;

¹H-NMR (400 MHz, DMSO-d₆): δ = 7.87 (d, 1H), 7.75 (dt, 1H), 7.64 (dt, 1H), 7.47 (dt, 1H), 7.24 (d, 2H), 7.18 (d, 2H), 4.69 (d, 1H), 4.05 (br d, 1H), 3.67-3.58 (m, 1H), 3.57 (br d, 1H), 3.54-3.45 (m, 3H), 3.07 (t, 1H), 3.01-2.84 (m, 4H), 2.58 (q, 2H), 2.38 (br d, 1H), 2.05 (q, 1H), 1.78-1.68 (m, 2H), 1.38-1.27 (m, 2H), 1.17 (t, 3H).

### Beispiel 134

### {3-[3-(3-Chlorphenyl)-1,2,4-oxadiazol-5-yl]-5-(4-ethylphenyl)piperidin-1-yl}(4-hydroxypiperidin-1-yl)methanon [racemisches cis-Isomer]

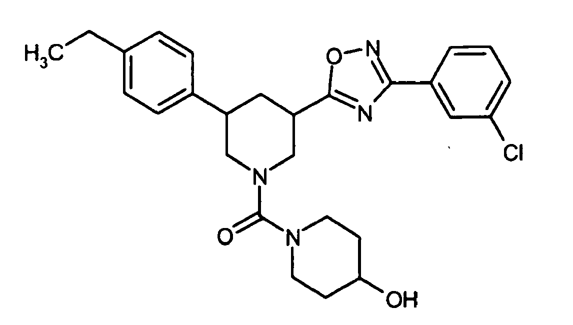

Nach der Allgemeinen Methode 2 wurden 80 mg (0.22 mmol) 5-(4-Ethylphenyl)-1-[(4-hydroxypiperidin-1-yl)carbonyl]piperidin-3-carbonsäure (Beispiel 59A) und 57 mg (0.33 mmol, 1.5 eq.) 3-Chlor-*N'*-hydroxybenzencarboximidamid umgesetzt. Ausbeute: 86 mg (78% d. Th.)

LC-MS (Methode 2B): Rₜ = 1.53 min; MS (ESIpos): m/z = 495 [M+H]⁺;

¹H-NMR (400 MHz, DMSO-d₆): δ = 8.02-7.97 (m, 2H), 7.69 (d, 1H), 7.62 (t, 1H), 7.24 (d, 2H), 7.18 (d, 2H), 4.69 (d, 1H), 4.05 (br d, 1H), 3.67-3.58 (m, 1H), 3.56 (br d, 1H), 3.54-3.45 (m, 3H), 3.08 (t, 1H), 3.01-2.84 (m, 4H), 2.58 (q, 2H), 2.38 (br d, 1H), 2.05 (q, 1H), 1.78-1.69 (m, 2H), 1.38-1.27 (m, 2H), 1.17 (t, 3H).

### Beispiel 135

### {3-(4-Ethylphenyl)-5-[3-(pyrazin-2-yl)-1,2,4-oxadiazol-S-yl]piperidin-1-yl } (4-hydroxypiperidin-1-yl)methanon [racemisches cis-Isomer]

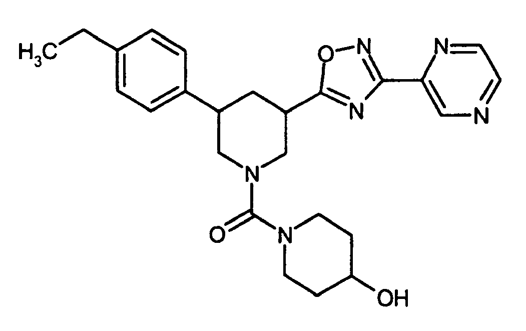

Nach der Allgemeinen Methode 2 wurden 80 mg (0.22 mmol) 5-(4-Ethylphenyl)-1-[(4-hydroxypiperidin-1-yl)carbonyl]piperidin-3-carbonsäure (Beispiel 59A) und 46 mg (0.33 mmol, 1.5 eq.) *N'*-Hydroxypyrazin-2-carboximidamid umgesetzt. Ausbeute: 24 mg (23% d. Th.)

LC-MS (Methode 3B): Rₜ = 1.75 min; MS (ESIpos): m/z = 463 [M+H]⁺;

¹H-NMR (400 MHz, DMSO-d₆): δ = 9.27 (d, 1H), 8.91-8.84 (m, 2H), 7.25 (d, 2H), 7.18 (d, 2H), 4.69 (d, 1H), 4.06 (br d, 1H), 3.68-3.45 (m, 5H), 3.10 (t, 1H), 3.01-2.86 (m, 4H), 2.58 (q, 2H), 2.40 (br d, 1H), 2.07 (q, 1H), 1.78-1.69 (m, 2H), 1.38-1.27 (m, 2H), 1.17 (t, 3H).

### Beispiel 136

### 1-({3-(4-Ethylphenyl)-5-[3-(3-fluorphenyl)-1,2,4-oxadiazol-5-yl]piperidin-1-yl}carbonyl)-piperidin-4-carbonitril [racemisches cis-Isomer]

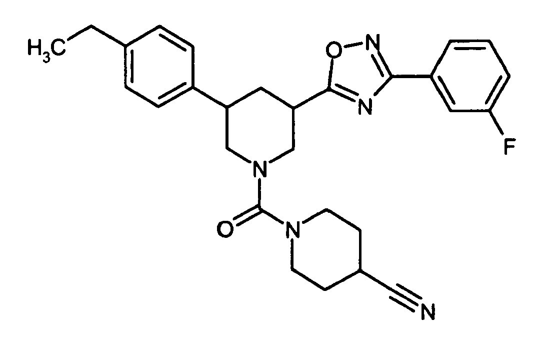

Nach der Allgemeinen Methode 2 wurden 80 mg (0.22 mmol) 5-(4-Ethylphenyl)-1-[(4-cyanopiperidin-1-yl)carbonyl]piperidin-3-carbonsäure (Beispiel 60A) und 50 mg (0.33 mmol, 1.5 eq.) 3-Fluor-*N'*-hydroxybenzencarboximidamid umgesetzt. Ausbeute: 81 mg (77% d. Th.)

LC-MS (Methode 1B): Rₜ = 2.99 min; MS (ESIpos): m/z = 488 [M+H]⁺;

¹H-NMR (400 MHz, DMSO-d₆): δ = 7.88 (d, 1H), 7.76 (dt, 1H), 7.64 (dt, 1H), 7.47 (dt, 1H), 7.24 (d, 2H), 7.18 (d, 2H), 4.06 (br d, 1H), 3.58 (br d, 1H), 3.55-3.45 (m, 1H), 3.43-3.34 (m, 2H), 3.15-3.02 (m, 4H), 3.01-2.86 (m, 2H), 2.58 (q, 2H), 2.38 (br d, 1H), 2.04 (q, 1H), 1.93-1.84 (m, 2H), 1.75-1.64 (m, 2H), 1.17 (t, 3H).

### Beispiel 137

### {3-[3-(3,5-Difluorphenyl)-1,2,4-oxadiazol-5-yl]-5-[4-(trifluormethoxy)phenyl]piperidin-1-yl}-(morpholin-4-yl)methanon [racemisches cis-Isomer]

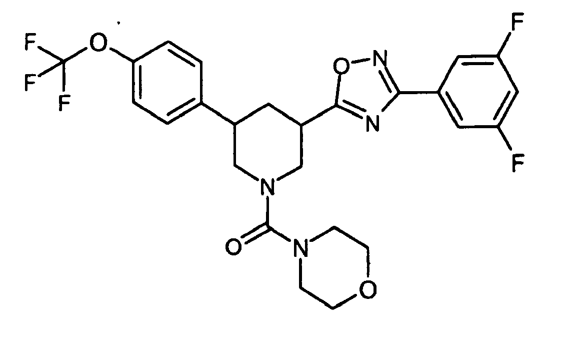

Nach der Allgemeinen Methode 1 wurden 80 mg (0.20 mmol) 1-(Morpholin-4-ylcarbonyl)-5-[4-(trifluormethoxy)phenyl]piperidin-3-carbonsäure (Beispiel 44A) und 38 mg (0.22 mmol, 1.1 eq.) 3,5-Difluor-*N'*-hydroxybenzencarboximidamid umgesetzt. Ausbeute: 75 mg (68% d. Th.)

HPLC (Methode 1A): Rₜ = 5.28 min; MS (ESIpos): m/z = 539 [M+H]⁺;

¹H-NMR (400 MHz, DMSO-d₆): δ = 7.71-7.65 (m, 2H), 7.61-7.53 (m, 1H), 7.49 (d, 2H), 7.35 (d, 2H), 4.09 (br d, 1H), 3.64 (br d, 1H), 3.61-3.53 (m, 4H), 3.58-3.48 (m, 1H), 3.26-3.18 (m, 4H), 3.13 (t, 1H), 3.08-2.98 (m, 2H), 2.41 (br d, 1H), 2.07 (q, 1H).

### Beispiel 138

### 4-({3-(3-Phenyl-1,2,4-oxadiazol-5-yl)-5-[4-(trifluormethyl)phenyl]piperidin-1-yl}carbonyl)-morpholin [racemisches cis-Isomer]

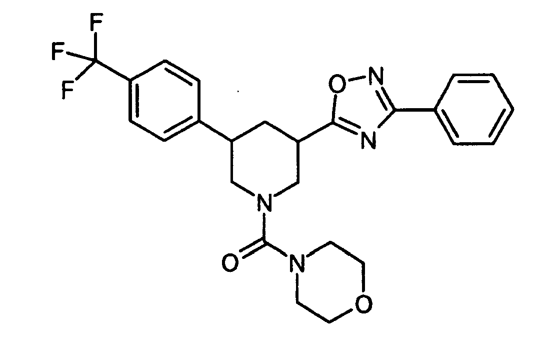

Nach der Allgemeinen Methode 1 wurden 250 mg (0.65 mmol) 1-(Morpholin-4-ylcarbonyl)-5-[4-(trifluormethyl)phenyl]piperidin-3-carbonsäure (Beispiel 49A) und 97 mg (0.71 mmol) *N'-*Hydroxybenzencarboximidamid umgesetzt. Ausbeute: 35 mg (12% d. Th.)

LC-MS (Methode 1B): Rₜ = 2.75 min; MS (ESIpos): m/z = 487 [M+H]⁺;

¹H-NMR (400 MHz, DMSO-d₆): δ = 8.02 (dd, 2H), 2.71 (d, 2H), 7.61-7.55 (m, 5H), 4.10 (br d, 1H), 3.66 (br d, 1H), 3.60-3.56 (m, 4H), 3.52 (tt, 1H), 3.24-3.20 (m, 4H), 3.16 (t, 1H), 3.08-3.05 (m, 2H), 2.42 (br d, 1H), 2.11 (q, 1H).

### Beispiel 139

### 4-({3-(3-Benzyl-1,2,4-oxadiazol-5-yl)-5-[4-(trifluormethyl)phenyl]piperidin-1-yl}carbonyl)-morpholin [racemisches cis-Isomer]

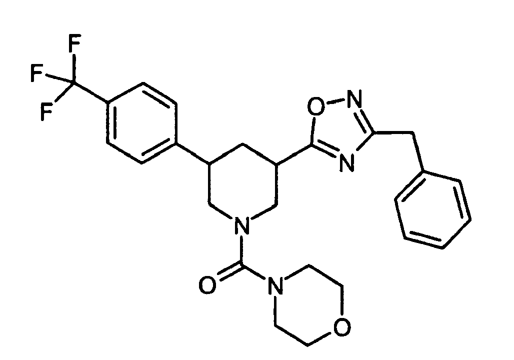

Nach der Allgemeinen Methode 1 wurden 250 mg (0.65 mmol) 1-(Morpholin-4-ylcarbonyl)-5-[4-(trifluormethyl)phenyl]piperidin-3-carbonsäure und 107 mg (0.71 mmol) *N'*-Hydroxy-2-phenyl-ethanimidamid umgesetzt. Ausbeute: 44 mg (14% d. Th.)

LC-MS (Methode 1B): Rₜ = 2.67 min; MS (ESIpos): m/z = 501 [M+H]⁺;

¹H-NMR (400 MHz, DMSO-d₆): δ = 7.69 (d, 2H), 7.56 (d, 2H), 7.35-7.23 (m, 5H), 4.08 (s, 2H), 3.98 (br d, 1H), 3.61 (br d, 1H), 3.56-3.54 (m, 4H), 3.41 (tt, 1H), 3.21-3.17 (m, 4H), 3.07-2.97 (m, 3H), 2.30 (br d, 1H), 1.99 (m, 1H).

### Beispiel 140

### 4-({3-(3-(Pyridin-4-yl)-1,2,4-oxadiazol-5-yl)-5-[4-(trifluormethyl)phenyl]piperidin-1-yl}carbonyl)-morpholin [racemisches cis-Isomer]

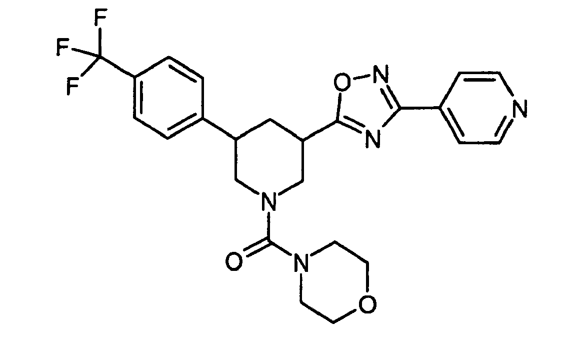

Nach der Allgemeinen Methode 1 wurden 250 mg (ca. 0.65 mmol) 1-(Morpholin-4-ylcarbonyl)-5-[4-(trifluormethyl)phenyl]piperidin-3-carbonsäure und 97 mg (0.71 mmol) *N'*-Hydroxypyridin-4-carboximidamid umgesetzt. Ausbeute: 45 mg (14% d. Th.)

LC-MS (Methode 3B): Rₜ = 1.98 min; MS (ESIpos): m/z = 488 [M+H]⁺;

¹H-NMR (400 MHz, DMSO-d₆): δ = 8.81 (d, 2H), 7.94 (d, 2H), 7.72 (d, 2H), 7.60 (d, 2H), 4.10 (br d, 1H), 3.65 (br d, 1H), 3.58-3.53 (m, 5H), 3.24-3.20 (m, 4H), 3.13-3.00 (m, 3H), 2.42 (br d, 1H), 2.11 (q, 1H).

### Beispiel 141

### 4-({3-(3-(Pyridin-3-yl)-1,2,4-oxadiazol-5-yl)-5-[4-(trifluormethyl)phenyl]piperidin-1-yl}-carbonyl)morpholin [racemisches cis-Isomer]

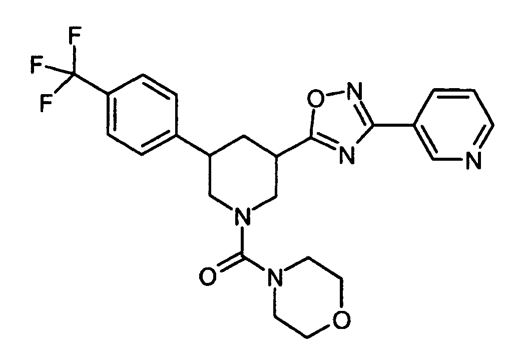

Nach der Allgemeinen Methode 1 wurden 250 mg (ca. 0.65 mmol) 1-(Morpholin-4-ylcarbonyl)-5-[4-(trifluonnethyl)phenyl]piperidin-3-carbonsäure und 98 mg (0.71 mmol) *N'*-Hydroxypyridin-3-carboximidamid umgesetzt. Ausbeute: 35 mg (11% d. Th.)

LC-MS (Methode 1B): Rₜ = 2.35 min; MS (ESIpos): m/z = 488 [M+H]⁺;

¹H-NMR (400 MHz, DMSO-d₆): δ = 9.17 (d, 1H), 8.78 (dd, 1H), 8.38 (d, 1H), 7.71 (d, 2H), 7.64-7.57 (m, 3H), 4.10 (br d, 1H), 3.66 (br d, 1H), 3.60-3.55 (m, 5H), 3.27-3.20 (m, 4H), 3.17-3.04 (m, 3H), 2.42 (br d, 1H), 2.12 (q, 1H).

### Beispiel 142

### 4-({3-(3-Methylsulfonylmethyl-1,2,4-oxadiazol-5-yl)-5-[4-(trifluormethyl)phenyl]piperidin-1-yl}carbonyl)morpholin [racemisches cis-Isomer]

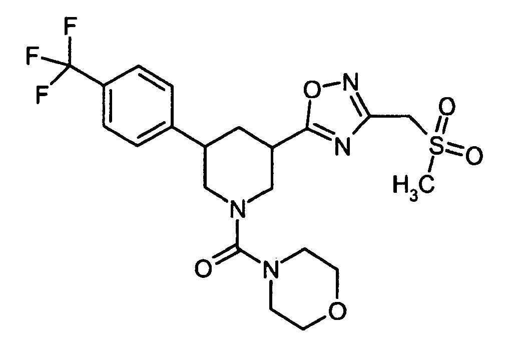

Nach der Allgemeinen Methode 1 wurden 250 mg (ca. 0.65 mmol) 1-(Morpholin-4-ylcarbonyl)-5-[4-(trifluormethyl)phenyl]piperidin-3-carbonsäure und 108 mg (0.71 mmol) *N'*-Hydroxy-2-(methylsulfonyl)ethanimidamid umgesetzt. Ausbeute: 5 mg (2% d. Th.)

LC-MS (Methode 1B): Rₜ = 2.17 min; MS (ESIpos): m/z = 503 [M+H]⁺;

¹H-NMR (400 MHz, DMSO-d₆): δ = 7.70 (d, 2H), 7.58 (d, 2H), 4.85 (d, 2H), 4.04 (br d, 1), 3.63 (br d, 1H), 3.60-3.58 (m, 4H), 3.45 (tt, 1H), 3.25-3.19 (m, 4H), 3.17 (s, 3H), 3.12-2.99 (m, 3H), 2.37 (br d, 1H), 2.05 (q, 1H).

### Beispiel 143

### 4-({3-(5-Phenyl-1,3,4-oxadiazol-2-yl)-5-[4-(trifluonmethyl)phenyl]piperidin-1-yl}carbonyl)-morpholin [racemisches cis-Isomer]

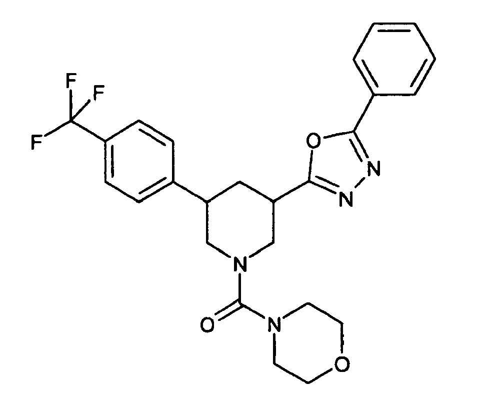

Nach der Allgemeinen Methode 4 wurden 200 mg (0.52 mmol) 1-(Morpholin-4-ylcarbonyl)-5-[4-(tritluormethyl)phenyl]piperidin-3-carbonsäure und 77 mg (0.57 mmol) *N*-Benzoylhydrazid umgesetzt. Ausbeute: 36 mg (14% d. Th.)

LC-MS (Methode 1B): Rₜ = 2.48 min; MS (ESIpos): m/z = 487 [M+H]⁺;

¹H-NMR (400 MHz, DMSO-d₆): δ = 8.02 (dd, 2H), 7.71 (d, 2H), 7.63-7.57 (m, 5H), 4.09 (br d, 1H), 3.67 (br d, 1H), 3.61-3.55 (m, 4H), 3.44 (tt, 1H), 3.25-3.20 (m, 4H), 3.13 (t, 1H), 3.09-3.02 (m, 2H), 2.42 (br d, 1H), 2.08 (q, 1H).

### Beispiel 144

### {3-(3-Cyclopropyl-1,2,4-oxadiazol-5-yl)-5-[4-(trifluormethoxy)phenyl]piperidin-1-yl}(3,3-difluorpyrrolidin-1-yl)methanon [racemisches cis-Isomer]

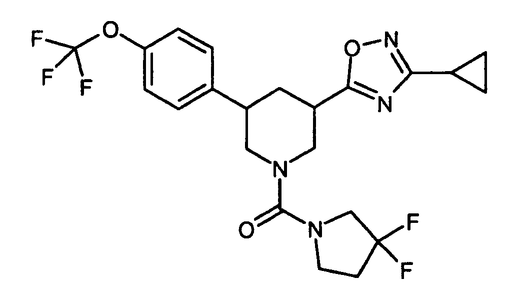

Nach der Allgemeinen Methode 6 wurden 100 mg (0.19 mmol) der Verbindung aus Beispiel 176A und 83 mg (0.58 mmol) 3,3-Difluorpyrrolidin-Hydrochlorid umgesetzt. Ausbeute: 44 mg (45% d. Th.)

LC-MS (Methode 1B): Rₜ = 2.80 min; MS (ESIpos): m/z = 487 [M+H]⁺;

¹H-NMR (400 MHz, DMSO-d₆): δ = 7.46 (d, 2H), 7.32 (d, 2H), 4.01 (br d, 1H), 3.77-3.64 (m, 3H), 3.58-3.53 (m, 2H), 3.38-3.34 (m, 1H), 3.04-2.94 (m, 3H), 2.46-2.20 (m, 3H), 2.14-2.07 (m, 1H), 1.98-1.89 (m, 1H), 1.08-1.03 (m, 2H), 0.90-0.86 (m, 2H).

### Beispiel 145

### (4,4-Difluorpiperidin-1-yl) {3-[3-(3-fluorphenyl)-1,2,4-oxadiazol-5-yl]-5-[4-(trifluormethoxy)-phenyl]piperidin-1-yl}methanon [racemisches cis-Isomer]

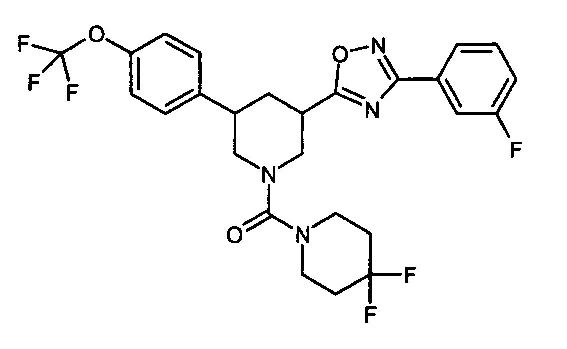

Nach der Allgemeinen Methode 5 wurden 100 mg (0.21 mmol) der Verbindung aus Beispiel 179A und 33 mg (0.2 mmol) 4,4-Difluorpiperidin-Hydrochlorid umgesetzt. Ausbeute: 37 mg (30% d. Th.)

LC-MS (Methode 1B): Rₜ = 3.04 min; MS (ESIpos): m/z = 555 [M+H]⁺;

¹H-NMR (400 MHz, DMSO-d₆): δ = 7.86 (d, 1H), 7.78-7.74 (m, 1H), 7.67-7.61 (m, 1H), 7.51-7.44 (m, 3H), 7.34 (d, 2H), 4.10 (br d, 1H), 3.68 (br d, 1H), 3.56-3.49 (m, 1H), 3.35-3.31 (m, 4H), 3.15 (t, 1H), 3.06-2.99 (m, 2H), 2.41 (br d, 1H), 2.09-1.95 (m, 5H).

### Beispiel 146

### {3-(3-Cyclopropyl-1,2,4-oxadiazol-5-yl)-5-[4-(trifluormethyl)phenyl]piperidin-1-yl}(3-hydroxy-pyrrolidin-1-yl)methanon [Diastereomerengemisch]

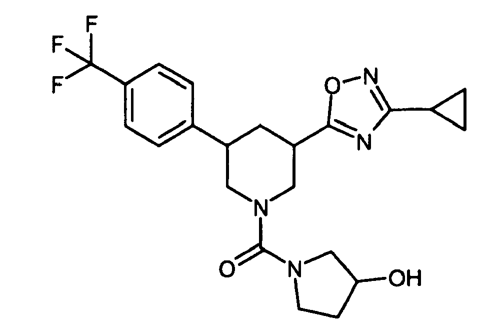

Nach der Allgemeinen Methode 6 wurden 100 mg (0.20 mmol) der Verbindung aus Beispiel 184A und 52 mg (0.6 mmol) 3-Pyrrolidinol umgesetzt. Ausbeute: 50 mg (55% d. Th.)

LC-MS (Methode 3B): Rₜ = 4.39 min; MS (ESIpos): m/z = 451 [M+H]⁺;

¹H-NMR (400 MHz, DMSO-d₆): δ = 7.69 (d, 2H), 7.56 (d, 2H), 4.88 (d, OH), 4.21 (br s, 1H), 4.04-4.00 (m, 1H), 3.69 (t, 1H), 3.48-3.42 (m, 2H), 3.30-3.25 (m, 2H), 3.10-2.90 (m, 4H), 2.32 (br d, 1H), 2.14-2.07 (m, 1H), 2.02-1.92 (m, 1H), 1.83-1.80 (m, 1H), 1.74-1.70 (m, 1H), 1.07-1.03 (m, 2H), 0.90-0.86 (m, 2H).

### Beispiel 147

### [3-(3-Cyclopropyl-1,2,4-oxadiazol-5-yl)-5-(4-ethylphenyl)piperidin-1-yl](3-hydroxyazetidin-1-yl)methanon] [racemisches cis-Isomer]

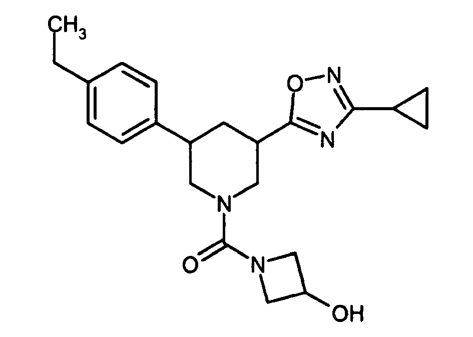

Nach der Allgemeinen Methode 2 wurden 60 mg (0.18 mmol) der Verbindung aus Beispiel 138A und 27 mg (0.27 mmol) *N'*-Hydroxycyclopropancarboximidamid umgesetzt. Ausbeute: 24 mg (33% d. Th.)

LC-MS (Methode 1B): Rₜ = 2.29 min; MS (ESIpos): m/z = 397 [M+H]⁺;

¹H-NMR (400 MHz, DMSO-d₆): δ = 7.20 (d, 2H), 7.16 (d, 2H), 5.57 (d, OH), 4.41-4.34 (m, 1H), 4.13-4.04 (m, 3H), 3.71-3.65 (m, 3H), 3.27-3.20 (m, 1H), 2.94 (t, 1H), 2.90 (t, 1H), 2.80-2.72 (m, 1H), 2.57 (q, 2H), 2.23 (br d, 1H), 2.14-2.07 (m, 1H), 1.92 (q, 1H), 1.16 (t, 3H), 1.07-1.02 (m, 2H), 0.91-0.87 (m, 2H).

### Beispiel 148

### {3-(3,4-Dimethylphenyl)-5-[3-(propan-2-yl)-1,2,4-oxadiazol-5-yl]piperidin-1-yl} (morpholin-4-yl)methanon [racemisches cis-Isomer]

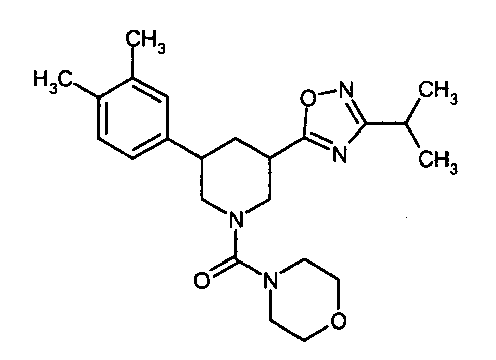

Nach der Allgemeinen Methode 2 wurden 100 mg (0.29 mmol) der Verbindung aus Beispiel 130A und 46 mg (0.43 mmol) *N'*-Hydroxy-2-methylpropanimidamid umgesetzt. Ausbeute: 107 mg (90% d. Th.)

LC-MS (Methode 3B): Rₜ = 2.25 min; MS (ESIpos): m/z = 413 [M+H]⁺;

¹H-NMR (400 MHz, DMSO-d₆): δ = 7.09-7.07 (m, 2H), 7.01 (d, 1H), 3.97 (br d, 1H), 3.58-3.51 (m, 5H), 3.40-3.33 (m, 1H), 3.20-3.17 (m, 4H), 3.10-2.93 (m, 3H), 2.82-2.76 (m, 1H), 2.25 (br d, 1H), 2.20 (s, 3H), 2.18 (s, 3H), 1.93 (q, 1H), 1.25 (d, 6H).

### Beispiel 149

### 1-{[3-[3-(2,6-Difluorphenyl)-1,2,4-oxadiazol-5-yl]-5-(4-ethylphenyl)piperidin-1-yl]carbonyl}-piperidin-4-carbonitril [racemisches cis-Isomer]

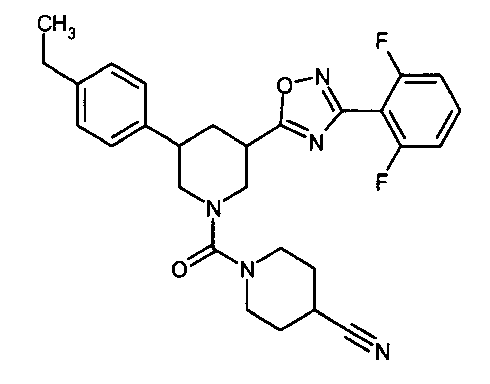

Nach der Allgemeinen Methode 2 wurden 60 mg (0.16 mmol) der Verbindung aus Beispiel 60A und 42 mg (0.24 mmol) 2,6-Difluor-*N'*-hydroxybenzolcarboximidamid umgesetzt. Ausbeute: 53 mg (65% d. Th.)

LC-MS (Methode 2B): Rₜ = 1.47 min; MS (ESIpos): m/z = 506 [M+H]⁺;

¹H-NMR (400 MHz, DMSO-d₆): δ = 7.75-7.71 (m, 1H), 7.38-7.33 (m, 2H), 7.24 (d, 2H), 7.17 (d, 2H), 4.05 (br d, 1H), 3.58-3.50 (m, 2H), 3.41-3.35 (m, 2H), 3.11-3.02 (m, 4H), 2.91 (q, 2H), 2.57 (q, 2H), 2.36 (br d, 1H), 2.03 (q, 1H), 1.89-1.85 (m, 2H), 1.72-1.64 (m, 2H), 1.17 (t, 3H).

### Beispiel 150

### {3-(4-Ehylphenyl)-5-[3-(2-methoxyethyl)-1,2,4-oxadiazol-5-yl]piperidin-1-yl}(morpholin-4-yl)methanon [racemisches cis-Isomer]

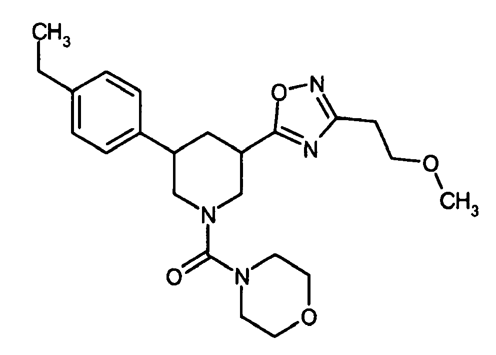

Nach der Allgemeinen Methode 2 wurden 60 mg (0.17 mmol) der Verbindung aus Beispiel 38A und 32 mg (0.26 mmol) *N'*-Hydroxy-3-methoxypropanimidamid umgesetzt. Ausbeute: 31 mg (42% d. Th.)

LC-MS (Methode 3B): Rₜ = 1.97 min; MS (ESIpos): m/z = 429 [M+H]⁺;

¹H-NMR (400 MHz, DMSO-d₆): δ = 7.22 (d, 2H), 7.16 (d, 2H), 3.98 (br d, 1H), 3.67 (t, 2H), 3.60-3.54 (m, 5H), 3.42-3.34 (m, 1H), 3.23 (s, 3H), 3.20-3.17 (m, 4H), 3.04-2.89 (m, 5H), 2.57 (q, 2H), 2.28 (br d, 1H), 1.94 (q, 1H), 1.16 (t, 3H).

### Beispiel 151

### [3-(3-Cyclopropyl-1,2,4-oxadiazol-5-yl)-5-(4-ethylphenyl)piperidin-1-yl](morpholin-4-yl)-methanon [racemisches cis-Isomer]

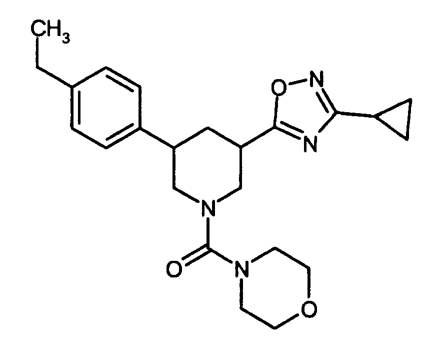

Nach der Allgemeinen Methode 2 wurden 60 mg (0.17 mmol) der Verbindung aus Beispiel 38A und 27 mg (0.26 mmol) *N'*-Hydroxycyclopropancarboximidamid umgesetzt. Ausbeute: 51 mg (70% d. Th.)

LC-MS (Methode 1B): Rₜ = 2.54 min; MS (ESIpos): m/z = 411 [M+H]⁺;

¹H-NMR (400 MHz, DMSO-d₆): δ= 7.21 (d, 2H), 7.16 (d, 2H), 3.95 (br d, 1H), 3.58-3.54 (m, 5H), 3.35-3.32 (m, 1H), 3.19-3.16 (m, 4H), 3.00-2.90 (m, 2H), 2.86-2.81 (m, 1H), 2.57 (q, 2H), 2.24 (br d, 1H), 2.14-2.07 (m, 1H), 1.90 (q, 1H), 1.16 (t, 3H), 1.07-1.03 (m, 2H), 0.90-0.86 (m, 2H).

### Beispiel 152

### 3-(3-tert-Butyl-1,2,4-oxadiazol-5-yl)-5-(4-ethylphenyl)piperidin-1-yl](4-hydroxypiperidin-1-yl)methanon [racemisches cis-Isomer]

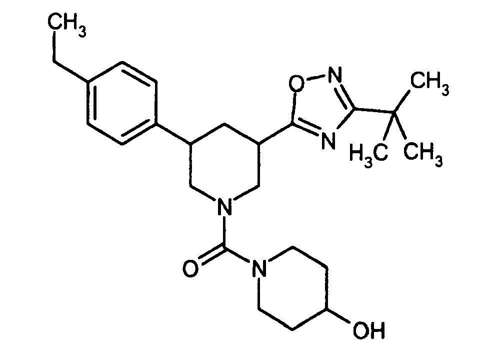

Nach der Allgemeinen Methode 2 wurden 80 mg (0.22 mmol) 5-(4-Ethylphenyl)-1-[(4-hydroxypiperidin-1-yl)carbonyl]piperidin-3-carbonsäure (Beispiel 59A) und 39 mg (0.33 mmol) *N'*-Hydroxy-2,2-dimethylpropanimidamid umgesetzt. Ausbeute: 66 mg (68% d. Th.)

LC-MS (Methode 1B): Rₜ = 2.56 min; MS (ESIpos): m/z = 441.2 [M+H]⁺;

¹H-NMR (400 MHz, DMSO-d₆): δ = 7.22 (d, 2H), 7.16 (d, 1H), 4.68 (d, 1H), 3.95 (bd, 1H), 3.66-3.42 (m, 4H), 3.41-3.29 (m, 1H), 2.79-3.03 (m, 5H), 2.91-3.09 (m, 2H), 2.52-2.62 (m, 2H), 2.28 (bd, 1h), 1.93 (q, 1H), 1.71 (bd, 2H), 1.30 (bs, 11H), 1.64 (t, 3H).

### Beispiel 153

### {3-(4-Ethylphenyl)-5-[3-(2-fluorphenyl)-1,2,4-oxadiazol-5-yl]piperidin-1-yl}(4-hydroxypiperidin-1-yl)methanon [racemisches cis-Isomer]

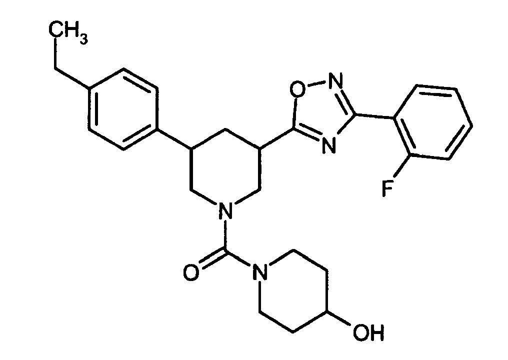

Nach der Allgemeinen Methode 2 wurden 80 mg (0.22 mmol) 5-(4-Ethylphenyl)-1-[(4-hydroxypiperidin-1-yl)carbonyl]piperidin-3-carbonsäure (Beispiel 59A) und 51 mg (0.33 mmol) 2-Fluor-*N'*-hydroxybenzolcarboximidamid umgesetzt. Ausbeute: 71 mg (67% d. Th.)

LC-MS (Methode 1B): Rₜ = 2.61 min; MS (ESIpos): m/z = 479 [M+H]⁺;

¹H-NMR (400 MHz, DMSO-d₆): δ = 8.03 (dt, 1H), 7.62-7.70 (m, 1H), 7.39-7.49 (m, 2H), 7.24 (d, 2H), 7.18 (d, 2H), 4.69 (d, 1H), 4.05 (bd, 1H), 3.44-3.69 (m, 5H), 3.07 (t, 1H), 2.84-3.01 (m, 4H), 2.58 (q, 2H), 2.38 (br d, 1H), 2.04 (q, 1H), 1.73 (br d, 2H), 1.26-1.39 (m, 2H), 1.17 (t, 3H).

### Beispiel 154

### [3-(4-Ethylphenyl)-5-{3-[3-(trifluormethyl)phenyl]-1,2,4-oxadiazol-5-yl}piperidin-1-yl](4-hydroxypiperidin-1-yl)methanon [racemisches cis-Isomer]

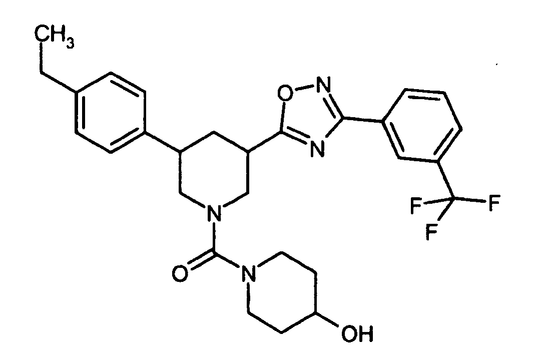

Nach der Allgemeinen Methode 2 wurden 80mg (0.22 mmol) 5-(4-Ethylphenyl)-1-[(4-hydroxypiperidin-1-yl)carbonyl]piperidin-3-carbonsäure (Beispiel 59A) und 68 mg (0.33 mmol) *N'*-Hydroxy-3-(trifluormethyl)benzolcarboximidamid umgesetzt. Ausbeute: 75 mg (64% d. Th.)

LC-MS (Methode 1B): Rₜ = 2.91 min; MS (ESIpos): m/z = 429 [M+H]⁺.

### Beispiel 155

### {3-[3-(2,6-Difluorphenyl)-1,2,4-oxadiazol-5-yl]-5-(4-ethylphenyl)piperidin-1-yl} (4-hydroxypiperidin-1-yl)methanon [racemisches cis-Isomer]

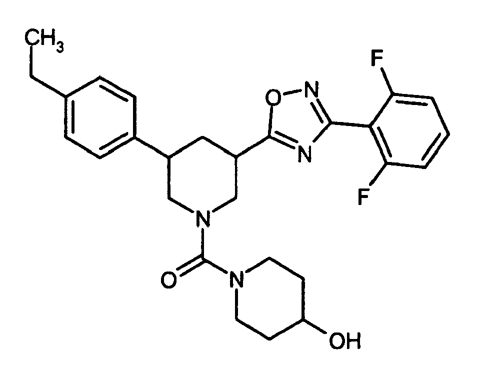

Nach der Allgemeinen Methode 2 wurden 80mg (0.22 mmol) 5-(4-Ethylphenyl)-1-[(4-hydroxypiperidin-1-yl)carbonyl]piperidin-3-carbonsäure (Beispiel 59A) und 57 mg (0.33 mmol) 2,6-Difluor-*N'*-hydroxybenzolcarboximidamid umgesetzt. Ausbeute: 57 mg (52% d. Th.)

HPLC (Methode 3B): Rₜ = 2.18 min; MS (ESIpos): m/z = 497 [M+H]⁺;

¹H-NMR (400 MHz, DMSO-d₆): δ = 7.69-7.78 (m, 1H), 7.36 (t, 2H), 7.24 (d, 2H), 7.17 (d, 2H), 4.69 (d, 1H), 4.04 (bd, 1H), 3.44-3.66 (m, 5H), 3.07 (t, 1H), 2.83-3.01 (m, 4H), 2.57 (dd, 2H), 2.38 (br d, 1H), 2.04 (q, 1H), 1.73 (br d, 2H), 1.25-1.41 (m, 2H), 1.17 (t, 3H).

### Beispiel 156

### {3-[3-(2,4-Difluorphenyl)-1,2,4-oxadiazol-5-yl]-5-(4-ethylphenyl)piperidin-1-yl} (4-hydroxypiperidin-1-yl)methanon [racemisches cis-Isomer]

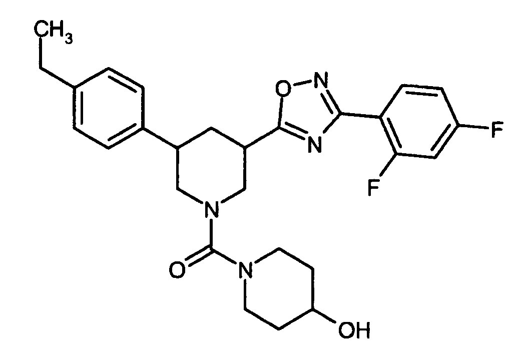

Nach der Allgemeinen Methode 2 wurden 80 mg (0.22 mmol) 5-(4-Ethylphenyl)-1-[(4-hydroxypiperidin-1-yl)carbonyl]piperidin-3-carbonsäure (Beispiel 59A) und 60 mg (0.33 mmol) 2,4-Difluor-*N'*-hydroxybenzolcarboximidamid umgesetzt. Ausbeute: 69 mg (60% d. Th.)

HPLC (Methode 3B): Rₜ = 2.28 min; MS (ESIpos): m/z = 497 [M+H]⁺;

¹H-NMR (400 MHz, DMSO-d₆): δ = 8.06-8.14 (m, 1H), 7.51-7.58 (m, 1H), 7.32 (dt, 1H), 7.26 (d, 2H), 7.18 (d, 2H), 4.69 (d, 1H), 4.04 (bd, 1H), 3.44-3.67 (m, 5H), 3.07 (t, 1H), 2.84-2.99 (m, 4H), 2.58 (dd, 2H), 2.38 (br d, 1H), 2.03 (q, 1H), 1.73 (br d, 2H), 1.26-1.39 (m, 2H), 1.17 (t, 3H).

### Beispiel 157

### [3-(4-Ethylphenyl)-5-{3-[4-methyl-5-(trifluormethyl)-4H-1,2,4-triazol-3-yl]-1,2,4-oxadiazol-5-yl}piperidin-1-yl](4-hydroxypiperidin-1-yl)methanon [racemisches cis-Isomer]

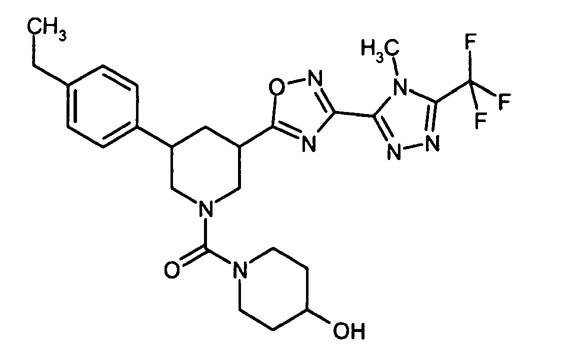

Nach der Allgemeinen Methode 2 wurden 80 mg (0.22 mmol) 5-(4-Ethylphenyl)-1-[(4-hydroxypiperidin-1-yl)carbonyl]piperidin-3-carbonsäure (Beispiel 59A) und 70 mg (0.33 mmol) *N'*-Hydroxy-4-methyl-5-(trifluormethyl)-4H-1,2,4-triazol-3-carboximidamid umgesetzt. Ausbeute: 37 mg (30% d. Th.)

HPLC (Methode 5B): Rₜ = 2.21 min; MS (ESIpos): m/z = 534 [M+H]⁺;

### Beispiel 158

### {3-(4-Ethylphenyl)-5-[3-(pyrimidin-2-yl)-1,2,4-oxadiazol-5-yl]piperidin-1-yl} (4-hydroxypiperidin-1-yl)methanon [racemisches cis-Isomer]

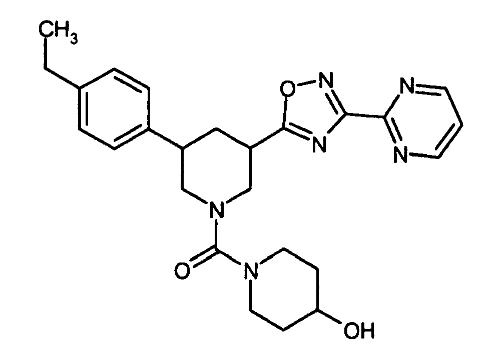

Nach der Allgemeinen Methode 2 wurden 80mg (0.22 mmol) 5-(4-Ethylphenyl)-1-[(4-hydroxypiperidin-1-yl)carbonyl]piperidin-3-carbonsäure (Beispiel 59A) und 46 mg (0.33 mmol) *N'*-Hydroxypyrimidin-2-carboximidamid umgesetzt. Ausbeute: 66 mg (64% d. Th.)

LC-MS (Methode 1B): Rₜ = 2.02 min; MS (ESIpos): m/z = 463 [M+H]⁺;

¹H-NMR (400 MHz, DMSO-d₆): δ = 9.03 (d, 2h), 7.72 (t, 1H), 7.24 (d, 2H), 7.18 (d, 2H), 4.69 (d, 1H), 4.04 (bd, 1H), 3.45-3.67 (m, 5H), 3.09 (t, 1H), 2.85-2.97 (m, 4H), 2.58 (dd, 2H), 2.39 (br d, 1H), 2.06 (q, 1H), 1.73 (br d, 2H), 1.26-1.39 (m, 2H), 1.17 (t, 3H).

### Beispiel 159

### {3-[5-(3-Fluorphenyl)-1,2,4-oxadiazol-3-yl]-5-[4-(trifluormethyl)phenyl]piperidin-1-yl}-(morpholin-4-yl)methanon [racemisches cis-Isomer]

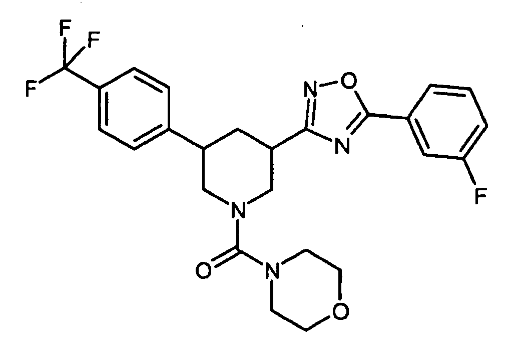

Nach der Allgemeinen Methode 2 wurden 60 mg (0.43 mmol) m-Fluorbenzoesäure und 120 mg (ca. 0.22 mmol) der Verbindung aus Beispiel 84A umgesetzt. Ausbeute: 61 mg (56% d. Th.).

LC-MS (Methode 2B): Rₜ = 1.48 min; MS (ESIpos): m/z = 505 [M+H]⁺;

¹H-NMR (400 MHz, DMSO-d₆): δ = 7.96 (d, 1H), 7.90 (br d, 1H), 7.73-7.67 (m, 3H), 7.60-7.57 (m, 3H), 4.05 (brd, 1H), 3.68 (d, 1H), 3.58-3.55 (m, 4H), 3.27-3.20 (m, 4H), 3.09-3.02 (m, 4H), 2.35 (br d, 1H), 2.03 (q, 1H).

### Beispiel 160

### Morpholin-4-yl {3-(5-phenyl-1,2,4-oxadiazol-3-yl)-5-[4(trifluormethyl)phenyl]piperidin-1-yl}-methanon [racemisches cis-Isomer]

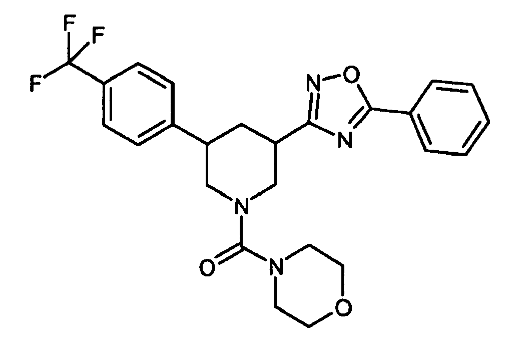

Nach der Allgemeinen Methode 2 wurden 52 mg (0.43 mmol) Benzoesäure und 120 mg (ca. 0.22 mmol) der Verbindung aus Beispiel 84A umgesetzt. Ausbeute: 9 mg (9% d. Th.).

LC-MS (Methode 2B): Rₜ = 1.46 min; MS (ESIpos): m/z = 487 [M+H]⁺;

¹H-NMR (400 MHz, DMSO-d₆): δ = 8.11 (d, 2H), 7.73-7.70 (m, 3H), 7.65-7.59 (m, 4H), 4.05 (br d, 1H), 3.68 (d, 1H), 3.61-3.55 (m, 4H), 3.26-3.19 (m, 5H), 3.10-3.02 (m, 3H), 2.34 (br d, 1H), 2.02 (q, 1H).

### Beispiel 161

### 3-(5-Cyclopropyl-1,2,4-oxadiazol-3-yl)-5-[4-(trifluormethyl)phenyl]piperidin-1-yl} (morpholin-4-yl)methanon [racemisches cis-Isomer]

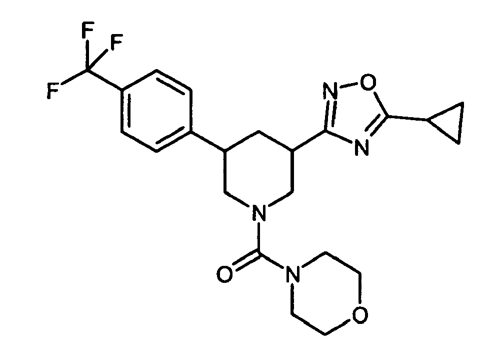

Nach der Allgemeinen Methode 2 wurden 36 mg (0.43 mmol) Cyclopropancarbonsäure und 100 mg (ca. 0.22 mmol) der Verbindung aus Beispiel 84A umgesetzt. Ausbeute: 33 mg (34% d. Th.).

LC-MS (Methode 2B): Rₜ = 1.31 min; MS (ESIpos): m/z = 451 [M+H]⁺;

¹H-NMR (400 MHz, DMSO-d₆): δ = 7.69 (d, 2H), 7.56 (d, 2H), 3.91 (br d, 1H), 3.64 (d, 1H), 3.58-3.52 (m, 4H), 3.21-3.13 (m, 4H), 3.10 (tt, 1H), 3.03-2.92 (m, 3H), 2.41-2.28 (m, 1H), 2.22 (br d, 1H), 1.90 (q, 1H), 1.25-1.20 (m, 2H), 1.10-1.06 (m, 2H).

### Beispiel 162

### 3-(5-Ethyl-1,2,4-oxadiazol-3-yl)-5-[4-(trifluormethyl)phenyl]piperidin-1-yl(morpholin-4-yl)-methanon [racemisches cis-Isomer]

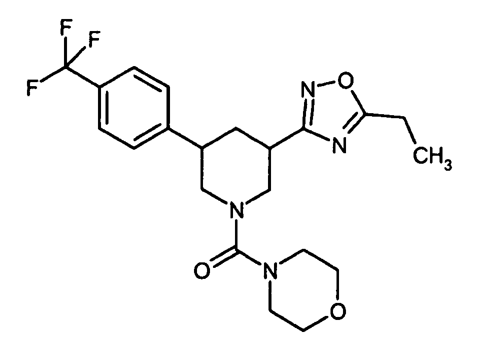

Nach der Allgemeinen Methode 2 wurden 31 mg (0.43 mmol) Propancarbonsäure und 100 mg (ca. 0.22 mmol) der Verbindung aus Beispiel 84A umgesetzt. Ausbeute: 32 mg (34% d. Th.).

LC-MS (Methode 3B): Rₜ = 2.08 min; MS (ESIpos): m/z = 439 [M+H]⁺;

¹H-NMR (400 MHz, DMSO-d₆): δ = 7.69 (d, 2H), 7.57 (d, 2H), 3.93 (br d, 1H), 3.65 (d, 1H), 3.59-3.53 (m, 4H), 3.21-3.11 (m, 5H), 3.07-2.98 (m, 3H), 2.92 (q, 2H), 2.26 (br d, 1H), 1.93 (q, 1H), 1.27 (t, 3H).

### Beispiel 163

### 3-(5-tert-Butyl-1,2,4-oxadiazol-3-yl)-5-[4-(trifluormethyl)phenyl]piperidin-1-yl}(morpholin-4-yl)methanon [racemisches cis-Isomer]

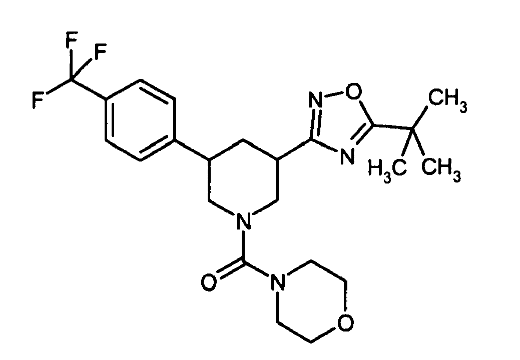

Nach der Allgemeinen Methode 2 wurden 43 mg (0.43 mmol) tert-Butylcarbonsäure und 100 mg (ca. 0.22 mmol) der Verbindung aus Beispiel 84A umgesetzt. Ausbeute: 58 mg (59% d. Th.).

LC-MS (Methode 3B): Rₜ = 2.38 min; MS (ESIpos): m/z = 467 [M+H]⁺;

¹H-NMR (400 MHz, DMSO-d₆): δ = 7.69 (d, 2H), 7.57 (d, 2H), 3.95 (br d, 1H), 3.64 (d, 1H), 3.59-3.53 (m, 4H), 3.22-3.10 (m, 5H), 3.04-2.95 (m, 3H), 2.26 (br d, 1H), 1.92 (q, 1H), 1.37 (s, 9H).

### Beispiel 164

### 3-(5-Benzyl-1,2,4-oxadiazol-3-yl)-5-[4-(trifluormethyl)phenyl]piperidin-1-yl}(morpholin-4-yl)-methanon [racemisches cis-Isomer]

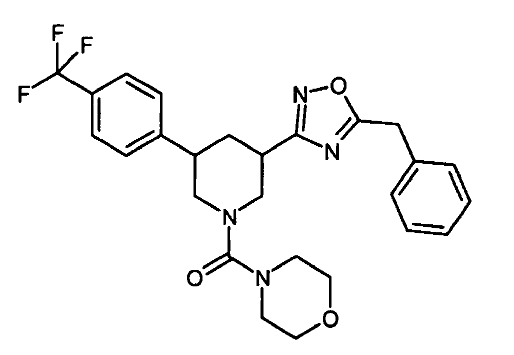

Nach der Allgemeinen Methode 1 wurden 69 mg (0.51 mmol) Phenylessigsäure und 200 mg (ca. 0.43 mmol) der Verbindung aus Beispiel 84A umgesetzt. Ausbeute: 17 mg (7% d. Th.).

LC-MS (Methode 1B): Rₜ = 2.68 min; MS (ESIpos): m/z = 501 [M+H]⁺;

¹H-NMR (400 MHz, DMSO-d₆): δ = 7.69 (d, 2H), 7.56 (d, 2H), 7.38-7.28 (m, 5H), 4.35 (s, 2H), 3.93 (br d, 1H), 3.64 (d, 1H), 3.58-3.52 (m, 4H), 3.21-3.13 (m, 5H), 3.05-2.94 (m, 3H), 2.25 (br d, 1H), 1.92 (q, 1H).

### Beispiel 165

### 3-(5-Cyclobutyl-1,2,4-oxadiazol-3-yl)-5-[4-(trifluormethyl)phenyl]piperidin-1-yl}(morpholin-4-yl)methanon [racemisches cis-Isomer]

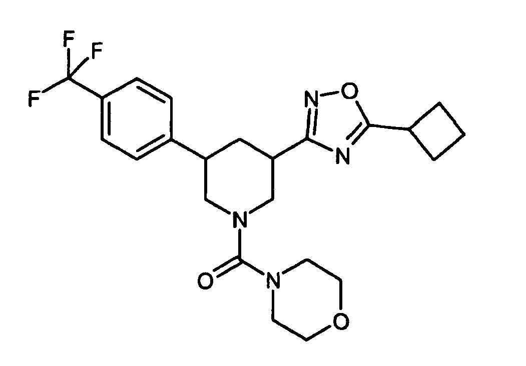

Nach der Allgemeinen Methode 2 wurden 47 mg (0.43 mmol) Cyclobutylcarbonsäure und 100 mg (ca. 0.22 mmol) der Verbindung aus Beispiel 84A umgesetzt. Ausbeute: 47 mg (47% d. Th.).

LC-MS (Methode 3B): Rₜ = 2.28 min; MS (ESIpos): m/z = 465 [M+H]⁺;

¹H-NMR (400 MHz, DMSO-d₆): δ = 7.69 (d, 2H), 7.57 (d, 2H), 3.95 (br d, 1H), 3.83 (quintuplet, 1H), 3.65 (d, 1H), 3.59-3.53 (m, 4H), 3.22-3.12 (m, 5H), 3.08-2.96 (m, 3H), 2.40-2.24 (m, 5H), 2.12-2.01 (m, 1H), 2.99-1.89 (m, 2H).

### Beispiel 166

### tert-Butyl-[1-(3-{1-(morpholin-4-ylcarbonyl)-5-[4-(trifluormethyl)phenyl]piperidin-3-yl}-1,2,4-oxadiazol-5-yl)cyclobutyl]carbamat [racemisches cis-Isomer]

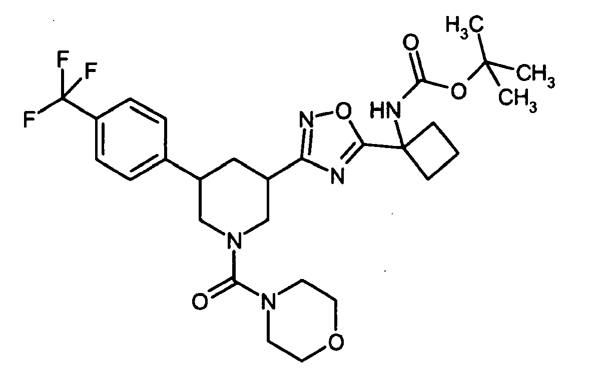

Nach der Allegemeinen Methode 2 wurden 170 mg (0.85 mmol) 1-[(*tert.*-Butoxycarbonyl)amin]cyclobutancarbonsäure und 200 mg (ca. 0.43 mmol) der Verbindung aus Beispiel 84A umgesetzt. Ausbeute: 106 mg (42% d. Th.).

LC-MS (Methode 3B): Rₜ = 2.34 min; MS (ESIpos): m/z = 580 [M+H]⁺;

¹H-NMR (400 MHz, DMSO-d₆): δ = 8.08 (s, 1H), 7.70 (d, 2H), 7.57 (d, 2H), 3.96 (br d, 1H), 3.65 (d, 1H), 3.59-3.55 (m, 4H), 3.21-3.14 (m, 5H), 3.08-2.96 (m, 3H), 2.41-2.33 (m, 2H), 2.27 (br d, 1H), 2.03-1.87 (m, 3H), 1.35 (s, 9H), 1.18-1.12 (m, 2H).

### Beispiel 167

### {3-[5-(2-Aminopropan-2-yl)-1,2,4-oxadiazol-3-yl]-5-[4-(trifluormethyl)phenyl]piperidin-1-yl}-(morpholin-4-yl)methanon Hydrochlorid [racemisches cis-Isomer]

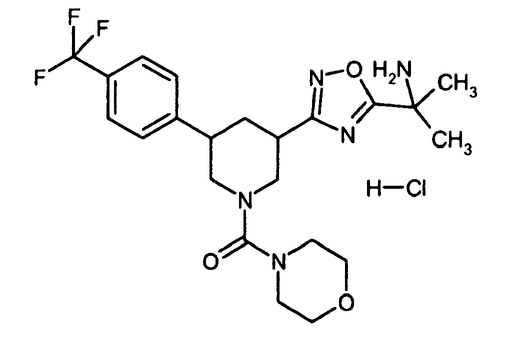

Zu einer Lösung von 29 mg (ca. 0.05 mmol) der Verbindung aus Beispiel 86A in 0.2 ml Dioxan wurden 0.12 ml (0.50 mmol) einer 4N Chlorwasserstoff-Dioxan-Lösung hinzugegeben. Das Reaktionsgemisch wurde 20 Stunden bei Raumtemperatur gerührt. Anschließend wurde die Mischung im Vakuum zur Trockne eingeengt und getrocknet bis zur Gewichtskonstanz. Ausbeute: 24 mg (96% d. Th).

LC-MS (Methode 3B): Rₜ = 1.22 min; MS (ESIpos): m/z = 468 [M+H-HCl]⁺;

¹H-NMR (400 MHz, DMSO-d₆): δ = 8.99 (br s, 1H), 7.71 (d, 2H), 7.56 (d, 2H), 4.01 (br d, 1H), 3.65 (d, 1H), 3.62-3.54 (m, 4H), 3.28-3.18 (m, 5H), 3.07-3.04 (m, 2H), 2.98 (t, 1H), 2.30 (br d, 1H), 1.95 (q, 1H), 1.70 (s, 6H).

### Beispiel 168

### {3-{5-[(1S)-1-Aminoethyl]-1,2,4-oxadiazol-3-yl}-5-[4-(trifluormethyl)phenyl]piperidin-1-yl}-(morpholin-4-yl)methanon Hydrochlorid [racemisches cis-Isomer]

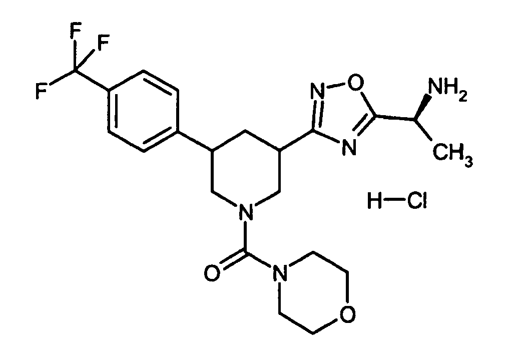

Zu einer Lösung von 25 mg (0.05 mmol) der Verbindung Beispiel 88A in 0.2 ml Dioxan wurden 0.11 ml (0.45 mmol) einer 4N Chlorwasserstoff-Dioxan-Lösung hinzugegeben. Das Reaktionsgemisch wurde 20 Stunden bei Raumtemperatur gerührt. Anschließend wurde die Mischung im Vakuum zur Trockne eingeengt und getrocknet bis zur Gewichtskonstanz. Ausbeute: 20 mg (93% d. Th).

LC-MS (Methode 3B): Rₜ = 1.17 min; MS (ESIpos): m/z = 454 [M+H-HCl]⁺;

¹H-NMR (400 MHz, DMSO-d₆): δ = 8.85 (br s, 1H), 7.73 (d, 2H), 7.56 (d, 2H), 4.93 (m, 1H), 3.99 (br d, 1H), 3.66 (d, 1H), 3.60-3.53 (m, 4H), 3.27 (tt, 1H), 3.26-3.12 (m, 4H), 3.10-3.04 (m, 2H), 2.98 (t, 1H), 2.30 (br d, 1H), 1.96 (q, 1H), 1.60 (d, 3H).

### Beispiel 169

### Morpholin-4-yl {3-[5-(propan-2-yl)-1,2,4-oxadiazol-3-yl]-5-[4-(trifluormethyl)phenyl]piperidin-1-yl}methanon [racemisches cis-Isomer]

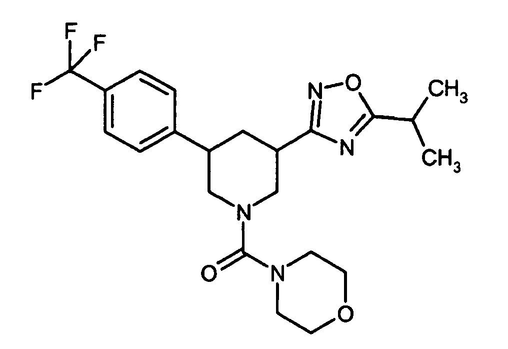

Nach der Allgemeinen Methode 2 wurden 26 mg (0.297 mmol) 2-Methylpropancarbonsäure und 70 mg (ca. 0.15 mmol) der Verbindung aus Beispiel 84A umgesetzt. Ausbeute: 9 mg (42% d. Th.).

LC-MS (Methode 2B): Rₜ = 1.36 min; MS (ESIpos): m/z = 453 [M+H]⁺;

¹H-NMR (400 MHz, DMSO-d₆): δ = 7.69 (d, 2H), 7.57 (d, 2H), 3.94 (br d, 1H), 3.65 (d, 1H), 3.59-3.53 (m, 4H), 3.30-3-23 (m, 1H), 3.22-3.13 (m, 5H), 3.06-2.95 (m, 3H), 2.26 (br d, 1H), 1.92 (q, 1H), 1.50 (d, 6H).

### Beispiel 170

### {3-{5-[(Dimethylamino)methyl]-1,2,4-oxadiazol-3-yl}-5-[4-(trifluormethyl)phenyl]piperidin-1-yl}-(morpholin-4-yl)methanon Hydrochlorid [enantiomerenreines cis-Isomer]

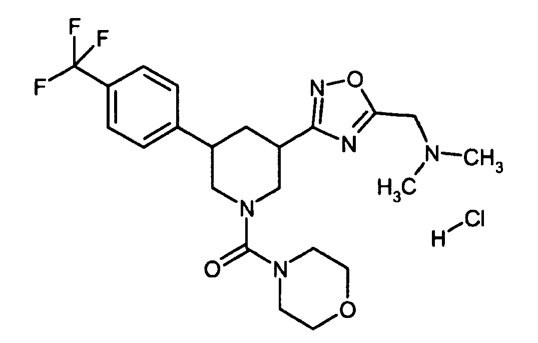

Nach der Allgemeinen Methode 1 wurden 10 mg (0.09 mmol) *N,N*-Dimethylglicin und 83 mg (ca. 0.09 mmol) der Verbindung aus Beispiel 84A umgesetzt. Enantiomerentrennung von 20 mg des Racemates nach Methode 8D ergab 5 mg der Titelverbindung aus Beispiel 170 und 4 mg der Titelverbindung aus Beispiel 171.

HPLC (Methode 6E): Rₜ = 12.56 min, >99% ee;

LC-MS (Methode 3B): Rₜ = 1.26 min; MS (ESIpos): m/z = 468[M+H]⁺;

¹H-NMR (400 MHz, DMSO-d₆): δ = 7.70 (d, 2H), 7.57 (d, 2H), 3.95 (brd, 1H), 3.81 (d, 2H), 3.66 (d, 1H), 3.59-3-52 (m, 4H), 3.22-3-17 (m, 4H), 3.07-2.97 (m, 3H), 2.31 (d, 1H), 2.24 (s, 6H), 1.94 (q, 1H).

### Beispiel 171

### {3-{5-[(Dimethylamino)methyl]-1,2,4-oxadiazol-3-yl}-5-[4-(trifluormethyl)phenyl]piperidin-1-yl}-(morpholin-4-yl)methanon Hydrochlorid [enantiomerenreines cis-Isomer]

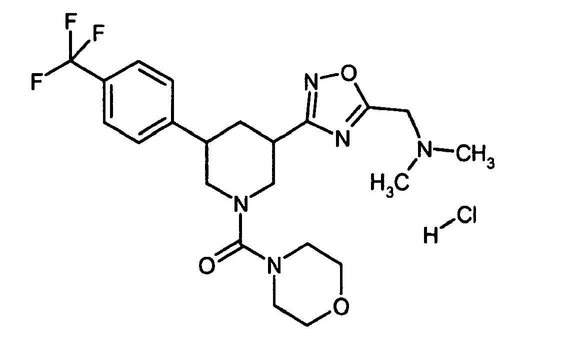

Nach der Allgemeinen Methode 1 wurden 10 mg (0.09 mmol) *N,N*-Dimethylglicin und 83 mg (ca. 0.09 mmol) der Verbindung aus Beispiel 84A umgesetzt. Enantiomerentrennung von 20 mg des Racemates nach Methode 8D ergab 5 mg der Titelverbindung aus Beispiel 170 und 4 mg der Titelverbindung aus Beispiel 171.

HPLC (Methode 6E): Rₜ = 24.18 min, >99% ee;

LC-MS (Methode 3B): Rₜ = 1.26 min; MS (ESIpos): m/z = 468[M+H]⁺;

¹H-NMR (400 MHz, DMSO-d₆): δ = 7.70 (d, 2H), 7.57 (d, 2H), 3.95 (br d, 1H), 3.81 (d, 2H), 3.66 (d, 1H), 3.59-3-52 (m, 4H), 3.22-3-17 (m, 4H), 3.07-2.97 (m, 3H), 2.31 (d, 1H), 2.24 (s, 6H), 1.94 (q, 1H).

### Beispiel 172

### {3-[5-(2-Methoxyethyl)-1,2,4-oxadiazol-3-yl]-5-[4-(trifluormethyl)phenyl]piperidin-1-yl}-(morpholin-4-yl)methanon [enantiomerenreines cis-Isomer]

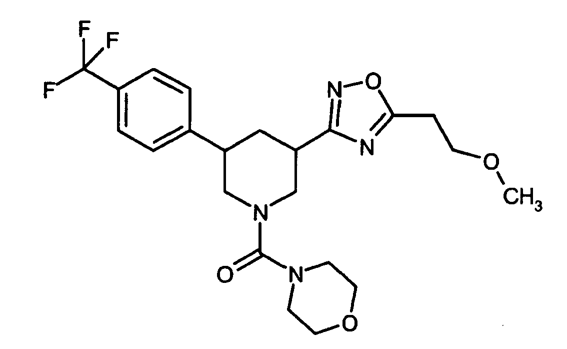

Nach der Allgemeinen Methode 1 wurden 53 mg (0.51 mmol) 3-Methoxypropancarbonsäure und 200 mg (ca. 0.42 mmol) der Verbindung aus Beispiel 84A umgesetzt. Enantiomerentrennung von 30 mg des Racemates nach Methode 8D ergab 11 mg der Titelverbindung aus Beispiel 172 und 11 mg der Titelverbindung aus Beispiel 173.

HPLC (Methode 6E): Rₜ = 12.23 min, >99% ee;

LC-MS (Methode 3B): Rₜ = 1.98 min; MS (ESIpos): m/z = 469[M+H]⁺;

¹H-NMR (400 MHz, DMSO-d₆): δ = 7.70 (d, 2H), 7.57 (d, 2H), 3.95 (br d, 1H), 3.72 (t, 2H), 3.65 (d, 1H), 3.59-3-53 (m, 4H), 3.24 (s, 3H), 3.25-3.14 (m, 7H), 3.05-2.95 (m, 3H), 2.26 (br d, 1H), 1.93 (q, 1H).

### Beispiel 173

### {3-[5-(2-Methoxyethyl)-1,2,4-oxadiazol-3-yl]-5-[4-(trifluormethyl)phenyl]piperidin-1-yl}-(morpholin-4-yl)methanon [enantiomerenreines cis-Isomer]

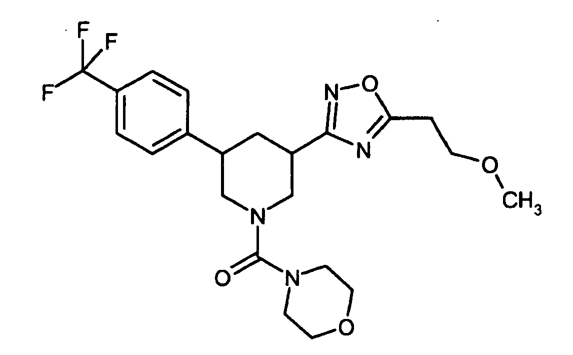

Nach der Allgemeinen Methode 1 wurden 53 mg (0.51 mmol) 3-Methoxypropancarbonsäure und 200 mg (ca. 0.43 mmol) der Verbindung aus Beispiel 84A umgesetzt. Enantiomerentrennung von 30 mg des Racemates nach Methode 8D ergab 11 mg der Titelverbindung aus Beispiel 172 und 11 mg der Titelverbindung aus Beispiel 173

HPLC (Methode 6E): Rₜ = 19.17 min, >99% ee;

LC-MS (Methode 3B): Rₜ = 1.98 min; MS (ESIpos): m/z = 469[M+H]⁺;

¹H-NMR (400 MHz, DMSO-d₆): δ = 7.70 (d, 2H), 7.57 (d, 2H), 3.95 (br d, 1H), 3.72 (t, 2H), 3.65 (d, 1H), 3.59-3-53 (m, 4H), 3.24 (s, 3H), 3.25-3.14 (m, 7H), 3.05-2.95 (m, 3H), 2.26 (br d, 1H), 1.93 (q, 1H).

### Beispiels 174

### {3-[5-(1-Aminocyclobutyl)-1,2,4-oxadiazol-3-yl]-5-[4-(trifluormethyl)phenyl]piperidin-1-yl}-(morpholin-4-yl)methanon Hydrochlorid [racemisches cis-Isomer]

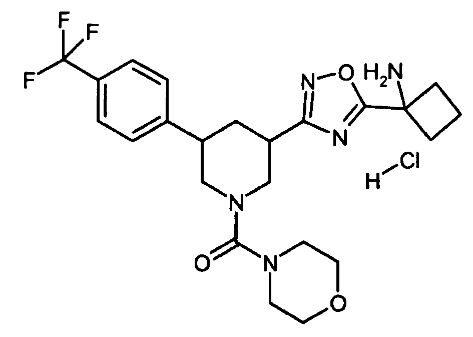

Zu einer Lösung von 100 mg (0.17 mmol) der Verbindung aus Beispiel 166 in 0.25 ml Dioxan wurden 0.4 ml (1.7 mmol) einer 4N Chlorwasserstoff-Dioxan-Lösung hinzugegeben. Das Reaktionsgemisch wurde 20 h bei Raumtemperatur gerührt. Die Mischung wurde im Vakuum eingeengt, erneut in 0.25 ml Dioxan aufgenommen, und noch einmal mit 0.4 ml (1.7 mmol) einer 4N Chlorwasserstoff-Dioxan-Lösung versetzt. Das Reaktionsgemisch wurde 16 h bei Raumtemperatur gerührt. Anschließend wurde die Mischung im Vakuum zur Trockne eingeengt und getrocknet bis zur Gewichtskonstanz. Ausbeute: 92 mg (100% d. Th).

LC-MS (Methode 1B): Rₜ = 1.51 min; MS (ESIpos): m/z = 480 [M+H-HCl]⁺.

### Beispiel 175

### {3-[5-(1-Aminocyclopropyl)-1,2,4-oxadiazol-3-yl]-5-[4-(trifluormethyl)phenyl]piperidin-1-yl}-(morpholin-4-yl)methanon Hydrochlorid [racemisches cis-Isomer]

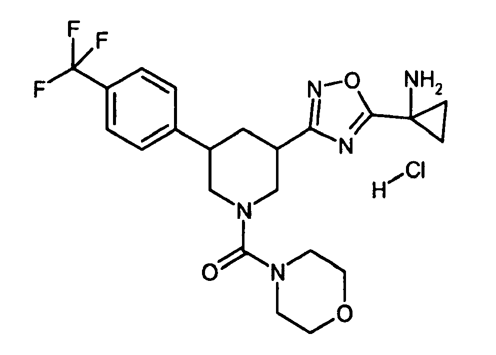

Zu einer Lösung von 130 mg (0.230 mmol) der Verbindung aus Beispiel 85A in 0.25 ml Dioxan wurden 0.6 ml (2.3 mmol) einer 4N Chlorwasserstoff-Dioxan-Lösung hinzugegeben. Das Reaktionsgemisch wurde 20 h bei Raumtemperatur gerührt. Die Mischung wurde im Vakuum eingeengt, erneut in 0.25 ml Dioxan aufgenommen, und noch einmal mit 0.6 ml (2.3 mmol) einer 4N Chlorwasserstoff-Dioxan-Lösung versetzt. Das Reaktionsgemisch wurde 16 h bei Raumtemperatur gerührt. Anschließend wurde die Mischung im Vakuum zur Trockne eingeengt und getrocknet bis zur Gewichtskonstanz. Ausbeute: 115 mg (92% d. Th).

LC-MS (Methode 1B): Rₜ = 1.65 min; MS (ESIpos): m/z = 466 [M+H-HCl]⁺.

### Beispiel 176

### {3-[5-(2-Ethoxypropan-2-yl)-1,2,4-oxadiazol-3-yl]-5-[4-(trifluormethyl)phenyl]piperidin-1-yl}-(morpholin-4-yl)methanon [racemisches cis-Isomer]

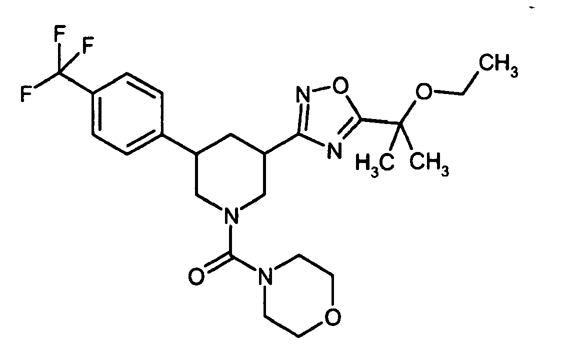

Nach der Allgemeinen Methode 2 wurden 57 mg (0.43 mmol) 2-Ethoxy-2-methylpropansäure und 120 mg (ca. 0.22 mmol) der Verbindung aus Beispiel 84A umgesetzt. Ausbeute: 24 mg (22% d. Th.).

LC-MS (Methode2B): Rₜ = 1.40 min; MS (ESIpos): m/z = 497 [M+H]⁺;

¹H-NMR (400 MHz, DMSO-d₆): δ = 7.70 (d, 2H), 7.57 (d, 2H), 3.97 (br d, 1H), 3.65 (d, 1H), 3.59-3.53 (m, 4H), 3.29 (q, 2H), 3.24-3.17 (m, 5H), 3.06-2.96 (m, 3H), 2.29 (br d, 1H), 1.92 (q, 1H), 1.58 (s, 6H), 1.06 (t, 3H).

### Beispiel 177

### {3-{5-[(Methylsulfonyl)methyl]-1,2,4-oxadiazol-3-yl}-5-[4-(trifluormethyl)phenyl]piperidin-1-yl}-(morpholin-4-yl)methanon [racemisches cis-Isomer]

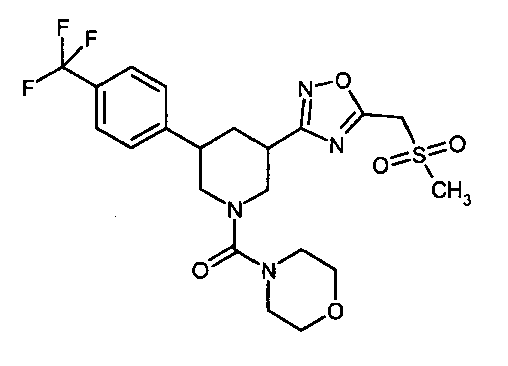

Nach der Allgemeinen Methode 2 wurden 60 mg (0.43 mmol) (Methylsulfonyl)essigsäure und 120 mg (ca. 0.216 mmol) der Verbindung aus Beispiel 84A umgesetzt. Ausbeute: 41 mg (38% d. Th.).

LC-MS (Methode 3B): Rₜ = 1.84 min; MS (ESIpos): m/z = 503 [M+H]⁺;

¹H-NMR (400 MHz, DMSO-d₆): δ = 7.70 (d, 2H), 7.57 (d, 2H), 5.20 (d, 2H), 3.98 (brd, 1H), 3.65 (d, 1H), 3.59-3.53 (m, 4H), 3.28-3.17 (8H), 3.10-2.98 (m, 3H), 2.30 (br d, 1H), 1.96 (q, 1H).

### Beispiel 178

### {3-[3-(2-Methoxyethyl)-1,2,4-oxadiazol-5-yl]-5-[4-(trifluormethoxy)phenyl]piperidin-1-yl}-(morpholin-4-yl)methanon [enantiomerenreines cis-Isomer]

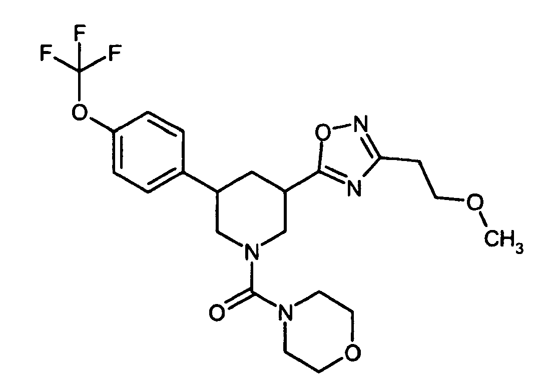

Die Enantiomerentrennung von 1.25 g (2.58 mmol) der Verbindung aus Beispiel 250 nach Methode 9D ergab 551 mg der Titelverbindung aus Beispiel 178 und 493 mg der Titelverbindung aus Beispiel 179.

HPLC (Methode 9E): Rₜ = 6.97 min, >99.0% ee;

LC-MS (Methode 3B): Rₜ = 2.01 min; MS (ESIpos): m/z = 485 [M+H]⁺;

¹H-NMR (400 MHz, DMSO-d₆): δ = 7.47 (d, 2H), 7.33 (d, 2H), 3.99 (br d, 1H), 3.68 (t, 2H), 3.61 (br d, 1H), 57-3.55 (m, 4H), 3.40-3.36 (m, 1H), 3.23 (s, 3H), 3.21-3.19 (m, 4H), 3.06-2.99 (m, 3H), 2.93 (t, 2H), 2.33-2.30 (m, 1H), 1.97 (q, 1H).

[α]₃₆₅ ²⁰ =-3.4, Methanol

### Beispiel 179

### {3-[3-(2-Methoxyethyl)-1,2,4-oxadiazol-5-yl]-5-[4-(trifluormethoxy)phenyl]piperidin-1-yl}-(morpholin-4-yl)methanon [enantiomerenreines cis-Isomer]

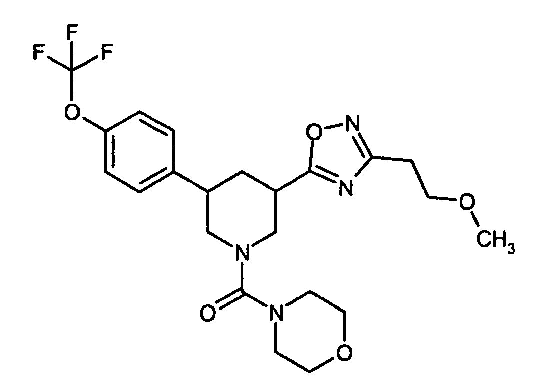

Die Enantiomerentrennung von 1.25 g (2.58 mmol) der Verbindung aus Beispiel 250 nach Methode 9D ergab 551 mg der Titelverbindung aus Beispiel 178 und 493 mg der Titelverbindung aus Beispiel 179.

HPLC (Methode 9E): Rₜ = 8.24 min, >99.0% ee;

LC-MS (Methode 3B): Rₜ = 2.01 min; MS (ESIpos): m/z = 485 [M+H]⁺.

¹H-NMR (400 MHz, DMSO-d₆): δ = 7.48 (d, 2H), 7.33 (d, 2H), 3.99 (br d, 1H), 3.68 (t, 2H), 3.61 (br d, 1H), .58-3.55 (m, 4H), 3.47-3.36 (m, 1H), 3.23 (s, 3H), 3.21-3.19 (m, 4H), 3.06-2.97 (m, 3H), 2.93 (t, 2H), 2.31 (br d, 1H), 1.97 (q, 1H).

### Beispiels 180

### {3-(3-Cyclopropyl-1,2,4-oxadiazol-5-yl)-5-[4-(trifluormethoxy)phenyl]piperidin-1-yl}(4,4-difluorpiperidin-1-yl)methanon [racemisches cis-Isomer]

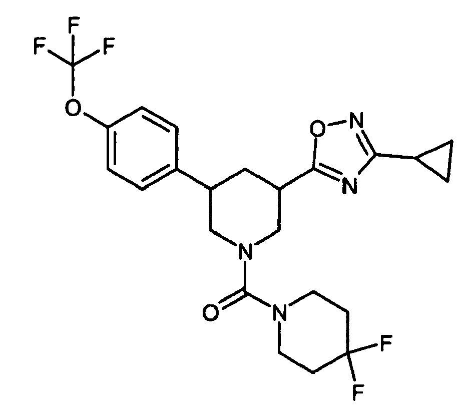

Nach der Allgemeinen Methode 6 wurden 100 mg (0.19 mmol) der Verbindung aus Beispiel 176A und 91 mg (0.58 mmol) 4,4-Difluorpiperidin-Hydrochlorid umgesetzt. Ausbeute: 48 mg (50% d. Th.)

LC-MS (Methode 3B): Rₜ = 2.01 min; MS (ESIpos): m/z = 413 [M+H]⁺;

¹H-NMR (400 MHz, DMSO-d₆): δ = 7.46 (d, 2H), 7.32 (d, 2H), 3.97 (br d, 1H), 3.64 (br d, 1H), 3.39-3.34 (m, 5H), 3.31-3.28 (m, 4H), 3.04-2.97 (m, 2H), 2.31 (br d, 1H), 2.14-2.07 (m, 1H), 2.00-1.86 (m, 2H), 1.08-1.03 (m, 2H), 1.90-1.86 (m, 2H).

### Beispiel 181

### {3-(3-Cyclopropyl-1,2,4-oxadiazol-5-yl)-5-[4-(trifluormethyl)phenyl]piperidin-1-yl}(3-methylazetidin-1-yl)methanon [racemisches cis-Isomer]

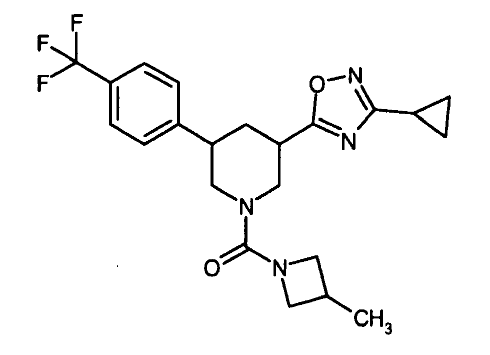

Nach der Allgemeinen Methode 6 wurden 95 mg (0.19 mmol) der Verbindung aus Beispiel 184A und 61 mg (0.57 mmol) 3-Methylazetidin-Hydrochlorid umgesetzt. Die Aufreinigung des Rohproduktes erfolgte zunächst säulenchromatographisch an Kieselgel (Laufmittel: Dichlormethan -> Dichlormethan/Methanol 100:5) und zusätzlich mittels präparativer HPLC. Ausbeute: 14 mg (17% d. Th.)

HPLC (Methode 2A): Rₜ = 4.83 min; MS (ESIpos): m/z = 435 [M+H]⁺;

¹H-NMR (400 MHz, DMSO-d₆): δ = 7.69 (d, 2H), 7.55 (d, 2H), 4.11 (br d, 1H), 4.05-4.00 (m, 2H), 3.75 (br d, 1H), 3.50 (q, 2H), 3.27-3.22 (m, 1H), 3.00-2.91 (m, 3H), 2.61-2.57 (m, 1H), 2.27 (br d, 1H), 2.14-2.07 (m, 1H), 2.03-1.94 (m, 1H), 1.14 (d, 3H). 1.07-1.03 (m, 2H), 0.90-0.86 (m, 2H).

### Beispiel 182

### {3-[3-(3-Fluorphenyl)-1,2,4-oxadiazol-5-yl]-5-[4-(trifluormethyl)phenyl]piperidin-1-yl}(3-hydroxypyrrolidin-1-yl)methanon [Diastereomerengemisch]

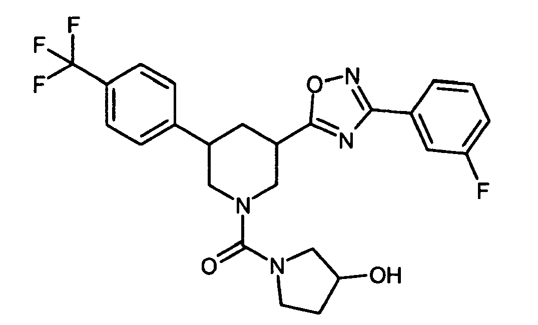

Nach der Allgemeinen Methode 6 wurden 100 mg (0.18 mmol) der Verbindung aus Beispiel 187A und 47 mg (0.54 mmol) 3-Pyrrolidinol umgesetzt. Ausbeute: 25 mg (26% d. Th.)

HPLC (Methode 2A): Rₜ = 4.74 min; MS (ESIpos): m/z = 505 [M+H]⁺;

¹H-NMR (400 MHz, DMSO-d₆): δ = 7.87 (d, 1H), 7.77 (d, 1H), 7.71 (d, 2H), 7.66-7.63 (m, 1H), 7.59 (d, 2H), 7.49-7.44 (m, 1H), 4.89 (d, OH), 4.22 (br s, 1H), 4.16 (br d, 1H), 3.74 (t, 1H), 3.57-3.47 (m, 3H), 3.39-3.33 (m, 1H), 3.16-2.92 (m, 4H), 2.45 (br d, 1H), 2.13-2.08 (m, 1H), 2.84-2.81 (m, 1H), 2.76-2.72 (m, 1H).

### Beispiel 183

### {3-(4-Ethylphenyl)-5-[3-(2-methoxyethyl)-1,2,4-oxadiazol-5-yl]piperidin-1-yl}(4-hydroxypiperidin-1-yl)methanon [racemisches cis-Isomer]

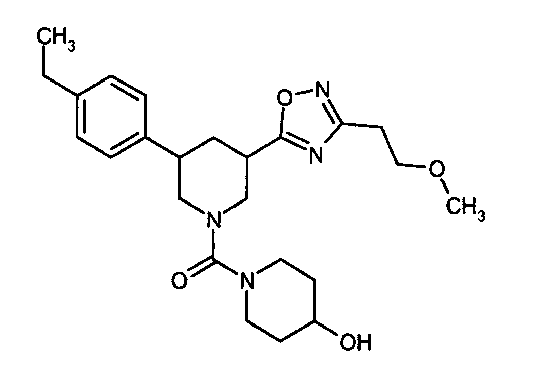

Nach der Allgemeinen Methode 2 wurden 60 mg (0.17 mmol) der Verbindung aus Beispiel 59A und 30 mg (0.25 mmol) *N'*-Hydroxy-3-methoxypropanimidamid umgesetzt. Ausbeute: 32 mg (44% d. Th.)

LC-MS (Methode 1B): Rₜ = 2.15 min; MS (ESIpos): m/z = 443 [M+H]⁺;

¹H-NMR (400 MHz, DMSO-d₆): δ = 7.21 (d, 2H), 7.16 (d, 2H), 4.67 (d, OH), 3.93 (br d, 1H), 3.67 (t, 2H), 3.59-3.45 (m, 4H), 3.42-3.34 (m, 1H), 3.32 (s, 3H), 3.00-2.85 (m, 7H), 2.57 (q, 2H), 2.30 (br d, 1H), 1.94 (q, 1H), 1.73-1.70 (m, 2H), 1.34-1.25 (m, 2H), 1.16 (t, 3H).

### Beispiel 184

### (3-Hydroxyazetidin-1-yl){3-[3-(2-ethoxyethyl)-1,2,4-oxadiazol-5-yl]-5-[4-(trifluoromethyl)phenyl]-piperidin-1-yl}methanon [racemisches cis-Isomer]

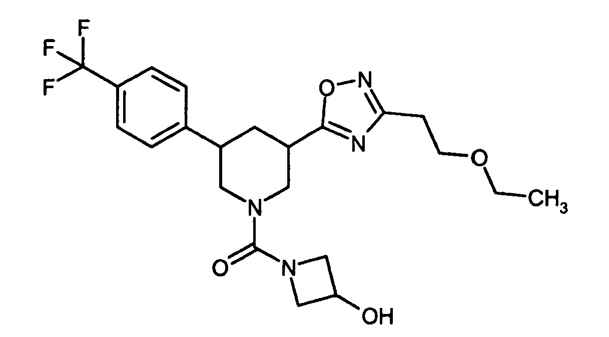

Nach der Allgemeinen Methode 2 wurden 100 mg (0.27 mmol) der Verbindung aus Beispiel 101A und 58 mg (0.40 mmol) der Verbindung aus Beispiel 76A umgesetzt. Ausbeute: 25 mg (19% d. Th.)

HPLC (Methode 2A): Rₜ = 4.29 min; MS (ESIpos): m/z = 469 [M+H]⁺;

¹H-NMR (400 MHz, DMSO-d₆): δ = 7.70 (d, 2H), 7.56 (d, 2H), 5.58 (d, OH), 4.38 (sextett, 1H), 4.14 (br d, 1H), 4.08 (q, 2H), 3.78 (br d, 1H), 3.72-3.67 (m, 4H), 3.42 (q, 2H), 3.06-2.92 (m, 3H), 2.90 (t, 2H), 2.32 (br d, 1H), 2.02 (q, 1H), 1.06 (t, 3H).

### Beispiel 185

### [3-(4-Ethylphenyl)-5-{3-[(methylsulfonyl)methyl]-1,2,4-oxadiazol-5-yl}piperidin-1-yl](morpholin-4-yl)methanon [racemisches cis-Isomer]

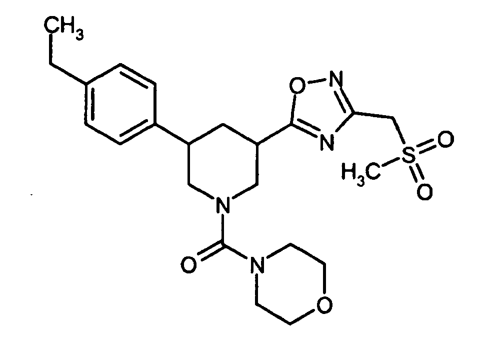

Nach der Allgemeinen Methode 2 wurden 60 mg (0.17 mmol) der Verbindung aus Beispiel 38A und 40 mg (0.26 mmol) *N'*-Hydroxy-2-(methylsulfonyl)ethanimidamid umgesetzt. Ausbeute: 15 mg (18% d. Th.)

LC-MS (Methode 3B): Rₜ = 1.81 min; MS (ESIpos): m/z = 463 [M+H]⁺.

¹H-NMR (400 MHz, DMSO-d₆): δ = 7.25 (d, 2H), 7.17 (d, 2H), 4.85 (s, 2H), 4.04 (br d, 1H), 3.60-3.55 (m, 5H), 3.49-3.42 (m, 1H), 3.23-3.18 (m, 4H), 3.16 (s, 3H), 3.12-2.84 (m, 3H), 2.57 (q, 2H), 2.36 (br d, 1H), 1.97 (q, 1H), 1.16 (t, 3H).

### Beispiel 186

### {3-(3,4-Dimethylphenyl)-5-[3-(2-methoxyethyl)-1,2,4-oxadiazol-5-yl]piperidin-1-yl}(4-hydroxypiperidin-1-yl)methanon [racemisches cis-Isomer]

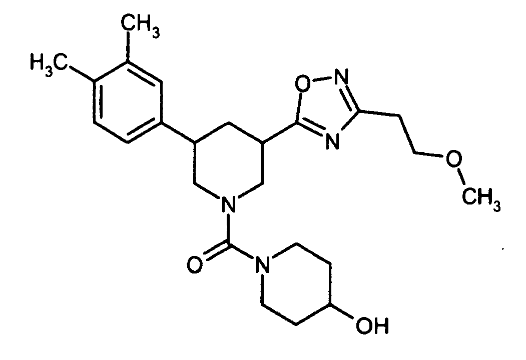

Nach der Allgemeinen Methode 2 wurden 200 mg (0.54 mmol) der Verbindung aus Beispiel 128A und 128 mg (0.81 mmol) *N'*-Hydroxy-3-methoxypropanimidamid umgesetzt. Ausbeute: 138 mg (56% d. Th.)

LC-MS (Methode 2B): Rₜ = 1.14 min; MS (ESIpos): m/z = 443 [M+H]⁺.

¹H-NMR (400 MHz, DMSO-d₆): δ = 7.09-7.07 (m, 2H), 6.68 (d, 1H), 4.68 (d, OH), 3.94 (br d, 1H), 3.67 (t, 2H), 3.63-3.57 (m, 1H), 3.53-3.45 (m, 3H), 3.40-3.34 (m, 1H), 3.23 (s, 3H), 3.00-2.86 (m, 6H), 2.83-2.77 (m, 1H), 2.26 (br d, 1H), 2.20 (s, 3H), 2.18 (s, 3H), 1.93 (q, 1H), 1.73-1.69 (m, 2H), 1.34-1.25 (m, 2H).

### Beispiel 187

### {3-(4-Ethylphenyl)-5-[3-(2-hydroxypropan-2-yl)-1,2,4-oxadiazol-5-yl]piperidin-1-yl}(4-hydroxypiperidin-1-yl)methanon [racemisches cis-Isomer]

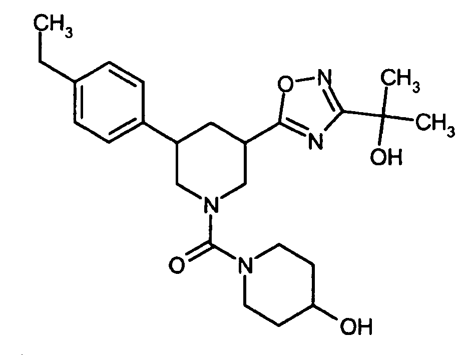

Nach der Allgemeinen Methode 2 wurden 60 mg (0.17 mmol) der Verbindung aus Beispiel 59A und 31 mg (0.25 mmol) der Verbindung aus Beispiel 66A umgesetzt. Ausbeute: 43 mg (55% d. Th.)

LC-MS (Methode 5B): Rₜ = 2.01 min; MS (ESIpos): m/z = 443 [M+H]⁺.

¹H-NMR (400 MHz, DMSO-d₆): δ = 7.22 (d, 2H), 7.17 (d, 2H), 5.53 (s, OH), 4.67 (d, OH), 3.95 (br d, 1H), 3.62-3.41 (m, 4H), 3.41-3.34 (m, 1H), 3.01-2.82 (m, 5H), 2.57 (q, 2H), 2.30 (br d, 1H), 1.95 (q, 1H), 1.77-1.70 (m, 2H), 1.47 (s, 6H), 1.34-1.25 (m, 2H), 1.17 (t, 3H).

### Beispiel 188

### {3-(3,4-Dimethylphenyl)-5-[3-(2-methoxyethyl)-1,2,4-oxadiazol-5-yl]piperidin-1-yl}(morpholin-4-yl)methanon [racemisches cis-Isomer]

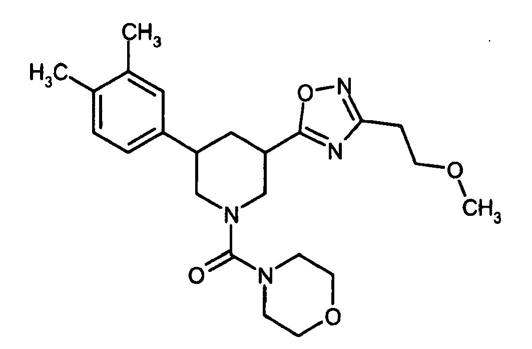

Nach der Allgemeinen Methode 2 wurden 200 mg (0.58 mmol) der Verbindung aus Beispiel 130A und 136 mg (0.87 mmol) *N*'-Hydroxy-3-methoxypropanimidamid umgesetzt. Ausbeute: 98 mg (40% d. Th.)

LC-MS (Methode 1B): Rₜ = 2.27 min; MS (ESIpos): m/z = 429 [M+H]⁺;

¹H-NMR (400 MHz, DMSO-d₆): δ = 7.09-7-07 (m, 2H), 7.00 (d, 1H), 3.97 (br d, 1H), 3.67 (t, 2H), 3.58-3.54 (m, 5H), 3.41-3.34 (m, 1H), 3.23 (s, 3H), 3.19-3.17 (m, 4H), 3.05-2.89 (m, 4H), 2.84-2.77 (m, 1H), 2.25 (br d, 1H), 2.20 (s, 3H), 2.18 (s, 3H), 1.95 (q, 1H).

### Beispiel 189

### {3-(3,4-Dimethylphenyl)-5-[3-(2-methoxyethyl)-1,2,4-oxadiazol-5-yl]piperidin-1-yl}(morpholin-4-yl)methanon [enantiomerenreines cis-Isomer]

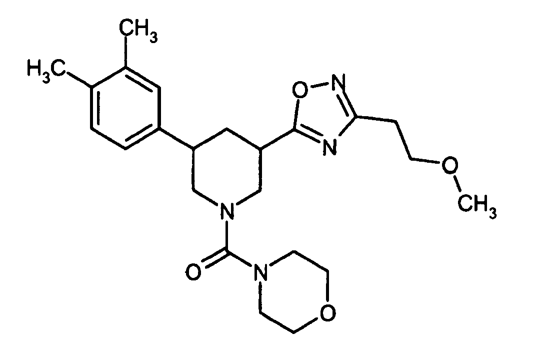

Die Enantiomerentrennung von 98 mg (0.2 mmol) der Verbindung aus Beispiel 188 nach Methode 17D ergab 30 mg der Titelverbindung aus Beispiel 189 und 25 mg der Titelverbindung aus Beispiel 190.

HPLC (Methode 13E): Rₜ = 7.40 min, >99.0% ee;

LC-MS (Methode 3B): Rₜ = 1.92 min; MS (ESIpos): m/z = 429 [M+H]⁺;

¹H-NMR (400 MHz, DMSO-d₆): δ = 7.08-7-07 (m, 2H), 7.01 (d, 1H), 3.98 (br d, 1H), 3.68 (t, 2H), 3.58-3.54 (m, 5H), 3.39-3.34 (m, 1H), 3.23 (s, 3H), 3.20-3.17 (m, 4H), 3.04-2.90 (m, 4H), 2.83-2.77 (m, 1H), 2.26 (br d, 1H), 2.20 (s, 3H), 2.18 (s, 3H), 1.94 (q, 1H).

### Beispiel 190

### {3-(3,4-Dimethylphenyl)-5-[3-(2-methoxyethyl)-1,2,4-oxadiazol-5-yl]piperidin-1-yl}(morpholin-4-yl)methanon [enantiomerenreines cis-Isomer]

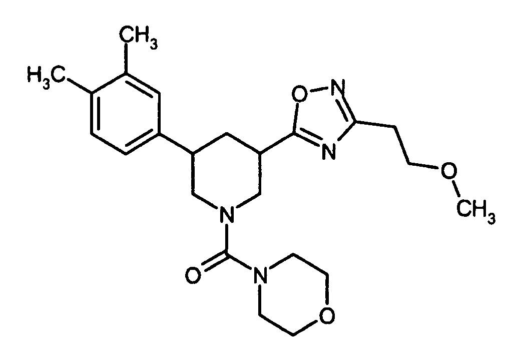

Die Enantiomerentrennung von 98 mg (0.2 mmol) der Verbindung aus Beispiel 188 nach Methode 17D ergab 30 mg der Titelverbindung aus Beispiel 189 und 25 mg der Titelverbindung aus Beispiel 190.

HPLC (Methode 13E): Rₜ = 9.82 min, >99.0% ee;

LC-MS (Methode 3B): Rₜ = 1.92 min; MS (ESIpos): m/z = 429 [M+H]⁺;

¹H-NMR (400 MHz, DMSO-d₆): δ = 7.09-7.07 (m, 2H), 7.01 (d, 1H), 3.99 (br d, 1H), 3.68 (t, 2H), 3.58-3.55 (m, 5H), 3.38-3.34 (m, 1H), 3.23 (s, 3H), 3.20-3.18 (m, 4H), 3.10-2.89 (m, 4H), 2.84-2.78 (m, 1H), 2.26 (br d, 1H), 2.20 (s, 3H), 2.18 (s, 3H), 1.95 (q, 1H).

### Beispiel 191

### 1-({3-[3-(2-Ethoxyethyl)-1,2,4-oxadiazol-5-yl]-5-[4-(trifluormethyl)phenyl]piperidin-1-yl}-carbonyl)piperidin-4-carbonitril [racemisches cis-Isomer]

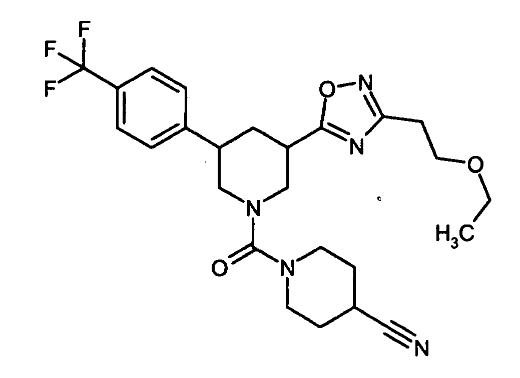

Nach der Allgemeinen Methode 2 wurden 100 mg (0.24 mmol) der Verbindung aus Beispiel 100A und 53 mg (0.37 mmol) der Verbindung aus Beispiel 76A umgesetzt. Ausbeute: 89 mg (72% d. Th.)

HPLC (Methode 2A): Rₜ = 4.63 min; MS (ESIpos): m/z = 506 [M+H]⁺;

¹H-NMR (400 MHz, DMSO-d₆): δ = 7.70 (d, 2H), 7.57 (d, 2H), 3.96 (br d, 1H), 3.71 (t, 2H), 3.59 (br d, 1H), 3.46-3.35 (m, 5H), 3.10-2.97 (m, 6H), 2.92 (t, 2H), 2.32 (br d, 1H), 2.01 (q, 1H), 1.88-1.85 (m, 2H), 1.71-1.64 (m, 2H), 1.06 (t, 3H).

### Beispiel 192

### 1-({3-(4-Ethylphenyl)-5-[3-(2-methoxyethyl)-1,2,4-oxadiazol-5-yl]piperidin-1-yl}carbonyl)-piperidin-4-carbonitril [racemisches cis-Isomer]

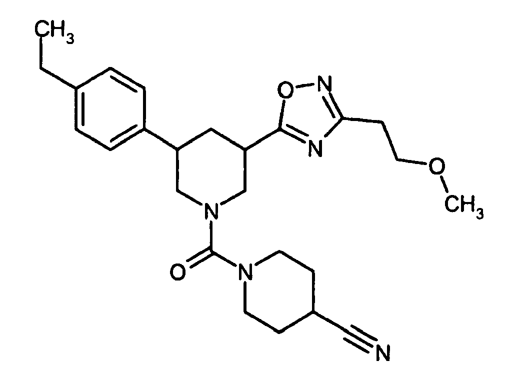

Nach der Allgemeinen Methode 2 wurden 60 mg (0.16 mmol) der Verbindung aus Beispiel 100A und 30 mg (0.37 mmol) *N*'-Hydroxy-3-methoxypropanimidamid umgesetzt. Ausbeute: 42 mg (55% d. Th.)

LC-MS (Methode 5B): Rₜ = 2.34 min; MS (ESIpos): m/z = 452 [M+H]⁺;

¹H-NMR (400 MHz, DMSO-d₆): δ = 7.22 (d, 2H), 7.17 (d, 2H), 3.96 (br d, 1H), 3.67 (t, 2H), 3.55 (br d, 1H), 3.41-3.29 (m, 3H), 3.23 (s, 3H), 3.09-2.97 (m, 4H), 2.95-2.82 (m, 4H), 2.57 (q, 2H), 2.29 (br d, 1H), 1.95 (q, 1H), 1.90-1.84 (m, 2H), 1.70-1.64 (m, 2H), 1.16 (t, 3H).

### Beispiel 193

### {3-[3-(2-Methoxyethyl)-1,2,4-oxadiazol-5-yl]-5-[3-(trifluormethoxy)phenyl]piperidin-1-yl}-(morpholin-4-yl)methanon [racemisches cis-Isomer]

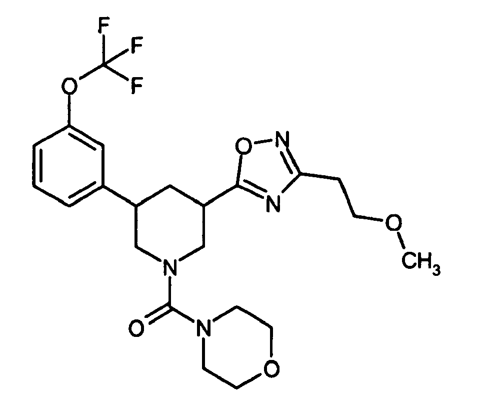

Nach der Allgemeinen Methode 2 wurden 200 mg (0.50 mmol) der Verbindung aus Beispiel 201A und 117 mg (0.75 mmol) *N*'-Hydroxy-3-methoxypropanimidamid umgesetzt. Ausbeute: 106 mg (44% d. Th.)

LC-MS (Methode 3B): Rₜ = 2.00 min; MS (ESIpos): m/z = 485 [M+H]⁺;

¹H-NMR (400 MHz, DMSO-dₛ): δ = 7.50-7.46 (m, 1H), 7.40-7.36 (m, 2H), 7.25 (d, 1H), 3.98 (br d, 1H), 3.68 (t, 2H), 3.62-3.55 (m, 5H), 3.42-3.36 (m, 1H), 3.23 (s, 3H), 3.21-3.19 (m, 4H), 3.09-3.00 (m, 3H), 2.93 (t, 2H), 2.34 (br d, 1H), 2.00 (q, 1H).

### Beispiel 194

### {3-[3-(2-Methoxyethyl)-1,2,4-oxadiazol-5-yl]-5-[3-(trifluoromethoxy)phenyl]piperidin-1-yl}-(morpholin-4-yl)methanon [enantiomerenreines cis-Isomer]

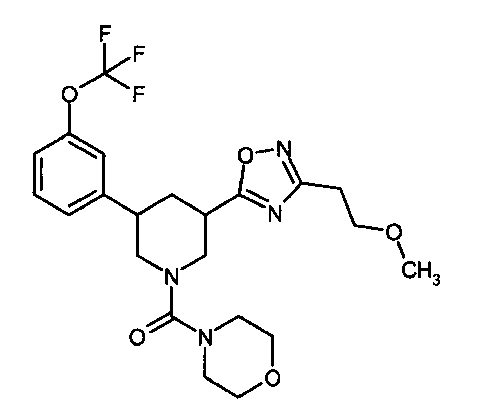

Die Enantiomerentrennung von 95 mg (0.2 mmol) der Verbindung aus Beispiel 193 nach Methode 18D ergab 16 mg der Titelverbindung aus Beispiel 194 und 22 mg der Titelverbindung aus Beispiel 195.

HPLC (Methode 14E): Rₜ = 5.34 min, >99.0% ee;

LC-MS (Methode 2B): Rₜ = 1.24 min; MS (ESIpos): m/z = 485 [M+H]⁺;

¹H-NMR (400 MHz, DMSO-d₆): δ = 7.50-7.45 (m, 1H), 7.40-7.36 (m, 2H), 7.25 (d, 1H), 3.98 (br d, 1H), 3.67 (t, 2H), 3.61-3.55 (m, 5H), 3.42-3.35 (m, 1H), 3.23 (s, 3H), 3.21-3.19 (m, 4H), 3.09-2.99 (m, 3H), 2.93 (t, 2H), 2.33 (br d, 1H), 2.02 (q, 1H).

### Beispiel 195

### {3-[3-(2-Methoxyethyl)-1,2,4-oxadiazol-5-yl]-5-[3-(trifluormethoxy)phenyl]piperidin-1-yl}-(morpholin-4-yl)methanon [enantiomerenreines cis-Isomer]

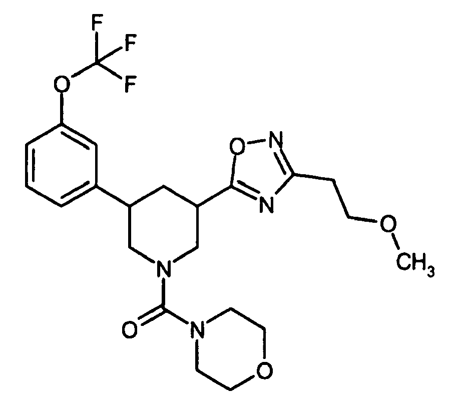

Die Enantiomerentrennung von 95 mg (0.2 mmol) der Verbindung aus Beispiel 193 nach Methode 18D ergab 16 mg der Titelverbindung aus Beispiel 194 und 22 mg der Titelverbindung aus Beispiel 195.

HPLC (Methode 14E): Rₜ = 6.21 min, >99.0% ee;

LC-MS (Methode 2B): Rₜ = 1.24 min; MS (ESIpos): m/z = 485 [M+H]⁺;

¹H-NMR (400 MHz, DMSO-d₆): δ = 7.50-7.46 (m, 1H), 7.40-7.36 (m, 2H), 7.25 (d, 1H), 3.97 (br d, 1H), 3.67 (t, 2H), 3.62-3.55 (m, 5H), 3.43-3.35 (m, 1H), 3.23 (s, 3H), 3.21-3.19 (m, 4H), 3.11-2.99 (m, 3H), 2.93 (t, 2H), 2.31 (br d, 1H), 2.00 (q, 1H).

### Beispiel 196

### 1-({3-(3-{[1-(Hydroxymethyl)cyclopropyl]methyl}-1,2,4-oxadiazol-5-yl)-5-[4-(trifluormethyl)-phenyl]piperidin-1-yl}carbonyl)piperidin-4-carbonitril [racemisches cis-Isomer]

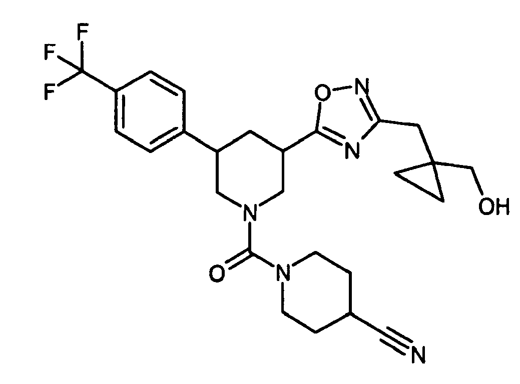

Nach der Allgemeinen Methode 2 wurden 100 mg (0.24 mmol) der Verbindung aus Beispiel 100A und 61 mg (0.37 mmol) der Verbindung aus Beispiel 67A umgesetzt. Ausbeute: 70 mg (56% d. Th.)

HPLC (Methode 2A): Rₜ = 4.41 min; MS (ESIpos): m/z = 518 [M+H]⁺;

¹H-NMR (400 MHz, DMSO-d₆): δ = 7.70 (d, 2H), 7.57 (d, 2H), 4.58 (t, OH), 4.00 (br d, 1H), 3.59 (br d, 1H), 3.43-3.34 (m, 3H), 3.25 (s, 2H), 3.11-3.01 (m, 6H), 2.77 (s, 2H), 2.36 (br d, 1H), 2.01 (q, 1H), 1.88-1.85 (m, 2H), 1.71-1.64 (m, 2H), 0.43-0.41 (m, 4H).

### Beispiel 197

### 1-({3-(3,4-Dimethylphenyl)-5-[3-(propan-2-yl)-1,2,4-oxadiazol-5-yl]piperidin-1-yl}carbonyl)-piperidin-4-carbonitril [racemisches cis-Isomer]

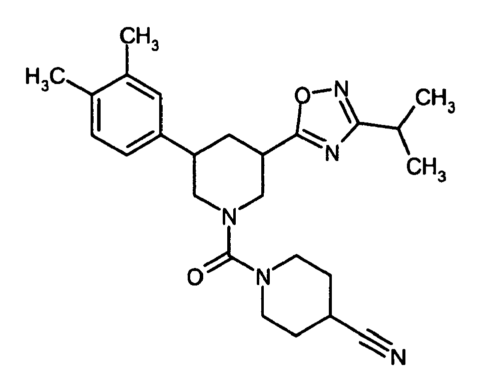

Nach der Allgemeinen Methode 2 wurden 80 mg (0.210 mmol) der Verbindung aus Beispiel 132A und 33 mg (0.315 mmol) *N*'-Hydroxy-2-methylpropanimidamid umgesetzt. Ausbeute: 47 mg (51% d. Th.)

LC-MS (Methode 3B): Rₜ = 2.36 min; MS (ESIpos): m/z = 436 [M+H]⁺;

¹H-NMR (400 MHz, DMSO-d₆): δ = 7.09-7.07 (m, 2H), 7.01 (d, 1H), 3.95 (br d, 1H), 3.53 (br d, 1H), 3.38-3.33 (m, 3H), 3.09-3.01 (m, 4H), 2.99-2.88 (m, 2H), 2.86-2.76 (m, 1H), 2.26 (br d, 1H), 2.20 (s, 3H), 2.18 (s, 3H), 1.93 (q, 1H), 1.88-1.84 (m, 2H), 1.68-1.63 (m, 2H), 1.25 (d, 6H).

### Beispiel 198

### [(3-(3,4-Dimethylphenyl)-5-(3-methyl-1,2,4-oxadiazol-5-yl)piperidin-1-yl](4-hydroxypiperidin-1-yl)methanon [racemisches cis-Isomer]

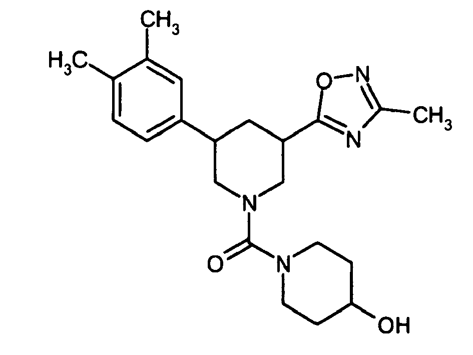

Nach der Allgemeinen Methode 2 wurden 200 mg (0.54 mmol) der Verbindung aus Beispiel 128A und 62 mg (0.81 mmol) *N'*-Hydroxyacetamidin umgesetzt. Ausbeute: 154 mg (69% d. Th.)

LC-MS (Methode 2B): Rₜ = 1.13 min; MS (ESIpos): m/z = 399 [M+H]⁺;

¹H-NMR (400 MHz, DMSO-d₆): δ = 7.09-7.07 (m, 2H), 6.99 (d, 1H), 4.68 (d, OH), 3.93 (br d, 1H), 3.63-3.57 (m, 1H), 3.52 (br d, 1H), 3.48-3.45 (m, 2H), 3.39-3.32 (m, 1H), 2.99-2.76 (m, 5H), 2.32 (s, 3H), 2.25 (br d, 1H), 2.20 (s, 3H), 2.18 (s, 3H), 1.93 (q, 1H), 1.73-1.69 (m, 2H), 1.34-1.25 (m, 2H).

### Beispiel 199

### N-(2-Methoxyethyl)-3-(3-methyl-1,2,4-oxadiazol-5-yl)-5-[4(trifluormethyl)phenyl]piperidin-1-carboxamid [racemisches cis-Isomer]

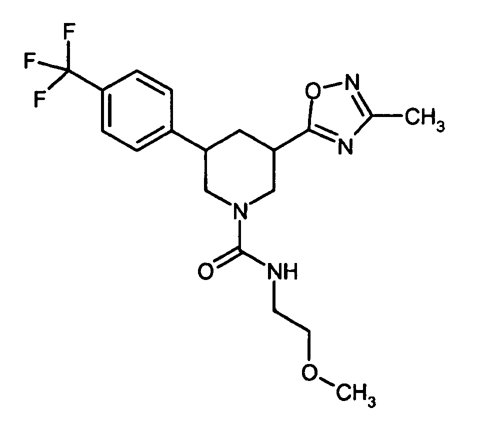

Nach der Allgemeinen Methode 2 wurden 100 mg (0.26 mmol) der Verbindung aus Beispiel 105A und 40 mg (0.534 mmol) *N'*-Hydroxyethanimidamid umgesetzt. Diastereomerentrennung von 62 mg des cis-/trans-Isomerengemisches nach Methode 12C ergab 46 mg der Titelverbindung und 5.7 7 mg des trans-Isomers.

LC-MS (Methode 3B): Rₜ = 1.85 min; MS (ESIpos): m/z = 413 [M+H]⁺;

¹H-NMR (400 MHz, DMSO-d₆): δ = 7.70 (d, 2H), 7.57 (d, 2H), 6.81 (dd, 1H), 4.43 (br d, 1H), 4.09 (d, 1H), 3.24 (s, 3H), 3.28-3.18 (m, 3H), 2.97-2.80 (m, 3H), 2.33 (s, 3H), 2.31 (br d, 1H), 1.98 (dd, 1H).

### Beispiel 200

### {3-(3-Cyclopropyl-1,2,4-oxadiazol-5-yl)-5-[3-(trifluormethoxy)phenyl]piperidin-1-yl}(4-hydroxypiperidin-1-yl)methanon [racemisches cis-Isomer]

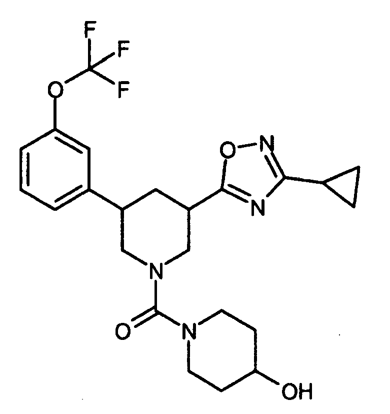

Nach der Allgemeinen Methode 2 wurden 60 mg (0.14 mmol) der Verbindung aus Beispiel 194A und 22 mg (0.22 mmol) *N'*-Hydroxycyclopropancarboximidamid umgesetzt. Ausbeute: 45 mg (64% d. Th.)

LC-MS (Methode 3B): Rₜ = 2.02 min; MS (ESIpos): m/z = 481 [M+H]⁺;

¹H-NMR (400 MHz, CDCl₃): δ = 7.36 (t, 1H), 7.18 (d, 1H), 7.12 (d, 1H), 7.09 (br s, 1H), 4.05 (br d, 1H), 3.90-3.85 (m, 1H), 3.81 (br d, 1H), 3.61 (br d, 2H), 3.34-3.26 (m, 1H), 3.06-3.01 (m, 3H), 2.99-2.92 (m, 1H), 2.85 (t, 1H), 2.44 (br d, 1H), 2.10-2.04 (m, 1H), 1.99-1.89 (m, 3H), 1.56-1.50 (m, 2H), 1.49 (d, 1H), 1.08-1.02 (m, 4H).

### Beispiel 201

### (3-Hydroxyazetidin-1-yl){3-{3-[2-(propan-2-yloxy)ethyl]-1,2,4-oxadiazol-5-yl}-5-[4-(trifluormethyl)phenyl]piperidin-1-yl}methanon [racemisches cis-Isomer]

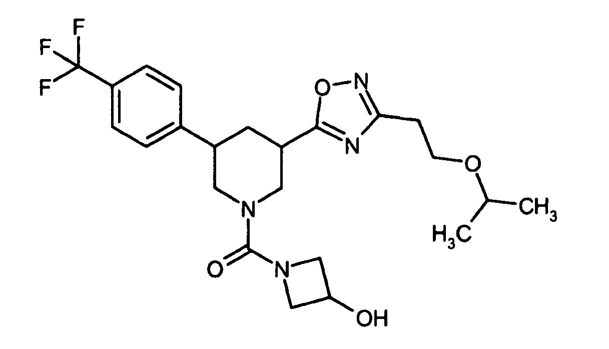

Nach der Allgemeinen Methode 2 wurden 100 mg (0.27 mmol) der Verbindung aus Beispiel 101A und 66 mg (0.40 mmol) der Verbindung aus Beispiel 65A umgesetzt. Ausbeute: 28 mg (20% d. Th.)

HPLC (Methode 2A): Rₜ = 4.41 min; MS (DCIpos): m/z = 483 [M+H]⁺;

¹H-NMR (400 MHz, DMSO-d₆): δ = 7.70 (d, 2H), 7.56 (d, 2H), 5.58 (d, OH), 3.43-3.35 (m, 1H), 4.15 (br d, 1H), 4.09 (q, 2H), 3.76 (br d, 1H), 3.72-3.67 (m, 4H), 3.55 (quintett, 1H), 3.05-2.97 (m, 3H), 2.89 (t, 2H), 2.32 (br d, 1H), 2.02 (q, 1H), 1.04 (d, 6H).

### Beispiel 202

### Morpholin-4-yl{3-[3-(propan-2-yl)-1,2,4-oxadiazol-5-yl]-5-[3-(trifluormethoxy)phenyl]piperidin-1-yl}methanon [racemisches cis-Isomer]

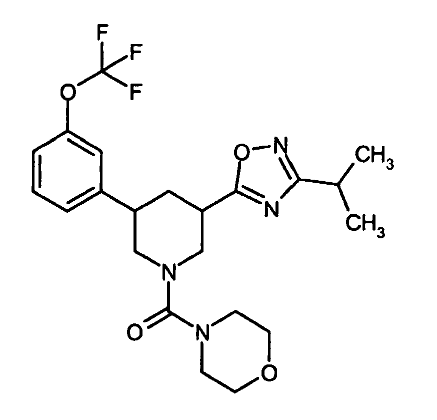

Nach der Allgemeinen Methode 2 wurden 100 mg (0.25 mmol) der Verbindung aus Beispiel 201A und 39 mg (0.37 mmol) *N'*-Hydroxy-2-propanimidamid umgesetzt. Ausbeute: 79 mg (68% d. Th.)

LC-MS (Methode 2B): Rₜ = 1.38 min; MS (ESIpos): m/z = 469 [M+H]⁺;

¹H-NMR (400 MHz, DMSO-d₆): δ = 7.49-7.45 (m, 1H), 7.40-7.36 (m, 2H), 7.25 (d, 1H), 3.98 (br d, 1H), 3.62 (br d, 1H), 3.59-3.55 (m, 4H), 3.41-3.34 (m, 1H), 3.21-3.18 (m, 4H), 3.10-2.85 (m, 4H), 2.31 (br d, 1H), 1.98 (q, 1H), 1.25 (d, 6H).

### Beispiel 203

### 1-{[3-(3-Cyclopropyl-1,2,4-oxadiazol-5-yl)-5-(3,4-dimethylphenyl)piperidin-1-yl]carbonyl}-piperidin-4-carbonitril [racemisches cis-Isomer]

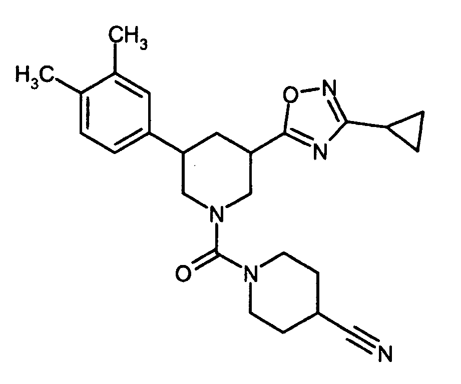

Nach der Allgemeinen Methode 2 wurden 80 mg (0.21 mmol) der Verbindung aus Beispiel 132A und 32 mg (0.32 mmol) *N'*-Hydroxycyclopropanimidamid umgesetzt. Ausbeute: 41 mg (45% d. Th.)

LC-MS (Methode 3B): Rₜ = 2.27 min; MS (ESIpos): m/z = 434 [M+H]⁺;

¹H-NMR (400 MHz, DMSO-d₆): δ = 7.09-7.07 (m, 2H), 6.99 (d, 1H), 3.92 (br d, 1H), 3.52 (br d, 1H), 3.37-3.27 (m, 3H), 3.09-3.03 (m, 3H), 2.96-2.84 (m, 2H), 2.81-2.75 (m, 1H), 2.22 (br d, 1H), 2.20 (s, 3H), 2.18 (s, 3H), 2.14-2.07 (m, 1H), 1.94-1.84 (m, 3H), 1.70-1.62 (m, 2H), 1.09-1.03 (m, 2H), 0.90-0.86 (m, 2H).

### Beispiel 204

### {3-[3-(3-Fluorphenyl)-1,2,4-oxadiazol-5-yl]-5-[4-(trifluormethoxy)phenyl]piperidin-1-yl}(3-hydroxypyrrolidin-1-yl)methanon [Diastereomerengemisch]

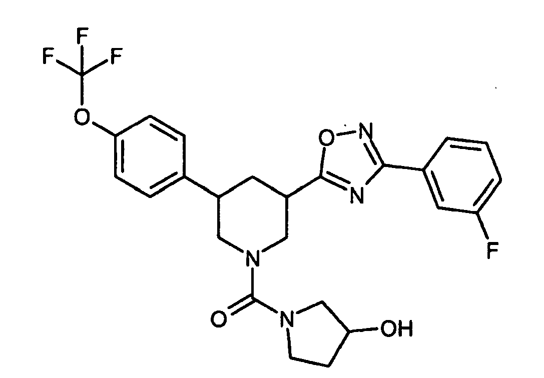

Nach der Allgemeinen Methode 5 wurden 100 mg (0.21 mmol) der Verbindung aus Beispiel 179A und 19 mg (0.21 mmol) 3-Pyrrolidinol umgesetzt. Ausbeute: 43 mg (39% d. Th.)

LC-MS (Methode 1B): Rₜ = 2.68 min; MS (ESIpos): m/z = 521 [M+H]⁺;

¹H-NMR (400 MHz, DMSO-d₆): δ = 7.87 (d, 1H), 7.75 (d, 1H), 7.66-7.61 (m, 1H), 7.51-7.44 (m, 3H), 7.34 (d, 2H), 4.89 (d, OH), 4.23 (br s, 1H), 4.17-4.13 (m, 1H), 3.72 (br t, 1H), 3.57-3.41 (m, 3H), 3.15-2.88 (m, 4H), 2.41 (br d, 1H), 2.12-2.01 (m, 1H), 1.87-1.82 (m, 1H), 1.75-1.73 (m, 1H).

### Beispiel 205

### 1-({3-[3-(3-Fluorphenyl)-1,2,4-oxadiazol-5-yl]-5-[4-(trifluormethoxy)phenyl]piperidin-1-yl}-carbonyl)piperidin-4-carbonitril [racemisches cis-Isomer]

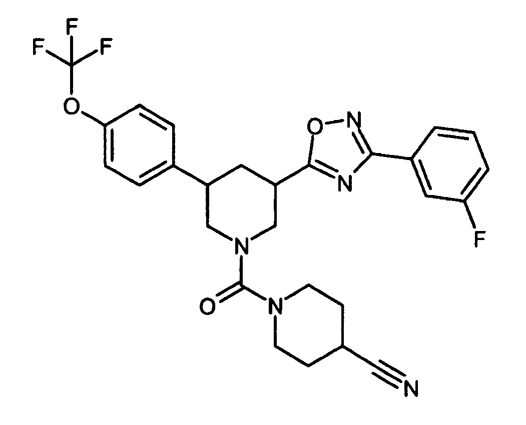

Nach der Allgemeinen Methode 2 wurden 85 mg (0.20 mmol) 1-[(4-Cyanpiperidin-1-yl)carbonyl]-5-[4-(trifluormethoxy)phenyl]piperidin-3-carbonsäure (Beispiel 108A) und 49 mg (0.3 mmol) 3-Fluor-*N'*-hydroxybenzolcarboximidamid umgesetzt. Ausbeute: 69 mg (63% d. Th.).

HPLC (Methode 3B): Rₜ = 2.59 min; MS (ESIpos): m/z = 544.2 [M+H]⁺;

¹H-NMR (400 MHz, DMSO-d₆): δ = 7.87 (d, 1H), 7.76 (d, 1H), 7.64 (dd, 1H), 7.44-7.52 (m, 3H), 7.34 (bd, 2H), 4.05 (bd, 1H), 3.46-3.65 (m, 2H), 3.35-3.45 (m, 2H), 2.97-3.17 (m, 6H), 2.41 (br d, 1H), 2.07 (q, 1H), 1.83-1.93 (bs, 2H), 1.64-1.75 (bs, 2H).

### Beispiel 206

### 1-({3-(3-tert-Butyl-1,2,4-oxadiazol-5-yl)-5-[4-(trifluormethoxy)phenyl]piperidin-1-yl}carbonyl)-piperidin-4-carbonitril [racemisches cis-Isomer]

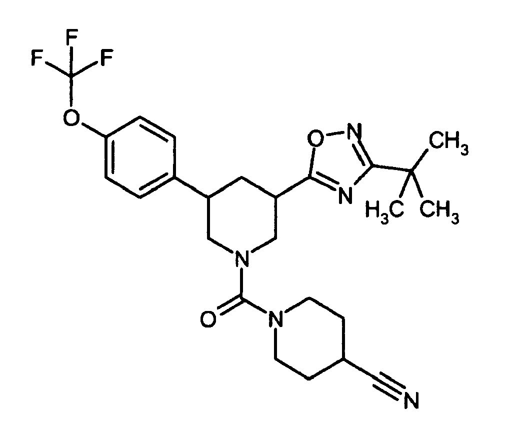

Nach der Allgemeinen Methode 2 wurden 85 mg (0.20 mmol) 1-[(4-Cyanpiperidin-1-yl)carbonyl]-5-[4-(trifluormethoxy)phenyl]piperidin-3-carbonsäure (Beispiel 108A) und 35 mg (0.3 mmol) *N'-*Hydroxy-2,2-dimethylpropanimidamid umgesetzt. Ausbeute: 61 mg (60% d. Th.).

HPLC (Methode 3B): Rₜ = 2.52 min; MS (ESIpos): m/z = 506 [M+H]⁺;

¹H-NMR (400 MHz, DMSO-d₆): δ = 7.47 (d, 2H), 7.33 (d, 2H), 3.96 (bd, 1H), 3.68 (t, 2H), 3.56 (bd, 1H), 3.32-3.43 (m, 3H), 2.93-3.14 (m, 6H), 2.31 (br d, 1H), 1.97 (q, 1H), 1.85 (bd, 2H), 1.61-1.71 (m, 2H), 1.30 (s, 9H).

### Beispiel 207

### 1-({3-[3-(Pyrazin-2-yl)-1,2,4-oxadiazol-5-yl]-5-[4-(trifluormethoxy)phenyl]piperidin-1-yl}-carbonyl)piperidin-4-carbonitril [racemisches cis-Isomer]

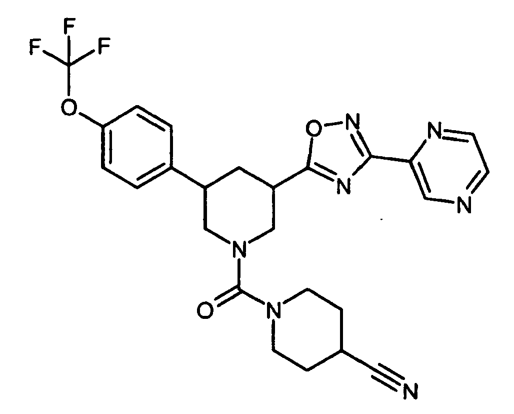

Nach der Allgemeinen Methode 2 wurden 100 mg (0.24 mmol) 1-[(4-Cyanpiperidin-1-yl)carbonyl]-5-[4-(trifluormethoxy)phenyl]piperidin-3-carbonsäure (Beispiel 108A) und 49 mg (0.35 mmol) *N'*-Hydroxypyrazin-2-carboximidamid umgesetzt. Ausbeute: 69 mg (56% d. Th.).

HPLC (Methode 2B): Rₜ = 1.28 min; MS (ESIpos): m/z = 528 [M+H]⁺;

¹H-NMR (400 MHz, DMSO-d₆): δ = 9.27 (d, 1H), 8.86-8.90 (m, 2H), 7.49 (d, 2H), 7.34 (d, 1H), 4.07 (bd, 1H), 3.51-3.66 (m, 2H), 3.34-3.45 (m, 2H), 2.97-3.20 (m, 6H), 2.43 (br d, 1H), 2.08 (q, 1H). 1.83-1.94 (m, 2H), 1.64-1.76 (m, 2H).

### Beispiel 208

### 1-({3-[3-(2-Fluorphenyl)-1,2,4-oxadiazol-5-yl]-5-[4-(trifluormethoxy)phenyl]piperidin-1-yl}-carbonyl)piperidin-4-carbonitril [racemisches cis-Isomer]

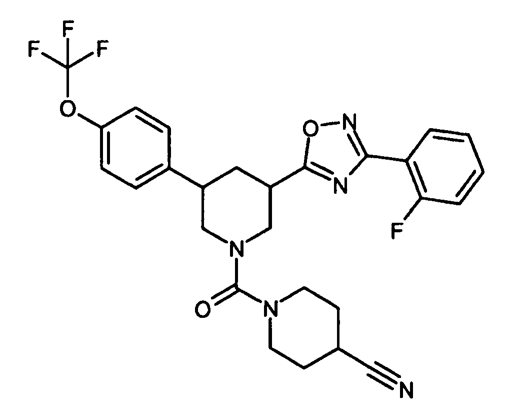

Nach der Allgemeinen Methode 2 wurden 85 mg (0.2 mmol) 1-[(4-Cyanpiperidin-1-yl)carbonyl]-5-[4-(trifluormethoxy)phenyl]piperidin-3-carbonsäure (Beispiel 108A) und 46 mg (0.3 mmol) 2-Fluor-*N'*-hydroxybenzolcarboximidamid umgesetzt. Ausbeute: 69 mg (63% d. Th.).

HPLC (Methode 3B): Rₜ = 2.48 min; MS (ESIpos): m/z = 544 [M+H]⁺;

¹H-NMR (400 MHz, DMSO-d₆): δ = 8.03 (dt, 1H), 7.62-7.70 (m, 1H), 7.39-7.52 (m, 4H), 7.34 (d, 1H), 4.06 (bd, 1H), 3.47-3.66 (m, 2H), 3.35-3.44 (m, 2H), 2.96-3.17 (m, 6H), 2.41 (br d, 1H), 2.07 (q, 1H), 1.82-1.93 (m, 2H), 1.64-1.76 (m, 2H).

### Beispiel 209

### {3-[3-(3-Fluorphenyl)-1,2,4-oxadiazol-5-yl]-5-[3-(trifluormethyl)phenyl]piperidin-1-yl}-(morpholin-4-yl)methanon [racemisches cis-Isomer]

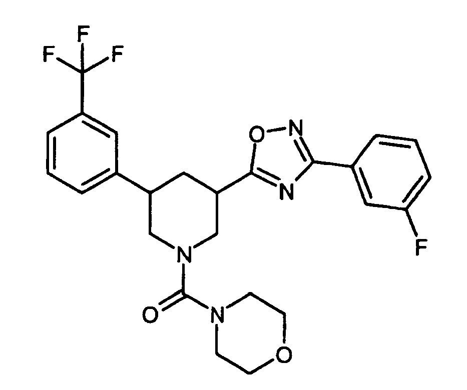

Nach der Allgemeinen Methode 2 wurden 100 mg (0.26 mmol) der Verbindung aus Beispiel 147A und 60 mg (0.39 mmol) der Verbindung aus Beispiel 73A umgesetzt. Ausbeute: 75 mg (57% d. Th.)

LC-MS (Methode 3B): Rₜ = 2.46 min; MS (ESIpos): m/z = 505 [M+H]⁺;

¹H-NMR (400 MHz, DMSO-d₆): δ = 7.87 (d, 1H), 7.78-7.76 (m, 2H), 7.68 (d, 1H), 7.62-7.57 (m, 3H), 7.49-7.44 (m, 1H), 4.09 (br d, 1H), 3.64 (br d, 1H), 3.59-3.54 (m, 4H), 3.52-3.47 (m, 1H), 3.24-3.21 (m, 4H), 3.17-3.07 (m, 3H), 2.40 (br d, 1H), 2.14 (q, 1H).

### Beispiel 210

### (3-Hydroxyazetidin-1-yl){3-(3-{[1-(hydroxymethyl)cyclopropyl]methyl}-1,2,4-oxadiazol-5-yl)-5-[4-(trifluormethyl)phenyl]piperidin-1-yl}methanon [racemisches cis-Isomer]

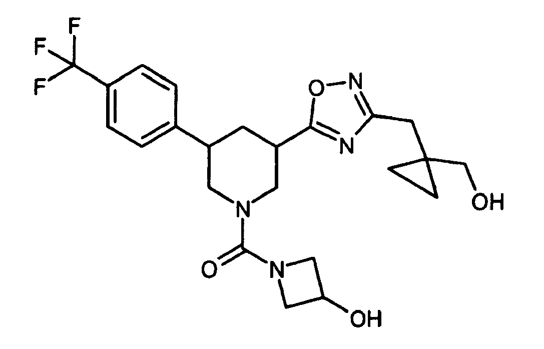

Nach der Allgemeinen Methode 2 wurden 100 mg (0.27 mmol) der Verbindung aus Beispiel 101A und 67 mg (0.40 mmol) der Verbindung aus Beispiel 67A umgesetzt. Ausbeute: 33 mg (26% d. Th.)

HPLC (Methode 2A): Rₜ = 4.10 min; MS (DCIpos): m/z = 481 [M+H]⁺;

¹H-NMR (400 MHz, CDCl₃): δ = 7.60 (d, 2H), 7.36 (d, 2H), 4.67-4.63 (m, 1H), 4.32-4.23 (m, 3H), 4.02 (br d, 1H), 3.92-3.89 (m, 2H), 3.51 (s, 2H), 3.32-3.26 (m, 1H), 3.04 (t, 1H), 2.96-2.92 (m, 1H), 2.88-2.78 (m, 2H), 2.85 (s, 2H), 2.50 (br d, 1H), 2.00 (q, 1H), 0.56 (s, 4H).

### Beispiel 211

### [3-(3,4-Dimethylphenyl)-5-{3-[(propan-2-ylsulfonyl)methyl]-1,2,4-oxadiazol-5-yl}piperidin-1-yl]-(morpholin-4-yl)methanon [racemisches cis-Isomer]

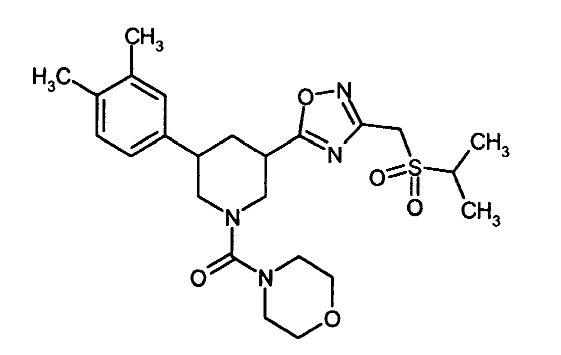

Nach der Allgemeinen Methode 2 wurden 100 mg (0.289 mmol) der Verbindung aus Beispiel 130A und 78 mg (0.433 mmol) der Verbindung aus *N'*-Hydroxy-2-(propan-2-ylsulfonyl)ethanimidamid umgesetzt. Ausbeute: 108 mg (75% d. Th.)

LC-MS (Methode 2B): Rₜ = 1.21 min; MS (ESIpos): m/z = 491 [M+H]⁺;

¹H-NMR (400 MHz, DMSO-d₆): δ = 7.10-7.08 (m, 2H), 7.01 (d, 1H), 4.82 (s, 2H), 4.06 (br d, 1H), 3.58-3.55 (m, 5H), 3.48-3.42 (m, 2H), 3.20-3.16 (m, 4H), 3.21-3.06 (m, 2H), 2.85-2.80 (m, 1H), 2.31 (br d, 1H), 2.21 (s, 3H), 2.18 (s, 3H), 1.95 (q, 1H), 1.31 (d, 6H).

### Beispiel 212

### 1-({3-[3-(2-Hydroxy-2-methylpropyl)-1,2,4-oxadiazol-5-yl]-5-[4-(trifluormethyl)phenyl]piperidin-1-yl}carbonyl)piperidin-4-carbonitril [racemisches cis-Isomer]

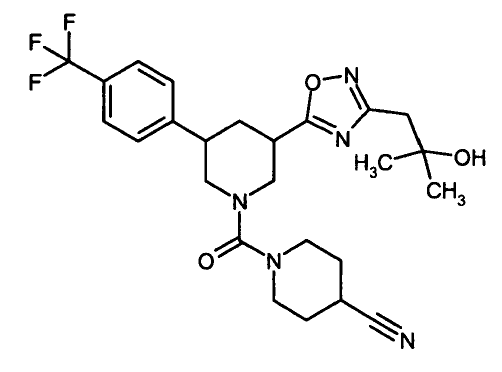

Nach der Allgemeinen Methode 2 wurden 80 mg (0.188 mmol) der Verbindung aus Beispiel 100A und 52 mg (0.281 mmol) *N'*,3-Dihydroxy-3-methylbutanimidamid umgesetzt. Ausbeute: 65 mg (67% d. Th.)

LC-MS (Methode 3B): Rₜ = 1.96 min; MS (ESIpos): m/z = 506 [M+H]⁺;

¹H-NMR (400 MHz, DMSO-d₆): δ = 7.70 (d, 2H), 7.57 (d, 2H), 3.98 (br d, 1H), 3.58 (br d, 1H), 3.43-3.36 (m, 3H), 3.11-2.98 (m, 6H), 2.78 (s, 2H), 2.35 (br d, 1H), 2.01 (q, 1H), 1.88-1.85 (m, 2H), 1.71-1.64 (m, 2H), 1.16 (s, 6H).

### Beispiel 213

### 1-({3-{3-[2-(Propan-2-yloxy)ethyl]-1,2,4-oxadiazol-5-yl}-5-[4-(trifluormethyl)phenyl]piperidin-1-yl}carbonyl)piperidin-4-carbonitril [racemisches cis-Isomer]

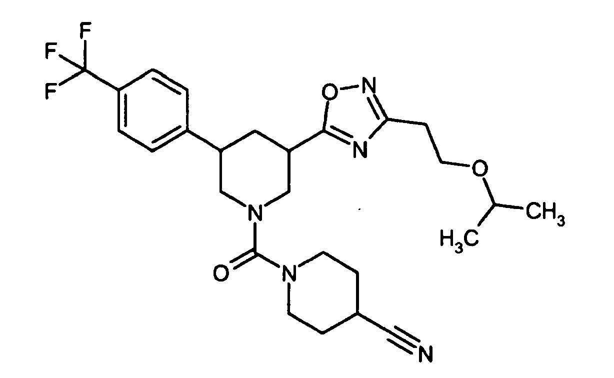

Nach der Allgemeinen Methode 2 wurden 100 mg (0.24 mmol) der Verbindung aus Beispiel 100A und 60 mg (0.37 mmol) der Verbindung aus Beispiel 65A umgesetzt. Ausbeute: 54 mg (42% d. Th.)

HPLC (Methode 1A): Rₜ = 4.75 min; MS (ESIpos): m/z = 420 [M+H]⁺;

¹H-NMR (400 MHz, DMSO-d₆): δ = 7.70 (d, 2H), 7.57 (d, 2H), 3.96 (br d, 1H), 3.71 (t, 2H), 3.61-3.51 (m, 2H), 3.44-3.35 (m, 3H), 3.10-2.97 (m, 6H), 2.89 (t, 2H), 2.32 (br d, 1H), 2.01 (q, 1H), 1.88-1.85 (m, 2H), 1.71-1.64 (m, 2H), 1.10 (d, 6H).

### Beispiel 214

### {3-[3-(3-Fluorphenyl)-1,2,4-oxadiazol-5-yl]-5-[4-(methylsulfonyl)phenyl]piperidin-1-yl}-(morpholin-4-yl)methanon [racemisches cis-Isomer]

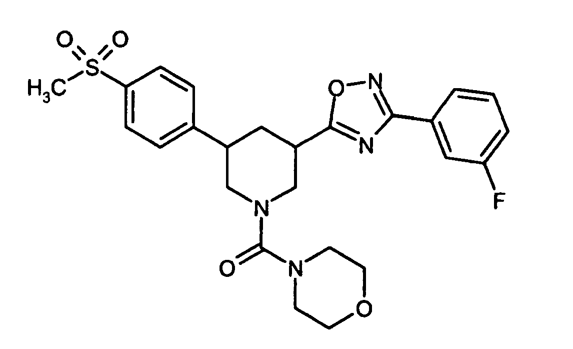

Nach der Allgemeinen Methode 2 wurden 60 mg (0.151 mmol) der Verbindung aus Beispiel 198A und 35 mg (0.227 mmol) 3-Fluor-*N'*-hydroxybenzolcarboximidamid umgesetzt. Ausbeute: 54 mg (67% d. Th.)

LC-MS (Methode 1B): Rₜ = 2.24 min; MS (ESIpos): m/z = 515 [M+H]⁺;

¹H-NMR (400 MHz, DMSO-d₆): δ = 7.90 (d, 2H), 7.86 (d, 1H), 7.77 (dd, 1H), 7.68-7.61 (m, 3H), 7.49-7.44 (m, 1H), 4.09 (br d, 1H), 3.70 (br d, 1H), 3.59-3.56 (m, 4H), 3.54-3.50 (m, 1H), 3.23-3.19 (m, 6H), 3.17-3.04 (m, 4H), 2.42 (br d, 1H), 2.12 (q, 1H).

### Beispiel 215

### (1-Aminocyclopentyl){3-(3-cyclopropyl-1,2,4-oxadiazol-5-yl)-5-[4-(trifluormethyl)phenyl-piperidin-1-yl}methanon [racemisches cis-Isomer]

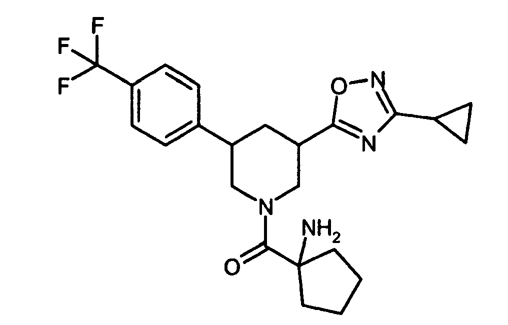

114 mg (0.21 mmol) der Verbindung aus Beispiel 189A wurden in 2 ml Dichlormethan vorgelegt und mit 160 µl (237 mg, 2.08 mmol) Trifluoressigsäure versetzt. Es wurde 16 h bei RT gerührt, 5 ml Essigsäureethylester zugesetzt und die organische Phase dreimal mit gesättigter, wässriger Natriumhydrogencarbonat-Lösung extrahiert. Die organische Phase wurde über Magnesiumsulfat getrocknet, filtriert und im Vakuum eingeengt. Ausbeute: 59 mg (63% d. Th.)

HPLC (Methode 1A): Rₜ = 4.32 min; MS (ESIpos): m/z = 449 [M+H]⁺;

¹H-NMR (400 MHz, DMSO-d₆): δ = 7.70 (d, 2H), 7.55 (d, 2H), 5.17-4.54 (2H, NH₂), 2.99 (br s, 2H), 2.30 (br d, 1H), 2.15-1.84 (m, 6H), 1.69-1.67 (m, 2H), 1.56-1.51 (m, 4H), 1.08-1.02 (m, 2H), 0.91-0.87 (m, 2H).

### Beispiel 216

### 3-(3,4-Dimethylphenyl)-5-[3-(2-methoxyethyl)-1,2,4-oxadiazol-5-yl]piperidin-1-yl}(3-hydroxyazetidin-1-yl)methanon [racemisches cis-Isomer]

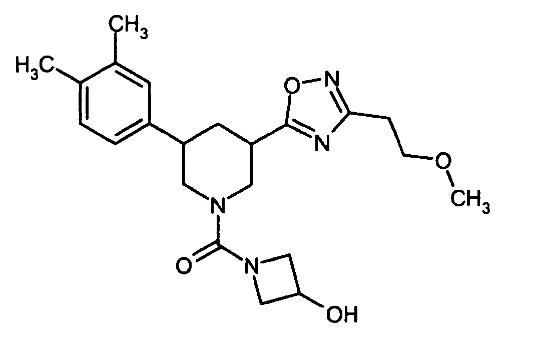

Nach der Allgemeinen Methode 2 wurden 200 mg (0.602 mmol) der Verbindung aus Beispiel 134A und 142 mg (0.903 mmol) *N'*-Hydroxy-3-methoxypropanimidamid umgesetzt. Ausbeute: 99 mg (40% d. Th.)

LC-MS (Methode 1B): Rₜ = 2.07 min; MS (ESIpos): m/z = 415 [M+H]⁺.

¹H-NMR (400 MHz, DMSO-d₆): δ = 7.09-7.07 (m, 2H), 6.99 (d, 1H), 5.57 (d, OH), 4.37 (sextett, 1H), 4.14 (br d, 1H), 4.07 (q, 2H), 3.71-3.66 (m, 5H), 3.23 (s, 3H), 3.00-2.85 (m, 4H), 2.77-2.71 (m, 1H), 2.25 (br d, 1H), 2.20 (s, 3H), 2.18 (s, 3H), 1.99 (q, 1H).

### Beispiel 217

### (4-Hydroxypiperidin-1-yl){3-[3-(2-methoxyethyl)-1,2,4-oxadiazol-5-yl]-5-[3-(trifluormethoxy)-phenyl]piperidin-1-yl}methanon [racemisches cis-Isomer]

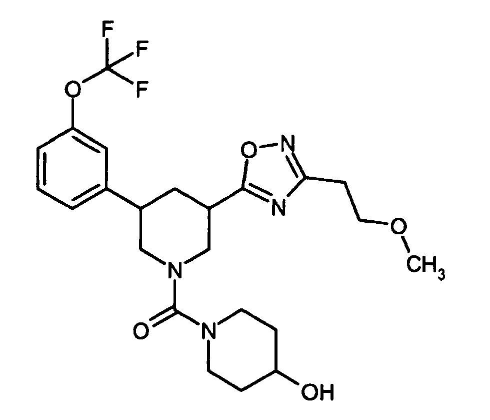

Nach der Allgemeinen Methode 2 wurden 200 mg (0.48 mmol) der Verbindung aus Beispiel 194A und 113 mg (0.72 mmol) *N'*-Hydroxy-3-methoxypropanimidamid umgesetzt. Ausbeute: 182 mg (73% d. Th.)

LC-MS (Methode 1B): Rₜ = 2.20 min; MS (ESIpos): m/z = 499 [M+H]⁺;

¹H-NMR (400 MHz, DMSO-d₆): δ = 7.50-7.44 (m, 1H), 7.39-7.35 (m, 2H), 7.25 (d, 1H), 4.68 (d, OH), 3.94 (br d, 1H), 3.68 (t, 2H), 3.64-3.58 (m, 1H), 3.57 (br d, 1H), 3.50-3.46 (m, 2H), 3.43-3.35 (m, 1H), 3.23 (s, 3H), 3.05-2.88 (m, 7H), 2.34 (br d, 1H), 2.00 (q, 1H), 1.73-1.70 (m, 2H), 1.35-1.26 (m, 2H).

### Beispiel 218

### [3-[4-(Methytsulfonyl)phenyl]-5-{3-[3-(trifluormethyl)phenyl]-1,2,4-oxadiazol-5-yl}piperidin-1-yl](morpholin-4-yl)methanon [racemisches cis-Isomer]

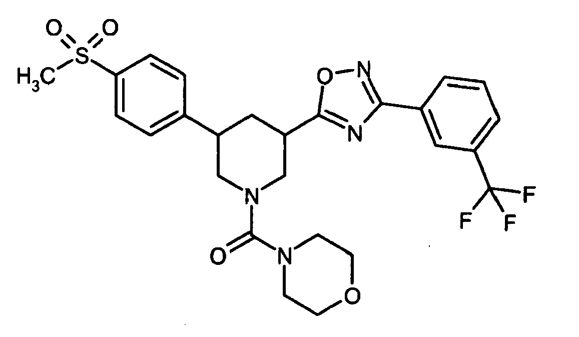

Nach der Allgemeinen Methode 2 wurden 60 mg (0.15 mmol) der Verbindung aus Beispiel 198A und 46 mg (0.23 mmol) *N'*-Hydroxy-3-(trifluormethyl)benzolcarboximidamid umgesetzt. Ausbeute: 61 mg (69% d. Th.)

LC-MS (Methode 2B): Rₜ = 1.28 min; MS (ESIpos): m/z = 565 [M+H]⁺;

¹H-NMR (400 MHz, DMSO-d₆): δ = 8.35 (d, 1H), 8.25 (s, 1H), 8.02 (d, 1H), 7.91 (d, 2H), 7.82 (dd, 1H), 7.65 (d, 2H), 4.10 (br d, 1H), 3.66 (br d, 1H), 3.59-3.53 (m, 5H), 3.24-3.21 (m, 7H), 3.18-3.08 (m, 3H), 2.42 (br d, 1H), 2.12 (q, 1H).

### Beispiel 219

### (4-Hydroxypiperidin-1-yl){3-[3-(pyrazin-2-yl)-1,2,4-oxadiazol-5-yl]-5-[3-(trifluormethoxy)-phenyl]piperidin-1-yl}methanon [racemisches cis-Isomer]

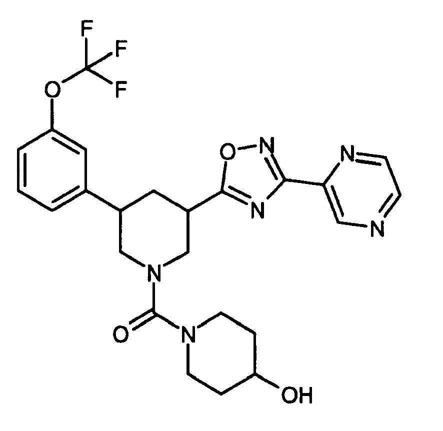

Nach der Allgemeinen Methode 2 wurden 60 mg (0.14 mmol) der Verbindung aus Beispiel 194A und 30 mg (0.22 mmol) *N'*-Hydroxypyrazin-2-carboximidamid umgesetzt. Ausbeute: 36 mg (48% d. Th.)

LC-MS (Methode 3B): Rₜ = 1.79 min; MS (ESIpos): m/z = 519 [M+H]⁺;

¹H-NMR (400 MHz, DMSO-d₆): δ = 9.27 (d, 1H), 8.90-8.86 (m, 2H), 7.50 (dd, 1H), 7.42-7.37 (m, 2H), 7.26 (d, 1H), 4.69 (d, OH), 4.05 (br d, 1H), 3.65-3.39 (m, 5H), 3.14 (t, 1H), 3.09-3.03 (m, 2H), 2.93 (br t, 2H), 2.36 (br d, 1H), 2.08 (q, 1H), 1.74-1.72 (m, 2H), 1.36-1.29 (m, 2H).

### Beispiel 220

### [3-(3,4-Dimethylphenyl)-5-(3-methyl-1,2,4-oxadiazol-5-yl)piperidin-1-yl](morpholin-4-yl)-methanon [racemisches cis-Isomer]

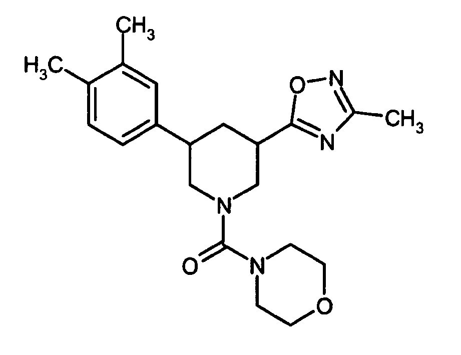

Nach der Allgemeinen Methode 2 wurden 100 mg (0.29 mmol) der Verbindung aus Beispiel 130A und 33 mg (0.43 mmol) *N'*-Hydroxyacetamidin umgesetzt. Ausbeute: 68 mg (61 % d. Th.)

LC-MS (Methode 3B): Rₜ = 1.91 min; MS (ESIpos): m/z = 385 [M+H]⁺;

¹H-NMR (400 MHz, DMSO-d₆): δ = 7.09-7.07 (m, 2H), 6.99 (d, 1H), 3.97 (br d, 1H), 3.59-3.55 (m, 5H), 3.40-3.34 (m, 1H), 3.19-3.16 (m, 4H), 3.00 (t, 1H), 2.90 (t, 1H), 2.84-2.75 (m, 1H), 2.33 (s, 3H), 2.26 (br d, 1H), 2.20 (s, 3H), 2.18 (s, 3H), 1.94 (q, 1H).

### Beispiel 221

### 1-({3-[3-(1-Methoxy-2-methylpropan-2-yl)-1,2,4-oxadiazol-5-yl]-5-[4-(trifluormethyl)phenyl]-piperidin-1-yl}carbonyl)piperidin-4-carbonitril [racemisches cis-Isomer]

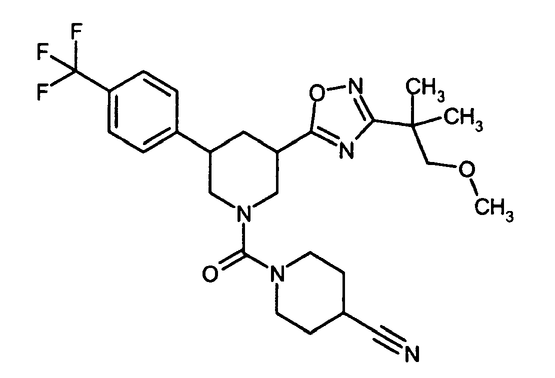

Nach der Allgemeinen Methode 2 wurden 80 mg (0.188 mmol) der Verbindung aus Beispiel 100A und 41 mg (0.281 mmol) *N'*-Hydroxy-3-methoxy-2,2-dimethylpropanimidamid umgesetzt. Ausbeute: 55 mg (56% d. Th.)

LC-MS (Methode 1B): Rₜ = 2.70 min; MS (ESIpos): m/z = 520 [M+H]⁺;

¹H-NMR (400 MHz, DMSO-d₆): δ = 7.70 (d, 2H), 7.58 (d, 2H), 5.57 (d, OH), 3.97 (br d, 1H), 3.58 (br d, 1H), 3.45 (s, 2H), 3.40-3.37 (m, 3H), 3.21 (s, 3H), 3.10-3.00 (m, 5H), 2.32 (br d, 1H), 1.99 (q, 1H), 1.87-1.83 (m, 2H), 1.71-1.67 (m, 2H), 1.26 (s, 6H).

### Beispiel 222

### 1-({3-[3-(1-Cyclopropylpiperidin-4-yl)-1,2,4-oxadiazol-5-yl]-5-[4-(trifluormethyl)phenyl]-piperidin-1-yl}carbonyl)piperidin-4-carbonitril [racemisches cis-Isomer]

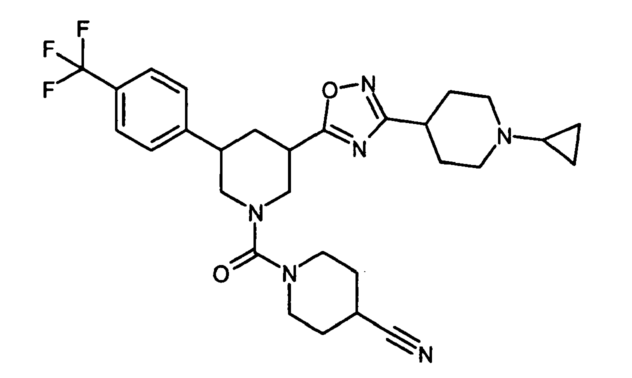

Nach der Allgemeinen Methode 2 wurden 155 mg (0.363 mmol) der Verbindung aus Beispiel 100A und 100 mg (0.545 mmol) der Verbindung aus Beispiel 75A umgesetzt. Ausbeute: 76 mg (38% d. Th.)

LC-MS (Methode 3B): Rₜ = 1.36 min; MS (ESIpos): m/z = 557 [M+H]⁺;

¹H-NMR (400 MHz, DMSO-d₆): δ = 7.70 (d, 2H), 7.57 (d, 2H), 3.96 (br d, 1H), 3.58 (br d, 1H), 3.43-3.35 (m, 2H), 3.11-2.93 (m, 8H), 2.80-2.71 (m, 2H), 2.36-2.24 (m, 3H), 2.00 (q, 1H), 1.90-1.85 (m, 4H), 1.71-1.55 (m, 5H), 0.43-0.39 (m, 2H), 0.30-0.27 (m, 2H).

### Beispiel 223

### {3-(3-Cyclopropyl-1,2,4-oxadiazol-5-yl)-5-[4-(trifluormethyl)phenyl]piperidin-l-yl}[1-(dimethylamino)cyclopropyl]methanon [racemisches cis-Isomer]

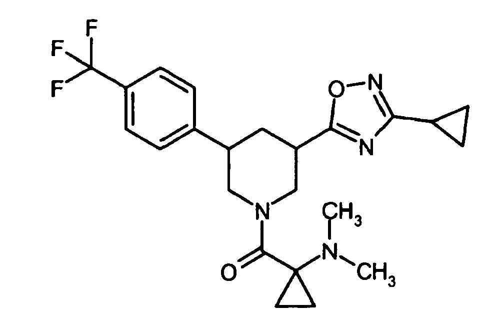

Nach der Allgemeinen Methode 7 wurden 100 mg (0.30 mmol) der Verbindung aus Beispiel 183A und 42 mg (0.33 mmol) 1-(Dimethylamino)cyclopropancarbonsäure umgesetzt. Ausbeute: 120 mg (90% d. Th.)

HPLC (Methode 1A): Rₜ = 4.31 min; MS (ESIpos): m/z = 449 [M+H]⁺;

¹H-NMR (400 MHz, DMSO-d₆): δ = 7.71 (d, 2H), 7.57 (d, 2H), 4.70 (br d, 1H), 4.38 (br d, 1H), 3.28-3.24 (m, 1H), 3.10-3.01 (m, 3H), 2.32 (br d, 1H), 2.15 (s, 6H), 2.14-2.04 (m, 2H), 1.08-1.03 (m, 2H), 0.91-0.87 (m, 4H), 0.82-0.77 (m, 2H).

### Beispiel 224

### (3-Aminopyrrolidin-1-yl){3-[3-(3-fluorphenyl)-1,2,4-oxadiazol-5-yl]-5-[4-(trifluormethoxy)-phenyl]piperidin-1-yl}methanon [Diastereomerengemisch]

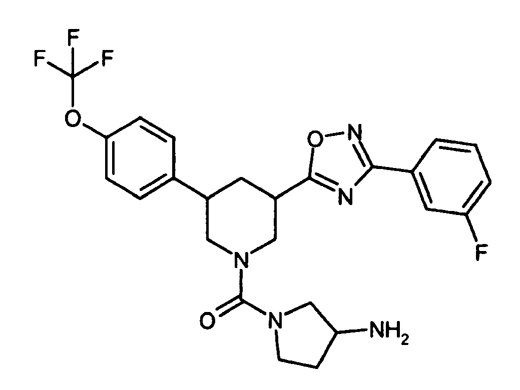

Nach der Allgemeinen Methode 5 wurden 100 mg (0.21 mmol) der Verbindung aus Beispiel 179A und 34 mg (0.21 mmol) Pyrrolidin-3-amin umgesetzt. Ausbeute: 32 mg (28% d. Th.)

LC-MS (Methode 2B): Rₜ = 1.17 min; MS (ESIpos): m/z = 520 [M+H]⁺;

¹H-NMR (400 MHz, DMSO-d₆): δ = 7.87 (d, 1H), 7.77 (dd, 1H), 7.66-7.61 (m, 1H), 7.50-7.45 (m, 3H), 7.34 (d, 2H), 4.14 (br d, 1H), 3.72 (br d, 1H), 3.52-3.41 (m, 3H), 3.37-3.35 (m, 2H), 3.06 (t, 1H), 3.01-2.92 (m, 3H), 2.42 (br d, 1H), 2.05 (q, 1H), 1.86 (sextett, 1H), 1.77 (br s, 1H), 1.53 (sextett, 1H).

### Beispiel 225

### {3-(3-tert-Butyl-1,2,4-oxadiazol-5-yl)-5-[3-(trifluormethyl)phenyl]piperidin-1-yl}(morpholin-4-yl)methanon [racemisches cis-Isomer]

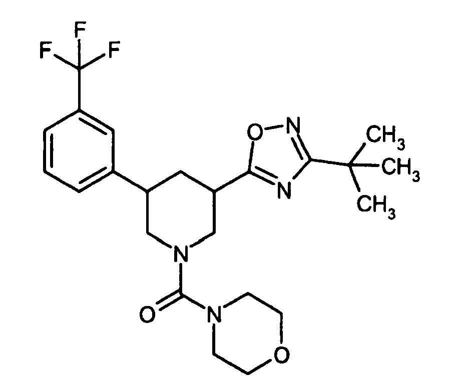

Nach der Allgemeinen Methode 2 wurden 100 mg (0.26 mmol) der Verbindung aus Beispiel 147A und 45 mg (0.39 mmol) *N'*-Hydroxy-2,2-dimethylpropanimidamid (A. Hamze et al. J. Org. Chem. 2003, 68, 7316) umgesetzt. Ausbeute: 89 mg (70% d. Th.)

LC-MS (Methode 2B): Rₜ = 1.44 min; MS (ESIpos): m/z = 467 [M+H]⁺;

¹H-NMR (400 MHz, DMSO-d₆): δ = 7.73 (br s, 1H), 7.67 (d, 1H), 7.62 (d, 1H), 7.59-7.55 (m, 1H), 3.98 (br d, 1H), 3.62 (br d, 1H), 3.58-3.54 (m, 4H), 3.42-3.34 (m, 1H), 3.21-3.19 (m, 4H), 3.14-3.04 (m, 3H), 2.32 (br d, 1H), 2.02 (q, 1H), 1.30 (s, 9H).

### Beispiel 226

### {3-[3-(3-Fluorphenyl)-1,2,4-oxadiazol-5-yl]-5-[3-(trifluormethyl)phenyl]piperidin-1-yl}(4-hydroxypiperidin-1-yl)methanon [racemisches cis-Isomer]

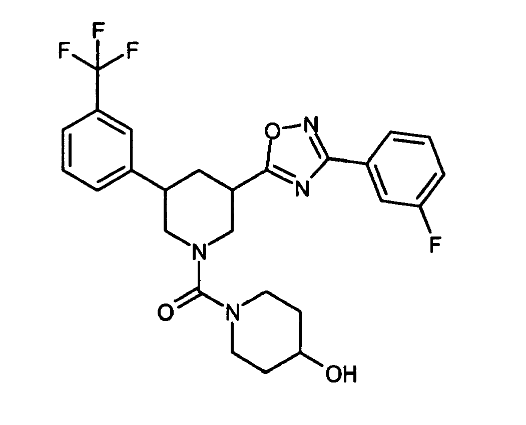

Nach der Allgemeinen Methode 2 wurden 75 mg (0.2 mmol) der Verbindung aus Beispiel 150A und 52 mg (0.3 mmol) 3-Fluor-*N'*-hydroxybenzolcarboximidamid umgesetzt. Ausbeute: 16 mg (18%d. Th.)

LC-MS (Methode 2B): Rₜ = 1.42 min; MS (ESIpos): m/z = 519 [M+H]⁺;

¹H-NMR (400 MHz, DMSO-d₆): δ = 7.87 (d, 1H), 7.78-7.73 (m, 2H), 7.69-7.59 (m, 4H), 7.49-7.44 (m, 1H), 4.69 (d, OH), 4.04 (br d, 1H), 3.65-3.58 (m, 2H), 3.52-3.49 (m, 3H), 3.18-3.07 (m, 3H), 2.93 (t, 2H), 2.40 (br d, 1H), 2.11 (q, 1H), 1.75-1.71 (m, 2H), 1.37-1.28 (m, 2H).

### Beispiel 227

### [1-(Aminomethyl)cyclopropyl]{3-[3-(3-fluorphenyl)-1,2,4-oxadiazol-5-yl]-5-[4-(trifluormethoxy)phenyl]piperidin-1-yl}methanon [racemisches cis-Isomer]

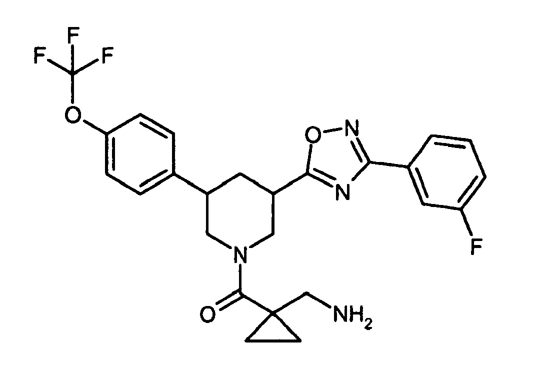

124 mg (0.21 mmol) der Verbindung aus Beispiel 79A wurden in 5 ml Dichlormethan vorgelegt und mit 158 µl (234 mg, 2.05 mmol) Trifluoressigsäure versetzt. Das Reaktionsgemisch wurde 4 h bei RT gerührt, anschließend in Essigsäureethylester aufgenommen und mit gesättigter, wässriger Natriumhydrogencarbonat-Lösung gewaschen. Die organische Phase wurde über Natriumsulfat getrocknet, filtriert und im Vakuum eingeengt. Ausbeute: 92 mg (88% d. Th.)

LC-MS (Methode 2B): Rₜ = 1.21 min; MS (ESIpos): m/z = 505 [M+H]⁺;

¹H-NMR (400 MHz, DMSO-d₆): δ = 7.87 (dd, 1H), 7.75 (dd, 1H), 7.67-7.61 (m, 1H), 7.51-7.45 (m, 3H), 7.36 (d, 2H), 4.81 (br d, 1H), 4.38 (br d, 1H), 3.50-3.45 (m, 1H), 3.25-2.95 (m, 3H), 2.71-2.66 (m, 2H), 2.40 (br d, 1H), 2.15 (q, 1H), 0.79-0.71 (m, 4H).

### Beispiel 228

### 2-Amino-1-{3-[3-(3-fluorphenyl)-1,2,4-oxadiazol-5-yl]-5-[4-(trifluormethoxy)phenyl]piperidin-1-yl}-2-methylpropan-1-on [racemisches cis-Isomer]

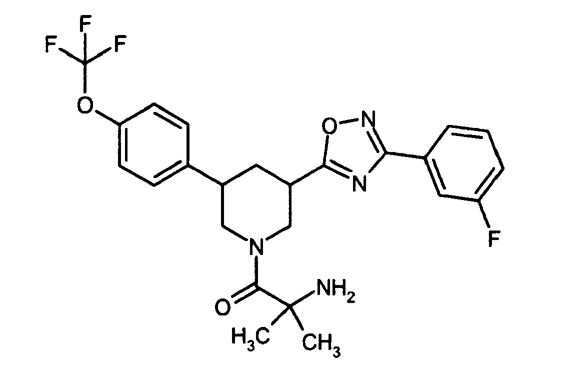

110 mg (0.186 mmol) der Verbindung aus Beispiel 188A wurden in 1.4 ml Dichlormethan vorgelegt und mit 143 µl (212 mg, 1.87 mmol) Trifluoressigsäure versetzt. Das Reaktionsgemisch wurde 12 h bei RT gerührt, anschließend in Essigsäureethylester aufgenommen und mit gesättigter, wässriger Natriumhydrogencarbonat-Lösung sowie gesättigter, wässriger Natriumchlorid-Lösung gewaschen. Die organische Phase wurde über Natriumsulfat getrocknet, filtriert und im Vakuum eingeengt. Das Rohprodukt wurde mittels präparativer HPLC aufgereinigt. Ausbeute: 48 mg (53% d. Th.)

LC-MS (Methode 1B): Rₜ = 1.84 min; MS (ESIpos): m/z = 493 [M+H]⁺;

¹H-NMR (400 MHz, DMSO-d₆): δ = 7.87 (dd, 1H), 7.75 (ddd, 1H), 7.67-7.62 (m, 1H), 7.50-7.45 (m, 3H), 7.35 (d, 2H), 5.39 (br s, NH), 5.02 (br s, NH), 3.46-3.42 (m, 1H), 3.08-2.89 (m, 1H), 3.08-2.95 (m, 3H), 2.50 (br d, 1H), 2.16 (q, 1H), 2.04-1.98 (m, 2H), 1.32 (s, 3H), 1.31 (s, 3H).

### Beispiel 229

### {3-[3-(3-Fluorphenyl)-1,2,4-oxadiazol-5-yl]-5-[3-methyl-4-(trifluormethoxy)phenyl]piperidin-1-yl}(4-hydroxypiperidin-1-yl)methanon [racemisches cis-Isomer]

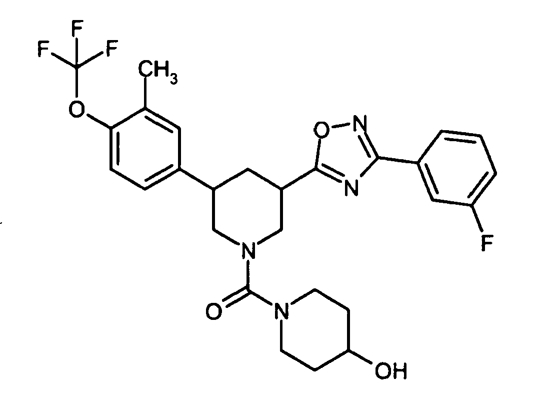

Nach der Allgemeinen Methode 2 wurden 100 mg (0.22 mmol) der Verbindung aus Beispiel 170A und 51 mg (0.33 mmol) 3-Fluor-*N*'-hydroxybenzolcarboximidamid umgesetzt. Ausbeute: 60 mg (50% d. Th.)

LC-MS (Methode 2B): Rₜ = 1.52 min; MS (ESIpos): m/z = 549 [M+H]⁺;

¹H-NMR (400 MHz, DMSO-d₆): δ = 7.87 (d, 1H), 7.75 (dd, 1H), 7.67-7.61 (m, 1H), 7.49-7.45 (m, 1H), 7.39 (s, 1H), 7.28 (s, 2H), 4.69 (d, OH), 4.05 (br d, 1H), 3.64-3.57 (m, 2H), 3.52-3.49 (m, 3H), 3.09 (t, 1H), 3.02-2.90 (m, 4H), 2.37 (br d, 1H), 2.28 (s, 3H), 2.05 (q, 1H), 1.74-1.71 (m, 2H), 1.37-1.28 (m, 2H).

### Beispiel 230

### {3-(3-Cyclopropyl-1,2,4-oxadiazol-5-yl)-5-[3-methyl-4-(trifluormethoxy)phenyl]piperidin-1-yl}(4-hydroxypiperidin-1-yl)methanon [racemisches cis-Isomer]

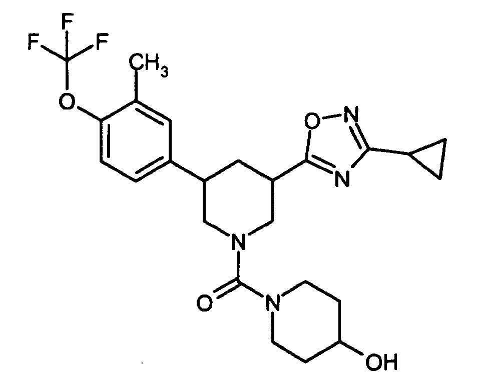

Nach der Allgemeinen Methode 2 wurden 100 mg (0.22 mmol) der Verbindung aus Beispiel 170A und 33 mg (0.331 mmol) *N*'-Hydroxycyclopropancarboximidamid umgesetzt. Ausbeute: 41 mg (37% d. Th.)

LC-MS (Methode 9B): Rₜ = 1.18 min; MS (ESIpos): m/z = 495 [M+H]⁺;

¹H-NMR (400 MHz, DMSO-d₆): δ = 7.36 (s, 1H), 7.26 (s, 2H), 4.68 (d, OH), 3.90 (br d, 1H), 3.62-3.58 (m, 1H), 3.53 (br d, 1H), 3.49-3.45 (m, 2H), 2.98-2.87 (m, 5H), 2.38-2.35 (m, 4H), 2.14-2.09 (m, 1H), 1.91 (q, 1H), 1.72-1.70 (m, 2H), 1.34-1.25 (m, 2H), 1.08-1.03 (m, 2H), 0.90-0.86 (m, 2H).

### Beispiel 231

### (4-Hydroxypiperidin-1-yl) {3-[3-(2-methoxyethyl)-1,2,4-oxadiazol-5-yl]-5-[3-methyl-4-(trifluormethoxy)phenyl]piperidin-1-yl}methanon [racemisches cis-Isomer]

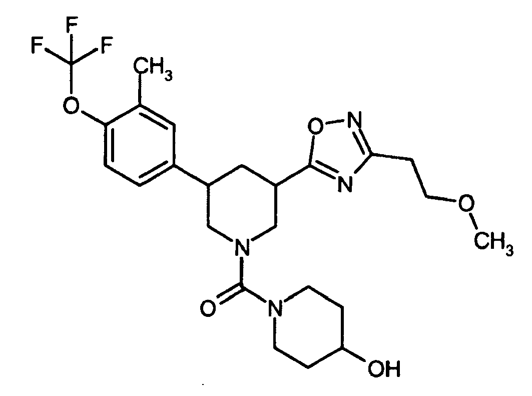

Nach der Allgemeinen Methode 2 wurden 100 mg (0.221 mmol) der Verbindung aus Beispiel 170A und 52 mg (0.331 mmol) der Verbindung aus Beispiel 64A umgesetzt. Ausbeute: 39 mg (35% d. Th.)

LC-MS (Methode 2B): Rₜ = 1.26 min; MS (ESIpos): m/z = 513 [M+H]⁺;

¹H-NMR (400 MHz, DMSO-d₆): δ = 7.36 (s, 1H), 7.26 (s, 2H), 4.67 (d, OH), 3.93 (br d, 1H), 3.68 (t, 2H), 3.62-3.58 (m, 1H), 3.56 (br d, 1H), 3.50-3.42 (m, 2H), 3.39-3.33 (m, 1H), 3.23 (s, 3H), 2.96-2.78 (m, 7H), 2.28-2.24 (m, 4H), 1.96 (q, 1H), 1.73-1.70 (m, 2H), 1.32-1.29 (m, 2H).

### Beispiel 232

### {3-(3,4-Dimethylphenyl)-5-[3-(3-fluorphenyl)-1,2,4-oxadiazol-5-yl]piperidin-1-yl}(4-hydroxypiperidin-1-yl)methanon [racemisches cis-Isomer]

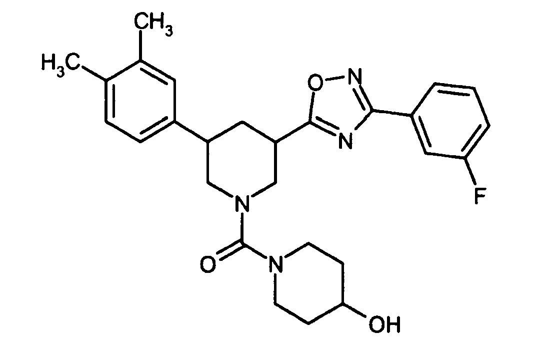

Nach der Allgemeinen Methode 2 wurden 80 mg (0.22 mmol) der Verbindung aus Beispiel 128A und 51 mg (0.33 mmol) der Verbindung aus Beispiel 73A umgesetzt. Ausbeute: 69 mg (65% d. Th.)

LC-MS (Methode 2B): Rₜ = 1.45 min; MS (ESIpos): m/z = 479 [M+H]⁺;

¹H-NMR (400 MHz, DMSO-d₆): δ = 7.87 (d, 1H), 7.75 (dd, 1H), 7.67-7.61 (m, 1H), 7.46 (ddd, 1H), 7.10-7.08 (m, 2H), 7.01 (d, 1H), 4.68 (d, OH), 4.04 (br d, 1H), 3.64-3.59 (m, 1H), 3.56-3.46 (m, 4H), 3.07 (t, 1H), 2.97-2.81 (m, 4H), 2.36 (br d, 1H), 2.21 (s, 3H), 2.19 (s, 3H), 2.03 (q, 1H), 1.74-1.71 (m, 2H), 1.36-1.27 (m, 2H).

### Beispiel 233

### {3-(3,4-Dimethylphenyl)-5-[3-(propan-2-yl)-1,2,4-oxadiazol-5-yl]piperidin-1-yl}(3-hydroxyazetidin-1-yl)methanon [racemisches cis-Isomer]

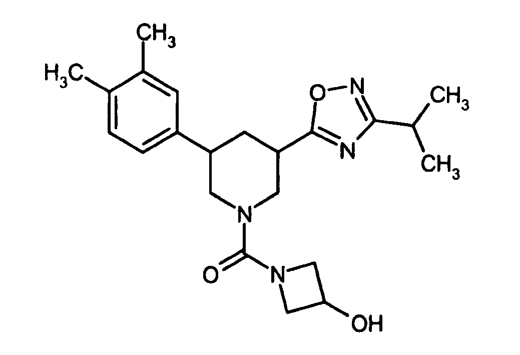

Nach der Allgemeinen Methode 2 wurden 72 mg (0.22 mmol) der Verbindung aus Beispiel 134A und 43 mg (0.33 mmol) *N*'-Hydroxy-2-methylpropanimidamid umgesetzt. Ausbeute: 17 mg (19% d. Th.)

LC-MS (Methode 2B): Rₜ = 1.27 min; MS (ESIpos): m/z = 399 [M+H]⁺;

¹H-NMR (400 MHz, DMSO-d₆): δ = 7.10-7.07 (m, 2H), 7.02 (d, 1H), 5.57 (d, OH), 4.38 (sextett, 1H), 4.15 (br d, 1H), 4.07 (q, 2H), 3.71-3.66 (m, 3H), 3.26-3.22 (m, 1H), 3.09-2.86 (m, 3H), 2.76-2.67 (m, 1H), 2.25 (br d, 1H), 2.21 (s, 3H), 2.18 (s, 3H), 1.96 (q, 1H), 1.25 (d, 6H).

### Beispiel 234

### {3-(3,4-Dimethylphenyl)-5-[3-(propan-2-yl)-1,2,4-oxadiazol-5-yl]piperidin-1-yl}(4-hydroxypiperidin-1-yl)methanon [racemisches cis-Isomer]

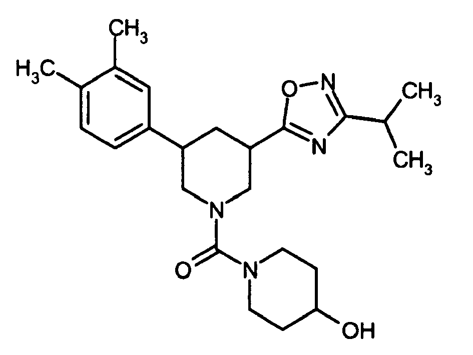

Nach der Allgemeinen Methode 2 wurden 80 mg (0.222 mmol) der Verbindung aus Beispiel 128A und 35 mg (0.333 mmol) *N*'-Hydroxy-2-methylpropanimidamid umgesetzt. Ausbeute: 55 mg (59% d.Th.)

LC-MS (Methode 2B): Rₜ = 1.32 min; MS (ESIpos): m/z = 427 [M+H]⁺;

¹H-NMR (400 MHz, DMSO-d₆): δ = 7.09-7.07 (m, 2H), 7.00 (d, 1H), 4.68 (d, OH), 3.93 (br d, 1H), 3.63-3.56 (m, 1H), 3.53-3.45 (m, 3H), 3.39-3.34 (m, 1H), 3.09-3.00 (m, 2H), 2.97-2.82 (m, 3H), 2.80-2.76 (m, 1H), 2.26 (br d, 1H), 2.20 (s, 3H), 2.18 (s, 3H), 1.93 (q, 1H), 1.73-1.70 (m, 2H), 1.34-1.26 (m, 2H), 1.25 (d, 6H).

### Beispiel 235

### (3-Aminopyrrolidin-1-yl){3-(3-cyclopropyl-1,2,4-oxadiazol-5-yl)-5-[4-(trifluormethoxy)phenyl]-piperidin-1-yl}methanon [Diastereomerengemisch]

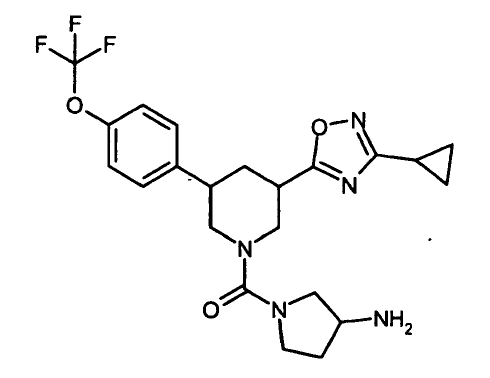

Nach der Allgemeinen Methode 6 wurden 90 mg (0.17 mmol) der Verbindung aus Beispiel 176A und 83 mg (0.52 mmol) 3-Aminopyrrolidin-Hydrochlorid umgesetzt. Ausbeute: 13 mg (15% d. Th.)

LC-MS (Methode 2B): Rₜ = 1.05 min; MS (ESIpos): m/z = 466 [M+H]⁺;

¹H-NMR (400 MHz, DMSO-d₆): δ = 7.46 (d, 2H), 7.32 (d, 2H), 4.01 (br d, 1H), 3.68 (br d, 1H), 3.46-3.38 (m, 4H), 2.96-2.87 (m, 4H), 2.28 (br d, 1H), 2.14-2.01 (m, 1H), 1.94-1.84 (m, 2H), 1.51 (sextett, 1H), 1.01-1.07 (m, 2H), 0.90-0.83 (m, 2H).

### Beispiel 236

### Morpholin-4-yl {3-[3-(propan-2-yl)-1,2,4-oxadiazol-5-yl]-5-[4-(trifluormethoxy)phenyl]piperidin-1-yl}methanon [racemisches cis-Isomer]

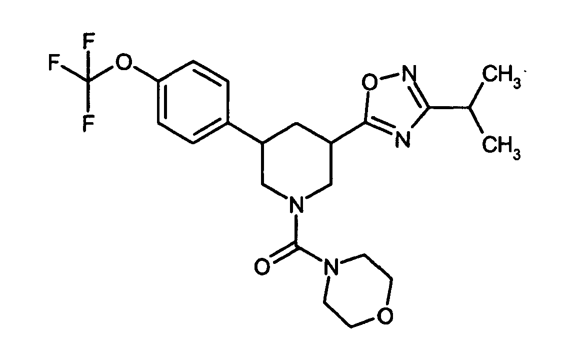

Nach der Allgemeinen Methode 2 wurden 200 mg (0.50 mmol) der Verbindung aus Beispiel 44A und 76 mg (0.75 mmol) *N'*-Hydroxy-2-methylpropanimidamid umgesetzt. Ausbeute: 144 mg (58% d. Th.)

LC-MS (Methode 1B): Rₜ = 2.58 min; MS (ESIpos): m/z = 469 [M+H]⁺;

¹H-NMR (400 MHz, DMSO-d₆): δ = 7.47 (d, 2H), 7.33 (d, 2H), 3.99 (d, 1H), 3.60 (d, 1H), 3.58-3.54 (m, 4H), 3.38 (tt, 1H), 3.20-3.18 (m, 4H), 3.08-2.92 (m, 4H), 2.31 (br d, 1H), 1.96 (q, 1H), 1.25 (d, 6H).

### Beispiel 237

### {3-(3-Methyl-1,2,4-oxadiazol-5-yl)-5-[4-(trifluormethoxy)phenyl]piperidin-1-yl}(morpholin-4-yl)methanon [racemisches cis-Isomer]

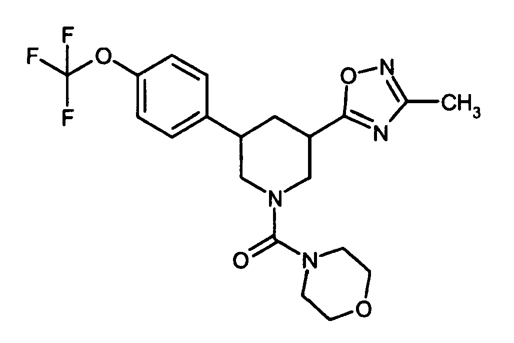

Nach der Allgemeinen Methode 2 wurden 200 mg (0.50 mmol) der Verbindung aus Beispiel 44A und 74 mg (0.75 mmol) *N'*-Hydroxyethanimidamid umgesetzt. Ausbeute: 119 mg (52% d. Th.)

LC-MS (Methode 1B): Rₜ = 2.31 min; MS (ESIpos): m/z = 441[M+H]⁺;

¹H-NMR (400 MHz, DMSO-d₆): δ = 7.46 (d, 2H), 7.33 (d, 2H), 3.98 (d, 1H), 3.61 (d, 1H), 3.58-3.54 (m, 4H), 3.38 (tt, 1H), 3.21-3.19 (m, 4H), 3.03 (t, 1H), 3.00-2.95 (m, 2H), 2.33 (s, 3H), 2.31 (br d, 1H), 1.96 (q, 1H).

### Beispiel 238

### {3-[3-(Methoxymethyl)-1,2,4-oxadiazol-5-yl]-5-[4-(trifluormethoxy)phenyl]piperidin-1-yl}-(morpholin-4-yl)methanon [racemisches cis-Isomer]

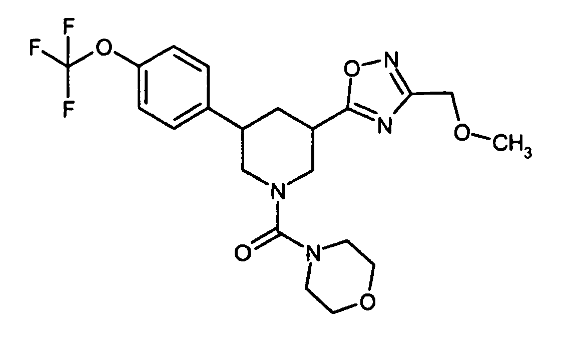

Nach der Allgemeinen Methode 1 wurden 100 mg (0.25 mmol) der Verbindung aus Beispiel 44A und 31 mg (0.30 mmol) *N'*-Hydroxy-2-methoxyethanimidamid (J. Med. Chem., 1997, 40, 8, 1230-1246) umgesetzt. Ausbeute: 19 mg (16% d. Th.).

LC-MS (Methode 2B): Rₜ = 1.25 min; MS (ESIpos): m/z = 471 [M+H]⁺;

¹H-NMR (400 MHz, DMSO-d₆): δ = 7.47 (d, 2H), 7.33 (d, 2H), 4.54 (s, 2H), 4.01 (br d, 1H), 3.61 (d, 1H), 3.58-3.54 (m, 4H), 3.44 (tt, 1H), 3.34 (s, 3H), 3.22-3.18 (m, 4H), 3.09-2.94 (m, 3H), 2.33 (br d, 1H), 1.98 (q, 1H).

### Beispiel 239

### {3-(3-Methyl-1,2,4-oxadiazol-5-yl)-5-[4-(trifluonnethoxy)phenyl]piperidin-1-yl}(morpholin-4-yl)-methanon [enantiomerenreines cis-Isomer]

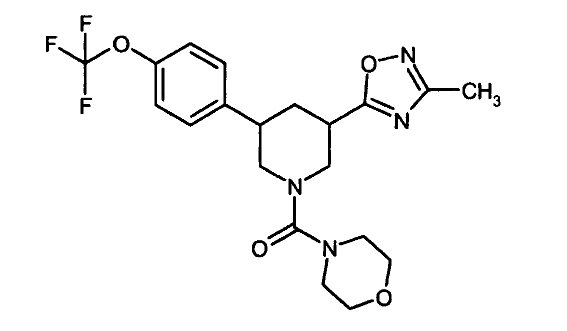

Enantiomerentrennung von 95 mg des Racemates aus Beispiel 237 nach Methode 9D ergab 42 mg der Titelverbindung aus Beispiel 239 und 41 mg der Titelverbindung aus Beispiel 240.

HPLC (Methode 7E): Rₜ = 17.58 min, >99.5% ee;

LC-MS (Methode 5B): Rₜ = 2.26 min; MS (ESIpos): m/z = 441 [M+H]⁺.

### Beispiel 240

### {3-(3-Methyl-1,2,4-oxadiazol-5-yl)-5-[4-(trifluormethoxy)phenyl]piperidin-1-yl}(morpholin-4-yl)-methanon [enantiomerenreines cis-Isomer]

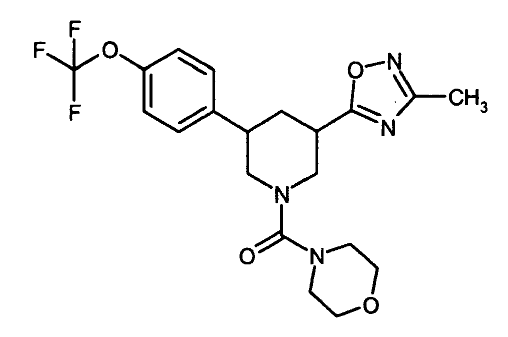

Enantiomerentrennung von 95 mg des Racemates aus Beispiel 237 nach Methode 9D ergab 42 mg der Titelverbindung aus Beispiel 239 und 41 mg der Titelverbindung aus Beispiel 240.

HPLC (Methode 7E): Rₜ = 42.67 min, >99.5% ee;

LC-MS (Methode 5B): Rₜ = 2.26 min; MS (ESIpos): m/z = 441 [M+H]⁺.

### Beispiel 241

### Morpholin-4-yl {-3-[3-(propan-2-yl)-1,2,4-oxadiazol-5-yl]-5-[4-(trifluormethoxy)phenyl]piperidin-1-yl}methanon [enantiomerenreines cis-Isomer]

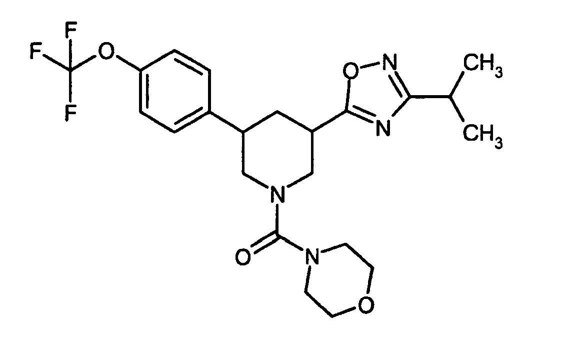

Enantiomerentrennung von 130 mg des Racemates aus Beispiel 236 nach Methode 9D ergab 52 mg der Titelverbindung aus Beispiel 241 und 55 mg der Titelverbindung aus Beispiel 242.

HPLC (Methode 7E): Rₜ = 12.40 min, >99.5% ee;

LC-MS (Methode 3B): Rₜ = 2.29 min; MS (ESIpos): m/z = 469[M+H]⁺.

### Beispiel 242

### Morpholin-4-yl{-3-[3-(propan-2-yl)-1,2,4-oxadiazol-5-yl]-5-[4-(trifluormethoxy)phenyl]piperidin-1-yl}methanon [enantiomerenreines cis-Isomer]

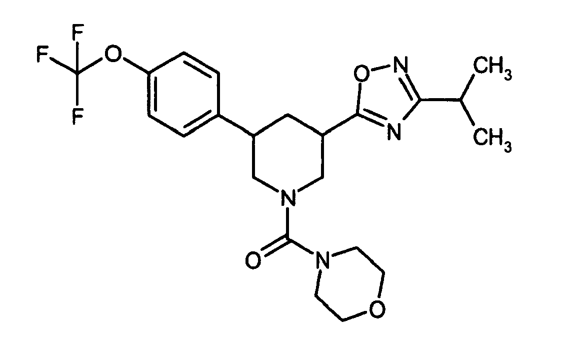

Enantiomerentrennung von 130 mg des Racemates aus Beispiel 236 nach Methode 9D ergab 52 mg der Titelverbindung aus Beispiel 241 und 55 mg der Titelverbindung aus Beispiel 242.

HPLC (Methode 7E): Rₜ = 19.54 min, >99.5% ee;

LC-MS (Methode 3B): Rₜ = 2.29 min; MS (ESIpos): m/z = 469[M+H]⁺.

### Beispiel 243

### Morpholin-4-yl {3-(3-propyl-1,2,4-oxadiazol-5-yl)-5-[4-(trifluormethoxy)phenyl]piperidin-1-yl}-methanon [racemisches cis-Isomer]

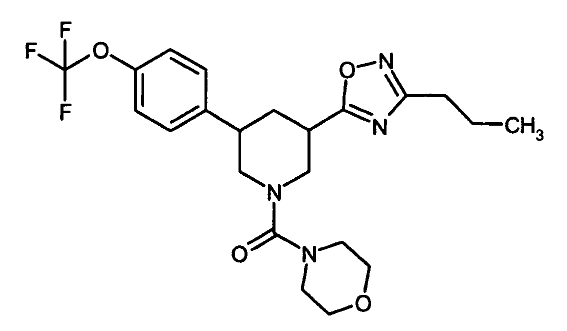

Nach der Allgemeinen Methode 2 wurden 150 mg (0.373 mmol) der Verbindung aus Beispiel 44A und 70 mg (0.746 mmol) *N*'-Hydroxybutanimidamid umgesetzt. Ausbeute: 174 mg (93% d. Th.).

LC-MS (Methode 2B): Rₜ = 1.39 min; MS (ESIpos): m/z = 469[M+H]⁺;

¹H-NMR (400 MHz, DMSO-d₆): δ = 7.47 (d, 2H), 7.33 (d, 2H), 3.98 (br d, 1H), 3.61 (d, 1H), 3.59-3.53 (m, 4H), 3.39 (tt, 1H), 3.22-3.16 (m, 4H), 3.06-2.94 (m, 3H), 2.67 (t, 2H), 2.31 (br d, 1H), 1.96 (q, 1H), 1.72-1.63 (m, 2H), 0.92 (t, 3H).

### Beispiel 244

### {3-(3-Ethyl-1,2,4-oxadiazol-5-yl)-5-[4-(trifluormethoxy)phenyl]piperidin-1-yl} (morpholin-4-yl)-methanon [racemisches cis-Isomer]

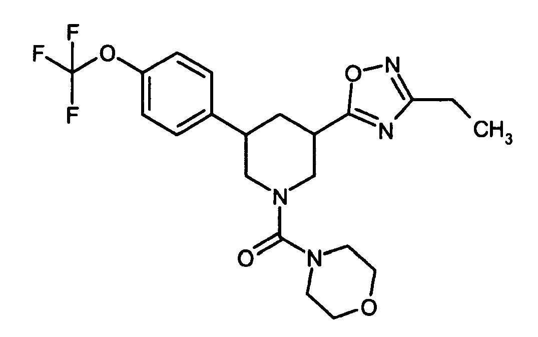

Nach der Allgemeinen Methode 2 wurden 150 mg (0.373 mmol) der Verbindungen aus Beispiel 44A und 66 mg (0.746 mmol) *N*'-Hydroxypropanimidamid umgesetzt. Ausbeute: 81 mg (93% d. Th.)

LC-MS (Methode 2B): Rₜ = 1.32 min; MS (ESIpos): m/z = 455 [M+H]⁺;

¹H-NMR (400 MHz, DMSO-d₆): δ = 7.47 (d, 2H), 7.33 (d, 2H), 3.98 (br d, 1H), 3.61 (d, 1H), 3.59-3.53 (m, 4H), 3.38 (tt, 1H), 3.22-3.16 (m, 4H), 3.07-2.94 (m, 3H), 2.70 (q, 2H), 2.30 (br d, 1H), 1.96 (q, 1H), 1.22 (t, 3H).

### Beispiel 245

### Morpholin-4-yl {3-[3-(pyrimidin-2-yl)-1,2,4-oxadiazol-5-yl]-5-[4-(trifluormethoxy)phenyl]-piperidin-1-yl}methanon [racemisches cis-Isomer]

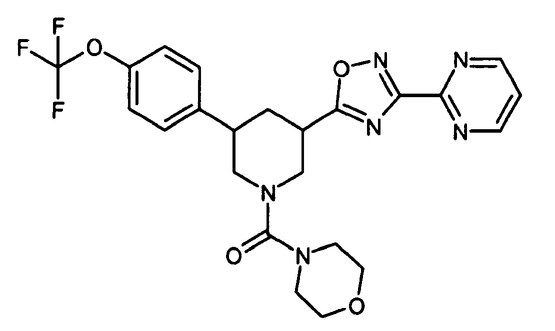

Nach der Allgemeinen Methode 2 wurden 150 mg (0.20 mmol) 1-(Morpholin-4-ylcarbonyl)-5-[4-(trifluormethoxy)phenyl]piperidin-3-carbonsäure (Beispiel 44A) und 77 mg (0.56 mmol) *N'-*Hydroxypyrimidin-2-carboximidamid umgesetzt. Ausbeute: 100 mg (51% d. Th.).

HPLC (Methode 3B): Rₜ = 1.84 min; MS (ESIpos): m/z = 505 [M+H]⁺;

¹H-NMR (400 MHz, DMSO-d₆): δ = 9.03 (d, 2H), 7.72 (t, 1H), 7.50 (d, 2H), 7.34 (d, 2H), 4.10 (br d, 1H), 3.65 (br d, 1H), 3.62-3.49 (m, 5H), 3.26-3.18 (m, 4H), 3.15 (t, 1H), 3.08-2.96 (m, 2H), 2.42 (br d, 1H), 2.08 (q, 1H).

### Beispiel 246

### Morpholin-4-yl(3-[4-(trifluormethoxy)phenyl]-5-{3-[3-(trifluormethyl)phenyl]-1,2,4-oxadiazol-5-yl}piperidin-1-yl)methanon [racemisches cis-Isomer]

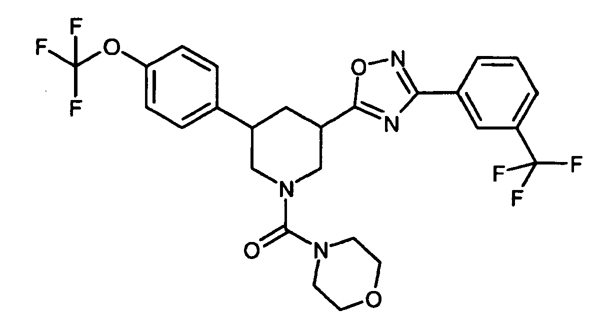

Nach der Allgemeinen Methode 2 wurden 150 mg (0.20 mmol) 1-(Morpholin-4-ylcarbonyl)-5-[4-(trifluormethoxy)phenyl]piperidin-3-carbonsäure (Beispiel 44A) und 114 mg (0.56 mmol, 1.5 eq.) *N*'-Hydroxy-3-(trifluormethyl)benzolcarboximidamid umgesetzt. Ausbeute: 123 mg (58% d. Th.).

HPLC (Methode 3B): Rₜ = 2.69 min; MS (ESIpos): m/z = 571 [M+H]⁺.

### Beispiel 247

### {3-(3-Cyclopropyl-1,2,4-oxadiazol-5-yl)-5-[4-(trifluormethoxy)phenyl)piperidin-1-yl}(morpholin-4-yl)methanon [racemisches cis-Isomer]

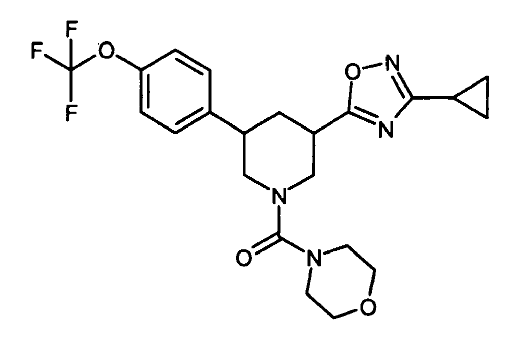

Nach der Allgemeinen Methode 2 wurden 150 mg (0.20 mmol) 1-(Morpholin-4-ylcarbonyl)-5-[4-(trifluormethoxy)phenyl]piperidin-3-carbonsäure (Beispiel 44A) und 60 mg (0.56 mmol, 1.5 eq.) *N*'-Hydroxycyclopropancarboximidamid umgesetzt. Ausbeute: 104 mg (59% d. Th.)

HPLC (Methode 3B): Rₜ = 2.22 min; MS (ESIpos): m/z = 467 [M+H]⁺;

¹H-NMR (400 MHz, DMSO-d₆): δ = 7.46 (d, 2H), 7.32 (d, 2H), 3.96 (br d, 1H), 3.65-3.49 (m, 6H), 3.26-3.39 (m, 1H), 3.19 (t, 4H), 3.09-2.91 (m, 3H), 2.07-2.15 (m, 1H), 1.94 (q, 1H), 1.01-1.10 (m, 2H), 0.85-0.92 (m, 2H).

### Beispiel 248

### {3-[3-(3,5-Difluorpyridin-2-yl)-1,2,4-oxadiazol-5-yl]-5-[4-(trifluormethoxy)phenyl]piperidin-1-yl}(morpholin-4-yl)methanon [racemisches cis-Isomer]

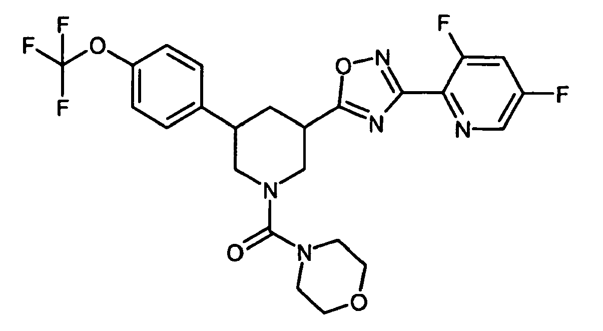

Nach der Allgemeinen Methode 2 wurden 150 mg (0.20 mmol) 1-(Morpholin-4-ylcarbonyl)-5-[4-(trifluormethoxy)phenyl]piperidin-3-carbonsäure (Beispiel 44A) und 91 mg (0.56 mmol) 3,5-Difluor-*N*'-hydroxypyridin-2-carboximidamid umgesetzt. Ausbeute: 131 mg (63% d. Th.).

HPLC (Methode 2B): Rₜ = 1.33 min; MS (ESIpos): m/z = 540 [M+H]⁺.

### Beispiel 249

### {3-[3-(5-Methylisoxazol-3-yl)-1,2,4-oxadiazol-5-yl]-5-[4-(trifluormethoxy)phenyl]piperidin-1-yl}(morpholin-4-yl)methanon [racemisches cis-Isomer]

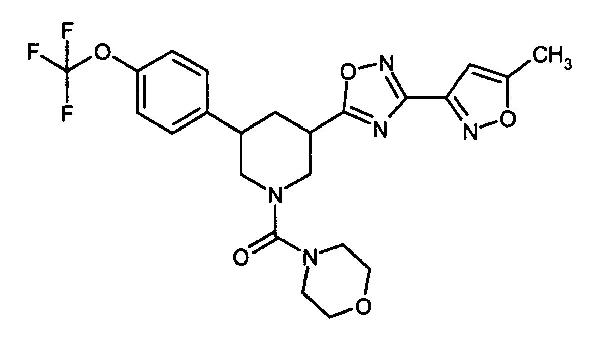

Nach der Allgemeinen Methode 2 wurden 150 mg (0.20 mmol) 1-(Morpholin-4-ylearbonyl)-5-[4-(trifluormethoxy)phenyl]piperidin-3-carbonsäure (Beispiel 44A) und 80 mg (0.56 mmol) *N'-*Hydroxy-5-methylisoxazol-3-carboximidamid umgesetzt. Ausbeute: 101 mg (54% d. Th.).

HPLC (Methode 2B): Rₜ = 1.35 min; MS (ESIpos): m/z = 508.1 [M+H]⁺;

¹H-NMR (400 MHz, DMSO-d₆): δ = 7.49 (d, 2H), 7.34 (d, 2H), 4.08 (br d, 1H), 3.63 (br d, 1H), 3.62-3.49 (m, 5H), 3.22 (t, 4H), 3.13 (t, 1H), 3.01 (bd, 2H), 2.42 (br d, 1H), 2.07 (q, 1H).

### Beispiel 250

### {3-[3-(2-Methoxyethyl)-1,2,4-oxadiazol-5-yl]-5-[4-(trifluormethoxy)phenyl]piperidin-1-yl}-(morpholin-4-yl)methanon [racemisches cis-Isomer]

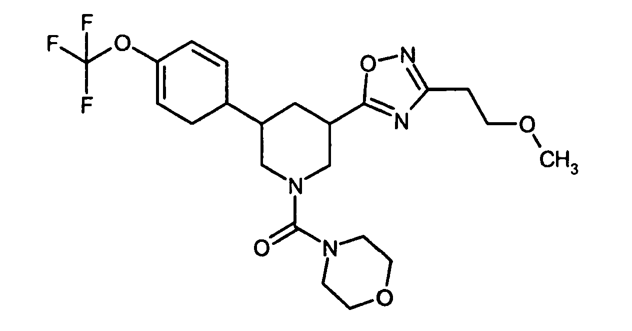

Nach der Allgemeinen Methode 2 wurden 150 mg (0.20 mmol) 1-(Morpholin-4-ylcarbonyl)-5-[4-(trifluormethoxy)phenyl]piperidin-3-carbonsäure (Beispiel 44A) und 66 mg (0.56 mmol) *N'-*Hydroxy-3-methoxypropanimidamid umgesetzt. Ausbeute: 77 mg (42% d. Th.).

HPLC (Methode 2B): Rₜ = 1.26 min; MS (ESIpos): m/z = 485 [M+H]⁺;

¹H-NMR (400 MHz, DMSO-d₆): δ = 7.47 (d, 2H), 7.33 (d, 1H), 3.99 (bd, 1H), 3.68 (t, 2H), 3.49-3.64 (m, 5H), 3.34-3.47 (m, 1H), 3.14-3.28 (m, 5H), 2.91-3.09 (m, 5H), 2.32 (br d, 1H), 1.98 (q, 1H).

### Beispiel 251

### {3-[3-(2-Methylpropyl)isoxazol-5-yl]-5-[4-(trifluormethoxy)phenyl]piperidin-1-yl}(morpholin-4-yl)methanon [racemisches cis-Isomer]

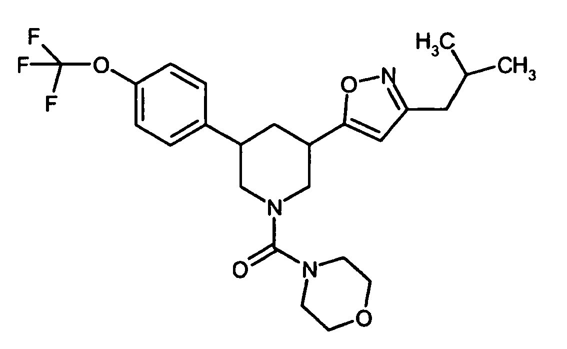

Zu einer Lösung von 72 mg (0.62 mmol) 4-Methylpentan-2-onoxim in 0.65 ml THF wurden bei 0°C 0.5 ml einer 2.5 M Lösung von n-Butyllithium in Hexan hinzugegeben. Das Reaktionsgemisch wurde 1 h bei 0°C gerührt. Anschließend wurde eine Lösung von 100 mg (ca. 0.24 mmol) der Verbindung aus Beispiel 43A in 0.45 ml THF zugetropft. Man ließ auf RT erwärmen und anschließend wurde über Nacht bei RT gerührt. Das Reaktionsgemisch wurde mit gesättigter, wässriger Natriumcarbonat-Lösung versetzt und mit Dichlormethan extrahiert. Die organische Phase wurde über Natriumsulfat getrocknet, filtriert und im Vakuum eingeengt. Der Rückstand wurde mittels präparativer HPLC gereinigt. Ausbeute: 51 mg (42% d. Th.).

LC-MS (Methode 9B): Rₜ = 1.14 min; MS (ESIpos): m/z = 482 [M+H]⁺.

### Beispiel 252

### (4-Hydroxypiperidin-1-yl){-3-(3-methyl-1,2,4-oxadiazol-5-yl)-5-[3-(trifluormethyl)phenyl]-piperidin-1-yl}methanon [racemisches cis-Isomer]

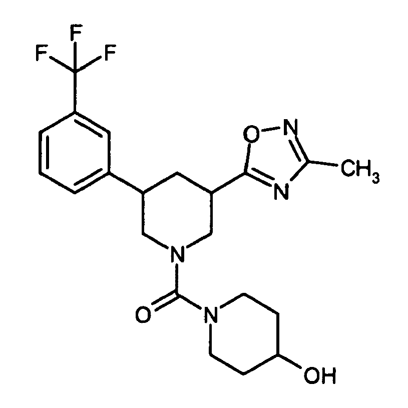

Nach der Allgemeinen Methode 2 wurden 150 mg (0.34 mmol) der Verbindung aus Beispiel 150A und 39 mg (0.51 mmol) *N*'-Hydroxyacetamidin umgesetzt. Ausbeute: 71 mg (48% d. Th.)

LC-MS (Methode 3B): Rₜ = 1.76 min; MS (ESIpos): m/z = 439 [M+H]⁺.

¹H-NMR (400 MHz, DMSO-d₆): δ = 7.70 (s, 1H), 7.66-7.56 (m, 3H), 4.68 (d, OH), 3.93 (br d, 1H), 3.63-3.35 (m, 4H), 3.07-2.97 (m, 3H), 2.93-2.82 (m, 3H), 2.33-2.30 (m, 4H), 2.03 (q, 1H), 1.73-1.70 (m, 2H), 1.32-1.29 (m, 2H).

### Beispiel 253

### (4-Hydroxypiperidin-1-yl){3-[3-(propan-2-yl)-1,2,4-oxadiazol-5-yl]-5-[3-(trifluormethyl)phenyl]-piperidin-1-yl}methanon [racemisches cis-Isomer]

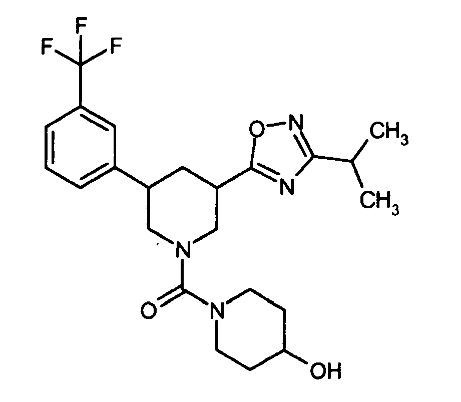

Nach der Allgemeinen Methode 2 wurden 75 mg (0.17 mmol) der Verbindung aus Beispiel 150A und 27 mg (0.25 mmol) *N*'-Hydroxy-2-methylpropanimidamid umgesetzt. Ausbeute: 26 mg (33% d. Th.)

LC-MS (Methode 3B): Rₜ = 2.02 min; MS (ESIpos): m/z = 467 [M+H]⁺;

¹H-NMR (400 MHz, DMSO-d₆): δ = 7.71 (s, 1H), 7.67-7.56 (m, 3H), 4.68 (d, OH), 3.94 (br d, 1H), 3.63-3.57 (m, 1H), 3.57 (br d, 1H), 3.50-3.47 (m, 2H), 3.41-3.34 (m, 1H), 3.08-3.01 (m, 4H), 2.91 (t, 2H), 2.33 (br d, 1H), 2.04 (q, 1H), 1.73-1.70 (m, 2H), 1.34-1.29 (m, 2H), 1.25 (d, 6H).

### Beispiel 254

### (3-Aminopyrrolidin-1-yl){3-(3-cyclopropyl-1,2,4-oxadiazol-5-yl)-5-[4-(trifluormethyl)phenyl]-piperidin-1-yl}methanon [Diastereomerengemisch]

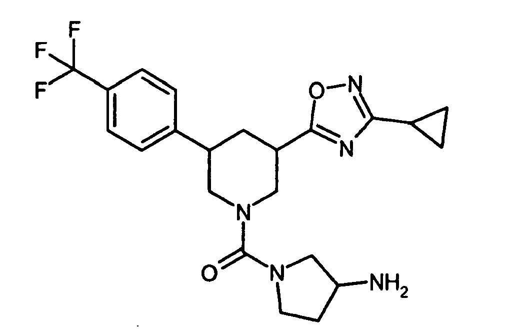

Nach der Allgemeinen Methode 6 wurden 100 mg (0.20 mmol) der Verbindung aus Beispiel 184A und 95 mg (0.60 mmol) 3-Aminopyrrolidin-Dihydrochlorid umgesetzt. Ausbeute: 25 mg (27% d. Th.)

HPLC (Methode 2A): Rₜ = 4.17 min; MS (ESIpos): m/z = 450 [M+H]⁺;

¹H-NMR (400 MHz, DMSO-d₆): δ = 7.69 (d, 2H), 7.56 (d, 2H), 4.02 (br d, 1H), 3.70 (br d, 1H), 3.48-3.40 (m, 2H), 3.01-2.91 (m, 4H), 2.29 (br d, 1H), 2.15-2.11 (m, 1H), 1.96 (q, 1H), 1.07-0.99 (m, 2H), 0.90-0.86 (m, 2H).

### Beispiel 255

### (4-Hydroxypiperidin-1-yl)-{3-(3-phenyl-1,2,4-oxadiazol-5-yl)-5-[4-trifluormethyl)phenyl]-piperidin-1-yl}methanon [racemisches cis-Isomer]

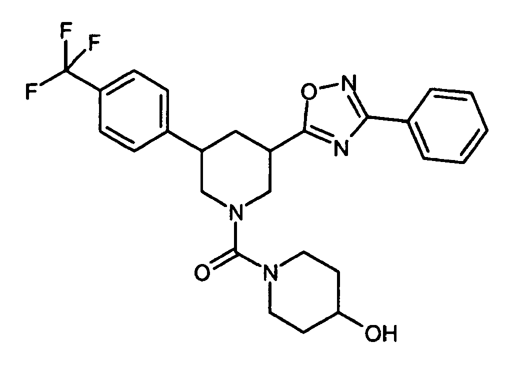

Nach der Allgemeinen Methode 1 wurden 100 mg (0.25 mmol) 1-[(4-Hydroxypiperidin-1-yl)-carbonyl]-5-[4-(trifluormethyl)phenyl]piperidin-3-carbonsäure (Beispiel 99A) und 39.4 mg (0.28 mmol) *N*'-Hydroxybenzolcarboximidamid (95%ig) umgesetzt. Ausbeute: 68.3 mg (53% d. Th.).

LC-MS (Methode1B): Rₜ = 2.62 min; MS (ESIpos): m/z = 501 [M+H]⁺;

¹H-NMR (400 MHz, DMSO-d₆): δ = 8.02 (dd, 2H), 7.72 (d, 2H), 7.63-7.54 (m, 5H), 4.69 (d, 1H), 4.05 (br d, 1H), 3.66-3.58 (m, 2H), 3.57-3.45 (m, 3H), 3.17-2.99 (m, 3H), 2.94 (br t, 2H), 2.43 (br d, 1H), 2.11 (dd, 1H), 1.73 (br d, 2H), 1.33 (br q, 2H).

### Beispiel 256

### {3-[3-(3-Chlorphenyl)-1,2,4-oxadiazol-5-yl]-5-[4-(trifluormethyl)phenyl]piperidin-1-yl}(4-hydroxy-piperidin-1-yl)methanon [racemisches cis-Isomer]

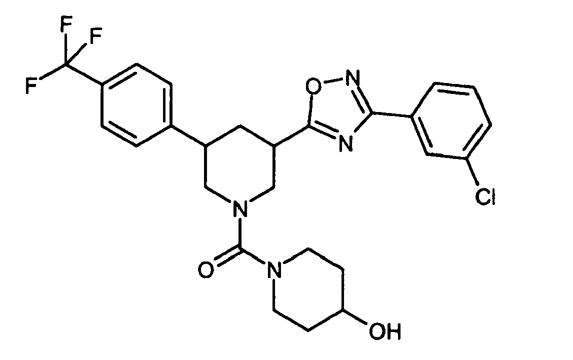

Nach der Allgemeinen Methode 1 wurden 100 mg (0.25 mmol) 1-[(4-Hydroxypiperidin-1-yl)-carbonyl]-5-[4-(trifluormethyl)phenyl]piperidin-3-carbonsäure (Beispiel 99A) und 46.9 mg (0.28 mmol) 3-Chlor-*N*'-hydroxybenzolcarboximidamid umgesetzt. Ausbeute: 69.5 mg (52% d. Th.).

LC-MS (Methode 2B): Rₜ = 1.49 min; MS (ESIpos): m/z = 553 [M+H]⁺;

¹H-NMR (400 MHz, DMSO-d₆): δ = 8.02-7.96 (m, 2H), 7.75-7.67 (m, 3H), 7.65-7.56 (m, 3H), 4.69 (d, 1H), 4.05 (br d, 1H), 3.67-3.56 (m, 2H), 3.56-3.45 (m, 3H), 3.17-3.02 (m, 3H), 2.93 (br t, 2H), 2.43 (br d, 1H), 2.11 (dd, 1H), 1.74 (br d, 2H), 1.33 (br q, 2H).

### Beispiel 257

### {3-[3-(3-Fluorphenyl)-1,2,4-oxadiazol-5-yl]-5-[4-(trifluormethyl)phenyl]piperidin-1-yl}(4-hydroxy-piperidin-1-yl)methanon [racemisches cis-Isomer]

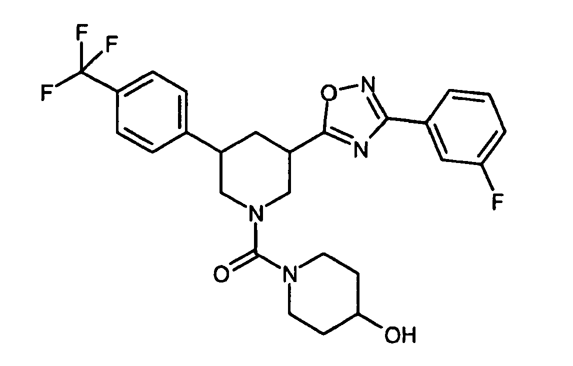

Nach der Allgemeinen Methode 1 wurden 100 mg (0.250 mmol) 1-[(4-Hydroxypiperidin-1-yl)-carbonyl]-5-[4-(trifluormethyl)phenyl]piperidin-3-carbonsäure (Beispiel 99A) und 42.3 mg (0.275 mmol) 3-Fluor-*N*'-hydroxybenzolcarboximidamid (Beispiel 73A) umgesetzt. Ausbeute: 63.9 mg (48% d. Th.).

LC-MS (Methode 2B): Rₜ = 1.41 min; MS (ESIpos): m/z = 519 [M+H]⁺;

¹H-NMR (400 MHz, DMSO-d₆): δ = 7.88 (br d, 2H), 7.76 (ddd, 1H), 7.72 (d, 2H), 7.68-7.61 (m, 1H), 7.59 (d, 2H), 7.47 (ddd, 1H), 4.69 (d, 1H), 4.05 (br d, 1H), 3.67-3.58 (m, 2H), 3.56-3.45 (m, 3H), 3.17-2.99 (m, 3H), 2.93 (br t, 2H), 2.43 (br d, 1H), 2.11 (dd, 1H), 1.73 (br d, 2H), 1.33 (br q, 2H).

### Beispiel 258

### {3-(3-Cyclopropyl-1,2,4-oxadiazol-5-yl)-5-[4-(trifluormethyl)phenyl]piperidin-1-yl}(4-hydroxypiperidin-1-yl)methanon [racemisches cis-Isomer]

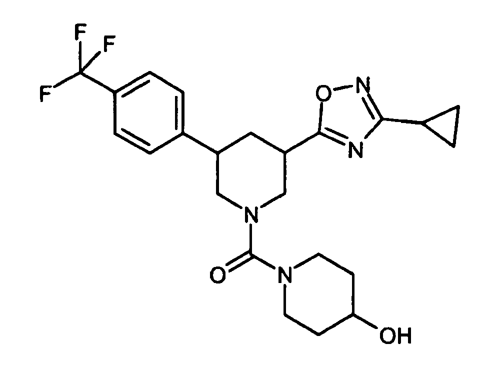

Nach der Allgemeinen Methode 1 wurden 100 mg (0.250 mmol) 1-[(4-Hydroxypiperidin-1-yl) carbonyl]-5-[4-(trifluormethyl)phenyl]piperidin-3-carbonsäure (Beispiel 99A) und 27.5 mg (0.275 mmol) *N*'-Hydroxycyclopropancarboximidamid (Beispiel 78A) umgesetzt. Ausbeute: 51.8 mg (45% d. Th.).

LC-MS (Methode 3B): Rₜ = 1.99 min; MS (ESIpos): m/z = 465 [M+H]⁺;

¹H-NMR (400 MHz, DMSO-d₆): δ = 7.70 (d, 2H), 7.56 (d, 2H), 4.68 (d, 1H), 3.92 (br d, 1H), 3.66-3.53 (m, 2H), 3.52-3.42 (m, 2H), 3.41-3.30 (m, 2H), 3.05-2.94 (m, 3H), 2.90 (br t, 2H), 2.30 (br d, 1H), 2.11 (sept, 1H), 1.97 (dd, 1H), 1.71 (br d, 2H), 1.30 (br q, 2H), 1.09-1.02 (m, 2H), 0.91-0.85 (m, 2H).

### Beispiel 259

### (4-Hydroxypiperidin-1-yl){(3-(3-methyl-1,2,4-oxadiazol-5-yl)-5-[4-trifluormethyl)phenyl]-piperidin-1-yl}methanon [racemisches cis-Isomer]

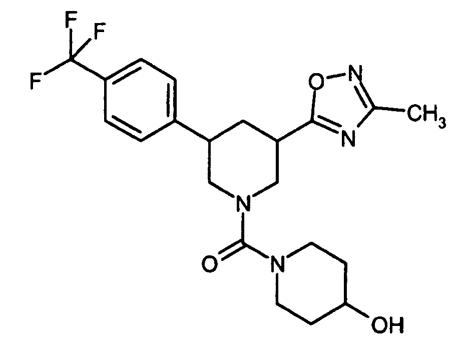

Nach der Allgemeinen Methode 1 wurden 100 mg (0.250 mmol) 1-[(4-Hydroxypiperidin-1-yl)-carbonyl]-5-[4-(trifluormethyl)phenyl]piperidin-3-carbonsäure (Beispiel 99A) und 20.4 mg (0.275 mmol) *N*'-Hydroxyacetamidin umgesetzt. Ausbeute: 57.9 mg (51% d. Th.).

LC-MS (Methode 3B): Rₜ = 1.78 min; MS (ESIpos): m/z = 439 [M+H]⁺;

¹H-NMR (400 MHz, DMSO-d₆): δ = 7.70 (d, 2H), 7.56 (d, 2H), 4.69 (d, 1H), 3.95 (br d, 1H), 3.66-3.54 (m, 2H), 3.52-3.44 (m, 2H), 3.40 (tt, 1H), 3.10-2.95 (m, 3H), 2.90 (br t, 2H), 2.33 (br s, 4H), 2.01 (dd, 1H), 1.72 (br d, 2H), 1.30 (br q, 2H).

### Beispiel 260

### (4-Hydroxypiperidin-1-yl){(3-(3-methyl-1,2,4-oxadiazol-5-yl)-5-[4-trifluormethyl)phenyl]-piperidin-1-yl}methanon [enantiomerenreines cis-Isomer]

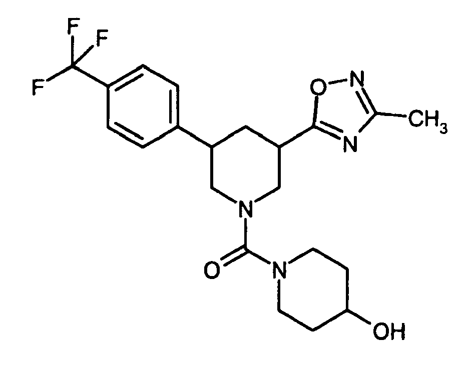

Enantiomerentrennung von 175 mg des Racemates aus Beispiel 259 nach Methode 10D ergab 73 mg der Titelverbindung aus Beispiel 260 und 73 mg der Titelverbindung aus Beispiel 261.

HPLC (Methode 18E): Rₜ = 5.09 min, >99% ee;

LC-MS (Methode 3B): Rₜ = 1.78 min; MS (ESIpos): m/z = 439 [M+H]⁺.

### Beispiel 261

### (4-Hydroxypiperidin-1-yl){(3-(3-methyl-1,2,4-oxadiazol-5-yl)-5-[4-trifluormethyl)phenyl]-piperidin-1-yl}methanon [enantiomerenreines cis-Isomer]

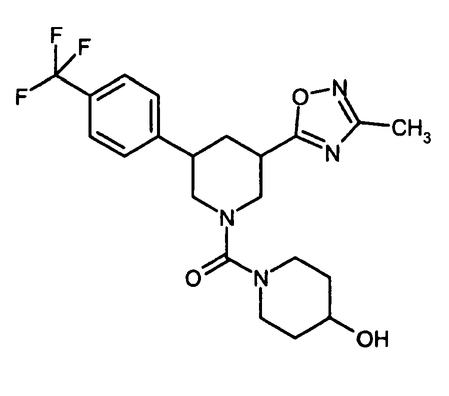

Enantiomerentrennung von 175 mg des Racemates aus Beispiel 259 nach Methode 10D ergab 73 mg der Titelverbindung aus Beispiel 260 und 73 mg der Titelverbindung aus Beispiel 261.

HPLC (Methode 18E): Rₜ = 19.4 min, >99% ee;

LC-MS (Methode 3B): Rₜ = 1.78 min; MS (ESIpos): m/z = 439 [M+H]⁺.

### Beispiel 262

### {3-(3-tert.-Butyl-1,2,4-oxadiazol-5-yl)-5-[4-(trifluormethyl)phenyl]piperidin-1-yl}(4-hydroxypiperidin-1-yl)methanon [racemisches cis-Isomer]

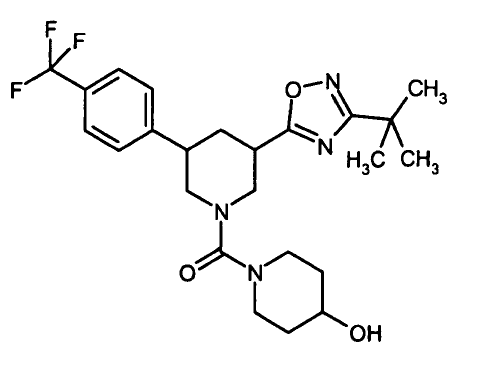

Nach der Allgemeinen Methode 1 wurden 100 mg (0.250 mmol) 1-[(4-Hydroxypiperidin-1-yl) carbonyl]-5-[4-(trifluormethyl)phenyl]piperidin-3-carbonsäure (Beispiel 99A) und 31.9 mg (0.275 mmol) *N*'-Hydroxy-2,2-dimethylpropanimidamid umgesetzt. Ausbeute: 55.6 mg (46% d. Th.).

LC-MS (Methode 3B): Rₜ = 2.20 min; MS (ESIpos): m/z = 481 [M+H]⁺.

¹H-NMR (400 MHz, DMSO-d₆): δ = 7.70 (d, 2H), 7.57 (d, 2H), 4.68 (d, 1H), 3.95 (br d, 1H), 3.66-3.53 (m, 2H), 3.52-3.44 (m, 2H), 3.39 (tt, 1H), 3.10-2.98 (m, 3H), 2.90 (br t, 2H), 2.33 (br d, 4H), 2.01 (dd, 1H), 1.72 (br d, 2H), 1.30 (br s, 2H).

### Beispiel 263

### (4-Hydroxypiperidin-1-yl){(3-[3-(propan-2-yl)-1,2,4-oxadiazol-5-yl]-5-[4-(trifluormethyl)phenyl]-piperidin-1-yl}methanon [racemisches cis-Isomer]

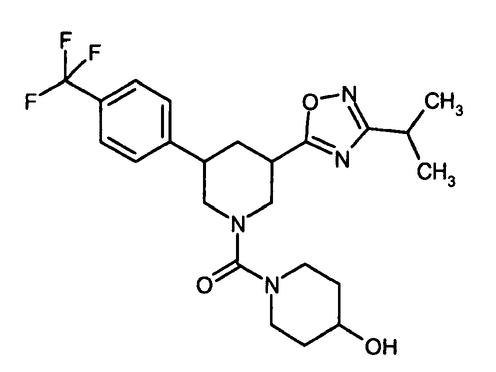

Nach der Allgemeinen Methode 1 wurden 100 mg (0.250 mmol) 1-[(4-Hydroxypiperidin-1-yl)-carbonyl]-5-[4-(trifluormethyl)phenyl]piperidin-3-carbonsäure (Beispiel 99A) und 28.1 mg (0.275 mmol) *N*'-Hydroxy-2-methylpropanimidamid umgesetzt. Ausbeute: 71.4 mg (60% d. Th.).

LC-MS (Methode1B): Rₜ = 2.40 min; MS (ESIpos): m/z = 467 [M+H]⁺;

¹H-NMR (400 MHz, DMSO-d₆): δ = 7.70 (d, 2H), 7.57 (d, 2H), 4.67 (d, 1H), 3.95 (br d, 1H), 3.66-3.54 (m, 2H), 3.53-3.44 (m, 2H), 3.40 (tt, 1H), 3.10-2.96 (m, 4H), 2.90 (br t, 2H), 2.33 (br d, 1H), 2.01 (dd, 1H), 1.72 (br d, 2H), 1.31 (br q, 2H), 1.25 (d, 6H).

### Beispiel 264

### (4-Hydroxypiperidin-1-yl){(3-[3-(propan-2-yl)-1,2,4-oxadiazol-5-yl]-5-[4-(trifluormethyl)phenyl]-piperidin-1-yl}methanon [enantiomerenreines cis-Isomer]

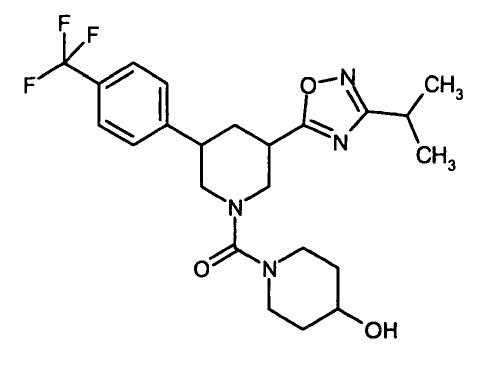

Enantiomerentrennung von 209 mg des Racemates aus Beispiel 263 nach Methode 10D ergab 90.4 mg der Titelverbindung aus Beispiel 264 und 94.5 mg der Titelverbindung aus Beispiel 265.

HPLC (Methode 18E) Rₜ = 4.34 min, >99% ee;

LC-MS (Methode 1B): Rₜ = 2.40 min; MS (ESIpos): m/z = 467 [M+H]⁺.

### Beispiel 265

### (4-Hydroxypiperidin-1-yl) {(3-[3-(propan-2-yl)-1,2,4-oxadiazol-5-yl]-5-[4-(trifluormethyl)phenyl]-piperidin-1-yl}methanon [enantiomerenreines cis-Isomer]

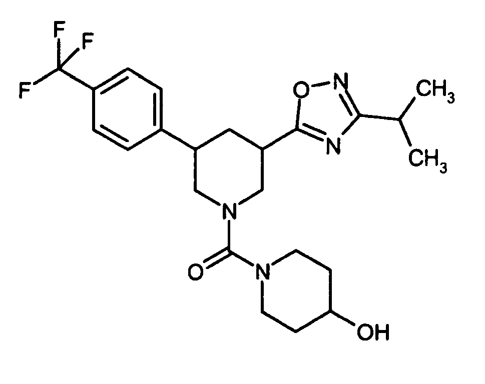

Enantiomerentrennung von 209 mg des Racemates aus Beispiel 263 nach Methode 10D ergab 90.4 mg der Titelverbindung aus Beispiel 264 und 94.5 mg der Titelverbindung aus Beispiel 265.

HPLC (Methode 18E): Rₜ = 7.37 min, >99% ee;

LC-MS (Methode 1B): Rₜ = 2.40 min; MS (ESIpos): m/z = 467 [M+H]⁺.

### Beispiel 266

### (4-Hydroxypiperidin-1-yl){3-[3-(pyridin-3-yl)-1,2,4-oxadiazol-5-yl]-5-[4-(trifluormethyl)phenyl]-piperidin-1-yl}methanon [racemisches cis-Isomer]

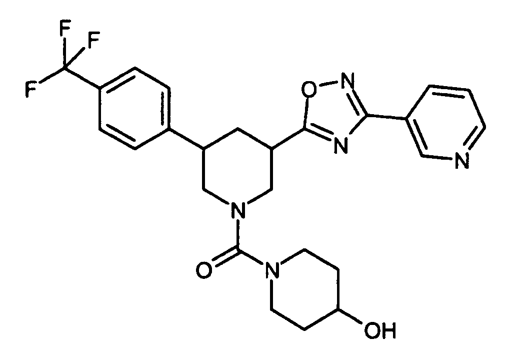

Nach der Allgemeinen Methode 1 wurden 100 mg (0.250 mmol) 1-[(4-Hydroxypiperidin-1-yl)-carbonyl]-5-[4-(trifluormethyl)phenyl]piperidin-3-carbonsäure (Beispiel 99A) und 37.7 mg (0.275 mmol) *N*'-Hydroxypyridin-3-carboximidamid umgesetzt. Ausbeute: 26.0 mg (21% d. Th.).

LC-MS (Methode 1B): Rₜ = 2.20 min; MS (ESIpos): m/z = 502 [M+H]⁺;

¹H-NMR (400 MHz, DMSO-d₆): δ = 9.17 (dd, 1H), 8.79 (dd, 1H), 8.37 (dt, 1H), 7.72 (d, 2H), 7.62 (ddd, 1H), 7.60 (d, 2H), 7.69 (d, 1H), 4.69 (d, 1H), 4.06 (br d, 1H), 3.67-3.45 (m, 5H), 3.18-3.00 (m, 3H), 2.93 (br t, 2H), 2.43 (br d, 1H), 2.12 (dd, 1H), 1.73 (br d, 2H), 1.33 (br q, 2H).

### Beispiel 267

### {3-(3-Benzyl-1,2,4-oxadiazol-5-yl)-5-[4-(trifluormethyl)phenyl]piperidin-1-yl}(4-hydroxypiperidin-1-yl)methanon [racemisches cis-Isomer]

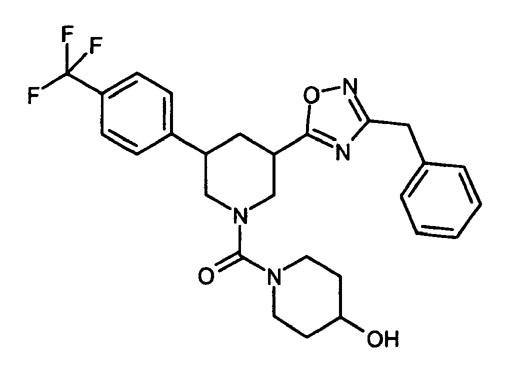

Nach der Allgemeinen Methode 1 wurden 100 mg (0.250 mmol) 1-[(4-Hydroxypiperidin-1-yl)-carbonyl]-5-[4-(trifluormethyl)phenyl]piperidin-3-carbonsäure (Beispiel 99A) und 41.3 mg (0.275 mmol) *N*'-Hydroxy-2-phenylethanimidamid umgesetzt. Ausbeute: 61.5 mg (47% d. Th.).

LC-MS (Methode 2B): Rₜ = 1.34 min; MS (ESIpos): m/z = 515 [M+H]⁺;

¹H-NMR (400 MHz, DMSO-d₆): δ = 7.69 (d, 2H), 7.55 (d, 2H), 7.36-7.22 (m, 5H), 4.67 (d, 1H), 4.09 (s, 2H), 3.94 (br d, 1H), 3.65-3.53 (m, 2H), 3.52-3.44 (m, 2H), 3.40 (tt, 1H), 3.07-2.94 (m, 3H), 2.90 (br t, 2H), 2.31 (br d, 1H), 1.99 (dd, 1H), 1.71 (br d, 2H), 1.30 (br q, 2H).

### Beispiel 268

### (4-Hydroxypiperidin-1-yl) {3-[3-(pyridin-4-yl)-1,2,4-oxadiazol-5-yl]-5-[4-(trifluormethyl)phenyl]-piperidin-1-yl}methanon [racemisches cis-Isomer]

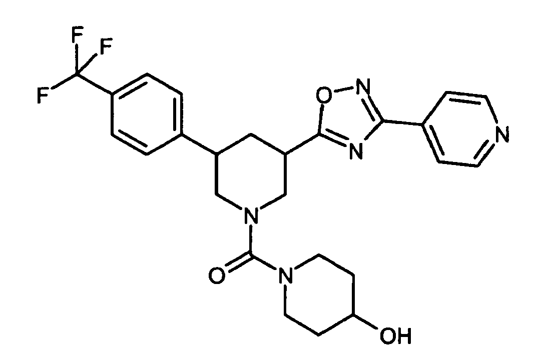

Nach der Allgemeinen Methode 1 wurden 100 mg (0.250 mmol) 1-[(4-Hydroxypiperidin-1-yl)-carbonyl]-5-[4-(trifluormethyl)phenyl]pipendin-3-carbonsäure (Beispiel 99A) und 37.7 mg (0.275 mmol) *N*'-Hydroxypyridin-4-carboximidamid umgesetzt. Ausbeute: 88.7 mg (69% d. Th.).

LC-MS (Methode 2B): Rₜ = 1.18 min; MS (ESIpos): m/z = 502 [M+H]⁺;

¹H-NMR (400 MHz, DMSO-d₆): δ = 8.83 (dd, 2H), 7.98 (dd, 2H), 7.72 (d, 2H), 7.59 (d, 2H), 4.03 (br d, 1H), 3.68-3.45 (m, 3H), 3.18-3.00 (m, 3H), 2.93 (br t, 2H), 2.44 (br d, 1H), 2.11 (dd, 2H), 1.73 (br d, 2H), 1.32 (br q, 2H).

### Beispiel 269

### {3-[3-(2-Fluorphenyl)-1,2,4-oxadiazol-5-yl]-5-[4-(trifluormethyl)phenyl]piperidin-1-yl}(4-hydroxypiperidin-1-yl)methanon [racemisches cis-Isomer]

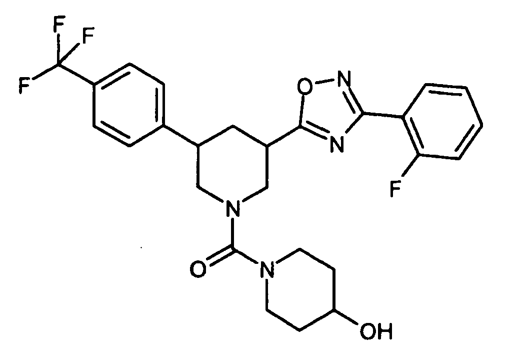

Nach der Allgemeinen Methode 1 wurden 100 mg (0.250 mmol) 1-[(4-Hydroxypiperidin-1-yl)-carbonyl]-5-[4-(trifluormethyl)phenyl]piperidin-3-carbonsäure (Beispiel 99A) und 42.3 mg (0.275 mmol) 2-Fluor-*N*'-hydroxybenzolcarboximidamid umgesetzt. Ausbeute: 55.6 mg (42% d. Th.).

LC-MS (Methode 3B): Rₜ = 2.19 min; MS (ESIpos): m/z = 519 [M+H]⁺;

¹H-NMR (400 MHz, DMSO-d₆): δ = 8.03 (dt, 1H), 7.72 (d, 2H), 7.70-7.63 (m, 1H), 7.59 (d, 2H), 4.69 (d, 1H), 4.05 (br d, 1H), 3.66-3.45 (m, 5H), 3.17-2.99 (m, 3H), 2.93 (br t, 2H), 2.43 (br d, 1H), 2.11 (dd, 1H), 1.74 (br d, 2H), 1.33 (br q, 2H).

### Beispiel 270

### {3-[3-(4-Fluorphenyl)-1,2,4-oxadiazol-5-yl]-5-[4-(trifluormethyl)phenyl]piperidin-1-yl}(4-hydroxypiperidin-1-yl)methanon [racemisches cis-Isomer]

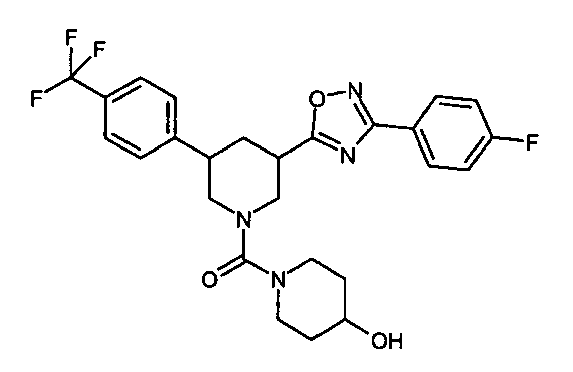

Nach der Allgemeinen Methode 1 wurden 100 mg (0.250 mmol) 1-[(4-Hydroxypiperidin-1-yl)-carbonyl]-5-[4-(trifluormethyl)phenyl]piperidin-3-carbonsäure (Beispiel 99A) und 42.3 (0.275 mmol) 4-Fluor-*N*'-hydroxybenzolcarboximidamid umgesetzt. Ausbeute: 59.1 mg (46% d. Th.).

LC-MS (Methode 3B): Rₜ = 2.29 min; MS (ESIpos): m/z = 519 [M+H]⁺;

¹H-NMR (400 MHz, DMSO-d₆): δ = 8.07 (m_{c}, 2H), 7.72 (d, 2H), 7.59 (d, 2H), 7.41 (tt, 2H), 4.69 (d, 1H), 4.05 (br d, 1H), 3.67-3.57 (m, 2H), 3.56-3.45 (m, 3H), 3.16-2.98 (m, 3H), 2.93 (br t, 2H), 2.42 (br d, 1H), 2.10 (dd, 1H), 1.73 (br d, 2H), 1.32 (br q, 2H).

### Beispiel 271

### (4-Hydroxypiperidin-1-yl) {3-{3-[(methylsulfonyl)methyl]-1,2,4-oxadiazol-5-yl}-5-[4-(trifluormethyl)phenyl]piperidin-1-yl}methanon [racemisches cis-Isomer]

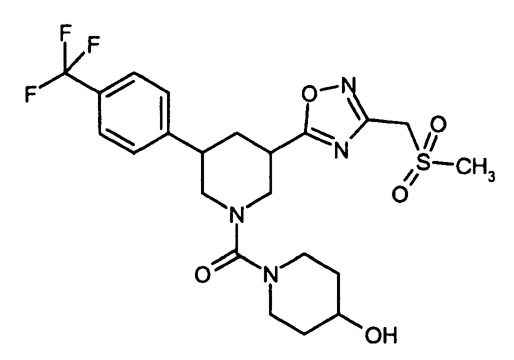

Nach der Allgemeinen Methode 1 wurden 100 mg (0.250 mmol) 1-[(4-Hydroxypiperidin-1-yl)-carbonyl]-5-[4-(trifluormethyl)phenyl]piperidin-3-carbonsäure (Beispiel 99A) und 41.8 mg (0.275 mmol) *N*'-Hydroxy-2-(methylsulfonyl)ethanimidamid umgesetzt. Ausbeute: 20.6 mg (16% d. Th.).

LC-MS (Methode 1B): Rₜ = 2.03 min; MS (ESIpos): m/z = 517 [M+H]⁺;

¹H-NMR (400 MHz, DMSO-d₆): δ = 7.71 (d, 2H), 7.57 (d, 2H), 4.85 (s, 2H), 4.69 (d, 1H), 3.99 (br d, 1H), 3.67-3.55 (m, 2H), 3.55-3.44 (m, 3H), 3.17 (s, 3H), 3.03 (quin, 3H), 2.92 (br t, 2H), 2.37 (br d, 1H), 2.04 (dd, 1H), 1.72 (br d, 2H), 1.31 (br q, 2H).

### Beispiel 272

### {3-[3-(2,4-Difluorphenyl)-1,2,4-oxadiazol-5-yl]-5-[4-(trifluormethyl)phenyl]piperidin-1-yl}(4-hydroxypiperidin-1-yl)methanon [racemisches cis-Isomer]

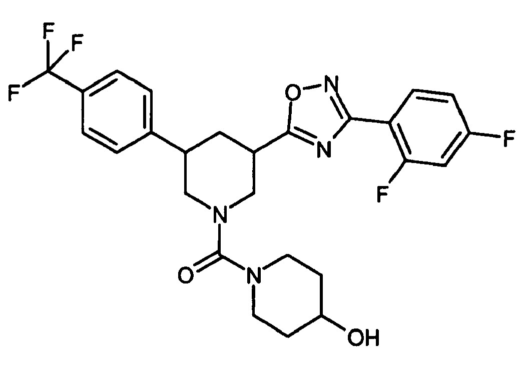

Nach der Allgemeinen Methode 1 wurden 100 mg (0.250 mol) 1-[(4-Hydroxypiperidin-1-yl)-carbonyl]-5-[4-(trifluormethyl)phenyl]piperidin-3-carbonsäure (Beispiel 99A) und 47.3 (0.275 mmol) 2,4-Difluor-*N*'-hydroxybenzolcarboximidamid umgesetzt. Ausbeute: 52.1 mg (38% d. Th.).

LC-MS (Methode 1B): Rₜ = 2.62 min; MS (ESIpos): m/z = 537 [M+H]⁺;

¹H-NMR (400 MHz, DMSO-d₆): δ = 8.09 (m_{c} 1H), 7.72 (d, 2H), 7.59 (d, 2H), 7.54 (m_{c}, 1H), 7.32 (dt, 1H), 4.69 (d, 1H), 4.05 (br d, 1H), 3.67-3.56 (m, 2H), 3.55-3.45 (m, 3H), 3.17-2.99 (m, 3H), 2.93 (br t, 2H), 2.42 (br d, 1H), 2.10 (dd, 1H), 1.73 (br d, 2H), 1.33 (br q, 2H).

### Beispiel 273

### {3-[3-(2,5-Difluorphenyl)-1,2,4-oxadiazol-5-yl]-5-[4-(trifluormethyl)phenyl]piperidin-1-yl}(4-hydroxypiperidin-1-yl)methanon [racemisches cis-Isomer]

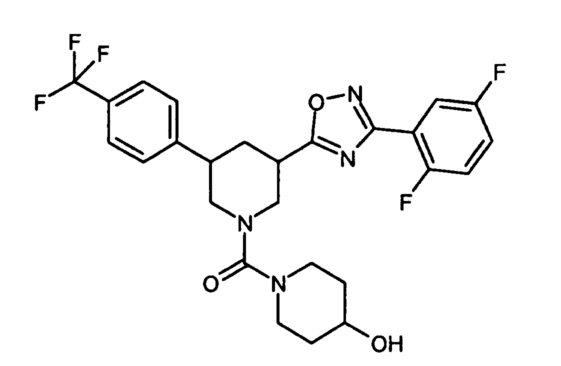

Nach der Allgemeinen Methode 1 wurden 100 mg (0.250 mmol) 1-[(4-Hydroxypiperidin-1-yl)-carbonyl]-5-[4-(trifluormethyl)phenyl]piperidin-3-carbonsäure (Beispiel 99A) und 47.3 (0.275 mmol) 2,5-Difluor-*N*'-hydroxybenzolcarboximidamid (Beispiel 74A) umgesetzt. Ausbeute: 68.1 mg (51% d. Th.).

LC-MS (Methode 1B): Rₜ = 2.62 min; MS (ESIpos): m/z = 537 [M+H]⁺;

¹H-NMR (400 MHz, DMSO-d₆): δ = 7.82-7.75 (m, 1H), 7.72 (d, 2H), 7.59 (d, 2H), 7.54 (dt, 2H), 4.69 (d, 1H), 4.05 (br d, 1H), 3.68-3.45 (m, 5H), 3.17-2.99 (m, 3H), 2.93 (br t, 2H), 2.43 (br d, 1H), 2.11 (dd, 1H), 1.73 (br d, 2H), 1.33 (br q, 2H).

### Beispiel 274

### {3-[3-(2-Chlorphenyl)-1,2,4-oxadiazol-5-yl]-5-[4-(trifluormethyl)phenyl]piperidin-1-yl}(4-hydroxypiperidin-1-yl)methanon [racemisches cis-Isomer]

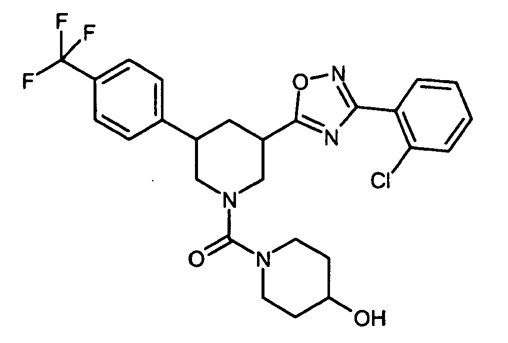

Nach der Allgemeinen Methode 1 wurden 100 mg (0.250 mmol) 1-[(4-Hydroxypiperidin-1-yl)-carbonyl]-5-[4-(trifluormethyl)phenyl]piperidin-3-carbonsäure (Beispiel 99A) und 46.9 (0.275 mmol) 2-Chlor-*N*'-hydroxybenzolcarboximidamid umgesetzt. Ausbeute: 53.0 mg (39% d. Th.).

LC-MS (Methode 1B): Rₜ = 2.65 min; MS (ESIpos): m/z = 535 [M+H]⁺;

¹H-NMR (400 MHz, DMSO-d₆): δ = 7.92 (dd, 1H), 7.72 (d, 2H), 7.69 (dd, 1H), 7.64-7.57 (m, 3H), 7.54 (dt, 1H), 4.69 (d, 1H), 4.06 (br d, 1H), 3.67-3.45 (m, 5H), 3.17-2.99 (m, 3H), 2.93 (br t, 2H), 2.44 (br d, 1H), 2.10 (dd, 1H), 1.74 (br d, 2H), 1.33 (br q, 2H).

### Beispiel 275

### (4-Hydroxypiperidin-1-yl){3-[3-(2-methoxyethyl)-1,2,4-xadiazol-5-yl]-5-[4-(trifluormethyl)-phenyl]piperidin-1-yl}methanon [enantiomerenreines cis-Isomer]

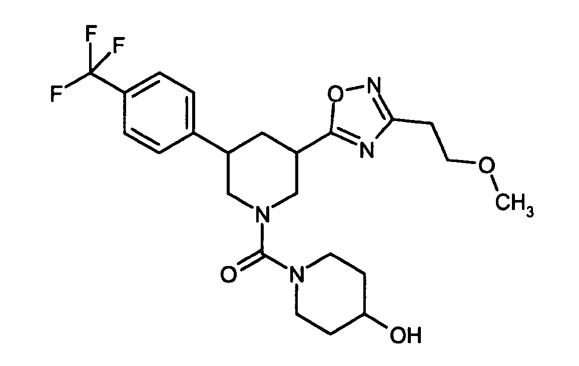

Nach der Allgemeinen Methode 1 wurden 250 mg (0.624 mmol) 1-[(4-Hydroxypiperidin-1-yl)-carbonyl]-5-[4-(trifluormethyl)phenyl]piperidin-3-carbonsäure (Beispiel 99A) und 108 mg (0.687 mmol, 75% Reinheit) *N*'-Hydroxy-3-methoxypropanimidamid (Beispiel 64A) umgesetzt. Enantiomerentrennung des Racemates nach Methode 16D ergab 56.0 mg der Titelverbindung aus Beispiel 275 und 55.0 mg der Titelverbindung aus Beispiel 276 (36% d. Th.).

HPLC (Methode 18E): Rₜ = 5.70 min, >99.5% ee;

LC-MS (Methode 2B): Rₜ = 1.15 min; MS (ESIpos): m/z = 483 [M+H]⁺;

¹H-NMR (400 MHz, DMSO-d₆): δ = 7.70 (d, 2H), 7.57 (d, 2H), 4.68 (d, 1H), 3.95 (br d, 1H), 3.68 (t, 2H), 3.65-3.53 (m, 2H), 3.52-3.36 (m, 3H), 3.23 (s, 3H), 3.09-2.98 (m, 3H), 2.98-2.86 (m, 4H), 2.33 (br d, 1H), 2.01 (dd, 1H), 1.73 (br d, 2H), 1.31 (br q, 2H).

### Beispiel 276

### (4-Hydroxypiperidin-1-yl){3-[3-(2-methoxyethyl)-1,2,4-oxadiazol-5-yl]-5-[4-(trifluormethyl)-phenyl]piperidin-1-yl}methanon [enantiomerenreines cis-Isomer]

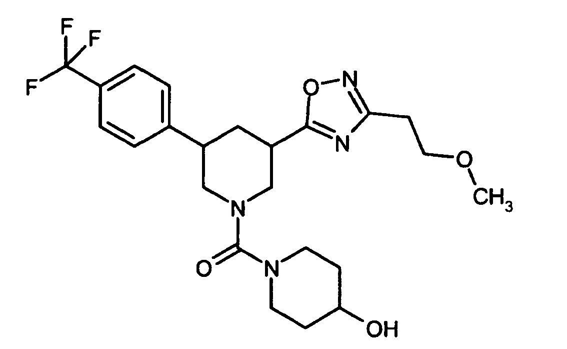

Nach der Allgemeinen Methode 1 wurden 250 mg (0.624 mmol) 1-[(4-Hydroxypiperidin-1-yl)-carbonyl]-5-[4-(trifluormethyl)phenyl]piperidin-3-carbonsäure (Beispiel 99A) und 108 mg (0.687 mmol, 75% Reinheit) *N*'-Hydroxy-3-methoxypropanimidamid (Beispiel 64A) umgesetzt. Enantiomerentrennung des Racemates nach Methode 16D ergab 56.0 mg der Titelverbindung aus Beispiel 275 und 55.0 mg der Titelverbindung aus Beispiel 276 (36% d. Th.).

HPLC (Methode 18E): Rₜ = 17.4 min, >99.5% ee;

LC-MS (Methode 2B): Rₜ = 1.15 min; MS (ESIpos): m/z = 483 [M+H]⁺.

### Beispiel 277

### 1-({3-[3-(2-Methoxyethyl)-1,2,4-oxadiazol-5-yl]-5-[4-(trifluormethyl)phenyl]piperidin-1-yl}-carbonyl)piperidin-4-carbonitril [enantiomerenreines cis-Isomer]

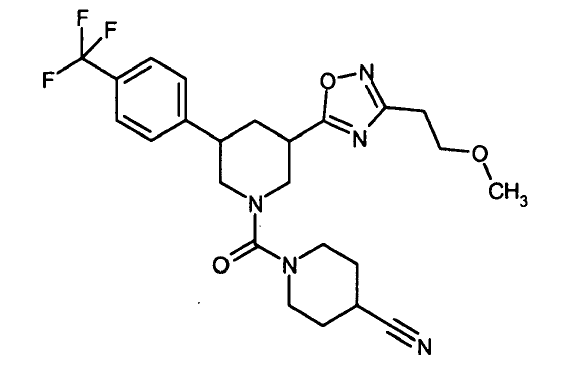

Nach der Allgemeinen Methode 1 wurden 250 mg (0.611 mmol) 1-[(4-Cyanpiperidin-1-yl)carbonyl]-5-[4-(trifluormethyl)phenyl]piperidin-3-carbonsäure (Beispiel 100A) und 106 mg (0.672 mmol, 75% Reinheit) *N*'-Hydroxy-3-methoxypropanimidamid (Beispiel 64A) umgesetzt. Enantiomerentrennung des Racemates nach Methode 16D ergab 77.0 mg der Titelverbindung aus Beispiel 277 und 70.0 mg der Titelverbindung aus Beispiel 278 (47% d. Th.).

HPLC (Methode 16E): Rₜ = 6.88 min, >99.0% ee;

LC-MS (Methode 2B): Rₜ = 1.26 min; MS (ESIpos): m/z = 492 [M+H]⁺;

¹H-NMR (400 MHz, DMSO-d₆): δ = 7.70 (d, 2H), 7.57 (d, 2H), 3.98 (br d, 1H), 3.68 (t, 2H), 3.59 (br d, 1H), 3.43-3.34 (m, 3H), 3.23 (s, 3H), 3.12-2.99 (m, 6H), 2.93 (t, 2H), 2.34 (br d, 1H), 2.02 (dd, 1H), 1.91 (m, 2H), 1.74-1.62 (m, 2H).

### Beispiel 278

### 1-({3-[3-(2-Methoxyethyl)-1,2,4-oxadiazol-5-yl]-5-[4-(trifluormethyl)phenyl]piperidin-1-yl}-carbonyl)piperidin-4-carbonitril [enantiomerenreines cis-Isomer]

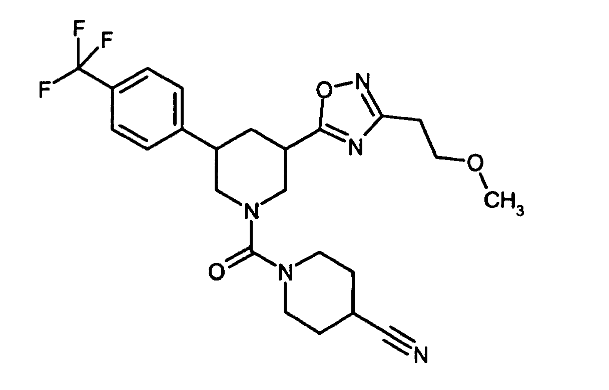

Nach der Allgemeinen Methode 1 wurden 250 mg (0.611 mmol) 1-[(4-Cyanpiperidin-1-yl)carbonyl]-5-[4-(trifluormethyl)phenyl]piperidin-3-carbonsäure (Beispiel 100A) und 106 mg (0.672 mmol, 75% Reinheit) *N*'-Hydroxy-3-methoxypropanimidamid (Beispiel 64A) umgesetzt. Enantiomerentrennung des Racemates nach Methode 16D ergab 77.0 mg der Titelverbindung aus Beispiel 277 und 70.0 mg der Titelverbindung aus Beispiel 278 (47% d. Th.).

HPLC (Methode 16E): Rₜ = 9.41 min, >98.0% ee;

LC-MS (Methode 2B): Rₜ = 1.26 min; MS (ESIpos): m/z = 492 [M+H]⁺.

### Beispiel 279

### (3-Hydroxyazetidin-1-yl){3-[3-(2-methoxyethyl)-1,2,4-oxadiazol-5-yl]-5-[4-trifluormethyl)-phenyl]piperidin-1-yl}methanon [enantiomerenreines cis-Isomer]

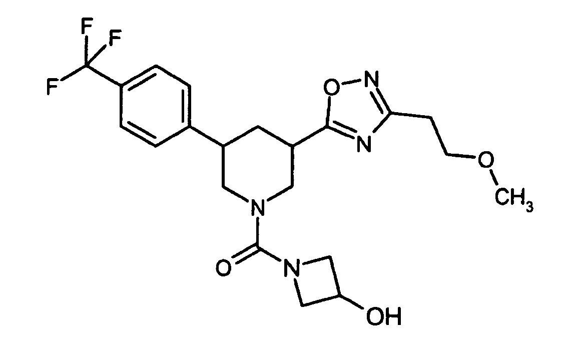

Nach der Allgemeinen Methode 1 wurden 250 mg (0.671 mmol) 1-[(3-Hydroxyazetidin-1-yl)carbonyl]-5-[4-(trifluormethyl)phenyl]piperidin-3-carbonsäure (Beispiel 101A) und 116 mg (0.739 mmol, 75% Reinheit) *N*'-Hydroxy-3-methoxypropanimidamid (Beispiel 64A) umgesetzt. Enantiomerentrennung des Racemates nach Methode 10D ergab 40.0 mg der Titelverbindung aus Beispiel 279 und 30.0 mg der Titelverbindung aus Beispiel 280 (22% d. Th.).

HPLC (Methode 16E): Rₜ = 4.89 min, >99.0% ee; Rₜ = 1.11 min;

LC-MS (Methode 2B): Rₜ = 1.11 min; MS (ESIpos): m/z = 455 [M+H]⁺;

¹H-NMR (400 MHz, DMSO-d₆): δ = 7.70 (d, 2H), 7.56 (d, 1H), 5.58 (d, 1H), 4.43-4.34 (m, 1H), 4.16 (br d, 1H), 4.10 (q, 2H), 3.79-3.65 (m, 5H), 3.23 (s, 3H), 3.07-2.90 (m, 5H), 2.32 (br d, 1H), 2.04 (dd, 1H).

### Beispiel 280

### (3-Hydroxyazetidin-1-yl){3-[3-(2-methoxyethyl)-1,2,4-oxadiazol-5-yl]-5-[4-trifluormethyl)-phenyl]piperidin-1-yl}methanon [enantiomerenreines cis-Isomer]

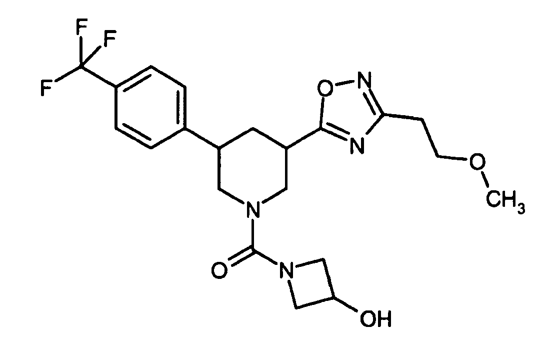

Nach der Allgemeinen Methode 1 wurden 250 mg (0.671 mmol) 1-[(3-Hydroxyazetidin-1-yl)carbonyl]-5-[4-(trifluormethyl)phenyl]piperidin-3-carbonsäure (Beispiel 101A) und 116 mg (0.739 mmol, 75% Reinheit) *N*'-Hydroxy-3-methoxypropanimidamid (Beispiel 64A) umgesetzt. Enantiomerentrennung des Racemates nach Methode 10D ergab 40.0 mg der Titelverbindung aus Beispiel 279 und 30.0 mg der Titelverbindung aus Beispiel 280 (22% d. Th.).

HPLC (Methode 16E): Rₜ = 7.62 min, >99.0% ee;

LC-MS (Methode 2B): Rₜ = 1.11 min; MS (ESIpos): m/z = 455 [M+H]⁺.

### Beispiel 281

### 1-({3-(3-Cyclopropyl-1,2,4-oxadiazol-5-yl)-5-[4-(trifluormethyl)phenyl]piperidin-1-yl} carbonyl)-piperidin-4-carbonitril [racemisches cis-Isomer]

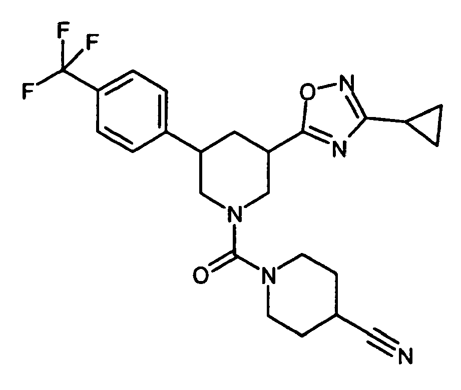

Nach der Allgemeinen Methode 1 wurden 100 mg (0.244 mmol) 1-[(4-Cyanpiperidin-1-yl)carbonyl]-5-[4-(trifluormethyl)phenyl]piperidin-3-carbonsäure (Beispiel 100A) und 26.9 mg (0.269 mmol) *N'*-Hydroxycyclopropancarboximidamid (Beispiel 78A) umgesetzt. Ausbeute: 78.3 mg (66% d. Th.).

LC-MS (Methode 1B): Rₜ = 2.60 min; MS (ESIpos): m/z = 474 [M+H]⁺;

¹H-NMR (400 MHz, DMSO-d₆): δ = 7.70 (dd, 2H), 7.56 (d, 2H), 3.93 (br d, 1H), 3.58 (d, 1H), 3.41-3.31 (m, 3H), 3.12-2.95 (m, 6H), 2.30 (br d, 1H), 2.11 (sept, 1H), 1.97 (dd, 1H), 1.90-1.81 (m, 2H), 1.74-1.62 (m, 2H), 1.09-1.02 (m, 2H), 0.91-0.85 (m, 2H).

### Beispiel 282

### 1-({3-(3-Methyl-1,2,4-oxadiazol-5-yl)-5-[4-(trifluormethyl)phenyl]piperidin-1-yl}carbonyl)-piperidin-4-carbonitril [racemisches cis-Isomer]

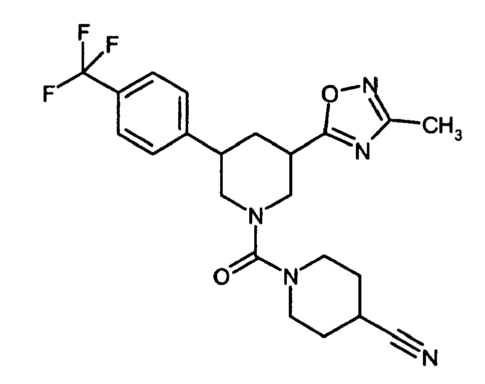

Nach der Allgemeinen Methode 1 wurden 100 mg (0.244 mmol) 1-[(4-Cyanpiperidin-1-yl)carbonyl]-5-[4-(trifluormethyl)phenyl]piperidin-3-carbonsäure (Beispiel 100A) und 19.9 mg (0.269 mmol) *N*'-Hydroxyethanimidamid umgesetzt. Ausbeute: 55.5 mg (47% d. Th.).

LC-MS (Methode 1B): Rₜ = 2.39 min; MS (ESIpos): m/z = 448 [M+H]⁺;

¹H-NMR (400 MHz, DMSO-d₆): δ = 7.70 (dd, 2H), 7.56 (d, 2H), 3.96 (br d, 1H), 3.60 (d, 1H), 3.45-3.32 (m, 3H), 3.13-2.94 (m, 6H), 2.33 (br s, 4H), 2.01 (dd, 1H), 1.91-1.82 (m, 2H), 1.74-1.63 (m, 2H).

### Beispiel 283

### 1-({3-(3-tert-Butyl-1,2,4-oxadiazol-5-yl)-5-[4-(trifluormethyl)phenyl]piperidin-1-yl}carbonyl)-piperidin-4-carbonitril [racemisches cis-Isomer]

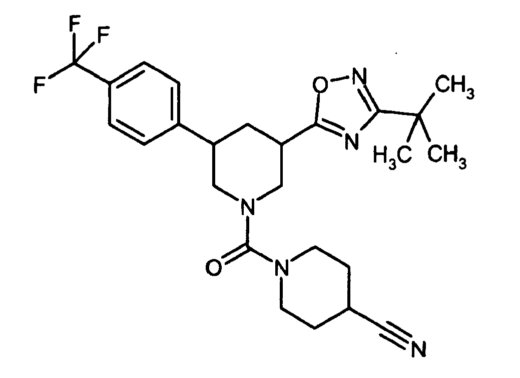

Nach der Allgemeinen Methode 1 wurden 100 mg (0.244 mmol) 1-[(4-Cyanpiperidin-1-yl)carbonyl]-5-[4-(trifluormethyl)phenyl]piperidin-3-carbonsäure (Beispiel 100A) und 31.2 mg (0.269 mmol) *N*'-Hydroxy-2,2-dimethylpropanimidamid umgesetzt. Ausbeute: 79.1 mg (66% d. Th.).

LC-MS (Methode 1B): Rₜ = 2.80 min; MS (ESIpos): m/z = 490 [M+H]⁺;

¹H-NMR (400 MHz, DMSO-d₆): δ = 7.70 (dd, 2H), 7.58 (d, 2H), 3.96 (br d, 1H), 3.58 (d, 1H), 3.45-3.32 (m, 3H), 3.13-2.98 (m, 6H), 2.36-2.29 (m, 1H), 2.01 (dd, 1H), 1.91-1.82 (m, 2H), 1.68 (dd, 2H), 1.30 (s, 9H).

### Beispiel 284

### 1-({3-[3-(Propan-2-yl)-1,2,4-oxadiazol-5-yl]-5-[4-(trifluormethyl)phenyl]piperidin-1-yl}carbonyl)-piperidin-4-carbonitril [racemisches cis-Isomer]

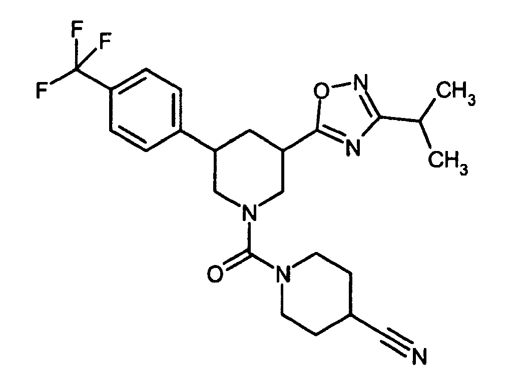

Nach der Allgemeinen Methode 1 wurden 100 mg (0.244 mmol) 1-[(4-Cyanpiperidin-1-yl)carbonyl]-5-[4-(trifluormethyl)phenyl]piperidin-3-carbonsäure (Beispiel 100A) 27.4 mg (0.269 mmol) *N*'-Hydroxy-2-methylpropanimidamid umgesetzt. Ausbeute: 78.9 mg (68% d. Th.).

LC-MS (Methode 1B): Rₜ = 2.66 min; MS (ESIpos): m/z = 477 [M+H]⁺;

¹H-NMR (400 MHz, DMSO-d₆): δ = 7.70 (dd, 2H), 7.57 (d, 2H), 3.96 (br d, 1H), 3.56 (d, 1H), 3.45-3.34 (m, 3H), 3.13-2.97 (m, 7H), 2.36-2.29 (m, 1H), 2.01 (dd, 1H), 1.91-1.82 (m, 2H), 1.74-1.62 (m, 2H), 1.26 (d, 6H).

### Beispiel 285

### 1-({3-(3-Phenyl-1,2,4-oxadiazol-5-yl)-5-[4-(trifluormethyl)phenyl]piperidin-1-yl}carbonyl)-piperidin-4-carbonitril [racemisches cis-Isomer]

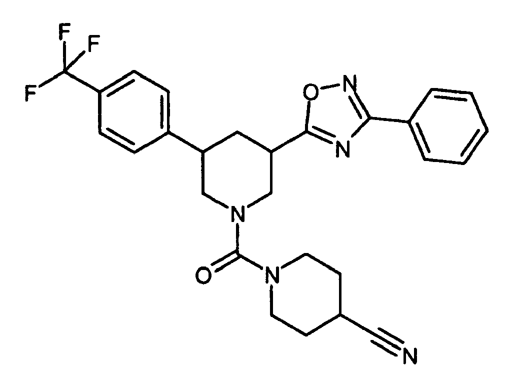

Nach der Allgemeinen Methode 1 wurden 100 mg (0.244 mmol) 1-[(4-Cyanpiperidin-1-yl)carbonyl]-5-[4-(trifluormethyl)phenyl]piperidin-3-carbonsäure (Beispiel 100A) und 38.5 mg (0.269 mmol) *N*'-Hydroxybenzolcarboximidamid umgesetzt. Ausbeute: 84.2 mg (66% d. Th.).

LC-MS (Methode 1B): Rₜ = 2.86 min; MS (ESIpos): m/z = 510 [M+H]⁺;

¹H-NMR (400 MHz, DMSO-d₆): δ = 8.02 (d, 2H), 7.72 (d, 2H), 7.63-7.54 (m, 5H), 4.07 (br d, 1H), 3.62 (d, 1H), 3.51 (tt, 1H), 3.45-3.35 (m, 2H), 3.20-3.00 (m, 6H), 2.42 (br d, 1H), 2.11 (dd, 1H), 1.92-1.83 (m, 2H), 1.76-1.64 (m, 2H).

### Beispiel 286

### 1-({3-[3-(3-Fluorphenyl)-1,2,4-oxadiazol-5-yl]-5-[4-(trifluormethyl)phenyl]piperidin-1-yl}-carbonyl)piperidin-4-carbonitril [racemisches cis-Isomer]

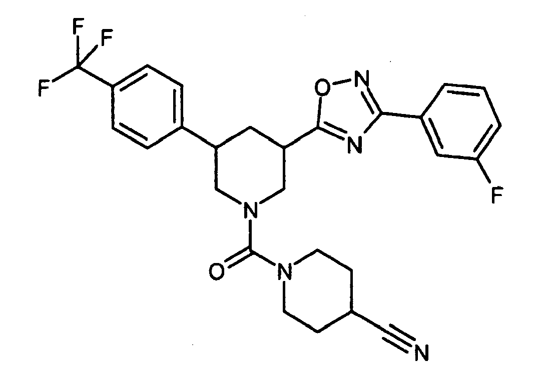

Nach der Allgemeinen Methode 1 wurden 100 mg (0.244 mmol) 1-[(4-Cyanpiperidin-1-yl)carbonyl]-5-[4-(trifluormethyl)phenyl]piperidin-3-carbonsäure (Beispiel 100A) und 41.4 mg (0.269 mmol) 3-Fluor-*N'-*hydroxybenzolcarboximidamid umgesetzt. Ausbeute: 76.4 mg (58% d. Th.).

LC-MS (Methode 1B): Rₜ = 2.92 min; MS (ESIpos): m/z = 528 [M+H]⁺;

¹H-NMR (400 MHz, DMSO-d₆): δ = 7.87 (d, 1H), 7.75 (d, 1H), 7.71 (d, 2H), 7.63 (dd, 1H), 7.59 (d, 2H), 7.47 (dd, 1H), 4.06 (br d,1H), 3.62 (d, 1H), 3.52 (tt, 1H), 3.44-3.36 (m, 2H), 3.18-3.01 (m, 6H), 2.42 (br d, 1H), 2.11 (dd, 1H), 1.92-1.83 (m, 2H), 1.75-1.65 (m, 2H).

### Beispiel 287

### 1-({3-[3-(Pyridin-3-yl)-1,2,4-oxadiazol-5-yl]-5-[4-(trifluormethyl)phenyl]piperidin-1-yl}-carbonyl)piperidin-4-carbonitril [racemisches cis-Isomer]

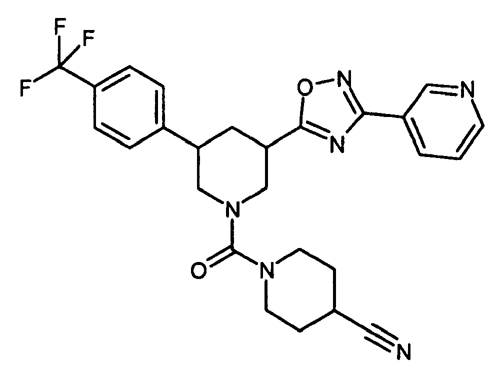

Nach der Allgemeinen Methode 1 wurden 100 mg (0.244 mmol) 1-[(4-Cyanpiperidin-1-yl)carbonyl]-5-[4-(trifluormethyl)phenyl]piperidin-3-carbonsäure (Beispiel 100A) und 36.8 mg (0.269 mmol) *N'*-Hydroxypyridin-3-carboximidamid umgesetzt. Ausbeute: 58.6 mg (46% d. Th.).

LC-MS (Methode 1 B): Rₜ = 2.47 min; MS (ESIpos): m/z = 511 [M+H]⁺;

¹H-NMR (400 MHz, DMSO-d₆): δ = 9.17 (d, 1H), 8.78 (dd, 1H), 8.37 (dd, 1H), 7.71 (d, 2H), 7.63-7.59 (m, 3H), 4.07 (br d, 1H), 3.62 (d, 1H), 3.55 (tt, 1H), 3.43-3.35 (m, 1H), 3.18-3.02 (m, 6H), 2.42 (br d, 1H), 2.11 (dd, 1H), 1.92-1.84 (m, 2H), 1.75-1.65 (m, 2H).

### Beispiel 288

### 1-({3-(3-Benzyl-1,2,4-oxadiazol-5-yl)-5-[4-(trifluormethyl)phenyl]piperidin-1-yl}carbonyl)-piperidin-4-carbonitril [racemisches cis-Isomer]

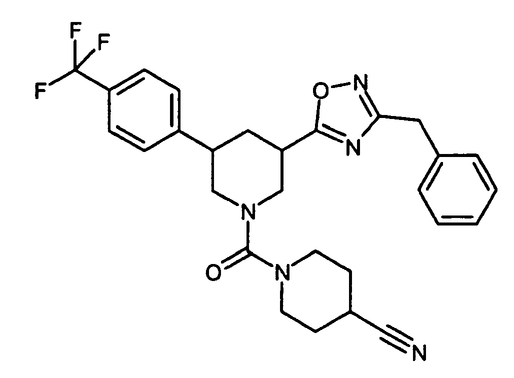

Nach der Allgemeinen Methode 1 wurden 100 mg (0.244 mmol) 1-[(4-Cyanpiperidin-1-yl)carbonyl]-5-[4-(trifluormethyl)phenyl]piperidin-3-carbonsäure (Beispiel 100A) und 40.3 mg (0.269 mmol) *N'*-Hydroxy-2-phenylethanimidamid umgesetzt. Ausbeute: 48.3 mg (38% d. Th.).

LC-MS (Methode 3B): Rₜ = 2.41 min; MS (ESIpos): m/z = 524 [M+H]⁺;

¹H-NMR (400 MHz, DMSO-d₆): δ = 7.69 (d, 2H), 7.55 (d, 2H), 7.34-7.23 (m, 5H), 4.08 (d, 1H), 3.94 (br d, 1H), 3.57 (d, 1H), 3-44-3.33 (m, 3H), 3.09-2.95 (m, 5H), 2.30 (br d, 1H), 1.99 (dd, 1H), 1.90-1.80 (m, 2H), 1.72-1.62 (m, 2H).

### Beispiel 289

### 1-({3-[3-(Pyridin-4-yl)-1,2,4-oxadiazol-5-yl]-5-[4-(trifluormethyl)phenyl]piperidin-1-yl}-carbonyl)piperidin-4-carbonitril [racemisches cis-Isomer]

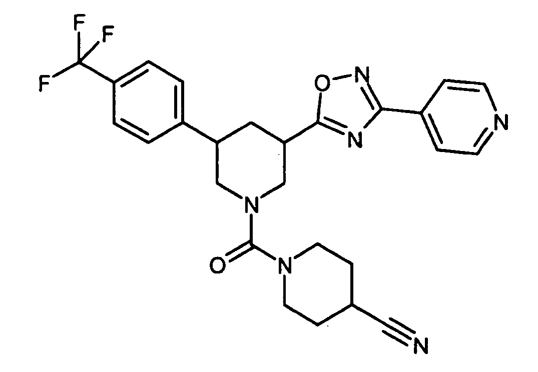

Nach der Allgemeinen Methode 1 wurden 100 mg (0.244 mmol) 1-[(4-Cyanpiperidin-1-yl)carbonyl]-5-[4-(trifluormethyl)phenyl]piperidin-3-carbonsäure (Beispiel 100A) und 36.8 mg (0.269 mmol) *N'*-Hydroxypyridin-4-carboximidiamid umgesetzt. Ausbeute: 70.3 mg (56% d. Th.).

LC-MS (Methode 3B): Rₜ = 2.10 min; MS (ESIpos): m/z = 511 [M+H]⁺;

¹H-NMR (400 MHz, DMSO-d₆): δ = 8.80 (d, 2H), 7.94 (d, 2H), 7.71 (d, 2H), 7.59 (d, 2H), 4.07 (br d, 1H), 3.62 (d, 1H), 3.55 (tt, 1H), 3.44-3.35 (m, 2H), 3.18-3.01 (m, 6H), 2.42 (br d, 1H), 2.11 (dd, 1H), 1.92-1.83 (m, 2H), 1.75-1.64 (m, 2H).

### Beispiel 290

### 1-({3-[3-(2-Fluorphenyl)-1,2,4-oxadiazol-5-yl]-5-[4-(trifluormethyl)phenyl]piperidin-1-yl}-carbonyl)piperidin-4-carbonitril [racemisches cis-Isomer]

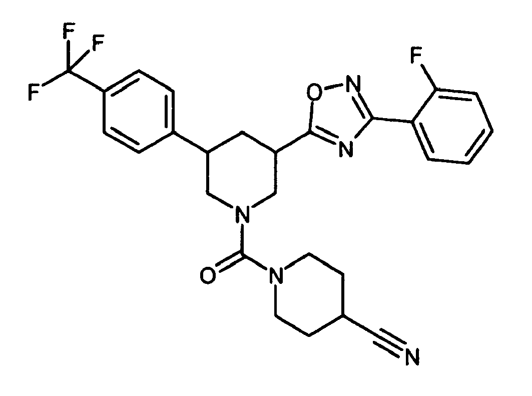

Nach der Allgemeinen Methode 1 wurden 100 mg (0.244 mmol) 1-[(4-Cyanpiperidin-1-yl)carbonyl]-5-[4-(trifluormethyl)phenyl]piperidin-3-carbonsäure (Beispiel 100A) und 41.4 mg (0.269 mmol) 2-Fluor-*N'*-hydroxybenzolcarboximidamid umgesetzt. Ausbeute: 72.8 mg (55% d. Th.).

LC-MS (Methode 2B): Rₜ = 1.46 min; MS (ESIpos): m/z = 528 [M+H]⁺;

¹H-NMR (400 MHz, DMSO-d₆): δ = 8.03 (td, 1H), 7.71 (d, 2H), 7.69-7.63 (m, 1H), 7.60 (d, 2H), 7.47-7.40 (m, 2H), 4.06 (d, 1H), 3.62 (d, 1H), 3.55 (tt, 1H), 3.44-3.36 (m, 2H), 3.22-3.01 (m, 6H), 2.42 (br d, 1H), 2.10 (dd, 1H), 1.93-1.84 (m, 2H), 1.75-1.64 (m, 2H).

### Beispiel 291

### 1-({3-[3-(4-Fluorphenyl)-1,2,4-oxadiazol-5-yl]-5-[4-(trifluormethyl)phenyl]piperidin-1-yl}-carbonyl)piperidin-4-carbonitril [racemisches cis-Isomer]

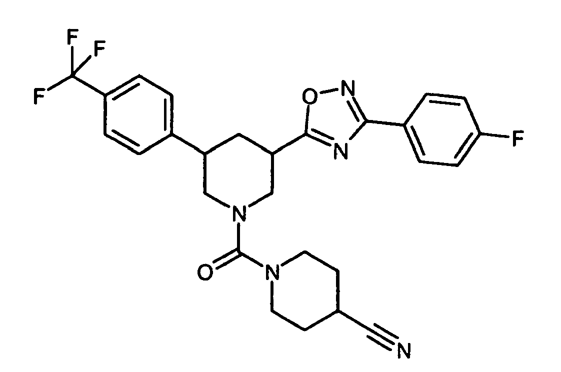

Nach der Allgemeinen Methode 1 wurden 100 mg (0.244 mmol) 1-[(4-Cyanpiperidin-1-yl)carbonyl]-5-[4-(trifluormethyl)phenyl]piperidin-3-carbonsäure (Beispiel 100A) und 41.4 mg (0.269 mmol) 4-Fluor-*N'*-hydroxybenzolcarboximidamid umgesetzt. Ausbeute: 78.2 mg (59% d. Th.).

LC-MS (Methode 2B): Rₜ = 1.50 min; MS (ESIpos): m/z = 528 [M+H]⁺;

¹H-NMR (400 MHz, DMSO-d₆): δ = 8.07 (dd, 2H), 7.71 (d, 2H), 7.59 (d, 2H), 7.42 (dd, 2H), 4.05 (d, 1H), 3.62 (d, 1H), 3.51 (tt, 1H), 3.43-3.35 (m, 2H), 3.17-3.00 (m, 6H), 2.41 (br d, 1H), 2.09 (dd, 1H), 1.92-1.83 (m, 2H), 1.75-1.64 (m, 2H).

### Beispiel 292

### 1-({3-{3-[(Methylsulfonyl)methyl]-1,2,4-oxadiazol-5-yl}-5-[4-(trifluormethyl)phenyl]piperidin-1-yl}carbonyl)piperidin-4-carbonitril [racemisches cis-Isomer]

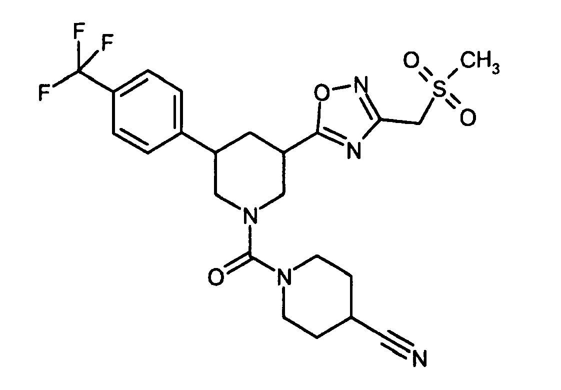

Nach der Allgemeinen Methode 1 wurden 100 mg (0.244 mmol) 1-[(4-Cyanpiperidin-1-yl)carbonyl]-5-[4-(trifluormethyl)phenyl]piperidin-3-carbonsäure (Beispiel 100A) und 40.9 mg (0.269 mmol) *N'*-Hydroxy-2-(methylsulfonyl)ethanimidamid umgesetzt. Ausbeute: 31.2 mg (24% d. Th.).

LC-MS (Methode 2B): Rₜ = 1.18 min; MS (ESIpos): m/z = 526 [M+H]⁺;

¹H-NMR (400 MHz, DMSO-d₆): δ = 7.70 (d, 2H), 7.58 (d, 2H), 4.85 (s, 2H), 4.00 (br d, 1H), 3.59 (d, 1H), 3.49 (tt, 1H), 3.42-3.35 (m, 2H), 3.17 (s, 3H), 3.11-2.98 (m, 6H), 2.37 (br d, 1H), 2.03 (dd, 1H), 1.90-1.82 (m, 2H), 1.72-1.63 (m, 2H).

### Beispiel 293

### 1-({3-[3-(2,4-Difluorphenyl)-1,2,4-oxadiazol-5-yl]-5-[4-(trifluormethyl)phenyl]piperidin-1-yl}-carbonyl)piperidin-4-carbonitril [racemisches cis-Isomer]

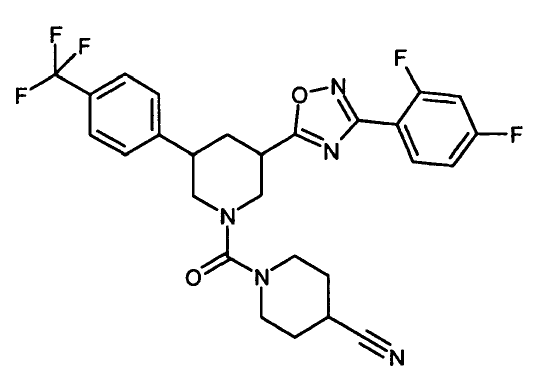

Nach der Allgemeinen Methode 1 wurden 100 mg (0.244 mmol) 1-[(4-Cyanpiperidin-1-yl)carbonyl]-5-[4-(trifluormethyl)phenyl]piperidin-3-carbonsäure (Beispiel 100A) und 46.2 mg (0.269 mmol) 2,4-Difluor-*N'*-hydroxybenzolcarboximidamid umgesetzt. Ausbeute: 63.9 mg (47% d. Th.).

LC-MS (Methode 3B): Rₜ = 2.50 min; MS (ESIpos): m/z = 546 [M+H]⁺;

¹H-NMR (400 MHz, DMSO-d₆): δ = 8.09 (d, 2H), 7.71 (d, 2H), 7.59 (d, 2H), 7.54 (dd, 1H), 7.32 (dd, 1H), 4.06 (br d, 1H), 3.62 (d, 1H), 3.53 (tt, 1H), 3.43-3.35 (m, 2H), 3.17-3.01 (m, 6H), 2.41 (br d, 1H), 2.09 (dd, 1H), 1.92-1.83 (m, 2H), 1.75-1.63 (m, 2H).

### Beispiel 294

### 1-({3-[3-(2,5-Difluorphenyl)-1,2,4-oxadiazol-5-yl]5-[4-(trifluormethyl)phenyl]piperidin-1-yl}-carbonyl)piperidin-4-carbonitril [racemisches cis-Isomer]

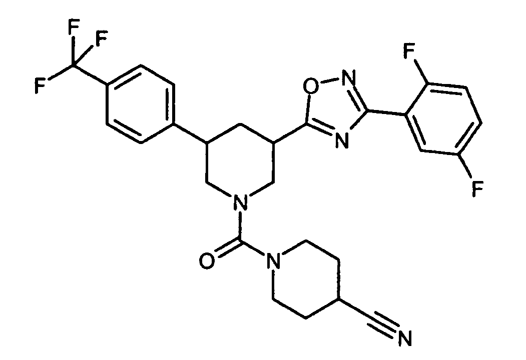

Nach der Allgemeinen Methode 1 wurden 100 mg (0.244 mmol) 1-[(4-Cyanpiperidin-1-yl)carbonyl]-5-[4-(trifluormethyl)phenyl]piperidin-3-carbonsäure (Beispiel 100A) und 46.2 mg (0.269 mmol) 2,5-Difluor-*N'*-hydroxybenzolcarboximidamid (Beispiel 74A) umgesetzt. Ausbeute: 72.5 mg (54% d. Th.).

LC-MS (Methode 3B): Rₜ = 2.49 min; MS (ESIpos): m/z = 546 [M+H]⁺;

¹H-NMR (400 MHz, DMSO-d₆): δ = 7.78 (br s, 1H), 7.71 (d, 2H), 7.59 (d, 2H), 7.55 (dd, 2H), 4.06 (br d, 1H), 3.62 (d, 1H), 3.59-3.50 (m, 1H), 3.39 (br d, 1H), 3.17-3.02 (m, 6H), 2.42 (br d, 1H), 2.11 (dd, 1H), 1.93-1.83 (m, 2H), 1.75-1.65 (m, 2H).

### Beispiel 295

### 1-({3-[3-(2-Chlorphenyl)-1,2,4-oxadiazol-5-yl]-5-[4-(trifluormethyl)phenyl]piperidin-1-yl} - carbonyl)piperidin-4-carbonitril [racemisches cis-Isomer]

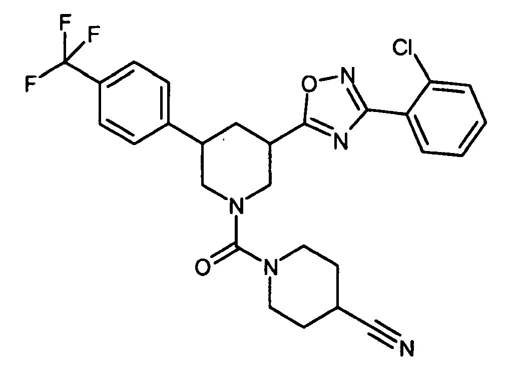

Nach der Allgemeinen Methode 1 wurden 100 mg (0.244 mmol) 1-[(4-Cyanpiperidin-1-yl)carbonyl]-5-[4-(trifluormethyl)phenyl]piperidin-3-carbonsäure (Beispiel 100A) und 45.8 mg (0.269 mmol) 2-Chlor-*N'*-hydroxybenzolcarboximidamid umgesetzt. Ausbeute: 72.4 mg (55% d. Th.).

LC-MS (Methode 3B): Rₜ = 2.53 min; MS (ESIpos): m/z = 544 [M+H]⁺;

¹H-NMR (400 MHz, DMSO-d₆): δ = 7.92 (dd, 1H), 7.71 (d, 2H), 7.69-7.52 (m, 5H), 4.06 (br d, 1H), 3.62 (d, 1H), 3.54 (tt, 1H), 3.43-3.37 (m, 2H), 3.18-3.01 (m, 6H), 2.42 (br d, 1H), 2.10 (dd, 1H), 1.92-1.84 (m, 2H), 1.75-1.64 (m, 2H).

### Beispiel 296

### 1-({3-[3-(3-Chlorphenyl)-1,2,4-oxadiazol-5-yl]-5-[4-(trifluormethyl)phenyl]piperidin-1-yl}-carbonyl)piperidin-4-carbonitril [racemisches cis-Isomer]

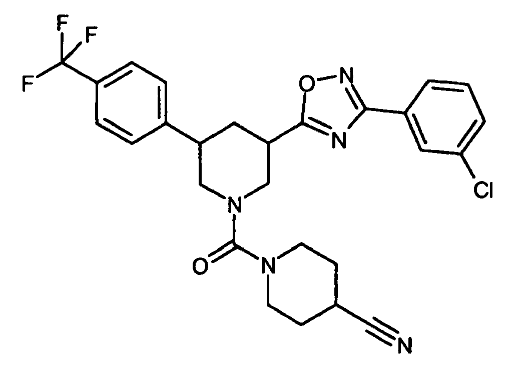

Nach der Allgemeinen Methode 1 wurden 100 mg (0.244 mmol) 1-[(4-Cyanpiperidin-1-yl)carbonyl]-5-[4-(trifluormethyl)phenyl]piperidin-3-carbonsäure (Beispiel 100A) und 45.8 mg (0.269 mmol) 3-Chlor-*N'*-hydroxybenzolcarboximidamid umgesetzt. Ausbeute: 73.3 mg (55% d. Th.).

LC-MS (Methode 3B): Rₜ = 2.68 min; MS (ESIpos): m/z = 544 [M+H]⁺;

¹H-NMR (400 MHz, DMSO-d₆): δ = 7.99-7.97 (m, 2H), 7.71-7.68 (m, 3H), 7.64-7.58 (m, 3H), 4.06 (br d, 1H), 3.62 (d, 1H), 3.53 (tt, 1H), 3.43-3.36 (m, 2H), 3.18-2.99 (m, 6H), 2.42 (br d, 1H), 2.10 (dd, 1H), 1.92-1.84 (m, 2H), 1.74-1.64 (m, 2H).

### Beispiel 297

### {3-(3-Cyclopropyl-1,2,4-oxadiazol-5-yl)-5-[4-(trifluormethyl)phenyl]piperidin-1-yl}(3-hydroxyazetidin-1-yl)methanon [racemisches cis-Isomer]

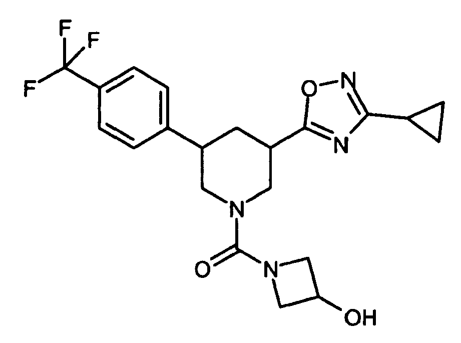

Nach der Allgemeinen Methode 1 wurden 100 mg (0.269 mmol) 1-[(3-Hydroxyazetidin-1-yl)carbonyl]-5-[4-(trifluormethyl)phenyl]piperidin-3-carbonsäure (Beispiel 101A) und 29.6 mg (0.295 mmol) *N'*-Hydroxycyclopropancarboximidamid (Beispiel 78A) umgesetzt. Ausbeute: 54 mg (44% d. Th.).

LC-MS (Methode 2B): Rₜ = 1.19 min; MS (ESIpos): m/z = 437 [M+H]⁺;

¹H-NMR (400 MHz, DMSO-d₆): δ = 7.70 (d, 2H), 7.56 (d, 2H), 5.58 (d, 1H), 4.43-4.34 (m, 1H), 4.12 (br d, 1H), 4.09 (q, 2H), 3.75 (br d, 1H), 3.66-3.73 (m, 2H), 3.26 (tt, 1H), 3.04-2.92 (m, 3H), 2.28 (br d, 1H), 2.11 (sept, 1H), 2.00 (dd, 1H), 1.09-1.02 (m, 2H), 0.88 (m, 2H).

### Beispiel 298

### (3-Hydroxyazetidin-1-yl){3-(3-methyl-1,2,4-oxadiazol-5-yl)-5-[4-(trifluormethyl)phenyl]piperidin-1-yl}methanon [racemisches cis-Isomer]

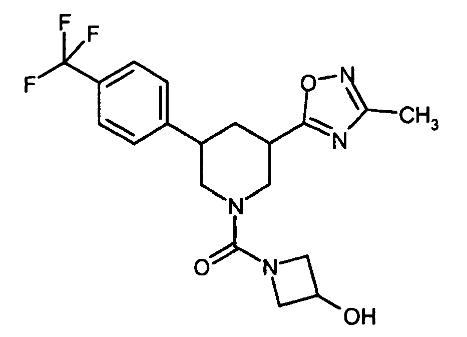

Nach der Allgemeinen Methode 1 wurden 100 mg (0.269 mmol) 1-[(3-Hydroxyazetidin-1-yl)carbonyl]-5-[4-(trifluormethyl)phenyl]piperidin-3-carbonsäure (Beispiel 101A) und 21.9 mg (0.269 mmol) *N'*-Hydroxyethanimidamid umgesetzt. Ausbeute: 41.6 mg (32% d. Th.).

LC-MS (Methode 2B): Rₜ = 1.09 min; MS (ESIpos): m/z = 411 [M+H]⁺;

¹H-NMR (400 MHz, DMSO-d₆): δ = 7.70 (d, 2H), 7.56 (d, 2H), 5.58 (d, 1H), 4.43-4.34 (m, 1H), 4.15 (br d, 1H), 4.10 (q, 2H), 3.77 (br d, 1H), 3.73-3.66 (m, 2H), 3.30 (verdeckt, 1H), 3.07-2.92 (m, 3H), 2.2.35-2.28 (m, 4H), 2.04 (dd, 1H).

### Beispiel 299

### {3-(3-tert.-Butyl-1,2,4-oxadiazol-5-yl)-5-[4-(trifluormethyl)phenyl]piperidin-1-yl}(3-hydroxyazetidin-1-yl)methanon [racemisches cis-Isomer]

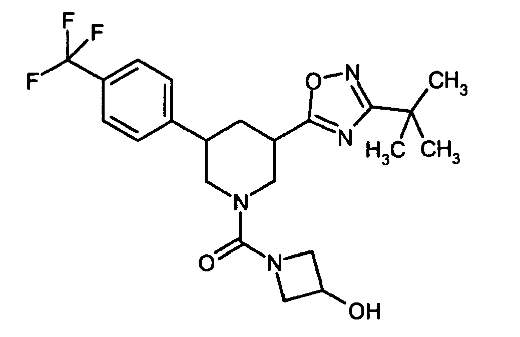

Nach der Allgemeinen Methode 1 wurden 100 mg (0.269 mmol) 1-[(3-Hydroxyazetidin-1-yl)carbonyl]-5-[4-(trifluormethyl)phenyl]piperidin-3-carbonsäure (Beispiel 101A) und 34.3 mg (0.295 mmol) *N'*-Hydroxy-2,2-dimethylpropanimidamid umgesetzt. Ausbeute: 45 mg (37% d. Th.).

LC-MS (Methode 3B): Rₜ = 2.13 min; MS (ESIpos): m/z = 453 [M+H]⁺;

¹H-NMR (400 MHz, DMSO-d₆): δ = 7.70 (d, 2H), 7.56 (d, 2H), 5.58 (d, 1H), 4.43-4.33 (m, 1H), 4.16 (br d, 1H), 4.09 (q, 2H), 3.74 (br d, 1H), 3.71-3.64 (m, 2H), 3.06-2.93 (m, 3H), 2.31 (br d, 1H), 2.03 (dd, 1H), 1.30 (s, 9H).

### Beispiel 300

### (3-Hydroxyazetidin-1-yl) {3-[3-(propan-2-yl)-1,2,4-oxadiazol-5-yl]-5-[4-(trifluormethyl)phenyl]-piperidin-1-yl}methanon [racemisches cis-Isomer]

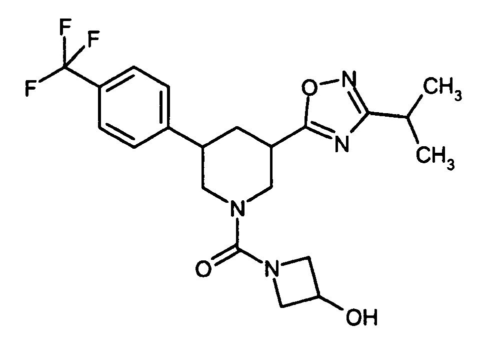

Nach der Allgemeinen Methode 1 wurden 100 mg (0.269 mmol) 1-[(3-Hydroxyazetidin-1-yl)carbonyl]-5-[4-(trifluormethyl)phenyl]piperidin-3-carbonsäure (Beispiel 101A) und 30.7 mg (0.295 mmol) *N'*-Hydroxy-2-methylpropanimidamid umgesetzt. Ausbeute: 47 mg (39% d. Th.).

LC-MS (Methode 3B): Rₜ = 1.99 min; MS (ESIpos): m/z = 439 [M+H]⁺;

¹H-NMR (400 MHz, DMSO-d₆): δ = 7.70 (d, 2H), 7.56 (d, 2H), 5.58 (d, 1H), 4.43-4.36 (m, 1H), 4.16 (br d, 1H), 4.09 (q, 2H), 3.75 (br d, 1H), 3.73-3.67 (m, 2H), 3.08-2.96 (m, 4H), 2.31 (br d, 1H), 2.03 (dd, 1H), 1.25 (d, 6H).

### Beispiel 301

### (3-Hydroxyazetidin-1-yl){3-(3-phenyl-1,2,4-oxadiazol-5-yl)-5-[4-(trifluormethyl)phenyl]piperidin-1-yl}methanon [racemisches cis-Isomer]

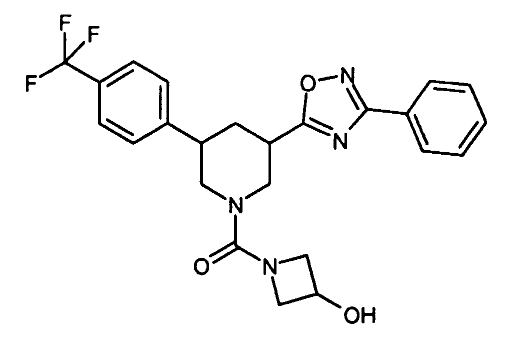

Nach der Allgemeinen Methode 1 wurden 100 mg (0.269 mmol) 1-[(3-Hydroxyazetidin-1-yl)carbonyl]-5-[4-(trifluormethyl)phenyl]piperidin-3-carbonsäure (Beispiel 101A) und 42.3 mg (0.295 mmol) *N'*-Hydroxybenzolcarboximidamid (95%ig) umgesetzt. Ausbeute: 48 mg (36% d. Th.).

LC-MS (Methode 3B): Rₜ = 2.17 min; MS (ESIpos): m/z = 473 [M+H]⁺;

¹H-NMR (400 MHz, DMSO-d₆): δ = 8.01 (dd, 2H), 7.71 (d, 2H), 7.62-7.53 (m, 5H), 5.06 (d, 1H), 4.44-4.36 (m, 1H), 4.25 (br d, 1H), 4.11 (q, 2H), 3.80 (br d, 1H), 3.75-3.70 (m, 2H), 3.43 (tt, 1H), 3.13 (t, 1H), 3.07-2.98 (m, 2H), 2.40 (br d, 1H), 2.12 (dd, 1H).

### Beispiel 302

### {3-[3-(3-Fluorphenyl)-1,2,4-oxadiazol-5-yl]-5-[4-(trifluormethyl)phenyl]piperidin-1-yl}(3-hydroxyazetidin-1-yl)methanon [racemisches cis-Isomer]

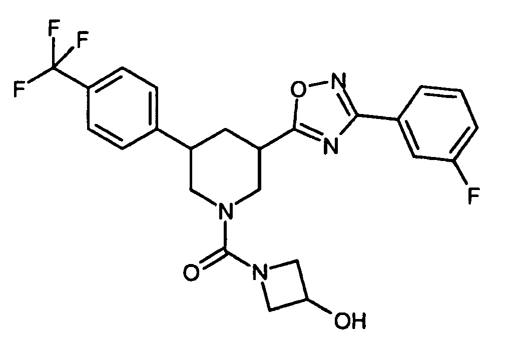

Nach der Allgemeinen Methode 1 wurden 100 mg (0.269 mmol) 1-[(3-Hydroxyazetidin-1-yl)carbonyl]-5-[4-(trifluormethyl)phenyl]piperidin-3-carbonsäure (Beispiel 101A) und 45.5 mg (0.295 mmol) 3-Fluor-*N'*-hydroxybenzolcarboximidamid umgesetzt. Ausbeute: 46 mg (34% d. Th.).

LC-MS (Methode 3B): Rₜ = 2.23 min; MS (ESIpos): m/z = 491 [M+H]⁺;

¹H-NMR (400 MHz, DMSO-d₆): δ = 7.87 (d, 1H), 7.75 (d, 1H), 7.71 (d, 2H), 7.66-7.63 (m, 1H), 7.61 (d, 2H), 7.46 (dd, 1H), 5.59 (d, 1H), 4.44-4.36 (m, 1H), 4.25 (br d, 1H), 4.11 (q, 2H), 3.79 (br d, 1H), 3.75-3.66 (m, 2H), 3.44 (tt, 1H), 3.13 (t, 1H), 3.05-3.01 (m, 2H), 2.40 (br d, 1H), 2.12 (dd, 1H).

### Beispiel 303

### (3-Hydroxyazetidin-1-yl){3-[3-(pyridin-3-yl)-1,2,4-oxadiazol-5-yl]-5-[4-(trifluormethyl)phenyl]-piperidin-1-yl}methanon [racemisches cis-Isomer]

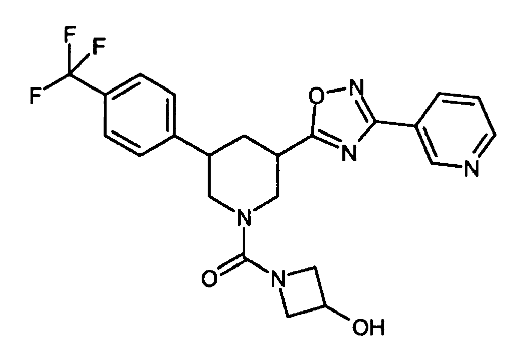

Nach der Allgemeinen Methode 1 wurden 100 mg (0.269 mmol) 1-[(3-Hydroxyazetidin-1-yl)carbonyl]-5-[4-(trifluormethyl)phenyl]piperidin-3-carbonsäure (Beispiel 101A) und 40.5 mg (0.295 mmol) *N'*-Hydroxypyridin-3-carboximidamid umgesetzt. Ausbeute: 28.8 mg (21 % d. Th.).

LC-MS (Methode 3B): Rₜ = 1.77 min; MS (ESIpos): m/z = 474 [M+H]⁺;

¹H-NMR (400 MHz, DMSO-d₆): δ = 9.18 (d, 1H), 8.77 (dd, 1H), 8.37 (tt, 1H), 7.72 (d, 2H), 7.62 (dd, 1H), 7.59 (d, 2H), 5.60 (d, 1H), 4.44-4.36 (m, 1H), 4.26 (br d, 1H), 4.12 (q, 2H), 3.80 (br d, 1H), 3.76-3.68 (m, 2H), 3.48 (tt, 1H), 3.14 (t, 1H), 3.03 (dd, 2H), 2.42 (br d, 1H), 2.14 (dd, 1H).

### Beispiel 304

### {3-(3-Benzyl-1,2,4-oxadiazol-5-yl)-5-[4-(trifluormethyl)phenyl]piperidin-1-yl} (3-hydroxyazetidin-1-yl)methanon [racemisches cis-Isomer]

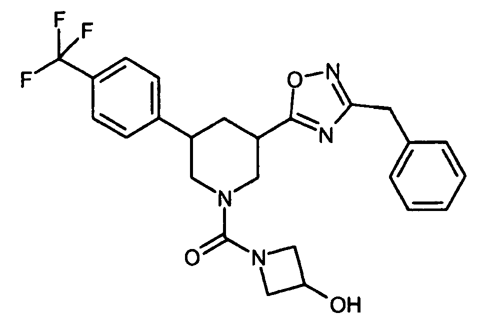

Nach der Allgemeinen Methode 1 wurden 100 mg (0.269 mmol) 1-[(3-Hydroxyazetidin-1-yl)carbonyl]-5-[4-(trifluormethyl)phenyl]piperidin-3-carbonsäure (Beispiel 101A) und 44.4 mg (0.295 mmol) *N'*-Hydroxy-2-phenylethanimidamid umgesetzt. Ausbeute: 40.0 mg (29% d. Th.).

LC-MS (Methode 2B): Rₜ = 1.31 min; MS (ESIpos): m/z = 487 [M+H]⁺;

¹H-NMR (400 MHz, DMSO-d₆): δ = 7.69 (d, 2H), 7.54 (d, 1H), 7.34-7.23 (m, 5H), 5.57 (d, 1H), 4.41-4.34 (m, 1H), 4.14 (br d, 1H), 4.11-4.05 (m, 4H), 3.74 (br d, 1H), 3.72-3.66 (m, 2H), 3.04-2.93 (m, 3H), 2.30 (br d, 1H), 2.01 (dd, 1H).

### Beispiel 305

### {3-[3-(4-Fluorphenyl)-1,2,4-oxadiazol-5-yl]-5-[4-(trifluormethyl)phenyl]piperidin-1-yl} (3-hydroxyazetidin-1-yl)methanon [racemisches cis-Isomer]

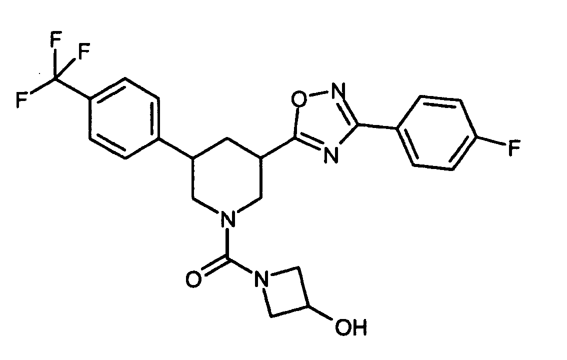

Nach der Allgemeinen Methode 1 wurden 90.0 mg (0.242 mmol) 1-[(3-Hydroxyazetidin-1-yl)carbonyl]-5-[4-(trifluormethyl)phenyl]piperidin-3-carbonsäure (Beispiel 101A) und 41.0 mg (0.266 mmol) 4-Fluor-*N'*-hydroxybenzolcarboximidamid umgesetzt. Ausbeute: 52.1 mg (42% d. Th.).

LC-MS (Methode 3B): Rₜ = 2.22 min; MS (ESIpos): m/z = 491 [M+H]⁺;

¹H-NMR (400 MHz, DMSO-d₆): δ = 8.11-8.04 (m, 2H), 7.72 (d, 2H), 7.59 (d, 2H), 7.42 (tt, 2H), 5.59 (d, 1H), 4.44-4.35 (m, 1H), 4.25 (br d, 1H), 4.12 (q, 2H), 3.82-3.77 (br d, 1H), 3.76-3.68 (m, 2H), 3.43 (tt, 1H), 3.13 (t, 1H), 3.07-2.98 (m, 2H), 2.41 (br d, 1H), 2.13 (dd, 1H).

### Beispiel 306

### {3-[3-(2,4-Difluorphenyl)-1,2,4-oxadiazol-5-yl]-5-[4-(trifluormethyl)phenyl]piperidin-1-yl}(3-hydroxyazetidin-1-yl)methanon [racemisches cis-Isomer]

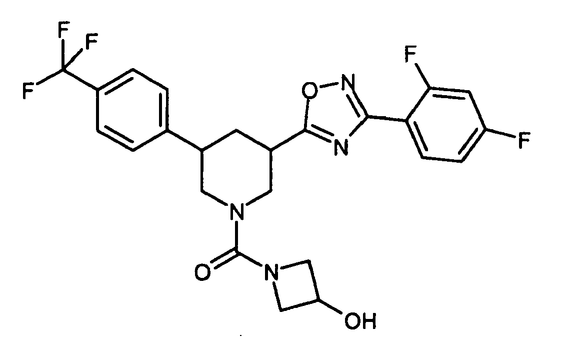

Nach der Allgemeinen Methode 1 wurden 90.0 mg (0.242 mmol) 1-[(3-Hydroxyazetidin-1-yl)carbonyl]-5-[4-(trifluormethyl)phenyl]piperidin-3-carbonsäure (Beispiel 101A) und 47.3 (0.266 mmol) 2,4-Difluor-*N'*-hydroxybenzolcarboximidamid umgesetzt. Ausbeute: 35.2 mg (27% d. Th.).

LC-MS (Methode 3B): Rₜ = 2.19 min; MS (ESIpos): m/z = 509 [M+H]⁺;

¹H-NMR (400 MHz, DMSO-d₆): δ = 8.10 (q, 1H), 7.71 (d, 2H), 7.58 (d, 2H), 7.54 (t, 1H), 7.32 (td, 1H), 4.43-4.37 (m, 1H), 4.24 (br d, 1H), 4.11 (q, 2H), 3.79 (br d, 1H), 3.75-3.65 (m, 2H), 3.44 (tt, 1H), 3.12 (t, 1H), 3.06-2.97 (m, 2H), 2.40 (brd, 1H), 2.12 (dd, 1H).

### Beispiel 307

### {3-[3-(2,5-Difluorphenyl)-1,2,4-oxadiazol-5-yl]-5-[4-(trifluormethyl)phenyl]piperidin-1-yl} (3-hydroxyazetidin-1-yl)methanon [racemisches cis-Isomer]

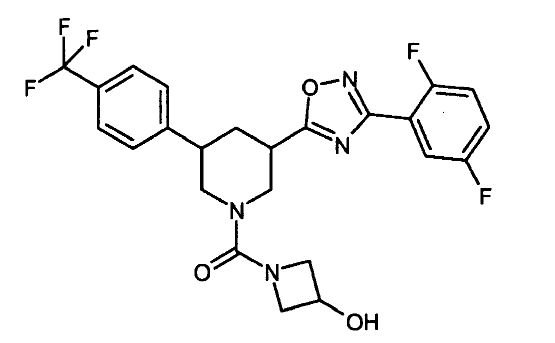

Nach der Allgemeinen Methode 1 wurden 90.0 mg (0.242 mmol) 1-[(3-Hydroxyazetidin-1-yl)carbonyl]-5-[4-(trifluormethyl)phenyl]piperidin-3-carbonsäure (Beispiel 101A) und 45.7 (0.266 mmol) 2,5-Difluor-*N'-*hydroxybenzolcarboximidamid (Beispiel 74A) umgesetzt. Ausbeute: 42.9 mg (34% d. Th.).

LC-MS (Methode 3B): Rₜ = 2.18 min; MS (ESIpos): m/z = 509 [M+H]⁺;

¹H-NMR (400 MHz, DMSO-d₆): δ = 7.81-7.77 (m, 1H), 7.71 (d, 2H), 7.58 (d, 1H), 7.54 (td, 2H), 5.59 (d, 1H), 4.44-4.36 (m, 1H), 4.25 (br d, 1H), 4.11 (q, 2H), 3.82 (br d, 1H), 3.75-3.69 (m, 2H), 3.45 (tt, 1H), 3.13 (t, 1H), 3.07-2.98 (m, 2H), 2.40 (brd, 1H), 2.12 (dd, 1H).

### Beispiel 308

### {3-[3-(2-Chlorphenyl)-1,2,4-oxadiazol-5-yl]-5-[4-(trifluormethyl)phenyl]piperidin-1-yl} (3-hydroxyazetidin-1-yl)methanon [racemisches cis-Isomer]

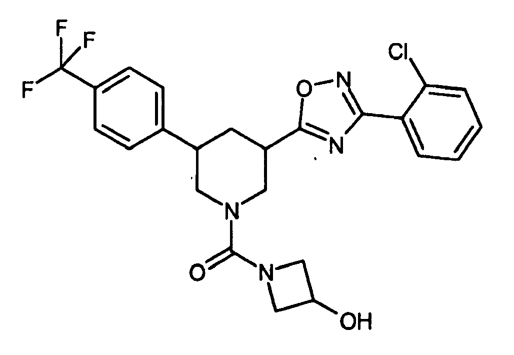

Nach der Allgemeinen Methode 1 wurden 90.0 mg (0.242 mmol) 1-[(3-Hydroxyazetidin-1-yl)carbonyl]-5-[4-(trifluormethyl)phenyl]piperidin-3-carbonsäure (Beispiel 101A) und 34.8 mg (0.266 mmol) 2-Chlor-*N'*-hydroxybenzolcarboximidamid umgesetzt. Ausbeute: 34.8 mg (27% d. Th.).

LC-MS (Methode 3B): Rₜ = 2.21 min; MS (ESIpos): m/z = 507 [M+H]⁺;

¹H-NMR (400 MHz, DMSO-d₆): δ = 7.92 (dd, 1H), 7.72-7.67 (m, 3H), 7.63-7.52 (m, 4H), 5.59 (d, 1H), 4.43-4.36 (m, 1H), 4.25 (br d, 1H), 4.11 (q, 2H), 3.79 (br d, 1H), 3.75-3.69 (m, 2H), 3.45 (tt, 1H), 3.12 (t, 1H), 3.06-3.01 (m, 2H), 2.41 (br d, 1H), 2.12 (dd, 1H).

### Beispiel 309

### {3-[3-(3-Chlorphenyl)-1,2,4-oxadiazol-5-yl]-5-[4-(trifluormethyl)phenyl]piperidin-1-yl}(3-hydroxyazetidin-1-yl)methanon [racemisches cis-Isomer]

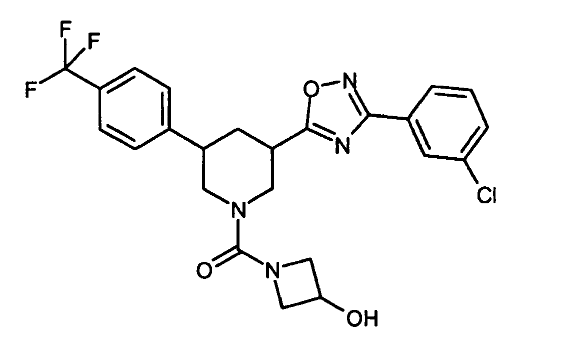

Nach der Allgemeinen Methode 1 wurden 90.0 mg (0.242 mmol) 1-[(3-Hydroxyazetidin-1-yl)carbonyl]-5-[4-(trifluormethyl)phenyl]piperidin-3-carbonsäure (Beispiel 101A) und 45.4 mg (0.266 mmol) 3-Chlor-*N'*-hydroxybenzolcarboximidamid umgesetzt. Ausbeute: 34.7 mg (28% d. Th.).

LC-MS (Methode 3B): Rₜ = 2.38 min; MS (ESIpos): m/z = 507 [M+H]⁺;

¹H-NMR (400 MHz, DMSO-d₆): δ = 7.99-7.97 (m, 2H), 7.73-7.68 (m, 3H), 7.64-7.58 (m, 3H), 5.59 (d, 1H), 4.43-4.36 (m, 1H), 4.25 (br d, 1H), 4.11 (q, 2H), 3.79 (br d, 1H), 3.75-3.69 (m, 2H), 3.44 (tt, 1H), 3.13 (t, 1H), 3.03-3.01 (m, 2H), 2.41 (br d, 1H), 2.13 (q, 1H).

### Beispiel 310

### {3-[3-(3,5-Difluorpyridin-2-yl)-1,2,4-oxadiazol-5-yl]-5-[4-(trifluormethyl)phenyl]piperidin-1-yl}-(3-hydroxyazetidin-1-yl)methanon [racemisches cis-Isomer]

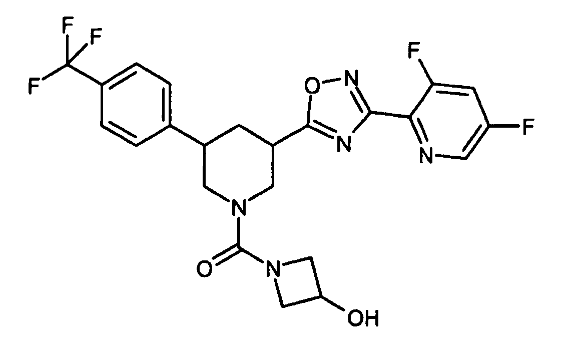

Nach der Allgemeinen Methode 1 wurden 90.0 mg (0.242 mmol) 1-[(3-Hydroxyazetidin-1-yl)carbonyl]-5-[4-(trifluormethyl)phenyl]piperidin-3-carbonsäure (Beispiel 101A) und 61.0 mg (0.266 mmol) 3,5-Difluor-*N'*-hydroxypyridin-2-carboximidamid umgesetzt. Ausbeute: 24 mg (19% d. Th.).

LC-MS (Methode 3B): Rₜ = 1.90 min; MS (ESIpos): m/z = 510 [M+H]⁺;

¹H-NMR (400 MHz, DMSO-d₆): δ = 8.75 (d, 1H), 8.25 (dd, 1H), 7.71 (d, 2H), 7.58 (d, 2H), 5.59 (d, 1H), 4.42-4.36 (m, 1H), 4.26 (br d, 1H), 4.11 (q, 2H), 3.79 (br d, 1H), 3.75-3.69 (m, 2H), 3.47 (tt, 1H), 3.13 (t, 1H), 3.00 (br d, 2H), 2.41 (br d, 1H), 2.14 (q, 1H).

### Beispiel 311

### (3-Methoxyazetidin-1-yl) {3-(3-methyl-1,2,4-oxadiazol-5-yl)-5-[4-(trifluormethyl)phenyl]piperidin-1-yl}methanon [enantiomerenreines cis-Isomer]

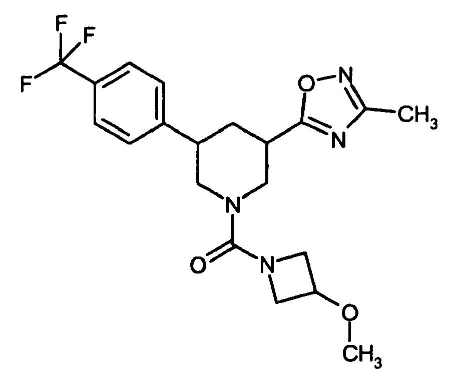

Nach der Allgemeinen Methode 1 wurden 250 mg (0.647 mmol) 1-[(3-Methoxyazetidin-1-yl)carbonyl]-5-[4-(trifluormethyl)phenyl]piperidin-3-carbonsäure (Beispiel 103A) und 52.7 mg (0.712 mmol) *N'*-Hydroxyethanimidamid umgesetzt. Enantiomerentrennung des Racemates nach Methode 16D ergab 73.0 mg der Titelverbindung aus Beispiel 311 und 64.0 mg der Titelverbindung aus Beispiel 312 (50% d. Th.).

HPLC (Methode 16E): Rₜ = 7.27 min, >99.0% ee; LC-MS (Methode 3B): Rₜ = 1.96 min; MS (ESIpos): m/z = 425 [M+H]⁺;

¹H-NMR (400 MHz, DMSO-d₆): δ = 7.70 (d, 2H), 7.56 (d, 2H), 4.20-4.05 (m, 4H), 3.82-3.72 (m, 3H), 3.36-3.27 (verdeckt, 1H), 3.18 (s, 3H), 3.08-2.93 (m, 3H), 2.33 (s, 3H), 2.31 (br d, 1H), 2.03 (dd, 1H).

### Beispiel 312

### (3-Methoxyazetidin-1-yl) {3-(3-methyl-1,2,4-oxadiazol-5-yl)-5-[4-(trifluormethyl)phenyl]piperidin-1-yl}methanon [enantiomerenreines cis-Isomer]

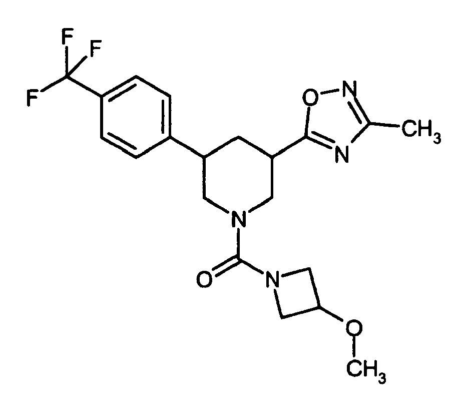

Nach der Allgemeinen Methode 1 wurden 250 mg (0.647 mmol) 1-[(3-Methoxyazetidin-1-yl)carbonyl]-5-[4-(trifluormethyl)phenyl]piperidin-3-carbonsäure (Beispiel 103A) und 52.7 mg (0.712 mmol) *N'*-Hydroxyethanimidamid umgesetzt. Enantiomerentrennung des Racemates nach Methode 16D ergab 73.0 mg der Titelverbindung aus Beispiel 311 und 64.0 mg der Titelverbindung aus Beispiel 312 (50% d. Th.).

HPLC (Methode 16E): Rₜ = 15.66 min, >99.0% ee; LC-MS (Methode 3B): Rₜ = 1.96 min; MS (ESIpos): m/z = 425 [M+H]⁺.

### Beispiel 313

### (3-Methoxyazetidin-1-yl) {3-[3-(2-methoxyethyl)-1,2,4-oxadiazol-5-yl]-5-[4-(trifluormethyl)-phenyl]piperidin-1-yl}methanon [enantiomerenreines cis-Isomer]

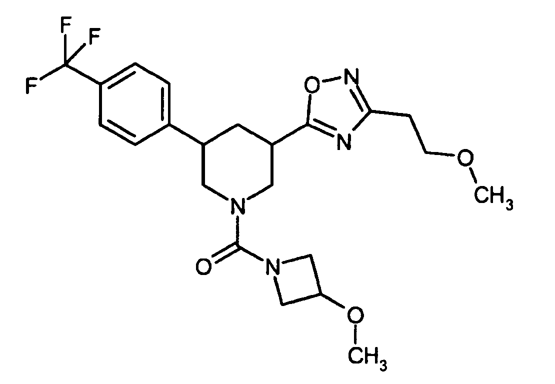

Nach der Allgemeinen Methode 1 wurden 250 mg (0.647 mmol) 1-[(3-Methoxyazetidin-1-yl)carbonyl]-5-[4-(trifluormethyl)phenyl]piperidin-3-carbonsäure (Beispiel 103A) und 112 mg (0.712 mmol, 75% Reinheit) *N'*-Hydroxy-3-methoxypropanimidamid (Beispiel 64A) umgesetzt. Enantiomerentrennung des Racemates nach Methode 16D ergab 67.0 mg der Titelverbindung aus Beispiel 313 und 67.0 mg der Titelverbindung aus Beispiel 314 (44% d. Th.).

HPLC (Methode 16E): Rₜ = 7.88 min, >99.0% ee; LC-MS (Methode 3B): Rₜ = 1.98 min; MS (ESIpos): m/z = 469 [M+H]⁺;

¹H-NMR (400 MHz, DMSO-d₆): δ = 7.71 (d, 2H), 7.57 (d, 2H), 4.21-4.04 (m, 4H), 3.81-3.72 (m, 3H), 3.68 (t, 2H), 3.39-3.30 (verdeckt, 1H), 3.24 (s, 3H), 3.19 (s, 3H), 3.09-2.96 (m, 3H), 2.94 (t, 2H), 2.32 (br d, 1H), 2.04 (dd, 1H).

### Beispiel 314

### (3-Methoxyazetidin-1-yl) {3-[3-(2-methoxyethyl)-1,2,4-oxadiazol-5-yl]-5-[4-(trifluormethyl) phenyl]piperidin-1-yl}methanon [enantiomerenreines cis-Isomer]

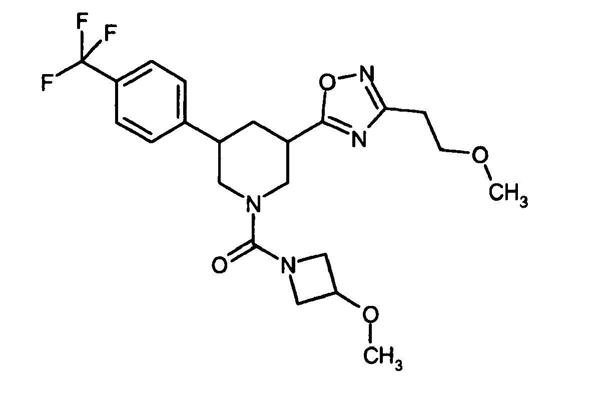

Nach der Allgemeinen Methode 1 wurden 250 mg (0.647 mmol) 1-[(3-Methoxyazetidin-1-yl)carbonyl]-5-[4-(trifluormethyl)phenyl]piperidin-3-carbonsäure (Beispiel 103A) und 112 mg (0.712 mmol, 75% Reinheit) *N'*-Hydroxy-3-methoxypropanimidamid (Beispiel 64A) umgesetzt. Enantiomerentrennung des Racemates nach Methode 16D ergab 67.0 mg der Titelverbindung aus Beispiel 313 und 67.0 mg der Titelverbindung aus Beispiel 314 (44% d. Th.).

HPLC (Methode 16E): Rₜ = 9.75 min, >99.0% ee; LC-MS (Methode 3B): Rₜ = 1.98 min; MS (ESIpos): m/z = 469 [M+H]⁺.

### Beispiel 315

### {3-(3-Methyl-1,2,4-oxadiazol-5-yl)-5-[4-(trifluormethyl)phenyl]piperidin-1-yl} (1,3-thiazolidin-3-yl)methanon [enantiomerenreines cis-Isomer]

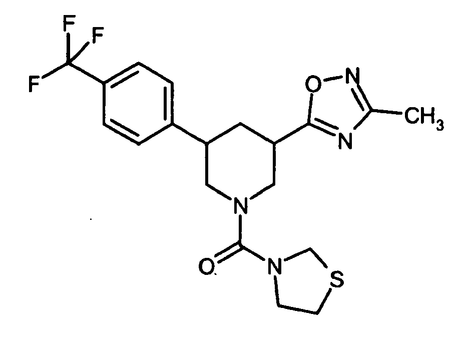

Nach der Allgemeinen Methode 1 wurden 250 mg (0.644 mmol) 1-(1,3-Thiazolidin-3-ylcarbonyl)-5-[4-(trifluormethyl)phenyl]piperidin-3-carbonsäure (Beispiel 102A) und 52.5 mg (0.708 mmol) *N'*-Hydroxyethanimidamid umgesetzt. Enantiomerentrennung des Racemates nach Methode 16D ergab 38.0 mg der Titelverbindung aus Beispiel 315 und 41.0 mg der Titelverbindung aus Beispiel 316 (29% d. Th.).

HPLC (Methode 16E): Rₜ = 8.92 min, >99.0% ee; LC-MS (Methode 3B): Rₜ = 2.17 min; MS (ESIpos): m/z = 427 [M+H]⁺;

¹H-NMR (400 MHz, DMSO-d₆): δ = 7.71 (d, 2H), 7.57 (d, 2H), 4.48 (d, 1H), 4.45 (d, 1H), 4.17 (br d, 1H), 3.84 (br d, 1H), 3.62-3.50 (m, 3H), 3.42 (tt, 1H), 3.13 (t, 1H), 3.07 (br d, 2H), 2.90 (t, 2H), 2.36-2.30 (m, 4H), 2.05 (dd, 1H).

### Beispiel 316

### {3-(3-Methyl-1,2,4-oxadiazol-5-yl)-5-[4-(trifluormethyl)phenyl]piperidin-1-yl}(1,3-thiazolidin-3-yl)methanon [enantiomerenreines cis-Isomer]

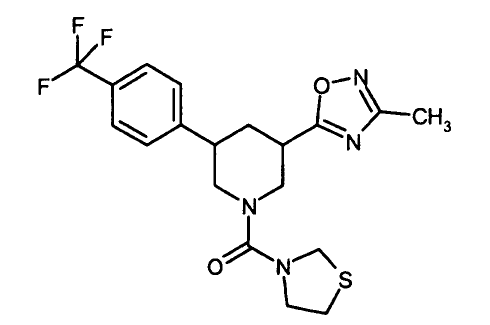

Nach der Allgemeinen Methode 1 wurden 250 mg (0.644 mmol) 1-(1,3-Thiazolidin-3-ylcarbonyl)-5-[4-(trifluormethyl)phenyl]piperidin-3-carbonsäure (Beispiel 102A) und 52.5 mg (0.708 mmol) *N'*-Hydroxyethanimidamid umgesetzt. Enantiomerentrennung des Racemates nach Methode 16D ergab 38.0 mg der Titelverbindung aus Beispiel 315 und 41.0 mg der Titelverbindung aus Beispiel 316 (29% d. Th.)

HPLC (Methode 16E): Rₜ = 35.97 min, >99.0% ee; LC-MS (Methode 3B): Rₜ = 2.17 min; MS (ESIpos): m/z = 427 [M+H]⁺.

### Beispiel 317

### {3-[3-(2-Methoxyethyl)-1,2,4-oxadiazol-5-yl]-5-[4-(trifluormethyl)phenyl]piperidin-1-yl}(1,3-thiazolidin-3-yl)methanon [enantiomerenreines cis-Isomer]

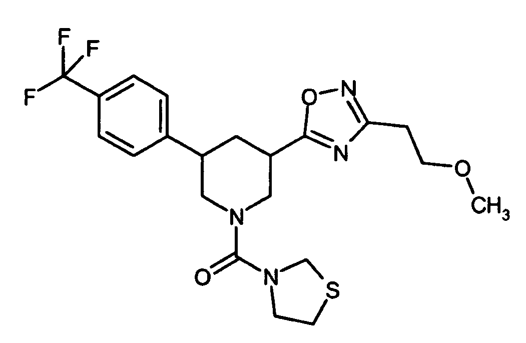

Nach der Allgemeinen Methode 1 wurden 250 mg (0.644 mmol) 1-(1,3-Thiazolidin-3-ylcarbonyl)-5-[4-(trifluormethyl)phenyl]piperidin-3-carbonsäure (Beispiel 102A) und 112 mg (0.708 mmol, 75% Reinheit) *N'*-Hydroxy-3-methoxypropanimidamid (Beispiel 64A) umgesetzt. Enantiomerentrennung des Racemates nach Methode 16D ergab 35.0 mg der Titelverbindung aus Beispiel 317 und 38.0 mg der Titelverbindung aus Beispiel 318 (24% d. Th.).

HPLC (Methode 16E): Rₜ = 8.21 min, >99.0% ee; LC-MS (Methode 3B): Rₜ = 8.21 min; MS (ESIpos): m/z = 471 [M+H]⁺;

¹H-NMR (400 MHz, DMSO-d₆): δ = 7.71 (d, 2H), 7.59 (d, 2H), 4.48 (d, 1H), 4.45 (d, 1H), 4.18 (br d, 1H), 3.84 (br d, 1H), 3.68 (t, 2H), 3.62-3.51 (m, 2H), 3.44 (tt, 1H), 3.23 (s, 3H), 3.18-3.05 (m, 3H), 2.94 (t, 2H), 2.90 (t, 2H), 2.35 (br d, 1H), 2.05 (dd, 1H).

### Beispiel 318

### {3-[3-(2-Methoxyethyl)-1,2,4-oxadiazol-5-yl]-5-[4-(trifluormethyl)phenyl]piperidin-1-yl}(1,3-thiazolidin-3-yl)methanon [enantiomerenreines cis-Isomer]

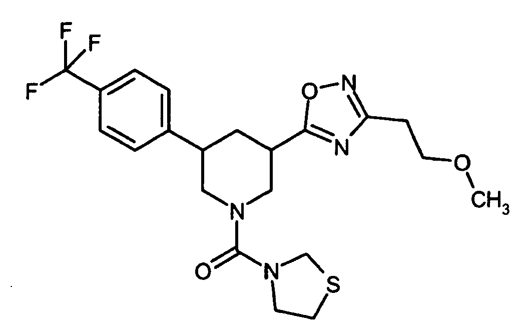

Nach der Allgemeinen Methode 1 wurden 250 mg (0.644 mmol) 1-(1,3-Thiazolidin-3-ylcarbonyl)-5-[4-(trifluormethyl)phenyl]piperidin-3-carbonsäure (Beispiel 102A) und 112 mg (0.708 mmol, 75% Reinheit) *N'*-Hydroxy-3-methoxypropanimidamid (Beispiel 64A) umgesetzt. Enantiomerentrennung des Racemates nach Methode 16D ergab 35.0 mg der Titelverbindung aus Beispiel 317 und 38.0 mg der Titelverbindung aus Beispiel 318 (24% d. Th.).

HPLC (Methode 16E): Rₜ = 16.51 min, >99.0% ee; LC-MS (Methode 3B): Rₜ = 8.21 min; MS (ESIpos): m/z = 471 [M+H]⁺.

### Beispiel 319

### {3-(4-Ethylphenyl)-5-[3-(2-methoxyethyl)-1,2,4-oxadiazol-5-yl]piperidin-1-yl}(3-hydroxyazetidin-1-yl)methanon [enantiomerenreines cis-Isomer]

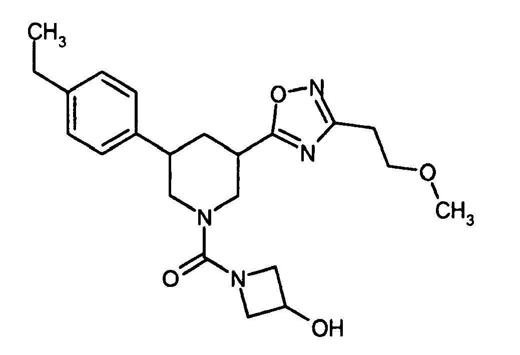

Enantiomerentrennung von 860 mg des Racemates aus Beispiel 394 nach Methode 14D ergab 415 mg der Titelverbindung aus Beispiel 319 (Enantiomer 1) und 420 mg der Titelverbindung aus Beispiel 320 (Enantiomer 2).

HPLC (Methode 19E): Rₜ = 4.72 min, >97.0% ee;

LC-MS (Methode 9B): Rₜ = 1.00 min; MS (ESIpos): m/z = 415 [M+H]⁺.

[α]₃₆₅ ²⁰= -17.5, Methanol

### Beispiel 320

### {3-(4-Ethylphenyl)-5-[3-(2-methoxyethyl)-1,2,4-oxadiazol-5-yl]piperidin-1-yl} (3-hydroxyazetidin-1-yl)methanon [enantiomerenreines cis-Isomer]

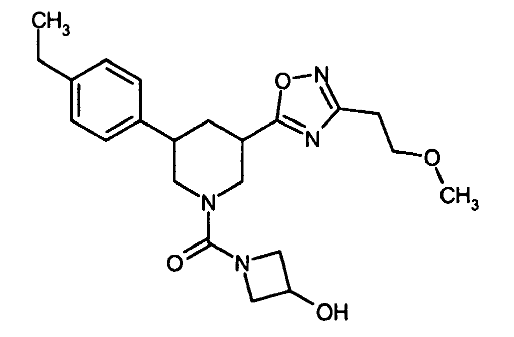

Enantiomerentrennung von 860 mg des Racemates aus Beispiel 394 nach Methode 14D ergab 415 mg der Titelverbindung aus Beispiel 319 (Enantiomer 1) und 420 mg der Titelverbindung aus Beispiel 320 (Enantiomer 2).

HPLC (Methode 19E): Rₜ = 8.12 min, >99.0% ee;

LC-MS (Methode 9B): Rₜ = 1.00 min; MS (ESIpos): m/z = 415 [M+H]⁺.

[α]₃₆₅²⁰= +15.8, Methanol

### Beispiel 321

### {3-[3-(2-Methoxyethyl)-1,2,4-oxadiazol-5-yl]-5-[3-methyl-4-(trifluormethoxy)phenyl]piperidin-1-yl}(morpholin-4-yl)methanon [enantiomerenreines cis-Isomer]

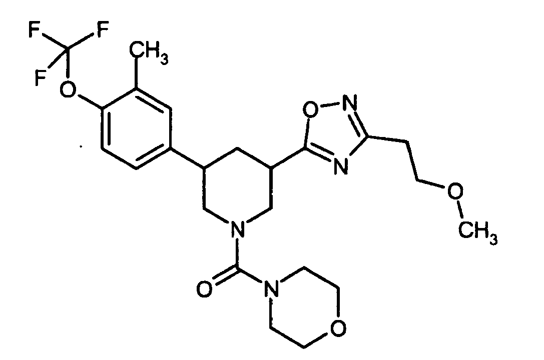

Enantiomerentrennung von 50 mg des Racemates aus Beispiel 399 nach Methode 23D ergab 13 mg der Titelverbindung aus Beispiel 321 (Enantiomer 1) und 12 mg der Titelverbindung aus Beispiel 322 (Enantiomer 2).

HPLC (Methode 20E): Rₜ = 24.15 min, >99.5% ee;

LC-MS (Methode 9B): Rₜ = 1.16 min; MS (ESIpos): m/z = 499 [M+H]⁺.

### Beispiel 322

### {3-[3-(2-Methoxyethyl)-1,2,4-oxadiazol-5-yl]-5-[3-methyl-4-(trifluormethoxy)phenyl]piperidin-1-yl}(morpholin-4-yl)methanon [enantiomerenreines cis-Isomer]

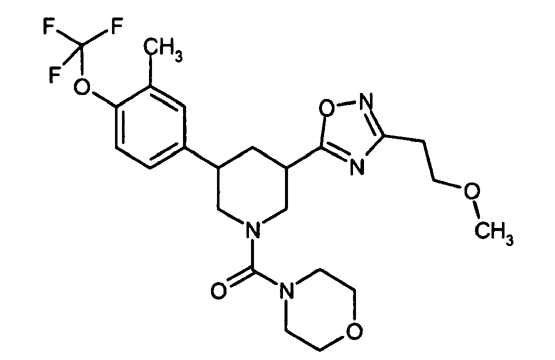

Enantiomerentrennung von 50 mg des Racemates aus Beispiel 399 nach Methode 23D ergab 13 mg der Titelverbindung aus Beispiel 321 (Enantiomer 1) und 12 mg der Titelverbindung aus Beispiel 322 (Enantiomer 2).

HPLC (Methode 20E): Rₜ = 68.57 min, >99.5% ee;

LC-MS (Methode 9B): Rₜ = 1.16 min; MS (ESIpos): m/z = 499 [M+H]⁺.

### Beispiel 323

### 1-({3-[3-(Propan-2-yl)-1,2,4-oxadiazol-5-yl]-5-[4-(trifluormethyl)phenyl]piperidin-1-yl}carbonyl)-piperidin-4-carbonitril [enantiomerenreines cis-Isomer]

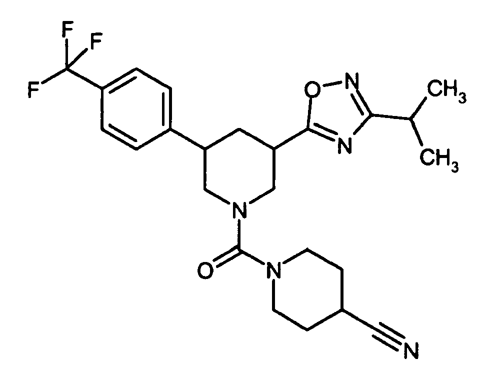

Enantiomerentrennung von 350 mg des Racemates aus Beispiel 284 nach Methode 13D ergab 160 mg der Titelverbindung aus Beispiel 323 (Enantiomer 1) und 170 mg der Titelverbindung aus Beispiel 324 (Enantiomer 2).

HPLC (Methode 16E): Rₜ = 5.33 min, >99.5% ee;

LC-MS (Methode 9B): Rₜ = 1.24 min; MS (ESIpos): m/z = 475 [M+H]⁺.

¹H-NMR (400 MHz, DMSO-d₆): δ = 7.70 (dd, 2H), 7.57 (d, 2H), 3.96 (br d, 1H), 3.56 (d, 1H), 3.45-3.34 (m, 3H), 3.13-2.97 (m, 7H), 2.36-2.29 (m, 1H), 2.01 (dd, 1H), 1.91-1.82 (m, 2H), 1.74-1.62 (m, 2H), 1.26 (d, 6H).

### Beispiel 324

### 1-({3-[3-(Propan-2-yl)- 1,2,4-oxadiazol-5-yl]-5-[4-(trifluormethyl)phenyl]piperidin-1-yl}carbonyl)-piperidin-4-carbonitril [enantiomerenreines cis-Isomer]

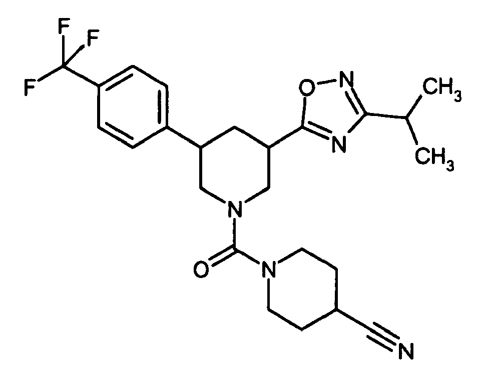

Enantiomerentrennung von 350 mg des Racemates aus Beispiel 284 nach Methode 13D ergab 160 mg der Titelverbindung aus Beispiel 323 (Enantiomer 1) und 170 mg der Titelverbindung aus Beispiel 324 (Enantiomer 2).

HPLC (Methode 16E): Rₜ = 6.18 min, >99.5% ee;

LC-MS (Methode 9B): Rₜ = 1.24 min; MS (ESIpos): m/z = 475 [M+H]⁺.

### Beispiel 325

### (3-Hydroxyazetidin-1-yl) {3-(3-methyl-1,2,4-oxadiazol-5-yl)-5-[4-(trifluormethyl)phenyl]piperidin-1-yl}methanon [enantiomerenreines cis-Isomer]

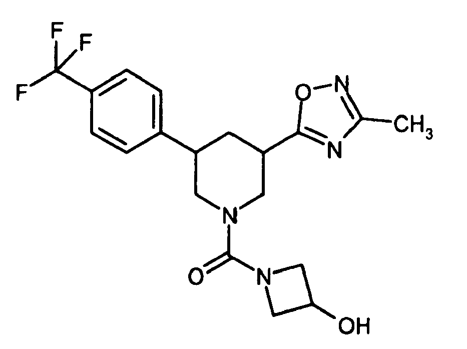

Enantiomerentrennung von 250 mg des Racemates aus Beispiel 298 nach Methode 24D ergab 86 mg der Titelverbindung aus Beispiel 325 (Enantiomer 1) und 77 mg der Titelverbindung aus Beispiel 326 (Enantiomer 2).

HPLC (Methode 11E): Rₜ = 4.69 min, >99.5% ee;

LC-MS (Methode 9B): Rₜ = 0.97 min; MS (ESIpos): m/z = 410 [M+H]⁺.

¹H-NMR (400 MHz, DMSO-d₆): δ = 7.70 (d, 2H), 7.56 (d, 2H), 5.58 (d, 1H), 4.43-4.34 (m, 1H), 4.15 (br d, 1H), 4.10 (q, 2H), 3.77 (br d, 1H), 3.73-3.66 (m, 2H), 3.30 (verdeckt, 1H), 3.07-2.92 (m, 3H), 2.2.35-2.28 (m, 4H), 2.04 (dd, 1H).

### Beispiel 326

### (3-Hydroxyazetidin-1-yl) {3-(3-methyl-1,2,4-oxadiazol-5-yl)-5-[4-(trifluormethyl)phenyl]piperidin-1-yl}methanon [enantiomerenreines cis-Isomer]

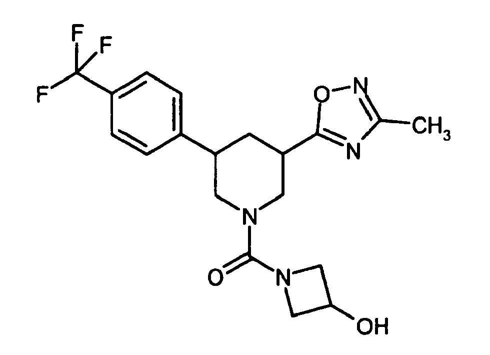

Enantiomerentrennung von 250 mg des Racemates aus Beispiel 298 nach Methode 24D ergab 86 mg der Titelverbindung aus Beispiel 325 (Enantiomer 1) und 77 mg der Titelverbindung aus Beispiel 326 (Enantiomer 2).

HPLC (Methode 11 E): Rₜ = 7.46 min, >99.5% ee;

LC-MS (Methode 9B): Rₜ = 0.97 min; MS (ESIpos): m/z = 410 [M+H]⁺.

### Beispiel 327

### {3-[3-Methyl-4-(trifluormethoxy)phenyl]-5-[3-(propan-2-yl)-1,2,4-oxadiazol-5-yl]piperidin-1-yl}-(morpholin-4-yl)methanon [enantiomerenreines cis-Isomer]

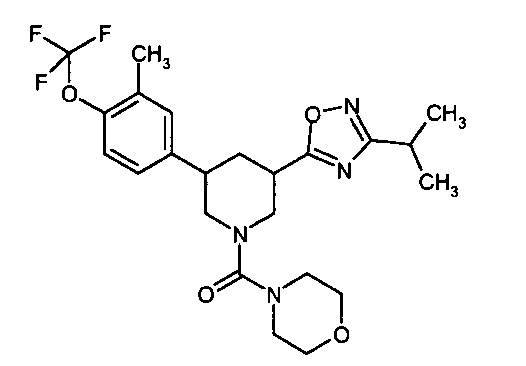

Enantiomerentrennung von 286 mg des Racemates aus Beispiel 403 nach Methode 25D ergab 117 mg der Titelverbindung aus Beispiel 327 (Enantiomer 1) und 121 mg der Titelverbindung aus Beispiel 328 (Enantiomer 2).

HPLC (Methode 21E): Rₜ = 6.67 min, >99.5% ee;

LC-MS (Methode 9B): Rₜ = 1.29 min; MS (ESIpos): m/z = 482 [M+H]⁺.

¹H-NMR (400 MHz, DMSO-d₆): δ = 7.37 (s, 2H), 7.27 (s, 2H), 3.99 (d, 1H), 3.51-3.64 (m, 5H), 3.35-3.44 (m, 1H), 3.15-3.25 (m, 4H), 2.85-3.10 (m, 4H), 2.22-2.35 (m, 4H), 1.96 (q, 1H), 1.26 (d, 6H).

### Beispiel 328

### {3-[3-Methyl-4-(trifluormethoxy)phenyl]-5-[3-(propan-2-yl)-1,2,4-oxadiazol-5-yl]piperidin-1-yl}-(morpholin-4-yl)methanon [enantiomerenreines cis-Isomer]

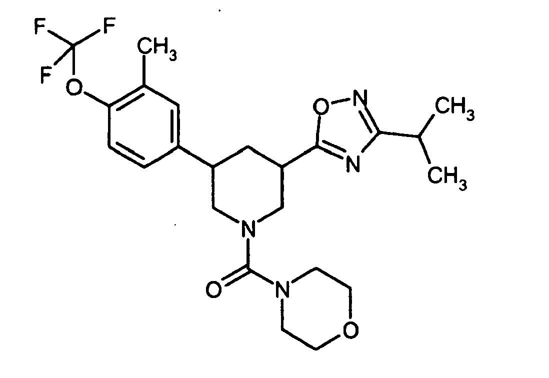

Enantiomerentrennung von 286 mg des Racemates aus Beispiel 403 nach Methode 25D ergab 117 mg der Titelverbindung aus Beispiel 327 (Enantiomer 1) und 121 mg der Titelverbindung aus Beispiel 328 (Enantiomer 2).

HPLC (Methode 21E): Rₜ = 10.82 min, >99.5% ee;

LC-MS (Methode 9B): Rₜ = 1.29 min; MS (ESIpos): m/z = 482 [M+H]⁺.

¹H-NMR (400 MHz, DMSO-d₆): δ = 7.37 (s, 2H), 7.27 (s, 2H), 3.99 (d, 1H), 3.51-3.64 (m, 5H), 3.35-3.44 (m, 1H), 3.15-3.25 (m, 4H), 2.85-3.10 (m, 4H), 2.22-2.35 (m, 4H), 1.96 (q, 1H), 1.26 (d, 6H).

### Beispiel 329

### {3-[3-(4-Methylpyridin-3-yl)-1,2,4-oxadiazol-5-yl]-5-[4-(trifluormethyl)phenyl]piperidin-1-yl}-(morpholin-4-yl)methanon [racemisches cis-Isomer]

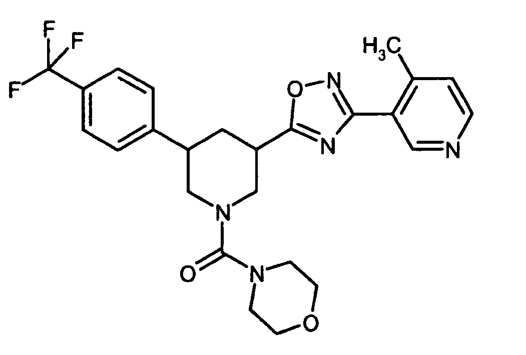

Nach der Allgemeinen Methode 1 wurden 250 mg (ca. 0.520 mmol) der Verbindung aus Beispiel 49A und 87 mg (0.580 mmol) *N'*-Hydroxy-4-methylpyridin-3-carboximidamid (Beispiel 70A) umgesetzt. Ausbeute: 22 mg (8% d. Th.).

LC-MS (Methode 1B): Rₜ = 2.37 min; MS (ESIpos): m/z = 502 [M+H]⁺;

¹H-NMR (400 MHz, DMSO-d₆): δ = 9.02 (s, 1H), 8.60 (d, 1H), 7.71 (d, 2H), 7.60 (d, 2H), 7.47 (d, 1H), 4.11 (br d, 1H), 3.66 (d, 1H), 3.62-3.50 (m, 5H), 3.26-3.14 (m, 5H), 3.12-3.06 (m, 2H), 2.59 (s, 3H), 2.43 (br d, 1H), 2.11 (q, 1H).

### Beispiel 330

### {3-[3-(5-Methylpyridin-2-yl)-1,2,4-oxadiazol-5-yl]-5-[4-(trifluormethyl)phenyl]piperidin-1-yl}-(morpholin-4-yl)methanon [racemisches cis-Isomer]

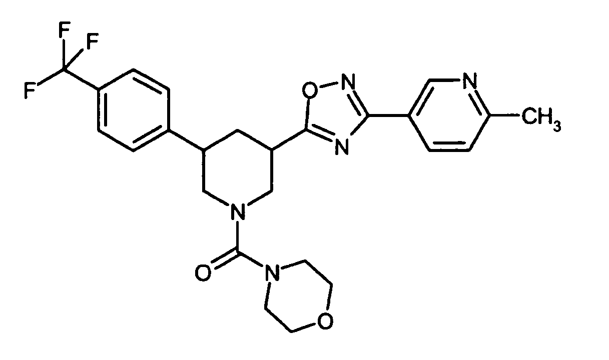

Nach der Allgemeinen Methode 1 wurden 250 mg (ca. 0.520 mmol) der Verbindung aus Beispiel 49A und 87 mg (0.58 mmol) *N'*-Hydroxy-5-methylpyridin-3-carboximidamid (Beispiel 71A) umgesetzt. Ausbeute: 47 mg (18% d. Th.).

LC-MS (Methode 1B): Rₜ = 2.38 min; MS (ESIpos): m/z = 502 [M+H]⁺;

¹H-NMR (400 MHz, DMSO-d₆): δ = 9.04 (s, 1H), 8.24 (dd, 1H), 7.71 (d, 2H), 7.60 (d, 2H), 7.47 (d, 1H), 4.09 (br d, 1H), 3.65(d, 1H), 3.61-3.50 (m, 5H), 3.23-3.21 (m, 4H), 3.16 (t, 1H), 3.08-3.03 (m, 2H), 2.56 (s, 3H), 2.42 (br d, 1H), 2.11 (q, 1H).

### Beispiel 331

### {3-(3-Ethyl-1,2,4-oxadiazol-5-yl)-5-[4-(trifluormethyl) phenyl]piperidin-1-yl}(morpholin-4-yl)-methanon [racemisches cis-Isomer]

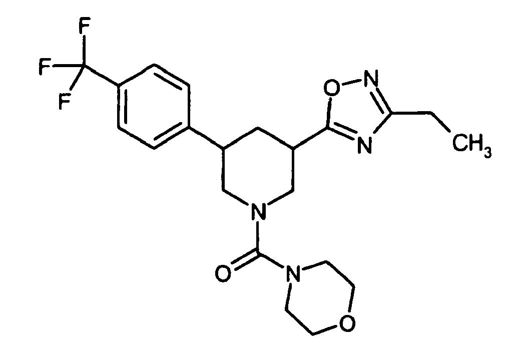

Nach der Allgemeinen Methode 2 wurden 150 mg (0.388 mmol) der Verbindungen aus Beispiel 81A und 68 mg (0.78 mmol) *N'*-Hydroxypropanimidamid umgesetzt. Ausbeute: 123 mg (70% d. Th.)

LC-MS (Methode 2B): Rₜ = 1.29 min; MS (ESIpos): m/z = 439 [M+H]⁺;

¹H-NMR (400 MHz, DMSO-d₆): δ = 7.70 (d, 2H), 7.57 (d, 2H), 3.98 (br d, 1H), 3.63 (d, 1H), 3.59-3.53 (m, 4H), 3.40 (tt, 1H), 3.23-3.15 (m, 4H), 3.09-2.98 (m, 3H), 2.71 (q, 2H), 2.33 (brd, 1H), 2.01 (q, 1H), 1.22 (t, 3H).

### Beispiel 332

### Morpholin-4-yl {3-(3-propyl-1,2,4-oxadiazol-5-yl)-5-[4-(trifluormethyl)phenyl]piperidin-1-yl}-methanon [racemisches cis-Isomer]

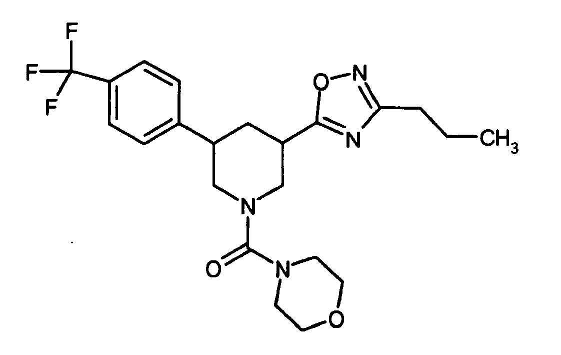

Nach der Allgemeinen Methode 2 wurden 150 mg (0.388 mmol) der Verbindung aus Beispiel 81A und 79 mg (0.78 mmol) *N'*-Hydroxybutananimidamid umgesetzt. Ausbeute: 185 mg (100% d. Th.)

LC-MS (Methode 2B): Rₜ = 1.36 min; MS (ESIpos): m/z = 453[M+H]⁺;

¹H-NMR (400 MHz, DMSO-d₆): δ = 7.70 (d, 2H), 7.57 (d, 2H), 3.99 (br d, 1H), 3.63 (d, 1H), 3.59-3.53 (m, 4H), 3.40 (tt, 1H), 3.23-3.17 (m, 4H), 3.08-2.98 (m, 3H), 2.66 (t, 2H), 2.32 (brd, 1H), 2.01 (q, 1H), 1.73-1.64 (m, 2H), 0.92 (t, 3H).

### Beispiel 333

### Morpholin-4-yl {3-[3-(propan-2-yl)-1,2,4-oxadiazol-5-yl]-5-[4-(trifluonnethyl)phenyl]piperidin-1 - yl}methanon [enantiomerenreines cis-Isomer]

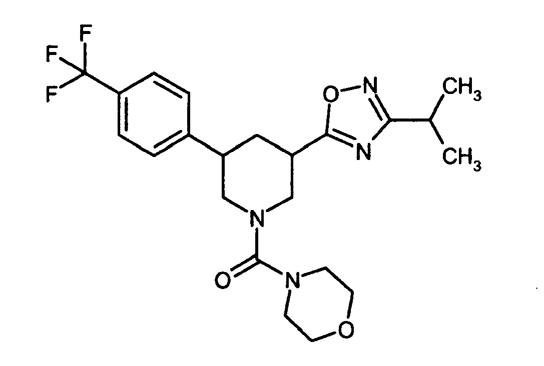

Enantiomerentrennung von 50 mg des Racemates aus Beispiel 402 nach Methode 8D ergab 22 mg der Titelverbindung aus Beispiel 333 und 21 mg der Titelverbindung aus Beispiel 334.

HPLC (Methode 6E): Rₜ = 9.65 min, >99% ee; LC-MS (Methode 2B): Rₜ = 1.36 min; MS (ESIpos): m/z = 453[M+H]⁺.

### Beispiel 334

### Morpholin-4-yl{3-[3-(propan-2-yl)-1,2,4-oxadiazol-5-yl]-5-[4-(trifluormethyl)phenyl]pipendin-1-yl}methanon [enantiomerenreines cis-Isomer]

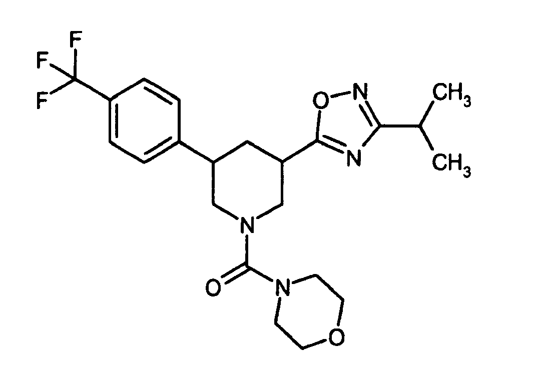

Enantiomerentrennung von 50 mg des Racemates aus Beispiel 402 nach Methode 8D ergab 22 mg der Titelverbindung aus Beispiel 333 und 21 mg der Titelverbindung aus Beispiel 334.

HPLC (Methode 6E): Rₜ = 20.25 min, >99% ee; LC-MS (Methode 2B): Rₜ = 1.36 min; MS (ESIpos): m/z = 453 [M+H]⁺.

### Beispiel 335

### {3-(3-Methyl-1,2,4-oxadiazol-5-yl)-5-[4-(trifluormethyl) phenyl]piperidin-1-yl}(morpholin-4-yl)-methanon [enantiomerenreines cis-Isomer]

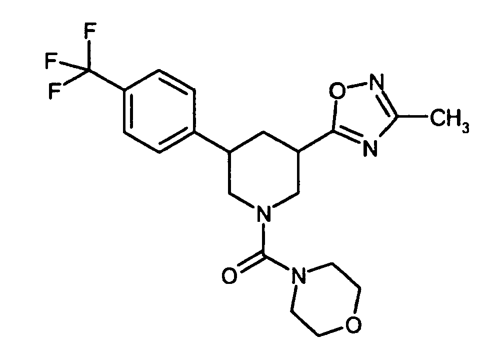

Enantiomerentrennung von 80 mg des Racemates aus Beispiel 416 nach Methode 10D ergab 34 mg der Titelverbindung aus Beispiel 335 und 34 mg der Titelverbindung aus Beispiel 336.

HPLC (Methode 8E): Rₜ = 6.93 min, >99.0% ee; LC-MS (Methode 2B): Rₜ = 1.21 min; MS (ESIpos): m/z = 425[M+H]⁺.

### Beispiel 336

### {3-(3-Methyl-1,2,4-oxadiazol-5-yl)-5-[4-(trifluormethyl) phenyl]piperidin-1-yl}(morpholin-4-yl)-methanon [enantiomerenreines cis-Isomer]

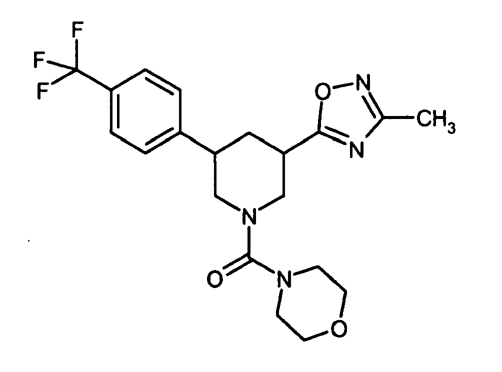

Enantiomerentrennung von 80 mg des Racemates aus Beispiel 416 nach Methode 10D ergab 34 mg der Titelverbindung aus Beispiel 335 und 34 mg der Titelverbindung aus Beispiel 336.

HPLC (Methode 8E): Rₜ = 13.30 min, >99.0% ee; LC-MS (Methode 2B): Rₜ = 1.21 min; MS (ESIpos): m/z = 425 [M+H]⁺.

### Beispiel 337

### Morpholin-4-yl{3-[5-(trifluormethyl)-1,2,4-oxadiazol-3-yl]-5-[4-(trifluormethyl)phenyl]piperidin-1-yl}methanon [racemisches cis-Isomer]

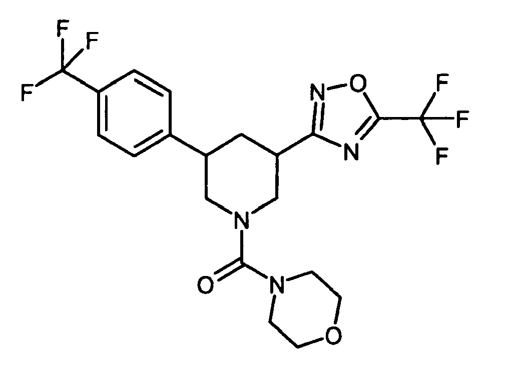

Nach der Allgemeinen Methode 2 wurden 49 mg (0.43 mmol) Trifluoressigsäure und 120 mg (ca. 0.216 mmol) der Verbindung aus Beispiel 84A umgesetzt. Ausbeute: 13 mg (12% d. Th.).

LC-MS (Methode 2B): Rₜ = 1.42 min; MS (ESIpos): m/z = 479 [M+H]⁺;

¹H-NMR (400 MHz, DMSO-d₆): δ = 7.70 (d, 2H), 7.58 (d, 2H), 3.98 (br d, 1H), 3.65 (d, 1H), 3.59-3.53 (m, 4H), 3.37 (tt, 1H), 3.23-3.16 (m, 4H), 3.08-3.02 (m, 3H), 2.32 (br d, 1H), 2.01 (q, 1H).

### Beispiel 338

### {3-{5-[(Methylamino)methyl]-1,2,4-oxadiazol-3-yl}-5-[4-(trifluormethyl)phenyl]piperidin-1-yl}-(morpholin-4-yl)methanon Hydrochlorid [racemisches cis-Isomer]

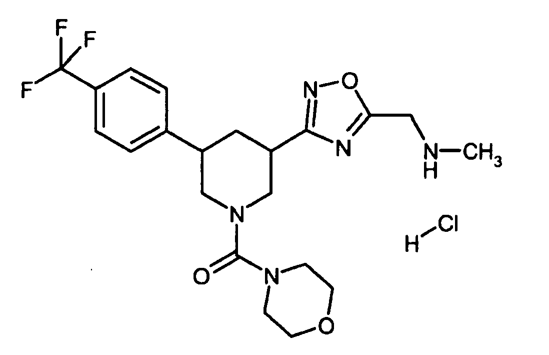

Zu einer Lösung von 96 mg (0.17 mmol) der Verbindung aus Beispiel 90A in 0.8 ml Dioxan wurden 0.43 ml (1.73 mmol) einer 4N Chlorwasserstoff-Dioxan-Lösung zugegeben, und die Mischung wurde 24 h bei Raumtemperatur gerührt. Anschließend wurde das Reaktionsgemisch im Vakuum eingeengt, mit Dichlormethan versetzt und wieder eingeengt. Ausbeute: 85 mg (99% d. Th).

LC-MS (Methode 2B): Rₜ = 0.91 min; MS (ESIpos): m/z = 454 [M+H-HCl]⁺;

¹H-NMR (400 MHz, DMSO-d₆): δ = 9.64 (br s, 1H), 7.71 (d, 2H), 7.57 (d, 2H), 4.65 (d, 2H), 4.00 (br d, 1H), 3.66 (d, 2H), 3.24 (tt, 1H), 3.22-3.19 (m, 4H), 3.09-2.93 (m, 3H), 2.70 (s, 3H), 2.30 (br d, 1H), 1.96 (q, 1H).

### Beispiel 339

### Morpholin-4-yl{3-(5-phenyl-1,3-thiazol-2-yl)-5-[4-(trifluormethyl)phenyl]piperidin-1-yl}methanon [racemisches cis-Isomer]

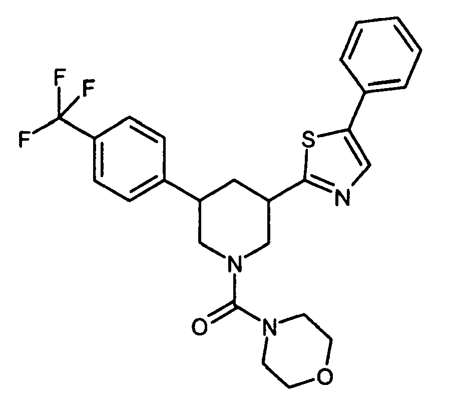

Zu einer Lösung von 300 mg (ca. 0.371 mmol) der Verbindung aus Beispiel 106A in 5 ml Dioxan wurden 600 mg (1.484 mmol) Lawesson-Reagenz hinzugegeben. Das Reaktionsgemisch wurde 5 h bei 50°C gerührt und ohne weitere Reinigung mittels präparativer HPLC gereinigt. Ausbeute: 5 mg (3% d. Th.)

LC-MS (Methode 2B): Rₜ = 1.49 min; MS (ESIpos): m/z = 502 [M+H]⁺;

¹H-NMR (400 MHz, DMSO-d₆): δ = 8.14 (s, 1H), 7.71 (d, 2H), 7.62 (dd, 4H), 7.44 (dd, 2H), 7.35 (dd, 1H), 4.03 (br d, 1H), 3.68 (d, 1H), 3.59-3.55 (m, 4H), 3.41 (tt, 1H), 3.23-3.18 (m, 4H), 3.09-2.99 (m, 3H), 2.36 (d, 1H), 2.05 (q, 1H).

Die in Tabelle 1 aufgeführten Verbindungen wurden nach der Allgemeinen Methode 2 aus den entsprechenden Edukten hergestellt.

**Tabelle 1:**

| **Bsp. Nr.** | **Struktur** | **Ausbeute (% d. Th.)** | **MS (ESI) [M+H]⁺; LC-MS: Rₜ (Methode)** | **¹H-NMR (400 MHz, DMSO-d₆)** |
|---|---|---|---|---|
| **340** | 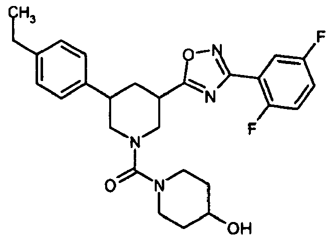 | 51 | m/z = 497; 1.41 min (Methode 2B) | δ = 7.81-7.76 (m, 1H), 7.56-7.52 (m, 2H), 7.24 (d, 2H), 7.18 (d, 2H), 4.69 (d, OH), 4.06 (br d, 1H), 3.63-3.47 (m, 5H), 3.07 (t, 1H), 2.99-2.86 (m, 4H), 2.58 (q, 2H), 2.38 (br d, 1H), 2.03 (q, 1H), 1.74-1.71 (m, 2H), 1.36-1.27 (m, 2H), 1.17 (t, 3H). |
| **341** | 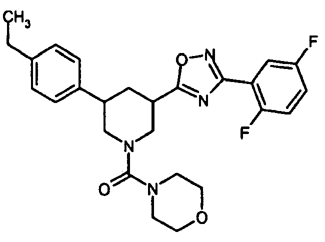 | 54 | m/z = 483; 1.48 min (Methode 2B) | δ = 7.81-7.77 (m, 1H), 7.57-7.53 (m, 2H), 7.24 (d, 2H), 7.18 (d, 2H), 4.09 (br d, 1H), 3.62 (br d, 1H), 3.58-3.56 (m, 4H), 3.54-3.48 (m, 1H), 3.22-3.19 (m, 4H), 3.11 (t, 1H), 3.01-2.88 (m, 2H), 2.56 (q, 2H), 2.38 (br d, 1H), 2.04 (q, 1H), 1.17 (t, 3H). |
| **342** | 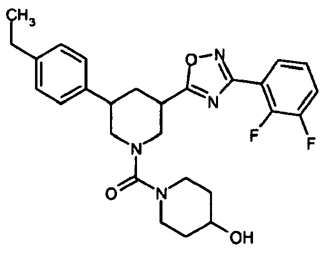 | 57 | m/z = 487; 1.42 min (Methode 2B) | δ = 7.85 (dd, 1H), 7.75-7.68 (m, 1H), 7.46-7.40 (m, 1H), 7.24 (d, 2H), 7.18 (d, 2H), 4.68 (d, OH), 4.05 (br d, 1H), 3.63-3.48 (m, 5H), 3.08 (t, 1H), 2.96-2.86 (m, 4H), 2.56 (q, 2H), 2.37 (br d, 1H), 2.04 (q, 1H), 1.75-1.71 (m, 2H), 1.36-1.28 (m, 2H), 1.17 (t, 3H). |
| **343** | 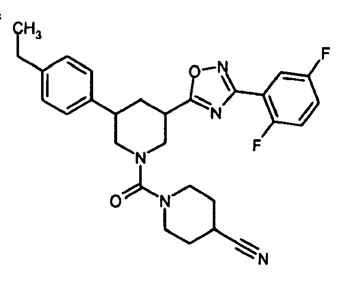 | 68 | m/z = 506; 1.52 min (Methode 2B) | δ = 7.78 (dd, 1H), 7.56-7.50 (m, 2H), 7.24 (d, 2H), 7.18 (d, 2H), 4.06 (br d, 1H), 3.57 (br d, 1H), 3.54-3.47 (m, 1H), 3.41-3.37 (m, 2H), 3.13-3.03 (m, 4H), 3.00-2.87 (m, 2H), 2.58 (q, 2H), 2.37 (br d, 1H), 2.03 (q, 1H), 1.90-1.86 (m, 2H), 1.73-1.65 (m, 2H), 1.17 (t, 3H). |
| **344** | 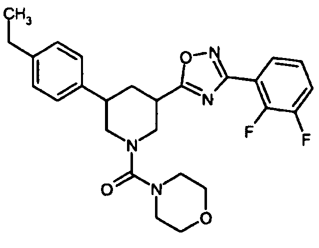 | 60 | m/z = 483; 2.85 min (Methode 1B) | δ = 7.87-7.83 (m, 1H), 7.74-7.67 (m, 1H), 7.46-7.41 (m, 1H), 7.24 (d, 2H), 7.18 (d, 2H), 4.10 (br d, 1H), 3.63-3.48 (m, 6H), 3.22-3.20 (m, 4H), 3.11 (t, 1H), 3.02-2.87 (m, 2H), 2.58 (q, 2H), 2.39 (br d, 1H), 2.05 (q, 1H), 1.17 (t, 3H). |
| **345** | 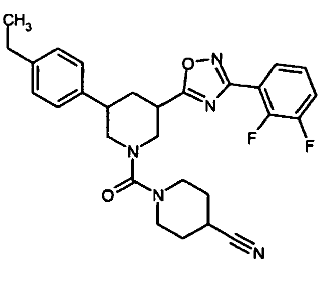 | 71 | m/z = 506; 1.53 min (Methode 2B) | δ = 7.86 (dd, 1H), 7.74-7.67 (m, 1H), 7.46-7.41 (m, 1H), 7.24 (d, 2H), 7.18 (d, 2H), 4.07 (br d, 1H), 3.57 (br d, 1H), 3.55-3.48 (m, 1H), 3.40-3.37 (m, 2H), 3.16-3.02 (m, 4H), 2.97-2.87 (m, 2H), 2.58 (q, 2H), 2.36 (br d, 1H), 2.04 (q, 1H), 1.90-1.86 (m, 2H), 1.73-1.65 (m, 2H), 1.17 (t, 3H). |
| **346** | 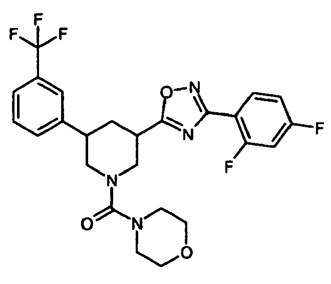 | 68 | m/z = 523; 2.73 min (Methode 1B) | δ = 8.13-8.07 (m, 1H), 7.74 (s, 1H), 7.69 (d, 1H), 7.64-7.52 (m, 3H), 7.32 (dd, 1H), 4.08 (br d, 1H), 3.64 (br d, 1H), 3.58-3.54 (m, 4H), 3.53-3.48 (m, 1H), 3.23-3.21 (m, 4H), 3.17-3.07 (m, 3H), 2.41 (br d, 1H), 2.13 (q, 1H). |
| **347** | 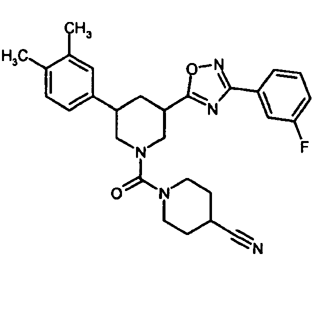 | 73 | m/z = 488; 1.54 min (Methode 2B) | δ = 7.87 (d, 1H), 7.76 (dd, 1H), 7.67-7.61 (m, 1H), 7.49-7.44 (m, 1H), 7.11-7.08 (m, 2H), 7.03 (d, 1H), 4.05 (br d, 1H), 3.56 (br d, 1H), 3.55-3.43 (m, 1H), 3.40-3.37 (m, 2H), 3.12-3.02 (m, 4H), 2.95 (t, 1H), 2.88-2.82 (m, 1H), 2.35 (br d, 1H), 2.21 (s, 3H), 2.19 (s, 3H), 2.07-1.99 (m, 1H), 1.90-1.86 (m, 2H), 1.70-1.65 (m, 2H). |
| **348** | 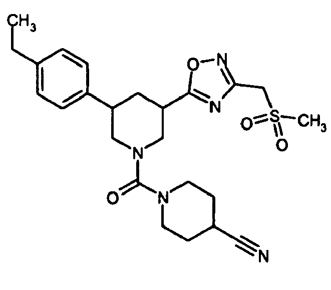 | 41 | m/z = 486; 1.93 min (Methode 3B) | δ = 7.22 (d, 2H), 7.17 (d, 2H), 4.85 (s, 2H), 4.00 (br d, 1H), 3.55 (br d, 1H), 3.50-3.42 (m, 1H), 3.39-3.35 (m, 2H), 3.16 (s, 3H), 3.10-2.97 (m, 4H), 2.94-2.84 (m, 2H), 2.57 (q, 2H), 2.33 (br d, 1H), 1.97 (q, 1H), 1.88-1.85 (m, 2H), 1.69-1.64 (m, 2H), 1.16 (t, 3H). |
| **349** | 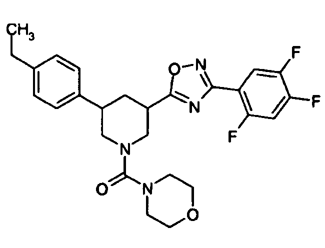 | 64 | m/z = 501; 2.91 min (Methode 1B) | δ = 8.08-8.02 (m, 1H), 7.91-7.84 (m, 1H), 7.24 (d, 2H), 7.18 (d, 2H), 4.09 (br d, 1H), 3.63-3.49 (m, 6H), 3.22-3.20 (m, 4H), 3.10 (t, 1H), 3.01-2.90 (m, 2H), 2.57 (q, 2H), 2.36 (br d, 1H), 2.03 (q, 1H), 1.17 (t, 3H). |
| **350** | 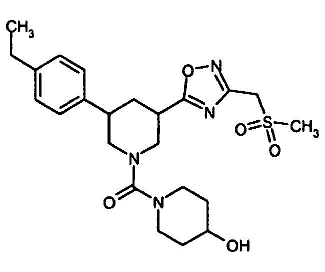 | 34 | m/z = 477; 2.03 min (Methode 1B) | δ = 7.22-7.18 (m, 4H), 4.85 (s, 2H), 4.68 (br s, 1H), 3.99 (br d, 1H), 3.60-3.46 (m, 6H), 3.16 (s, 3H), 3.01 (t, 1H), 2.92-2.87 (m, 4H), 2.34 (br d, 1H), 2.01 (q, 1H), 1.73-1.67 (m, 2H), 1.31-1.29 (m, 2H), 1.16 (t, 3H). |
| **351** | 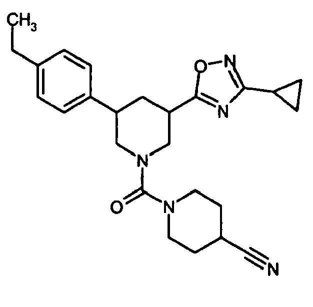 | 36 | m/z = 434; 2.29 min (Methode 3B) | δ = 7.19-7.13 (m, 4H), 4.07 (br d, 1H), 3.75 (br d, 1H), 3.52-3.47 (m, 2H), 3.33-3.19 (m, 3H), 3.01 (t, 1H), 2.88-2.80 (m, 3H), 2.63 (q, 2H), 2.42 (br d, 1H), 2.10-2.05 (m, 1H), 1.97-1.80 (m, 5H), 1.23 (t, 3H), 1.07-0.99 (m, 4H). |
| **352** | 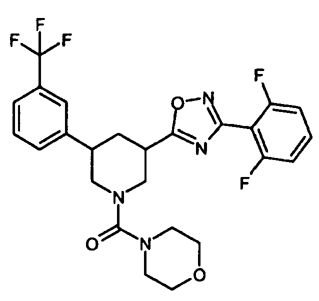 | 51 | m/z = 523; 2.30 min (Methode 3B) | δ = 7.77-7.73 (m, 2H), 7.68 (d, 1H), 7.64-7.57 (m, 2H), 7.36 (dd, 2H), 4.07 (br d, 1H), 3.63 (br d, 1H), 3.58-3.53 (m, 5H), 3.23-3.21 (m, 4H), 3.17-3.06 (m, 3H), 2.41 (br d, 1H), 2.14 (q, 1H). |
| **353** | 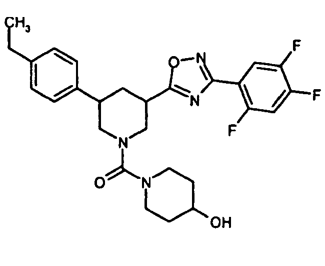 | 49 | m/z = 515; 1.45 min (Methode 2B) | δ = 8.08-8.03 (m, 1H), 7.90-7.83 (m, 1H), 7.23 (d, 2H), 7.18 (d, 2H), 4.68 (d, OH), 4.07 (br d, 1H), 3.64-3.48 (m, 5H), 3.07 (t, 1H), 2.99-2.68 (m, 4H), 2.58 (q, 2H), 2.37 (br d, 1H), 2.03 (q, 1H), 1.74-1.71 (m, 2H), 1.36-1.27 (m, 2H), 1.17 (t, 3H). |
| **354** | 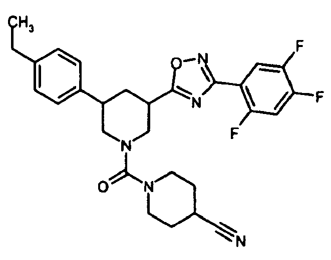 | 73 | m/z = 524; 2.94 min (Methode 1B) | δ = 8.08-8.02 (m, 1H), 7.90-7.84 (m, 1H), 7.24 (d, 2H), 7.18 (d, 2H), 4.06 (br d, 1H), 3.57 (br d, 1H), 3.54-3.47 (m, 1H), 3.40-3.37 (m, 2H), 3.12-3.02 (m, 4H), 2.99-2.87 (m, 2H), 2.58 (q, 2H), 2.34 (br d, 1H), 2.03 (q, 1H), 1.89-1.86 (m, 2H), 1.72-1.65 (m, 2H), 1.17 (t, 3H). |
| **355** | 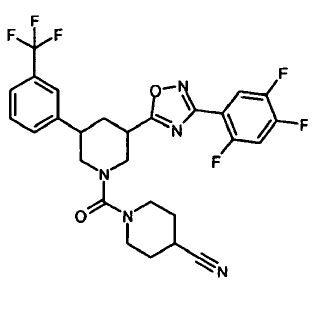 | 62 | m/z = 541; 2.45 min (Methode 3B) | δ = 8.08-8.01 (m, 1H), 7.91-7.83 (m, 1H), 7.73 (s, 1H), 7.68 (d, 1H), 7.64-7.57 (m, 2H), 4.08 (br d, 1H), 3.64 (br d, 1H), 3.58-3.50 (m, 5H), 3.23-3.19 (m, 4M, 3.16-3.09 (m, 3H), 2.40 (br d, 1H), 2.12 (q, 1H). |
| **356** | 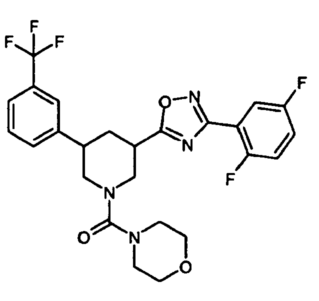 | 70 | m/z = 523; 2.73 min (Methode 1B) | δ = 7.80-7.76 (m, 1H), 7.73 (s, 1H), 7.68 (d, 1H), 7.64-7.61 (m, 2H), 7.61-7.57 (m, 2H), 4.08 (br d, 1H), 3.64 (br d, 1H), 3.58-3.49 (m, 4H), 3.23-3.21 (m, 4H), 3.17-3.07 (m, 3H), 2.41 (br d, 1H), 2.14 (q, 1H). |
| **357** | 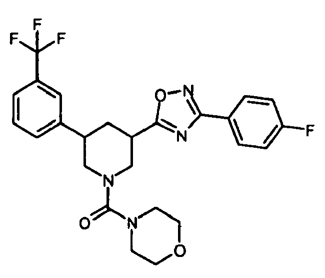 | 73 | m/z = 505; 2.44 min (Methode 3B) | δ = 8.10-8.05 (m, 2H), 7.74 (s, 1H), 7.68 (d, 1H), 7.64-7.54 (m, 2H), 7.45-7.39 (m, 2H), 4.08 (br d, 1H), 3.64 (br d, 1H), 3.58-3.54 (m, 4H), 3.53-3.46 (m, 1H), 3.23-3.21 (m, 4H), 3.17-3.05 (m, 3H), 2.41 (br d, 1H), 2.13 (q, 1H). |
| **358** | 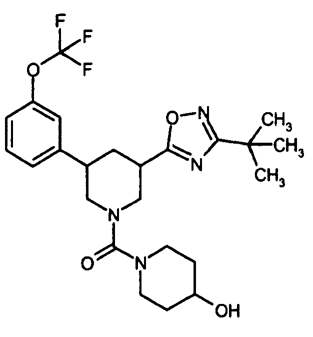 | 36 | m/z = 497; 2.24 min (Methode 3B) | δ = 7.48 (dd, 1H), 7.39-7.35 (m, 2H), 7.25 (d, 1H), 4.68 (d, OH), 3.94 (br d, 1H), 3.63-3.58 (m, 1H), 3.55 (br d, 1H), 3.50-3.46 (m, 2H), 3.39-3.36 (m, 1H), 3.05-2.87 (m, 5H), 2.31 (br d, 1H), 1.98 (q, 1H), 1.73-1.70 (m, 2H), 1.34-1.27 (m, 11H). |
| **359** | 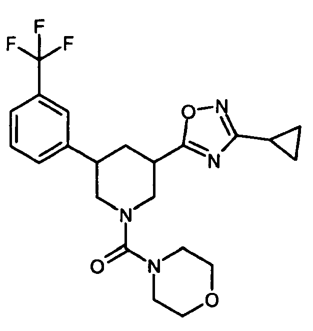 | 56 | m/z = 451; 1.32 min (Methode 2B) | δ = 7.71 (s, 1H), 7.64-7.55 (m, 3H), 3.95 (br d, 1H), 3.63-3.54 (m, 5H), 3.38-3.32 (m, 1H), 3.20-3.16 (m, 4H), 3.06-3.01 (m, 3H), 2.30 (br d, 1H), 2.13-2.07 (m, 1H), 2.00 (q, 1H), 1.08-1.03 (m, 2H), 0.90-0.86 (m, 2H). |
| **360** | 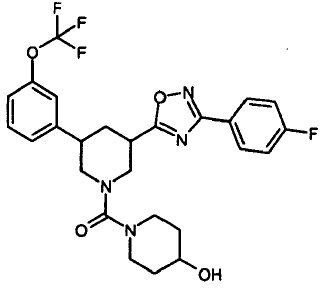 | 45 | m/z = 535; 2.33 min (Methode 3B) | δ = 8.09-8.05 (m, 2H), 7.50 (dd, 1H), 7.44-7.36 (m, 4H), 7.26 (d, 1H), 4.69 (d, OH), 4.03 (br d, 1H), 3.63-3.57 (m, 2H), 3.52-3.49 (m, 3H), 3.17-3.02 (m, 3H), 2.93 (t, 2H), 2.39 (br d, 1H), 2.08 (q, 1H), 1.74-1.71 (m, 2H), 1.36-1.28 (m, 2H). |
| **361** | 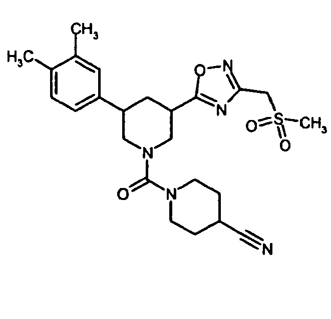 | 59 | m/z = 486; 1.18 min (Methode 2B) | δ = 7.10-7.08 (m, 2H), 7.01 (d, 1H), 4.85 (s, 2H), 4.00 (br d, 1H), 3.53 (br d, 1H), 3.49-3.41 (m, 1H), 3.39-3.34 (m, 2H), 3.32 (s, 3H), 3.30 (s, 3H), 3.16 (s, 3H), 3.10-3.00 (m, 4H), 2.92 (t, 1H), 2.85-2.77 (m, 1H), 2.30 (br d, 1H), 1.96 (q, 1H), 1.88-1.84 (m, 2H), 1.71-1.63 (m, 2H). |
| **362** | 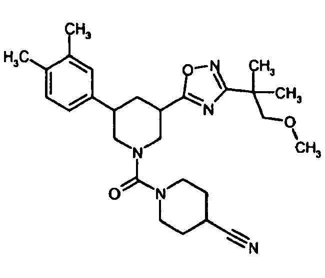 | 73 | m/z = 480; 1.43 min (Methode 2B) | δ = 7.09-7.07 (m, 2H), 7.00 (d, 1H), 3.95 (br d, 1H), 3.52 (br d, 1H), 3.44 (s, 2H), 3.39-3.33 (m, 3H), 3.20 (s, 3H), 3.10-2.90 (m, 5H), 2.83-2.76 (m, 1H), 2.25 (br d, 1H), 2.20 (s, 3H), 2.18 (s, 3H), 1.97-1.85 (m, 3H), 1.71-1.62 (m, 2H), 1.26 (s, 6H). |
| **363** | 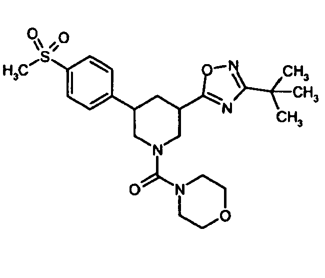 | 57 | m/z = 477; 2.08 min (Methode 1B) | δ = 7.89 (d, 2H), 7.63 (d, 2H), 4.00 (br d, 1H), 3.62 (br d, 1H), 3.57-3.55 (m, 4H), 3.44-3.34 (m, 1H), 3.20-3.16 (m, 7H), 3.09-3.03 (m, 3H), 2.34 (br d, 1H), 2.02 (q, 1H), 1.30 (s, 9H). |
| **364** | 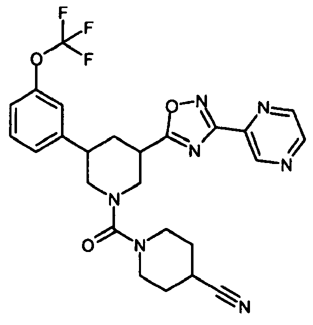 | 42 | m/z = 528; 2.05 min (Methode 3B) | δ = 9.28 (d, 1H), 8.90-8.87 (m, 2H), 7.50 (dd, 1H), 7.42-7.38 (m, 2H), 7.27 (d, 1H), 4.05 (br d, 1H), 3.61 (br d, 1H), 3.58-3.51 (m, 1H), 3.42-3.38 (m, 2H), 3.19-3.00 (m, 6H), 2.41 (br d, 1H), 2.11 (q, 1H), 1.91-1.87 (m, 2H), 1.74-1.66 (m, 2H). |
| **365** | 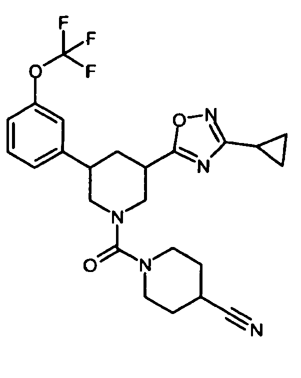 | 88 | m/z = 490; 1.40 min (Methode 2B) | δ = 7.48 (dd, 1H), 7.38-7.34 (m, 2H), 7.24 (d, 1H), 3.91 (br d, 1H), 3.55 (br d, 1H), 3.39-3.34 (m, 4H), 3.10-2.94 (m, 6H), 2.30 (br d, 1H), 1.95 (q, 1H), 1.88-1.84 (m, 2H), 1.71-1.63 (m, 2H), 1.08-1.03 (m, 2H), 0.90-0.86 (m, 2H). |
| **366** | 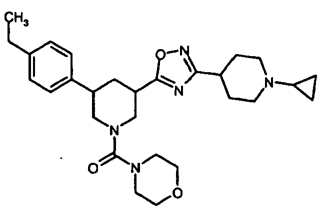 | 22 | m/z = 494; 1.49 min (Methode 1B) | δ = 7.22 (d, 2H), 7.16 (d, 2H), 3.99 (br d, 1H), 3.59-3.44 (m, 5H), 3.39-3.34 (m, 1H), 3.20-3.18 (m, 4H), 3.03-2.85 (m, 4H), 2.80-2.71 (m, 2H), 2.57 (q, 2H), 2.30-2.25 (m, 3H), 1.98-1.88 (m, 3H), 1.64-1.54 (m, 3H), 1.16 (t, 3H), 0.43-0.39 (m, 2H), 0.30-0.27 (m, 2H). |
| **367** | 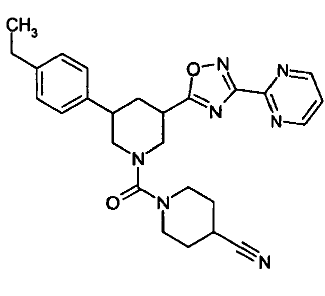 | 70 | m/z = 472; 1.21 min (Methode 2B) | δ = 9.03 (d, 2H), 7.72 (dd, 1H), 7.25 (d, 2H), 7.18 (d, 2H), 4.09 (br d, 1H), 3.58 (br d, 1H), 3.54-3.50 (m, 1H), 3.41-3.37 (m, 2H), 3.19-3.02 (m, 4H), 2.95-2.88 (m, 2H), 2.58 (q, 2H), 2.38 (br d, 1H), 2.06 (q, 1H), 1.90-1.86 (m, 2H), 1.73-1.65 (m, 2H), 1.17 (t, 3H). |
| **368** | 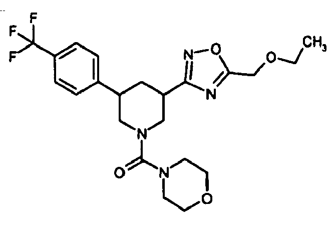 | 46 | m/z = 547; 1.18 min (Methode 2B) | δ = 7.37 (s, 1H), 7.27 (br s, 2H), 4.85 (s, 2H), 4.66 (d, OH), 3.98 (br d, 1H), 3.68-3.60 (m, 1H), 3.55 (br d, 1H), 3.50-3.44 (m, 3H), 3.17 (s, 3H), 3.00 (t, 1H), 3.00-2.88 (m, 4H), 2.34 (br d, 1H), 2.27 (s, 3H), 1.98 (q, 1H), 1.73-1.70 (m, 2H), 1.35-1.24 (m, 2H). |
| **369** | 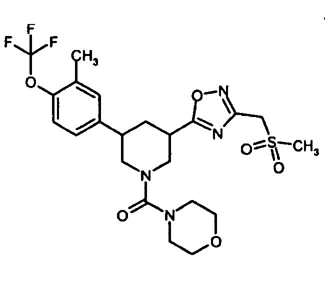 | 72 | m/z = 533; 1.09 min (Methode 9B) | δ = 7.37 (s, 1H), 7.27 (br s, 2H), 4.85 (s, 2H), 4.03 (br d, 1H), 3.63 (br d, 1H), 3.60-3.55 (m, 4H), 3.51-3.44 (m, 1H), 3.21-3.19 (m, 4H), 3.17 (s, 3H), 3.06 (t, 1H), 3.01-2.90 (m, 2H), 2.34 (br d, 1H), 2.27 (s, 3H), 1.99 (q, 1H). |
| **370** | 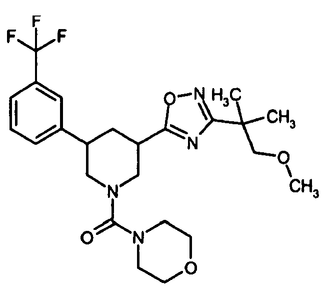 | 87 | m/z = 497; 1.39 min (Methode 2B) | δ = 7.73 (s, 1H), 7.67 (d, 1H), 7.63-7.56 (m, 2H), 3.97 (br d, 1H), 3.61 (br d, 1H), 3.57-3.54 (m, 4H), 3.44 (s, 2H), 3.42-3.34 (m, 1H), 3.22-3.16 (m, 7H), 3.14-3.02 (m, 3H), 2.31 (br d, 1H), 2.03 (q, 1H), 1.27 (s, 6H). |
| **371** | 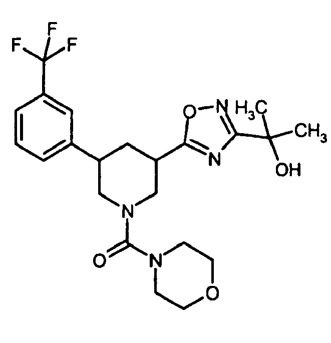 | 40 | m/z = 469; 2.06 min (Methode 5B) | δ = 7.72 (s, 1H), 7.67 (d, 1H), 7.64-7.56 (m, 2H), 5.52 (s, OH), 3.99 (br d, 1H), 3.61 (br d, 1H), 3.60-3.55 (m, 4H), 3.44-3.37 (m, 1H), 3.21-3.16 (m, 4H), 3.12-3.02 (m, 3H), 2.32 (br d, 1H), 2.05 (q, 1H), 1.48 (s, 6H). |
| **372** | 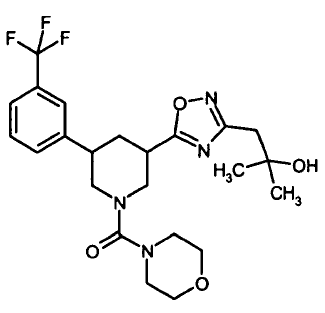 | 59 | m/z = 483; 1.19 min (Methode 2B) | δ = 7.72 (s, 1H), 7.67 (d, 1H), 7.63-7.56 (m, 2H), 4.59 (s, OH), 3.99 (br d, 1H), 3.61 (br d, 1H), 3.58-3.55 (m, 4H), 3.42-3.34 (m, 1H), 3.21-3.19 (m, 4H), 3.12-3.04 (m, 3H), 2.78 (s, 2H), 2.32 (br d, 1H), 2.04 (q, 1H), 1.18 (s, 6H). |
| **373** | 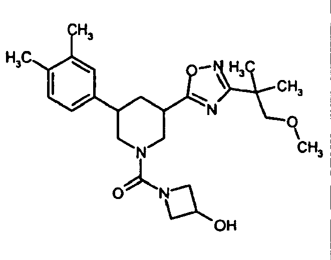 | 45 | m/z = 443; 1.99 min (Methode 3B) | δ = 7.09-7.07 (m, 2H), 7.00 (d, 1H), 5.58 (d, OH), 4.42-4.34 (m, 1H), 4.15 (br d, 1H), 4.08 (q, 2H), 3.71-3.65 (m, 3H), 3.45 (s, 2H), 3.29-3.23 (m, 1H), 3.20 (s, 3H), 3.00-2.89 (m, 2H), 2.77-2.67 (m, 1H), 2.24 (br d, 1H), 2.21 (s, 3H), 2.18 (s, 3H), 1.94 (q, 1H), 1.27 (s, 6H). |
| **374** | 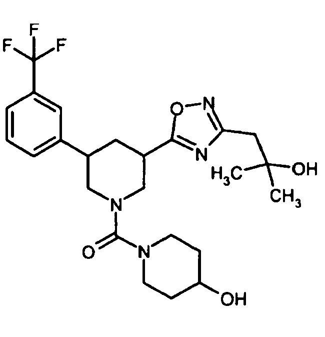 | 26 | m/z = 497; 0.97 min (Methode 9B) | δ = 7.71 (s, 1H), 7.67-7.56 (m, 3H), 4.68 (d, OH), 4.59 (s, OH), 3.95 (br d, 1H), 3.63-3.55 (m, 2H), 3.50-3.47 (m, 2H), 3.42-3.34 (m, 1H), 3.07-3.01 (m, 3H), 2.88 (t, 2H), 2.78 (s, 2H), 2.33 (br d, 1H), 2.03 (q, 1H), 1.73-1.70 (m, 2H), 1.34-1.26 (m, 2H), 1.18 (s, 6H). |
| **375** | 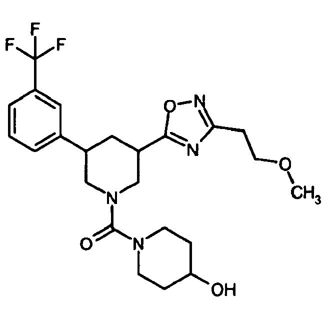 | 37 | m/z = 483; 1.16 min (Methode 2B) | δ = 7.71 (s, 1H), 7.67-7.55 (m, 3H), 4.68 (d, OH), 3.94 (br d, 1H), 3.67 (t, 2H), 3.61-3.55 (m, 1H), 3.50-3.35 (m, 4H), 3.23 (s, 3H), 3.07-3.00 (m, 3H), 2.95-2.85 (m, 4H), 2.32 (br d, 1H), 2.05 (q, 1H), 1.73-1.70 (m, 2H), 1.34-1.27 (m, 2H). |
| **376** | 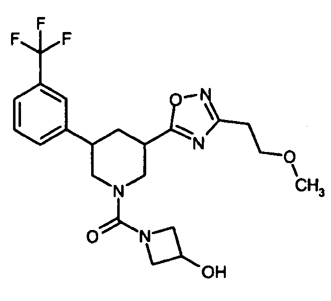 | 15 | m/z = 455; 0.98 min (Methode 9B) | δ = 7.70 (s, 1H), 7.67-7.56 (m, 3H), 5.58 (d, OH), 4.42-4.35 (m, 1H), 4.15 (br d, 1H), 4.12-4.06 (m, 2H), 3.75-3.65 (m, 5H), 3.30-3.24 (m, 1H), 3.23 (s, 3H), 3.05 (t, 2H), 2.98-2.91 (m, 2H), 2.30 (br d, 1H), 2.06 (q, 1H). |
| **377** | 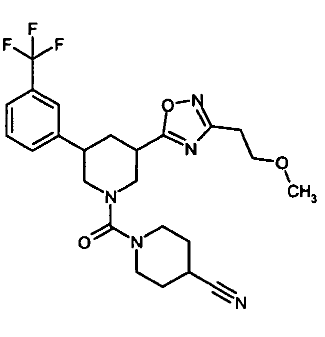 | 50 | m/z = 492; 1.12 min (Methode 9B) | δ = 7.71 (s, 1H), 7.67-7.56 (m, 3H), 3.95 (br d, 1H), 3.67 (t, 2H), 3.57 (br d, 1H), 3.43-3.36 (m, 3H), 3.23 (s, 3H), 3.11-3.01 (m, 6H), 2.91 (t, 2H), 2.33 (br d, 1H), 2.04 (q, 1H), 1.89-1.85 (m, 2H), 1.71-1.65 (m, 2H). |
| **378** | 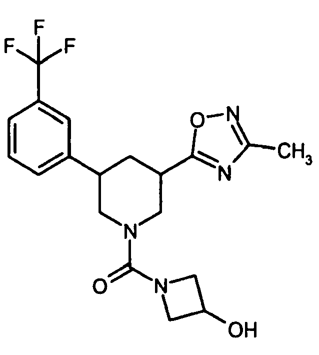 | 19 | m/z = 411; 0.96 min (Methode 9B) | δ = 7.75 (s, 1H), 7.69-7.56 (m, 3H), 5.58 (d, OH), 4.40-4.37 (m, 1H), 4.16-4.06 (m, 3H), 3.76-3.66 (m, 3H), 3.07-2.97 (m, 3H), 2.33-2.28 (m, 4H), 2.06 (q, 1H). |

Die in Tabelle 2 aufgeführten Verbindungen wurden nach der Allgemeinen Methode 5 aus den entsprechenden Edukten hergestellt.

**Tabelle 2:**

| **Bsp. Nr.** | **Struktur** | **Ausbeute (% d. Th.)** | **MS (ESI) [M+H]⁺; LC-MS: Rₜ (Methode)** | **¹H-NMR (400 MHz, DMSO-d₆)** |
|---|---|---|---|---|
| **379** | 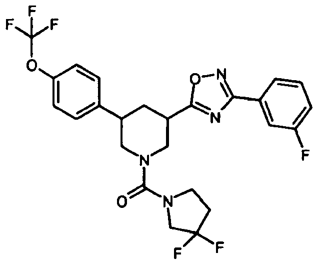 | 48 | m/z = 541; 3.07 min (Methode 1B) | δ = 7.86 (dd, 1H), 7.76 (dd, 1H), 7.66-7.61 (m, 1H), 7.51-7.44 (m, 3H), 7.34 (d, 2H), 4.15 (br d, 1H), 3.81-3.71 (m, 3H), 3.61-3.48 (m, 3H), 3.14 (t, 1H), 3.04-3.01 (m, 2H), 2.42-2.32 (m, 3H), 2.07 (q, 1H). |
| **380** | 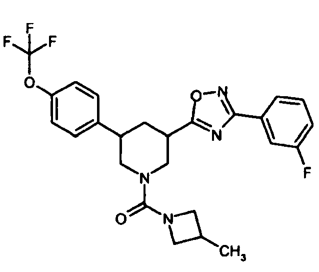 | 37 | m/z = 505; 2.69 min (Methode 3B) | δ = 7.87 (d, 1H), 7.76 (d, 1H), 7.67-7.61 (m, 1H), 7.50-7.45 (m, 3H), 7.34 (d, 2H), 4.26 (br d, 1H), 4.08-4.03 (m, 2H), 3.78 (br d, 1H), 3.53 (q, 2H), 3.48-3.39 (m, 1H), 3.09 (t, 1H), 3.01-2.91 (m, 2H), 2.67-2.56 (m, 1H), 2.37 (br d, 1H), 2.06 (q, 1H), 1.16 (d, 3H). |
| **381** | 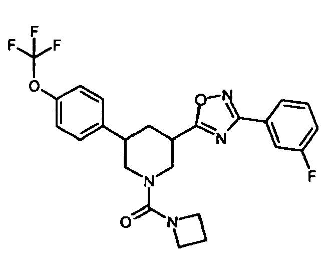 | 39 | m/z = 491; 2.95 min (Methode 1B) | δ = 7.87 (d, 1H), 7.76 (dd, 1H), 7.67-7.61 (m, 1H), 7.49-7.44 (m, 3H), 7.34 (d, 2H), 4.25 (br d, 1H), 3.98-3.93 (m, 4H), 3.77 (br d, 1H), 3.46-3.38 (m, 1H), 3.10 (t, 1H), 3.02-2.91 (m, 2H), 2.38 (br d, 1H), 2.20-2.04 (m, 3H). |
| **382** | 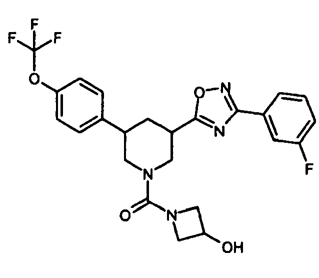 | 47 | m/z = 507; 2.65 min (Methode 1B) | δ = 7.87 (d, 1H), 7.76 (dd, 1H), 7.66-7.61 (m, 1H), 7.50-7.45 (m, 3H), 7.34 (d, 2H), 5.59 (d, OH), 4.39 (quintett, 1H), 4.25 (br d, 1H), 4.11 (q, 2H), 3.78 (br d, 1H), 3.73-3.69 (m, 2H), 3.46-3.38 (m, 1H), 3.11 (t, 1H), 3.02-2.91 (m, 2H), 2.37 (br d, 1H), 2.09 (q, 1H). |
| **383** | 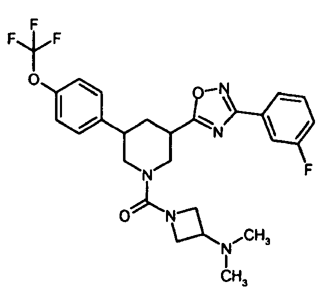 | 55 | m/z = 534; 1.86 min (Methode 1B) | δ = 7.87 (d, 1H), 7.77 (dd, 1H), 7.67-7.61 (m, 1H), 7.50-7.43 (m, 3H), 7.34 (d, 2H), 4.25 (br d, 1H), 3.94 (q, 2H), 3.83-3.72 (m, 3H), 3.47-3.40 (m, 1H), 3.11 (t, 1H), 3.02-2.92 (m, 3H), 2.37 (br d, 1H), 2.12-2.05 (m, 7H). |

Die in Tabelle 3 aufgeführten Verbindungen wurden nach der Allgemeinen Methode 6 aus den entsprechenden Edukten hergestellt.

**Tabelle 3:**

| **Bsp. Nr.** | **Struktur** | **Ausbeute (% d. Th.)** | **MS (ESI) [M+H]⁺; LC-MS: Rₜ (Methode)** | **¹H-NMR (400 MHz, DMSO-d₆)** |
|---|---|---|---|---|
| **384** | 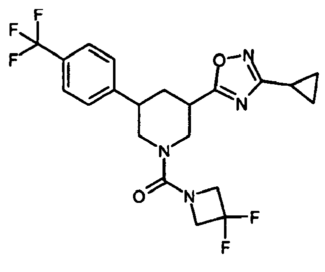 | 45 | m/z = 457; 1.41 min (Methode 2B) | δ = 7.70 (d, 2H), 7.56 (d, 2H), 4.42-4.35 (m, 4H), 4.17 (br d, 1H), 3.79 (br d, 1H), 3.11-3.00 (m, 3H), 2.27 (br d, 1H), 2.14-2.05 (m, 1H), 2.00 (q, 1H), 1.33-1.24 (m, 1H), 1.11-1.03 (m, 2H), 0.91-0.85 (m, 2H). |
| **385** | 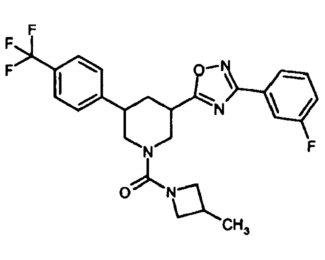 | 33 | m/z = 489; 5.22 min (Methode 1A) | δ = 7.87 (d, 1H), 7.76 (d, 1H), 7.71 (d, 2H), 7.67-7.61 (m, 1H), 7.59 (d, 2H), 7.45 (dd, 1H), 4.26 (br d, 1H), 4.09-4.03 (m, 2H), 3.80 (br d, 1H), 3.53 (q, 2H), 3.47-3.38 (m, 1H), 3.11 (t, 1H), 3.05-2.96 (m, 2H), 2.40 (br d, 1H), 2.14-2.05 (m, 1H), 2.11 (q, 1H), 1.16 (d, 3H). |
| **386** | 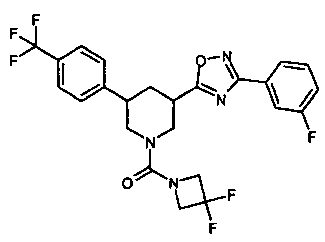 | 30 | m/z =511; 5.19 min (Methode 1A) | δ = 7.87 (d, 1H), 7.78 (d, 1H), 7.73 (d, 2H), 7.67-7.63 (m, 1H), 7.60 (d, 2H), 7.47 (dd, 1H), 4.41 (dt, 4H), 4.29 (br d, 1H), 3.86 (br d, 1H), 3.54-3.47 (m, 1H), 3.21 (t, 1H), 3.13-3.06 (m, 2H), 2.40 (br d, 1H), 2.14 (q, 1H). |
| **387** | 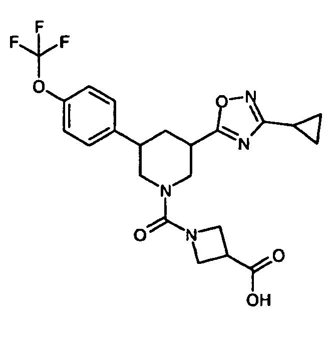 | 57 | m/z = 481; 2.41 min (Methode 1B) | δ = 12.63 (br s, COOH), 7.45 (d, 2H), 7.32 (d, 2H), 4.12-4.06 (m, 3H), 4.01-3.95 (m, 2H), 3.73 (br d, 1H), 3.30-3.23 (m, 1H), 3.02-2.85 (m, 3H), 2.26 (br d, 1H), 2.14-2.07 (m, 1H), 1.94 (q, 1H), 1.08-1.03 (m, 2H), 0.90-0.87 (m, 2H). |
| **388** | 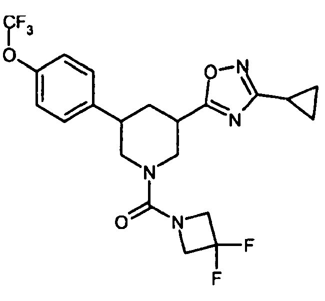 | 26 | m/z = 473; 2.41 min (Methode 3B) | δ = 7.46 (d, 2H), 7.33 (d, 2H), 4.44-4.34 (m, 4H), 4.16 (br d, 1H), 3.77 (br d, 1H), 3.32-3.27 (m, 1H), 3.08-2.90 (m, 3H), 2.25 (br d, 1H), 2.15-2.07 (m, 1H), 1.96 (q, 1H), 1.08-1.03 (m, 2H), 0.91-0.87 (m, 2H). |

Die in Tabelle 4 aufgeführten Verbindungen wurden nach der Allgemeinen Methode 1 aus den entsprechenden Edukten hergestellt.

**Tabelle 4:**

| **Bsp. Nr.** | **Struktur** | **Ausbeute (% d. Th.)** | **MS (ESI) [M+H]⁺; LC-MS: Rₜ (Methode)** | **¹H-NMR (400 MHz, DMSO-d₆)** |
|---|---|---|---|---|
| **389** | 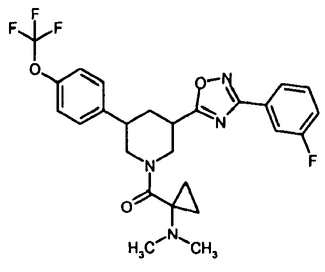 | 68 | m/z = 519; 2.35 min (Methode 3B) | δ = 7.87 (d, 1H), 7.78-7.75 (m, 1H), 7.67-7.62 (m, 1H), 7.51-7.45 (m, 3H), 7.36 (d, 2H), 4.82 (br d, 1H), 4.41 (br d, 1H), 3.36-3.34 (m, 1H), 3.18-3.12 (m, 1H), 3.01-2.98 (m, 2H), 2.44 (br d, 1H), 2.20-2.16 (br s, 5H), 0.95-0.91 (m, 2H), 0.83-0.79 (m, 2H). |
| **390** | 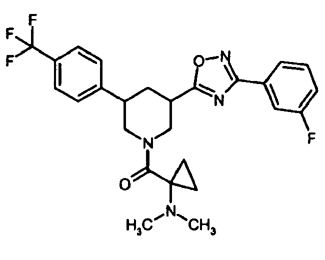 | 89 | m/z = 503; 4.63 min (Methode 1A) | δ = 7.87 (d, 1H), 7.78-7.72 (m, 3H), 7.67-7.59 (m, 3H), 7.50-7.45 (m, 1H), 4.83 (br d, 1H), 4.42 (br d, 1H), 3.39-3.35 (m, 1H), 3.20-3.15 (m, 1H), 3.08-3.02 (m, 2H), 2.44 (br d, 1H), 2.23 (q, 1H), 2.18 (br s, 6H), 0.93-0.89 (m, 2H), 0.84-0.80 (m, 2H). |

### Beispiel 391

### {3-[3-(2-Methoxyethyl)-1,2,4-oxadiazol-5-yl]-5-[4-methyl-3-(trifluormethyl)phenyl]piperidin-1-yl}(morpholin-4-yl)methanon [racemisches cis-Isomer]

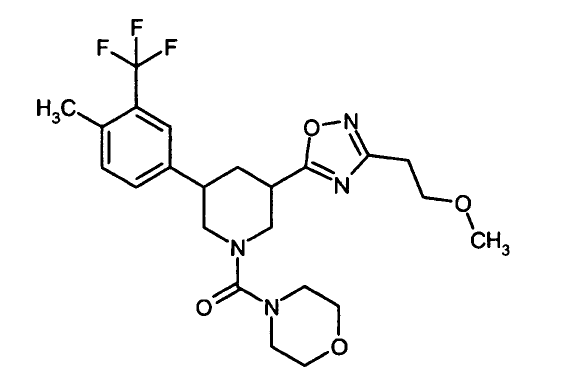

Nach der Allgemeinen Methode 2 wurden 200 mg (0.48 mmol) der Verbindung aus Beispiel 160A und 113 mg (0.71 mmol) *N'*-Hydroxy-3-methoxypropanimidamid umgesetzt. Ausbeute: 104 mg (45% d. Th.)

LC-MS (Methode 5B): Rₜ = 2.27 min; MS (ESIpos): m/z = 483 [M+H]⁺;

¹H-NMR (400 MHz, DMSO-d₆): δ = 7.62 (s, 1H), 7.52 (d, 1H), 7.40 (d, 1H), 3.98 (br d, 1H), 3.67 (t, 2H), 3.60-3.55 (m, 5H), 3.42-3.33 (m, 1H), 3.23 (s, 3H), 3.21-3.16 (m, 4H), 3.10-3.00 (m, 3H), 2.93 (t, 2H), 2.42 (s, 3H), 2.30 (br d, 1H), 2.01 (q, 1H).

### Beispiel 392

### 1-({3-[3-(2-Hydroxypropan-2-yl)-1,2,4-oxadiazol-5-yl]-5-[3-(trifluormethyl)phenyl]piperidin-1-yl}carbonyl)piperidin-4-carbonitril [racemisches cis-Isomer]

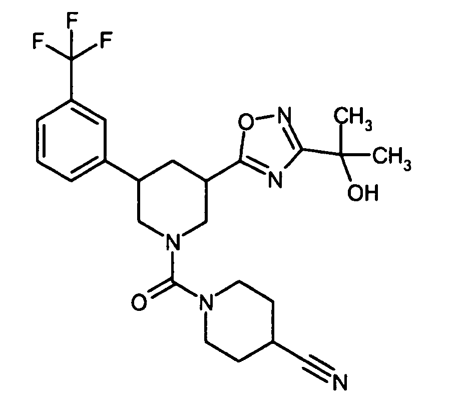

Nach der Allgemeinen Methode 2 wurden 100 mg (0.24 mmol) der Verbindung aus Beispiel 152A und 45 mg (0.37 mmol) der Verbindung aus Beispiel 77A umgesetzt. Ausbeute: 45 mg (35% d. Th.)

LC-MS (Methode 2B): Rₜ = 1.21 min; MS (ESIpos): m/z = 492 [M+H]⁺;

¹H-NMR (400 MHz, DMSO-d₆): δ = 7.72 (s, 1H), 7.67-7.56 (m, 3H), 5.54 (s, OH), 3.96 (br d, 1H), 3.57 (br d, 1H), 3.43-3.36 (m, 3H), 3.12-3.01 (m, 6H), 2.33 (br d, 1H), 2.05 (q, 1H), 1.88-1.85 (m, 2H), 1.71-1.63 (m, 2H), 1.48 (s, 6H).

### Beispiel 393

### {3-(3-Cyclopropyl-1,2,4-oxadiazol-5-yl)-5-[4-(trifluormethoxy)phenyl]piperidin-1-yl} (3-hydroxy-pyrrolidin-1-yl)methanon [Diastereomerengemisch]

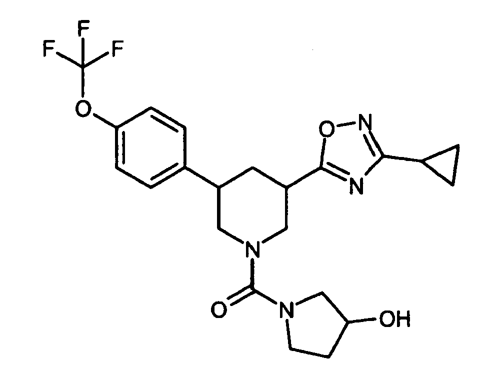

Nach der Allgemeinen Methode 6 wurden 100 mg (0.19 mmol) der Verbindung aus Beispiel 176A und 50 mg (0.58 mmol) 3-Pyrrolidinol umgesetzt. Ausbeute: 50 mg (53% d. Th.)

LC-MS (Methode 1B): Rₜ = 2.34 min; MS (ESIpos): m/z = 467 [M+H]⁺;

¹H-NMR (400 MHz, DMSO-d₆): δ = 7.46 (d, 2H), 7.32 (d, 2H), 4.87 (d, OH), 4.22-4.18 (m, 1H), 4.03-3.99 (m, 1H), 3.67 (br t, 1H), 3.52-3.44 (m, 2H), 3.38-3.34 (m, 1H), 3.10 (br t, 1H), 3.03-2.86 (m, 3H), 2.28 (br d, 1H), 2.14-2.07 (m, 1H), 1.98-1.88 (m, 1H), 1.84-1.80 (m, 1H), 1.74-1.70 (m, 1H), 1.08-1.03 (m, 2H), 0.90-0.86 (m, 2H).

### Beispiel 394

### {3-(4-Ethylphenyl)-5-[3-(2-methoxyethyl)-1,2,4-oxadiazol-5-yl]piperidin-1-yl}(3-hydroxyazetidin-1-yl)methanon [racemisches cis-Isomer]

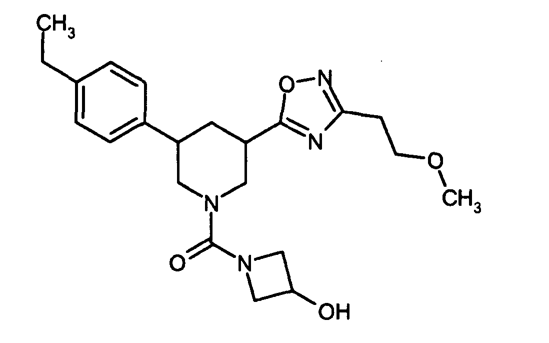

Nach der Allgemeinen Methode 2 wurden 60 mg (0.18 mmol) der Verbindung aus Beispiel 138A und 43 mg (0.27 mmol) *N'-*Hydroxy-3-methoxypropanimidamid umgesetzt. Ausbeute: 25 mg (33% d. Th.)

LC-MS (Methode 1B): Rₜ = 2.08 min; MS (ESIpos): m/z = 415 [M+H]⁺;

¹H-NMR (400 MHz, DMSO-d₆): δ = 7.22 (d, 2H), 7.17 (d, 2H), 5.58 (d, OH), 4.41-4.35 (m, 1H), 4.16 (br d, 1H), 4.08 (q, 2H), 3.72-3.66 (m, 5H), 3.23 (s, 3H), 3.02-2.87 (m, 4H), 2.82-2.76 (m, 1H), 2.57 (q, 2H), 2.27 (br d, 1H), 1.96 (q, 1H), 1.17 (t, 3H).

### Beispiel 395

### (4-Hydroxypiperidin-1-yl){3-{3-[2-(propan-2-yloxy)ethyl]-1,2,4-oxadiazol-5-yl}-5-[4-(trifluormethyl)phenyl]piperidin-1-yl}methanon [racemisches cis-Isomer]

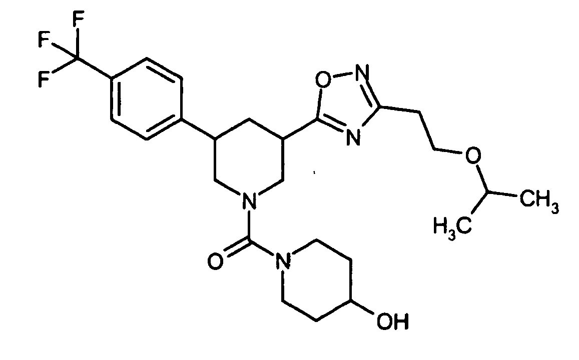

Nach der Allgemeinen Methode 2 wurden 100 mg (0.25 mmol) der Verbindung aus Beispiel 99A und 61 mg (0.38 mmol) der Verbindung aus Beispiel 65A umgesetzt. Ausbeute: 82 mg (61% d. Th.)

HPLC (Methode 2A): Rₜ = 2.29 min; MS (ESIpos): m/z = 511 [M+H]⁺;

¹H-NMR (400 MHz, DMSO-d₆): δ = 7.70 (d, 2H), 7.56 (d, 2H), 4.68 (d, OH), 3.94 (br d, 1H), 3.71 (t, 2H), 3.63-3.38 (m, 6H), 3.07-3.00 (m, 3H), 2.96-2.84 (m, 4H), 2.33 (br d, 1H), 2.00 (q, 1H), 1.73-1.70 (m, 2H), 1.34-1.27 (m, 2H), 1.04 (d, 6H).

### Beispiel 396

### {3-(3-{[1-(Hydroxymethyl)cyclopropyl]methyl -1,2,4-oxadiazol-5-yl)-5-[4-(trifluormethyl)-phenyl]piperidin-1-yl}(4-hydroxypiperidin-1-yl)methanon [racemisches cis-Isomer]

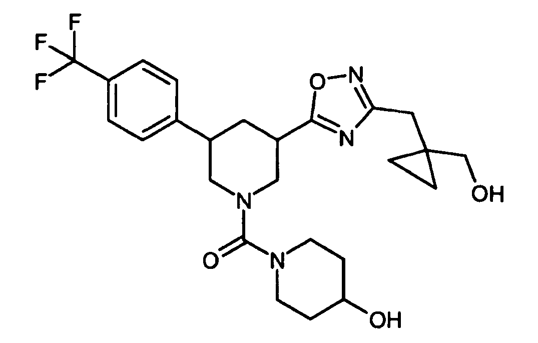

Nach der Allgemeinen Methode 2 wurden 100 mg (0.25 mmol) der Verbindung aus Beispiel 99A und 62 mg (0.38 mmol) der Verbindung aus Beispiel 67A umgesetzt. Ausbeute: 66 mg (52% d. Th.)

HPLC (Methode 2A): Rₜ = 4.19 min; MS (ESIpos): m/z = 509 [M+H]⁺;

¹H-NMR (400 MHz, DMSO-d₆): δ = 7.70 (d, 2H), 7.57 (d, 2H), 4.68 (d, OH), 4.57 (t, OH), 3.95 (br d, 1H), 3.62-3.57 (m, 2H), 3.54-3.37 (m, 3H), 3.26 (d, 2H), 3.07-2.99 (m, 3H), 2.91 (br t, 2H), 2.78 (s, 2H), 2.35 (br d, 1H), 2.00 (q, 1H), 1.73-1.70 (m, 2H), 1.34-1.24 (m, 2H), 0.45-0.41 (m, 4H).

### Beispiel 397

### {3-{3-[(Methylsulfonyl)methyl]-1,2,4-oxadiazol-5-yl}-5-[4-methyl-3-(trifluormethyl)phenyl]-piperidin-1-yl}(morpholin-4-yl)methanon [racemisches cis-Isomer]

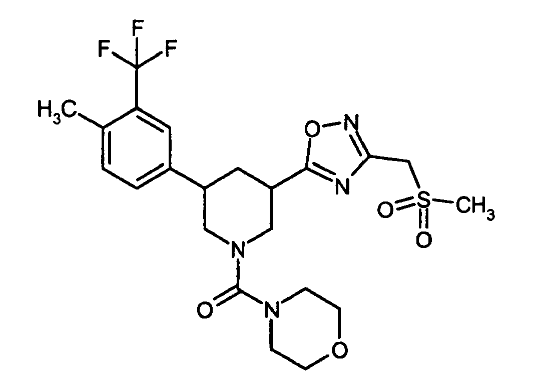

Nach der Allgemeinen Methode 2 wurden 100 mg (0.23 mmol) der Verbindung aus Beispiel 160A und 53 mg (0.35 mmol) *N'*-Hydroxy-2-(methylsulfonyl)ethanimidamid umgesetzt. Ausbeute: 65 mg (54% d. Th.)

LC-MS (Methode 5B): Rₜ = 2.16 min; MS (ESIpos): m/z = 517 [M+H]⁺;

¹H-NMR (400 MHz, DMSO-d₆): δ = 7.60 (s, 1H), 7.53 (d, 1H), 7.40 (d, 1H), 4.85 (s, 2H), 4.02 (br d, 1H), 3.60-3.55 (m, 5H), 3.49-3.44 (m, 1H), 3.21-3.19 (m, 4H), 3.17 (s, 3H), 3.10 (t, 1H), 3.04-3.01 (m, 2H), 2.42 (s, 3H), 2.34 (br d, 1H), 2.03 (q, 1H).

### Beispiel 398

### (4-Hydroxypiperidin-1-yl){3-{3-[(methylsulfonyl)methyl]-1,2,4-oxadiazol-5-yl}-5-[4-methyl-3-(trifluormethyl)phenyl]piperidin-1-yl}methanon [racemisches cis-Isomer]

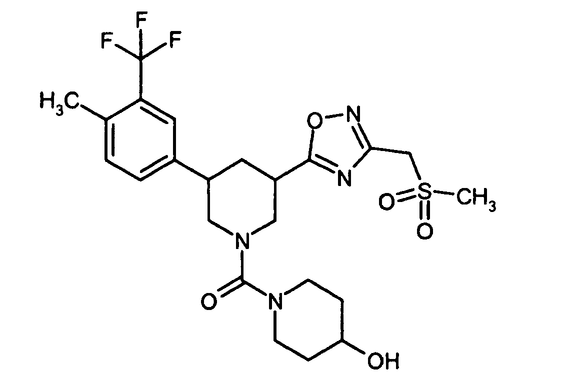

Nach der Allgemeinen Methode 2 wurden 100 mg (0.24 mmol) der Verbindung aus Beispiel 163A und 55 mg (0.36 mmol) *N'*-Hydroxy-2-(methylsulfonyl)ethanimidamid umgesetzt. Ausbeute: 72 mg (56% d. Th.)

LC-MS (Methode 2B): Rₜ = 1.11 min; MS (ESIpos): m/z = 531 [M+H]⁺;

¹H-NMR (400 MHz, DMSO-d₆): δ = 7.61 (s, 1H), 7.52 (d, 1H), 7.40 (d, 1H), 4.85 (s, 2H), 4.66 (d, OH), 3.97 (br d, 1H), 3.64-3.61 (m, 1H), 3.57 (br d, 1H), 3.58-3.46 (m, 3H), 3.17 (s, 3H), 3.09-3.00 (m, 3H), 2,89 (br t, 2H), 2.41 (s, 3H), 2.32 (br d, 1H), 2.02 (q, 1H), 1.73-1.70 (m, 2H), 1.34-1.27 (m, 2H).

### Beispiel 399

### {3-[3-(2-Methoxyethyl)-1,2,4-oxadiazol-5-yl]-5-[3-methyl-4-(trifluormethoxy)phenyl]piperidin-1-yl}(morpholin-4-yl)methanon [racemisches cis-Isomer]

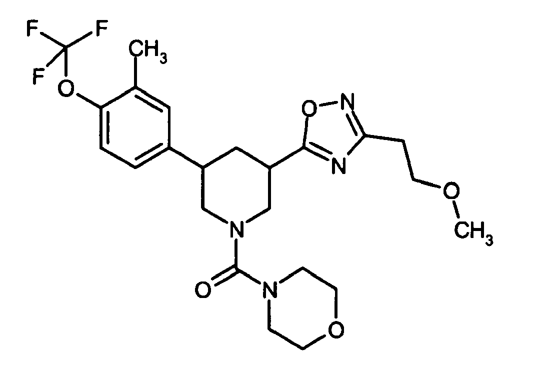

Nach der Allgemeinen Methode 2 wurden 100 mg (0.240 mmol) der Verbindung aus Beispiel 167A und 57 mg (0.360 mmol) *N'*-Hydroxy-3-methoxypropanimidamid umgesetzt. Ausbeute: 58 mg (48% d. Th.).

LC-MS (Methode 2B): Rₜ = 1.33 min; MS (ESIpos): m/z = 499 [M+H]⁺;

¹H-NMR (400 MHz, DMSO-d₆): δ = 7.37 (s, 1H), 7.27 (s, 2H), 3.98 (br d, 1H), 3.67 (t, 2H), 3.60 (br d, 1H), 3.58-3.53 (m, 4H), 3.39 (tt, 1H), 3.23 (s, 3H), 3.22-3.17 (m, 4H), 3.05-2.89 (m, 5H), 2.30 (br d, 1H), 2.27 (s, 3H), 1.97 (dd, 1H).

### Beispiel 400

### {3-[3-(3-Fluorphenyl)-1,2,4-oxadiazol-5-yl]-5-[3-methyl-4-trifluormethoxy)phenyl]piperidin-1-yl}(morpholin-4-yl)methanon [racemisches cis-Isomer]

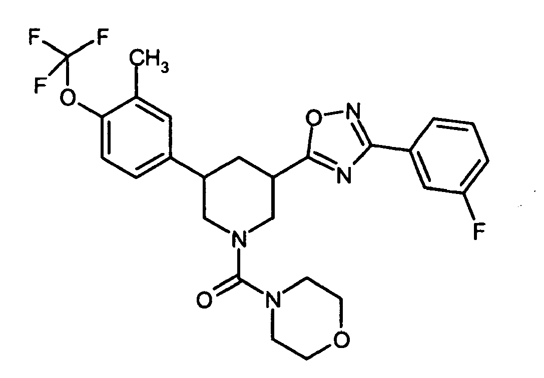

Nach der Allgemeinen Methode 2 wurden 100 mg (0.240 mmol) der Verbindung aus Beispiel 167A und 55 mg (0.360 mmol) 3-Fluor-*N'*-hydroxybenzolcarboximidamid umgesetzt. Ausbeute: 95 mg (74% d. Th.).

LC-MS (Methode 1B): Rₜ = 3.00 min; MS (ESIpos): m/z = 535 [M+H]⁺;

¹H-NMR (400 MHz, DMSO-d₆): δ = 7.87 (br d, 1H), 7.51 (d, 1H), 7.63 (dd, 1H), 7.46 (dd, 1H), 7.39 (s, 1H), 7.29 (s, 2H), 4.08 (br d, 1H), 3.63 (br d, 1H), 3.61-3.54 (m, 4H), 3.51 (tt, 1H), 3.25-3.18 (m, 4H), 3.15-2.93 (m, 3H), 2.38 (br d, 1H), 2.28 (s, 3H), 2.06 (dd, 1H).

### Beispiel 401

### {3-(3-Cyclopropyl-1,2,4-oxadiazol-5-yl)-5-[3-methyl-4-(trifluormethoxy)phenyl]piperidin-1-yl}-(morpholin-4-yl)methanon [racemisches cis-Isomer]

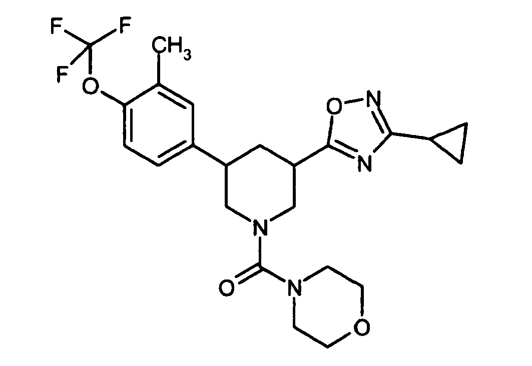

Nach der Allgemeinen Methode 2 wurden 100 mg (0.24 mmol) der Verbindung aus Beispiel 167A und 36 mg (0.36 mmol) *N'*-Hydroxycyclopropancarboximidamid umgesetzt. Ausbeute: 72 mg (62% d. Th.).

LC-MS (Methode 2B): Rₜ = 1.43 min; MS (ESIpos): m/z = 481 [M+H]⁺;

¹H-NMR (400 MHz, DMSO-d₆): δ = 7.36 (s, 1H), 7.26 (s, 2H), 3.99 (br d, 1H), 3.58 (br d, 1H), 3.58-3.52 (m, 4H), 3.22-3.15 (m, 4H), 3.01-2.87 (m, 3H), 2.27 (s, 3H), 2.25 (d, 1H), 2.14-2.07 (m, 1H), 1.92 (dd, 1H), 1.08-1.03 (m, 2H), 0.90-0.86 (m, 2H).

### Beispiel 402

### 3-[3-(Propan-2-yl)-1,2,4-oxadiazol-5-yl]-5-[4-(trifluormethyl)phenyl]piperidin-1-yl}methanon [racemisches cis-Isomer]

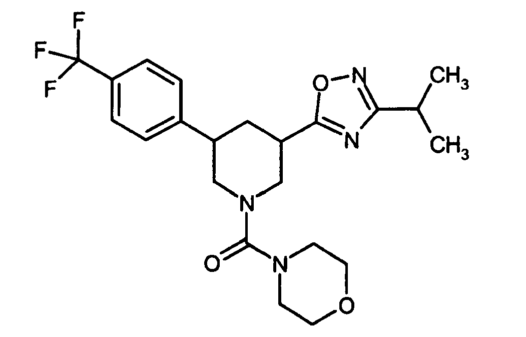

Nach der Allgemeinen Methode 2 wurden 200 mg (0.518 mmol) der Verbindung aus Beispiel 49A und 79 mg (0.78 mmol) *N'*-Hydroxy-2-methylpropanimidamid umgesetzt. Ausbeute: 82 mg (35% d.Th.).

LC-MS (Methode 1B): Rₜ = 2.53 min; MS (ESIpos): m/z = 453 [M+H]⁺;

¹H-NMR (400 MHz, DMSO-d₆): δ = 7.70 (d, 2H), 7.57 (d, 2H), 3.99 (br d, 1H), 3.62 (d, 1H), 3.60-3.55 (m, 4H), 3.41-3.37 (1H), 3.24-3.18 (m, 4H), 3.10-3.01 (4H), 2.34 (br d, 1H), 2.03 (q, 1H), 1.26 (s, 3H), 1.25 (s, 3H).

### Beispiel 403

### {3-[3-Methyl-4-(trifluormethoxy)phenyl]-5-[3-(propan-2-yl)-1,2,4-oxadiazol-5-yl]piperidin-1-yl}-(morpholin-4-yl)methanon [racemisches cis-Isomer]

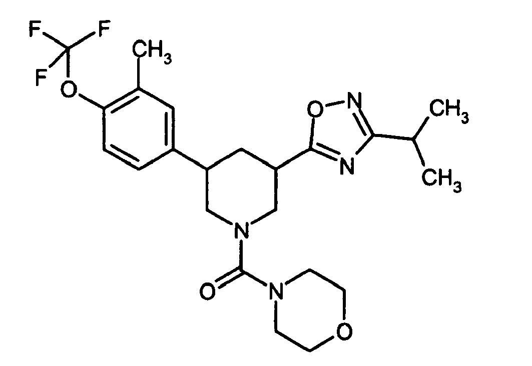

Nach der Allgemeinen Methode 1 wurden 500 mg (1.15 mmol) der Verbindung aus Beispiel 167A und 130 mg (1.27 mmol) *N'*-Hydroxy-2-methylpropanimidamid umgesetzt. Ausbeute: 286 mg (51% d. Th.)

LC-MS (Methode 9B): Rₜ = 1.29 min; MS (ESIpos): m/z = 482 [M+H]⁺.

### Beispiel 404

### (4-Hydroxypiperidin-1-yl){3-[3-methyl-4-(trifluormethoxy)phenylJ-5-[3-(propan-2-yl)-1,2,4-oxadiazol-5-yl]piperidin-1-yl}methanon [racemisches cis-Isomer]

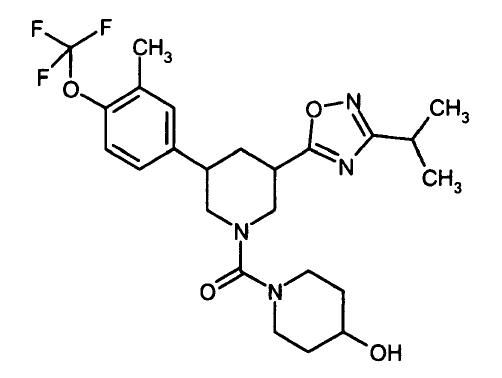

Nach der Allgemeinen Methode 1 wurden 500 mg (1.12 mmol) der Verbindung aus Beispiel 169A und 125 mg (1.23 mmol) *N'*-Hydroxy-2-methylpropanimidamid umgesetzt. Ausbeute: 373 mg (67% d. Th.).

LC-MS (Methode 2B): Rₜ = 1.39 min; MS (ESIpos): m/z = 496 [M+H]⁺;

### Beispiel 405

### {3-(4-Ethylphenyl)-5-[3-(2-methoxyethyl)-1,2,4-oxadiazol-5-yl]piperidin-1-yl} (morpholin-4-yl)methanon [enantiomerenreines cis-Isomer]

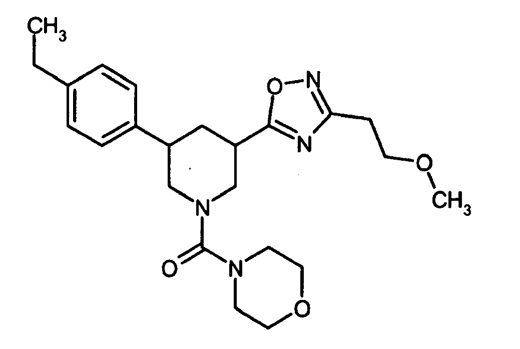

Die Enantiomerentrennung von 315 mg (0.7 mmol) der Verbindung aus Beispiel 150 nach Methode 14D ergab 198 mg der Titelverbindung aus Beispiel 405 (Enantiomer 1) und 216 mg der Titelverbindung aus Beispiel 406 (Enantiomer 2).

HPLC (Methode 11E): Rₜ = 5.17 min, >98.5% ee; LC-MS (Methode 9B): Rₜ = 1.11 min; MS (ESIpos): m/z = 429 [M+H]⁺;

¹H-NMR (400 MHz, DMSO-d₆): δ = 7.22 (d, 2H), 7.17 (d, 1H), 3.99 (br d, 1H), 3.68 (t, 2H), 3.58 (br d, 1H), 3.57-3.55 (m, 4H), 3.42-3.35 (m, 1H), 3.23 (s, 3H), 3.20-3.18 (m, 4H), 3.05-3.01 (m, 1H), 2.98-2.82 (m, 4H), 2.57 (q, 2H), 2.29 (br d, 1H) 1.94 (q, 1H), 1.16 (t, 3H).

[α]₃₆₅²⁰ = -31.9, Methanol

### Beispiel 406

### {3-(4-Ethylphenyl)-5-[3-(2-methoxyethyl)-1,2,4-oxadiazol-5-yl]piperidin-1-yl}(morpholin-4-yl)methanon [enantiomerenreines cis-Isomer]

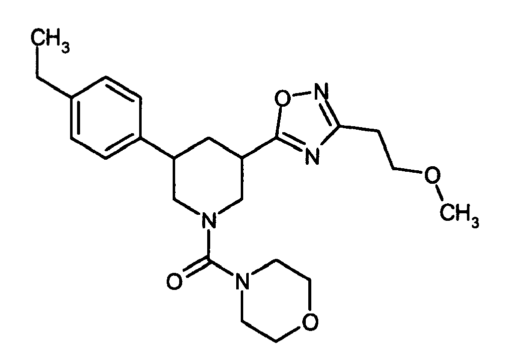

Die Enantiomerentrennung von 315 mg (0.7 mmol) der Verbindung aus Beispiel 150 nach Methode 14D ergab 198 mg der Titelverbindung aus Beispiel 405 (Enantiomer 1) und 216 mg der Titelverbindung aus Beispiel 406 (Enantiomer 2).

HPLC (Methode 11E): Rₜ = 7.39 min, >98.5% ee; LC-MS (Methode 9B): Rₜ = 1.11 min; MS (ESIpos): m/z = 429 [M+H]⁺;

¹H-NMR (400 MHz, DMSO-d₆): δ = 7.22 (d, 2H), 7.17 (d, 1H), 3.98 (br d, 1H), 3.67 (t, 2H), 3.58 (br d, 1H), 3.56-3.52 (m, 4H), 3.42-3.36 (m, 1H), 3.23 (s, 3H), 3.20-3.18 (m, 4H), 3.04-3.00 (m, 1H), 2.98-2.82 (m, 4H), 2.57 (q, 2H), 2.30 (br d, 1H) 1.94 (q, 1H), 1.16 (t, 3H).

[α]₃₆₅²⁰ = +24.0, Methanol

### Beispiel 407

### {3-(3,4-Dimethylphenyl)-5-[3-(2-methoxyethyl)-1,2,4-oxadiazol-5-yl]piperidin-1-yl}(4-hydroxypiperidin-1-yl)methanon [enantiomerenreines cis-Isomer]

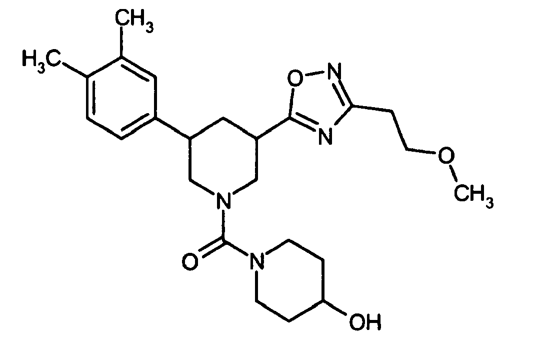

Die Enantiomerentrennung von 138 mg des Racemates Beispiel 186 nach Methode 6D ergab 44 mg der Titelverbindung aus Beispiel 407 und 25 mg der Titelverbindung aus Beispiel 408.

HPLC (Methode 5E): Rₜ = 4.67 min, >99.5% ee; LC-MS (Methode 2B): Rₜ = 1.15 min; MS (ESIpos): m/z = 443[M+H]⁺.

### Beispiel 408

### {3-(3,4-Dimethylphenyl)-5-[3-(2-methoxyethyl)-1,2,4-oxadiazol-5-yl]piperidin-1-yl}(4-hydroxypiperidin-1-yl)methanon [enantiomerenreines cis-Isomer]

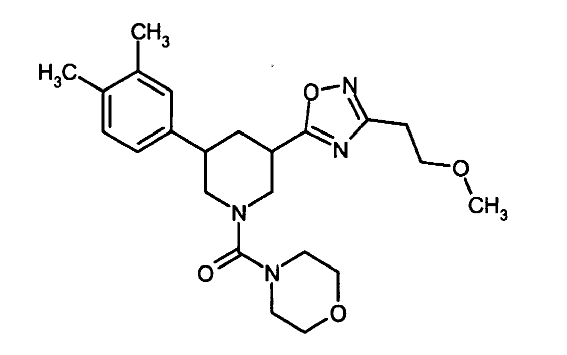

Die Enantiomerentrennung von 138 mg des Racemates Beispiel 186 nach Methode 6D ergab 44 mg der Titelverbindung aus Beispiel 407 und 25 mg der Titelverbindung aus Beispiel 408.

HPLC (Methode 5E): Rₜ = 6.27 min, >99.5% ee; LC-MS (Methode 2B): Rₜ = 1.15 min; MS (ESIpos): m/z = 443[M+H]⁺.

### Beispiel 409

### {3-(3,4-Dimethylphenyl)-5-[3-(2-methoxyethyl)-1,2,4-oxadiazol-5-yl]piperidin-1-yl}(3-hydroxyazetidin-1-yl)methanon [enantiomerenreines cis-Isomer]

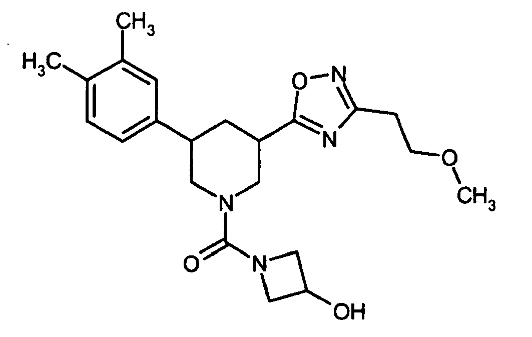

Die Enantiomerentrennung von 99 mg des Racemates Beispiel 216 nach Methode 19D ergab 28 mg der Titelverbindung aus Beispiel 409 (Enantiomer 1) und 28 mg der Titelverbindung aus Beispiel 410 (Enantiomer 2).

HPLC (Methode 15E): Rₜ = 7.99 min, >99.5% ee; LC-MS (Methode 2B): Rₜ = 1.09 min; MS (ESIpos): m/z = 415[M+H]⁺.

### Beispiel 410

### {3-(3,4-Dimethylphenyl)-5-[3-(2-methoxyethyl)-1,2,4-oxadiazol-5-yl]piperidin-1-yl}(3-hydroxyazetidin-1-yl)methanon [enantiomerenreines cis-Isomer]

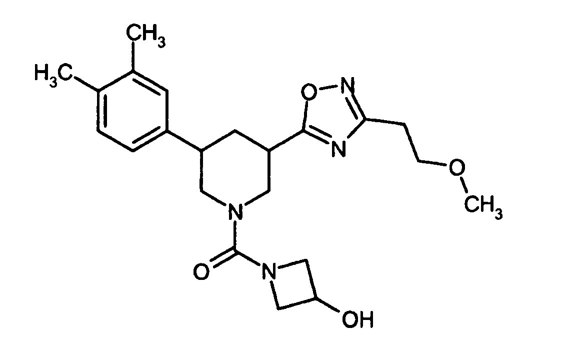

Die Enantiomerentrennung von 99 mg des Racemates Beispiel 216 nach Methode 19D ergab 28 mg der Titelverbindung aus Beispiel 409 und 28 mg der Titelverbindung aus Beispiel 410.

HPLC (Methode 15E): Rₜ = 15.89 min, >99.5% ee; LC-MS (Methode 2B): Rₜ = 1.09 min; MS (ESIpos): m/z = 415[M+H]⁺.

### Beispiel 411

### {3-[3-(3-Fluophenyl)-1,2,4-oxadiazol-5-yl]-5-[4-methyl-3-(trifluormethyl)phenyl]piperidin-1-yl}-(morpholin-4-yl)methanon [racemisches cis-Isomer]

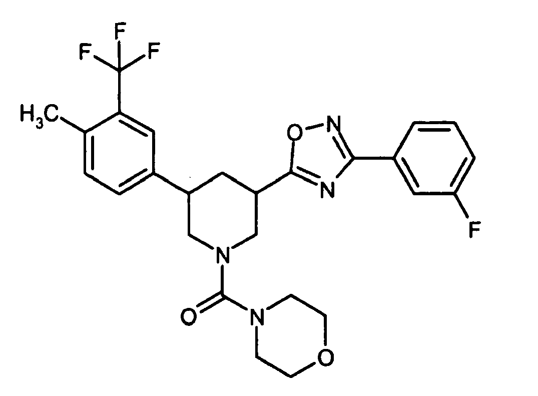

Nach der Allgemeinen Methode 2 wurden 100 mg (ca. 0.24 mmol) der Verbindung aus Beispiel 160A und 55 mg (0.36 mmol) 3-Fluor-*N*'-hydroxybenzolcarboximidamid umgesetzt. Ausbeute: 67 mg (54% d. Th.).

LC-MS (Methode 2B): Rₜ = 1.54 min; MS (ESIpos): m/z = 519 [M+H]⁺;

¹H-NMR (400 MHz, DMSO-d₆): δ = 7.87 (d, 1H), 7.75 (d, 1H), 7.64 (dd, 2H), 7.55 (d, 1H), 7.46 (dd, 1H), 7.41 (d, 1H), 4.07 (br d, 1H), 3.59-3.55 (m, 4H), 3.50 (tt, 1H); 3.23-3.18 (m, 4H), 3.16-3.00 (m, 3H), 2.41 (s, 3H), 2.38 (br d, 1H), 2.09 (dd, 1H).

### Beispiel 412

### 1-({3-[3-(3-Fluorphenyl)-1,2,4-oxadiazol-5-yl]-5-[3-(trifluormethyl)phenyl]piperidin-1-yl}-carbonyl)piperidin-4-carbonitril [racemisches cis-Isomer]

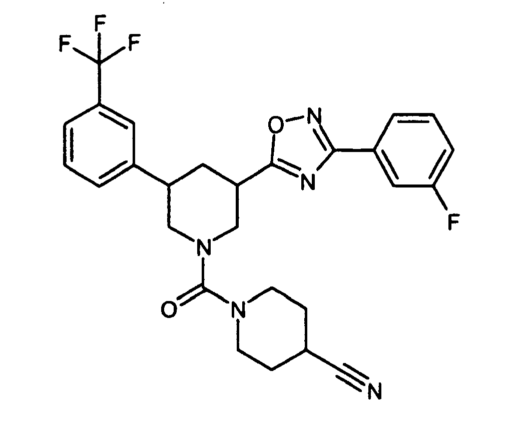

Nach der Allgemeinen Methode 2 wurden 100 mg (0.24 mmol) der Verbindung aus Beispiel 152A und 56 mg (0.37 mmol) 3-Fluor-N-hydroxybenzolcarboximidamid umgesetzt. Ausbeute: 87 mg (68% d. Th.).

LC-MS (Methode 5B): Rₜ = 2.70 min; MS (ESIpos): m/z = 528 [M+H]⁺;

¹H-NMR (400 MHz, DMSO-d₆): δ = 7.87 (d, 1H), 7.74 (d, 2H), 7.70-7.57 (m, 4H), 7.45 (dd, 1H), 4.05 (br d, 1H), 3.60 (d, 1H), 3.51 (tt, 1H); 3.39 (br d, 1H), 3.19-3.03 (m, 6H), 2.40 (br d, 1H), 2.13 (dd, 1H), 1.92-1.83 (m, 2H), 1.75-1.63 (m, 2H).

### Beispiel 413

### {3-[3-(3-Fluorphenyl)-1,2,4-oxadiazol-5-yl]-5-[4-methyl-3-(trifluormethyl)phenyl]piperidin-1-yl}-(4-hydroxypiperidin-1-yl)methanon [racemisches cis-Isomer]

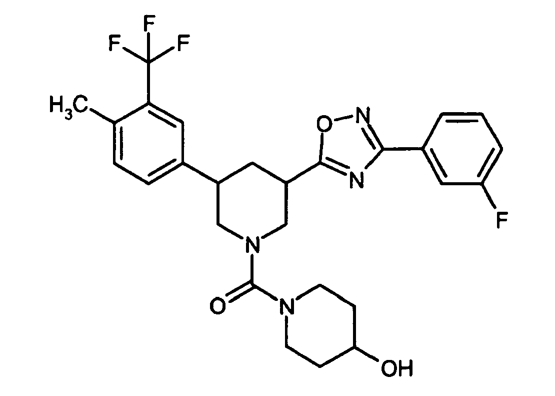

Nach der Allgemeinen Methode 2 wurden 100 mg (0.24 mmol) der Verbindung aus Beispiel 163A und 55 mg (0.36 mmol) 3-Fluor-N-hydroxybenzolcarboximidamid umgesetzt. Ausbeute: 71 mg (55% d. Th.).

LC-MS (Methode 9B): Rₜ = 1.29 min; MS (ESIpos): m/z = 533 [M+H]⁺;

¹H-NMR (400 MHz, DMSO-d₆): δ = 7.86 (d, 1H), 7.75 (d, 1H), 7.67-7.61 (m, 2H), 7.54 (d, 1H), 7.46 (dd, 1H), 7.41 (d, 1H), 4.69 (d, 1H), 4.03 (br d, 1H), 3.65-3.60 (m, 1H), 3.56 (d, 1H), 3.52-3.46 (m, 3H), 3.13 (t, 1H), 3.02 (dd, 2H), 2.92 (dd, 2H), 2.42 (s, 3H), 2.39 (br d, 1H), 2.08 (dd, 1H), 1.74 (br d, 2H), 1.37-1.27 (m, 2H).

### Beispiel 414

### {3-(3-Cyclopropyl-1,2,4-oxadiazol-5-yl)-5-[4-methyl-3-(trifluormethyl)phenyl]piperidin-1-yl}-(morpholin-4-yl)methanon [racemisches cis-Isomer]

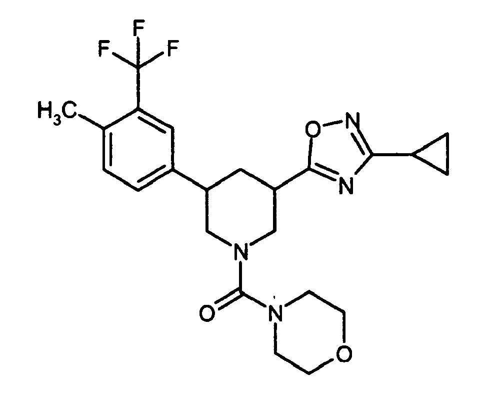

Nach der Allgemeinen Methode 2 wurden 100 mg (0.25 mmol) der Verbindung aus Beispiel 160A und 37 mg (0.38 mmol) *N*-Hydroxycyclopropancarboximidamid umgesetzt. Ausbeute: 43 mg (37% d. Th.).

LC-MS (Methode 2B): Rₜ = 1.39 min; MS (ESIpos): m/z = 465 [M+H]⁺;

¹H-NMR (400 MHz, DMSO-d₆): δ = 7.61 (s, 1H), 7.51 (d, 1H), 7.40 (d, 1H), 3.94 (br d, 1H), 3.57 (d, 1H), 3.57-3.53 (m, 4H), 3.21-3.16 (m, 4H), 3.06-2.94 (m, 3H), 2.41 (s, 3H); 2.25 (br d, 1H), 2.14-2.07 (m, 1H), 1.95 (dd, 1H), 1.08-1.03 (m, 2H), 0.90-0.86 (m, 2H).

### Beispiels 415

### {3-(3-Cyclopropyl-1,2,4-oxadiazol-5-yl)-5-[4-methyl-3-(trifluormethyl)phenyl]piperidin-1-yl}(4-hydroxypiperidin-1-yl)methanon [racemisches cis-Isomer]

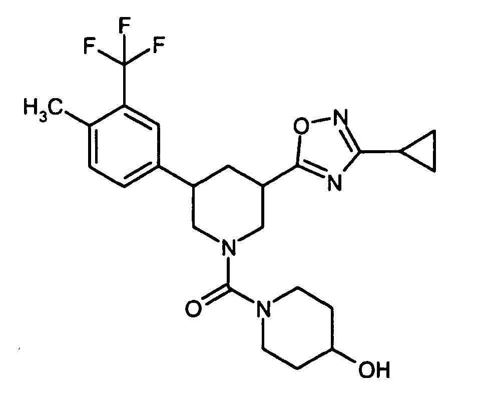

Nach der Allgemeinen Methode 2 wurden 100 mg (0.24 mmol) der Verbindung aus Beispiel 163A und 36 mg (0.36 mmol) *N*-Hydroxycyclopropancarboximidamid umgesetzt. Ausbeute: 60 mg (51% d. Th.).

LC-MS (Methode 9B): Rₜ = 1.15 min; MS (ESIpos): m/z = 479 [M+H]⁺;

¹H-NMR (400 MHz, DMSO-d₆): δ = 7.60 (s, 1H), 7.51 (d, 1H), 7.39 (d, 1H), 4.68 (d, 1H), 3.89 (br d, 1H), 3.64-3.57 (m, 2H), 3.53-3.45 (m, 3H), 3.02-2.96 (m, 3H), 2.89 (dd, 2H), 2.43-2.39 (s, 3H), 2.25 (d, 1H), 2.14-2.07 (m, 1H), 1.95 (dd, 1H), 1.70 (br d, 1H), 1.29 (dd, 2H), 1.07-1.03 (m, 2H), 0.90-0.86 (m, 2H).

### Beispiel 416

### (3-(3-Methyl-1,2,4-oxadiazol-5-yl)-5-[4-(trifluormethyl) phenyl]piperidin-1-yl}(morpholin-4-yl)methanon [racemisches cis-Isomer]

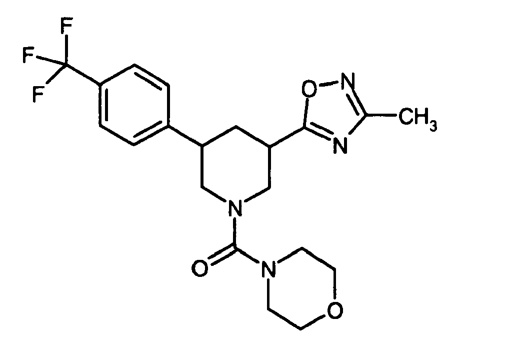

Nach der Allgemeinen Methode 2 wurden 200 mg (0.518 mmol) der Verbindung aus 81A und 57 mg (0.78 mmol) *N'*-Hydroxyethanimidamid umgesetzt. Ausbeute: 94 mg (41% d. Th.).

LC-MS (Methode 1B): Rₜ = 2.26 min; MS (ESIpos): m/z = 425 [M+H]⁺;

¹H-NMR (400 MHz, DMSO-d₆): δ = 7.70 (d, 2H), 7.57 (d, 2H), 3.98 (br d, 1H), 3.63 (d, 1H), 3.62-3.52 (m, 4H), 3.39 (t, 1H), 3.23-3.18 (m, 4H), 3.08-2.96 (m, 3H), 2.35 (s, 3H), 2.02 (q, 1H).

### Beispiel 417

### (4-Hydroxypiperidin-1-yl) {3-[3-(2-methoxyethyl)-1,2,4-oxadiazol-5-yl]-5-[4-methyl-3-(trifluormethyl)phenyl]piperidin-1-yl}methanon [racemisches cis-Isomer]

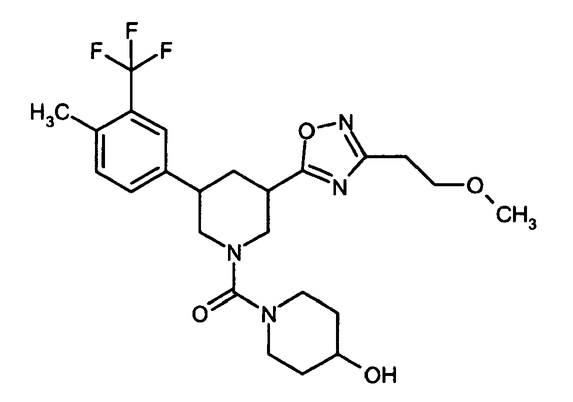

Nach der Allgemeinen Methode 2 wurden 200 mg (0.48 mmol) der Verbindung aus Beispiel 163A und 114 mg (0.72 mmol) *N*-Hydroxy-3-methoxypropanimidamid (Beispiel 64A) umgesetzt. Ausbeute: 105 mg (44% d. Th.).

LC-MS (Methode 9B): Rₜ = 1.07 min; MS (ESIpos): m/z = 497 [M+H]⁺;

¹H-NMR (400 MHz, DMSO-d₆): δ = 7.61 (s, 1H), 7.52 (d, 1H), 7.40 (d, 1H), 4.68 (d, 1H), 3.92 (br d, 1H), 3.67 (t, 2H), 3.64-3.56 (m, 1H), 3.53 (br d, 1H), 3.51-3.43 (m, 2H), 3.38 (tt, 1H), 3.23 (s, 3H), 3.06-2.98 (m, 3H), 2.93 (t, 2H), 2.88 (br d, 1H), 2.41 (s, 3H), 2.30 (br d, 1H), 2.00 (dd, 1H), 1.71 (br d, 2H), 1.35-1.24 (m, 2H).

### Beispiel 418

### (3-Hydroxypyrrolidin-1-yl){3-[3-(propan-2-yl)-1,2,4-oxadiazol-5-yl]-5-[4-(trifluormethoxy)-phenyl]piperidin-1-yl}methanon [Diastereomerengemisch]

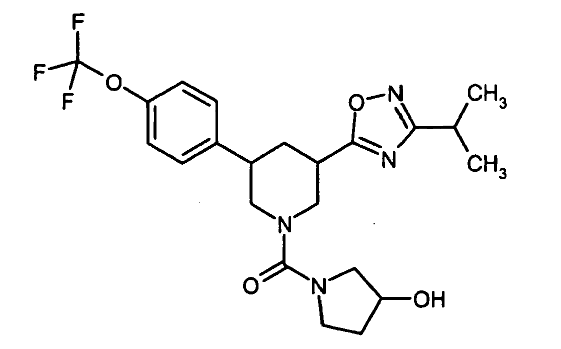

Nach der Allgemeinen Methode 2 wurden 200 mg (0.50 mmol) der Verbindung aus Beispiel 112A und 76 mg (0.75 mmol) *N*'-Hydroxy-2-methylpropanimidamid umgesetzt. Ausbeute: 125 mg (54% d. Th.).

LC-MS (Methode 2B): Rₜ = 1.28 min; MS (ESIpos): m/z = 469 [M+H]⁺;

¹H-NMR (400 MHz, DMSO-d₆): δ = 7.47 (d, 2H), 7.32 (d, 2H), 4.88 (d, 1H), 4.25-4.18 (m, 1H), 4.09-4.00 (m, 1H), 3.72-3.64 (m, 1H), 3.52-3.37 (m, 3H), 3.15-2.84 (m, 5H), 2.35-2.28 (m, 1H), 1.96 (dd, 1H), 1.84-1.78 (m, 1H), 1.75-1.68 (m, 1H), 1.25 (d, 6H).

### Beispiel 419

### {3-(3-Cyclopropyl-1,2,4-oxadiazol-5-yl)-5-[4-(trifluormethoxy)phenyl]piperidin-1-yl} (3-hydroxy-pyrrolidin-1-yl)methanon [Diastereomerengemisch]

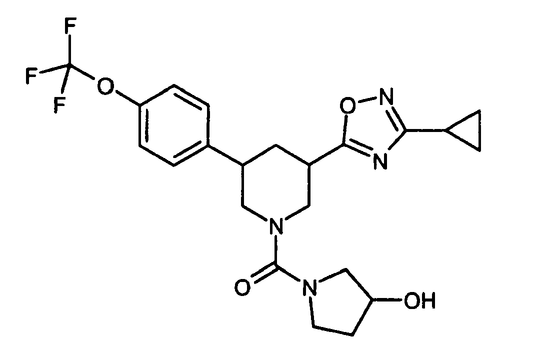

Nach der Allgemeinen Methode 2 wurden 200 mg (0.50 mmol) der Verbindung aus Beispiel 112A und 75 mg (0.75 mmol) *N*'-Hydroxycyclopropancarboximidamid umgesetzt. Ausbeute: 127 mg (55% d. Th.).

LC-MS (Methode 2B): Rₜ = 1.24 min; MS (ESIpos): m/z = 467 [M+H]⁺;

¹H-NMR (400 MHz, DMSO-d₆): δ = 7.46 (d, 2H), 7.32 (d, 2H), 4.87 (d, 1H), 4.24-4.18 (m, 1H), 4.06-3.96 (m, 1H), 3.73-3.63 (m, 1H), 3.52-3.42 (m, 4H, 3.14-3.05 (m, 1H), 3.00-2.83 (m, 3H), 2.35-2.25 (m, 1H), 2.16-2.07 (m, 1H),1.93 (dd, 1H), 1.87-1.77 (m, 1H), 1.75-1.65 (m, 1H), 1.08-1.02 (m, 2H), 0.92-0.85 (m, 2H).

### Beispiel 420

### (3-Hydroxypyrrolidin-1-yl){3-[3-(2-methoxyethyl)-1,2,4-oxadiazol-5-yl]-5-[4-(trifluormethoxy)-phenyl]piperidin-1-yl}methanon [Diastereomerengemisch]

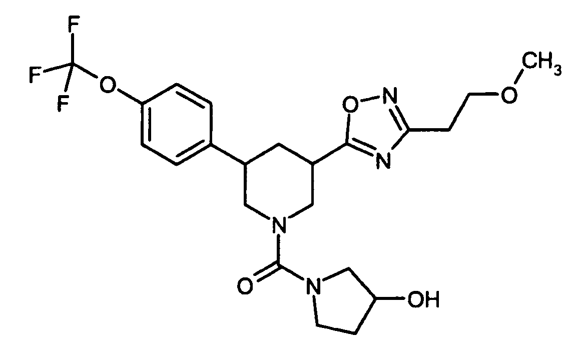

Nach der Allgemeinen Methode 2 wurden 200 mg (0.50 mmol) der Verbindung aus Beispiel 112A und 88 mg (0.75 mmol) N-Hydroxy-3-methoxypropanimidamid umgesetzt. Ausbeute: 67 mg (27% d. Th.).

LC-MS (Methode 3B): Rₜ = 1.81 min; MS (ESIpos): m/z = 485 [M+H]⁺;

¹H-NMR (400 MHz, DMSO-d₆): δ = 7.47 (d, 2H), 7.32 (d, 2H), 4.88 (d, 1H), 4.25-4.18 (m, 1H), 4.09-4.00 (m, 1H), 3.73-3.65 (m, 3H), 3.52-3.40 (m, 3H), 3.23 (s, 3H), 3.10 (dd, 1H), 3.03-2.84 (m, 5H), 2.36-2.29 (m, 1H), 1.98 (dd, 1H), 1.87-1.77 (m, 1H), 1.76-1.68 (m, 1H).

### Beispiel 421

### {3-(3-Ethyl-1,2,4-oxadiazol-5-yl)-5-[4-(trifluormethoxy)phenyl]piperidin-1-yl}(3-hydroxy-pyrrolidin-1-yl)methanon [Diastereomerengemisch]

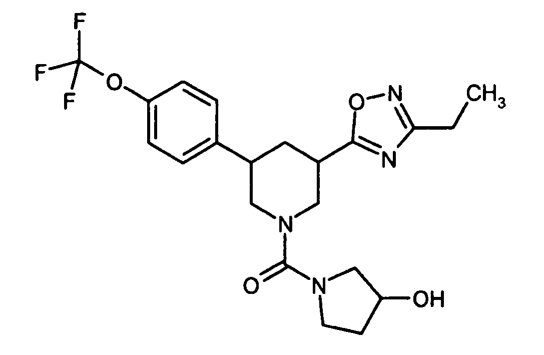

Nach der Allgemeinen Methode 2 wurden 200 mg (0.50 mmol) der Verbindung aus Beispiel 112A und 67 mg (0.75 mmol) *N*-Hydroxypropanimidamid umgesetzt. Ausbeute: 17 mg (7% d. Th.).

LC-MS (Methode 3B): Rₜ = 1.92 min; MS (ESIpos): m/z = 455 [M+H]⁺;

¹H-NMR (400 MHz, DMSO-d₆): δ = 7.46 (d, 2H), 7.32 (d, 2H), 4.88 (d, 1H), 4.25-4.18 (m, 1H), 4.09-4.00 (m, 1H, 3.69 (dd, 1H), 3.52-3.41 (m, 3H), 3.10 (dd, 1H), 3.04-2.86 (m, 3H), 2.74-2.67 (m, 3H), 2.37-2.28 (m, 1H), 1.97 (dd, 1H), 1.86-1.77 (m, 1H), 1.76-1.68 (m, 1H), 1.22 (t, 3H).

### Beispiel 422

### (3-Hydroxypyrrolidin-1-yl){3-(3-methyl-1,2,4-oxadiazol-5-yl)-5-[4-(trifluormethoxy)phenyl]-piperidin-1-yl}methanon [Diastereomerengemisch]

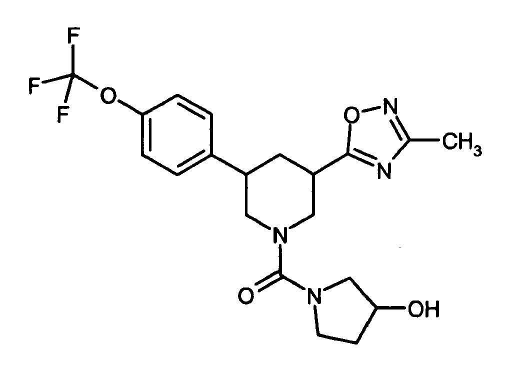

Nach der Allgemeinen Methode 2 wurden 200 mg (0.50 mmol) der Verbindung aus Beispiel 112A und 55 mg (0.75 mmol) *N*'-Hydroxyethanimidamid umgesetzt. Ausbeute: 15 mg (6% d. Th.).

LC-MS (Methode 2B): Rₜ = 1.14 min; MS (ESIpos): m/z = 441 [M+H]⁺;

¹H-NMR (400 MHz, DMSO-d₆): δ = 7.46 (d, 2H), 7.33 (d, 2H), 4.88 (d, 1H), 4.25-4.18 (m, 1H), 4.09-4.00 (m, 1H), 3.69 (dd, 1H), 3.52-3.37 (m, 3H), 3.10 (dd, 1H), 3.03-2.82 (m, 4H), 2.32 (s, 3H), 2.30 (br d, 1H), 1.97 (dd, 1H), 1.87-1.77 (m, 1H), 1.77-1.68 (m, 1H).

### Beispiel 423

### 1-({3-[5-(2-Methoxyethyl)-1,3,4-oxadiazol-2-yl]-5-[4-(trifluormethoxy)phenyl]piperidin-1-yl}-carbonyl)piperidin-4-carbonitril [racemisches cis-Isomer]

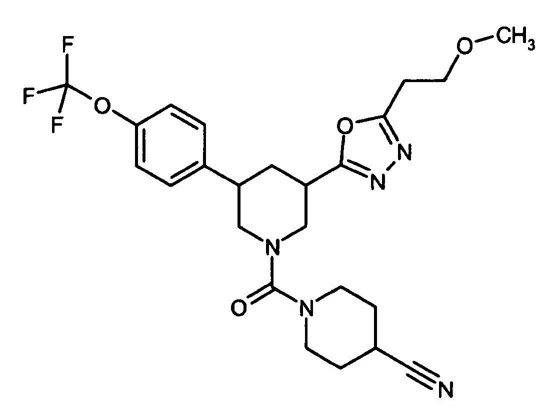

Nach der Allgemeinen Methode 4 wurden 100 mg (0.15 mmol) der Verbindung aus Beispiel 108A und 36 mg (0.31 mmol) 3-Methoxypropanhydrazid umgesetzt. Ausbeute: 5 mg (7% d. Th.).

LC-MS (Methode 2B): Rₜ = 1.20 min; MS (ESIpos): m/z = 508 [M+H]⁺;

¹H-NMR (400 MHz, DMSO-d₆): δ = 7.46 (d, 2H), 7.33 (d, 2H), 3.95 (br d, 1H), 3.68 (t, 2H), 3.57 (br d, 1H), 3.24 (s, 3H), 3.12-2.96 (m, 7H), 2.28 (br d, 1H), 1.93 (q, 1H), 1.90-1.82 (m, 2H), 1.73-1.62 (m, 2H).

### Beispiel 424

### [3-(4-Ethylphenyl)-5-(5-methyl-1,3-oxazol-2-yl)piperidin-1-yl](morpholin-4-yl)methanon [racemisches cis-Isomer]

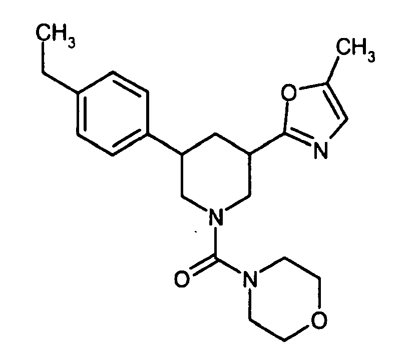

50 mg (0.13 mmol) der Verbindung aus Beispiel 199A wurden in 0.5 ml Acetonitril vorgelegt und mit 2 mg (0.01 mmol) Goldtrichlorid versetzt. Das Reaktionsgemisch wurde 16 h bei 50°C gerührt, eingeengt und mittels präparativer HPLC aufgereinigt. Ausbeute: 10 mg (15% d. Th.).

HPLC (Methode 2A): Rₜ = 1.28 min; MS (ESIpos): m/z = 384 [M+H]⁺;

¹H-NMR (400 MHz, DMSO-d₆): δ = 7.22 (d, 2H), 7.16 (d, 2H), 6.74 (s, 1H), 3.95 (br d, 1H), 3.60 (br d, 1H), 3.58-3.55 (m, 4H), 3.23-3.17 (m, 4H), 3.11-3.07 (m, 1H), 2.98-2.79 (m, 3H), 2.58 (q, 2H), 2.26 (s, 3H), 2.25 (br d, 1H), 1.89 (q, 1H), 1.16 (t, 3H).

### Beispiel 425

### {3-[5-(Furan-2-yl)-1,2,4-oxadiazol-3-yl]-5-[4-(trifluormethyl)phenyl]piperidin-1-yl}(morpholin-4-yl)methanon

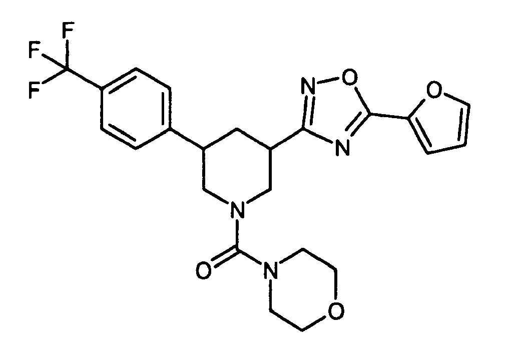

Die Titelverbindung wurde nach der Allgemeinen Methode 8 hergestellt. Ausbeute: 0.48 mg (12% d. Th.)

LC-MS (Methode 10B): Rₜ = 2.25 min; MS (ESIpos): m/z = 477 [M+H]⁺.

Nach der Allgemeinen Methode 8 wurden die in Tabelle 5 aufgeführten Verbindungen aus 0.1 mmol der Verbindung aus Beispiel 84A und der entsprechenden Carbonsäure hergestellt.

**Tabelle 5:**

| **Beispiel Nr.** | **Struktur** | **Ausbeute (% d. Th.)** | **LC-MS:Rₜ (Methode 10B)** |
|---|---|---|---|
| **426** | 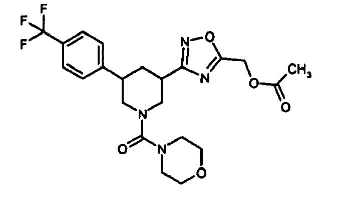 | 3 | m/z = 482; 2.10 min |
| **427** | 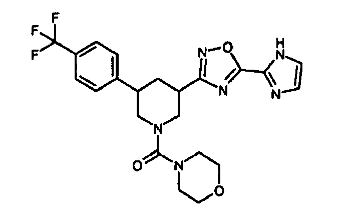 | 5 | m/z = 476; 1.91 min |
| **428** | 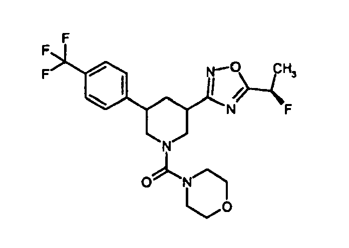 | 9 | m/z = 456; 2.19 min |
| **429** | 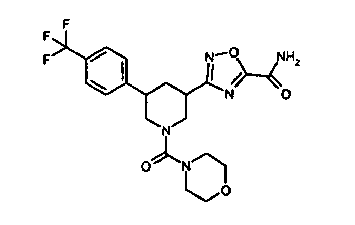 | 2 | m/z = 453; 1.90 min |
| **430** | 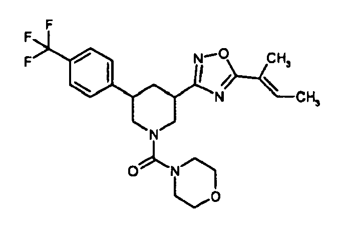 | 15 | m/z = 464; 2.36 min |
| **431** | 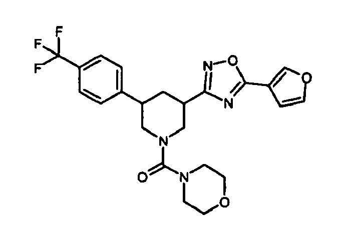 | 15 | m/z = 476; 2.25 min |
| **432** | 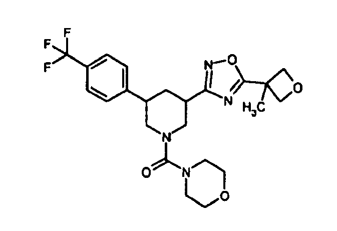 | 40 | m/z = 480; 2.12 min |
| **433** | 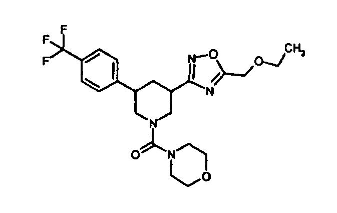 | 8 | m/z = 468; 2.17 min |
| **434** | 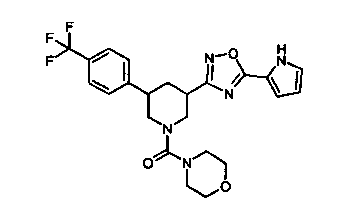 | 6 | m/z=475; 2.19 min |
| **435** | 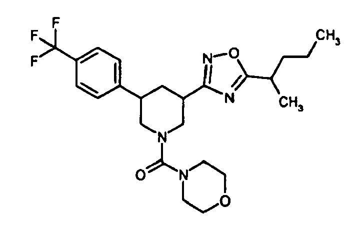 | 18 | m/z = 480; 2.41 min |
| **436** | 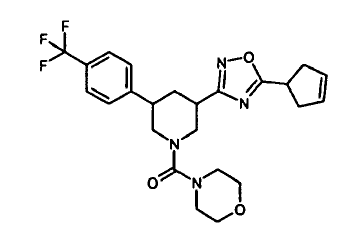 | 47 | m/z = 476; 2.32 min |
| **437** | 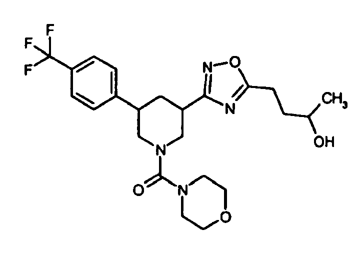 | 11 | m/z = 468; 2.01 min |
| **438** | 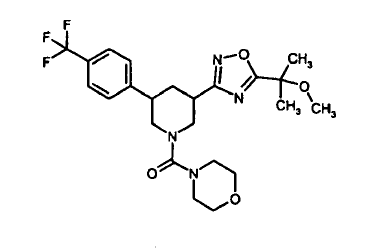 | 19 | m/z = 482; 2.25 min |
| **439** | 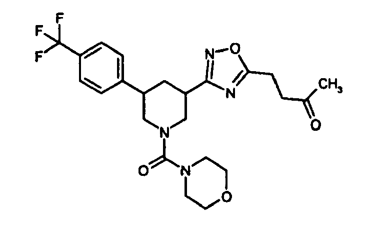 | 15 | m/z = 480; 2.04 min |
| **440** | 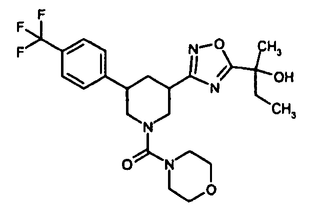 | 18 | m/z = 182; 2.04 min |

### Beispiel 441

### {3-(3-Cyclopropyl-1,2,4-oxadiazol-5-yl)-5-[4-(trifluormethoxy)phenyl]piperidin-1-yl}(4-hydroxypiperidin-1-yl)methanon [racemisches cis-Isomer]

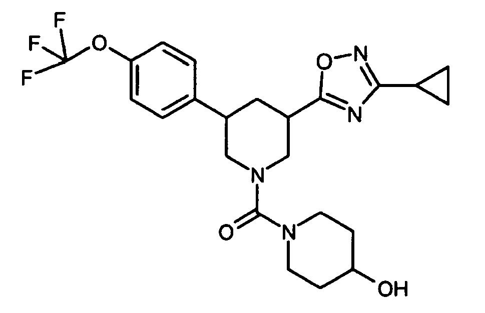

Nach der Allgemeinen Methode 2 wurden 200 mg (0.480 mmol) der Verbindung aus Beispiel 63A und 96 mg (0.961 mmol) *N*-Hydroxycyclopropancarboximidamid umgesetzt. Ausbeute: 130 mg (56% d. Th.).

LC-MS (Methode 2B): Rₜ = 1.30 min; MS (ESIpos): m/z = 481 [M+H]⁺;

¹H-NMR (400 MHz, DMSO-d₆): δ = 7.45 (d, 2H), 7.32 (d, 2H), 4.66 (d, 1H), 3.91 (br d, 1H), 3.64-3.57 (m, 1H), 3.54 (d, 1H), 3.50-3.42 (m, 2H), 2.99-2.86 (m, 5H), 2.27 (br d, 1H), 2.14-2.07 (m, 1H), 1.92 (dd, 1H), 1.71 (br d, 1H), 1.33-1.24 (m, 2H), 1.07-1.03 (m, 2H), 0.90-0.86 (m, 2H).

### Beispiel 442

### {3-(3-Cyclopropyl-1,2,4-oxadiazol-5-yl)-5-[4-(trifluormethoxy)phenyl]piperidin-1-yl}(4-hydroxypiperidin-1-yl)methanon [enantiomerenreines cis-Isomer]

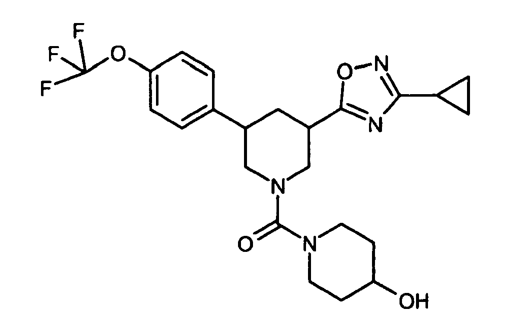

Die Enantiomerentrennung von 130 mg des Racemates aus Beispiel 441 nach Methode 11D ergab 48 mg der Titelverbindung aus Beispiel 442 und 48 mg der Titelverbindung aus Beispiel 443.

HPLC (Methode 9E): Rₜ = 4.14 min, >99.0% ee; LC-MS (Methode 2B): Rₜ = 1.26 min; MS (ESIpos): m/z = 481 [M+H]⁺.

[α]₃₆₅²⁰ = +2.4, c= 0.63, Methanol

### Beispiel 443

### {3-(3-Cyclopropyl-1,2,4-oxadiazol-5-yl)-5-[4-(trifluormethoxy)phenyl)piperidin-1-yl}(4-hydroxypiperidin-1-yl)methanon [enantiomerenreines cis-Isomer]

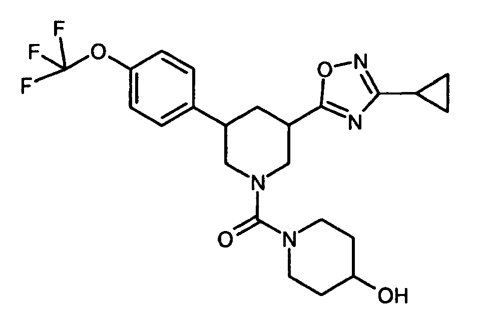

Die Enantiomerentrennung von 130 mg des Racemates aus Beispiel 441 nach Methode 11D ergab 48 mg der Titelverbindung aus Beispiel 442 und 48 mg der Titelverbindung aus Beispiel 443.

HPLC (Methode 9E): Rₜ = 6.35 min, >99.0% ee; LC-MS (Methode 2B): Rₜ = 1.26 min; MS (ESIpos): m/z = 481 [M+H]⁺.

[α]₃₆₅²⁰= +0.3, c= 0.61, Methanol

### Beispiel 444

### Ethyl-5-{1-(morpholin-4-ylcarbonyl)-5-[4-(trifluormethyl) phenyl]piperidin-3-yl}-1,2,4-oxadiazol-3-carboxylat [racemisches cis-Isomer]

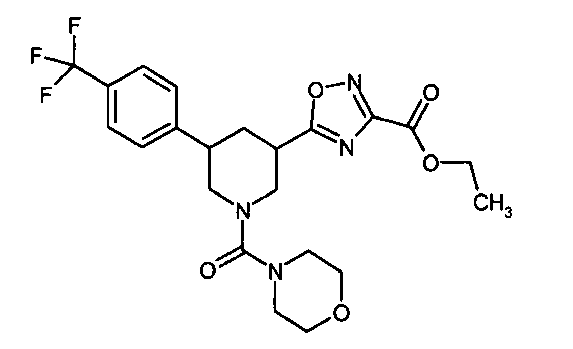

Nach der Allgemeinen Methode 1 wurden 200 mg (ca. 0.47 mmol) der Verbindung aus Beispiel 49A und 54 mg (0.41 mmol) Ethyl-amino(hydroximin)ethanoat umgesetzt. Ausbeute: 17 mg (8% d. Th.).

LC-MS (Methode 2B): Rₜ = 1.30 min; MS (ESIpos): m/z = 483 [M+H]⁺;

¹H-NMR (400 MHz, DMSO-d₆): δ = 7.71 (d, 2H), 7.58 (d, 2H), 4.41 (q, 2H), 4.03 (br d, 1H), 3.63 (d, 1H), 3.60-3.51 (m, 5H), 3.22-3.10 (m, 4H), 3.15-3.00 (m, 3H), 2.36 (d, 1H), 2.07 (q, 1H), 1.32 (t, 3H).

### Beispiel 445

### {3-[3-(3-Fluorphenyl)-1,2,4-oxadiazol-5-yl]-5-[4-(trifluormethoxy)phenyl]piperidin-1-yl}(4-hydroxypiperidin-1-yl)methanon [enantiomerenreines cis-Isomer]

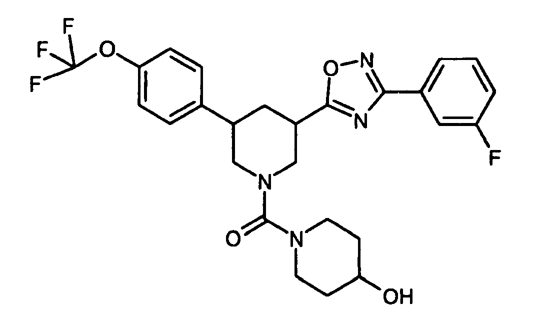

Enantiomerentrennung von 130 mg des Racemates aus Beispiel 444 nach Methode 12D ergab 31 mg der Titelverbindung aus Beispiel 445 und 32 mg der Titelverbindung aus Beispiel 446.

HPLC (Methode 9E): Rₜ = 5.25 min, >99.0% ee; LC-MS (Methode 2B): Rₜ = 1.41 min; MS (ESIpos): m/z = 534 [M+H]⁺.

[α]₃₆₅²⁰ = +3.0, c=0.62, Methanol

### Beispiel 446

### {3-[3-(3-Fluorphenyl)-1,2,4-oxadiazol-5-yl]-5-[4-(trifluormethoxy)phenyl]piperidin-1-yl}(4-hydroxypiperidin-1-yl)methanon [enantiomerenreines cis-Isomer]

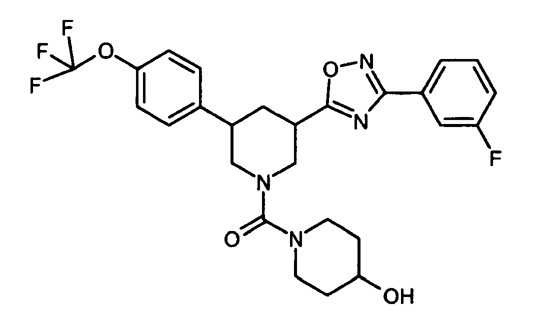

Enantiomerentrennung von 130 mg des Racemates aus Beispiel 444 nach Methode 12D ergab 31 mg der Titelverbindung aus Beispiel 445 und 32 mg der Titelverbindung aus Beispiel 446.

HPLC (Methode 9E): Rₜ = 9.33 min, >99.0% ee; LC-MS (Methode 2B): Rₜ = 1.41 min; MS (ESIpos): m/z = 534 [M+H]⁺.

[a]₃₆₅ ²⁰= -3.0, c=0.61, Methanol

### Beispiel 447

### (4-Hydroxypiperidin-1-yl) {3-[3-(2-methoxyethyl)-1,2,4-oxadiazol-5-yl]-5-[4-(trifluormethoxy)-phenyl]piperidin-1-yl)methanon [racemisches cis-Isomer]

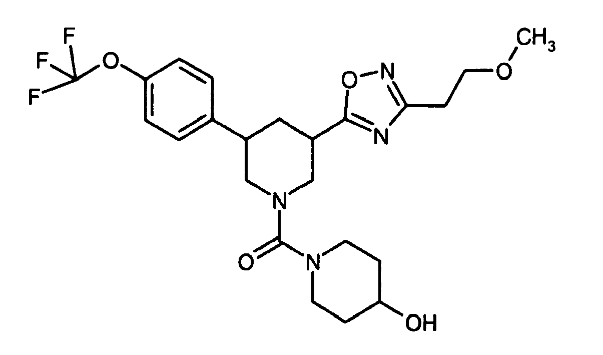

Nach der Allgemeinen Methode 2 wurden 200 mg (ca. 0.432 mmol) der Verbindung aus Beispiel 63A und 102 mg (0.865 mmol) *N'*-Hydroxy-3-methoxypropanimidamid umgesetzt. Ausbeute: 92 mg (36% d. Th.)

LC-MS (Methode 2B): Rₜ = 1.20 min; MS (ESIpos): m/z = 499 [M+H]⁺;

¹H-NMR (400 MHz, DMSO-d₆): δ = 7.46 (d, 2H), 7.33 (d, 2H), 4.68 (d, 1H), 3.94 (br d, 1H), 3.67 (t, 2H), 3.63-3.57 (m, 1H), 3.55 (d, 1H), 3.51-3.35 (m, 3H), 3.23 (s, 3H), 3.03-2.90 (m, 7H), 2.31 (br d, 1H), 1.96 (dd, 1H), 1.71 (br d,2H), 1.35-1.26 (m, 2H).

### Beispiel 448

### (4-Hydroxypiperidin-1-yl){3-[3-(2-methoxyethyl)-1,2,4-oxadiazol-5-yl]-5-[4-(trifluormethoxy)-phenyl]piperidin-1-yl}methanon [enantiomerenreines cis-Isomer]

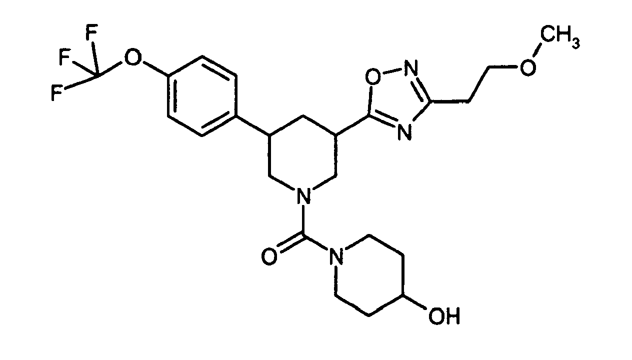

Enantiomerentrennung von 91 mg des Racemates aus Beispiel 447 nach Methode 12D ergab 33 mg der Titelverbindung aus Beispiel 448 und 33 mg der Titelverbindung aus Beispiel 449.

HPLC (Methode 9E): Rₜ = 5.11 min, >99.0% ee; LC-MS (Methode 9B ): Rₜ = 1.04 min; MS (ESIpos): m/z = 499 [M+H]⁺.

¹H-NMR (400 MHz, DMSO-d₆): δ = 7.48 (d, 2H), 7.33 (d, 2H), 4.68 (d, 1H), 3.94 (br d, 1H), 3.67 (t, 2H), 3.61-3.37 (m, 5H), 3.23 (s, 3H), 3.03-2.88 (m, 7H), 2.32 (br d, 1H), 1.96 (dd, 1H), 1.73 (br d, 2H), 1.37-1.25 (m, 2H).

### Beispiel 449

### (4-Hydroxypiperidin-1-yl) {3-[3-(2-methoxyethyl)-1,2,4-oxadiazol-5-yl]-5-[4-(trifluormethoxy)-phenyl]piperidin-1-yl}methanon [enantiomerenreines cis-Isomer]

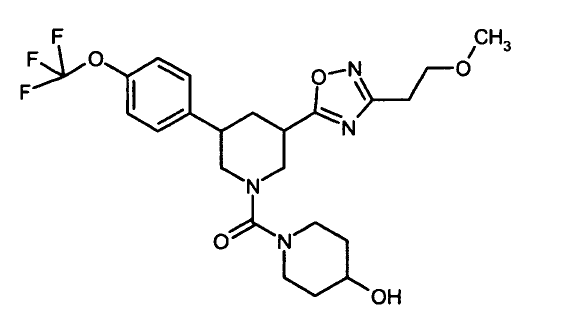

Enantiomerentrennung von 91 mg des Racemates aus Beispiel 447 nach Methode 12D ergab 33 mg der Titelverbindung aus Beispiel 448 und 33 mg der Titelverbindung aus Beispiel 449.

HPLC (Methode 9E): Rₜ = 11.94 min, >99.0% ee; LC-MS (Methode 9B): Rₜ = 1.04 min; MS (ESIpos): m/z = 499 [M+H]⁺.

¹H-NMR (400 MHz, DMSO-d₆): δ = 7.48 (d, 2H), 7.33 (d, 2H), 4.68 (d, 1H), 3.94 (br d, 1H, 3.67 (t, 2H), 3.61-3.37 (m, 5H), 3.23 (s, 3H), 3.03-2.88 (m, 7H), 2.32 (br d, 1H), 1.96 (dd, 1H), 1.73 (br d, 2H), 1.37-1.25 (m, 2H).

### Beispiel 450

### (4-Hydroxypiperidin-1-yl){3-[3-(propan-2-yl)-1,2,4-oxadiazol-5-yl]-5-[4-(trifluormethoxy)-phenyl]piperidin-1-yl}methanon [racemisches cis-Isomer]

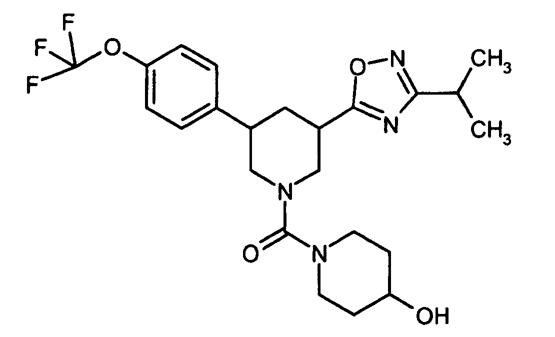

Nach der Allgemeinen Methode 2 wurden 200 mg (0.480 mmol) der Verbindung aus Beispiel 63A und 98 mg (0.961 mmol) N-Hydroxy-2-methylpropanimidamid umgesetzt. Ausbeute: 62 mg (27% d. Th.)

LC-MS (Methode 9B): Rₜ = 1.16 min; MS (ESIpos): m/z = 483 [M+H]⁺;

¹H-NMR (400 MHz, DMSO-d₆): δ = 7.46 (d, 2H), 7.32 (d, 2H), 4.67 (d, 1H), 3.94 (br d, 1H), 3.64-3.57 (m, 1H), 3.55 (br d, 1H), 3.52-3.47 (m, 2H), 3.42-3.34 (m, 1H), 3.08-2.85 (m, 6H), 2.31 (br d, 1H), 1.96 (dd, 1H), 7.71 (br d, 1H), 1.37-1.28 (m, 2H), 1.26 (s, 3H), 1.24 (s, 3H).

### Beispiel 451

### (4-Hydroxypiperidin-1-yl){3-[3-(propan-2-yl)-1,2,4-oxadiazol-5-yl]-5-[4-(trifluormethoxy)phenyl]-piperidin-1-yl}methanon [enantiomerenreines cis-Isomer]

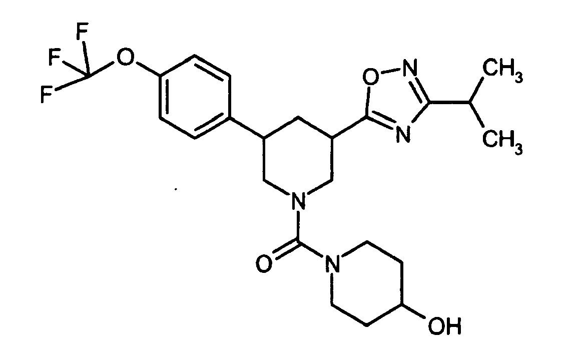

Die Enantiomerentrennung von 60 mg des Racemates aus Beispiel 450 nach Methode 13D ergab 25 mg der Titelverbindung aus Beispiel 451 und 24 mg der Titelverbindung aus Beispiel 452.

HPLC (Methode 10E): Rₜ = 4.04 min, >99.0% ee; LC-MS (Methode 9B ): Rₜ = 1.1 min; MS (ESIpos): m/z = 483 [M+H]⁺.

### Beispiel 452

### (4-Hydroxypipendin-1-yl){3-[3-(propan-2-yl)-1,2,4-oxadiazol-5-yl]-5-[4-(trifluormethoxy)phenyl]-piperidin-1-yl}methanon [enantiomerenreines cis-Isomer]

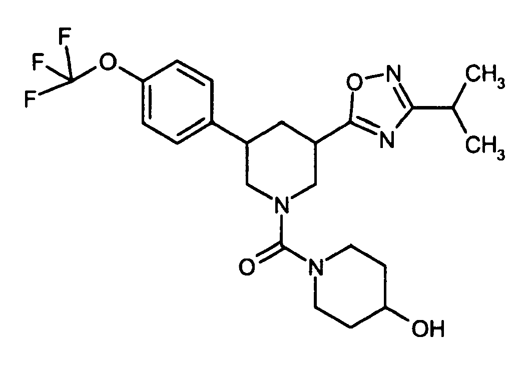

Die Enantiomerentrennung von 60 mg des Racemates aus Beispiel 450 nach Methode 13D ergab 25 mg der Titelverbindung aus Beispiel 451 und 24 mg der Titelverbindung aus Beispiel 452.

HPLC (Methode 10E): Rₜ = 6.14 min, >99.0% ee; LC-MS (Methode 9B ): Rₜ = 1.15 min; MS (ESIpos): m/z = 483 [M+H]⁺.

### Beispiel 453

### (4-Hydroxypiperidin-1-yl){3-(3-methyl-1,2,4-oxadiazol-5-yl)-5-[4-(trifluormethoxy)phenyl]-piperidin-1-yl}methanon [racemisches cis-Isomer]

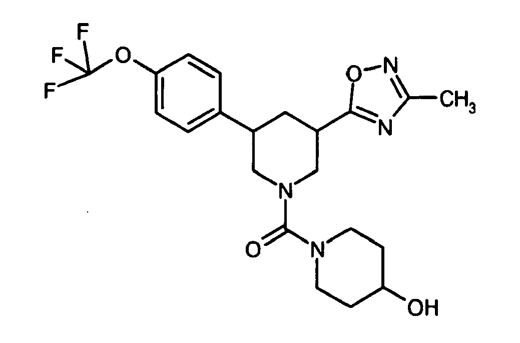

Nach der Allgemeinen Methode 2 wurden 200 mg (0.480 mmol) der Verbindung aus Beispiel 63A und 71 mg (0.961 mmol) N-Hydroxyethanimidamid umgesetzt. Ausbeute: 144 mg (62% d. Th.)

LC-MS (Methode 2B): Rₜ = 1.19 min; MS (ESIpos): m/z = 455 [M+H]⁺;

¹H-NMR (400 MHz, DMSO-d₆): δ = 7.45 (d, 2H), 7.33 (d, 2H), 4.68 (d, 1H), 3.93 (br d, 1H), 3.65-3.54 (m, 2H), 3.51-3.44 (m, 2H), 3.37 (tt, 1H), 3.03-2.85 (m, 5H), 2.33 (s, 3H), 2.30 (br d, 1H), 1.96 (dd, 1H), 1.71 (br d, 1H), 1.35-1.25 (m, 2H).

### Beispiel 454

### (4-Hydroxypiperidin-1-yl) {3-(3-methyl-1,2,4-oxadiazol-5-yl)-5-[4-(trifluormethoxy)phenyl]-piperidin-1-yl}methanon [enantiomerenreines cis-Isomer]

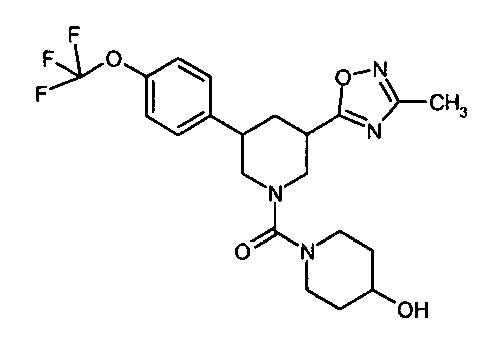

Enantiomerentrennung von 140 mg des Racemates aus Beispiel 453 nach Methode 12D ergab 54 mg der Titelverbindung aus Beispiel 454 und 54 mg der Titelverbindung aus Beispiel 455.

HPLC (Methode 9E): Rₜ = 4.48 min, >99.0% ee; LC-MS (Methode 2B): Rₜ = 1.16 min; MS (ESIpos): m/z = 455 [M+H]⁺.

### Beispiel 455

### (4-Hydroxypiperidin-1-yl){3-(3-methyl-1,2,4-oxadiazol-5-yl)-5-[4-(trifluormethoxy)phenyl]-piperidin-1-yl}methanon [enantiomerenreines cis-Isomer]

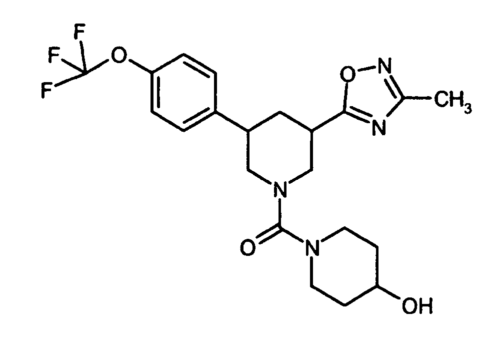

Enantiomerentrennung von 140 mg des Racemates aus Beispiel 453 nach Methode 12D ergab 54 mg der Titelverbindung aus Beispiel 454 und 54 mg der Titelverbindung aus Beispiel 455.

HPLC (Methode 9E): Rₜ = 12.43 min, >99.0% ee; LC-MS (Methode 2B): Rₜ = 1.16 min; MS (ESIpos): m/z = 455 [M+H]⁺.

### Beispiel 456

### [3-(3,4-Dimethylphenyl)-5-(3-methyl-1,2,4-oxadiazol-5-yl)piperidin-1-yl](4-hydroxypiperidin-1-yl)methanon [enantiomerenreines cis-Isomer]

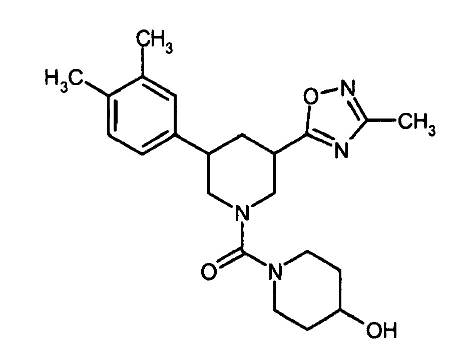

Die Enantiomerentrennung von 150 mg des Racemates aus Beispiel 198 nach Methode 6D ergab 75 mg der Titelverbindung aus Beispiel 456 und 66 mg der Titelverbindung aus Beispiel 457.

HPLC (Methode 5E): Rₜ = 4.42 min, >99.0% ee; LC-MS (Methode 2B): Rₜ = 1.16 min; MS (ESIpos): m/z = 455 [M+H]⁺.

[α]₃₆₅²⁰ = -28.9, Methanol

### Beispiel 457

### [3-(3,4-Dimethylphenyl)-5-(3-methyl-1,2,4-oxadiazol-5-yl)piperidin-1-yl](4-hydroxypiperidin-1-yl)methanon [enantiomerenreines cis-Isomer]

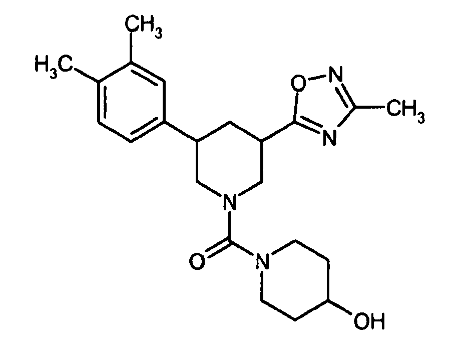

Die Enantiomerentrennung von 150 mg des Racemates aus Beispiel 198 nach Methode 6D ergab 75 mg der Titelverbindung aus Beispiel 456 und 66 mg der Titelverbindung aus Beispiel 457.

HPLC (Methode 5E): Rₜ = 6.86 min, >99.0% ee; LC-MS (Methode 2B): Rₜ = 1.16 min; MS (ESIpos): m/z = 455 [M+H]⁺.

[α]₃₆₅²⁰= +25.0, Methanol

### Beispiel 458

### {3-[3-(2-Methoxyethyl)-1,2,4-oxadiazol-5-yl]-5-[4-methyl-3-(trifluormethyl)phenyl]piperidin-1-yl}(morpholin-4-yl)methanon [enantiomerenreines cis-Isomer]

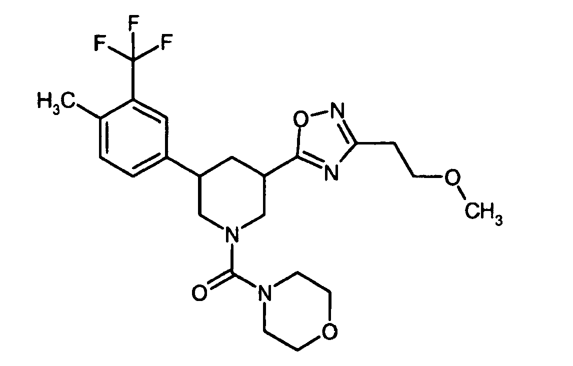

Die Enantiomerentrennung von 100 mg des Racemates aus Beispiel 416 nach Methode 13D ergab 41 mg der Titelverbindung aus Beispiel 458 und 41 mg der Titelverbindung aus Beispiel 459.

HPLC (Methode 10E): Rₜ = 7.21 min, >99.0% ee; LC-MS (Methode 2B): Rₜ = 1.16 min; MS (ESIpos): m/z = 455 [M+H]⁺.

[α]₃₆₅ ²⁰= -18.4, Methanol

### Beispiel 459

### {3-[3-(2-Methoxyethyl)-1,2,4-oxadiazol-5-yl]-5-[4-methyl-3-(trifluormethyl)phenyl]piperidin-1-yl}(morpholin-4-yl)methanon [enantiomerenreines cis-Isomer]

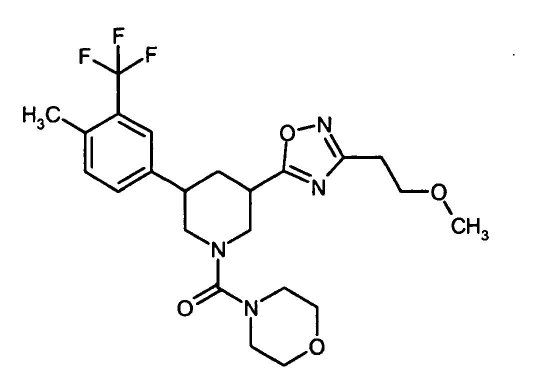

Die Enantiomerentrennung von 100 mg des Racemates aus Beispiel 416 nach Methode 13D ergab 41 mg der Titelverbindung aus Beispiel 458 und 41 mg der Titelverbindung aus Beispiel 459.

HPLC (Methode 10E): Rₜ = 9.83 min, >99.0% ee; LC-MS (Methode 2B): Rₜ = 1.16 min; MS (ESIpos): m/z = 455 [M+H]⁺.

[α]₃₆₅ ²⁰= +16.7, Methanol

### Beispiel 460

### (4-Hydroxypiperidin-1-yl) {3-[3-(2-methoxyethyl)-1,2,4-oxadiazol-5-yl]-5-[4-methyl-3-(trifluormethyl)phenyl]piperidin-1-yl}methanon [enantiomerenreines cis-Isomer]

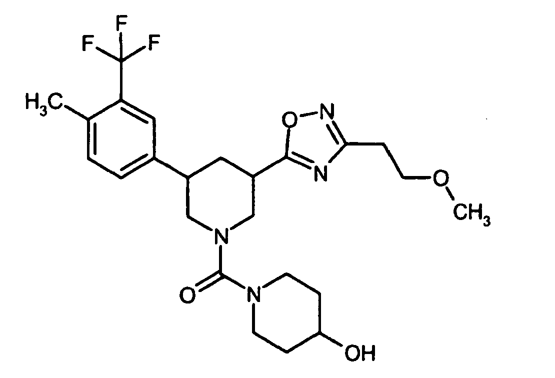

Die Enantiomerentrennung von 100 mg (0.20 mmol) des Racemates aus Beispiel 417 nach Methode 11D ergab 47 mg der Titelverbindung aus Beispiel 460 (Enantiomer 1) und 46 mg der Titelverbindung aus Beispiel 461 (Enantiomer 2).

HPLC (Methode 10E): Rₜ = 6.89 min, >99.0% ee; LC-MS (Methode 2B): Rₜ = 1.16 min; MS (ESIpos): m/z = 455 [M+H]⁺.

### Beispiel 461

### (4-Hydroxypiperidin-1-yl){3-[3-(2-methoxyethyl)-1,2,4-oxadiazol-5-yl]-5-[4-methyl-3-(trifluormethyl)phenyl]piperidin-1-yl}methanon [enantiomerenreines cis-Isomer]

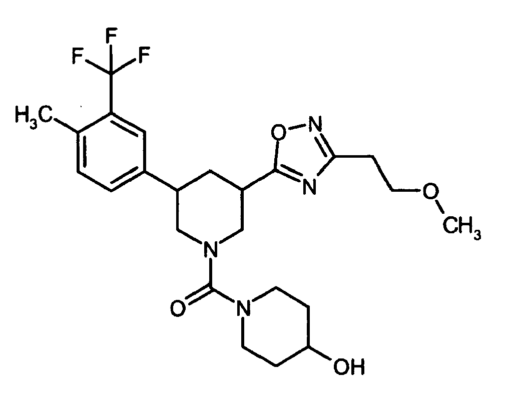

Die Enantiomerentrennung von 100 mg (0.20 mmol) des Racemates aus Beispiel 417 nach Methode 11D ergab 47 mg der Titelverbindung aus Beispiel 460 (Enantiomer 1) und 46 mg der Titelverbindung aus Beispiel 461 (Enantiomer 2).

HPLC (Methode 10E): Rₜ = 24.50 min, >99.0% ee; LC-MS (Methode 2B): Rₜ = 1.16 min; MS (ESIpos): m/z = 455 [M+H]⁺.

### Beispiel 462

### (2,2-Dimethylmorpholin-4-yl){3-(3-methyl-1,2,4-oxadiazol-5-yl)-5-[4-(trifluormethyl)phenyl]-piperidin-1-yl}methanon [racemisches cis-Isomer]

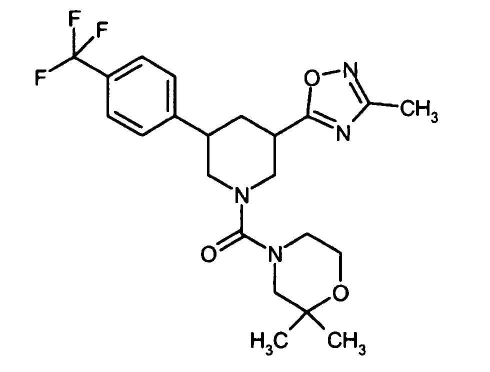

Nach der Allgemeinen Methode 2 wurden 250 mg (0.410 mmol, 68% Reinheit) 1-[(2,2-Dimethylmorpholin-4-yl)carbonyl]-5-[4-(trifluormethyl)phenyl]piperidin-3-carbonsäure und 33.4 mg (0.451 mmol) *N'*-Hydroxyethanimidamid umgesetzt. Ausbeute: 117 mg (62% d. Th.).

LC-MS (Methode 2B): Rₜ =1.31 min; MS (ESIpos): m/z = 453 [M+H]⁺;

¹H-NMR (400 MHz, DMSO-d₆): δ = 7.71 (d, 2H), 7.57 (d, 2H), 3.99 (br d, 1H), 3.68-3.57 (m, 3H), 3.41 (tt, 1H), 3.19-3.11 (m, 2H), 3.10-2.96 (m, 5H), 2.38-2.30 (m, 4H), 2.04 (dd, 1H), 1.14 (s, 6H).

### Beispiel 463

### Morpholin-4-yl{3-[5-(pyrrolidin-2-yl)-1,2,4-oxadiazol-3-yl]-5-[4-(trifluormethyl)phenyl]piperidin-1-yl}methanon Hydrochlorid [racemisches cis-Isomer]

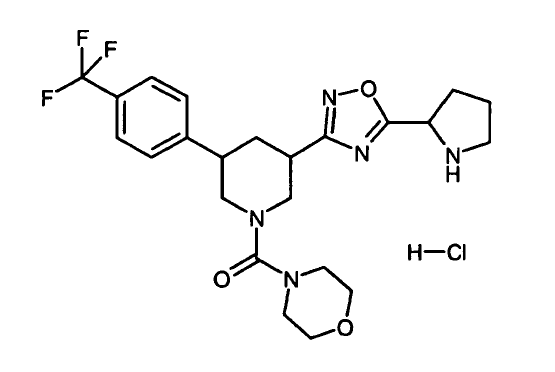

Zu einer Lösung von 84 mg (0.146 mmol) der Verbindung aus Beispiel 91A in 0.7 ml Dioxan wurden 0.36 ml (1.46 mmol) einer 4N Chlorwasserstoff-Dioxan-Lösung zugegeben, und die Mischung wurde 20 Stunden bei Raumtemperatur gerührt. Anschließend wurde das Reaktionsgemisch im Vakuum eingeengt, mit Dichlormethan versetzt und wieder eingeengt. Ausbeute: 74 mg (98% d. Th).

LC-MS (Methode2B): Rₜ = 0.93 min; MS (ESIpos): m/z = 480 [M+H-HCl]⁺

### Beispiel 464

### (3-[5-(Azetidin-3-yl)-1,2,4-oxadiazol-3-yl]-5-[4-(trifluormethyl)phenyl]piperidin-1-yl)(morpholin-4-yl)methanon Hydrochlorid [racemisches cis-Isomer]

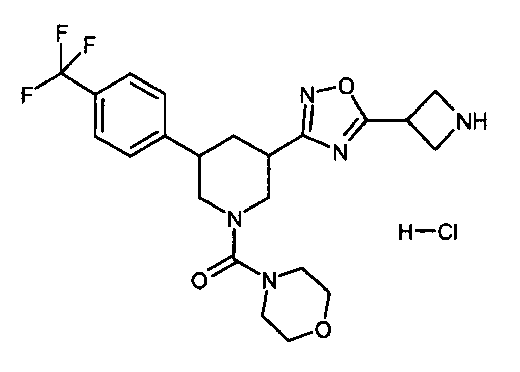

Zu einer Lösung von 67 mg (0.117 mmol) der Verbindung aus Beispiel 92A in 0.6 ml Dioxan wurden 0.30 ml (1.16 mmol) einer 4N Chlorwasserstoff-Dioxan-Lösung hinzugegeben. Das Reaktionsgemisch wurde 20 Stunden bei Raumtemperatur gerührt. Die Mischung wurde einrotiert, erneut in 0.6 ml Dioxan aufgenommen, und mit 0.3 ml (1.16 mmol) einer 4N Chlorwasserstoff-Dioxan-Lösung versetzt. Das Reaktionsgemisch wurde über Nacht bei Raumtemperatur gerührt, dann im Vakuum zur Trockne eingeengt und getrocknet bis zur Gewichtskonstanz. Ausbeute: 58 mg (100% d. Th).

LC-MS (Methode 1B): Rₜ = 1.44 min; MS (ESIpos): m/z = 466 [M+H-HCl]⁺.

### Beispiel 465

### {3-[5-(Methoxymethyl)-1,2,4-oxadiazol-3-yl]-5-[4-(trifluormethyl)phenyl]piperidin-1-yl}-(morpholin-4-yl)methanon

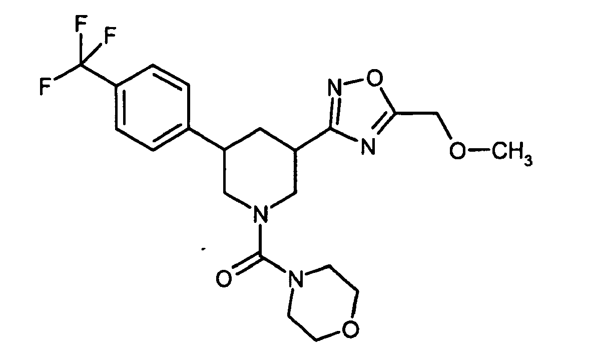

Nach der Allgemeinen Methode 2 wurden 38 mg (0.425 mmol) 2-Methoxyethansäure und 100 mg (ca. 0.212 mmol) der Verbindung aus Beispiel 84A umgesetzt. Ausbeute: 16 mg (17% d. Th.).

LC-MS (Methode 2B): Rₜ = 1.22 min; MS (ESIpos): m/z = 455 [M+H]⁺;

¹H-NMR (400 MHz, DMSO-d₆): δ = 7.70 (d, 2H), 7.57 (d, 2H), 4.74 (s, 2H), 3.96 (brd, 1H), 3.66 (d, 1H), 3.58-3.54 (m, 4H), 3.28 (s, 3H), 3.27-3.16 (m, 5H), 3.09-2.97 (m, 3H), 2.27 (br d, 1H), 1.96 (q, 1H).

### Beispiel 466

### Morpholin-4-yl {3-[5-(pyrazin-2-yl)-1,2,4-oxadiazol-3-yl]-5-[4-(trifluormethyl)phenyl]piperidin-1-yl}methanon [enantiomerenreines cis-Isomer]

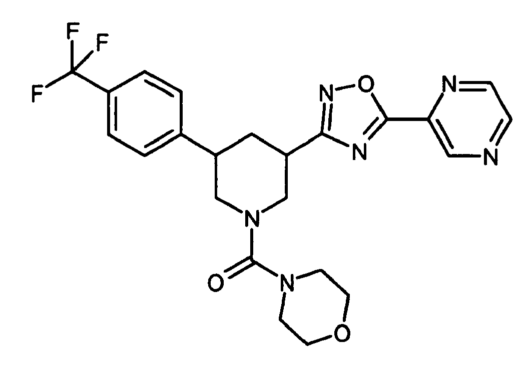

Nach der Allgemeinen Methode 1 wurden 63 mg (0.509 mmol) Pyrazin-2-carbonsäure und 200 mg (ca. 0.425 mmol) der Verbindung aus Beispiel 84A umgesetzt. Enantiomerentrennung von 38 mg des Racemates nach Methode 14D ergab 10 mg der Titelverbindung aus Beispiel 466 und 10 mg der Titelverbindung aus Beispiel 467.

HPLC (Methode 11E): Rₜ = 5.48 min, >99% ee; LC-MS (Methode 1B): Rₜ = 2.32 min; MS (ESIpos): m/z = 489 [M+H]⁺;

¹H-NMR (400 MHz, DMSO-d₆): δ = 9.41 (d, 1H), 8.95 (d, 1H), 8.91 (dd, 1H), 7.71 (d, 2H), 7.60 (d, 2H), 4.06 (br d, 1H), 3.66 (d, 1H), 3.60-3.56 (m, 4H), 3.35 (tt, 1H), 3.24-3.19 (m, 4H), 3.14-2.99 (m, 3H), 2.38 (br d, 1H), 2.06 (q, 1H).

### Beispiel 467

### Morpholin-4-yl{3-[5-(pyrazin-2-yl)-1,2,4-oxadiazol-3-yl]-5-[4-(trifluormethyl)phenyl]piperidin-1-yl}methanon [enantiomerenreines cis-Isomer]

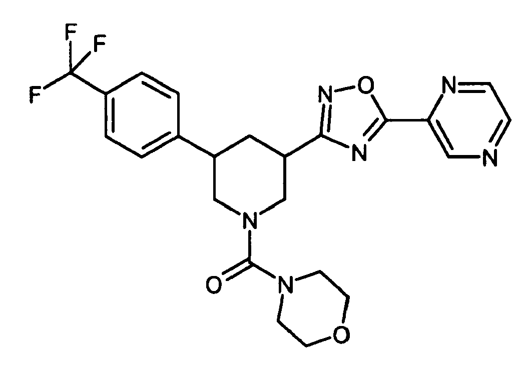

Nach der Allgemeinen Methode 1 wurden 63 mg (0.509 mmol) Pyrazin-2-carbonsäure und 200 mg (ca. 0.425 mmol) der Verbindung aus Beispiel 84A umgesetzt. Enantiomerentrennung von 38 mg des Racemates nach Methode 14D ergab 10 mg der Titelverbindung aus Beispiel 466 und 10 mg der Titelverbindung aus Beispiel 467.

HPLC (Methode 11E): Rₜ = 10.62 min, >99% ee; LC-MS (Methode 1B): Rₜ = 2.32 min; MS (ESIpos): m/z = 489 [M+H]⁺;

¹H-NMR (400 MHz, DMSO-d₆): δ = 9.41 (d, 1H), 8.95 (d, 1H), 8.91 (dd, 1H), 7.71 (d, 2H), 7.60 (d, 2H), 4.06 (br d, 1H), 3.66 (d, 1H), 3.60-3.56 (m, 4H), 3.35 (tt, 1H), 3.24-3.19 (m, 4H), 3.14-2.99 (m, 3H), 2.38 (br d, 1H), 2.06 (q, 1H).

### Beispiel 468

### Morpholin-4-yl{3-[5-(propan-2-yl)-1,3,4-oxadiazol-2-yl]-5-[4-(trifluormethoxy)phenyl]piperidin-1-yl}methanon [racemisches cis-Isomer]

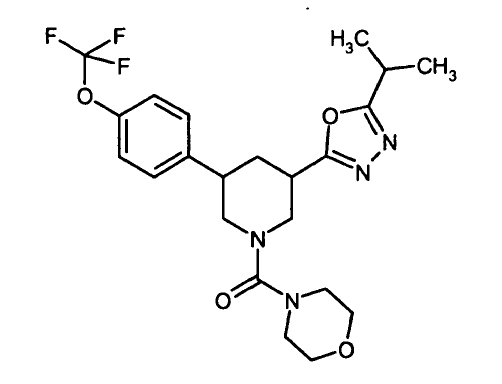

Nach der Allgemeinen Methode 4 wurden 100 mg der Verbindung aus Beispiel 44A und 28 mg (0.273 mmol) 2-Methylpropanhydrazid umgesetzt. Ausbeute: 45 mg (39% d. Th.).

LC-MS (Methode 1B): Rₜ = 2.34 min; MS (ESIpos): m/z = 469 [M+H]⁺;

¹H-NMR (400 MHz, DMSO-d₆): δ = 7.47 (d, 2H), 7.33 (d, 2H), 4.98 (br d, 1H), 3.61 (d, 1H), 3.60-3.53 (m, 4H), 3.22-3.13 (m, 5H), 3.03-2.96 (m, 3H), 2.28 (br d, 1H), 1.92 (q, 1H), 1.28 (d, 6H).

### Beispiel 469

### Ethyl-(5-{1-(morpholin-4-ylcarbonyl)-5-[4-(trifluormethoxy)phenyl]piperidin-3-yl}-1,3,4-oxadiazol-2-yl)acetat [racemisches cis-Isomer]

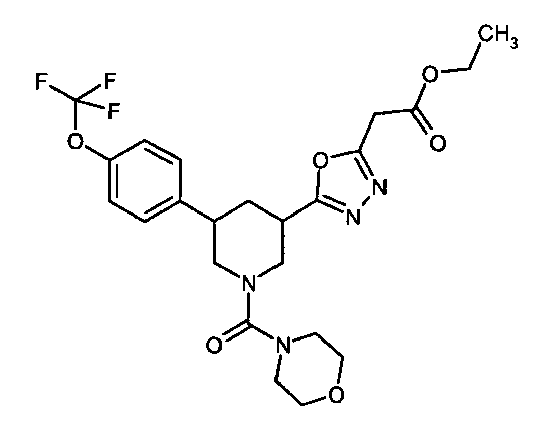

Nach der Allgemeinen Methode 4 wurden 100 mg (0.249 mmol) der Verbindung aus Beispiel 44A und 40 mg (0.273 mmol) Ethyl-3-hydrazinyl-3-oxopropanoat umgesetzt. Ausbeute: 9 mg (6% d. Th.).

LC-MS (Methode 3B): Rₜ = 1.95 min; MS (ESIpos): m/z = 513 [M+H]⁺;

¹H-NMR (400 MHz, DMSO-d₆): δ = 7.47 (d, 2H), 7.33 (d, 2H), 4.16 (s, 2H), 4.13 (q, 2H), 3.98 (br d, 1H), 3.61 (d, 1H), 3.58-3.54 (m, 4H), 3.21-3.15 (m, 4H), 3.04-2.94 (m, 3H), 2.29 (br d, 1H), 1.94 (q, 1H), 1.19 (t, 3H).

### Beispiel 470

### (3-(5-[(Dimethylarnino)methyl]-1,3,4-oxadiazol-2-yl)-5-[4-(trifluormethoxy)phenyl]piperidin-1-yl}(morpholin-4-yl)methanon [racemisches cis-Isomer]

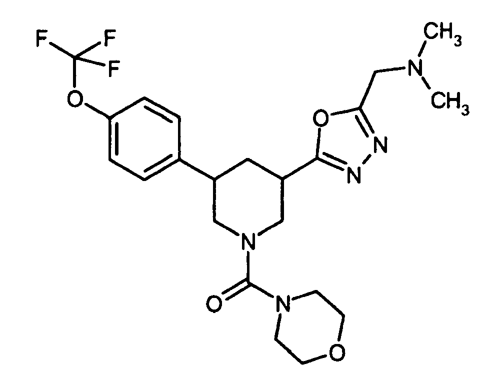

Nach der Allgemeinen Methode 4 wurden 100 mg (0.249 mmol) der Verbindung aus Beispiel 44A und 42 mg (0.275 mmol) 2-(Dimethylamino)acetohydrazid umgesetzt. Ausbeute: 13 mg (11% d. Th.).

LC-MS (Methode 2B): Rₜ = 0.93 min; MS (ESIpos): m/z = 484 [M+H]⁺.

### Beispiel 471

### {3-[5-(4-Fluorphenyl)-1,3,4-oxadiazol-2-yl]-5-[4-(trifluormethyl)phenyl]piperidin-1-yl}-(morpholin-4-yl)methanon [racemisches cis-Isomer]

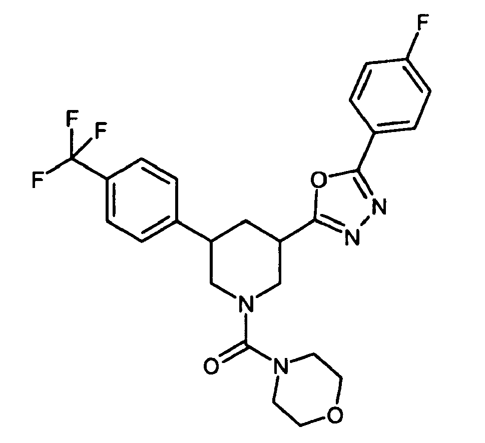

Nach der Allgemeinen Methode 4 wurden 200 mg (0.518 mmol) der Verbindung aus Beispiel 49A und 87 mg (0.569 mmol) 4-Fluorbenzylhydrazid umgesetzt. Ausbeute: 9 mg (3% d. Th.).

LC-MS (Methode 2B): Rₜ = 1.34 min; MS (ESIpos): m/z = 505 [M+H]⁺;

¹H-NMR (400 MHz, DMSO-d,₆): δ = 8.07 (dd, 2H), 7.73 (d, 2H), 7.60 (d, 2H), 7.45 (dd, 2H), 4.09 (br d, 1H), 3.67 (d, 1H), 3.60-3.56 (m, 4H), 3.43 (tt, 1H), 3.24-3.19 (m, 4H), 3.12 (t, 1H), 3.10-2.99 (m, 2H), 2.42 (br d, 1H), 2.07 (q, 1H).

### Beispiel 472

### {3-[5-(3-Chlorphenyl)-1,3,4-oxadiazol-2-yl]-5-[4-trifluormethyl)phenyl]piperidin-1-yl)-(morpholin-4-yl)methanon [racemisches cis-Isomer]

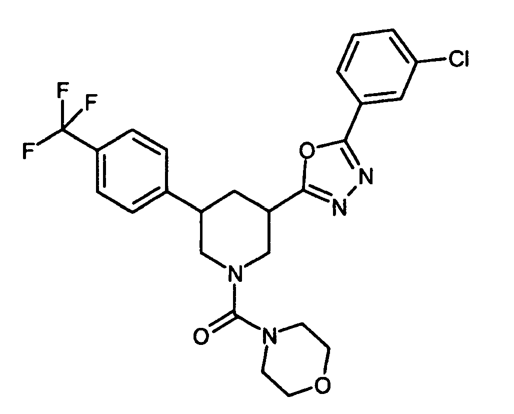

Nach der Allgemeinen Methode 4 wurden 200 mg (0.518 mmol) der Verbindung aus Beispiel 49A und 97 mg (0.569 mmol) 3-Chlorbenzolcarbohydrazid umgesetzt. Ausbeute: 13 mg (11% d. Th.).

LC-MS (Methode 2B): Rₜ = 1.42 min; MS (ESIpos): m/z = 521 [M+H]⁺.

### Beispiel 473

### {3-[5-(3-Fluorphenyl)-1,3,4-oxadiazol-2-yl]-5-[4-(trifluormethyl)phenyl]piperidin-1-yl}-(morpholin-4-yl)methanon [racemisches cis-Isomer]

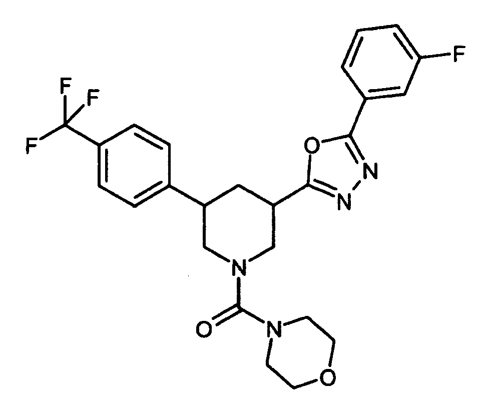

Nach der Allgemeinen Methode 4 wurden 200 mg (0.518 mmol) der Verbindung aus Beispiel 49A und 87 mg (0.569 mmol) 3-Fluorbenzylhydrazid umgesetzt. Ausbeute: 14 mg (5% d. Th.).

LC-MS (Methode 5B): Rₜ = 2.41 min; MS (ESIpos): m/z = 505 [M+H]⁺;

¹H-NMR (400 MHz, DMSO-d₆): δ = 7.87 (d, 1H), 7.81 (d, 1H), 7.71 (d, 2H), 7.66 (dd, 1H), 7.60 (d, 2H), 7.50 (td, 1H), 4.09 (br d, 1H), 3.67 (d, 1H), 3.62-3.56 (m, 4H), 3.44 (tt, 1H), 3.28-3.20 (m, 4H), 3.13 (t, 1H), 3.10-3.01 (m, 2H), 2.43 (br d, 1H), 2.08 (q, 1H).

### Beispiel 474

### {3-[5-(2-Methylpropyl)-1,3,4-oxadiazol-2-yl]-5-[4-(trifluormethoxy)phenyl]piperidin-1-yl} - (morpholin-4-yl)methanon [racemisches cis-Isomer]

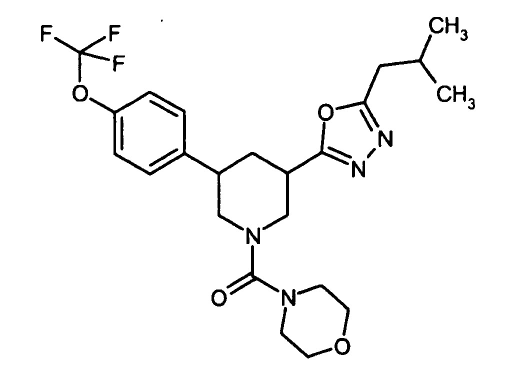

Nach der Allgemeinen Methode 4 wurden 100 mg (0.249 mmol) der Verbindung aus Beispiel 44A und 32 mg (0.273 mmol) 3-Methylbutanhydrazid umgesetzt. Diastereomerentrennung des cis-/trans-Isomerengemisches nach Methode 14C ergab 44 mg der Titelverbindung (cis-Isomer) und 12 mg des trans-Isomers.

LC-MS (Methode 1B): Rₜ = 2.48 min; MS (ESIpos): m/z = 483 [M+H]⁺;

¹H-NMR (400 MHz, DMSO-d₆): δ = 7.47 (d, 2H), 7.33 (d, 2H), 3.98 (br d, 1H), 3.61 (d, 1H), 3.59-3.53 (m, 4H), 3.21-3.18 (m, 4H), 3.03-2.96 (m, 3H), 2.71 (d, 2H), 2.28 (br d, 1H), 2.08-1.98 (m, 1H), 1.93 (q, 1H), 0.93 (d, 6H).

### Beispiel 475

### Morpholin-4-yl{3-{4-[(propan-2-ylsulfanyl)methyl]-1,3-thiazol-2-yl}-5-[4-(trifluormethyl)-phenyl]piperidin-1-yl}methanon [racemisches cis-Isomer]

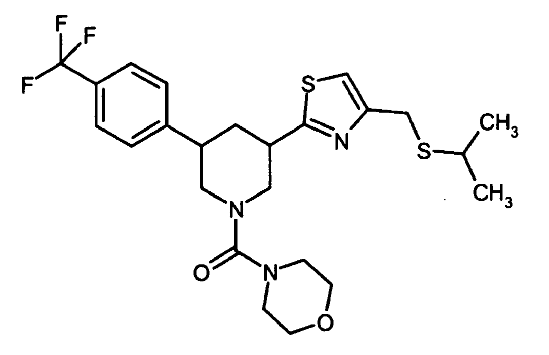

Zu einer Lösung von 138 mg (ca. 0.293 mmol) der Verbindung aus Beispiel 214A in 2.6 ml Dioxan wurden 115 mg (0.352 mmol) Cäsiumcarbonat und 34 µl (0.352 mmol) Propan-2-thiol hinzugegeben. Das Reaktionsgemisch wurde über Nacht bei 70°C gerührt. Ohne weitere Aufarbeitung wurde die Mischung mittels präparativer HPLC aufgereinigt. Ausbeute: 43 mg (28% d. Th.).

LC-MS (Methode 2B): Rₜ = 1.48 min; MS (ESIpos): m/z = 514 [M+H]⁺;

¹H-NMR (400 MHz, DMSO-d₆): δ = 7.70 (d, 2H), 7.58 (d, 2H), 7.39 (s, 1H), 3.98 (brd, 1H), 3.84 (s, 2H), 3.65 (d, 1H), 3.58-3.54 (m, 4H), 3.40-3.33 (m, 1H), 3.24-3.16 (m, 4H), 3.08-2.89 (m, 4H), 2.31 (brd, 1H), 1.97 (q, 1H), 1.20 (d, 6H).

### Beispiel 476

### Morpholin-4-yl{3-{4-[(pyridin-4-ylsulfanyl)methyl]-1,3-thiazol-2-yl}-5-[4-(trifluormethyl)-phenyl]piperidin-1-yl}methanon [racemisches cis-Isomer]

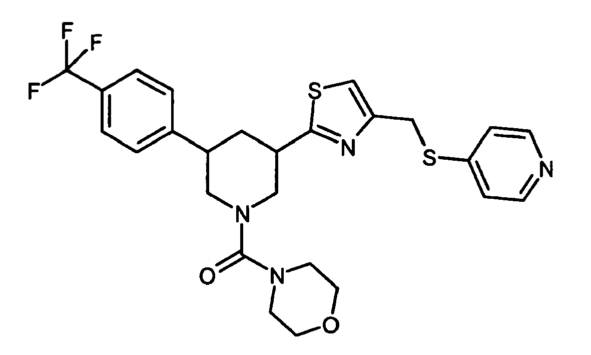

Zu einer Lösung von 138 mg (0.291 mmol) der Verbindung aus Beispiel 214A in 2.4 ml Dioxan wurden 114 mg (0.349 mmol) Cäsiumcarbonat und 39 mg (0.349 mmol) 4-Mercaptopyridin hinzugegeben. Das Reaktionsgemisch wurde über Nacht bei 70°C gerührt. Ohne weitere Aufarbeitung wurde die Mischung mittels präparativer HPLC aufgereinigt. Ausbeute: 20 mg (13% d. Th.).

LC-MS (Methode 2B): Rₜ = 1.79 min; MS (ESIpos): m/z = 549 [M+H]⁺;

¹H-NMR (400 MHz, DMSO-d₆): δ = 8.36 (dd, 2H), 7.70 (d, 2H), 7.58 (d, 2H), 7.55 (s, 1H), 7.37 (d, 2H), 4.45 (s, 2H), 3.96 (brd, 1H), 3.65 (d, 1H), 3.57-3.53 (m, 4H), 3.36 (tt, 1H), 3.21-3.16 (m, 4H), 3.06-2.95 (m, 3H), 2.30 (brd, 1H), 1.97 (q, 1H).

### Beispiel 477

### {3-[4-(3-Fluorphenyl)-1,3-thiazol-2-yl]-5-[4-(trifluormethyl)phenyl]piperidin-1-yl} (morpholin-4-yl)methanon [racemisches cis-Isomer]

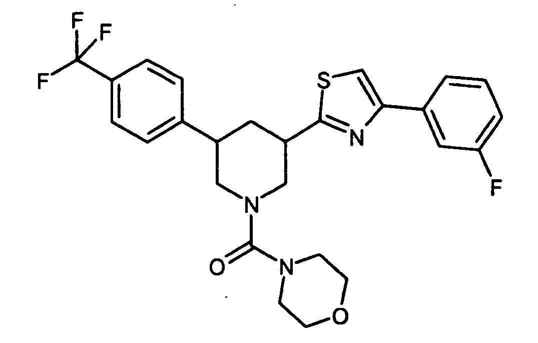

Nach der Allgemeinen Methode 3 wurden 250 mg (ca. 0.211 mmol) der Verbindung aus Beispiel 53A und 154 mg (0.710 mmol) 1-Brom-3,3-dimethylbutan-2-on umgesetzt. Ausbeute: 88 mg (29% d. Th.).

LC-MS (Methode 3B): Rₜ = 2.58 min; MS (ESIpos): m/z = 520 [M+H]⁺;

¹H-NMR (400 MHz, DMSO-d₆): δ = 8.18 (s, 1H), 7.82 (d, 1H), 7.77 (d, 1H), 7.72 (d, 2H), 7.61 (d, 2H), 7.49 (dd, 1H), 7.18 (td, 1H), 4.07 (br d, 1H), 3.68 (d, 1H), 3.61-3.56 (m, 4H), 3.44 (tt, 1H), 3.23-3.19 (m, 4H), 3.11-3.03 (m, 3H), 2.38 (br d, 1H), 2.06 (q, 1H).

### Beispiel 478

### {3-{4-[(1H-Imidazol-2-ylsulfanyl)methyl]-1,3-thiazol-2-yl}-5-[4-(trifluormethyl)phenyl]piperidin-1-yl}(morpholin-4-yl)methanon [racemisches cis-Isomer]

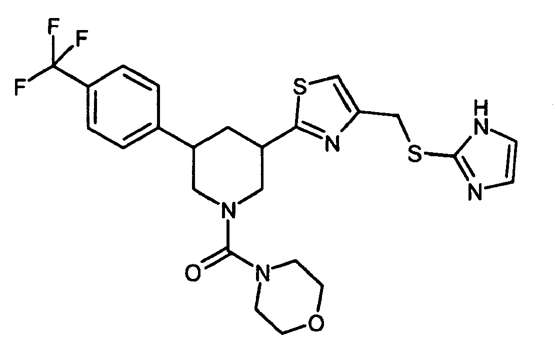

Zu einer Lösung von 139 mg (0.293 mmol) der Verbindung aus Beispiel 214A in 2.4 ml Dioxan wurden 115 mg (0.352 mmol) Cäsiumcarbonat und 42 µl (0.442 mmol) 1-*H*-Imidazol-2-thiol hinzugegeben. Das Reaktionsgemisch wurde 4 Stunden bei 70°C gerührt. Diastereomerentrennung von 123 mg des cis-/trans-Isomerengemisches nach Methode 13C ergab 40 mg der Titelverbindung (cis-Isomer) und 21 mg des trans-Isomers.

LC-MS (Methode 2B): Rₜ = 1.01 min; MS (ESIpos): m/z = 538 [M+H]⁺;

¹H-NMR (400 MHz, DMSO-d₆): δ = 7.71 (d, 2H), 7.57 (d, 2H), 7.33 (s, 1H), 7.23 (br s, 2H), 4.37 (s, 2H), 3.93 (brd, 1H), 3.65 (d, 1H), 3.57-3.54 (m, 4H), 3.21-3.18 (m, 4H), 3.08-2.91 (m, 3H), 2.28 (brd, 1H), 1.93 (q, 1H).

### Beispiel 479

### {3-[4-(2-Methylpropyl)-1,3-thiazol-2-yl]-5-[4-(trifluormethyl)phenyl]piperidin-1-yl}(morpholin-4-yl)methanon [racemisches cis-Isomer]

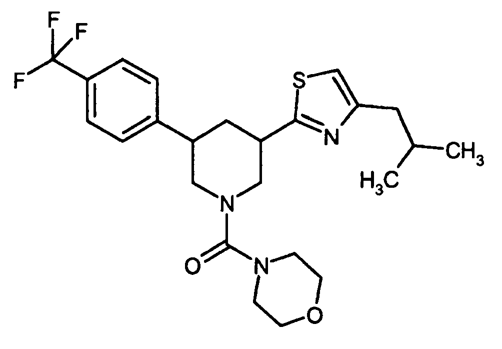

Nach der Allgemeinen Methode 3 wurden 150 mg (ca. 0.315 mmol) der Verbindung aus Beispiel 53A und 76 mg (0.426 mmol) 1-Brom-4-methylpentan-2-on (J. Org. Chem, 19, 2005, 4141-4153) umgesetzt. Ausbeute: 68 mg (40% d. Th.).

LC-MS (Methode 2B): Rₜ = 1.52 min; MS (ESIpos): m/z = 482 [M+H]⁺;

¹H-NMR (400 MHz, DMSO-d₆): δ = 7.70 (d, 2H), 7.58 (d, 2H), 7.17 (s, 1H), 3.97 (brd, 1H), 3.65 (d, 1H), 3.59-3.53 (m, 4H), 3.22-3.17 (m, 4H), 3.05-2.94 (m, 3H), 2.30 (brd, 1H), 2.06-1.87 (m, 2H), 0.88 (6H).

### Beispiel 480

### {3-(4-Ethyl-1,3-thiazol-2-yl)-5-[4-(trifluormethyl)phenyl]piperidin-1-yl}(morpholin-yl)methanon [racemisches cis-Isomer]

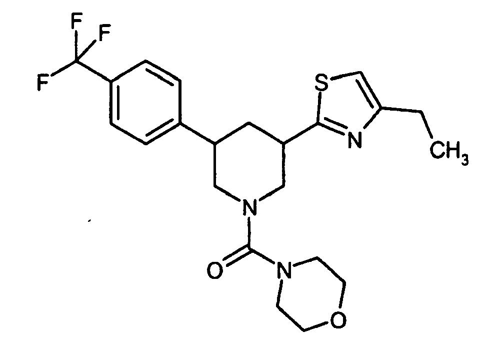

Nach der Allgemeinen Methode 3 wurden 220 mg (ca. 0.384 mmol) der Verbindung aus Beispiel 53A und 70 mg (0.460 mmol) 1-Brombutan-2-on umgesetzt. Ausbeute: 63 mg (36% d. Th.).

LC-MS (Methode 2B): Rₜ = 1.39 min; MS (ESIpos): m/z = 454 [M+H]⁺;

¹H-NMR (400 MHz, DMSO-d₆): δ = 7.70 (d, 2H), 7.58 (d, 2H), 7.17 (s, 1H), 3.96 (brd, 1H), 3.65 (d, 1H), 3.58-3.54 (m, 4H), 3.21-3.17 (m, 4H), 3.12-2.95 (m, 4H), 2.70 (q, 2H), 2.30 (brd, 1H), 1.97 (q, 1H), 1.21 (t, 3H).

### Beispiel 481

### [3-{4-[(Cyclopropylamino)methyl]-1,3-thiazol-2-yl}-5-(4-ethylphenyl)piperidin-1-yl](morpholin-4-yl)methanon [racemisches cis-Isomer]

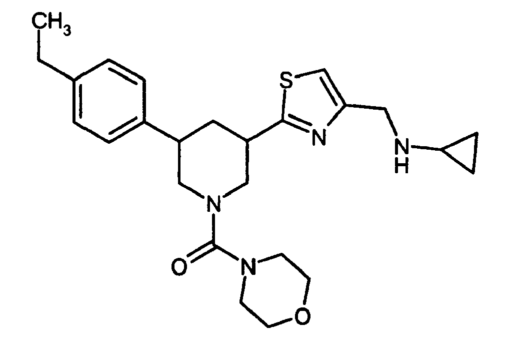

Eine Lösung von 119 mg (0.274 mmol) der Verbindung aus Beispiel 141A in 2 ml N-Methylpyrrolidon wurden mit 24 mg (0.411 mmol) Cyclopropylamin und 70 mg (0.685 mmol) Triethylamin versetzt. Das Reaktionsgemisch wurde über Nacht bei 80°C gerührt. Diastereomerentrennung von 13 mg des cis-/trans-Isomerengemisches nach Methode 15C ergab 5 mg der Titelverbindung.

LC-MS (Methode 1B): Rₜ = 1.51 min; MS (ESIpos): m/z = 455 [M+H]⁺;

¹H-NMR (400 MHz, DMSO-d₆): δ = 7.74 (s, 1H), 7.23 (d, 2H), 7.18 (d, 2H), 4.36 (br s, 2H), 4.02 (br d, 1H), 3.62 (d, 1H), 3.58-3.53 (m, 4H), 3.40 (tt, 1H), 3.23-3.18 (m, 4H), 3.02-2.87 (m, 3H), 2.74 (br s, 1H), 2.57 (q, 2H), 2.28 (br d, 1H), 1.95 (q, 1H), 1.16 (t, 3H), 0.80-0.72 (m, 4H).

### Beispiel 482

### [3-(4-Ethylphenyl)-5-{4-[(propan-2-ylamino)methyl]-1,3-thiazol-2-yl]piperidin-1-yl](morpholin-4-yl)methanon [racemisches cis-Isomer]

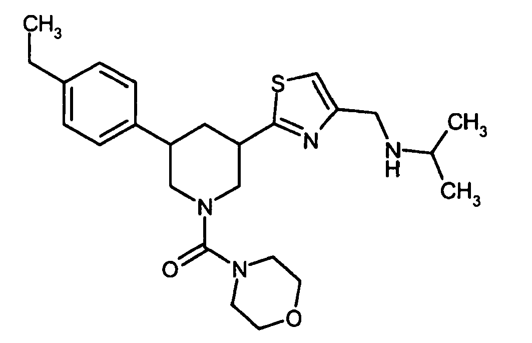

Eine Lösung von 119 mg (0.274 mmol) der Verbindung aus Beispiel 141A in 2 ml *N-*Methylpyrrolidon wurden mit 24 mg (0.411 mmol) Isopropylamin und 70 mg (0.685 mmol) Triethylamin versetzt. Das Reaktionsgemisch wurde über Nacht bei 80°C gerührt und ohne weitere Aufarbeitung mittels präparativer HPLC aufgereinigt. Ausbeute: 10 mg (8% d. Th.)

LC-MS (Methode 3B): Rₜ = 1.27 min; MS (ESIpos): m/z = 457 [M+H]⁺;

¹H-NMR (400 MHz, DMSO-d₆): δ = 7.30 (s, 1H), 7.23 (d, 2H), 7.17 (d, 2H), 3.97 (br d, 1H), 3.75 (s, 2H), 3.61 (d, 1H), 3.59-3.52 (m, 4H), 3.22-3.13 (m, 4H), 2.97-2.85 (m, 3H), 2.75-2.71 (m, 1H), 2.57 (q, 2H), 2.25 (d, 1H), 1.92 (q, 1H), 1.16 (t, 3H), 1.00 (d, 6H).

### Beispiel 483

### 2-[5-(4-Ethylphenyl)-1-(morpholin-4-ylcarbonyl)piperidin-3-yl]-N-(pyridin-4-yl)-1,3-thiazol-4-carboxamid [racemisches cis-Isomer]

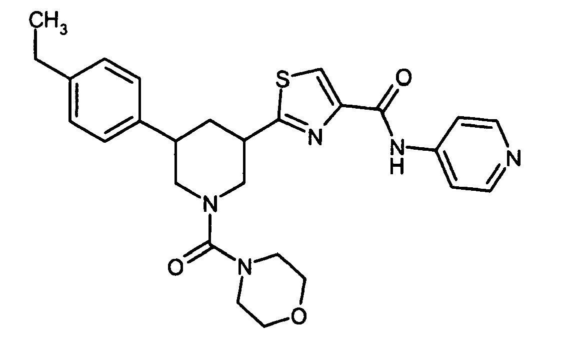

Einer Mischung von 80 mg (0.186 mmol) der Verbindung aus Beispiel 143A in 0.5 ml THF wurde mit 145 mg (0.279 mmol) PYBOP und 169 mg (1.304 mmol) *N,N-*Diisopropylethylamin versetzt. Das Reaktionsgemisch wurde 40 Minuten bei Raumtemperatur gerührt. Anschließend wurden 21 mg (0.223 mmol) 4-Aminopyridin hinzugegeben, und die Mischung wurde über Nacht bei RT weiter gerührt. Zur Aufarbeitung wurde das Lösungsmittel eingedampft und der Rückstand in Essigsäureethylester aufgenommen. Die organische Phase wurde nacheinander mit Wasser und gesättigter wässriger Natriumchlorid-Lösung gewaschen, über Magnesiumsulfat getrocknet und eingeent. Diastereomerentrennung des cis-/trans-Isomerengemisches nach Methode 12C ergab 14 mg der Titelverbindung (cis-Isomer) und 23 mg des trans-Isomers.

LC-MS (Methode 3B): Rₜ = 1.42 min; MS (ESIpos): m/z = 506 [M+H]⁺;

¹H-NMR (400 MHz, DMSO-d₆): δ = 10.45 (s, 1H), 8.48 (d, 2H), 7.86 (d, 2H), 7.26 (d, 2H), 7.18 (d, 2H), 4.09 (br d, 1H), 3.63 (d, 1H), 3.59-3.55 (m, 4H), 3.48 (tt, 1H), 3.22-3.16 (m, 4H), 3.06 (t, 1H), 3.00 (t, 1H), 2.90 (t, 1H), 2.58 (q, 2H), 2.36 (br d, 1H), 2.03 (q, 1H), 1.17 (t, 3H).

### Beispiel 484

### [3-(4-Ethylphenyl)-5-{4-[(ethylsulfanyl)methyl]-1,3-thiazol-2-yl}piperidin-1-yl](morpholin-4-yl)-methanon [racemisches cis-Isomer]

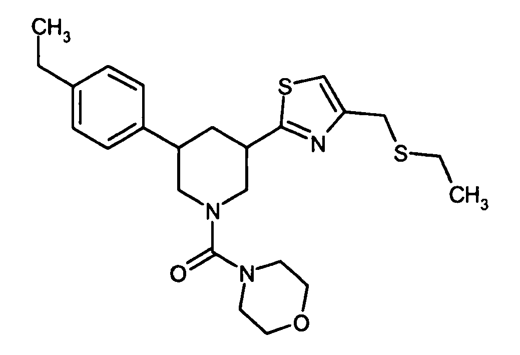

Zu einer Lösung von 100 mg (ca. 0.184 mmol) der Verbindung aus Beispiel 141A in 1.5 ml Dioxan wurden 73 mg (0.221 mmol) Cäsiumcarbonat und 36 µl (0.442 mmol) Ethanthiol hinzugegeben. Das Reaktionsgemisch wurde 4 Stunden bei 70°C gerührt. Ohne weitere Aufarbeitung wurde die Mischung mittels präparativer HPLC aufgereinigt. Ausbeute: 40 mg (47% d. Th).

LC-MS (Methode 1B): Rₜ = 2.71 min; MS (ESIpos): m/z = 460 [M+H]⁺;

¹H-NMR (400 MHz, DMSO-d₆): δ = 7.38 (s, 1H), 7.23 (d, 2H), 7.17 (d, 2H), 4.98 (br d, 1H), 3.81 (s, 2H), 3.61 (d, 2H), 3.58-3.54 (m, 4H), 3.20-3.15 (m, 4H), 2.97-2.84 (m, 3H), 2.57 (q, 2H), 2.26 (br d, 1H), 1.91 (q, 1H), 1.17 (t, 3H), 1.16 (t, 3H).

### Beispiel 485

### {3-[4-(2-Methylpropyl)-1,3-thiazol-2-yl]-5-[4-(trifluormethoxy)phenyl]piperidin-1-yl}(morpholin-4-yl)methanon [racemisches cis-Isomer]

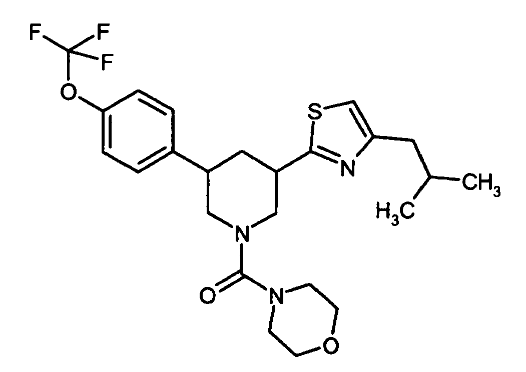

Nach der Allgemeinen Methode 3 wurden 100 mg (ca. 0.182 mmol) der Verbindung aus Beispiel 115A und 49 mg (0.273 mmol) 1-Brom-4-methyl-pentan-2-on (J. Org. Chem, 19, 2005, 4141-4153) umgesetzt. Ausbeute: 50 mg (55% d. Th.).

LC-MS (Methode 1B): Rₜ = 2.92 min; MS (ESIpos): m/z = 498 [M+H]⁺;

¹H-NMR (400 MHz, DMSO-d₆): δ = 7.48 (d, 2H), 7.32 (d, 2H), 7.17 (s, 1H), 3.97 (br d, 1H), 3.63 (d, 1H), 3.58-3.54 (m, 4H), 3.20-3.16 (m, 4H), 3.02-2.92 (m, 3H), 2.29 (br d, 1H), 2.02-1.87 (m, 2H), 0.88 (d, 6H).

### Beispiel 486

### {3-(4-Cyclopropyl-1,3-thiazol-2-yl)-5-[4-(trifluormethoxy)phenyl]piperidin-1-yl}(morpholin-4-yl)-methanon [racemisches cis-Isomer]

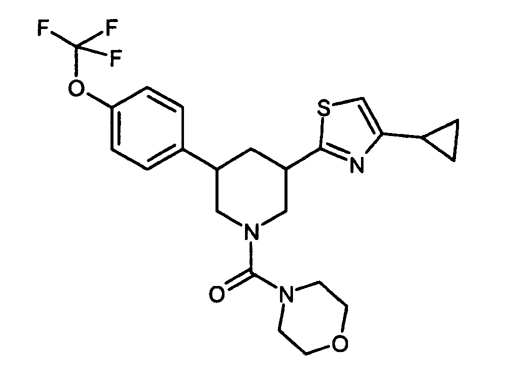

Nach der Allgemeinen Methode 3 wurden 100 mg (ca. 0.182 mmol) der Verbindung aus Beispiel 115A und 44 mg (0.273 mmol) 2-Brom-1-cyclopropylethanon (Tetrahedron, 43, 20 1987, 4609-4619) umgesetzt. Ausbeute: 49 mg (56% d. Th.).

LC-MS (Methode 1B): Rₜ = 2.73 min; MS (ESIpos): m/z = 482 [M+H]⁺;

¹H-NMR (400 MHz, DMSO-d₆): δ = 7.47 (d, 2H), 7.32 (d, 2H), 7.15 (s, 1H), 3.94 (br d, 1H), 3.63 (d, 1H), 3.58-3.54 (m, 4H), 3.28 (tt, 1H), 3.20-3.16 (m, 4H), 2.99-2.89 (m, 3H), 2.26 (br d, 1H), 2.08-2.01 (m, 1H), 1.90 (q, 1H), 0.90-0.85 (m, 2H), 0.79-0.76 (m, 2H).

### Beispiel 487

### {3-(4-tert-Butyl-1,3-thiazol-2-yl)-5-[4-(trifluormethoxy)phenyl]piperidin-1-yl}(morpholin-4-yl)-methanon [racemisches cis-Isomer]

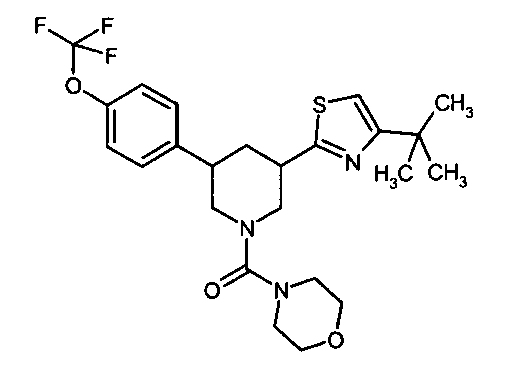

Nach der Allgemeinen Methode 3 wurden 100 mg (ca. 0.182 mmol) der Verbindung aus Beispiel 115A und 49 mg (0.273 mmol) 1-Brom-3,3-dimethylbutan-2-on umgesetzt. Ausbeute: 56 mg (59% d. Th.).

LC-MS (Methode 1B): Rₜ = 3.0 min; MS (ESIpos): m/z = 498 [M+H]⁺;

¹H-NMR (400 MHz, DMSO-d₆): δ = 7.48 (d, 2H), 7.33 (d, 2H), 7.16 (s, 1H), 4.00 (br d, 1H), 3.63 (d, 1H), 3.58-3.54 (m, 4H), 3.20-3.18 (m, 4H), 3.01-2.91 (m, 3H), 2.31 (br d, 1H), 1.91 (q, 1H), 1.28 (s, 9H).

### Beispiel 488

### {3-[4-(3-Fluorphenyl)-1,3-thiazol-2-yl]-5-[4-(trifluormethoxy)phenyl]piperidin-1-yl}(morpholin-4-yl)methanon [racemisches cis-Isomer]

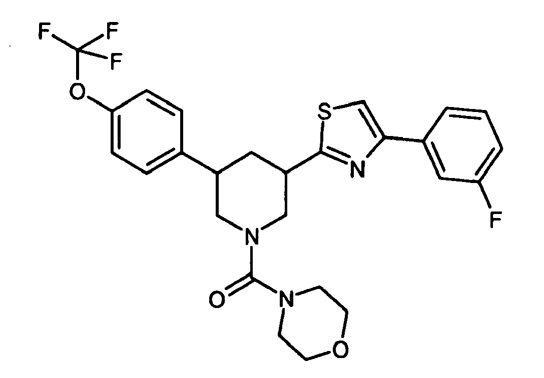

Nach der Allgemeinen Methode 3 wurden 100 mg (ca. 0.211 mmol) der Verbindung aus Beispiel 115A und 55 mg (0.253 mmol) 2-Brom-1-(3-fluorphenyl)ethanon umgesetzt. Ausbeute: 78 mg (69% d. Th.).

LC-MS (Methode 2B): Rₜ = 1.57 min; MS (ESIpos): m/z = 536 [M+H]⁺;

¹H-NMR (400 MHz, DMSO-d₆): δ = 8.17 (s, 1H), 7.82 (d, 1H), 7.75 (d, 1H), 7.52-7.46 (m, 3H), 7.35-7.33 (d, 2H), 7.17 (td, 1H), 4.06 (br d, 1H), 3.65 (d, 1H), 3.60-3.56 (m, 4H), 3.43 (tt, 1H), 3.23-3.20 (m, 4H), 3.08-3.00 (m 3H), 2.36 (br d, 1H), 2.03 (q, 1H).

### Beispiel 489

### {3-(4-Methyl-1,3-thiazol-2-yl)-5-[4-(trifluormethoxy)phenyl]piperidin-1-yl}(morpholin-4-yl)-methanon [racemisches cis-Isomer]

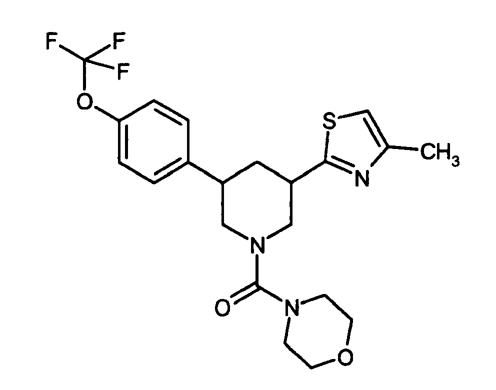

Nach der Allgemeinen Methode 3 wurden 100 mg (ca. 0.182 mmol) der Verbindung aus Beispiel 115A und 25 mg (0.273 mmol) 1-Chlorpropan-2-on umgesetzt. Ausbeute: 40 mg (44% d. Th.).

LC-MS (Methode 2B): Rₜ = 2.48 min; MS (ESIpos): m/z = 455 [M+H]⁺;

¹H-NMR (400 MHz, DMSO-d₆): δ = 7.47 (d, 2H), 7.32 (d, 2H), 7.17 (s, 1H), 3.95 (br d, 1H), 3.63 (d, 1H), 3.58-3.54 (m, 4H), 3.20-3.16 (m, 4H), 2.99-2.93 (m 3H), 2.37 (s, 3H), 2.27 (br d, 1H), 1.95 (q, 1H).

### Beispiel 490

### {3-(4-Ethyl-1,3-thiazol-2-yl)-5-[4-(trifluormethoxy)phenyl]piperidin-1-yl}(morpholin-4-yl)-methanon [racemisches cis-Isomer]

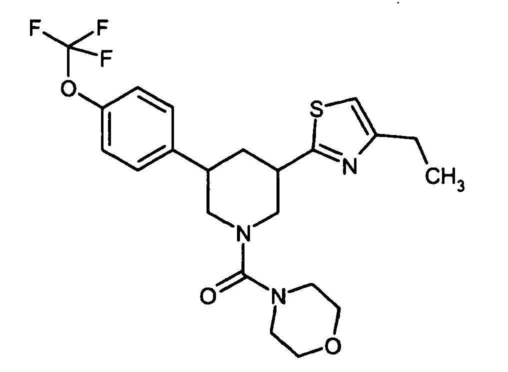

Nach der Allgemeinen Methode 3 wurden 100 mg (ca. 0.211 mmol) der Verbindung aus Beispiel 115A und 38 mg (0.253 mmol) 1-Brombutan-2-on umgesetzt. Ausbeute: 49 mg (47% d. Th.).

LC-MS (Methode 2B): Rₜ = 1.42 min; MS (ESIpos): m/z = 470 [M+H]⁺;

¹H-NMR (400 MHz, DMSO-d₆): δ = 7.48 (d, 2H), 7.32 (d, 2H), 7.17 (s, 1H), 3.63 (br d, 1H), 3.57-3.53 (m, 4H), 3.21-3.15 (m, 4H), 2.99-2.93 (m, 3H), 2.70 (q, 2H), 2.27 (br d, 1H), 1.93 (q, 1H), 1.21(t, 3H).

### Beispiel 491

### 1-({3-(4-tert-Butyl-1,3-thiazol-2-yl)-5-[4-(trifluormethoxy)phenyl]piperidin-1-yl}carbonyl)-piperidin-4-carbonitril [racemisches cis-Isomer]

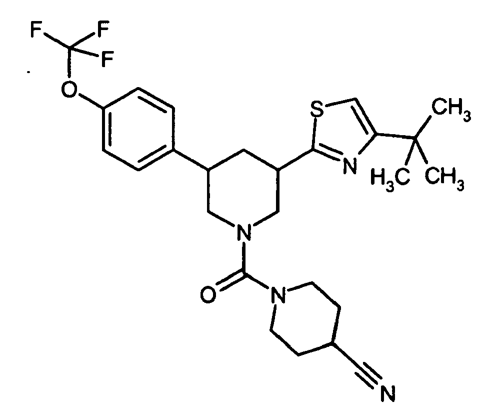

Nach der Allgemeinen Methode 3 wurden 200 mg (ca. 0.204 mmol) der Verbindung aus Beispiel 117A und 55 mg (0.300 mmol) 1-Brom-3,3-dimethylbutan-2-on umgesetzt. Ausbeute: 43 mg (40% d. Th.).

LC-MS (Methode 2B): Rₜ = 1.64 min; MS (ESIpos): m/z = 521 [M+H]⁺;

¹H-NMR (400 MHz, DMSO-d₆): δ = 7.48 (d, 2H), 7.33 (d, 2H), 7.15 (s, 1H), 3.97 (br d, 1H), 3.60 (d, 1H), 3.09-2.87 (m, 5H), 2.71 (s, 1H), 2.33 (br d, 1H), 1.98-1.87 (m, 2H), 1.72-1.64 (m, 1H), 1.28 (s, 9H).

### Beispiel 492

### {3-[5-(2-Methoxyethyl)-1,3,4-thiadiazol-2-yl]-5-[4-(trifluormethoxy)phenyl]piperidin-1-yl}-(morpholin-4-yl)methanon [racemisches cis-Isomer]

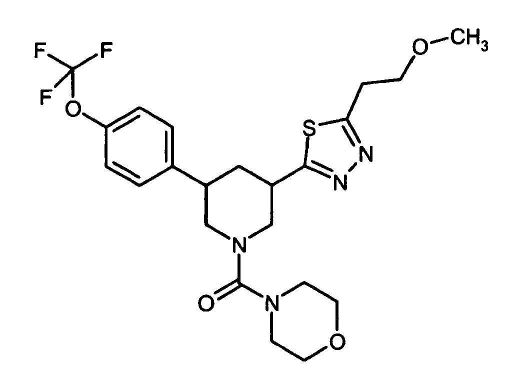

Nach der Allgemeinen Methode 9 wurden 93 mg (0.185 mmol) der Verbindung aus Beispiel 120A und 150 mg (0.371 mmol) Lawessons-Reagenz umgesetzt. Ausbeute: 9 mg (10% d. Th.)

LC-MS (Methode 2B): Rₜ = 1.21 min; MS (ESIpos): m/z = 501 [M+H]⁺;

¹H-NMR (400 MHz, DMSO-d₆): δ = 7.48 (d, 2H), 7.33 (d, 2H), 3.97 (br d, 1H); 3.66-3.47 (m, 8H), 3.27 (s, 3H), 3.20-3.18 8 (m, 4H), 3.08-2.99 (m, 3H), 2.30 (br d, 1H), 1.98 (q, 1H).

### Beispiel 493

### {3-(5-Methyl-1,3,4-thiadiazol-2-yl)-5-[4-(trifluormethoxy)phenyl]piperidin-1-yl} (morpholin-4-yl)methanon [racemisches cis-Isomer]

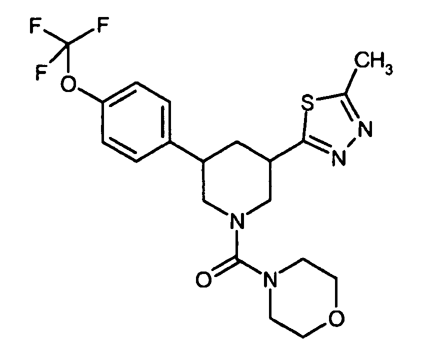

Nach der Allgemeinen Methode 9 wurden 106 mg (0.231 mmol) der Verbindung aus Beispiel 121A und 187 mg (0.462 mmol) Lawessons-Reagenz umgesetzt. Ausbeute: 10 mg (10% d. Th.)

LC-MS (Methode 2B): Rₜ = 1.18 min; MS (ESIpos): m/z = 457 [M+H]⁺;

¹H-NMR (400 MHz, DMSO-d₆): δ = 7.47 (d, 2H), 7.33 (d, 2H), 3.97 (br d, 1H); 3.67-3.45 (m, 6H), 3.20-3.18 (m, 4H), 3.09-2.96 (m, 3H), 2.70 (s, 3H), 2.28 (br d, 1H), 1.97 (q, 1H).

### Beispiel 494

### Morpholin-4-yl {3-[5-(propan-2-yl)-1,3,4-thiadiazol-2-yl]-5-[4-(trifluormethoxy)phenyl]piperidin-1-yl}methanon [racemisches cis-Isomer]

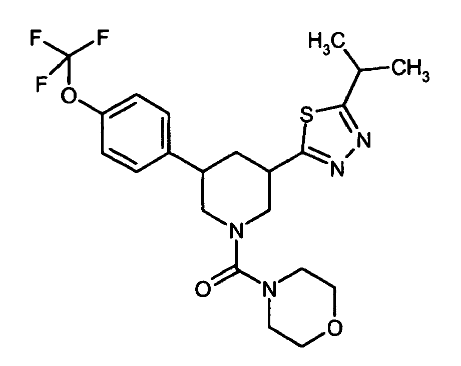

Nach der Allgemeinen Methode 9 wurden 79 mg (0.155 mmol) der Verbindung aus Beispiel 122A und 126 mg (0.310 mmol) Lawessons-Reagenz umgesetzt. Ausbeute: 17 mg (22% d. Th.)

LC-MS (Methode 2B): Rₜ = 1.32 min; MS (ESIpos): m/z = 485 [M+H]⁺;

¹H-NMR (400 MHz, DMSO-d₆): δ = 7.47 (d, 2H), 7.33 (d, 2H), 3.98 (br d, 1H); 3.64 (d, 2H), 3.58-3.54 (m, 3H), 3.50 (tt, 1H), 3.45-3.38 (m, 1H), 3.20-3.18 (m, 4H), 3.07-2.95 (m, 3H), 2.30 (d, 1H), 1.98 (q, 1H), 1.34 (d, 6H).

### Beispiel 495

### Morpholin-4-yl{3-(5-phenyl-1,3,4-thiadiazol-2-yl)-5-[4-(trifluormethoxy)phenyl]piperidin-1-yl}-methanon [racemisches cis-Isomer]

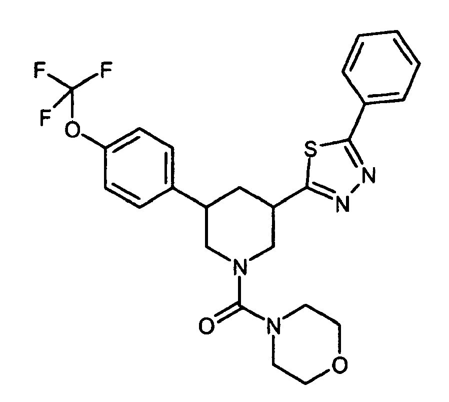

Nach der Allgemeinen Methode 9 wurden 132 mg (0.239 mmol) der Verbindung aus Beispiel 123A und 193 mg (0.478 mmol) Lawessons-Reagenz umgesetzt. Ausbeute: 40 mg (32% d. Th.)

LC-MS (Methode 2B): Rₜ = 1.40 min; MS (ESIpos): m/z = 519 [M+H]⁺;

¹H-NMR (400 MHz, DMSO-d₆): δ = 7.95 (dd, 2H), 7.59-7.53 (m, 3H), 7.50 (d, 2H), 7.34 (d, 2H), 4.07 (br d, 1H), 3.75-3.57 (m, 6H), 3.23-3.20 (m, 4H), 3.13 (t, 1H), 3.07-3.00 (m, 2H), 2.38 (br d, 1H), 2.06 (q, 1H).

### Beispiel 496

### (3-Methoxyazetidin-1-yl){(3-[3-(propan-2-yl)-1,2,4-oxadiazol-5-yl]-5-[4-(trifluormethyl)phenyl] piperidin-1-yl}methanon [racemisches cis-Isomer]

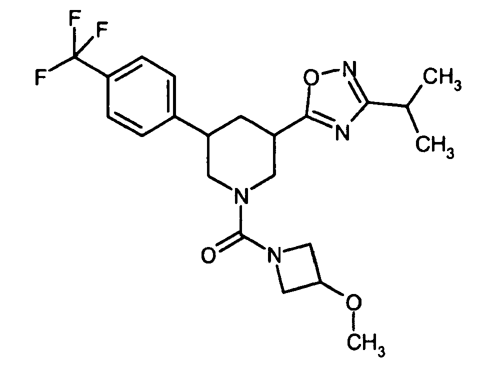

Nach der Allgemeinen Methode 1 wurden 250 mg (0.647 mmol) 1-[(3-Methoxyazetidin-1-yl)carbonyl]-5-[4-(trifluormethyl)phenyl]piperidin-3-carbonsäure (Beispiel 103A) und 72.7 mg (0.712 mmol) *N'-*Hydroxy-2-methylpropanimidamid umgesetzt. Ausbeute: 207 mg (69% d. Th.).

LC-MS (Methode 9B): Rₜ = 1.21 min; MS (ESIpos): m/z = 453 [M+H]⁺;

¹H-NMR (400 MHz, DMSO-d₆): δ = 7.70 (d, 2H), 7.57 (d, 2H), 4.20-4.05 (m, 4H), 3.81-3.71 (m, 3H), 3.36-3.27 (verdeckt, 1H), 3.18 (s, 3H), 3.10-2.93 (m, 3H), 2.31 (s, 3H), 2.31 (br d, 1H), 2.03 (dd, 1H), 1.26 (d, 6H).

### Beispiel 497

### {3-(3-Ethyl-1,2,4-oxadiazol-5-yl)-5-[4-(trifluormethyl)phenyl]piperidin-1-yl}(3-methoxyazetidin-1-yl)methanon [racemisches cis-Isomer]

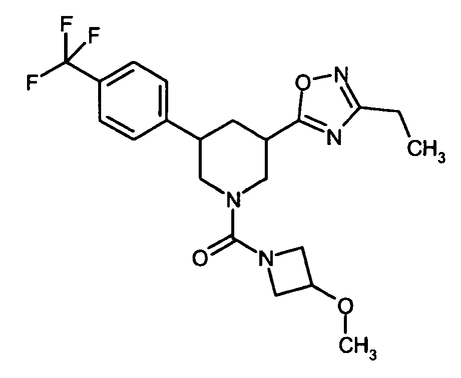

Nach der Allgemeinen Methode 1 wurden 250 mg (0.647 mmol) 1-[(3-Methoxyazetidin-1-yl)carbonyl]-5-[4-(trifluormethyl)phenyl]piperidin-3-carbonsäure (Beispiel 103A) und 88.7 mg (0.712 mmol) *N'*-Hydroxypropanimidamid Hydrochlorid umgesetzt. Ausbeute: 151 mg (52% d. Th.).

LC-MS (Methode 9B): Rₜ = 1.1 min; MS (ESIpos): m/z = 439 [M+H]⁺.

¹H-NMR (400 MHz, DMSO-d₆): δ = 7.70 (d, 2H), 7.57 (d, 2H), 4.20-4.05 (m, 4H), 3.81-3.71 (m, 3H), 3.37-3.28 (verdeckt, 1H), 3.19 (s, 3H), 3.08-2.94 (m, 3H), 2.71 (q, 2H), 2.32 (br d, 1H), 2.03 (dd, 1H), 1.22 (t, 3H).

### Beispiel 498

### 3-Methoxypiperidin-1-yl){3-[3-(propan-2-yl)-1,2,4-oxadiazol-5-yl]-5-[4-(trifluormethyl)phenyl]-piperidin-1-yl}methanon [Diastereomerengemisch]

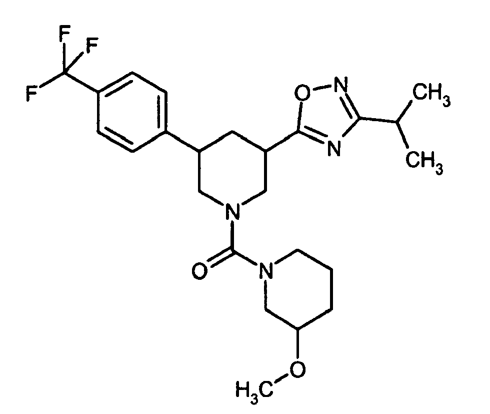

Nach der Allgemeinen Methode 1 wurden 250 mg (0.434 mmol, 72% Reinheit) 1-[(3-Methoxypiperidin-1-yl)carbonyl]-5-[4-(trifluormethyl)phenyl]piperidin-3-carbonsäure (Beispiel 207A) und 48.8 mg (0.712 mmol) *N'*-Hydroxy-2-methylpropanimidamid umgesetzt. Ausbeute: 120 mg (57% d. Th.).

LC-MS (Methode 2B): Rₜ = 1.44 min; MS (ESIpos): m/z = 481 [M+H]⁺;

¹H-NMR (400 MHz, DMSO-d₆): δ = 7.71 (d, 2H), 7.57 (d, 2H), 3.96 (br d, 1H), 3.67-3.56 (m, 1H), 3.52-3.32 (m, 2H), 3.27-3.17 (m, 5H), 3.12-2.91 (m, 6H), 2.34 (br d, 1H), 2.01 (dd, 1H), 1.90-1.78 (m, 1H), 1.51-1.30 (m, 2H), 1.25 (d, 6H).

### Beispiel 499

### {3-(3-Ethyl-1,2,4-oxadiazol-5-yl)-5-[4-(trifluormethyl)phenyl]piperidin-1-yl}(3-methoxypiperidin-1-yl)methanon [Diastereomerengemisch]

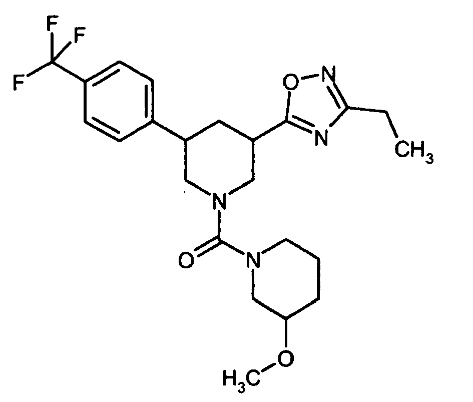

Nach der Allgemeinen Methode 1 wurden 250 mg (0.434 mmol, 72% Reinheit) 1-[(3-Methoxypiperidin-1-yl)carbonyl]-5-[4-(trifluormethyl)phenyl]piperidin-3-carbonsäure (Beispiel 207A) und 59.5 mg (0.712 mmol) *N'-*Hydroxypropanimidamid Hydrochlorid umgesetzt. Ausbeute: 93.9 mg (46% d. Th.).

LC-MS (Methode 2B): Rₜ = 1.44 min; MS (ESIpos): m/z = 481 [M+H]⁺;

¹H-NMR (400 MHz, DMSO-d₆): δ = 7.70 (d, 2H), 7.57 (d, 2H), 3.96 (br d, 1H), 3.66-3.58 (m, 1H), 3.52-3.30 (m, 3H), 3.27-3.18 (m, 5H), 3.11-2.91 (m, 5H), 2.71 (q, 2H), 2.34 (br d, 1H), 2.09-1.96 (m, 1H), 1.94-1.79 (m, 1H), 1.71-1.62 (m, 1H), 1.50-1.32 (m, 1H), 1.22 (t, 6H).

### Beispiel 500

### {3-[3-(2-Methoxyethyl)-1,2,4-oxadiazol-5-yl]-5-[4-(trifluormethyl)phenyl]piperidin-1-yl}(3-methoxypiperidin-1-yl)methanon [Diastereomerengemisch]

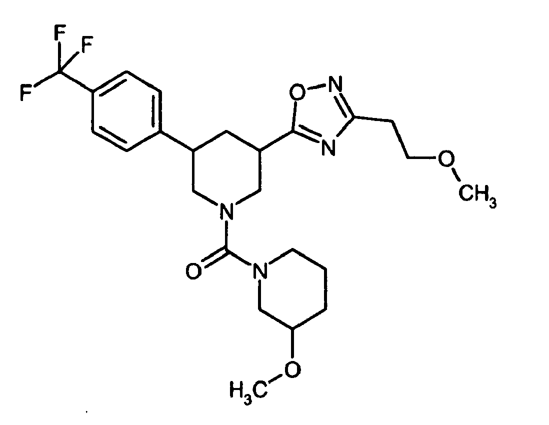

Nach der Allgemeinen Methode 1 wurden 250 mg (0.434 mmol, 72% Reinheit) 1-[(3-Methoxypiperidin-1-yl)carbonyl]-5-[4-(trifluormethyl)phenyl]piperidin-3-carbonsäure (Beispiel 207A) und 75.3 mg (0.478 mmol, 75% Reinheit) *N'-*Hydroxy-3-methoxypropanimidamid umgesetzt. Ausbeute: 105 mg (48% d. Th.).

LC-MS (Methode 2B): Rₜ = 1.30 min; MS (ESIpos): m/z = 497 [M+H]⁺;

¹H-NMR (400 MHz, DMSO-d₆): δ = 7.71 (d, 2H), 7.57 (d, 2H), 3.96 (br d, 1H), 3.68 (t, 2H), 3.65-3.58 (m, 1H), 3.53-3.35 (m, 2H), 3.27-3.17 (m, 8H), 3.12-2.95 (m, 5H), 2.93 (t, 2H), 2.35 (br d, 1H), 2.01 (dd, 1H), 1.90-1.78 (m, 1H), 1.72-1.63 (m, 1H), 1.51-1.30 (m, 2H).

### Beispiel 501

### 4-({3-[3-(2-Methoxyethyl)-1,2,4-oxadiazol-5-yl]-5-[4-(trifluormethyl)phenyl]piperidin-1-yl}carbonyl)piperazin-2-on [racemisches cis-Isomer]

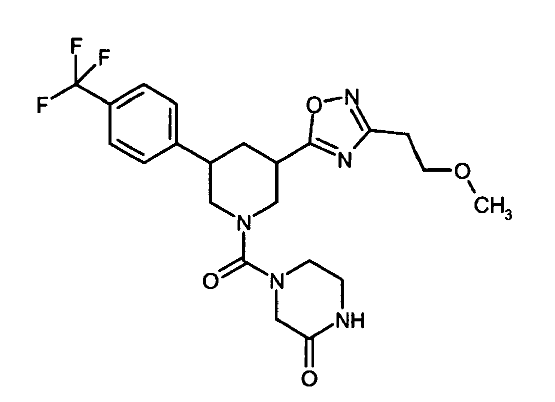

Nach der Allgemeinen Methode 1 wurden 250 mg (0.626 mmol) 1-[(3-Oxopiperazin-1-yl)carbonyl]-5-[4-(trifluormethyl)phenyl]piperidin-3-carbonsäure (Beispiel 206A) und 108 mg (0.689 mmol, 75% Reinheit) *N'-*Hydroxy-3-methoxypropanimidamid umgesetzt. Ausbeute: 72.0 mg (23% d. Th.).

LC-MS (Methode 3B): Rₜ = 1.68 min; MS (ESIpos): m/z = 482 [M+H]⁺;

¹H-NMR (400 MHz, DMSO-d₆): δ = 7.98 (s, 1H), 7.70 (d, 2H), 7.58 (d, 2H), 4.00 (br d, 1H), 3.78 (s, 2H), 3.71-3.60 (m, 3H), 3.49-3.38 (m, 3H), 3.26-3.18 (m, 5H), 3.13-3.00 (m, 3H), 2.93 (t, 2H), 2.34 (br d, 1H), 2.02 (dd, 1H).

### Beispiel 502

### 4-({3-(3-Ethyl-1,2,4-oxadiazol-5-yl)-5-[4-(trifluormethyl)phenyl]piperidin-1-yl}carbonyl)-piperazin-2-on [racemisches cis-Isomer]

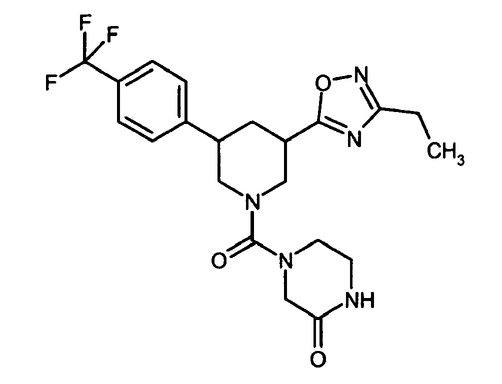

Nach der Allgemeinen Methode 1 wurden 250 mg (0.626 mmol) 1-[(3-Oxopiperazin-1-yl)carbonyl]-5-[4-(trifluormethyl)phenyl]piperidin-3-carbonsäure (Beispiel 206A) und 85.8 mg (0.689 mmol) *N'-*Hydroxypropanimidamid Hydrochlorid umgesetzt. Ausbeute: 127 mg (44% d. Th.).

LC-MS (Methode 2B): Rₜ = 1.14 min; MS (ESIpos): m/z = 452 [M+H]⁺;

¹H-NMR (400 MHz, DMSO-d₆): δ = 7.97 (s, 1H), 7.70 (d, 2H), 7.58 (d, 2H), 4.00 (br d, 1H), 3.78 (s, 2H), 3.65 (br d, 1H), 3.48-3.38 (m, 3H), 3.24-3.17 (m, 2H), 3.13-3.00 (m, 3H), 2.71 (q, 2H), 2.34 (br d, 1H), 2.02 (dd, 1H), 1.22 (t, 3H).

### Beispiel 503

### 4-({3-[3-(Propan-2-yl)-1,2,4-oxadiazol-5-yl]-5-[4-(trifluormethyl)phenyl]piperidin-1-yl}carbonyl)-piperazin-2-on [racemisches cis-Isomer]

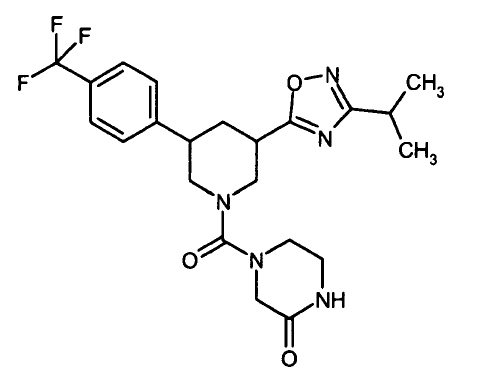

Nach der Allgemeinen Methode 1 wurden 250 mg (0.626 mmol) 1-[(3-Oxopiperazin-1-yl)carbonyl]-5-[4-(trifluormethyl)phenyl]piperidin-3-carbonsäure (Beispiel 206A) und 70.3 mg (0.689 mmol) *N'-*Hydroxy-2-methylpropanimidamid umgesetzt. Ausbeute: 190 mg (62% d. Th.).

LC-MS (Methode 9B): Rₜ = 1.07 min; MS (ESIpos): m/z = 466 [M+H]⁺;

¹H-NMR (400 MHz, DMSO-d₆): δ = 7.97 (s, 1H), 7.70 (d, 2H), 7.57 (d, 2H), 4.00 (br d, 1H), 3.77 (s, 2H), 3.64 (br d, 1H), 3.47-3.35 (m, 3H), 3.24-3.17 (m, 2H), 3.13-3.00 (m, 4H), 2.34 (br d, 1H), 2.02 (dd, 1H), 1.25 (d, 6H).

### Beispiel 504

### (2,2-Dimethylmorpholin-4-yl){3-[3-(propan-2-yl)-1,2,4-oxadiazol-5-yl]-5-[4-(trifluormethyl)-phenyl] piperidin-1-yl}methanon [racemisches cis-Isomer]

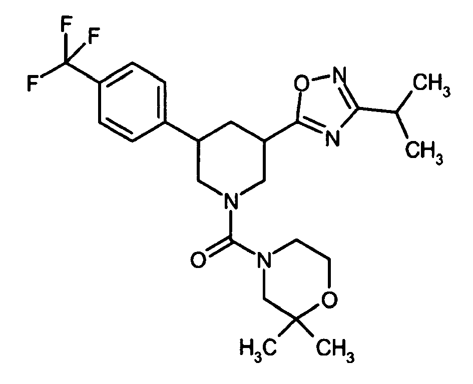

Nach der Allgemeinen Methode 1 wurden 250 mg (0.410 mmol, 68% Reinheit) 1-[(2,2-Dimethylmorpholin-4-yl)carbonyl]-5-[4-(trifluormethyl)phenyl]piperidin-3-carbonsäure (Beispiel 208A) und 46.1 mg (0.451 mmol) *N'-*Hydroxy-2-methylpropanimidamid umgesetzt. Ausbeute: 118 mg (59% d. Th.).

LC-MS (Methode 2B): Rₜ = 1.44 min; MS (ESIpos): m/z = 481 [M+H]⁺;

¹H-NMR (400 MHz, DMSO-d₆): δ = 7.71 (d, 2H), 7.57 (d, 2H), 4.00 (br d, 1H), 3.66-3.57 (m, 3H), 3.41 (tt, 1H), 3.18-3.12 (m, 2H), 3.10-3.00 (m, 6H), 2.34 (br d, 1H), 2.04 (dd, 1H), 1.25 (d, 6H), 1.14 (s, 6H).

### Beispiel 505

### (2,2-Dimethylmorpholin-4-yl){3-[3-(2-methoxyethyl)-1,2,4-oxadiazol-5-yl]-5-[4-(trifluormethyl)-phenyl]piperidin-1-yl}methanon [racemisches cis-Isomer]

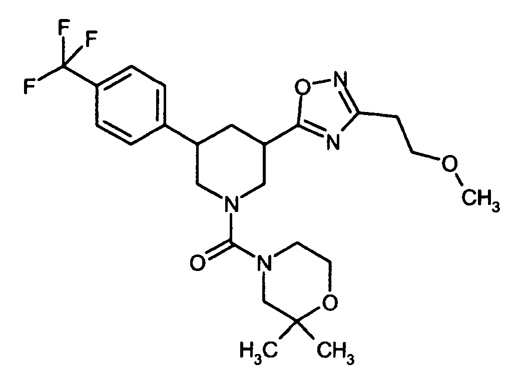

Nach der Allgemeinen Methode 1 wurden 250 mg (0.410 mmol, 68% Reinheit) 1-[(2,2-Dimethylmorpholin-4-yl)carbonyl]-5-[4-(trifluormethyl)phenyl]piperidin-3-carbonsäure (Beispiel 208A) und 71.1 mg (0.451 mmol, 75% Reinheit) *N'-*Hydroxy-3-methoxypropanimidamid umgesetzt. Ausbeute: 103 mg (50% d. Th.).

LC-MS (Methode 2B): Rₜ = 1.31 min; MS (ESIpos): m/z = 497 [M+H]⁺;

¹H-NMR (400 MHz, DMSO-d₆): δ = 7.71 (d, 2H), 7.57 (d, 2H), 4.00 (br d, 1H), 3.67 (t, 2H), 3.65-3.57 (m, 3H), 3.43 (tt, 1H), 3.23 (s, 3H), 3.18-3.12 (m, 2H), 3.10-2.98 (m, 5H), 2.93 (t, 2H), 2.35 (br d, 1H), 2.04 (dd, 1H), 1.14 (s, 6H).

### Beispiel 506

### (2,2-Dimethylmorpholin-4-yl){3-(3-ethyl-1,2,4-oxadiazol-5-yl)-5-[4-(trifluormethyl)phenyl]-piperidin-1-yl}methanon [racemisches cis-Isomer]

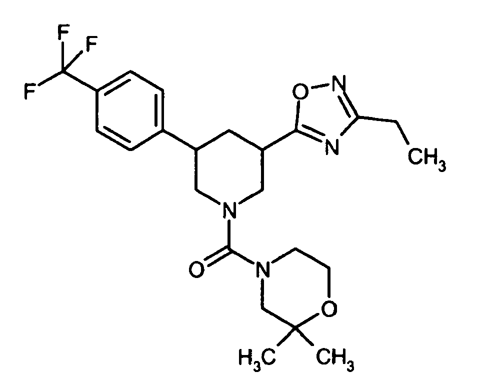

Nach der Allgemeinen Methode 1 wurden 250 mg (0.410 mmol, 68% Reinheit) 1-[(2,2-Dimethylmorpholin-4-yl)carbonyl]-5-[4-(trifluormethyl)phenyl]piperidin-3-carbonsäure (Beispiel 208A) und 56.2 mg (0.451 mmol) *N'-*Hydroxypropanimidamid Hydrochlorid umgesetzt. Ausbeute: 80.6 mg (42% d. Th.).

LC-MS (Methode 2B): Rₜ = 1.36 min; MS (ESIpos): m/z = 467 [M+H]⁺;

¹H-NMR (400 MHz, DMSO-d₆): δ = 7.71 (d, 2H), 7.57 (d, 2H), 4.00 (br d, 1H), 3.68-3.57 (m, 3H), 3.41 (tt, 1H), 3.19-3.12 (m, 2H), 3.10-2.96 (m, 5H), 2.71 (q, 2H), 2.34 (br d, 1H), 2.04 (dd, 1H), 1.22 (t, 3H), 1.14 (s, 6H).

### Beispiel 507

### 4-({3-(3-Ethyl-1,2,4-oxadiazol-5-yl)-5-[4-(trifluormethyl)phenyl]piperidin-1-yl}carbonyl)-1-methylpiperazin-2-on [racemisches cis-Isomer]

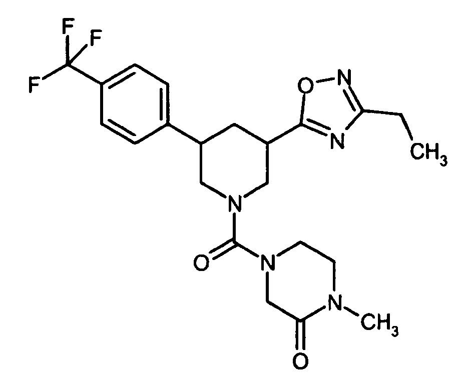

Nach der Allgemeinen Methode 1 wurden 250 mg (0.605 mmol) 1-[(4-Methyl-3-oxopiperazin-1-yl)carbonyl]-5-[4-(trifluormethyl)phenyl]piperidin-3-carbonsäure (Beispiel 209A) und 82.9 mg (0.665 mmol) *N'*-Hydroxypropanimidamid Hydrochlorid umgesetzt. Ausbeute: 110 mg (39% d. Th.).

LC-MS (Methode 9B): Rₜ = 1.05 min; MS (ESIpos): m/z = 466 [M+H]⁺;

¹H-NMR (400 MHz, DMSO-d₆): δ = 7.70 (d, 2H), 7.57 (d, 2H), 4.01 (br d, 1H), 3.81 (s, 2H), 3.65 (br d, 1H), 3.50-3.37 (m, 3H), 3.14-3.03 (m, 3H), 2.84 (s, 3H), 2.71 (q, 2H), 2.36-2.30 (m, 1H), 2.01 (dd, 1H), 1.22 (t, 3H).

### Beispiel 508

### (4-Aminopiperidin-1-yl){3-[3-(propan-2-yl)-1,2,4-oxadiazol-5-yl]-5-[4-(trifluormethoxy)-phenyl]piperidin-1-yl}methanon Hydrochlorid [racemisches cis-Isomer]

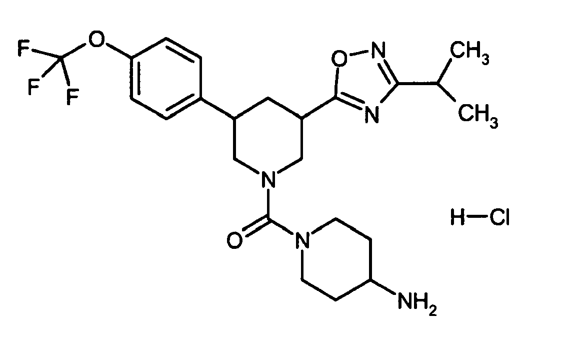

Zu einer Lösung von 50 mg (0.087 mmol) der Verbindung aus Beispiel 210 A in 1.13 ml Dioxan wurden 0.31 ml (1.27 mmol) einer 4N Chlorwasserstoff-Dioxan-Lösung hinzugegeben. Das Reaktionsgemisch wurde 20 Stunden bei Raumtemperatur gerührt. Anschließend wurde die Mischung im Vakuum zur Trockne eingeengt und getrocknet bis zur Gewichtskonstanz. Ausbeute: 18 mg (36% d. Th).

LC-MS (Methode 2B): Rₜ = 1.10 min; MS (ESIpos): m/z = 482 [M+H-HCl]⁺;

¹H-NMR (400 MHz, DMSO-d₆): δ = 7.94 (bs, 2H), 7.47 (d, 2H), 7.33 (d, 2H), 3.94 (br d, 1H), 3.65 (d, 2H), 3.55 (d, 1H), 3.23-3.10 (m, 1H), 3.10-2.94 (m, 4H), 2.84 (dd, 2H), 2.31 (d, 1H), 1.98 (dd, 1H), 1.88 (br d, 2H), 1.43 (dd, 2H), 1.25 (d, 6H).

### Beispiel 509

### (4-Aminopiperidin-1-yl){3-[3-(2-methoxyethyl)-1,2,4-oxadiazol-5-yl]-5-[4-(trifluormethoxy)-phenyl]piperidin-1-yl}methanon [racemisches cis-Isomer]

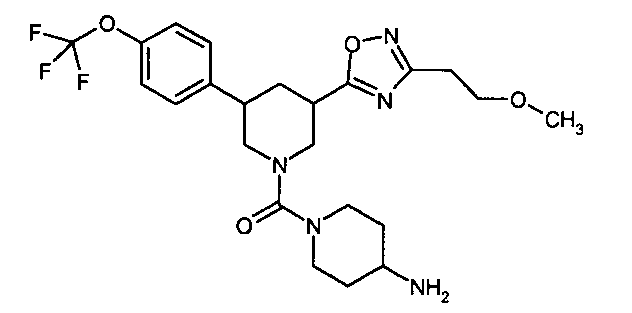

Zu einer Lösung von 50 mg (0.082 mmol) der Verbindung aus Beispiel 211A in 0.4 ml Dioxan wurden 0.255 ml (1.022 mmol) einer 4N Chlorwasserstoff-Dioxan-Lösung hinzugegeben. Das Reaktionsgemisch wurde 20 Stunden bei Raumtemperatur gerührt. Anschließend wurde die Mischung im Vakuum zur Trockne eingeengt und getrocknet bis zur Gewichtskonstanz. Ausbeute: 43 mg (97% d. Th).

LC-MS (Methode 9B): Rₜ = 0.85 min; MS (ESIpos): m/z = 499 [M+H-HCl]⁺;

¹H-NMR (400 MHz, DMSO-d₆): δ = 7.95 (bs, 1H), 7.93 (bs, 1H), 7.47 (d, 2H), 7.33 (d, 2H), 3.95 (br d, 1H), 3.65-3.64 (m, 4H), 3.55 (d, 1H), 3.45-3.36 (m, 1H), 3.23(s, 3H), 3.21-3.13 (br s, 1H), 3.07-2.92 (m, 5H), 2.84 (dd, 2H), 2.33 (br d, 1H), 1.98 (dd, 1H), 1.88 (br d, 2H), 1.43 (dd, 2H).

### Beispiel 510

### (4-Aminopiperidin-1-yl){3-(3-methyl-1,2,4-oxadiazol-5-yl)-5-[4-(trifluormethoxy)phenyl]-piperidin-1-yl}methanon Hydrochlorid [racemisches cis-Isomer]

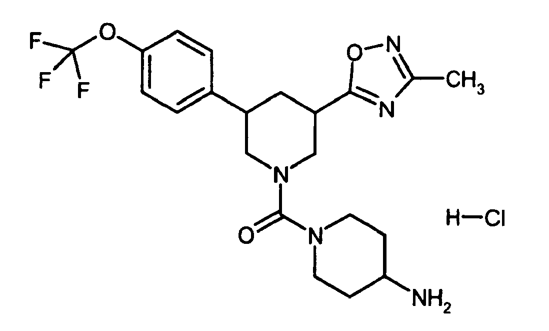

Zu einer Lösung von 70 mg (0.126 mmol) der Verbindung aus Beispiel 212A in 0.6 ml Dioxan wurden 0.365 ml (1.463 mmol) einer 4N Chlorwasserstoff-Dioxan-Lösung hinzugegeben. Das Reaktionsgemisch wurde 20 Stunden bei Raumtemperatur gerührt. Anschließend wurde die Mischung im Vakuum zur Trockne eingeengt und getrocknet bis zur Gewichtskonstanz. Ausbeute: 60 mg (97% d. Th).

LC-MS (Methode 9B): Rₜ = 0.83 min; MS (ESIpos): m/z = 454 [M+H-HCl]⁺;

¹H-NMR (400 MHz, DMSO-d₆): δ = 7.86 (bs, 2H), 7.46 (d, 2H), 7.33 (d, 2H), 3.96 (br d, 1H), 3.64 (d, 2H), 3.56 (d, 1H), 3.18 (bs, 1H), 3.09-2.97 (m, 3H), 2.84 (dd, 2H), 2.33 (s, 3H), 2.31 (d, 1H), 1.98 (dd, 1H), 1.87 (br d, 2H), 1.42 (dd, 2H).

### Beispiel 511

### (3-Hydroxyazetidin-1-yl){3-[3-(2-methoxyethyl)-1,2,4-oxadiazol-5-yl]-5-[4-(trifluormethoxy)-phenyl]piperidin-1-yl}methanon [racemisches cis-Isomer]

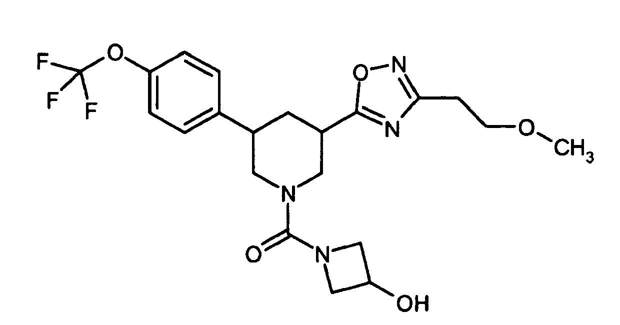

Nach der Allgemeinen Methode 2 wurden 150 mg (0.324 mmol) der Verbindung aus Beispiel 110A und 77 mg (0.649 mmol) *N'*-Hydroxy-3-methoxypropanimidamid umgesetzt. Ausbeute: 23 mg (15% d. Th.).

LC-MS (Methode 5B): Rₜ = 2.06 min; MS (ESIpos): m/z = 471 [M+H]⁺;

¹H-NMR (400 MHz, DMSO-d₆): δ = 7.46 (d, 2H), 7.33 (d, 2H), 5.58 (d, 1H), 4.42-4.35 (m, 1H), 4.16 (br d, 1H), 4.09 (dd, 2H), 3.75-3.66 (m, 5H), 3.23 (s, 3H), 3.04-2.89 (m, 5H), 2.30 (br d, 1H), 1.99 (dd, 1H).

### Beispiel 512

### (3-Hydroxypyrrolidin-1-yl){3-[3-(methoxymethyl)-1,2,4-oxadiazol-5-yl]-5-[4-(trifluormethoxy)-phenyl]piperidin-1-yl}methanon [Diastereomerengemisch]

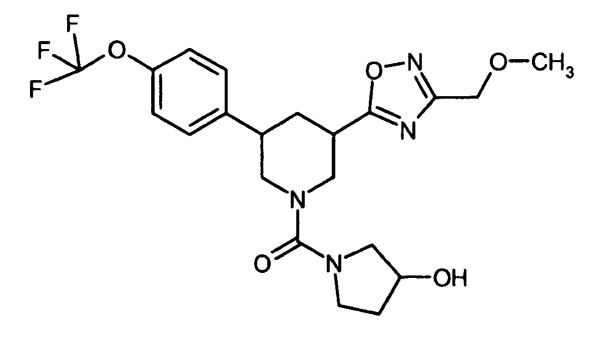

Nach der Allgemeinen Methode 2 wurden 200 mg (0. 497 mmol) der Verbindung aus Beispiel 112A und 77 mg (0.746 mmol) *N'-*Hydroxy-2-methoxyethanimidamid umgesetzt. Ausbeute: 55 mg (24% d. Th.).

LC-MS (Methode 2B): Rₜ = 1.14 min; MS (ESIpos): m/z = 471 [M+H]⁺;

¹H-NMR (400 MHz, DMSO-d₆): δ = 7.47 (d, 2H), 7.32 (d, 2H), 4.86 (d, 1H), 4.53 (s, 2H), 4.24-4.19 (m, 1H), 4.10-4.04 (m, 1H), 3.70 (dd, 1H), 3.51-3.44 (m, 3H), 3.33 (s, 3H), 3.11 (d, 1H), 3.05-2.84 (m, 3H), 2.34 (br d, 1H), 2.04-1.94 (m, 1H), 1.86-1.78 (m, 1H), 1.75-1.66 (m, 1H).

### Beispiel 513

### {3-(3-Cyclobutyl-1,2,4-oxadiazol-5-yl)-5-[4-(trifluormethoxy)phenyl]piperidin-1-yl}(morpholin-4-yl)methanon [racemisches cis-Isomer]

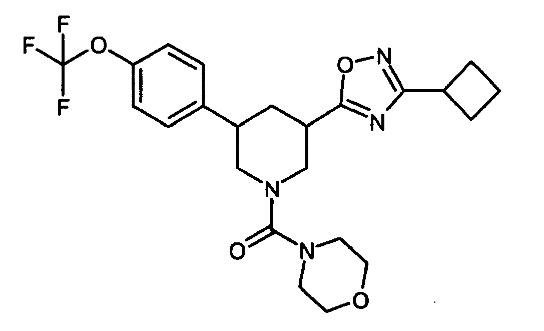

Nach der Allgemeinen Methode 2 wurden 150 mg (0.373 mmol) der Verbindung aus Beispiel 44A und 85 mg (0.746 mmol) *N'*-Hydroxycyclobutancarboximidamid umgesetzt. Ausbeute: 58 mg (48% d. Th.)

LC-MS (Methode 2B): Rₜ = 1.42 min; MS (ESIpos): m/z = 481 [M+H]⁺;

¹H-NMR (400 MHz, DMSO-d₆): δ = 7.47 (d, 2H), 7.33 (d, 2H), 3.99 (br d, 1H), 3.69-3.60 (m, 2H), 3.59-3.53 (m, 4H), 3.41 (tt, 1H), 3.23-3.16 (m, 4H), 3.08-2.97 (m, 3H), 2.37-2.20 (m, 5H), 2.11-1.88 (m, 3H).

### Beispiel 514

### {3-(3-Cyclobutyl-1,2,4-oxadiazol-5-yl)-5-[4-(trifluormethyl)phenyl]piperidin-1-yl}(morpholin-4-yl)methanon [racemisches cis-Isomer]

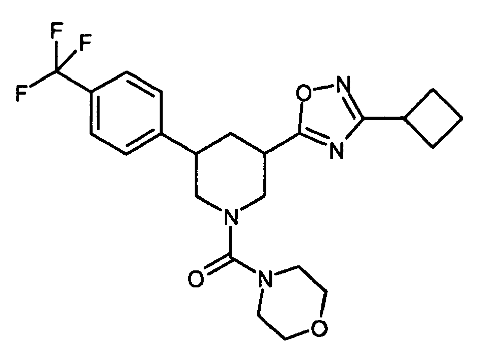

Nach der Allgemeinen Methode 2 wurden 150 mg (0.388 mmol) der Verbindung aus Beispiel 81A und 88 mg (0.776 mmol) *N'-*Hydroxycyclobutancarboximidamid umgesetzt. Ausbeute: 124 mg (65% d. Th.)

LC-MS (Methode 2B): Rₜ = 1.39 min; MS (ESIpos): m/z = 465 [M+H]⁺;

¹H-NMR (400 MHz, DMSO-d₆): δ = 7.70 (d, 2H), 7.58 (d, 2H), 3.99 (br d, 1H), 3.68-3.60 (m, 2H), 3.58-3.53 (m, 4H), 3.40 (tt, 1H), 3.23-3.17 (m, 4H), 3.10-2.99 (m, 3H), 2.37-2.23 (m, 5H), 2.09-1.92 (m, 3H).

### Beispiel 515

### {3-[3-(3-Fluorphenyl)-1,2,4-oxadiazol-5-yl]-5-[4-(trifluormethyl)phenyl]piperidin-1-yl}-(morpholin-4-yl)methanon [racemisches cis-Isomer]

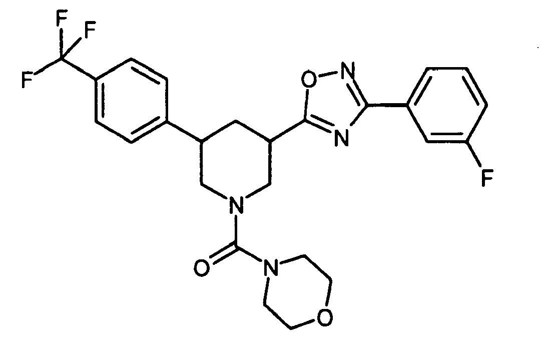

Nach der Allgemeinen Methode 1 wurden 250 mg (ca. 0.52 mmol) der Verbindung aus Beispiel 49A und 89 mg (0.58 mmol) 3-Fluor-*N'-*hydroxybenzencarboximidamid umgesetzt. Ausbeute: 26 mg (10% d. Th.).

LC-MS (Methode 1B): Rₜ = 2.81 min; MS (ESIpos): m/z = 505 [M+H]⁺;

¹H-NMR (400 MHz, DMSO-d₆): δ = 7.87 (d, 1H), 7.76 (d, 1H), 7.71 (d, 2H), 7.64 (d, 1H), 7.60 (d, 2H), 7.47 (td, 1H), 4.10 (brd, 1H), 3.65 (d, 1H), 3.60-3.50 (m, 5H), 3.25-3.20 (m, 4H), 3.16 (t, 1H), 3.10-3.06 (m, 2H), 2.42 (br d, 1H), 2.10 (q, 1H).

### Beispiel 516

### 1-({3-(3-Cyclopropyl-1,2,4-oxadiazol-5-yl)-5-[4-(trifluormethoxy)phenyl]piperidin-1-yl}-carbonyl)piperidin-4-carbonitril [racemisches cis-Isomer]

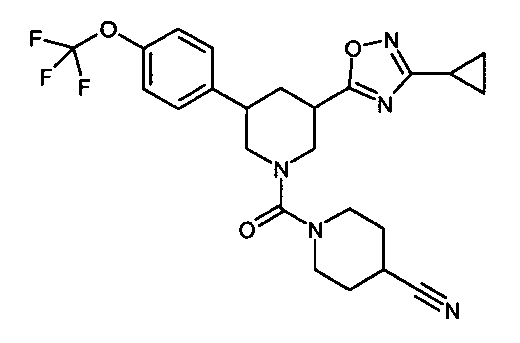

Nach der Allgemeinen Methode 2 wurden 100 mg (ca. 0.153 mmol) der Verbindung aus Beispiel 108A und 30 mg (0.306 mmol) *N'*-Hydroxycyclopropancarboximidamid umgesetzt. Ausbeute: 312 mg (41 % d. Th.)

LC-MS (Methode 3B): Rₜ = 2.29 min; MS (ESIpos): m/z = 490 [M+H]⁺;

¹H-NMR (400 MHz, DMSO-d₆): δ = 7.47 (d, 2H), 7.32 (d, 2H), 3.92 (br d, 1H), 3.58 (d, 1H), 3.39-3.33 (m, 3H), 3.10-2.91 (m, 6H), 2.27 (br d, 1H), 2.07-2.14 (m, 1H), 1.92 (dd, 1H), 1.90-1.80 (m, 2H), 1.71-1.63 (m, 2H), 1.08-1.03 (m, 2H), 0.90-0.86 (m, 2H).

### Beispiel 517

### 1-({3-[3-(2-Methoxyethyl)-1,2,4-oxadiazol-5-yl]-5-[4-(trifluormethoxy)phenyl]piperidin-1-yl}-carbonyl)piperidin-4-carbonitril [racemisches cis-Isomer]

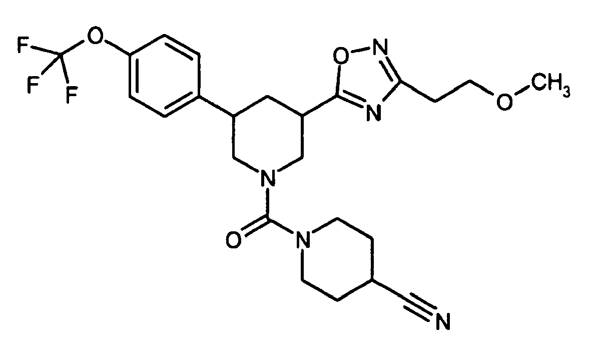

Nach der Allgemeinen Methode 2 wurden 100 mg (ca. 0.153 mmol) der Verbindung aus Beispiel 108A und 36 mg (0.306 mmol) *N'*-Hydroxy-3-methoxypropanimidamid umgesetzt. Ausbeute: 14 mg (18% d. Th.)

LC-MS (Methode 3B): Rₜ = 2.11 min; MS (ESIpos): m/z = 508 [M+H]⁺;

¹H-NMR (400 MHz, DMSO-d₆): δ = 7.47 (d, 2H), 7.33 (d, 2H), 3.96 (br d, 1H), 3.67 (t, 2H), 3.57 (d, 1H), 3.41-3.35 (m, 2H), 3.23 (s, 3H), 3.10-2.96 (m, 6H), 2.95 (t, 2H), 2.31 (br d, 1H), 1.96 (dd, 1H), 1.90-1.80 (m, 2H), 1.74-1.63 (m, 2H).

### Beispiel 518

### 1-({3-(3-Methyl-1,2,4-oxadiazol-5-yl)-5-[4-(trifluormethoxy)phenyl]piperidin-1-yl}carbonyl)-piperidin-4-carbonitril [racemisches cis-Isomer]

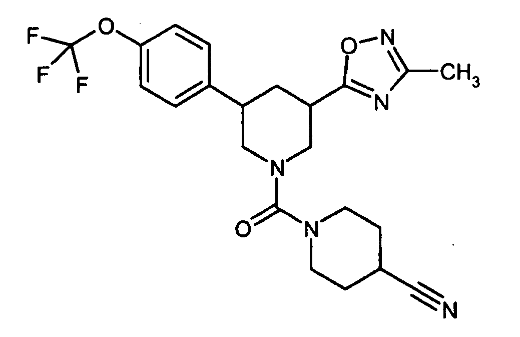

Nach der Allgemeinen Methode 2 wurden 100 mg (ca. 0.153 mmol) der Verbindung aus Beispiel 108A und 22 mg (0.306 mmol) *N'*-Hydroxyethanimidamid umgesetzt. Ausbeute: 12 mg (17% d. Th.)

LC-MS (Methode 3B): Rₜ = 2.11 min; MS (ESIpos): m/z = 464 [M+H]⁺;

¹H-NMR (400 MHz, DMSO-d₆): δ = 7.46 (d, 2H), 7.33 (d, 2H), 3.95 (br d, 1H), 3.58 (d, 1H), 3.41-3.35 (m, 3H), 3.10-2.91 (m, 6H), 2.31 (s, 3H), 2.30 (br d, 1H), 1.96 (q, 1H), 1.90-1.82 (m, 2H), 1.73-1.63 (m, 2H).

### Beispiel 519

### 1-({3-(3-Ethyl-1,2,4-oxadiazol-5-yl)-5-[4-(trifluormethoxy)phenyl]pipendin-1-yl} carbonyl)-piperidin-4-carbonitril [racemisches cis-Isomer]

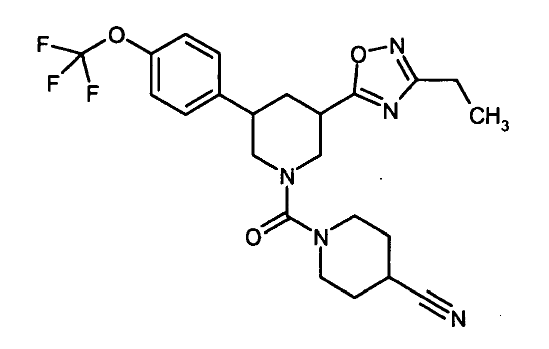

Nach der Allgemeinen Methode 2 wurden 100 mg (ca. 0.153 mmol) der Verbindung aus Beispiel 108A und 27 mg (0.306 mmol) *N'*-Hydroxypropanimidamid umgesetzt. Ausbeute: 7 mg (10% d. Th.)

LC-MS (Methode 3B): Rₜ = 2.24 min; MS (ESIpos): m/z = 478 [M+H]⁺;

¹H-NMR (400 MHz, DMSO-d₆): δ = 7.46 (d, 2H), 7.33 (d, 2H), 3.95 (br d, 1H), 3.57 (d, 1H), 3.39-3.34 (m, 3H), 3.10-2.93 (m, 6H), 2.71 (q, 2H), 2.30 (br d, 1H), 1.96 (q, 1H), 1.90-1.83 (m, 2H), 1.73-1.63 (m, 2H); 1.22 (t, 3H).

### Beispiel 520

### 1-({3-[3-(Propan-2-yl)-1,2,4-oxadiazol-5-yl]-5-[4-(trifluormethoxy)phenyl]piperidin-1-yl}-carbonyl)piperidin-4-carbonitril [racemisches cis-Isomer]

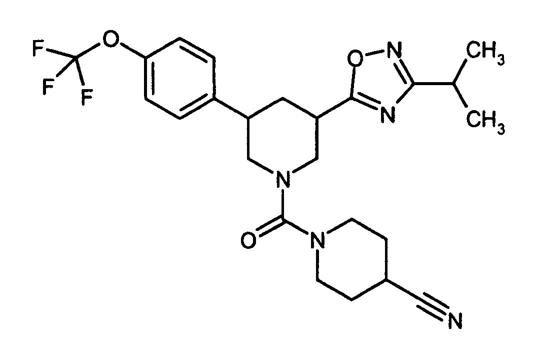

Nach der Allgemeinen Methode 2 wurden 100 mg (ca. 0.153 mmol) der Verbindung aus Beispiel 108A und 31 mg (0.306 mmol) *N'*-Hydroxy-2-methylpropanimidamid umgesetzt. Ausbeute: 60 mg (80% d. Th.)

LC-MS (Methode 3B): Rₜ = 2.38 min; MS (ESIpos): m/z = 492 [M+H]⁺;

¹H-NMR (400 MHz, DMSO-d₆): δ = 7.47 (d, 2H), 7.32 (d, 2H), 3.96 (br d, 1H), 3.57 (d, 1H), 3.42-3.35 (m, 4H), 3.11-2.94 (m, 6H), 2.31 (br d, 1H), 2.01-1.91 (m, 1H), 1.90-1.80 (m, 1H), 1.67 (q, 1H), 1.25 (d, 6H).

### Beispiel 521

### 3-[3-(3-Fluorphenyl)-1,2,4-oxadiazol-5-yl]-N-(2-methoxyethyl)-5-[4-(trifluormethyl)phenyl]-piperidin-1-carboxamid [racemisches cis-Isomer]

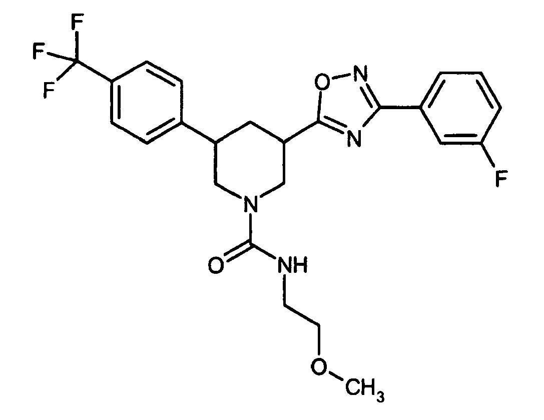

Nach der Allgemeinen Methode 2 wurden 100 mg (0.267 mmol) der Verbindung aus Beispiel 105A und 77 mg (0.497 mmol) 3-Fluor-*N'*-hydroxybenzolcarboximidamid umgesetzt. Ausbeute: 64 mg (55% d. Th.)

LC-MS (Methode 3B): Rₜ = 2.39 min; MS (ESIpos): m/z = 493 [M+H]⁺;

¹H-NMR (400 MHz, DMSO-d₆): δ = 7.87 (d, 1H), 7.76 (dd, 1H), 7.72 (d, 2H), 7.64 (dd, 1H), 7.59 (d, 2H), 7.46 (dd, 1H), 6.84 (dd, 1H), 4.53 (br d, 1H), 4.11 (dd, 1H), 3.36 (t, 3H), 3.25 (s, 3H), 3.27-3.20 (m, 2H), 3.11-2.99 (m, 1H), 2.95 (dd, 2H), 2.42 (br d, 1H), 2.08 (dd, 1H).

### Beispiel 522

### N-(2-Methoxyethyl)-3-[3-(2-methoxyethyl)-1,2,4-oxadiazol-5-yl]-5-[4-(trifluormethyl)phenyl]-piperidin-1-carboxamid [racemisches cis-Isomer]

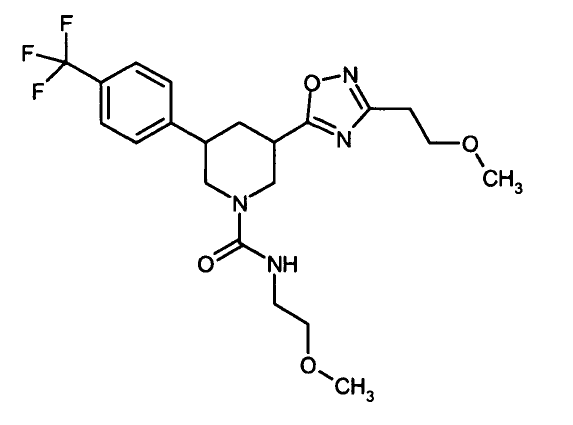

Nach der Allgemeinen Methode 2 wurden 100 mg (0.267 mmol) der verbindung aus Beispiel 105A und 78 mg (0.497 mmol) *N'*-Hydroxy-3-methoxypropanimidamid umgesetzt. Ausbeute: 22 mg (17% d. Th.)

LC-MS (Methode 3B): Rₜ = 1.87 min; MS (ESIpos): m/z = 456 [M+H]⁺;

¹H-NMR (400 MHz, DMSO-d₆): δ = 7.70 (d, 2H), 7.57 (d, 2H), 6.82 (dd, 1H), 4.43 (br d, 1H), 4.08 (d, 1H), 3.68 (t, 2H), 3.24 (s, 3H), 3.23 (s, 3H), 3.26-3.18 (m, 3H), 2.98-2.89 (m, 5H), 2.32 (br d, 1H), 1.98 (dd, 1H).

### Beispiel 523

### N-(2-Methoxyethyl)-3-[3-(propan-2-yl)-1,2,4-oxadiazol-5-yl]-5-[4-(trifluormethyl)phenyl]-piperidin-1-carboxamid [racemisches cis-Isomer]

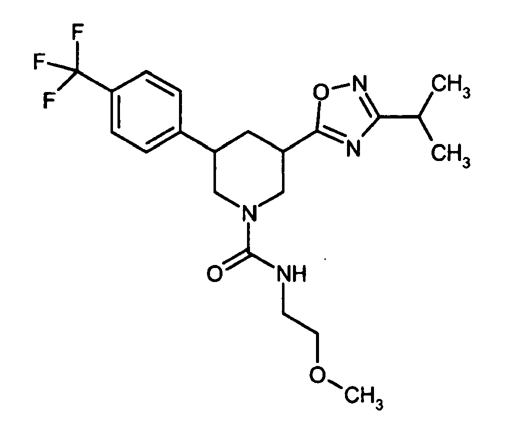

Nach der Allgemeinen Methode 2 wurden 100 mg (0.267 mmol) der Verbindung aus Beispiel 105A und 51 mg (0.497 mmol) *N'*-Hydroxy-2-methylpropanimidamid umgesetzt. Diastereomerentrennung von 83 mg des cis-/trans-Isomerengemisches nach Methode 14C ergab 60.5 mg der Titelverbindung und 6.7 mg des trans-Isomers.

LC-MS (Methode 2B): Rₜ = 1.34 min; MS (ESIpos): m/z = 441 [M+H]⁺;

¹H-NMR (400 MHz, DMSO-d₆): δ = 7.70 (d, 2H), 7.57 (d, 2H), 6.80 (dd, 1H), 4.42 (br d, 1H), 4.08 (dd, 1H), 3.24 (s, 3H), 3.27-3.18 (m, 3H), 3.09-3.02 (m, 1H), 2.97 (br d, 1H), 2.91 (br d, 2H), 2.31 (br d, 1H), 1.98 (dd, 1H), 1.25 (d, 6H).

### Beispiel 524

### 3-(3-Cyclopropyl-1,2,4-oxadiazol-5-yl)-N-(2-methoxyethyl)-5-[4-(trifluormethyl)phenyl]piperidin-1-carboxamid [racemisches cis-Isomer]

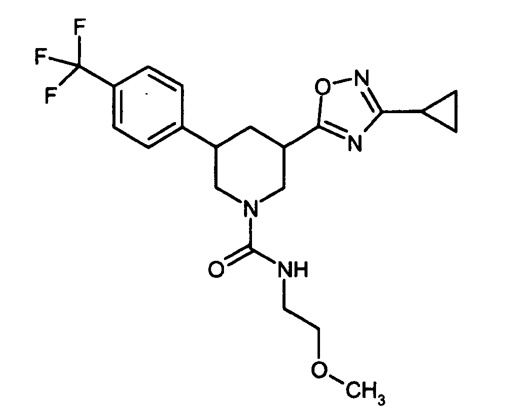

Nach der Allgemeinen Methode 2 wurden 100 mg (0.267 mmol) der Verbindung aus Beispiel 105A und 54 mg (0.534 mmol) *N'-*Hydroxycyclopropancarboximidamid umgesetzt. Diastereomerentrennung von 64 mg des cis-/trans-Isomerengemisches nach Methode 14C ergab 45.4 mg der Titelverbindung und 5.5 mg des trans-Isomers.

LC-MS (Methode 2B): Rₜ = 1.30 min; MS (ESIpos): m/z = 439 [M+H]⁺;

¹H-NMR (400 MHz, DMSO-d₆): δ = 7.70 (d, 2H), 7.56 (d, 2H), 6.79 (dd, 1H), 4.40 (br d, 1H), 4.07 (dd, 1H), 3.24 (s, 3H), 3.22-3.15 (m, 3H), 2.94-2.86 (m, 3ist), 2.38 (br d, 1H), 2.14-2.08 (m, 1H), 1.94 (dd, 1H), 1.08-1.03 (m, 2H), 0.90-0.86 (m, 2H).

### Beispiel 525

### Morpholin-4-yl{3-[3-(pyridin-2-yl)-1,2,4-oxadiazol-5-yl]-5-[4(trifluormethyl)phenyl]piperidin-1-yl}methanon [racemisches cis-Isomer]

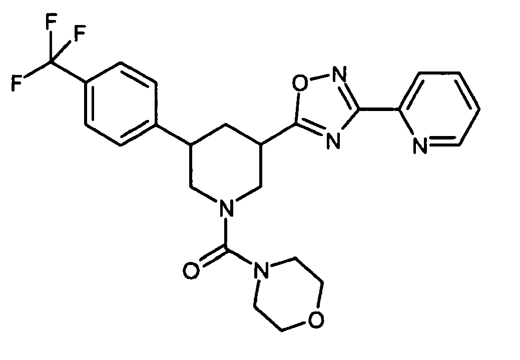

Nach der Allgemeinen Methode 1 wurden 250 mg (ca. 0.520 mmol) der Verbindung aus Beispiel 49A und 79 mg (0.580 mmol) *N'-*Hydroxypyridin-2-carboximidamid (Beispiel 69A) umgesetzt. Ausbeute: 49 mg (19% d. Th.).

LC-MS (Methode 3B): Rₜ = 1:99 min; MS (ESIpos): m/z = 488 [M+H]⁺;

¹H-NMR (400 MHz, DMSO-d₆): δ = 8.76 (dm, 1H), 8.08 (d, 1H), 8.02 (td, 1H), 7.73 (d, 2H), 7.62-7.59 (m, 3H), 4.10 (br d, 1H), 3.66 (d, 1H), 3.60-3.50 (m, 5H), 3.25-3.21 (m, 4H), 3.16-3.02 (m, 3H), 2.43 (br d, 1H), 2.12 (q, 1H).

### Beispiel 526

### {3-(3-Ethyl-1,2,4-oxadiazol-5-yl)-5-[4-(trifluormethoxy)phenyl]piperidin-1-yl}(4-hydroxypiperidin-1-yl)methanon

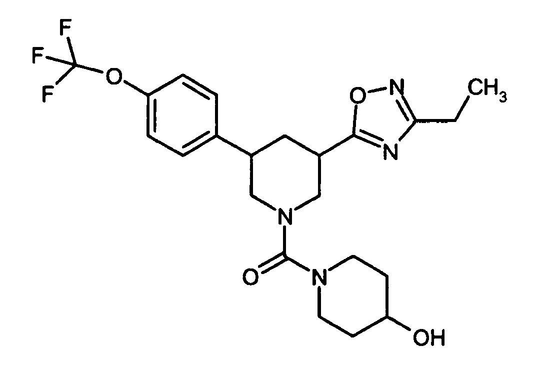

Nach der Allgemeinen Methode 2 wurden 200 mg (0.480 mmol) der Verbindung aus Beispiel 63A und 85 mg (0.960 mmol) *N'-*Hydroxyethanimidamid umgesetzt. Ausbeute: 101 mg (43% d. Th.)

LC-MS (Methode 9B): Rₜ = 1.09 min; MS (ESIpos): m/z = 469 [M+H]⁺;

¹H-NMR (400 MHz, DMSO-d₆): δ = 7.46 (d, 2H), 7.33 (d, 2H), 4.69 (d, 1H), 3.93 (br d, 1H), 3.66-3.59 (m, 1H), 3.56 (d, 1H), 3.52-3.44 (m, 2H), 3.38 (tt, 1H), 3.03-2.88 (m, 5H), 2.70 (dd, 2H), 2.31 (d, 1H), 1.97 (dd, 1H), 1.71 (d, 2H), 1.36-1.25 (dd, 2H), 1.22 (t, 3H).

### Beispiel 527

### 1-({3-[3-(Methoxymethyl)-1,2,4-oxadiazol-5-yl]-5-[4-(trifluormethoxy)phenyl]piperidin-1-yl}-carbonyl)piperidin-4-carbonitril [enantiomerenreines cis-Isomer]

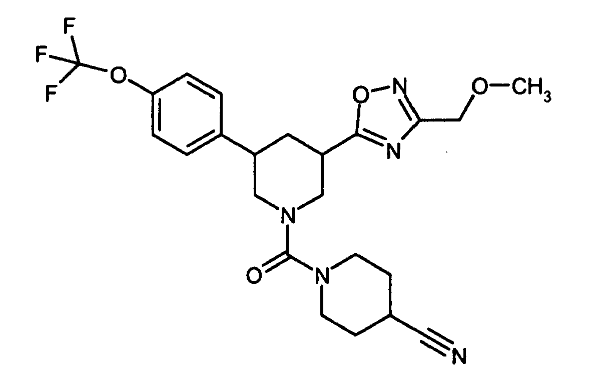

Nach der Allgemeinen Methode 2 wurden 100 mg (ca. 0.153 mmol) der Verbindung aus Beispiel 108A und 79 mg (0.306 mmol) *N'*-Hydroxymethylmethoxycarboxamidin umgesetzt. Enantiomerentrennung von 21 mg des Racemates nach Methode 15D ergab 4 mg der Titelverbindung aus Beispiel 527 und 4 mg der Titelverbindung aus Beispiel 528.

HPLC (Methode 12E): Rₜ = 7.81 min, >99.0% ee;

LC-MS (Methode 9B): Rₜ = 1.13 min; MS (ESIpos): m/z = 494 [M+H]⁺.

### Beispiel 528

### 1-({3-[3-(Methoxymethyl)-1,2,4-oxadiazol-5-yl]-5-[4-(trifluormethoxy)phenyl]piperidin-1-yl}-carbonyl)piperidin-4-carbonitril [enantiomerenreines cis-Isomer]

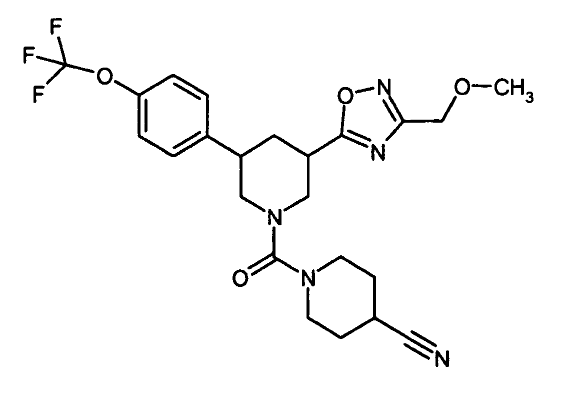

Nach der Allgemeinen Methode 2 wurden 100 mg (ca. 0.153 mmol) der Verbindung aus Beispiel 108A und 79 mg (0.306 mmol) *N'*-Hydroxymethylmethoxycarboxamidin umgesetzt. Enantiomerentrennung von 21 mg des Racemates nach Methode 15D ergab 4 mg der Titelverbindung aus Beispiel 527 und 4 mg der Titelverbindung aus Beispiel 528.

HPLC (Methode 12E): Rₜ = 9.75 min, >99.0% ee;

LC-MS (Methode 9B): Rₜ = 1.13 min; MS (ESIpos): m/z = 494 [M+H]⁺.

### Beispiel 529

### (3-Hydroxyazetidin-1-yl){3-[3-(propan-2-yl)-1,2,4-oxadiazol-5-yl]-5-[4-(trifluormethoxy)phenyl]-piperidin-1-yl}methanon [racemisches cis-Isomer]

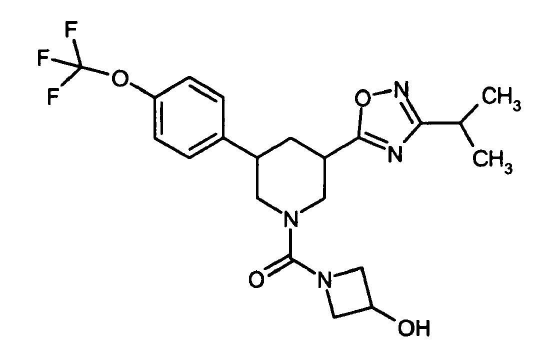

Nach der Allgemeinen Methode 2 wurden 185 mg (ca. 0.400 mmol) der Verbindung aus Beispiel 110A und 55 mg (0.600 mmol) *N'*-Hydroxy-2-methylpropanimidamid umgesetzt. Ausbeute: 28 mg (16% d. Th.)

LC-MS (Methode 9B): Rₜ = 1.12 min; MS (ESIpos): m/z = 455 [M+H]⁺;

¹H-NMR (400 MHz, DMSO-d₆): δ = 7.46 (d, 2H), 7.33 (d, 2H), 5.58 (d, 1H), 4.46-4.35 (m, 1H), 4.15 (br d, 1H), 4.08 (dd, 2H), 3.75-3.66 (m, 3H), 3.08-2.87 (m, 4H), 2.29 (br d, 1H), 1.99 (dd, 1H), 1.25 (d, 6H).

### Beispiel 530

### (3-Hydroxyazetidin-1-yl){3-(3-methyl-1,2,4-oxadiazol-5-yl)-5-[4-(trifluomethoxy)phenyl]-piperidin-1-yl}methanon [racemisches cis-Isomer]

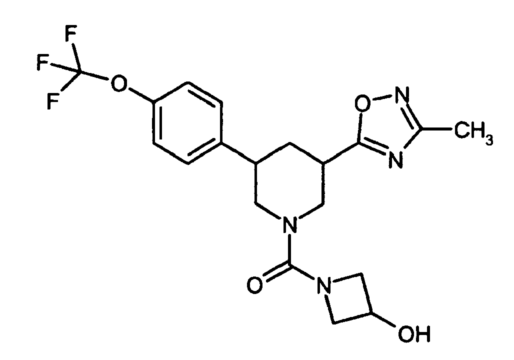

Nach der Allgemeinen Methode 2 wurden 185 mg (ca. 0.400 mmol) der Verbindung aus Beispiel 110A und 55 mg (0.600 mmol) *N'*-Hydroxyethanimidamid umgesetzt. Ausbeute: 16 mg (9 % d. Th.)

LC-MS (Methode 9B): Rₜ = 0.99 min; MS (ESIpos): m/z = 427 [M+H]⁺;

¹H-NMR (400 MHz, DMSO-d₆): δ = 7.45 (d, 2H), 7.33 (d, 2H), 5.58 (d, 1H), 4.42-4.35 (m, 1H), 4.13 (br d, 1H), 4.09 (dd, 2H), 3.76-3.67 (m, 3H), 3.03-2.91 (m, 3H), 2.33 (s, 3H), 2.29 (br d, 1H), 1.98 (dd, 1H).

### Beispiel 531

### (3-Hydroxypyrrolidin-1-yl){3-(3-methyl-1,2,4-oxadiazol-5-yl)-5-[4-(trifluormethoxy)phenyl]-piperidin-1-yl}methanon [enantiomerenreines cis-Isomer]

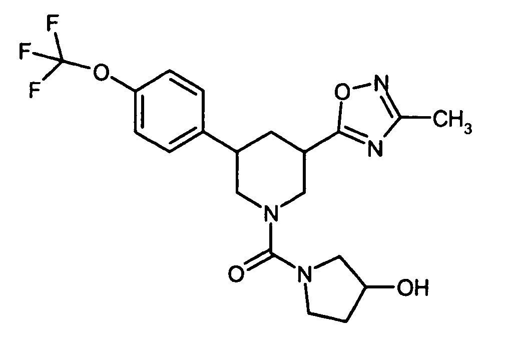

Enantiomerentrennung von 62 mg des Racemates aus Beispiel 422 nach Methode 13D ergab 12 mg der Titelverbindung aus Beispiel 531, 13 mg der Titelverbindung aus Beispiel 532, 13 mg der Titelverbindung aus Beispiel 533 und 13 mg der Titelverbindung aus Beispiel 534.

HPLC (Methode 10E): Rₜ = 9.45 min, >98.0% ee;

LC-MS (Methode 9B): Rₜ = 1.01 min; MS (ESIpos): m/z = 441 [M+H]⁺.

### Beispiel 532

### (3-Hydroxypyrrolidin-1-yl){3-(3-methyl-1,2,4-oxadiazol-5-yl)-5-[4-(trifluormethoxy)phenyl]-piperidin-1-yl}methanon [enantiomerenreines cis-Isomer]

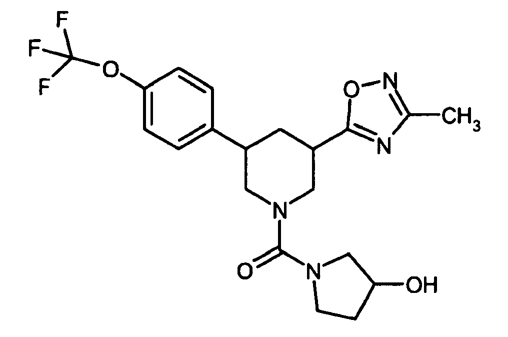

Enantiomerentrennung von 62 mg des Racemates aus Beispiel 422 nach Methode 13D ergab 12 mg der Titelverbindung aus Beispiel 531, 13 mg der Titelverbindung aus Beispiel 532, 13 mg der Titelverbindung aus Beispiel 533 und 13 mg der Titelverbindung aus Beispiel 534.

HPLC (Methode 10E): Rₜ = 17.75 min, >98.0% ee;

LC-MS (Methode 9B): Rₜ = 1.01 min; MS (ESIpos): m/z = 441 [M+H]⁺.

### Beispiel 533

### (3-Hydroxypyrrolidin-1-yl){3-(3-methyl-1,2,4-oxadiazol-5-yl)-5-[4-(trifluormethoxy)phenyl]-piperidin-1-yl}methanon [enantiomerenreines cis-Isomer]

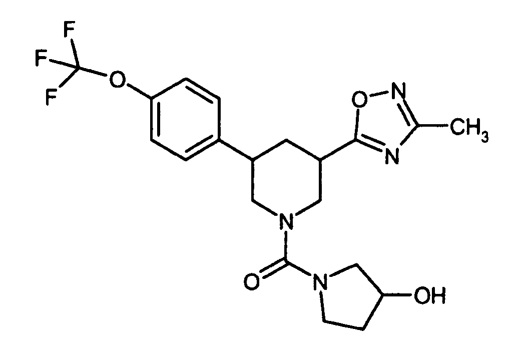

Enantiomerentrennung von 62 mg des Racemates aus Beispiel 422 nach Methode 13D ergab 12 mg der Titelverbindung aus Beispiel 531, 13 mg der Titelverbindung aus Beispiel 532, 13 mg der Titelverbindung aus Beispiel 533 und 13 mg der Titelverbindung aus Beispiel 534.

HPLC (Methode 10E): Rₜ = 22.22 min, >98.0% ee;

LC-MS (Methode 9B): Rₜ = 1.01 min; MS (ESIpos): m/z = 441 [M+H]⁺.

### Beispiel 534

### (3-Hydroxypyrrolidin-1-yl){3-(3-methyl-1,2,4-oxadiazol-5-yl)-5-[4-(trifluormethoxy)phenyl]-piperidin-1-yl}methanon [enantiomerenreines cis-Isomer]

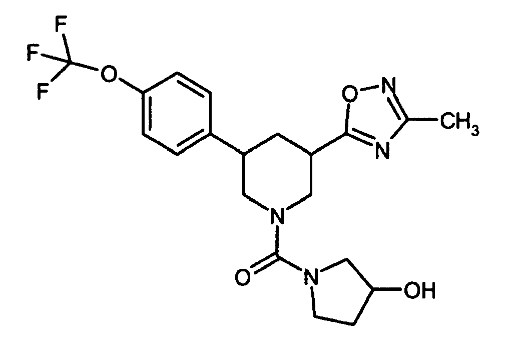

Enantiomerentrennung von 62 mg des Racemates aus Beispiel 422 nach Methode 13D ergab 12 mg der Titelverbindung aus Beispiel 531, 13 mg der Titelverbindung aus Beispiel 532, 13 mg der Titelverbindung aus Beispiel 533 und 13 mg der Titelverbindung aus Beispiel 534.

HPLC (Methode 10E): Rₜ = 42.69 min, >98.0% ee;

LC-MS (Methode 9B): Rₜ = 1.01 min; MS (ESIpos): m/z = 441 [M+H]⁺.

### Beispiel 535

### {3-(3-Ethyl-1,2,4-oxadiazol-5-yl)-5-[4-(trifluormethoxy)phenyl]piperidin-1-yl}(3-hydroxy-pyrrolidin-1-yl)methanon [enantiomerenreines cis-Isomer]

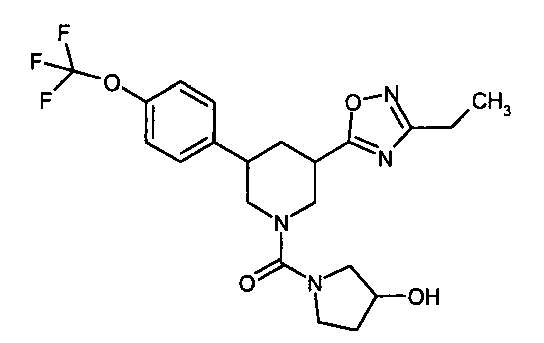

Enantiomerentrennung von 61 mg des Racemates aus Beispiel 421 nach Methode 13D ergab 13 mg der Titelverbindung aus Beispiel 535, 13 mg der Titelverbindung aus Beispiel 536, 13 mg der Titelverbindung aus Beispiel 537 und 13 mg der Titelverbindung aus Beispiel 538.

HPLC (Methode 10E): Rₜ = 7.49 min, >98.0% ee;

LC-MS (Methode 9B): Rₜ = 1.07 min; MS (ESIpos): m/z = 455 [M+H]⁺.

### Beispiel 536

### {3-(3-Ethyl-1,2,4-oxadiazol-5-yl)-5-[4-(trifluormethoxy)phenyl]piperidin-1-yl}(3-hydroxy-pyrrolidin-1-yl)methanon [enantiomerenreines cis-Isomer]

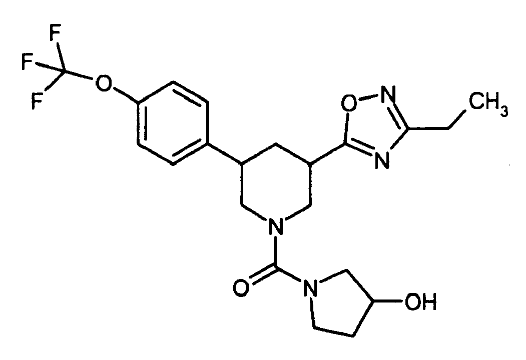

Enantiomerentrennung von 61 mg des Racemates aus Beispiel 421 nach Methode 13D ergab 13 mg der Titelverbindung aus Beispiel 535, 13 mg der Titelverbindung aus Beispiel 536, 13 mg der Titelverbindung aus Beispiel 537 und 13 mg der Titelverbindung aus Beispiel 538.

HPLC (Methode 10E): Rₜ = 13.79 min, >98.0% ee;

LC-MS (Methode 9B): Rₜ = 1.07 min; MS (ESIpos): m/z = 455 [M+H]⁺.

### Beispiel 537

### {3-(3-Ethyl-1,2,4-oxadiazol-5-yl)-5-[4-(trifluormethoxy)phenyl]piperidin-1-yl}(3-hydroxy-pyrrolidin-1-yl)methanon [enantiomerenreines cis-Isomer]

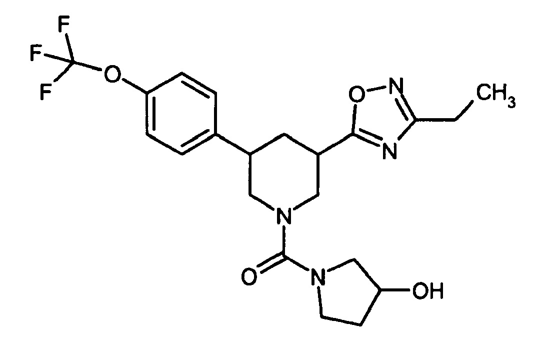

Enantiomerentrennung von 61 mg des Racemates aus Beispiel 421 nach Methode 13D ergab 13 mg der Titelverbindung aus Beispiel 535, 13 mg der Titelverbindung aus Beispiel 536, 13 mg der Titelverbindung aus Beispiel 537 und 13 mg der Titelverbindung aus Beispiel 538.

HPLC (Methode 10E): Rₜ = 17.99 min, >98.0% ee;

LC-MS (Methode 9B): Rₜ = 1.07 min; MS (ESIpos): m/z = 455 [M+H]⁺.

### Beispiel 538

### {3-(3-Ethyl-1,2,4-oxadiazol-5-yl)-5-[4-(trifluormethoxy)phenyl]piperidin-1-yl}(3-hydroxy-pyrrolidin-1-yl)methanon [enantiomerenreines cis-Isomer]

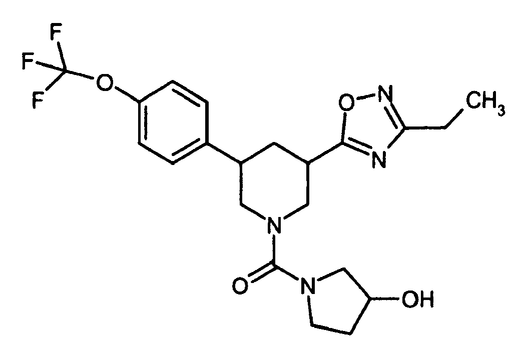

Enantiomerentrennung von 61 mg des Racemates aus Beispiel 421 nach Methode 13D ergab 13 mg der Titelverbindung aus Beispiel 535, 13 mg der Titelverbindung aus Beispiel 536, 13 mg der Titelverbindung aus Beispiel 537 und 13 mg der Titelverbindung aus Beispiel 538.

HPLC (Methode 10E): Rₜ = 23.43 min, >98.0% ee;

LC-MS (Methode 9B): Rₜ = 1.07 min; MS (ESIpos): m/z = 455 [M+H]⁺.

### Beispiel 539

### (3-Hydroxypyrrolidin-1-yl) {3-[3-(propan-2-yl)-1,2,4-oxadiazol-5-yl]-5-[4-(trifluormethoxy)-phenyl]piperidin-1-yl}methanon [enantiomerenreines cis-Isomer]

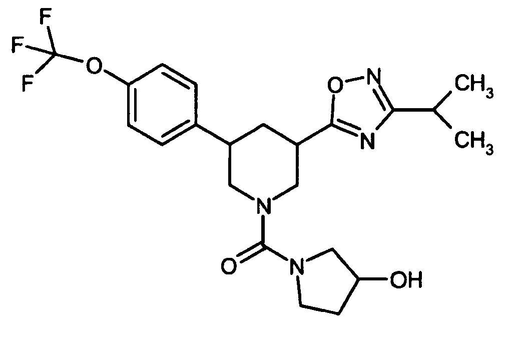

Enantiomerentrennung von 117 mg des Racemates aus Beispiel 418 nach Methode 13D ergab 26 mg der Titelverbindung aus Beispiel 539, 26 mg der Titelverbindung aus Beispiel 540, 25 mg der Titelverbindung aus Beispiel 541 und 24 mg der Titelverbindung aus Beispiel 542.

HPLC (Methode 10E): Rₜ = 5.64 min, >99.0% ee;

LC-MS (Methode 9B): Rₜ = 1.13 min; MS (ESIpos): m/z = 469 [M+H]⁺.

¹H-NMR (400 MHz, DMSO-d₆): δ = 7.47 (d, 2H), 7.32 (d, 2H), 4.88 (d, 1H), 4.25-4.18 (m, 1H), 4.04 (br d, 1H), 3.70 (br d, 1H), 3.45-3.88 (m, 2H), 3.13-2.98 (m, 4H), 2.88 (dd, 1H), 1.95 (dd, 1H), 1.88-1.78 (m, 1H), 1.76-1.68 (m, 1H), 1.25 (d, 6H).

### Beispiel 540

### (3-Hydroxypyrrolidin-1-yl) {3-[3-(propan-2-yl)-1,2,4-oxadiazol-5-yl]-5-[4-(trifluormethoxy)-phenyl]piperidin-1-yl}methanon [enantiomerenreines cis-Isomer]

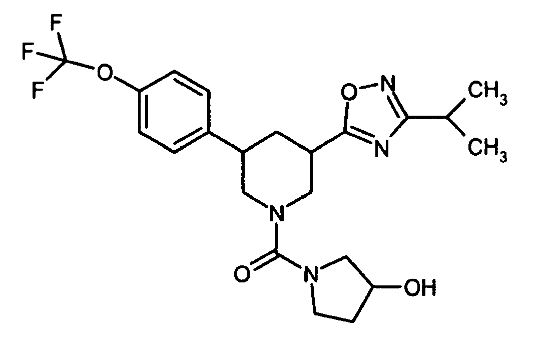

Enantiomerentrennung von 117 mg des Racemates aus Beispiel 418 nach Methode 13D ergab 26 mg der Titelverbindung aus Beispiel 539, 26 mg der Titelverbindung aus Beispiel 540, 25 mg der Titelverbindung aus Beispiel 541 und 24 mg der Titelverbindung aus Beispiel 542.

HPLC (Methode 10E): Rₜ = 8.47 min, >99.0% ee;

LC-MS (Methode 9B): Rₜ = 1.13 min; MS (ESIpos): m/z = 469 [M+H]⁺.

¹H-NMR (400 MHz, DMSO-d₆): δ = 7.47 (d, 2H), 7.32 (d, 2H), 4.88 (d, 1H), 4.24-4.18 (m, 1H), 4.05 (br d, 1H), 3.67 (br d, 1H), 3.52-3.37 (m, 4H), 3.12-2.89 (m, 5H), 2.32 (br d, 1H), 1.97 (dd, 1H), 1.87-1.77 (m, 1H), 1.76.1.67 (m, 1H), 1.26 (d, 6H).

### Beispiel 541

### (3-Hydroxypyrrolidin-1-yl){3-[3-(propan-2-yl)-1,2,4-oxadiazol-5-yl]-5-[4-(trifluormethoxy)-phenyl]piperidin-1-yl}methanon [enantiomerenreines cis-Isomer]

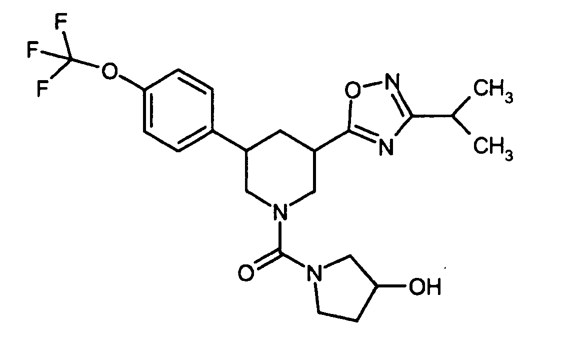

Enantiomerentrennung von 117 mg des Racemates aus Beispiel 418 nach Methode 13D ergab 26 mg der Titelverbindung aus Beispiel 539, 26 mg der Titelverbindung aus Beispiel 540, 25 mg der Titelverbindung aus Beispiel 541 und 24 mg der Titelverbindung aus Beispiel 542.

HPLC (Methode 10E): Rₜ = 12.34 min, >99.0% ee;

LC-MS (Methode 9B): Rₜ = 1.13 min; MS (ESIpos): m/z = 469 [M+H]⁺.

¹H-NMR (400 MHz, DMSO-d₆): δ = 7.47 (d, 2H), 7.32 (d, 2H), 4.88 (d, 1H), 4.24-4.18 (m, 1H), 4.05 (br d, 1H), 3.67 (br d, 1H), 3.52-3.37 (m, 4H), 3.12-2.89 (m, 5H), 2.32 (br d, 1H), 1.97 (dd, 1H), 1.87-1.77 (m, 1H), 1.76.1.67 (m, 1H), 1.26 (d, 6H).

### Beispiel 542

### (3-Hydroxypyrrolidin-1-yl) {3-[3-(propan-2-yl)-1,2,4-oxadiazol-5-yl]-5-[4-(trifluormethoxy)-phenyl]piperidin-1-yl}methanon [enantiomerenreines cis-Isomer]

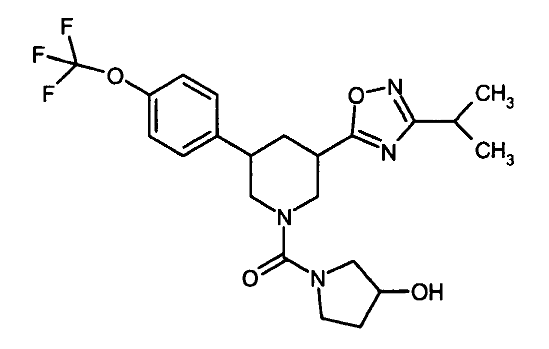

Enantiomerentrennung von 117 mg des Racemates aus Beispiel 418 nach Methode 13D ergab 26 mg der Titelverbindung aus Beispiel 539, 26 mg der Titelverbindung aus Beispiel 540, 25 mg der Titelverbindung aus Beispiel 541 und 24 mg der Titelverbindung aus Beispiel 542.

HPLC (Methode 10E): Rₜ = 13.96 min, >99.0% ee;

LC-MS (Methode 9B): Rₜ = 1.13 min; MS (ESIpos): m/z = 469 [M+H]⁺.

¹H-NMR (400 MHz, DMSO-d₆): δ = 7.47 (d, 2H), 7.32 (d, 2H), 4.88 (d, 1H), 4.25-4.18 (m, 1H), 4.04 (br d, 1H), 3.70 (br d, 1H), 3.45-3.88 (m, 2H), 3.13-2.98 (m, 4H), 2.88 (dd, 1H), 1.95 (dd, 1H), 1.88-1.78 (m, 1H), 1.76-1.68 (m, 1H), 1.25 (d, 6H).

### Beispiel 543

### {3-(3-Ethyl-1,2,4-oxadiazol-5-yl)-5-[4-(trifluormethoxy)phenyl]piperidin-1-yl}(4-hydroxypiperidin-1-yl)methanon [enantiomerenreines cis-Isomer]

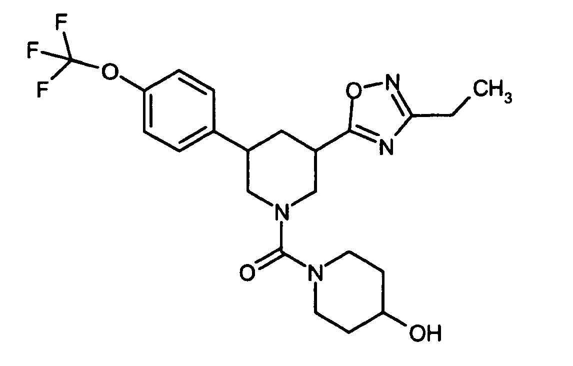

Enantiomerentrennung von 100 mg des Racemates aus Beispiel 526 nach Methode 16D ergab 35 mg der Titelverbindung aus Beispiel 543 und 36 mg der Titelverbindung aus Beispiel 544.

HPLC (Methode 16E): Rₜ = 5.02 min, >99.0% ee;

LC-MS (Methode 9B): Rₜ = 1.09 min; MS (ESIpos): m/z = 469 [M+H]⁺.

### Beispiel 544

### {3-(3-Ethyl-1,2,4-oxadiazol-5-yl)-5-[4-(trifluormethoxy)phenyl]piperidin-1-yl} (4-hydroxypiperidin-1-yl)methanon [enantiomerenreines cis-Isomer]

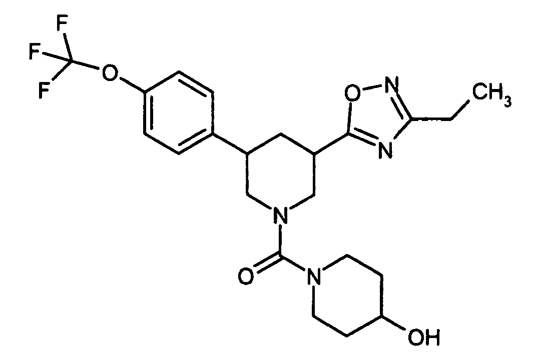

Enantiomerentrennung von 100 mg des Racemates aus Beispiel 526 nach Methode 16D ergab 35 mg der Titelverbindung aus Beispiel 543 und 36 mg der Titelverbindung aus Beispiel 544.

HPLC (Methode 16E): Rₜ = 11.23 min, >99.0% ee;

LC-MS (Methode 9B): Rₜ = 1.09 min; MS (ESIpos): m/z = 469 [M+H]⁺.

### Beispiel 545

### (3-Hydroxypyrrolidin-1-yl){3-[3-(2-methoxyethyl)-1,2,4-oxadiazol-5-yl]-5-[4-(trifluormethoxy)-phenyl]piperidin-1-yl}methanon [enantiomerenreines cis-Isomer]

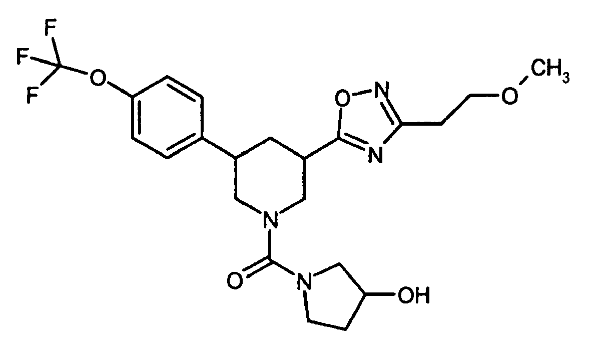

Enantiomerentrennung von 53 mg des Racemates aus Beispiel 420 nach Methode 13D ergab 13 mg der Titelverbindung aus Beispiel 545, 13 mg der Titelverbindung aus Beispiel 546, 11 mg der Titelverbindung aus Beispiel 547 und 11 mg der Titelverbindung aus Beispiel 548.

HPLC (Methode 16E): Rₜ = 6.45 min, >99.0% ee;

LC-MS (Methode 9B): Rₜ =1.02 min; MS (ESIpos): m/z = 485 [M+H]⁺.

¹H-NMR (400 MHz, DMSO-d₆): δ = 7.46 (d, 2H), 7.32 (d, 2H), 4.88 (d, 1H), 4.18-4.25 (m, 1H), 4.04 (br d, 1H), 3.72 (br d, 1H), 3.67 (t, 2H), 3.56-3.43 (m, 2H), 3.23 (s, 3H), 3.11 (br d, 1H), 3.05-2.97 (m, 2H), 2.93 (t, 2H), 2.87 (dd, 1H), 2.35 (brd, 1H), 1.97 (dd, 1H), 1.88-1.77 (m, 1H), 1.75-1.68 (m, 1H).

### Beispiel 546

### (3-Hydroxypyrrolidin-1-yl) {3-[3-(2-methoxyethyl)-1,2,4-oxadiazol-5-yl]-5-[4-(trifluormethoxy)-phenyl]piperidin-1-yl}methanon [enantiomerenreines cis-Isomer]

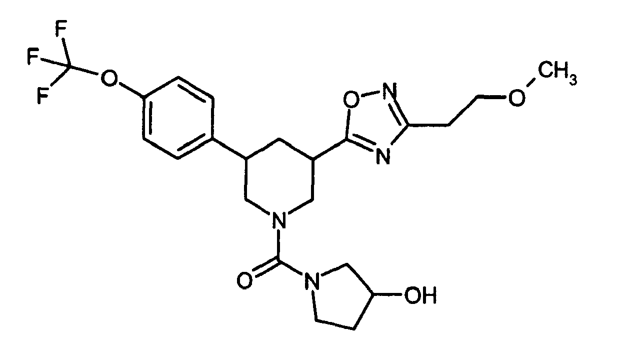

Enantiomerentrennung von 53 mg des Racemates aus Beispiel 420 nach Methode 13D ergab 13 mg der Titelverbindung aus Beispiel 545, 13 mg der Titelverbindung aus Beispiel 546, 11 mg der Titelverbindung aus Beispiel 547 und 11 mg der Titelverbindung aus Beispiel 548.

HPLC (Methode 16E): Rₜ = 5.76 min, >99.0% ee;

LC-MS (Methode 9B): Rₜ = 1.02 min; MS (ESIpos): m/z = 485 [M+H]⁺.

¹H-NMR (400 MHz, DMSO-d₆): δ = 7.47 (d, 2H), 7.33 (d, 2H), 4.87 (d, 1H), 4.24-4.17 (m, 1H), 4.06 (d, 1H), 3.69-3.65 (m, 3H), 3.52-3.40 (m, 3H), 3.23 (s, 3H), 3.09 (br d, 1H), 3.00-2.92 (m, 5H), 2.31 (br d, 1H), 1.97 (dd, 1H), 1.87-1.76 (m, 1H), 1.75-1.67 (m, 1H).

### Beispiels 547

### (3-Hydroxypyrrolidin-1-yl){3-[3-(2-methoxyethyl)-1,2,4-oxadiazol-5-yl]-5-[4-(trifluormethoxy)-phenyl]piperidin-1-yl}methanon [enantiomerenreines cis-Isomer]

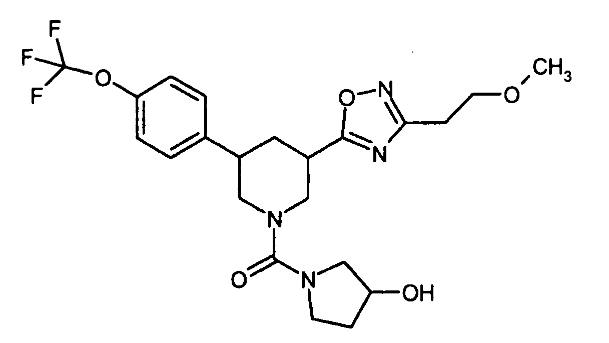

Enantiomerentrennung von 53 mg des Racemates aus Beispiel 420 nach Methode 13D ergab 13 mg der Titelverbindung aus Beispiel 545, 13 mg der Titelverbindung aus Beispiel 546, 11 mg der Titelverbindung aus Beispiel 547 und 11 mg der Titelverbindung aus Beispiel 548.

HPLC (Methode 16E): Rₜ = 8.94 min, >99.0% ee;

LC-MS (Methode 9B): Rₜ = 1.02 min; MS (ESIpos): m/z = 485 [M+H]⁺.

¹H-NMR (400 MHz, DMSO-d₆): δ = 7.46 (d, 2H), 7.32 (d, 2H), 4.88 (d, 1H), 4.18-4.25 (m, 1H), 4.04 (br d, 1H), 3.72 (br d, 1H), 3.67 (t, 2H), 3.56-3.43 (m, 2H), 3.23 (s, 3H), 3.11 (br d, 1H), 3.05-2.97 (m, 2H), 2.93 (t, 2H), 2.87 (dd, 1H), 2.35 (brd, 1H), 1.97 (dd, 1H), 1.88-1.77 (m, 1H), 1.75-1.68 (m, 1H).

### Beispiel 548

### (3-Hydroxypyrrolidin-1-yl){3-[3-(2-methoxyethyl)-1,2,4-oxadiazol-5-yl]-5-[4-(trifluormethoxy)-phenyl]piperidin-1-yl}methanon [enantiomerenreines cis-Isomer]

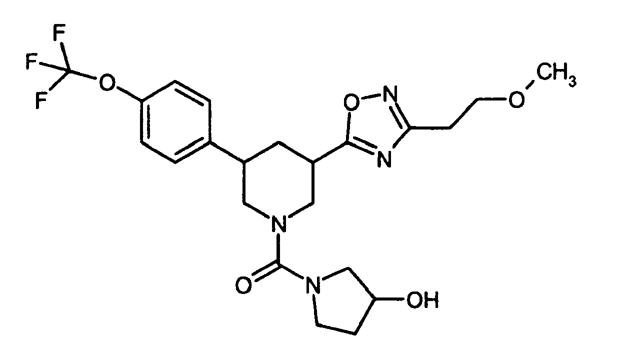

Enantiomerentrennung von 53 mg des Racemates aus Beispiel 420 nach Methode 13D ergab 13 mg der Titelverbindung aus Beispiel 545, 13 mg der Titelverbindung aus Beispiel 546, 11 mg der Titelverbindung aus Beispiel 547 und 11 mg der Titelverbindung aus Beispiel 548.

HPLC (Methode 16E): Rₜ = 11.45 min, >99.0% ee;

LC-MS (Methode 9B): Rₜ = 1.02 min; MS (ESIpos): m/z = 485 [M+H]⁺.

¹H-NMR (400 MHz, DMSO-d₆): δ = 7.47 (d, 2H), 7.33 (d, 2H), 4.87 (d, 1H), 4.24-4.17 (m, 1H), 4.06 (d, 1H), 3.69-3.65 (m, 3H), 3.52-3.40 (m, 3H), 3.23 (s, 3H), 3.09 (br d, 1H), 3.00-2.92 (m, 5H), 2.31 (br d, 1H), 1.97 (dd, 1H), 1.87-1.76 (m, 1H), 1.75-1.67 (m, 1H).

### Beispiel 549

### {3-(4-Ethylphenyl)-5-[3-(2-methoxyethyl)-1,2,4-oxadiazol-5-yl]piperidin-1-yl}(4-hydroxypiperidin-1-yl)methanon [enantiomerenreines cis-Isomer]

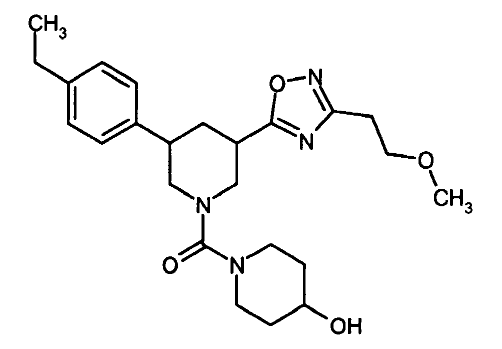

Die Enantiomerentrennung von 394 mg (0.9 mmol) der Verbindung aus Beispiel 183 nach Methode 22D ergab 190 mg der Titelverbindung aus Beispiel 549 (Enantiomer 1) und 183 mg der Titelverbindung aus Beispiel 550 (Enantiomer 2).

HPLC (Methode 15E): Rₜ = 5.12 min, >99.0% ee;

LC-MS (Methode 9B): Rₜ = 1.04 min; MS (ESIpos): m/z = 443 [M+H]⁺;

¹H-NMR (400 MHz, DMSO-d₆): δ = 7.21 (d, 2H), 7.16 (d, 2H), 4.68 (d, OH), 3.95 (br d, 1H), 3.68 (t, 2H), 3.66-3.59 (m, 1H), 3.55 (br d, 1H), 3.49-3.46 (m, 2H), 3.42-3.34 (m, 1H), 3.23 (s, 3H), 3.01-2.85 (m, 7H), 2.57 (q, 2H), 2.29 (br d, 1H), 1.94 (q, 1H), 1.73-1.70 (m, 2H), 1.35-1.25 (m, 2H), 1.16 (t, 3H).

### Beispiel 550

### {3-(4-Ethylphenyl)-5-[3-(2-methoxyethyl)-1,2,4-oxadiazol-5-yl]piperidin-1-yl}(4-hydroxypiperidin-1-yl)methanon [enantiomerenreines cis-Isomer]

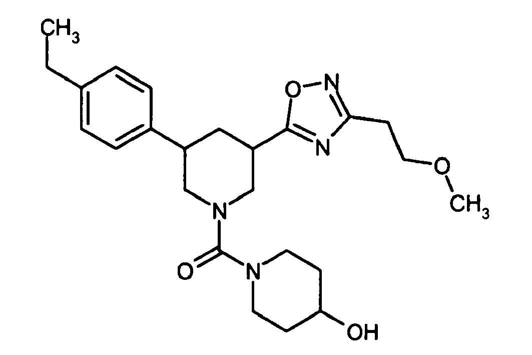

Die Enantiomerentrennung von 394 mg (0.9 mmol) der Verbindung aus Beispiel 183 nach Methode 22D ergab 190 mg der Titelverbindung aus Beispiel 549 (Enantiomer 1) und 183 mg der Titelverbindung aus Beispiel 550 (Enantiomer 2).

HPLC (Methode 15E): Rₜ = 7.47 min, >99.0% ee;

LC-MS (Methode 9B): Rₜ = 1.04 min; MS (ESIpos): m/z = 443 [M+H]⁺;

¹H-NMR (400 MHz, DMSO-d₆): δ = 7.21 (d, 2H), 7.17 (d, 2H), 4.68 (d, OH), 3.94 (br d, 1H), 3.68 (t, 2H), 3.63-3.58 (m, 1H), 3.55 (br d, 1H), 3.49-3.46 (m, 2H), 3.41-3.35 (m, 1H), 3.23 (s, 3H), 3.01-2.85 (m, 7H), 2.57 (q, 2H), 2.28 (br d, 1H), 1.95 (q, 1H), 1.73-1.70 (m, 2H), 1.35-1.26 (m, 2H), 1.16 (t, 3H).

### Beispiel 551

### (3-Hydroxypyrrolidin-1-yl){3-[3-(methoxymethyl)-1,2,4-oxadiazol-5-yl]-5-[4-(trifluormethoxy)-phenyl]piperidin-1-yl}methanon [enantiomerenreines cis-Isomer]

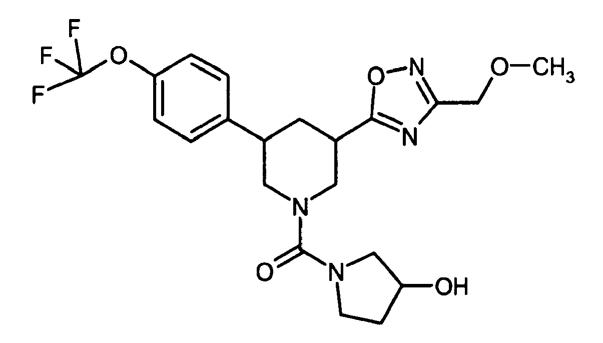

Enantiomerentrennung von 34 mg des Racemates aus Beispiel 512 nach Methode 21D ergab 17 mg der Titelverbindung aus Beispiel 551, 16 mg der Titelverbindung aus Beispiel 552, 17 mg der Titelverbindung aus Beispiel 553 und 17 mg der Titelverbindung aus Beispiel 554.

HPLC (Methode 18E): Rₜ = 5.07 min, >99.0% ee;

LC-MS (Methode 9B): Rₜ = 1.02 min; MS (ESIpos): m/z = 471 [M+H]⁺.

¹H-NMR (400 MHz, DMSO-d₆): δ = 7.47 (d, 2H), 7.32 (d, 2H), 4.88 (d, 1H), 4.53 (d, 2H), 4.24-4.18 (m, 1H), 4.07 (br d, 1H), 3.68 (br d, 1H), 3.34 (s, 3H), 3.55-3.40 (m, 3H), 3.09 (br d, 1H), 3.00-2.85 (m, 3H), 2.33 (br d, 1H), 1.99 (dd, 1H), 1.87-1.77 (m, 1H), 1.76-1.68 (m, 1H).

### Beispiel 552

### (3-Hydroxypyrrolidin-1-yl){3-[3-(methoxymethyl)-1,2,4-oxadiazol-5-yl]-5-[4-(trifluormethoxy)-phenyl]piperidin-1-yl}methanon [enantiomerenreines cis-Isomer]

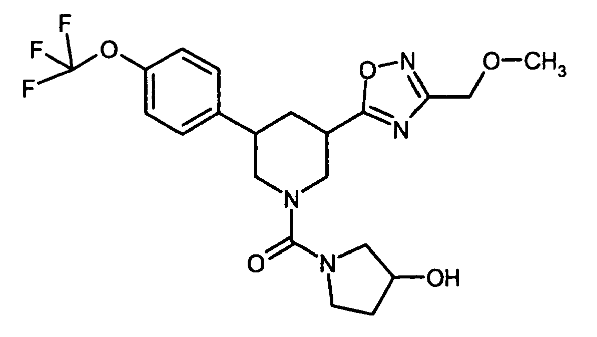

Enantiomerentrennung von 34 mg des Racemates aus Beispiel 512 nach Methode 21D ergab 17 mg der Titelverbindung aus Beispiel 551, 16 mg der Titelverbindung aus Beispiel 552, 17 mg der Titelverbindung aus Beispiel 553 und 17 mg der Titelverbindung aus Beispiel 554.

HPLC (Methode 18E): Rₜ = 5.78 min, >99.0% ee;

LC-MS (Methode 9B): Rₜ = 1.02 min; MS (ESIpos): m/z = 471 [M+H]⁺.

¹H-NMR (400 MHz, DMSO-d₆): δ = 7.47 (d, 2H), 7.33 (d, 2H), 4.88 (d, 1H), 4.53 (d, 2H), 4.25-4.19 (m, 1H), 4.05 (br d, 1H), 3.70 (br d, 1H), 3.52-3.45 (m, 2H), 3.42-3.35 (m, 1H), 3.34 (s, 3H), 3.11 (br d, 1H), 3.03 (dd, 2H), 2.86 (dd, 1H), 2.35 (br d, 1H), 1.98 (dd, 1H), 1.88-1.78 (m, 1H), 1.78-1.68 (m, 1H).

### Beispiel 553

### (3-Hydroxypyrrolidin-1-yl){3-[3-(methoxymethyl)-1,2,4-oxadiazol-5-yl]-5-[4-(trifluormethoxy)-phenyl]piperidin-1-yl}methanon [enantiomerenreines cis-Isomer]

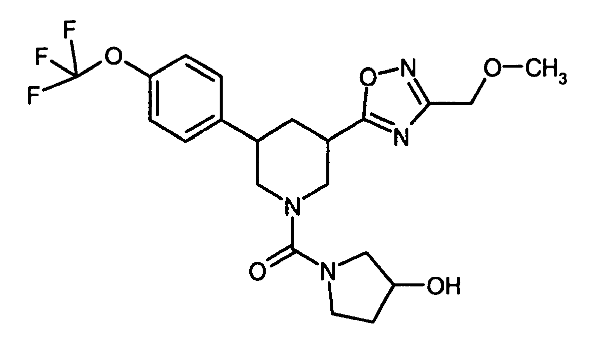

Enantiomerentrennung von 34 mg des Racemates aus Beispiel 512 nach Methode 21D ergab 17 mg der Titelverbindung aus Beispiel 551, 16 mg der Titelverbindung aus Beispiel 552, 17 mg der Titelverbindung aus Beispiel 553 und 17 mg der Titelverbindung aus Beispiel 554.

HPLC (Methode 18E): Rₜ = 8.62 min, >99.0% ee;

LC-MS (Methode 9B): Rₜ = 1.02 min; MS (ESIpos): m/z = 471 [M+H]⁺.

¹H-NMR (400 MHz, DMSO-d₆): δ = 7.47 (d, 2H), 7.33 (d, 2H), 4.88 (d, 1H), 4.53 (d, 2H), 4.25-4.19 (m, 1H), 4.05 (br d, 1H), 3.70 (br d, 1H), 3.52-3.45 (m, 2H), 3.42-3.35 (m, 1H), 3.34 (s, 3H), 3.11 (br d, 1H), 3.03 (dd, 2H), 2.86 (dd, 1H), 2.35 (br d, 1H), 1.98 (dd, 1H), 1.88-1.78 (m, 1H), 1.78-1.68 (m, 1H).

### Beispiel 554

### (3-Hydroxypyrrolidin-1-yl){3-[3-(methoxymethyl)-1,2,4-oxadiazol-5-yl]-5-[4-(trifluormethoxy)-phenyl]piperidin-1-yl}methanon [enantiomerenreines cis-Isomer]

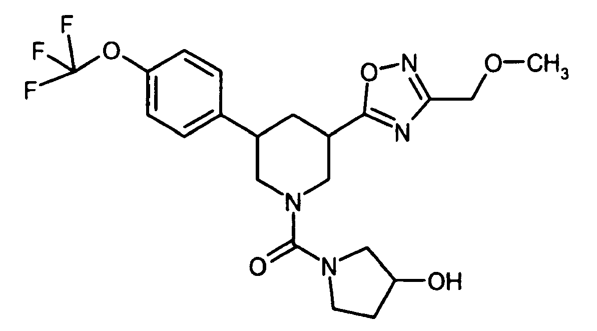

Enantiomerentrennung von 34 mg des Racemates aus Beispiel 512 nach Methode 21D ergab 17 mg der Titelverbindung aus Beispiel 551, 16 mg der Titelverbindung aus Beispiel 552, 17 mg der Titelverbindung aus Beispiel 553 und 17 mg der Titelverbindung aus Beispiel 554.

HPLC (Methode 18E): Rₜ = 12.78 min, >99.0% ee;

LC-MS (Methode 9B): Rₜ = 1.02 min; MS (ESIpos): m/z = 471 [M+H]⁺.

¹H-NMR (400 MHz, DMSO-d₆): δ = 7.47 (d, 2H), 7.32 (d, 2H), 4.88 (d, 1H), 4.53 (d, 2H), 4.24-4.18 (m, 1H), 4.07 (br d, 1H), 3.68 (br d, 1H), 3.34 (s, 3H), 3.55-3.40 (m, 3H), 3.09 (br d, 1H), 3.00-2.85 (m, 3H), 2.33 (br d, 1H), 1.99 (dd, 1H), 1.87-1.77 (m, 1H), 1.76-1.68 (m, 1H).

### Beispiel 555

### (3-Hydroxyazetidin-1-yl){3-[3-(propan-2-yl)-1,2,4-oxadiazol-5-yl]-5-[4-(trifluormethoxy)-phenyl]piperidin-1-yl}methanon [enantiomerenreines cis-Isomer]

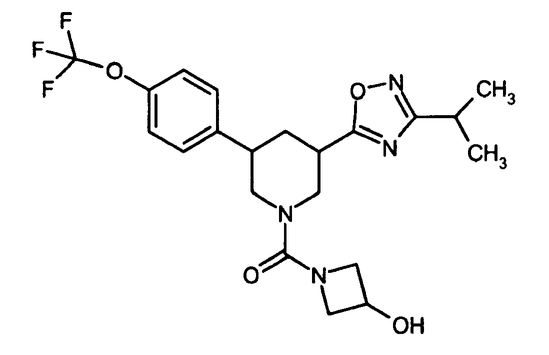

Enantiomerentrennung von 28 mg des Racemates aus Beispiel 529 nach Methode 20D ergab 10 mg der Titelverbindung aus Beispiel 555 und 9 mg der Titelverbindung aus Beispiel 556.

HPLC (Methode 17E): Rₜ = 4.14 min, >99.0% ee;

LC-MS (Methode 9B): Rₜ = 1.12 min; MS (ESIpos): m/z = 455 [M+H]⁺.

### Beispiel 556

### (3-Hydroxyazetidin-1-yl){3-[3-(propan-2-yl)-1,2,4-oxadiazol-5-yl]-5-[4-(trifluormethoxy)-phenyl]piperidin-1-yl}methanon [enantiomerenreines cis-Isomer]

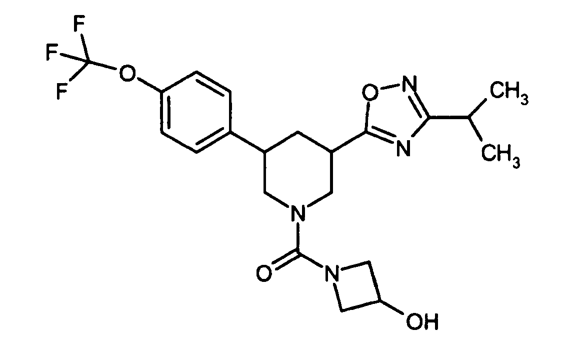

Enantiomerentrennung von 28 mg des Racemates aus Beispiel 529 nach Methode 20D ergab 10 mg der Titelverbindung aus Beispiel 555 und 9 mg der Titelverbindung aus Beispiel 556.

HPLC (Methode 17E): Rₜ = 4.96 min, >99.0% ee;

LC-MS (Methode 9B): Rₜ = 1.12 min; MS (ESIpos): m/z = 455 [M+H]⁺.

### B) Bewertung der physiologischen Wirksamkeit

### Abkürzungen:

- BSA: Rinderserum-Albumin
- DMEM: Dulbecco's Modified Eagle Medium
- EGTA: Ethylenglykol-glykol-bis-(2-aminoäthyl)-*N,N,N',N'*-tetra-essigsäure
- FCS: Fetal Calf Serum
- HEPES: 4-(2-Hydroxyethyl)-1-piperazinethansulfonsäure
- [3H]haTRAP: tritiated high affinity thrombin receptor activating peptide
- PRP: Plättchenreiches Plasma

Die Eignung der erfindungsgemäßen Verbindungen zur Behandlung von thromboembolischen Erkrankungen kann in folgenden Assaysystemen gezeigt werden:

### 1.) In vitro Assays

### 1.a) Zellulärer, funktioneller in vitro-Test

Die Identifizierung von Antagonisten des humanen Protease Aktivierten Rezeptors 1 (PAR-1) sowie die Quantifizierung der Wirksamkeit der hier beschriebenen Substanzen erfolgt mit Hilfe einer rekombinanten Zelllinie. Die Zelle leitet sich ursprünglich von einer embryonalen Nierenzelle des Menschen (HEK293; ATCC: American Type Culture Collection, Manassas, VA 20108, USA) ab. Die Testzelllinie exprimiert konstitutiv eine modifizierte Form des calciumsensitiven Photoproteins Aequorin, das nach Rekonstitution mit dem Co-Faktor Coelenterazin bei Erhöhungen der freien Calcium-Konzentration im inneren mitochondrialen Kompartiment Licht emittiert (Rizzuto R, Simpson AW, Brini M, Pozzan T.; Nature 1992, 358, 325-327). Zusätzlich exprimiert die Zelle stabil den endogenen humanen PAR-1-Rezeptor sowie den endogenen purinergen Rezeptor P2Y2. Die resultierende PAR-1-Testzelle reagiert auf Stimulation des endogenen PAR-1 oder P2Y2-Rezeptors mit einer intrazellulären Freisetzung von Calcium-Ionen, die durch die resultierende Aequorin-Lumineszens mit einem geeigneten Luminometer quantifiziert werden kann (Milligan G, Marshall F, Rees S, Trends in Pharmacological Sciences 1996,17, 235-237).

Für die Prüfung der Substanz-Spezifität wird deren Wirkung nach Aktivierung des endogenen PAR-1-Rezeptors mit der Wirkung nach Aktivierung des endogenen purinergen P2Y2-Rezeptors verglichen, der den gleichen intrazellulären Signalweg nutzt.

Testablauf: Die Zellen werden zwei Tage (48 Std.) vor dem Test in Kulturmedium (DMEM F12, ergänzt mit 10% FCS, 2 mM Glutamine, 20 mM HEPES, 1.4 mM Pyruvat, 0.1 mg/ml Gentamycin, 0.15% Na-Bicarbonat; BioWhittaker Cat.# BE04-687Q; B-4800 Verviers, Belgien) in 384-Loch-Mikrotiterplatten ausplattiert und in einem Zellinkubator (96% Luftfeuchtigkeit, 5% v/v CO₂, 37°C) gehalten. Am Testtag wird das Kulturmedium durch eine Tyrodelösung (in mM: 140 Natriumchlorid, 5 Kaliumchlorid, 1 Magnesiumchlorid, 2 Calciumchlorid, 20 Glucose, 20 HEPES), das zusätzlich den Co-Faktor Coelenterazin (25 µM) und Glutathion (4 mM) enthält, ausgetauscht und die Mikrotiterplatte anschließend für weitere 3-4 Stunden inkubiert. Dann werden die Testsubstanzen auf die Mikrotiterplatte pipettiert und 5 Minuten nach Übertragung der Testsubstanzen in die Wells der Mikrotiterplatte wird die Platte in das Luminometer transferiert, eine PAR-1-Agonist-Konzentration, die EC₅₀ entspricht, zugeschossen und sofort das resultierende Lichtsignal im Luminometer gemessen. Zur Unterscheidung einer Antagonist-Substanzwirkung von einer toxischen Wirkung wird unmittelbar anschließend der endogene purinerge Rezeptor mit Agonist aktiviert (ATP, 10 µM Endkonzentration) und das resultierende Lichtsignal gemessen. Die Ergebnisse sind in Tabelle A gezeigt:

**Tabelle A:**

| **Beispiel-Nr.** | **IC₅₀ [nM]** | | **Beispiel-Nr.** | **IC₅₀ [nM]** |
|---|---|---|---|---|
| **28** | 28 | | **276** | 25,3 |
| **37** | 21 | | **279** | 14,7 |
| **38** | 31 | | **283** | 14,9 |
| **39** | 6.8 | | **284** | 9,73 |
| **60** | 10 | | **322** | 3,01 |
| **113** | 47 | | **329** | 24 |
| **119** | 68 | | **330** | 87.8 |
| **144** | 8.51 | | **335** | 25.1 |
| **146** | 9.39 | | **352** | 4.77 |
| **148** | 14 | | **356** | 2.73 |
| **150** | 19 | | **368** | 11.7 |
| **152** | 48.1 | | **420** | 23.8 |
| **159** | 4.61 | | **449** | 86.3 |
| **160** | 5.4 | | **451** | 19.6 |
| **169** | 6.74 | | **454** | 47.5 |
| **170** | 18.8 | | **459** | 84.2 |
| **178** | 17.05 | | **465** | 24.4 |
| **181** | 28.8 | | **470** | 4.16 |
| **183** | 31.5 | | **474** | 23.4 |
| **187** | 53.4 | | **484** | 76.6 |
| **194** | 66.1 | | **487** | 107 |
| **197** | 77.5 | | **490** | 25.1 |
| **212** | 142 | | **494** | 5.67 |
| **229** | 1.72 | | **495** | 3.51 |
| **230** | 2.81 | | **513** | 5.16 |
| **232** | 6.44 | | **519** | 20.8 |
| **234** | 15.4 | | **520** | 11 |
| **239** | 5.74 | | **549** | 12.5 |
| **249** | 13.1 | | **559** | 49.8 |
| **260** | 11.4 | | **561** | 5.76 |
| **275** | 10.3 | | | |

### 1.b) PAR-1 Rezeptor Bindungsassay

Thrombozytenmembranen werden mit 12 nM [3H]haTRAP und Testsubstanz in verschiedenen Konzentrationen in einem Puffer (50 mM Tris pH 7.5, 10 mM Magnesiumchlorid, 1 mM EGTA, 0.1% BSA) bei Raumtemperatur für 80 min inkubiert. Danach wird der Ansatz auf eine Filterplatte übertragen und zweimal mit Puffer gewaschen. Nach Zugabe von Scintillationsflüssigkeit wird die Radioaktivität auf dem Filter in einem beta-Counter vermessen.

### 1.c) Thrombozytenaggregation in Plasma

Zur Bestimmung der Thrombozytenaggregation wird Blut von gesunden Probanden beiderlei Geschlechts, die innerhalb der letzten zehn Tage keine die Thrombozytenaggregation beeinflussende Medikation erhalten hatten, verwendet. Das Blut wird in Monovetten (Sarstedt, Nümbrecht, Deutschland) die als Antikoagulans Natrium Citrat 3.8% (1 Teil Citrat + 9 Teile Blut) enthalten, aufgenommen. Zur Gewinnung von plättchenreichem Plasma wird das Citrat-Vollblut bei 140g für 20 min zentrifugiert.

Für die Aggregationsmessungen werden Aliquots des plättchenreichen Plasmas mit aufsteigenden Konzentrationen an Prüfsubstanz 10 min bei 37°C inkubiert. Anschließend wird die Aggregation durch Zugabe eines Thrombin-Rezeptor Agonisten (TRAP6, SFLLRN) in einem Aggregometer ausgelöst und mittels der turbidimetrischen Methode nach Born (Born, G.V.R., Cross M.J., The Aggregation of Blood Platelets; J. Physiol. 1963, 168, 178-195) bei 37°C bestimmt. Die SFLLRN-Konzentration, die zur maximalen Aggregation führt, wird gegebenenfalls jeweils für jeden Spender individuell ermittelt.

Zur Berechnung der inhibitorischen Wirkung wird die maximale Zunahme der Lichttransmission (Amplitude der Aggregationskurve in %) innerhalb 5 Minuten nach Zugabe des Agonisten in Gegenwart und Abwesenheit von Prüfsubstanz ermittelt und die Inhibition berechnet. Aus den Inhibitionskurven wird die Konzentration berechnet, die die Aggregation zu 50% hemmt. Die Ergebnisse sind in Tabelle B gezeigt:

**Tabelle B:**

| **Bsp. Nr.** | **IC₅₀ [µM]** |
|---|---|
| **38** | 93 |
| **39** | 5.6 |
| **60** | 19.6 |

### 1.d) Thrombozytenaggregation in Puffer

Zur Bestimmung der Thrombozytenaggregation wird Blut von gesunden Probanden beiderlei Geschlechts, die innerhalb der letzten zehn Tage keine die Thrombozytenaggregation beeinflussende Medikation erhalten hatten, verwendet. Das Blut wird in Monovetten (Sarstedt, Nümbrecht, Deutschland) die als Antikoagulans Natriumcitrat 3.8% (1 Teil Citrat + 9 Teile Blut) enthalten, aufgenommen. Zur Gewinnung von plättchenreichem Plasma wird das Citrat-Vollblut bei 140g für 20 min zentrifugiert. Zu dem PRP wird ein Viertel des Volumens ACD-Puffer (44.8 mM Natriumcitrat, 20.9 mM Citronensäure, 74.1 mM Glucose und 4 mM Kaliumchlorid) zugegeben und 10 Minuten bei 1000g zentrifugiert. Das Thrombozyten-Pellet wird mit WaschPuffer resuspendiert und 10 Minuten bei 1000g zentrifugiert. Die Thrombozyten werden in Inkubations-Puffer resuspendiert und auf 200000 Z/µl eingestellt. Vor Versuchsbeginn wird Calciumchlorid und Magnesiumchlorid, Endkonzentration je 2mM (Stammlösung 2M, 1:1000 Verdünnung) zugegeben. Besonderheit: bei ADP-induzierter Aggregation wird nur Calciumchlorid zugegeben. Folgende Agonisten können eingesetzt werden: TRAP6-Trifluoracetat- Salz, Kollagen, Humanes α-Thrombin und U-46619. Die Konzentration des Agonisten wird zu jedem Spender ausgetestet.

Testdurchführung: Es werden 96er-Loch Mikrotiterplatten verwendet. Die Prüfsubstanz wird in DMSO verdünnt und 2 µl je Loch vorgelegt. 178 µl Thrombozyten-Suspension werden zugegeben und 10 Minuten bei Raumtemperatur vorinkubiert. 20 µl Agonist werden zugegeben und die Messung im Spectramax, OD 405 nm, sofort gestartet. Die Kinetik wird in 11 Messungen von je 1 Minute bestimmt. Zwischen den Messungen wird 55 Sekunden geschüttelt.

### 1.e) Thrombozytenaggregation in fibrinogendepletiertem Plasma

Zur Bestimmung der Thrombozytenaggregation wird Blut von gesunden Probanden beiderlei Geschlechts, die innerhalb der letzten zehn Tage keine die Thrombozytenaggregation beeinflussende Medikation erhalten hatten, verwendet. Das Blut wird in Monovetten (Sarstedt, Nümbrecht, Deutschland) die als Antikoagulans Natriumcitrat 3.8% (1 Teil Citrat + 9 Teile Blut) enthalten, aufgenommen.

Herstellung von fibrinogendepletiertem Plasma: Zur Gewinnung von plättchenarmem Plasma wird das Citrat-Vollblut bei 140g für 20 min abzentrifugiert. Das plättchenarme Plasma wird im Verhältnis 1:25 mit Reptilase (Roche Diagnostic, Deutschland) versetzt und vorsichtig invertiert. Es folgen 10 min Inkubation bei 37°C im Wasserbad mit direkt folgender Inkubation auf Eis für 10 min. Das Plasma-Reptilase Gemisch wird bei 1300g für 15 min zentrifugiert und der Überstand (fibrinogendepletiertes Plasma) wird gewonnen.

Thrombozyten-Isolierung: Zur Gewinnung von plättchenreichem Plasma wird das Citrat-Vollblut bei 140g für 20 min zentrifugiert. Zu dem PRP wird ein Viertel des Volumens ACD-Puffer (44.8 mM Natriumcitrat, 20.9 mM Citronensäure, 74.1 mM Glucose und 4 mM Kaliumchlorid) zugegeben und 10 Minuten bei 1300g zentrifugiert. Das Thrombozyten-Pellet wird mit WaschPuffer resuspendiert und 10 Minuten bei 1300g zentrifugiert. Die Thrombozyten werden in Inkubations-Puffer resuspendiert und auf 400000 Z/µl eingestellt und Calciumchloridlösung mit einer Endkonzentration von 5 mM (1/200Verdünnung) zugegeben.

Für die Aggregationsmessungen werden Aliquots (98 µl fibrinogendepletiertes Plasma und 80 µl Thrombozytensuspension) mit aufsteigenden Konzentrationen an Prüfsubstanz 10 min bei RT inkubiert. Anschließend wird die Aggregation durch Zugabe von humanem alpha Thrombin in einem Aggregometer ausgelöst und mittels der turbidimetrischen Methode nach Born (Born, G.V.R., Cross M.J., The Aggregation of Blood Platelets; J. Physiol. 1963, 168, 178-195) bei 37°C bestimmt. Die alpha Thrombin Konzentration, die gerade eben zur maximalen Aggregation führt, wird jeweils für jeden Spender individuell ermittelt.

Zur Berechnung der inhibitorischen Wirkung wird die Zunahme der maximalen Lichttransmission (Amplitude der Aggregationskurve in %) innerhalb 5 Minuten nach Zugabe des Agonisten in Gegenwart und Abwesenheit von Prüfsubstanz ermittelt und die Inhibition berechnet. Aus den Inhibitionskurven wird die Konzentration berechnet, die die Aggregation zu 50% hemmt.

### 1.f) Stimulation gewaschener Thrombozyten und Analyse in der Durchflusszytometrie

Isolierung gewaschener Thrombozyten: Humanes Vollblut wird mittels Venenpunktion von freiwilligen Spendern gewonnen und in Monovetten (Sarstedt, Nümbrecht, Deutschland) überführt, die als Antikoagulans Natriumcitrat enthalten (1 Teil Natriumcitrat 3.8% + 9 Teile Vollblut). Die Monovetten werden bei 900 Umdrehungen pro Minute und 4°C über einen Zeitraum von 20 Minuten zentrifugiert (Heraeus Instruments, Deutschland; Megafuge 1.0RS). Das plättchenreiche Plasma wird vorsichtig abgenommen und in ein 50 ml-Falconröhrchen überführt. Nun wird das Plasma mit ACD-Puffer (44 mM Natriumcitrat, 20.9 mM Zitronensäure, 74.1 mM Glucose) versetzt. Das Volumen des ACD-Puffers entspricht einem Viertel des Plasmavolumens. Durch zehnminütige Zentrifugation bei 2500 Umdrehungen und 4°C werden die Thrombozyten sedimentiert. Danach wird der Überstand vorsichtig abdekantiert und verworfen. Die präzipitierten Thrombozyten werden zunächst vorsichtig mit einem Milliliter Waschpuffer (113 mM Natriumchlorid, 4 mM Dinatriumhydrogenphosphat, 24 mM Natriumdihydrogenphosphat, 4 mM Kaliumchlorid, 0.2 mM Ethylenglycol-bis-(2-aminoethyl)-*N,N,N',N'*-tetraessigsäure, 0.1% Glucose) resuspendiert und dann mit Waschpuffer auf ein Volumen aufgefüllt, das dem der Plasmamenge entspricht. Der Waschvorgang wird ein zweites Mal durchgeführt. Nachdem die Thrombozyten durch eine erneute zehnminütige Zentrifugation bei 2500 Umdrehungen und 4°C präzipitiert worden sind, werden sie vorsichtig in einem Milliliter Inkubationspuffer (134 mM Natriumchlorid, 12 mM Natriumhydrogencarbonat, 2.9 mM Kaliumchlorid, 0.34 mM Natriumdihydrogencarbonat, 5 mM HEPES, 5 mM Glucose, 2 mM Calciumchlorid und 2 mM Magnesiumchlorid) resuspendiert und mit Inkubationspuffer auf eine Konzentration von 300.000 Thrombozyten pro µl eingestellt.

Färbung und Stimulierung der humanen Thrombozyten mit humanem α-Thrombin in Gegenwart oder Abwesenheit eines PAR-1-Antagonisten: Die Thrombozytensuspension wird mit der zu prüfenden Substanz bzw. des entsprechenden Lösungsmittels für 10 Minuten bei 37°C vorinkubiert (Eppendorf, Deutschland; Thermomixer Comfort). Durch Zugabe des Agonisten (0.5 µM bzw. 1 µM α-Thrombin; Kordia, Niederlande, 3281 NIH Units/mg; oder 30µg/ml Thrombin receptor activating peptide (TRAP6); Bachem, Schweiz) bei 37° und unter Schütteln von 500 Umdrehungen pro Minute wird die Thrombozytenaktivierung ausgelöst. Zu den Zeitpunkten 0, 1, 2.5, 5, 10 und 15 Minuten wird jeweils ein Aliquot von 50µl entnommen und in einen Milliliter einfach-konzentrierte CellFix™-Lösung (Becton Dickinson Immunocytometry Systems, USA) überführt. Zur Fixierung der Zellen werden sie 30 Minuten bei 4°C in der Dunkelheit inkubiert. Durch eine zehnminütige Zentrifugation bei 600 g und 4°C werden die Thrombozyten präzipitiert. Der Überstand wird verworfen und die Thrombozyten werden in 400 µl CellWash™ (Becton Dickinson Immunocytometry Systems, USA) resuspendiert. Ein Aliquot von 100 µl wird in ein neues FACS-Röhrchen überführt, 1 µl des thrombozyten-identifizierenden Antikörpers und 1 µl des aktivierungszustands-detektierenden Antikörpers werden mit CellWash™ auf ein Volumen von 100 µl aufgefüllt. Diese Antikörperlösung wird dann zur Thrombozytensuspension gegeben und 20 Minuten bei 4°C in der Dunkelheit inkubiert. Im Anschluss an die Färbung wird das Ansatzvolumen durch Zugabe von weiteren 400 µl CellWash™ erhöht.

Zur Identifizierung der Thrombozyten wird ein fluorescein-isothiocyanat-konjugierter Antikörper eingesetzt, der gegen das humane Glykoprotein IIb (CD41) gerichtet ist (Immunotech Coulter, Frankreich; Cat. No. 0649). Mit Hilfe des phycoerythrin-konjugierten Antikörpers, der gegen das humane Glykoprotein P-Selektin (Immunotech Coulter, Frankreich; Cat. No. 1759) gerichtet ist, lässt sich der Aktivierungszustand der Thrombozyten bestimmen. P-Selektin (CD62P) ist in den α-Granula ruhender Thrombozyten lokalisiert. Es wird jedoch nach *in-vitro-* bzw. *in-vivo-*Stimulierung zur äußeren Plasmamembran translokalisiert.

Durchflusszytometrie und Auswertung der Daten: Die Proben werden im Gerät FACSCalibur™ Flow Cytometry System der Firma Becton Dickinson Immunocytometry Systems, USA, vermessen und mit Hilfe der Software CellQuest, Version 3.3 (Becton Dickinson Immunocytometry Systems, USA) ausgewertet und graphisch dargestellt. Das Maß der Thrombozytenaktivierung wird durch den Prozentsatz der CD62P-positiven Thrombozyten (CD41-positive Ereignisse) bestimmt. Es werden von jeder Probe 10.000 CD41-positive Ereignisse gezählt.

Die inhibitorische Wirkung der zu prüfenden Substanzen wird anhand der Reduktion der Thrombozytenaktivierung berechnet, die sich auf die Aktivierung durch den Agonisten bezieht.

### 1.g) Thrombozytenaggregationsmessung mit der Parallelplattenflußkammer

Zur Bestimmung der Thrombozytenaggregation wird Blut von gesunden Probanden beiderlei Geschlechts, die innerhalb der letzten zehn Tage keine die Thrombozytenaggregation beeinflussende Medikation erhalten hatten, verwendet. Das Blut wird in Monovetten (Sarstedt, Nümbrecht, Deutschland) die als Antikoagulans Natriumcitrat 3.8% (1 Teil Citrat + 9 Teile Blut) enthalten, aufgenommen. Zur Gewinnung von plättchenreichem Plasma wird das Citrat-Vollblut bei 140g für 20 min zentrifugiert. Zu dem PRP wird ein Viertel des Volumens ACD-Puffer (44.8 mM Natriumcitrat, 20.9 mM Citronensäure, 74.1 mM Glucose und 4 mM Kaliumchlorid) zugegeben und 10 Minuten bei 1000g zentrifugiert. Das Thrombozyten-Pellet wird mit WaschPuffer resuspendiert und 10 Minuten bei 1000g zentrifugiert. Für die Perfusionsstudie wird eine Mischung von 40% Erythrozyten und 60% gewaschene Thrombozyten (200.000/µl) hergestellt und in HEPES-Tyrode Puffer suspendiert. Die Messung der Thrombozytenaggregation unter Flußbedingungen wird mittels der Parallelplattenflußkammer durchgeführt (B. Nieswandt et al., EMBO J. 2001, 20, 2120-2130; C. Weeterings, Arterioscler Thromb. Vasc. Biol. 2006, 26, 670-675; JJ Sixma, Thromb. Res. 1998, 92, 43-46). Glasobjektträger werden mit 100 µl humaner α-Thrombinlösung (gelöst in Tris-Puffer) über Nacht bei 4°C benetzt (α-Thrombin in verschiedenen Konzentrationen, z.B. 10 bis 50 µg/ml) und abschließend mittels 2% BSA abgeblockt.

Rekonstituiertes But wird über die Thrombin-benetzten Glasobjektträger über 5 Minuten mit konstanter Flußrate geleitet (z.B. Scherrate 300/Sekunde) und mittels Mikroskop-Videosystem beobachtet und aufgezeichnet. Die inhibitorische Wirkung der zu prüfenden Substanzen wird morphometrisch anhand der Reduktion der Plättchenaggregatsbildung ermittelt. Alternativ kann die Inhibierung der Plättchenaktivierung durch Durchflußzytometrie, z.B. über p-Selektin-Expression (CD62p), bestimmt werden (siehe Methode 1.f).

### 2.) Ex vivo Assay

### 2.a) Thrombozytenaggregation (Primaten, Meerschweinchen)

Meerschweinchen oder Primaten werden in wachem oder narkotisiertem Zustand oral, intravenös oder intraperitoneal mit Prüfsubstanzen in geeigneter Formulierung behandelt. Als Kontrolle werden andere Meerschweinchen oder Primaten in identischer Weise mit dem entsprechenden Vehikel behandelt. Nach je nach Applikationsart unterschiedlich langer Zeit wird aus den tief narkotisierten Tieren Blut durch Punktion des Herzens oder der Aorta gewonnen. Das Blut wird in Monovetten (Sarstedt, Nümbrecht, Deutschland) die als Antikoagulans Natriumcitrat 3.8% (1 Teil Citratlösung + 9 Teile Blut) enthalten, aufgenommen. Zur Gewinnung von plättchenreichem Plasma wird das Citrat-Vollblut bei 140g für 20 min zentrifugiert.

Die Aggregation wird durch Zugabe eines Thrombin-Rezeptor Agonisten (TRAP6, SFLLRN, 50 µg/ml; Konzentration wird in jedem Experiment je nach Tierart ermittelt) in einem Aggregometer ausgelöst und mittels der turbidimetrischen Methode nach Born (Horn, G.V.R., Cross M.J., The Aggregation of Blood Platelets; J. Physiol. 1963, 168, 178-195) bei 37°C bestimmt.

Zur Aggregationsmessung wird die maximale Zunahme der Lichttransmission (Amplitude der Aggregationskurve in %) innerhalb 5 Minuten nach Zugabe des Agonisten ermittelt. Die inhibitorische Wirkung der verabreichten Prüfsubstanzen in den behandelten Tieren wird durch die Reduktion der Aggregation, bezogen auf den Mittelwert der Kontrolltiere, berechnet.

### 3.) In vivo Assays

### 3.a) Thrombosemodelle

Die erfindungsgemäßen Verbindungen können in Thrombosemodellen in geeigneten Tierspezies, in denen die Thrombin-induzierte Plättchenaggregation über den PAR-1-Rezeptor vermittelt wird, untersucht werden. Als Tierspezies eignen sich Meerschweinchen und insbesondere Primaten (vergleiche: Lindahl, A.K., Scarborough, R.M., Naughton, M.A., Harker, L.A., Hanson, S.R., Thromb Haemost 1993, 69, 1196; Cook JJ, Sitko GR, Bednar B, Condra C, Mellott MJ, Feng D-M, Nutt RF, Shager JA, Gould RJ, Connolly TM, Circulation 1995, 91, 2961-2971; Kogushi M, Kobayashi H, Matsuoka T, Suzuki S, Kawahara T, Kajiwara A, Hishinuma I, Circulation 2003, 108 Suppl. 17, IV-280; Derian CK, Damiano BP, Addo MF, Darrow AL, D'Andrea MR, Nedelman M, Zhang H-C, Maryanoff BE, Andrade-Gordon P, J. Pharmacol. Exp. Ther. 2003, 304, 855-861). Alternativ können Meerschweinchen verwendet werden, die mit Inhibitoren von PAR-3 und/oder PAR-4 vorbehandelt werden (Leger AJ et al., Circulation 2006, 113, 1244-1254), oder transgene PAR-3- und/oder PAR-4-Knockdown-Meerschweinchen.

### 3.b) Gerinnungsstörung und Organdysfunktion bei Disseminierter Intravasaler Gerinnung (DIC)

Die erfindungsgemäßen Verbindungen können in Modellen für DIC und/oder Sepsis in geeigneten Tierspezies untersucht werden. Als Tierspezies eignen sich Meerschweinchen und insbesondere Primaten, bei Untersuchung der endothelvermittelten Effekte auch Mäuse und Ratten (vergleiche: Kogushi M, Kobayashi H, Matsuoka T, Suzuki S, Kawahara T, Kajiwara A, Hishinuma I, Circulation 2003, 108 Suppl. 17, IV-280; Derian CK, Damiano BP, Addo MF, Darrow AL, D'Andrea MR, Nedelman M, Zhang H-C, Maryanoff BE, Andrade-Gordon P, J. Pharmacol. Exp. Ther. 2003, 304, 855-861; Kaneider NC et al., Nat Immunol, 2007, 8, 1303-12; Carnerer E et al., Blood, 2006, 107, 3912-21; Riewald M et al., J Biol Chem, 2005, 280, 19808-14.). Alternativ können Meerschweinchen verwendet werden, die mit Inhibitoren von PAR-3 und/oder PAR-4 vorbehandelt werden (Leger AJ et al., Circulation 2006, 113, 1244-1254), oder transgene PAR-3-und/oder PAR-4-Knockdown-Meerschweinchen.

### 3.b.1) Thrombin-Antithrombin-Komplexe

Thrombin-Antithrombin-Komplexe (nachfolgend als "TAT" bezeichnet) sind ein Maß für das durch Gerinnungsaktivierung endogen gebildete Thrombin. TAT werden mittels eines ELISA-Assays bestimmt (Enzygnost TAT micro, Dade-Behring). Aus Citratblut wird durch Zentrifugation Plasma gewonnen. Zu 50 µl Plasma wird 50 µl TAT-Probenpuffer gegeben, kurz geschüttelt und 15 min bei Raumtemperatur inkubiert. Die Proben werden abgesaugt, und das Well 3-malig mit Waschpuffer gewaschen (300 µl/Well). Die Platte wird zwischen den Waschgängen abgeklopft. Es wird Konjugatlösung (100 µl) hinzugegeben und 15 min bei Raumtemperatur inkubiert. Die Proben werden abgesaugt, und das Well 3-malig mit Waschpuffer gewaschen (300 µ/lWell). Anschließend wird chromogenes Susbtrat hinzugegeben (100 µl/Well), 30 min im Dunkeln bei Raumtemperatur inkubiert, Stopplösung hinzugegeben (100 µl/ Well), und die Farbbildung bei 492 nm gemessen (Saphire Plate reader).

### 3.b.2) Parameter für Organdysfunktion

Es werden verschiedene Parameter bestimmt, aufgrund derer Rückschlüsse auf die Funktionseinschränkung verschiedener innerer Organe durch die LPS-Gabe gezogen werden können, und der therapeutische Effekt von Prüfsubstanzen abgeschätzt werden kann. Citratblut oder ggf. Lithium-Heparin-Blut wird zentrifugiert, und die Parameter aus dem Plasma bestimmt. Folgende Parameter werden typischerweise erhoben: Kreatinin, Harnstoff, Aspartat-Aminotransferase (AST), Alanin-Aminotransferase (ALT), Gesamt-Bilirubin, Laktatdehydrogenase (LDH), Gesamt-Protein, Gesamt-Albumin und Fibrinogen. Die Werte geben Aufschluss auf die Funktion der Niere, der Leber, des Kreislaufes und der Gefäße.

### 3.b.3) Parameter für Entzündung

Das Ausmaß der durch Endotoxin ausgelösten Entzündungsreaktion lässt sich aus dem Anstieg von Entzündungsmediatoren im Plasma nachweisen, z.B. Interleukine (1, 6, 8 und 10), Tumornekrosefaktor alpha oder Monocyte Chemoattractant Protein-1. Hierzu können ELISAs oder das Luminex-System verwendet werden.

### 3.c) Antitumor Wirksamkeit

Die erfindungsgemäßen Verbindungen können in Modellen für Krebs getestet werden, z.B. im humanen Brustkrebs-Modell in immundefizienten Mäusen (vergleiche: S. Even-Ram et. al., Nature Medicine, 1988, 4, 909-914).

### 3.d) Antiangiogenetische Wirksamkeit

Die erfindungsgemäßen Verbindungen können in *in vitro* und *in vivo* Modellen für Angiogenese getestet werden (vergleiche: Caunt et al., Journal of Thrombosis and Haemostasis, 2003,10, 2097-2102; Haralabopoulos et al., Am JPhysiol, 1997, C239-C245; Tsopanoglou et al., JBC, 1999, 274, 23969-23976; Zania et al., JPET, 2006, 318, 246-254).

### 3.e) Blutdruck- und Herzfrequenz-modulierende Wirkung

Die erfindungsgemäßen Verbindungen können in *in vivo* Modellen hinsichtlich Ihrer Wirkung auf den arteriellen Blutdruck und die Herzfrequenz untersucht werden. Hierzu werden Ratten (z.B. Wistar) mit implantierbaren Radiotelemetrieeinheiten instrumentiert, und es wird ein elektronisches Datenaquisitions- und Speicherungs-System (Data Sciences, MN, USA), bestehend aus einem chronisch implantierbaren Transducer/Transmitter-Einheit in Verbindung mit einem Flüssigkeits-gefüllten Katheter, verwendet. Der Transmitter wird in die Peritonealhöhle implantiert und der Sensor-Katheter in der deszendierenden Aorta positioniert. Die erfindungsgemäßen Verbindungen können appliziert werden (z.B. oral oder intravenös). Vor Behandlung wird der mittlere arterielle Blutdruck und die Heizfrequenz der unbehandelten und behandelten Tiere gemessen und sichergestellt, dass diese im Bereich von ca. 131-142 mmHg und 279-321 Schläge/Minute liegen. PAR-1-aktivierendes Peptid (SFLLRN; z.B. Dosen zwischen 0.1 und 5 mg/kg) wird intravenös verabreicht. Der Blutdruck und die Herzfrequenz werden in verschiedenen Zeitintervallen und Zeiträumen mit und ohne PAR-1-aktivierendem Peptid sowie mit und ohne einer der erfindungsgemäßen Verbindungen gemessen (vergleiche: Cicala C et al., The FASEB Journal, 2001, 15, 1433-5; Stasch JP et al., British Journal of Pharmacology 2002,135, 344-355).

### 4.) Bestimmung der Löslichkeit

### Herstellung der Ausgangslösung (Urlösung):

Mindestens 1.5 mg der Testsubstanz werden in ein Wide Mouth 10 mm Screw V-Vial (Fa. Glastechnik Gräfenroda GmbH, Art.-Nr. 8004-WM-H/V15µ) mit passender Schraubkappe und Septum genau eingewogen, mit DMSO zu einer Konzentration von 50 mg/ml versetzt und 30 Minuten mittels eines Vortexers geschüttelt.

### Herstellung der Kalibrierlösungen:

Die notwendigen Pipettierschritte erfolgen in 1.2 ml 96er Deep Well Plate (DWP) mittels eines Liquid-Handling-Roboters. Als Lösemittel wird ein Gemisch aus Acetonitril/Wasser 8:2 verwendet.

*Herstellung der Ausgangslösung für Kalibrierlösungen (Stammlösung):* 10 µl der Urlösung werden mit 833 µl des Lösemittelgemisch versetzt (Konzentration = 600 µg/ml) und homogenisiert. Es werden von jeder Testsubstanz 1:100 Verdünnungen in separaten DWP's hergestellt und wiederum homogenisiert.

*Kalibrierlösung 5 (600 ng*/*ml):* 30 µl der Stammlösung werden mit 270 µl Lösemittelgemisch versetzt und homogenisiert.

*Kalibrierlösung 4 (60 nglml):* 30 µl der Kalibrierlösung 5 werden mit 270 µl Lösemittelgemisch versetzt und homogenisiert.

*Kalibrierlösung 3 (12 nglml):* 100 µl der Kalibrierlösung 4 werden mit 400 µl Lösemittelgemisch versetzt und homogenisiert.

*Kalibrierlösung 2 (1.2 nglml):* 30 µl der Kalibrierlösung 3 werden mit 270 µl Lösemittelgemisch versetzt und homogenisiert.

*Kalibrierlösung 1 (0.6 ng*/*ml):* 150 µl der Kalibrierlösung 2 werden mit 150 µl Lösemittelgemisch versetzt und homogenisiert.

### Herstellung der Probenlösungen:

Die notwendigen Pipettierschritte erfolgen in 1.2 ml 96er DWP mittels eines Liquid-Handling-Roboters. 10.1 µl der Stammlösung werden mit 1000 µl PBS-Puffer pH 6.5 versetzt. (PBS-Puffer pH 6.5: 61.86 g Natriumchlorid, 39.54 g Natriumdihydrogenphosphat und 83.35 g 1 N Natronlauge werden in einen 1 Liter-Messkolben eingewogen, mit Wasser aufgefüllt und ca. 1 Stunde gerührt. Von dieser Lösung werden 500 ml in einen 5 Liter-Messkolben gegeben und mit Wasser aufgefüllt. Es wird mit 1 N Natronlauge auf pH 6.5 einstellen.)

### Durchführung:

Die notwendigen Pipettierschritte erfolgen in 1.2 ml 96er DWP mittels eines Liquid-Handling-Roboters. Die so hergestellten Probenlösungen werden 24 Stunden bei 1400 rpm mittels eines temperierbaren Schüttlers bei 20°C geschüttelt. Von diesen Lösungen werden jeweils 180 µl abgenommen und in Beckman Polyallomer Centrifuge Tubes überführt. Diese Lösungen werden 1 Stunde mit ca. 223.000 x g zentrifugiert. Von jeder Probenlösung werden 100 µl des Überstandes abgenommen und 1:10 und 1:1000 mit PBS-Puffer 6.5 verdünnt.

### Analytik:

Die Proben werden mittels HPLC/MS-MS analysiert. Quantifiziert wird über eine Fünf-Punkt-Kalibrationskurve der Testverbindung. Die Löslichkeit wird in mg/l ausgedrückt. Analysensequenz: 1) Blank (Lösemittelgemisch); 2) Kalibrierlösung 0.6 ng/ml; 3) Kalibrierlösung 1.2 ng/ml; 4) Kalibrierlösung 12 ng/ml; 5) Kalibrierlösung 60 ng/ml; 6) Kalibrierlösung 600 ng/ml; 7) Blank (Lösemittelgemisch); 8) Probenlösung 1:1000; 9) Probenlösung 1:10.

### HPLC/MS-MSMethode:

HPLC: Agilent 1100, quat. Pumpe (G1311A), Autosampler CTC HTS PAL, Degaser (G1322A) und Säulenthermostat (G1316A); Säule: Oasis HLB 20 mm x 2.1 mm, 25 µ; Temperatur: 40°C; Eluent A: Wasser + 0.5 ml Ameisensäure/l; Eluent B: Acetonitril + 0.5 ml Ameisensäure/l; Flussrate: 2.5 ml/min; Stoptime 1.5 min; Gradient: 0 min 95% A, 5% B; Rampe: 0-0.5 min 5% A, 95% B; 0.5-0.84 min 5% A, 95% B; Rampe: 0.84-0.85 min 95% A, 5% B; 0.85-1.5 min 95% A, 5% B.

MS/MS: WATERS Quattro Micro Tandem MS/MS; Z-Spray API-Interface; HPLC-MS-Eingangssplitter 1:20; Messung im ESI-Mode.

### C) Ausführungsbeispiele für pharmazeutische Zusammensetzungen

Die erfindungsgemäßen Substanzen können folgendermaßen in pharmazeutische Zubereitungen überführt werden:

### Tablette:

### Zusammensetzung:

100 mg der Verbindung des Beispiels 1, 50 mg Lactose (Monohydrat), 50 mg Maisstärke, 10 mg Polyvinylpyrolidon (PVP 25) (Fa. BASF, Deutschland) und 2 mg Magnesiumstearat.

Tablettengewicht 212 mg. Durchmesser 8 mm, Wölbungsradius 12 mm.

### Herstellung:

Die Mischung aus der Verbindung des Beispiels 1, Lactose und Stärke wird mit einer 5%-igen Lösung (m/m) des PVPs in Wasser granuliert. Das Granulat wird nach dem Trocknen mit dem Magnesiumstearat für 5 min. gemischt. Diese Mischung wird mit einer üblichen Tablettenpresse verpresst (Format der Tablette siehe oben).

### Orale Suspension:

### Zusammensetzung:

1000 mg der Verbindung des Beispiels 1, 1000 mg Ethanol (96%), 400 mg Rhodigel (Xanthan gum) (Fa. FMC, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die Verbindung des Beispiels 1 wird der Suspension zugefügt. Unter Rühren erfolgt die Zugabe des Wassers. Bis zum Abschluss der Quellung des Rhodigels wird ca. 6h gerührt.

### Intravenös applizierbare Lösung:

### Zusammensetzung:

1 mg der Verbindung von Beispiel 1, 15 g Polyethylenglykol 400 und 250 g Wasser für Injektionszwecke.

### Herstellung:

Die Verbindung von Beispiel 1 wird zusammen mit Polyethylenglykol 400 in dem Wasser unter Rühren gelöst. Die Lösung wird sterilfiltriert (Porendurchmesser 0.22 µm) und unter aseptischen Bedingungen in hitzesterilisierte Infusionsflaschen abgefüllt. Diese werden mit Infusionsstopfen und Bördelkappen verschlossen.

## Patentansprüche

1. Verbindung der Formel 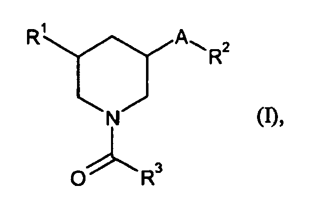 in welcher
A für eine Gruppe der Formel 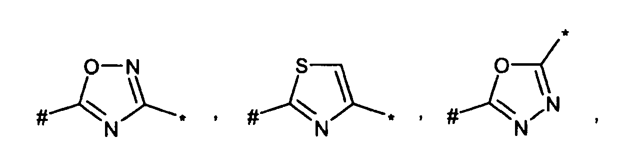 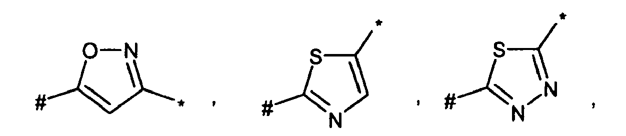 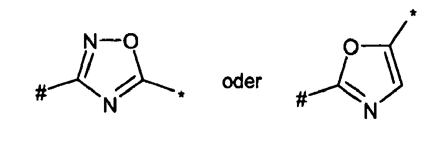 steht,
wobei
# die Anknüpfstelle an den Piperidinring ist,
und
* die Anknüpfstelle an R² ist,
R¹ für Phenyl steht,
wobei Phenyl substituiert sein kann mit 1 bis 3 Substituenten, unabhängig voneinander ausgewählt aus der Gruppe bestehend aus Monofluormethyl, Difluormethyl, Trifluormethyl, Monofluormethoxy, Difluormethoxy, Trifluormethoxy, Monofluormethylsulfanyl, Difluormethylsulfanyl, Trifluormethylsulfanyl, Methylsulfonyl, C₁-C₄-Alkyl, C₁-C₄-Alkoxy, C₁-C₄- Alkoxycarbonyl und C₃-C₆-Cycloalkyl,
worin C₂-C₄-Alkoxy substituiert sein kann mit einem Substituenten ausgewählt aus der Gruppe bestehend aus Methoxy und Ethoxy,
und
worin Cycloalkyl substituiert sein kann mit 1 bis 3 Substituenten, unabhängig voneinander ausgewählt aus der Gruppe bestehend aus Halogen und C₁-C₄-Alkyl,
R² für Wasserstoff, Trifluormethyl, Aminomethyl, C₁-C₆-Alkyl, C₂-C₆-Alkenyl, C₁-C₄-Alkoxycarbonyl, C₃-C₆-Cycloalkyl, Cyclopentenyl, 4- bis 6-gliedriges Heterocyclyl, Phenyl, 1,3-Benzodioxolyl, 5- oder 6-gliedriges Heteroaryl oder Pyridylaminocarbonyl steht,
wobei Cycloalkyl, Heterocyclyl, Phenyl und Heteroaryl substituiert sein können mit 1 bis 3 Substituenten, unabhängig voneinander ausgewählt aus der Gruppe bestehend aus Halogen, Cyano, Amino, Monofluormethyl, Difluormethyl, Trifluormethyl, Monofluormethoxy, Difluormethoxy, Trifluormethoxy, Monofluormethylsulfanyl, Difluormethylsulfanyl, Trifluormethylsulfanyl, C₁-C₄- Alkyl, C₁-C₄-Alkoxy, C₁-C₆-Alkylamino, C₁-C₄-Alkoxycarbonylamino, C₃-C₆- Cycloalkyl, 4- bis 6-gliedriges Heterocyclyl, Phenyl und 5- oder 6-gliedriges Heteroaryl,
worin Alkylamino substituiert sein kann mit einem Substituenten ausgewählt aus der Gruppe bestehend aus C₁-C₄-Alkoxy und C₁-C₆- Alkylamino,
und
wobei C₁-C₄-Alkyl substituiert sein kann mit einem Substituenten ausgewählt aus der Gruppe bestehend aus Halogen, Hydroxy, Amino, Aminocarbonyl, C₁-C₄- Alkoxy, C₁-C₆-Alkylamino, C₁-C₄-Alkylthio, C₁-C₄-Alkylcarbonyl, C₁-C₄- Alkylcarbonyloxy, C₁-C₄-Alkylsulfonyl, C₁-C₄-Alkoxycarbonyl, C₁-C₄- Alkoxycarbonylamino, C₃-C₆-Cycloalkyl, C₃-C₆-Cycloalkylamino, 4- bis 6- gliedriges Heterocyclyl, Phenyl, Phenoxy, 5- oder 6-gliedriges Heteroaryl und 5- oder 6-gliedriges Heteroarylthio,
worin Cycloalkyl, Heterocyclyl, Phenyl, Phenoxy, Heteroaryl und Heteroarylthio substituiert sein können mit 1 bis 3 Substituenten, unabhängig voneinander ausgewählt aus der Gruppe bestehend aus Halogen, Cyano, Hydroxymethyl, Monofluormethyl, Difluormethyl, Trifluormethyl, Monofluormethoxy, Difluormethoxy, Trifluormethoxy, Monofluormethylsulfanyl, Difluormethylsulfanyl, Trifluormethylsulfanyl, C₁-C₄-Alkyl, C₁-C₄-Alkoxy, C₃-C₆-Cycloalkyl, 4- bis 6-gliedriges Heterocyclyl, Phenyl und 5- oder 6-gliedriges Heteroaryl,
R³ für C₁-C₆-Alkyl, C₁-C₆-Alkoxy, C₁-C₆-Alkylamino, C₃-C₇-Cycloalkyl, 4- bis 6- gliedriges Heterocyclyl, Phenyl oder 5- oder 6-gliedriges Heteroaryl steht,
wobei Alkyl, C₂-C₆-Alkoxy und Alkylamino substituiert sein können mit einem Substituenten ausgewählt aus der Gruppe bestehend aus Hydroxy, Amino, Cyano und C₁-C₄-Alkoxy,
und
wobei Cycloalkyl, Heterocyclyl, Phenyl und Heteroaryl substituiert sein können mit 1 bis 3 Substituenten, unabhängig voneinander ausgewählt aus der Gruppe bestehend aus Halogen, Cyano, Nitro, Oxo, Hydroxy, Amino, Aminomethyl, Monofluormethyl, Difluormethyl, Trifluormethyl, Monofluormethoxy, Difluormethoxy, Trifluormethoxy, Monofluormethylsulfanyl, Difluormethylsulfanyl, Trifluormethylsulfanyl, Hydroxycarbonyl, Aminocarbonyl, C₁-C₄-Alkyl, C₁-C₄-Alkoxy, C₁-C₆-Alkylamino, C₁-C₄-Alkoxycarbonyl und C₁-C₄- Alkylaminocarbonyl,
oder eines ihrer Salze, ihrer Solvate oder der Solvate ihrer Salze.

2. Verbindung nach Anspruch 1, **dadurch gekennzeichnet, dass**
A für eine Gruppe der Formel 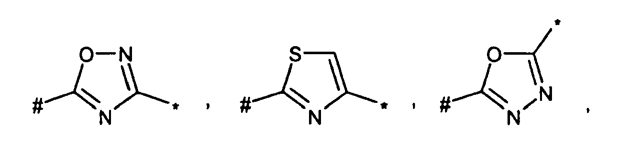 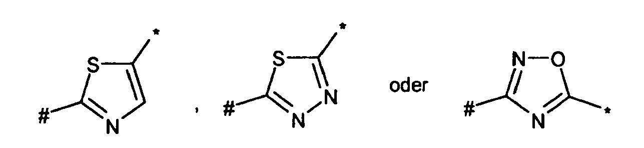 steht,
wobei
# die Anknüpfstelle an den Piperidinring ist,
und
* die Anknüpfstelle an R² ist,
R¹ für Phenyl steht,
wobei Phenyl substituiert ist mit 1 bis 2 Substituenten, unabhängig voneinander ausgewählt aus der Gruppe bestehend aus Trifluormethyl, Trifluormethoxy, Methyl, Ethyl, Isopropyl und Methoxy,
R² für Methyl, Ethyl, Isopropyl, n-Propyl, tert-Butyl, Methoxycarbonyl, Ethoxycarbonyl, Cyclopropyl, Cyclobutyl, Azetidinyl, Oxetanyl, Pyrrolidinyl, Tetrahydropyridinyl, Phenyl, 1,3-Benzodioxolyl, Thienyl, Furanyl, Pyrrolyl, Thiazolyl, Isoxazolyl, Imidazolyl, Triazolyl, Pyridyl, Pyrimidinyl oder Pyrazinyl steht,
wobei Azetidinyl, Oxetanyl, Pyrrolidinyl, Tetrahydropyridinyl, Phenyl, Thienyl, Furanyl, Pyrrolyl, Thiazolyl, Isoxazolyl, Imidazolyl, Triazolyl, Pyridyl, Pyrimidinyl und Pyrazinyl substituiert sein können mit 1 bis 2 Substituenten, unabhängig voneinander ausgewählt aus der Gruppe bestehend aus Halogen, Trifluormethyl, Trifluormethoxy, Methyl, Ethyl, Methoxy, Ethoxy und Ethylamino,
worin Ethylamino substituiert sein kann mit einem Substituenten ausgewählt aus der Gruppe bestehend aus Methoxy und Dimethylamino,
und
wobei Methyl und Ethyl substituiert sein können mit einem Substituenten ausgewählt aus der Gruppe bestehend aus Hydroxy, Amino, Methoxy, Ethoxy, Isopropoxy, Dialkylamino, Methylsulfonyl, Cyclopropylamino, Morpholinyl, Phenyl und Phenoxy,
worin Phenyl substituiert sein kann mit 1 bis 2 Substituenten, unabhängig voneinander ausgewählt aus der Gruppe bestehend aus Halogen, Hydroxymethyl, Trifluormethyl, Trifluormethoxy, Methyl, Ethyl, Methoxy und Ethoxy,
R³ für tert-Butyl, *N-*Methyl-*N-*ethylamino, Methoxyalkylamino, Cyclopropyl, Cyclopentyl, Azetidinyl, 3,3-Difluorazetidinyl, 3-Hydroxyazetidinyl, 3- Methylazetidinyl, 3-Methoxyazetidinyl, 3-Dimethylaminoazetidinyl, Pyrroldinyl, 3,3-Difluorpyrroldin-1-yl, 3-Hydroxypyrroldin-1-yl, 3-Aminopyrroldin-1-yl, 4,4- Difluorpiperidin-1-yl, 4-Hydroxy-piperidin-1-yl, 4-Aminopiperidin-1-yl, 4- Cyanopiperidin-1-yl, 3-Methoxypiperidin-1-yl, Thiazolidinyl, Morpholin-4-yl, 2,2- Dimethylmorpholin-4-yl, 2-Oxopiperazin-1-yl oder 3-Oxo-4-methyl-piperazin-1-yl steht,
oder eines ihrer Salze, ihrer Solvate oder der Solvate ihrer Salze.

3. Verbindung nach Anspruch 1 oder 2, **dadurch gekennzeichnet, dass**
A für eine Gruppe der Formel 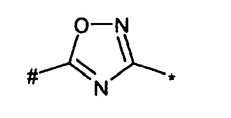 steht,
wobei
# die Anknüpfstelle an den Piperidinring ist,
und
* die Anknüpfstelle an R² ist,
R¹ für Phenyl steht,
wobei Phenyl substituiert ist mit 1 bis 2 Substituenten, unabhängig voneinander ausgewählt aus der Gruppe bestehend aus Trifluormethyl, Trifluormethoxy, Methyl und Ethyl,
R² für Methyl, Ethyl oder Isopropyl steht,
wobei Methyl und Ethyl substituiert sein können mit einem Substituenten Methoxy,
R³ für 3-Hydroxyazetidinyl, 3-Hydroxypyrroldin-1-yl, 4-Hydroxypiperidin-1-yl, 4- Cyano-piperidin-1-yl oder Morpholin-4-yl steht,
oder eines ihrer Salze, ihrer Solvate oder der Solvate ihrer Salze.

4. Verbindung nach einem der Ansprüche 1 bis 3, **dadurch gekennzeichnet, dass** die Substituenten -R¹ und -A-R² in cis-Position zueinander stehen.

5. Verfahren zur Herstellung einer Verbindung der Formel (I) oder eines ihrer Salze, ihrer Solvate oder der Solvate ihrer Salze nach Anspruch 1, **dadurch gekennzeichnet, dass** entweder
[A] eine Verbindung der Formel 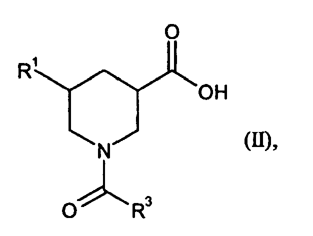 in welcher
R¹ und R³ die in Anspruch 1 angegebene Bedeutung aufweisen,
mit einer Verbindung der Formel 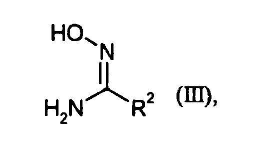 in welcher
R² die in Anspruch 1 angegebene Bedeutung aufweist,
umgesetzt wird
oder
[B] eine Verbindung der Formel 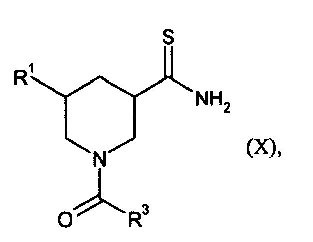 in welcher
R¹ und R³ die in Anspruch 1 angegebene Bedeutung aufweisen,
mit einer Verbindung der Formel 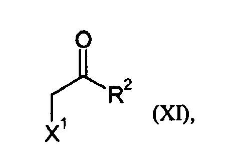 in welcher
R² die in Anspruch 1 angegebene Bedeutung aufweist, und
X¹ für Brom oder Chlor steht,
umgesetzt wird
oder
[C] eine Verbindung der Formel (II) mit einer Verbindung der Formel
R²CONHNH₂ (XVI),
in welcher
R² die in Anspruch 1 angegebene Bedeutung aufweist,
in Gegenwart von Phosphorylchlorid oder Thionylchlorid umgesetzt wird
oder
[D] eine Verbindung der Formel (II) in der ersten Stufe mit einer Verbindung der Formel
R²COCH₂NH₂ (XVII),
in welcher
R² die in Anspruch 1 angegebene Bedeutung aufweist,
in Gegenwart von Thionylchlorid oder Phosphorylchlorid,
und in der zweiten Stufe mit Lawesson-Reagens umgesetzt wird
oder
[E] eine Verbindung der Formel (H) mit einer Verbindung der Formel (XVII) in Gegenwart von Thionylchlorid oder Phosphorylchlorid umgesetzt wird
oder
[F] eine Verbindung der Formel (II) in der ersten Stufe mit einer Verbindung der Formel (XVI) in Gegenwart von Thionylchlorid oder Phosphorylchlorid und in der zweiten Stufe mit Lawesson-Reagens umgesetzt wird
oder
[G] eine Verbindung der Formel 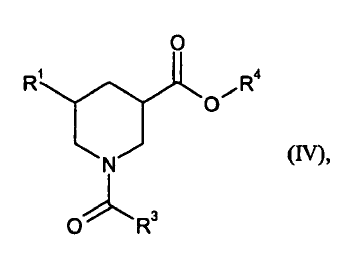 in welcher
R¹ und R³ die in Anspruch 1 angegebene Bedeutung aufweisen, und
R⁴ für Methyl oder Ethyl steht,
mit einer Verbindung der Formel 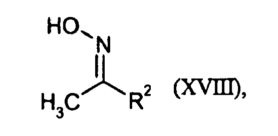 in welcher
R² die in Anspruch 1 angegebene Bedeutung aufweist,
in Gegenwart von Butyllithium umgesetzt wird
oder
[H] eine Verbindung der Formel 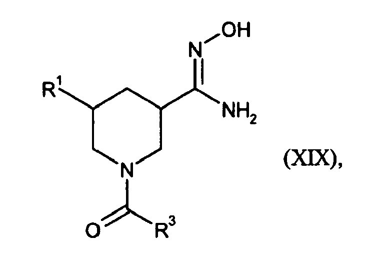 in welcher
R¹ und R³ die in Anspruch 1 angegebene Bedeutung aufweisen,
mit einer Verbindung der Formel 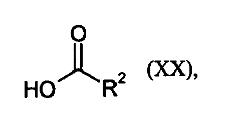 in welcher
R² die in Anspruch 1 angegebene Bedeutung aufweist,
umgesetzt wird
oder
[J] eine Verbindung der Formel 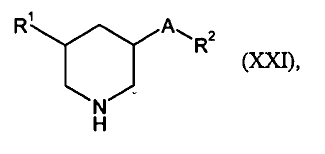 in welcher
A, R¹ und R² die in Anspruch 1 angegebene Bedeutung aufweisen,
in der ersten Stufe mit 4-Nitrophenylchloroformat und in der zweiten Stufe mit einer Verbindung der Formel
R³—H (XXII),
in welcher
R³ die in Anspruch 1 angegebene Bedeutung aufweist,
umgesetzt wird.

6. Verbindung nach einem der Ansprüche 1 bis 4 zur Behandlung und/oder Prophylaxe von Krankheiten.

7. Verwendung einer Verbindung nach einem der Ansprüche 1 bis 4 zur Herstellung eines Arzneimittels zur Behandlung und/oder Prophylaxe von Krankheiten.

8. Verwendung einer Verbindung nach einem der Ansprüche 1 bis 4 zur Herstellung eines Arzneimittels zur Behandlung und/oder Prophylaxe von Herz-Kreislauf Erkrankungen, thromboembolischen Erkrankungen und/oder von Tumorerkrankungen.

9. Verwendung einer Verbindung nach einem der Ansprüche 1 bis 4 zur Verhinderung der Blutkoagulation *in vitro.*

10. Arzneimittel enthaltend eine Verbindung nach einem der Ansprüche 1 bis 4 in Kombination mit einem inerten, nichttoxischen, pharmazeutisch geeigneten Hilfsstoff.

11. Arzneimittel enthaltend eine Verbindung nach einem der Ansprüche 1 bis 4 in Kombination mit einem weiteren Wirkstoff.

12. Arzneimittel nach Anspruch 10 oder 11 zur Behandlung und/oder Prophylaxe von Herz-Kreislauf-Erkrankungen, thromboembolischen Erkrankungen und/oder von Tumorerkrankungen.

13. Verfahren zur Verhinderung der Blutkoagulation *in vitro,* **dadurch gekennzeichnet, dass** eine antikoagulatorisch wirksame Menge einer Verbindung nach einem der Ansprüche 1 bis 4 zugegeben wird.

## Claims

1. Compounds of the formula 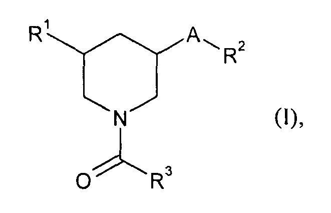 in which
A represents a group of the formula 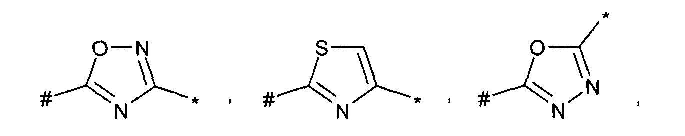 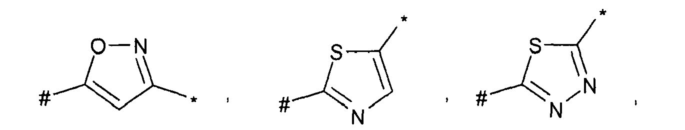 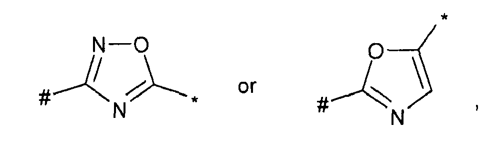 where
# is the point of attachment to the piperidine ring,
and
* is the point of attachment to R²,
R¹ represents phenyl,
where phenyl may be substituted by 1 to 3 substituents independently of one another selected from the group consisting of monofluoromethyl, difluoromethyl, trifluoromethyl, monofluoromethoxy, difluoromethoxy, trifluoromethoxy, monofluoromethylsulphanyl, difluoromethylsulphanyl, trifluoromethylsulphanyl, methylsulphonyl, C₁-C₄-alkyl, C₁-C₄-alkoxy, C₁-C₄-alkoxycarbonyl and C₃-C₆- cycloalkyl,
where C₂-C₄-alkoxy may be substituted by a substituent selected from the group consisting of methoxy and ethoxy,
and
where cycloalkyl may be substituted by 1 to 3 substituents independently of one another selected from the group consisting of halogen and C₁-C₄- alkyl,
R² represents hydrogen, trifluoromethyl, aminomethyl, C₁-C₆-alkyl, C₂-C₆-alkeny), C₁- C₄-alkoxycarbonyl, C₃-C₆-cycloalkyl, cyclopentenyl, 4- to 6-membered heterocyclyl, phenyl, 1,3-benzodioxolyl, 5- or 6-membered heteroaryl or pyridylaminocarbonyl,
where cycloalkyl, heterocyclyl, phenyl and heteroaryl may be substituted by 1 to 3 substituents independently of one another selected from the group consisting of halogen, cyano, amino, monofluoromethyl, difluoromethyl, trifluoromethyl, monofluoromethoxy, difluoromethoxy, trifluoromethoxy, monofluoro- methylsulphanyl, difluoromethylsulphanyl, trifluoromethylsulphanyl, C₁-C₄-alkyl, C₁-C₄-alkoxy, C₁-C₆-alkylamino, C₁-C₄-alkoxycarbonylamino, C₃-C₆-cycloalkyl, 4- to 6-membered heterocyclyl, phenyl and 5- or 6-membered heteroaryl,
where alkylamino may be substituted by a substituent selected from the group consisting of C₁-C₄-alkoxy and C₁-C₆-alkylamino,
and
where C₁-C₄-alkyl may be substituted by a substituent selected from the group consisting of halogen, hydroxyl, amino, aminocarbonyl, C₁-C₄-alkoxy, C₁-C₆- alkylamino, C₁-C₄-alkylthio, C₁-C₄-alkylcarbonyl, C₁-C₄-alkylcarbonyloxy, C₁-C₄- alkylsulphonyl, C₁-C₄-alkoxycarbonyl, C₁-C₄-alkoxycarbonylamino, C₃-C₆- cycloalkyl, C₃-C₆-cycloalkylamino, 4- to 6-membered heterocyclyl, phenyl, phenoxy, 5- or 6-membered heteroaryl and 5- or 6-membered heteroarylthio,
where cycloalkyl, heterocyclyl, phenyl, phenoxy, heteroaryl and heteroarylthio may be substituted by 1 to 3 substituents independently of one another selected from the group consisting of halogen, cyano, hydroxymethyl, monofluoromethyl, difluoromethyl, trifluoromethyl, monofluoromethoxy, difluoromethoxy, trifluoromethoxy, monofluoro- methylsulphanyl, difluoromethylsulphanyl, trifluoromethylsulphanyl, C₁- C₄-alkyl, C₁-C₄-alkoxy, C₃-C₆-cycloalkyl, 4- to 6-membered heterocyclyl, phenyl and 5- or 6-membered heteroaryl,
R³ represents C₁-C₆-alkyl, C₁-C₆-alkoxy, C₁-C₆-alkylamino, C₃-C₇-cycloalkyl, 4- to 6- membered heterocyclyl, phenyl or 5- or 6-membered heteroaryl,
where alkyl, C₂-C₆-alkoxy and alkylamino may be substituted by a substituent selected from the group consisting of hydroxyl, amino, cyano and C₁-C₄-alkoxy,
and
where cycloalkyl, heterocyclyl, phenyl and heteroaryl may be substituted by 1 to 3 substituents independently of one another selected from the group consisting of halogen, cyano, nitro, oxo, hydroxyl, amino, aminomethyl, monofluoromethyl, difluoromethyl, trifluoromethyl, monofluoromethoxy, difluoromethoxy, trifluoromethoxy, monofluoromethylsulphanyl, difluoromethylsulphanyl, trifluoromethylsulphanyl, hydroxycarbonyl, aminocarbonyl, C₁-C₄-alkyl, C₁-C₄- alkoxy, C₁-C₆-alkylamino, C₁-C₄-alkoxycarbonyl and C₁-C₄-alkylaminocarbonyl,
or one of its salts, its solvates or the solvates of its salts.

2. Compound according to Claim 1, **characterized in that**
A represents a group of the formula 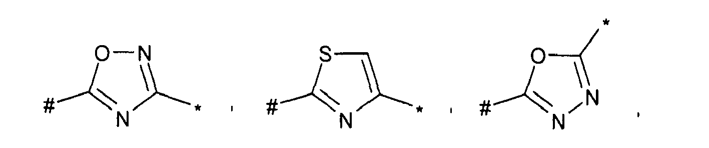 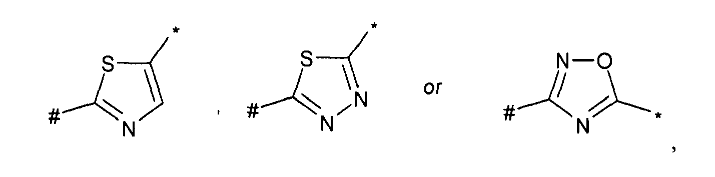 where
# is the point of attachment to the piperidine ring,
and
* is the point of attachment to R²,
R¹ represents phenyl,
where phenyl is substituted by 1 or 2 substituents independently of one another selected from the group consisting of trifluoromethyl, trifluoromethoxy, methyl, ethyl, isopropyl and methoxy,
R² represents methyl, ethyl, isopropyl, n-propyl, tert-butyl, methoxycarbonyl, ethoxycarbonyl, cyclopropyl, cyclobutyl, azetidinyl, oxetanyl, pyrrolidinyl, tetrahydropyridinyl, phenyl, 1,3-benzodioxolyl, thienyl, furanyl, pyrrolyl, thiazolyl, isoxazolyl, imidazolyl, triazolyl, pyridyl, pyrimidinyl or pyrazinyl,
where azetidinyl, oxetanyl, pyrrolidinyl, tetrahydropyridinyl, phenyl, thienyl, furanyl, pyrrolyl, thiazolyl, isoxazolyl, imidazolyl, triazolyl, pyridyl, pyrimidinyl and pyrazinyl may be substituted by 1 to 2 substituents independently of one another selected from the group consisting of halogen, trifluoromethyl, trifluoromethoxy, methyl, ethyl, methoxy, ethoxy and ethylamino,
where ethylamino may be substituted by a substituent selected from the group consisting of methoxy and dimethylamino,
and
where methyl and ethyl may be substituted by a substituent selected from the group consisting of hydroxyl, amino, methoxy, ethoxy, isopropoxy, dialkylamino, methylsulphonyl, cyclopropylamino, morpholinyl, phenyl and phenoxy,
where phenyl may be substituted by 1 or 2 substituents independently of one another selected from the group consisting of halogen, hydroxymethyl, trifluoromethyl, trifluoromethoxy, methyl, ethyl, methoxy and ethoxy,
R³ represents tert-butyl, *N*-methyl-*N*-ethylamino, methoxyalkylamino, cyclopropyl, cyclopentyl, azetidinyl, 3,3-difluoroazetidinyl, 3-hydroxyazetidinyl, 3- methylazetidinyl, 3-methoxyazetidinyl, 3-dimethylaminoazetidinyl, pyrroldinyl, 3,3-difluoropyrroldin-1-yl, 3-hydroxypyrroldin-1-yl, 3-aminopyrroldin-1-yl, 4,4- difluoropiperidin-1-yl, 4-hydroxypiperidin-1-yl, 4-aminopiperidin-1-yl, 4- cyanopiperidin-1-yl, 3-methoxypiperidin-1-yl, thiazolidinyl, morpholin-4-yl, 2,2- dimethylmorpholin-4-yl, 2-oxopiperazin-1-yl or 3-oxo-4-methylpiperazin-1-yl,
or one of its salts, its solvates or the solvates of its salts.

3. Compound according to Claim 1 or 2, **characterized in that**
A represents a group of the formula 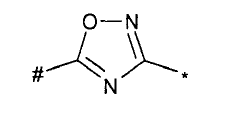 where
# is the point of attachment to the piperidine ring,
and
* is the point of attachment to R²,
R¹ represents phenyl,
where phenyl is substituted by 1 or 2 substituents independently of one another selected from the group consisting of trifluoromethyl, trifluoromethoxy, methyl and ethyl,
R² represents methyl, ethyl or isopropyl,
where methyl and ethyl may be substituted by a substituent methoxy,
R³ represents 3-hydroxyazetidinyl, 3-hydroxypyrroldin-1-yl, 4-hydroxypiperidin-1-yl, 4-cyanopiperidin-1-yl or morpholin-4-yl,
or one of its salts, its solvates or the solvates of its salts.

4. Compound according to any of Claims 1 to 3, **characterized in that** the substituents -R¹ and -A-R² are in the cis-position to one another.

5. Process for preparing a compound of the formula (I) or one of its salts, its solvates or the solvates of its salts according to Claim 1, **characterized in that** either
[A] a compound of the formula 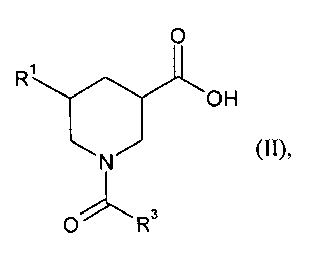 in which
R¹ and R³ have the meaning given in Claim I,
is reacted with a compound of the formula 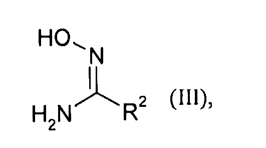 in which
R² has the meaning given in Claim 1,
or
[B] a compound of the formula 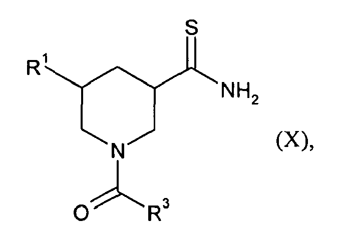 in which
R¹ and R³ have the meaning given in Claim 1,
is reacted with a compound of the formula 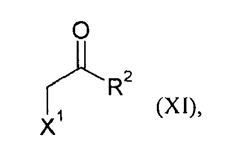 in which
R² has the meaning given in Claim 1, and
X¹ represents bromine or chlorine,
or
[C] a compound of the formula (II) is reacted with a compound of the formula
R²CONHNH₂ (XVI),
in which
R² has the meaning given in Claim 1,
in the presence of phosphoryl chloride or thionyl chloride
or
[D] a compound of the formula (II) is, in the first step, reacted with a compound of the formula
R²COCH₂NH₂ (XVII),
in which
R² has the meaning given in Claim 1,
in the presence of thionyl chloride or phosphoryl chloride,
and, in the second step, reacted with Lawesson reagent
or
[E] a compound of the formula (II) is reacted with a compound of the formula (XVII) in the presence of thionyl chloride or phosphoryl chloride
or
[F] a compound of the formula (II) is, in the first step, reacted with a compound of the formula (XVI) in the presence of thionyl chloride or phosphoryl chloride and, in the second step, reacted with Lawesson reagent
or
[G] a compound of the formula 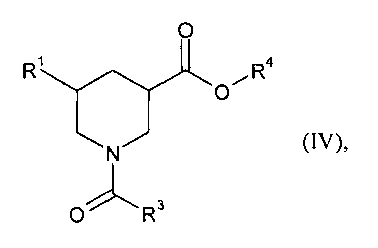 in which
R¹ and R³ have the meaning given in Claim 1, and
R⁴ represents methyl or ethyl,
is reacted with a compound of the formula 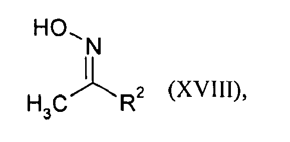 in which
R² has the meaning given in Claim 1,
in the presence of butyllithium
or
[H] a compound of the formula 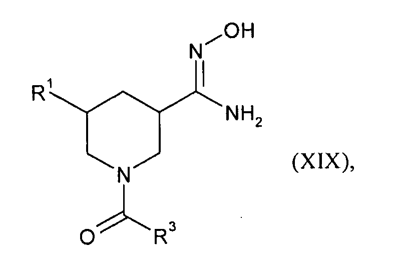 in which
R¹ and R³ have the meaning given in Claim 1,
is reacted with a compound of the formula 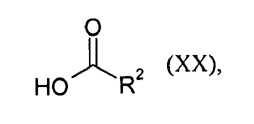 in which
R² has the meaning given in Claim 1,
or
[J] a compound of the formula 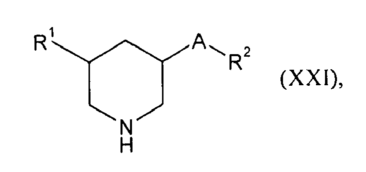 in which
A, R¹ and R² have the meaning given in Claim 1,
is, in the first step, reacted with 4-nitrophenyl chloroformate and, in the second step, reacted with a compound of the formula
R³—H (XXII),
in which
R³ has the meaning given in Claim 1.

6. Compound according to any of Claims 1 to 4 for the treatment and/or prophylaxis of diseases.

7. Use of a compound according to any of Claims 1 to 4 for preparing a medicament for the treatment and/or prophylaxis of diseases.

8. Use of a compound according to any of Claims 1 to 4 for preparing a medicament for the treatment and/or prophylaxis of cardiovascular disorders, thromboembolic disorders and/or tumour disorders.

9. Use of a compound according to any of Claims 1 to 4 for preventing blood coagulation *in vitro.*

10. Medicament, comprising a compound according to any of Claims 1 to 4 in combination with an inert non-toxic pharmaceutically acceptable auxiliary.

11. Medicament, comprising a compound according to any of Claims 1 to 4 in combination with a further active compound.

12. Medicament according to Claim 10 or 11 for the treatment and/or prophylaxis of cardiovascular disorders, thromboembolic disorders and/or tumour disorders.

13. Method for preventing blood coagulation *in vitro,* **characterized in that** an anticoagulatory amount of a compound according to any of Claims 1 to 4 is added.

## Revendications

1. Composé de formule 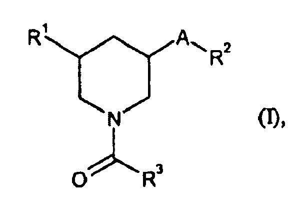 dans laquelle
A est un groupe de formule 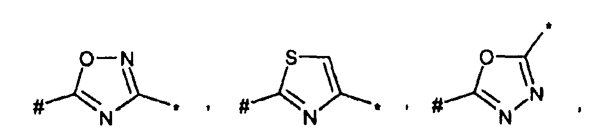 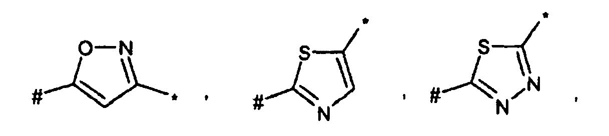 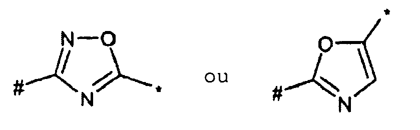 où
# est le point de liaison au cycle pipéridine,
et
* est le point de liaison à R²,
R¹ représente le groupe phényle,
le groupe phényle pouvant être substitué par 1 à 3 substituants choisis indépendamment les uns des autres dans le groupe consistant en les substituants monofluorométhyle, difluorométhyle, trifluorométhyle, monofluorométhoxy, difluorométhoxy, trifluorométhoxy, monofluorométhylsulfanyle, difluorométhylsulfanyle, trifluorométhylsulfanyle, méthylsulfonyle, alkyle en C₁-C₄, alcoxy en C₁-C₄, (alcoxy en C₁-C₄)carbonyle et cycloalkyle en C₃-C₆,
où le groupe alcoxy en C₂-C₄ peut être substitué par un substituant choisi dans le groupe consistant en les groupes méthoxy et éthoxy,
et
où le groupe cycloalkyle peut être substitué par 1 à 3 substituants choisis indépendamment les uns des autres dans le groupe consistant en les groupes halogéno et alkyle en C₁-C₄,
R² représente un atome d'hydrogène, un groupe trifluorométhyle, aminométhyle, alkyle en C₁-C₆, alcényle en C₂-C₆, (alcoxy en C₁-C₄) carbonyle, cycloalkyle en C₃-C₆, cyclopentényle, hétérocyclyle à 4 à 6 chaînons, phényle, 1,3-benzodioxolyle, hétéroaryle à 5 ou 6 chaînons ou pyridylaminocarbonyle,
les groupes cycloalkyle, hétérocyclyle, phényle et hétéroaryle pouvant être substitués par 1 à 3 substituants choisis chacun indépendamment des autres dans le groupe consistant en les substituants halogéno, cyano, amino, monofluorométhyle, difluorométhyle, trifluorométhyle, monofluorométhoxy, difluorométhoxy, trifluorométhoxy, monofluorométhylsulfanyle, difluorométhylsulfanyle, trifluorométhylsulfanyle, alkyle en C₁-C₄, alcoxy en C₁-C₄, alkylamino en C₁-C₆, (alcoxy en C₁-C₄)carbonylamino, cycloalkyle en C₃-C₆, hétérocyclyle à 4 à 6 chaînons, phényle et hétéroaryle à 5 ou 6 chaînons,
le groupe alkylamino pouvant être substitué par un substituant choisi dans le groupe consistant en les groupes alcoxy en C₁-C₄ et alkylamino en C₁-C₆,
et
où le groupe alkyle en C₁-C₄ peut être substitué par un substituant choisi dans le groupe consistant en les substituants halogéno, hydroxy, amino, aminocarbonyle, alcoxy en C₁-C₄, alkylamino en C₁-C₆, alkylthio en C₁-C₄, (alkyle en C₁-C₄) carbonyle, (alkyle en C₁-C₄) carbonyloxy, alkylsulfonyle en C₁-C₄, (alcoxy en C₁-C₄) carbonyle, (alcoxy en C₁-C₄) carbonylamino, cycloalkyle en C₃-C₆, cycloalkylamino en C₃-C₆, hétérocyclyle à 4 à 6 chaînons, phényle, phénoxy, hétéroaryle à 5 ou 6 chaînons et hétéroarylthio à 5 ou 6 chaînons,
où les groupes cycloalkyle, hétérocyclyle, phényle, phénoxy, hétéroaryle et hétéroarylthio peuvent être substitués par 1 à 3 substituants choisis indépendamment les uns des autres dans le groupe consistant en les substituants halogéno, cyano, hydroxyméthyle, monofluorométhyle, difluorométhyle, trifluorométhyl, monofluorométhoxy, difluorométhoxy, trifluorométhoxy, monofluorométhylsulfanyle, difluorométhylsulfanyle, trifluorométhylsulfanyle, alkyle en C₁-C₄, alcoxy en C₁-C₄, cycloalkyle en C₃-C₆, hétérocyclyle à 4 à 6 chaînons, phényle et hétéroaryle à 5 ou 6 chaînons,
R³ représente un groupe alkyle en C₁-C₆, alcoxy en C₁-C₆, alkylamino en C₁-C₆, cycloalkyle en C₃-C₇, hétérocyclyle à 4 à 6 chaînons, phényle ou hétéroaryle à 5 ou 6 chaînons,
où les groupes alkyle, alcoxy en C₂-C₆ et alkylamino peuvent être substitués par un substituant choisi dans le groupe consistant en les substituants hydroxy, amino, cyano et alcoxy en C₁-C₄,
et
où les groupes cycloalkyle, hétérocyclyle, phényle et hétéroaryle peuvent être substitués par 1 à 3 substituants choisis indépendamment les uns des autres dans le groupe consistant en les substituants halogéno, cyano, nitro, oxo, hydroxy, amino, aminométhyle, monofluorométhyle, difluorométhyle, trifluorométhyle, monofluorométhoxy, difluorométhoxy, trifluorométhoxy, monofluorométhylsulfanyle, difluorométhylsulfanyle, trifluorométhylsulfanyle, hydroxycarbonyle, aminocarbonyle, alkyle en C₁-C₄, alcoxy en C₁-C₄, alkylamino en C₁-C₆, (alcoxy en C₁-C₄) carbonyle et (alkyle en C₁-C₄) aminocarbonyle,
ou l'un de ses sels, de ses produits de solvatation ou l'un des produits de solvatation de ses sels.

2. Composé selon la revendication 1, **caractérisé en ce que**
A représente un groupe de formule 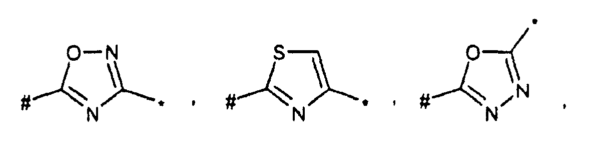 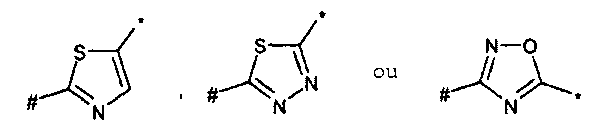 où
# est le point de liaison au cycle pipéridine,
et
* est le point de liaison à R²
R¹ représente le groupe phényle,
où le groupe phényle est substitué par 1 à 2 substituants choisis indépendamment l'un de l'autre dans le groupe consistant en les substituants trifluorométhyle, trifluorométhoxy, méthyle, éthyle, isopropyle et méthoxy,
R² représente le groupe méthyle, éthyle, isopropyle, n-propyle, tert-butyle, méthoxycarbonyle, éthoxycarbonyle, cyclopropyle, cyclobutyle, azétidinyle, oxétannyle, pyrrolidinyle, tétrahydropyridinyle, phényle, 1,3-benzodioxolyle, thiényle, furannyle, pyrrolyle, thiazolyle, isoxazolyle, imidazolyle, triazolyle, pyridyle, pyrimidinyle ou pyrazinyle,
où les groupes azétidinyle, oxétannyle, pyrrolidinyle, tétrahydropyridinyle, phényle, thiényle, furannyle, pyrrolyle, thiazolyle, isoxazolyle, imidazolyle, triazolyle, pyridyle, pyrimidinyle et pyrazinyle peuvent être substitués par 1 à 2 substituants choisis indépendamment l'un de l'autre dans le groupe consistant en les substituants halogéno, trifluorométhyle, trifluorométhoxy, méthyle, éthyle, méthoxy, éthoxy et éthylamino,
où le groupe éthylamino peut être substitué par un substituant choisi dans le groupe consistant en les substituants méthoxy et diméthylamino,
et
où les groupes méthyle et éthyle peuvent être substitués par un substituant choisi dans le groupe consistant en les substituants hydroxy, amino, méthoxy, éthoxy, isopropoxy, dialkylamino, méthylsulfonyle, cyclopropylamino, morpholinyle, phényle et phénoxy,
où le groupe phényle peut être substitué par 1 à 2 substituants choisis indépendamment l'un de l'autre dans le groupe consistant en les substituants halogéno, hydroxyméthyle, trifluorométhyle, trifluorométhoxy, méthyle, éthyle, méthoxy et éthoxy,
R³ représente un groupe tert-butyle, N-méthyl-N-éthylamino, méthoxyalkylamino, cyclopropyle, cyclopentyle, azétidinyle, 3,3-difluoroazétidinyle, 3-hydroxyazétidinyle, 3-méthylazétidinyle, 3-méthoxyazétidinyle, 3-diméthylaminoazétidinyle, pyrrolidinyle, 3,3-difluoropyrrolidin-1-yle, 3-hydroxypyrrolidin-1-yle, 3-aminopyrrolidin-1-yle, 4,4-difluoropipéridin-1-yle, 4-hydroxypipéridin-1-yle, 4-aminopipéridin-1-yle, 4-cyanopipéridin-1-yle, 3-méthoxypipéridin-1-yle, thiazolidinyle, morpholin-4-yle, 2,2-diméthylmorpholin-4-yle, 2-oxopipérazin-1-yle ou 3-oxo-4-méthylpipérazin-1-yle,
ou l'un de ses sels, l'un de ses produits de solvatation ou l'un des produits de solvatation de ses sels.

3. Composé selon la revendication 1 ou 2, **caractérisé en ce que**
A représente un groupe de formule 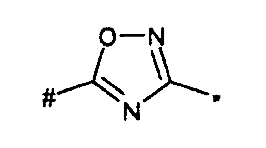 où
# représente le point de liaison au cycle pipéridine
et
* est le point de liaison à R²
R¹ représente le groupe phényle,
où le groupe phényle est substitué par 1 à 2 substituant choisis indépendamment l'un de l'autre dans le groupe consistant en les substituants trifluorométhyle, trifluorométhoxy, méthyle et éthyle, R² représente un groupe méthyle, éthyle ou isopropyle, où les groupes méthyle et éthyle peuvent être substitués par un substituant méthoxy,
R³ représente un groupe 3-hydroxyazétidinyle, 3-hydroxypyrrolidin-1-yle, 4-hydroxypipéridin-1-yle, 4-cyanopipéridin-1-yle ou morpholin-4-yle,
ou l'un de ses sels, l'un de ses produits de solvatation ou l'un des produits de solvatation de ses sels.

4. Composé selon l'une des revendications 1 à 3, **caractérisé en ce que** les substituants -R¹ et -A-R² sont en position cis l'un par rapport à l'autre.

5. Procédé de préparation d'un composé de formule (I) ou de l'un de ses sels, de l'un de ses produits de solvatation ou de l'un des produits de solvatation de ses sels selon la revendication 1, **caractérisé en ce qu'**on fait réagir
[A] un composé de formule 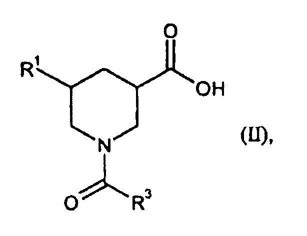 dans laquelle R¹ et R³ ont les significations données dans la revendication 1, avec un composé de formule 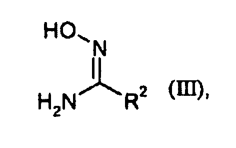 dans laquelle R² a les significations données dans la revendication 1,
ou
[B] un composé de formule 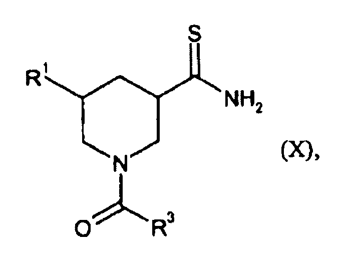 dans laquelle R¹ et R³ ont les significations données dans la revendication 1, avec un composé de formule 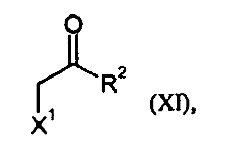 dans laquelle R² a les signiications données dans la revendication 1, et X¹ représente le brome ou le chlore, ou
[C] un composé de formule (II) avec un composé de formule
R²CONHNH₂ (XVI)
dans laquelle R² a les significations données dans la revendication 1, en présence de chlorure de phosphoryle ou de chlorure de thionyle,
ou
[D] un composé de formule (II), dans une première étape avec un composé de formule
R²COCH₂NH₂ (XVII)
dans laquelle R² a les significations données dans la revendication 1, en présence de chlorure de thionyle et de chlorure de phosphoryle, et dans la deuxième étape avec un réactif de Lawesson,
ou
[E] un composé de formule (II) avec un composé de formule (XVII) en présence de chlorure de thionyle ou de chlorure de phosphoryle,
ou
[F] un composé de formule (II), dans une première étape avec un composé de formule (XVI) en présence de chlorure de thionyle et de chlorure de phosphoryle, et dans la deuxième étape avec un réactif de Lawesson,
ou
[G] un composé de formule 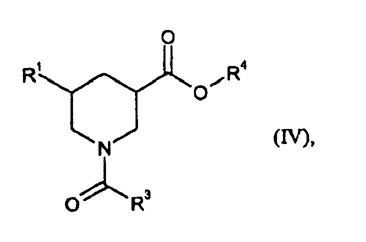 dans laquelle R¹ et R³ ont les significations données dans la revendication 1, et R⁴ est le groupe méthyle ou éthyle, avec un composé de formule 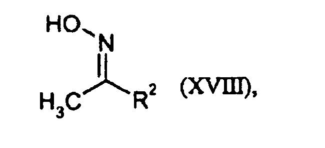 dans laquelle R² a les significations données dans la revendication 1, en présence de butyllythium,
ou
[H] un composé de formule 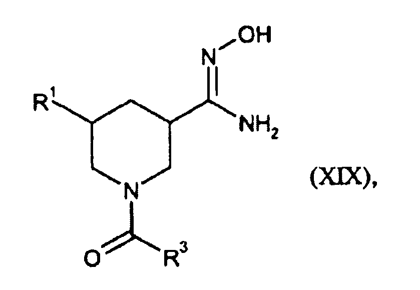 dans laquelle R¹ et R³ ont les significations données dans la revendication 1, avec un composé de formule 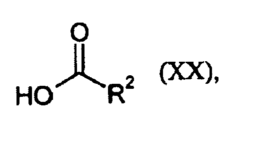 dans laquelle R² a les significations données dans la revendication 1,
ou
[J] un composé de formule 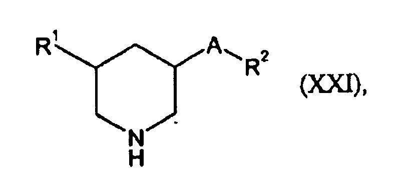 dans laquelle A, R¹ et R² ont les significations données dans la revendication 1, dans la première étape avec du chloroformiate de 4-nitrophényle et dans la deuxième étape avec un composé de formule
R³ - H (XXII)
dans laquelle R³ a les significations données dans la revendication 1.

6. Composé selon l'une des revendications 1 à 4 pour le traitement et/ou la prophylaxie de maladies.

7. Utilisation d'un composé selon l'une des revendications 1 à 4 pour préparer un médicament destiné au traitement et/ou à la prophylaxie de maladies.

8. Utilisation d'un composé selon l'une des revendications 1 à 4 pour préparer un médicament destiné au traitement et/ou à la prophylaxie de maladies cardiovasculaires, de maladies thrombo-emboliques et/ou de maladies tumorales.

9. Utilisation d'un composé selon l'une des revendications 1 à 4 pour empêcher une coagulation du sang in vitro.

10. Médicament contenant un composé selon l'une des revendications 1 à 4 en combinaison avec un adjuvant inerte, non toxique, pharmaceutiquement approprié.

11. Médicament contenant un composé selon l'une des revendications 1 à 4 en combinaison avec un autre principe actif.

12. Médicament selon la revendication 10 ou 11, pour le traitement et/ou la prophylaxie de maladies cardiovasculaires, de maladies thrombo-emboliques et/ou de maladies tumorales.

13. Procédé pour empêcher la coagulation du sang in vitro, **caractérisé en ce qu'**on ajoute une quantité à effet anticoagulant d'un composé selon l'une des revendications 1 à 4.
